# EUROPEAN PATENT APPLICATION

(11) **EP 3 789 378 A1**
(43) Date of publication of application: **10.03.2021**
(21) Application number: 19195942.8
(22) Date of filing: 06.09.2019
(51) Int. Cl.: C07D 213/64, C07D 401/04, C07D 401/12, C07D 401/14, C07D 405/14, C07D 413/14, C07D 417/04, C07D 417/14, C07D 419/14, A61P 37/08, A61P 31/12, A61P 11/00, A61K 31/506

(54) **PIPERIDINES OR PIPERIDONES SUBSTITUTED WITH UREA AND HETEROARYL**

(71) Applicant: Grünenthal GmbH, 52078 Aachen (DE)
(72) Inventor: Lesch, Bernhard, 52134 Herzogenrath (DE); Jakob, Florian, 52066 Aachen (DE); Pletinckx, Katrien, 52066 Aachen (DE); Arndt, Verena, 53113 Bonn (DE); Wagener, Markus, 52062 Aachen (DE); Dunkern, Torsten, 41363 Jüchen (DE)

(57) **Abstract**

The present invention relates to a compound according to general formula (I) 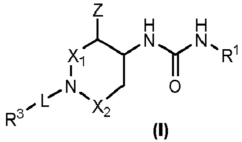 which acts as a modulator of FPR2 and can be used in the treatment and/or prophylaxis of disorders which are at least partially mediated by FPR2.

## Description

The present invention relates to a compound according to general formula (I) 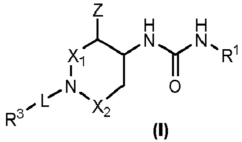 which acts as a modulator of FPR2 and can be used in the treatment and/or prophylaxis of disorders which are at least partially mediated by FPR2.

FPR2 (alias lipoxin A4 receptor, FPRL1, LXA4R, ALXR) is a G-protein coupled receptor family member that has been shown to mediate calcium mobilization in response to the eicosanoid family member lipoxin A4 (LXA4) and its analogues (Maddox et al., J. Biol. Chem., 1997, 272, 6972-6978). The receptor is widely expressed and has been shown to bind to a large number of different ligands, including endogenous proteins (serum amyloid A) bacterial products (the formyl peptide N- formyl-methionine-leucyl-phenylalanine), other lipid-derivatives (resolvin D1 (RvD1) and its analogues) and peptides, including neuropeptides (A_{β}42) and HIV (gp41 and gp120-derived peptides), amongst many others (Chiang et al., Pharmacol. Rev., 2006, 58, 463-487; Fredman and Serhan, Biochem. J., 2011, 437, 185-197; Migeotte et al., Cytokine Growth Factor Rev., 2006, 17, 501-519).

Wide ranging anti-inflammatory and pro-resolving effects of FPR2 ligands have been shown in pre-clinical models. For example, *in vivo* activities for LXA4, or derivatives, or stable analogues, and RvD1, or derivatives, or stable analogues have been demonstrated in arthritis, asthma, cardiovascular diseases, chronic obstructive pulmonary disease (COPD) colitis, corneal injury, cystic fibrosis, dermal inflammation, fibrosis of the lung and kidney, glomerulonephritis, graft versus host disease (GvHD), inflammatory pain, ischemia / reperfusion injury, periodontitis, peritonitis, post-operative pain, pancreatitis, retinopathy and sepsis (Fredman and Serhan, Biochem. J., 2011, 437, 185-197; Romano et al., Eur J Pharmacol. 2015, 760, 49-63; Yatomi et al., Physiol. Rep., 2015, 3, pii, e12628). It has been demonstrated that the FPR2 agonist 17-R-RvD1 is able to reduce the severity of arthritis in the K/BxN serum transfer model of arthritis (Norling et al., JCI Insight, 2016, 1, e85922) and the stable agonist BML-111 has similar actions in the collagen-induced arthritis model (Zhang et al., Inflamm. Res., 2008, 57, 157-162). The potential use of FPR2 modulators in lung diseases has been well documented. In terms of asthma, it has been demonstrated that the addition of LXA4 or RvD1, or their stable analogues, are able to improve asthmatic symptoms in animal models (Barnig et al., Sci. Transl. Med., 2013, 5, 174ra26; Levy et al., Nat Med. 2002, 8, 1018-23). It has also been demonstrated that in severe asthma patients there are reduced levels of LXA4 (Celik et al., Clin Exp Allergy, 2007, 37, 1494-1501). Similar reductions in pulmonary LXA4 levels were seen in patients with COPD (Vachier I et al,. J. Allergy Clin. Immunol., 2005, 115, 55-60) and cystic fibrosis (Karp et al., Nat. Immunol. 2004, 5, 388-392). RvD1 attenuated smoking-induced emphysema in vivo (Kim et al., Int. J. Chron. Obstruct. Pulmon. Dis., 2016, 11 1119-1128) and 17-R-RvD1 attenuates pulmonary fibrosis by inhibiting neutrophilic inflammation and promoting pulmonary restoration (Yatomi et al., Physiol. Rep., 2015, 3, pii, e12628).

In addition to anti-inflammatory and pro-resolution effects of FPR2-ligands, they have also been demonstrated to have effects on pain mechanisms. LXA4 has been directly shown to alleviate hyperalgesia and bone-cancer-related pain in animal models (Fredman and Serhan, Biochem. J., 2011, 437, 185-197; Hu et al., J. Neuroinflammation. 2012, 9, 278). Furthermore, the FPR2 agonist RvD1 has been shown to reduce inflammatory pain, spontaneous pain and post-operative pain and post-surgical pain (Ji et al., Trends Neurosci. 2011, 34, 599-609).

The biological properties of FPR2 agonists include, but are not limited to, regulation of inflammation, regulation of hyperalgesia, regulation of proinflammatory mediator production and/or release, regulation of migration and activation of monocytes/macrophages/microglia/astrocytes/dendritic cells and neutrophils, regulation of lymphocyte activation, regulate innalte lymphoid cell activation, proliferation and differentiation, regulation of cytokine production and/or release, regulation of immune reactions, regulation of phagocytosis/efferocytosis, regulation of apoptosis. Further, FPR2 is believed to be involved in the modulation of immune responses, such as those elicited through Graft versus Host Disease (GvHD).

Compounds which are active as modulators of FPR2 are also known from WO 2015/079692 and WO 2018/054549.

It was an object of the present invention to provide novel compounds which are modulators, preferably activators of FPR2, and which preferably have advantages over the compounds of the prior art. The novel compounds should in particular be suitable for use in the treatment and/or prophylaxis of disorders or diseases which are at least partially mediated by FPR2.

This object has been achieved by the subject-matter of the patent claims.

It was surprisingly found that the compounds according to the present invention are highly potent modulators of the FPR2 receptor.

The present invention relates to a compound according to general formula (I) 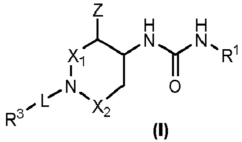 wherein
- X₂ represents CH₂ or C(O) and X₁ represents CH₂; or
- X₁ represents CH₂ or C(O) and X₂ represents CH₂;
and
- R¹: represents phenyl or 5 or 6-membered heteroaryl,
- Z: represents a 5 or 6 membered heteroaryl which is monosubstituted, disubstituted or trisubstituted with R² which in each case independently is selected from the group consisting of O-C₁₋₆-alkyl, H, F, Cl, Br, CN, C₁₋₆-alkyl, C₃₋₆-cycloalkyl, CHF₂, CH₂F, CF₃, OH, OCHF₂, OCH₂F, OCF₃, S-C₁₋₆-alkyl, S(O)-C₁₋₆-alkyl, S(O)₂-C₁₋₆-alkyl, O-C₃₋₆-cycloalkyl, S-C₃₋₆-cycloalkyl, S(O)-C₃₋₆-cycloalkyl, S(O)₂-C₃₋₆-cycloalkyl, NH₂, N(H)(C₁₋₆-alkyl), N(C₁₋₆-alkyl)₂, N(H)(C₃₋₆-cycloalkyl), N(C₁₋₆-alkyl)(C₃₋₆-cycloalkyl),NC(O)(C₁₋₆-alkyl), NC(O)(C₃₋₆-cycloalkyl), and NC(O)(3 to 6-membered heterocycloalkyl);
- L: represents bond, C₁₋₆-alkylene, C(O), S(O)₂, C(CH₃)₂, phenyl, or pyridyl; and
- R³: represents H, C₁₋₆-alkyl, C₃₋₆-cycloalkyl, 3 to 6-membered heterocycloalkyl, aryl, 5 or 6-membered heteroaryl, C₁₋₆-alkylene-OH, C₁₋₆-alkylene-O-C₁₋₆-alkyl, C₁₋₆-alkylene-NH₂, C₁₋₆-alkylene-N(H)-C₁₋₆-alkyl, C₁₋₆-alkylene-N(C₁₋₆-alkyl)₂, C₁₋₆-alkylene-C₃₋₆-cycloalkyl, C₁₋₆-alkylene-(3 to 6-membered heterocycloalkyl), C₁₋₆-alkylene-aryl, C₁₋₆-alkylene-(5 or 6-membered heteroaryl), C(O)NH₂, C(O)N(H)(C₁₋₆-alkyl), C(O)N(C₁₋₆-alkyl)₂, C(O)N(H)(C₃₋₆-cycloalkyl), C(O)N(H)(C₁₋₆-alkylene-C₃₋₆-cycloalkyl), C(O)N(H)(3 to 6-membered heterocycloalkyl), C(O)N(H)(C₁₋₆-alkylene-(3 to 6-membered heterocycloalkyl)), C(O)N(H)(aryl), C(O)N(H)(C₁₋₆-alkylene-aryl), C(O)N(H)(5 or 6-membered heteroaryl), C(O)N(H)(C₁₋₆-alkylene-(5 or 6-membered heteroaryl)), C(O)N(C₁₋₆-alkyl)(C₃₋₆-cycloalkyl), C(O)N(C₁₋₆-alkyl)(3 to 6-membered heterocycloalkyl), C(O)N(C₁₋₆-alkyl)(aryl), C(O)N(C₁₋₆-alkyl)(5 or 6-membered heteroaryl), C(O)N(C₃₋₆-cycloalkyl)(C₃₋₆-cycloalkyl), C(O)N(C₃₋₆-cycloalkyl)(3 to 6-membered heterocycloalkyl), C(O)N(C₃₋₆-cycloalkyl)(aryl), C(O)N(C₃₋₆-cycloalkyl)(5 or 6-membered heteroaryl), C(O)O-(C₁₋₆-alkyl), C(O)O-(C₃₋₆-cycloalkyl), C(O)O-(3 to 6-membered heterocycloalkyl), C(O)O-(aryl), C(O)O-(5 or 6-membered heteroaryl), S(O)-C₁₋₆-alkyl, S(O)₂-C₁₋₆-alkyl, S(O)-C₃₋₆-cycloalkyl, S(O)₂-C₃₋₆-cycloalkyl, S(O)-(3 to 6-membered heterocycloalkyl), S(O)₂-(3 to 6-membered heterocycloalkyl), S(O)-aryl; S(O)₂-aryl, S(O)-(5 or 6-membered heteroaryl), or S(O)₂-(5 or 6-membered heteroaryl),;
wherein C₁₋₆-alkyl in each case independently from one another is linear or branched, saturated or unsaturated;
wherein C₁₋₆-alkylene is linear and saturated or unsaturated;
wherein C₁₋₆-alkyl, C₁₋₆-alkylene, C₃₋₆-cycloalkyl and 3 to 6-membered heterocycloalkyl in each case independently from one another are unsubstituted or mono- or polysubstituted with one or more substituents selected from the group consisting of F, Cl, CN, C₁₋₆-alkyl, C₁₋₄-alkylene-OH, C₁₋₄-alkylene-OCH₃, CF₃, CF₂H, CFH₂, CF₂Cl, CFCl₂, C(O)-C₁₋₆-alkyl, C(O)-OH, C(O)-OC₁₋₆-alkyl, C(O)-NH₂, C(O)-N(H)(C₁₋₆-alkyl), C(O)-N(C₁₋₆-alkyl)₂, OH, =O, OCF₃, OCF₂H, OCFH₂, OCF₂Cl, OCFCl₂, O-C₁₋₆-alkyl, O-C(O)-C₁₋₆-alkyl, O-C(O)-O-C₁₋₆-alkyl, O-(CO)-N(H)(C₁₋₆-alkyl), O-C(O)-N(C₁₋₆-alkyl)₂, O-S(O)₂-NH₂, O-S(O)₂-N(H)(C₁₋₆-alkyl), O-S(O)₂-N(C₁₋₆-alkyl)₂, NH₂, N(H)(C₁₋₆-alkyl), N(C₁₋₆-alkyl)₂, N(H)-C(O)-C₁₋₆-alkyl, N(H)-C(O)-O-C₁₋₆-alkyl, N(H)-C(O)-NH₂, N(H)-C(O)-N(H)(C₁₋₆-alkyl), N(H)-C(O)-N(C₁₋₆-alkyl)₂, N(C₁₋₆-alkyl)-C(O)-C₁₋₆-alkyl, N(C₁₋₆-alkyl)-C(O)-O-C₁₋₆-alkyl, N(C₁₋₆-alkyl)-C(O)-NH₂, N(C₁₋₆-alkyl)-C(O)-N(H)(C₁₋₆-alkyl), N(C₁₋₆-alkyl)-C(O)-N(C₁₋₆-alkyl)₂, N(H)-S(O)₂OH, ON(H)-S(O)₂-C₁₋₆-alkyl, N(H)-S(O)₂-O-C₁₋₆-alkyl, N(H)-S(O)₂-NH₂, N(H)-S(O)₂-ON(H)(C₁₋₆-alkyl), N(H)-S(O)₂N(C₁₋₆-alkyl)₂, N(C₁₋₆-alkyl)-S(O)₂-OH, N(C₁₋₆-alkyl)-S(O)₂-C₁₋₆-alkyl, N(C₁₋₆-alkyl)-S(O)₂-O-C₁₋₆-alkyl, N(C₁₋₆-alkyl)-S(O)₂-NH₂, N(C₁₋₆-alkyl)-S(O)₂-N(H)(C₁₋₆-alkyl), N(C₁₋₆-alkyl)-S(O)₂-N(C₁₋₆-alkyl)₂, SCF₃, SCF₂H, SCFH2, S-C₁₋₆-alkyl, S(O)-C₁₋₆-alkyl, S(O)₂-C₁₋₆-alkyl, S(O)₂-OH, S(O)₂-O-C₁₋₆-alkyl, S(O)₂-NH₂, S(O)₂-N(H)(C₁₋₆-alkyl), S(O)₂-N(C₁₋₆-alkyl)₂, C₃₋₆-cycloalkyl, 3 to 6-membered heterocycloalkyl, phenyl, 5 or 6-membered heteroaryl, O-C₃₋₆-cycloalkyl, O-(3 to 6-membered heterocycloalkyl), O-phenyl, O-(5 or 6-membered heteroaryl), C(O)-C₃₋₆-cycloalkyl, C(O)-(3 to 6-membered heterocycloalkyl), C(O)-phenyl, C(O)-(5 or 6-membered heteroaryl), S(O)₂-(C₃₋₆-cycloalkyl), S(O)₂-(3 to 6-membered heterocycloalkyl) and S(O)₂-phenyl or S(O)₂-(5 or 6-membered heteroaryl);
wherein aryl, phenyl and 5 or 6-membered heteroaryl in each case independently from one another are unsubstituted or mono- or disubstituted with one or more substituents selected from the group consisting of F, Cl, Br, CN, C₁₋₆-alkyl, CF₃, CF₂H, CFH₂, C₁₋₄-alkylene-CF₃, C₁₋₄-alkylene-CF₂H, C₁₋₄-alkylene-CFH₂, C₁₋₄-alkylene-OH, C₁₋₄-alkylene-OCH₃, C(O)-C₁₋₆-alkyl, C(O)-OH, C(O)-OC₁₋₆-alkyl, C(O)-N(H)(OH), C(O)-NH₂, C(O)-N(H)(C₁₋₆-alkyl), C(O)-N(C₁₋₆-alkyl)₂, OH, OCF₃, OCF₂H, OCFH₂, OCF₂Cl, OCFCl₂, O-C₁₋₆-alkyl, O-C₃₋₆-cycloalkyl, O-(3 to 6-membered heterocycloalkyl), NH₂, N(H)(C₁₋₆-alkyl), N(H)(C₁₋₆-alkylene-OH), N(C₁₋₆-alkyl)₂, N(H)-C(O)-C₁₋₆-alkyl, N(C₁₋₆-alkyl)-C(O)-C₁₋₆-alkyl, N(H)-C(O)-NH₂, N(H)-C(O)-N(H)(C₁₋₆-alkyl), N(H)-C(O)-N(C₁₋₆-alkyl)₂, N(C₁₋₆-alkyl)-C(O)-N(H)(C₁₋₆-alkyl), N(C₁₋₆-alkyl)-C(O)-N(C₁₋₆-alkyl)₂, N(H)-S(O)₂-C₁₋₆-alkyl, SCF₃, S-C₁₋₆-alkyl, S(O)-C₁₋₆-alkyl, S(O)₂-C₁₋₆-alkyl, S(O)₂-NH₂, S(O)₂-N(H)(C₁₋₆-alkyl), S(O)₂-N(C₁₋₆-alkyl)₂, C₃₋₆-cycloalkyl, C₁₋₄-alkylene-C₃₋₆-cycloalkyl, 3 to 6-membered heterocycloalkyl, C₁₋₄-alkylene-(3 to 6-membered heterocycloalkyl), phenyl and 5 or 6-membered heteroaryl;
in the form of the free compound or a physiologically acceptable salt thereof;
with the proviso that the following compounds are excluded:
1-(4-Chlorophenyl)-3-[5-(6-methoxy-pyridin-3-yl)-2-oxo-1-pyrimidin-4-yl-piperidin-4-yl]-urea and
trans-1-[3-(5-Chloro-pyridin-2-yl)-1-(2-hydroxy-acetyl)-piperidin-4-yl]-3-phenyl-urea.

In a preferred embodiment, the compound according to the present invention is present in form of the free compound. For the purpose of specification, "free compound" preferably means that the compound according to the present invention is not present in form of a salt. Methods to determine whether a chemical substance is present as the free compound or as a salt are known to the skilled artisan such as ¹⁴N or ¹⁵N solid state NMR, x-ray diffraction, x-ray powder diffraction, IR, Raman, XPS. ¹H-NMR recorded in solution may also be used to consider the presence of protonation.

In another preferred embodiment, the compound according to the present invention is present in form of a physiologically acceptable salt. For the purposes of this specification, the term "physiologically acceptable salt" preferably refers to a salt obtained from a compound according to the present invention and a physiologically acceptable acid or base.

According to the present invention, the compound according to the present invention may be present in any possible form including solvates, cocrystals and polymorphs. For the purposes of this specification, the term "solvate" preferably refers to an adduct of (i) a compound according to the present invention and/or a physiologically acceptable salt thereof with (ii) distinct molecular equivalents of one or more solvents.

Further, the compound according to the present invention may be present in form of the racemate, enantiomers, diastereomers, tautomers or any mixtures thereof.
The compounds according to general formula (I) possess at least two stereogenic carbon atoms and may be stereochemically differentiated according to the relative structural orientation of the phenyl moiety and the urea moiety which are bound to the central nitrogen-containing 6-membered heterocycloalkyl.

In the sense of the present invention, the term "diastereomer" refers to a compound preferably having a diastereomeric ratio of > 90:10, more preferably > 92:8, even more preferably > 95:5, most preferably > 98:2 and in particular > 99:1 or > 99.9:1. Diastereomers differ from each other with respect to their physical and chemical properties. Methods to determine the diastereomeric ratio (dr) are well known to the person skilled in the art and include, but are not limited to, NMR-methods.

In the sense of the present invention, the term "enantiomerically pure compound" or "enantiomer" preferably refers to a compound having an enantiomeric excess of > 90 %ee, more preferably > 92 %ee, still more preferably > 95 %ee, most preferably > 98 %ee and in particular > 98 %ee. Methods to determine the enantiomeric excess are well known to the person skilled in the art and include, but are not limited to, optical rotary dispersion, circular dichroism, NMR-methods using chiral auxiliaries ("shift reagents") or separation via chiral HPLC (high performance liquid chromatography, using a chiral stationary phase), chiral GLC (gas-liquid chromatography, using a chiral stationary phase phase) or chiral SFC (supercritical fluid chromatography using a chiral stationary phase).

Further in the sense of the present invention, the term "racemic mixture" or "racemate" refers to a mixture (identified by the prefix "rac-trans" or "rac-cis" in the chemical name) of two corresponding enantiomers wherein said corresponding enantiomers are preferably contained in the mixture in a ratio of from 30:70 to 70:30, more preferably 40:60 to 60:40, most preferably 45:55 to 55:45 and in particular 50:50.

Further in the sense of the present invention, the term "iso-mix" refers to a mixture (identified by the prefix "iso-mix" in the chemical name) of two corresponding diastereomers, wherein said corresponding diastereomers are preferably contained in the mixture in a ratio of from 30:70 to 70:30, more preferably 40:60 to 60:40, most preferably 45:55 to 55:45 and in particular 50:50.

Determination of the absolute stereochemical structure is well known to the person skilled in the art and includes, but are not limited to, x-ray diffractometry.

In the sense of the present invention, the compounds wherein the substituent Z and urea moiety which are connected to the central nitrogen-containing 6-membered heterocycloalkyl have a different relative orientation, for instance Z moiety up ("bold bond", 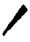 ) and urea moiety down ("hashed bond", 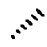 ) or vice versa, are referred to as the "trans" diastereomer and are identified hereinafter by the prefix "trans" in the chemical name and the term "AND Enantiomer" next to the chemical formula (see general formula trans-II below):

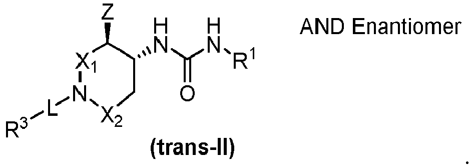

The trans diastereomer is a racemic mixture of two corresponding enantiomers which are identified via the suffix "ent-1" and "ent-2" in the chemical name, and which are according to general formulae (IIa) and (IIb) shown below:

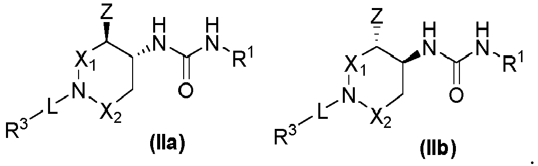

In the following, either one of the two enantiomers "ent-1" (identified by the prefix "trans" and the suffix"ent-l" in the chemical name) and "ent-2" (identified by the prefix "trans" and the suffix"ent-2" in the chemical name) is according to general formula (IIa) while the other is according to general formula (IIb). For the purpose of clarification, one of ent-1 and ent-2 has to be according to general formula (IIa) and the other one has to be according to general formula (IIb).

Further in the sense of the present invention, the compounds wherein the substituent Z and urea moiety which are connected to the central nitrogen-containing 6-membered heterocycloalkyl have the same relative orientation, for instance both, the Z moiety and the urea moiety, up ("bold bond", 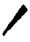 ) or both, the Z moiety and the urea moiety, down ("hashed bond", 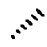 ) are referred to as the "cis" diastereomer and are identified hereinafter by the prefix "cis" in the chemical name and the term "AND Enantiomer" next to the chemical formula (see general formula cis-II below):

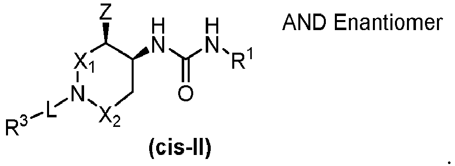

The cis diastereomer is a racemic mixture of two enantiomers which are identified via the suffix "ent-1" and "ent-2" in the chemical name, and which are according to general formulae (IIc) and (IId) shown below:

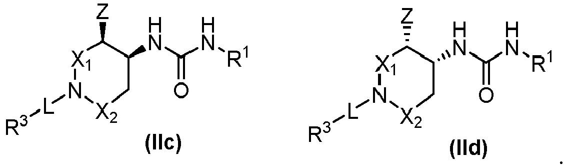

In the following, either one of the two enantiomers "ent-1" (identified by the prefix "cis" and the suffix"ent-l" in the chemical name) and "ent-2" (identified by the prefix "cis" and the suffix"ent-2" in the chemical name) is according to general formula (IIc) while the other is according to general formula (IId). For the purpose of clarification, one of ent-1 and ent-2 has to be according to general formula (IIc) and the other one has to be according to general formula (IId).

In the following, either one of the terms "diastereomer 1" (identified by the suffix "dia-1" in the chemical name) and "diastereomer 2" (identified by the suffix "dia-2" in the chemical name) refers to the cis diastereomer while the other refers to the trans diastereomer. For the purpose of clarification, one of the diastereomers 1 and 2 has to be cis and the other one has to be trans.

In the sense of the present invention, a chemical formula where the Z and the urea moieties are each connected to the central nitrogen-containing 6-membered heterocycloalkyl by "solid bonds" ( 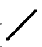 ) shall refer to a mixture of the trans diastereomer and the cis diastereomer, i.e. a mixture of diastereomer 1 and diastereomer 2.

The present invention also includes isotopic isomers of a compound of the invention, wherein at least one atom of the compound is replaced by an isotope of the respective atom which is different from the naturally predominantly occurring isotope, as well as any mixtures of isotopic isomers of such a compound. Preferred isotopes are ²H (deuterium), ³H (tritium), ¹³C and ¹⁴C. Isotopic isomers of a compound of the invention can generally be prepared by conventional procedures known to a person skilled in the art.

According to the present invention, the terms "C₁₋₆-alkyl" and "C₁₋₄-alkyl" preferably mean acyclic saturated or unsaturated aliphatic (i.e. non-aromatic) hydrocarbon residues, which can be linear (i.e. unbranched) or branched and which can be unsubstituted or mono- or polysubstituted (e.g. di- or trisubstituted), and which contain 1 to 6 (i.e. 1, 2, 3, 4, 5 or 6) and 1 to 4 (i.e. 1, 2, 3 or 4) carbon atoms, respectively. Preferably, C₁₋₆-alkyl and C₁₋₄-alkyl are saturated. Preferred C₁₋₆-alkyl groups are selected from the group consisting of methyl, ethyl, n-propyl, 2-propyl, n-butyl, isobutyl, sec-butyl, tert-butyl, n-pentyl, 2-pentyl, 3-pentyl, 2-methylbutyl, 3-methylbutyl, 3-methylbut-2-yl, 2-methylbut-2-yl, 2,2-dimethylpropyl, n-hexyl, 2-hexyl, 3-hexyl, 2-methylpentyl, 4-methylpentyl, 4-methylpent-2-yl, 2-methylpent-2-yl, 3,3-dimethylbutyl, 3,3-dimethylbut-2-yl, 3-methylpentyl, 3-methylpent-2-yl and 3-methylpent-3-yl; more preferably methyl, ethyl, n-propyl, 2-propyl, n-butyl, isobutyl, sec-butyl, tert-butyl, n-pentyl, 2-pentyl, 3-pentyl, 2-methylbutyl, 3-methylbutyl, 3-methylbut-2-yl, 2-methylbut-2-yl, 2,2-dimethylpropyl, n-hexyl. Particularly preferred C₁₋₆-alkyl groups are selected from C₁₋₄-alkyl groups. Preferred C₁₋₄-alkyl groups are selected from the group consisting of methyl, ethyl, n-propyl, 2-propyl, n-butyl, isobutyl, sec-butyl and tert-butyl.

Further according to the present invention, the terms "C₁₋₆-alkylene" and "C₁₋₄-alkylene" relate to a linear and preferably saturated aliphatic residues which are preferably selected from the group consisting of methylene (-CH₂-), ethylene (-CH2CH2-), propylene (-CH2CH2CH2-), butylene (-CH2CH2CH2CH2-), pentylene (-CH₂CH₂CH₂CH₂CH₂-) and hexylene (-CH₂CH₂CH₂CH₂CH₂CH₂-); more preferably methylene (-CH₂-) and ethylene (-CH₂CH₂-) and most preferably methylene (-CH₂-). Preferably, C₁₋₆-alkylene is selected from C₁₋₄-alkylene.

Still further according to the present invention, the term "C₃₋₆-cycloalkyl" preferably means cyclic aliphatic hydrocarbons containing 3, 4, 5 or 6 carbon atoms, wherein the hydrocarbons in each case can be saturated or unsaturated (but not aromatic), unsubstituted or mono- or polysubstituted. The C₃₋₆-cycloalkyl group can be bound to the respective superordinate general structure via any desired and possible ring member of the cycloalkyl group. The C₃₋₆-cycloalkyl group can also be condensed with further saturated, (partially) unsaturated, (hetero)cyclic, aromatic or heteroaromatic ring systems, i.e. with cycloalkyl, heterocyclyl, aryl or heteroaryl residues, which in each case can in turn be unsubstituted or mono- or polysubstituted. Further, the C₃₋₆-cycloalkyl group can be singly or multiply bridged such as, for example, in the case of adamantyl, bicyclo[2.2.1]heptyl or bicyclo[2.2.2]octyl. However, preferably, the C₃₋₆-cycloalkyl group is neither condensed with further ring systems nor bridged.

Preferred C₃₋₆-cycloalkyl groups are selected from the group consisting of cyclopropyl, cyclobutyl, cyclopentyl, cyclohexyl, cyclopentenyl and cyclohexenyl. Particularly preferred C₃₋₆-cycloalkyl groups are selected from the group consisting of cyclopropyl, cyclobutyl, cyclopentyl and cyclohexyl, most preferably cyclopropyl and cyclobutyl.

According to the present invention, the term "3 to 6-membered heterocycloalkyl" preferably means heterocycloaliphatic saturated or unsaturated (but not aromatic) residues having 3 to 6, i.e. 3, 4, 5 or 6 ring members, wherein in each case at least one, if appropriate also two or three carbon atoms are replaced by a heteroatom or a heteroatom group each selected independently of one another from the group consisting of O, S, S(=O), S(=O)₂, N, NH and N(C₁₋₄-alkyl) such as N(CH₃), wherein the carbon atoms of the ring can be unsubstituted or mono- or polysubstituted. The 3 to 6-membered heterocycloalkyl group can also be condensed with further saturated or (partially) unsaturated cycloalkyl or heterocyclyl, aromatic or heteroaromatic ring systems. However, preferably, the 3 to 6-membered heterocycloalkyl group is not condensed with further ring systems. The 3 to 6-membered heterocycloalkyl group can be bound to the superordinate general structure via any desired and possible ring member of the heterocycloaliphatic residue if not indicated otherwise. In a preferred embodiment, the 3 to 6-membered heterocycloalkyl group is bound to the superordinate general structure via a carbon atom.

Preferred 3 to 6-membered heterocycloalkyl groups are selected from the group consisting of tetrahydropyranyl, piperidinyl, oxetanyl, azetidinyl, tetrahydrothiopyran 1,1-dioxide, morpholinyl, pyrrolidinyl, 4-methylpiperazinyl, morpholinonyl, aziridinyl, dithiolanyl, dihydropyrrolyl, dioxanyl, dioxolanyl, dihydropyridinyl, dihydrofuranyl, dihydroisoxazolyl, dihydrooxazolyl, imidazolidinyl, isoxazolidinyl, oxazolidinyl, oxiranyl, piperazinyl, pyrazolidinyl, pyranyl, tetrahydropyrrolyl, more preferably tetrahydropyranyl, piperidinyl, oxetanyl, azetidinyl, and tetrahydrothiopyran 1,1-dioxide.

According to the present invention, the term "aryl" preferably means aromatic hydrocarbons having 6 to 14, i.e. 6, 7, 8, 9, 10, 11, 12, 13 or 14 ring members, preferably having 6 to 10, i.e. 6, 7, 8, 9 or 10 ring members, including phenyls and naphthyls. Each aryl residue can be unsubstituted or mono- or polysubstituted. The aryl can be bound to the superordinate general structure via any desired and possible ring member of the aryl residue. The aryl residues can also be condensed with further saturated or (partially) unsaturated cycloalkyl or heterocycloalkyl, aromatic or heteroaromatic ring systems, which can in turn be unsubstituted or mono- or polysubstituted. In a preferred embodiment, aryl is condensed with a further ring system. Examples of condensed aryl residues are 2H-benzo[b][1,4]oxazin-3(4H)-onyl, 1H-benzo[d]imidazolyl, 2,3-dihydro-1H-indenyl, tetrahydronaphthalenyl, isochroman, 1,3-dihydroisobenzofuranyl, benzodioxolanyl and benzodioxanyl. Preferably, aryl is selected from the group consisting of phenyl, 1H-benzo[d]imidazolyl, 2H-benzo[b][1,4]oxazin-3(4H)-onyl, 2,3-dihydro-1H-indenyl, tetrahydronaphthalenyl, isochroman, 1,3-dihydroisobenzofuranyl, 1-naphthyl, 2-naphthyl, fluorenyl and anthracenyl, each of which can be respectively unsubstituted or mono- or polysubstituted. In another preferred embodiment, aryl is not condensed with any further ring system. A particularly preferred aryl is phenyl, unsubstituted or mono- or polysubstituted.

According to the present invention, the term "5- to 6-membered heteroaryl" preferably means a 5 or 6-membered cyclic aromatic residue containing at least 1, if appropriate also 2, 3, 4 or 5 heteroatoms, wherein the heteroatoms are each selected independently of one another from the group S, N and O and the heteroaryl residue can be unsubstituted or mono- or polysubstituted, if not indicated otherwise. In the case of substitution on the heteroaryl, the substituents can be the same or different and be in any desired and possible position of the heteroaryl. The binding to the superordinate general structure can be carried out via any desired and possible ring member of the heteroaryl residue if not indicated otherwise. Preferably, the 5- to 6-membered heteroaryl is bound to the suprordinate general structure via a carbon atom of the heterocycle. The heteroaryl can also be part of a bi- or polycyclic system having up to 14 ring members, wherein the ring system can be formed with further saturated or (partially) unsaturated cycloalkyl or heterocycloalkyl, aromatic or heteroaromatic ring systems, which can in turn be unsubstituted or mono- or polysubstituted, if not indicated otherwise. In a preferred embodiment, the 5- to 6-membered heteroaryl is part of a bi- or polycyclic, preferably bicyclic, system. In another preferred embodiment, the 5- to 6-membered heteroaryl is not part of a bi- or polycyclic system.

Preferably, the 5- to 6-membered heteroaryl is selected from the group consisting of pyrimidinyl, pyrazinyl, pyridazinyl, pyridyl (i.e. 2-pyridyl, 3-pyridyl, 4-pyridyl), pyrazolyl, isoxazolyl, triazolyl, oxazolyl, oxadiazolyl, indazolyl, pyrazolopyridyl, isoindolinonyl, pyrrolyl, imidazolyl, thiazolyl, isothiazolyl, furanyl, thienyl (thiophenyl), thiadiazolyl, 4,5,6,7-tetrahydro-2H-indazolyl, 2,4,5,6-tetrahydrocyclopenta[c]pyrazolyl, benzofuranyl, benzoimidazolyl, benzothienyl, benzothiadiazolyl, benzothiazolyl, benzotriazolyl, benzooxazolyl, benzooxadiazolyl, quinazolinyl, quinoxalinyl, carbazolyl, quinolinyl, dibenzofuranyl, dibenzothienyl, imidazothiazolyl, indazolyl, indolizinyl, indolyl, isoquinolinyl, naphthyridinyl, phenazinyl, phenothiazinyl, phthalazinyl, purinyl, phenazinyl, tetrazolyl and triazinyl. Particularly preferred 5- to 6-membered heteroaryl are selected from the group consisting of pyrimidinyl, pyrazinyl, pyridazinyl, pyridyl, pyrazolyl, isoxazolyl, triazolyl, oxazolyl, and oxadiazolyl.

In connection with the terms "C₁₋₆-alkyl", "C₁₋₄-alkyl", "C₃₋₆-cycloalkyl", "3 to 6-membered heterocycloalkyl" and "C₁₋₆-alkylene", the term "substituted" refers in the sense of the present invention, with respect to the corresponding residues or groups, to the single substitution (monosubstitution) or multiple substitution (polysubstitution), e.g. disubstitution or trisubstitution; more preferably to monosubstitution or disubstitution;
of one or more hydrogen atoms each independently of one another by at least one substituent. In case of a multiple substitution, i.e. in case of polysubstituted residues, such as di- or trisubstituted residues, these residues may be polysubstituted either on different or on the same atoms, for example trisubstituted on the same carbon atom, as in the case of CF₃, CH₂CF₃ or disubstituted as in the case of 1,1-difluorocyclohexyl, or at various points, as in the case of CH(OH)-CH=CH-CHCl₂ or 1-chloro-3-fluorocyclohexyl. The multiple substitution can be carried out using the same or using different substituents.

In relation to the terms "phenyl", "aryl" and "5- to 6-membered heteroaryl", the term "substituted" refers in the sense of this invention to the single substitution (monosubstitution) or multiple substitution (disubstitution), of one or two hydrogen atoms each independently of one another by at least one substituent. The disubstitution can be carried out using the same or using different substituents.

If a residue occurs multiply within a molecule, then this residue can have respectively different meanings for various substituents: if, for example, both R² and R³ denote C₁₋₆-alkyl, then C₁₋₆-alkyl can e.g. represent ethyl for R² and can represent methyl for R³.

According to the present invention, C₁₋₆-alkyl, C₁₋₆-alkylene, C₃₋₆-cycloalkyl and 3 to 6-membered heterocycloalkyl in each case independently from one another are unsubstituted or mono- or polysubstituted with one or more substituents selected from the group consisting of F, Cl, CN, C₁₋₆-alkyl, C₁₋₄-alkylene-OH, C₁₋₄-alkylene-OCH₃, CF₃, CF₂H, CFH₂, CF₂Cl, CFCl₂, C(O)-C₁₋₆-alkyl, C(O)-OH, C(O)-OC₁₋₆-alkyl, C(O)-NH₂, C(O)-N(H)(C₁₋₆-alkyl), C(O)-N(C₁₋₆-alkyl)₂, OH, =O, OCF₃, OCF₂H, OCFH₂, OCF₂Cl, OCFCl₂, O-C₁₋₆-alkyl, O-C(O)-C₁₋₆-alkyl, O-C(O)-O-C₁₋₆-alkyl, O-(CO)-N(H)(C₁₋₆-alkyl), O-C(O)-N(C₁₋₆-alkyl)₂, O-S(O)₂-NH₂, O-S(O)₂-N(H)(C₁₋₆-alkyl), O-S(O)₂-N(C₁₋₆-alkyl)₂, NH₂, N(H)(C₁₋₆-alkyl), N(C₁₋₆-alkyl)₂, N(H)-C(O)-C₁₋₆-alkyl, N(H)-C(O)-O-C₁₋₆-alkyl, N(H)-C(O)-NH₂, N(H)-C(O)-N(H)(C₁₋₆-alkyl), N(H)-C(O)-N(C₁-₆-alkyl)₂, N(C₁₋₆-alkyl)-C(O)-C₁₋₆-alkyl, N(C₁₋₆-alkyl)-C(O)-O-C₁₋₆-alkyl, N(C₁₋₆-alkyl)-C(O)-NH₂, N(C₁₋₆-alkyl)-C(O)-N(H)(C₁₋₆-alkyl), N(C₁₋₆-alkyl)-C(O)-N(C₁₋₆-alkyl)₂, N(H)-S(O)₂OH, N(H)-S(O)₂-C₁₋₆-alkyl, N(H)-S(O)₂-O-C₁₋₆-alkyl, N(H)-S(O)₂-NH₂, N(H)-S(O)₂-N(H)(C₁₋₆-alkyl), N(H)-S(O)₂N(C₁₋₆-alkyl)₂, N(C₁₋₆-alkyl)-S(O)₂-OH, N(C₁₋₆-alkyl)-S(O)₂-C₁₋₆-alkyl, N(C₁₋₆-alkyl)-S(O)₂-O-C₁₋₆-alkyl, N(C₁₋₆-alkyl)-S(O)₂-NH₂, N(C₁₋₆-alkyl)-S(O)₂-N(H)(C₁₋₆-alkyl), N(C₁₋₆-alkyl)-S(O)₂-N(C₁₋₆-alkyl)₂, SCF₃, SCF₂H, SCFH₂, S-C₁₋₆-alkyl, S(O)-C₁₋₆-alkyl, S(O)₂-C₁₋₆-alkyl, S(O)₂-OH, S(O)₂-O-C₁₋₆-alkyl, S(O)₂-NH₂, S(O)₂-N(H)(C₁₋₆-alkyl), S(O)₂-N(C₁₋₆-alkyl)₂, C₃₋₆-cycloalkyl, 3 to 6-membered heterocycloalkyl, phenyl, 5 or 6-membered heteroaryl, O-C₃₋₆-cycloalkyl, O-(3 to 6-membered heterocycloalkyl), O-phenyl, O-(5 or 6-membered heteroaryl), C(O)-C₃₋₆-cycloalkyl, C(O)-(3 to 6-membered heterocycloalkyl), C(O)-phenyl, C(O)-(5 or 6-membered heteroaryl), S(O)₂-(C₃₋₆-cycloalkyl), S(O)₂-(3 to 6-membered heterocycloalkyl) and S(O)₂-phenyl or S(O)₂-(5 or 6-membered heteroaryl). Preferred substituents of C₁₋₆-alkyl, C₁₋₆-alkylene, C₃₋₆-cycloalkyl and 3 to 6-membered heterocycloalkyl are selected from the group consisting of F, Cl, Br, CN, C₁₋₆-alkyl, CH₂OH, CH₂OCH₃, CF₃, CF₂H, CFH₂, C(O)NH₂, C(O)N(H)CH₃, C(O)N(CH₃)₂, OH, O(C₁₋₆-alkyl), OCF₃, OCH₂CH₃, O(CH₂)₂CH₃, OCH(CH₃)₂, N(H)C(O)CH₃, N(H)S(O)₂CH₃, S(O)CH₃, and S(O)₂CH₃. Preferably, C₁₋₆-alkylene groups are unsubstituted.

According to the present invention, aryl, phenyl and 5 or 6-membered heteroaryl in each case independently from one another are unsubstituted or mono- or disubstituted with one or more substituents selected from the group consisting of F, Cl, Br, CN, C₁₋₆-alkyl, CF₃, CF₂H, CFH₂, C₁₋₄-alkylene-CF₃, C₁₋₄-alkylene-CF₂H, C₁₋₄-alkylene-CFH₂, C₁₋₄-alkylene-OH, C₁₋₄-alkylene-OCH₃, C(O)-C₁₋₆-alkyl, C(O)-OH, C(O)-OC₁₋₆-alkyl, C(O)-N(H)(OH), C(O)-NH₂, C(O)-N(H)(C₁₋₆-alkyl), C(O)-N(C₁₋₆-alkyl)₂, OH, OCF₃, OCF₂H, OCFH₂, OCF₂Cl, OCFCl₂, O-C₁₋₆-alkyl, O-C₃₋₆-cycloalkyl, O-(3 to 6-membered heterocycloalkyl), NH₂, N(H)(C₁₋₆-alkyl), N(H)(C₁₋₆-alkylene-OH), N(C₁₋₆-alkyl)₂, N(H)-C(O)-C₁₋₆-alkyl, N(C₁₋₆-alkyl)-C(O)-C₁₋₆-alkyl, N(H)-C(O)-NH₂, N(H)-C(O)-N(H)(C₁₋₆-alkyl), N(H)-C(O)-N(C₁₋₆-alkyl)₂, N(C₁₋₆-alkyl)-C(O)-N(H)(C₁₋₆-alkyl), N(C₁₋₆-alkyl)-C(O)-N(C₁₋₆-alkyl)₂, N(H)-S(O)₂-C₁₋₆-alkyl, SCF₃, S-C₁₋₆-alkyl, S(O)-C₁₋₆-alkyl, S(O)₂-C₁₋₆-alkyl, S(O)₂-NH₂, S(O)₂-N(H)(C₁₋₆-alkyl), S(O)₂-N(C₁₋₆-alkyl)₂, C₃₋₆-cycloalkyl, C₁₋₄-alkylene-C₃₋₆-cycloalkyl, 3 to 6-membered heterocycloalkyl, C₁₋₄-alkylene-(3 to 6-membered heterocycloalkyl), phenyl and 5 or 6-membered heteroaryl. Preferred substituents of aryl, phenyl and 5 or 6-membered heteroaryl are selected from the group consisting of F, Cl, Br, CN, C₁₋₆-alkyl, CF₃, CF₂H, CFH, cyclopropyl, CH₂OH, CH₂CH₂OH, CH₂OCH₃, CH₂CH₂OCH₃, OH, OCF₃, OCF₂H, OCFH₂, O-C₁₋₆-alkyl, N(H)CH₂CH₂OH, and S(O)₂CH₃.

In a preferred embodiment the compound according to the present invention is according to general formula (IIa) or (IIb)

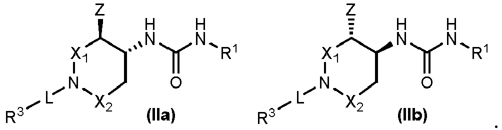

In another preferred embodiment, the compound according to the present invention is according to general formula (IIa). In still another preferred embodiment, the compound according to the present invention is according to general formula (IIb).

In a further preferred embodiment, the compound according to the present invention is according to general formula (IIc) or (IId)

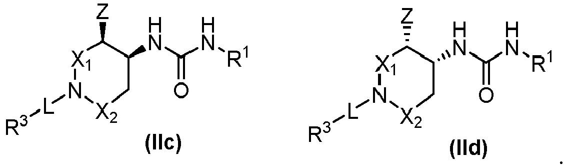

In a preferred embodiment, the compound according to the present invention is according to general formula (IIa) or (IIb).

According to the present invention,
- X₂ represents CH₂ or C(O) and X₁ represents CH₂; or
- X₁ represents CH₂ or C(O) and X₂ represents CH₂.

Preferably, X₂ represents CH₂ or C(O) and X₁ represents CH₂; more preferably X₂ and X₁ represent CH₂.

In a preferred embodiment, the compound according to the present invention is according to general formula (III)

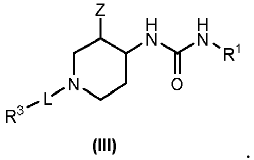

In another preferred embodiment, the compound according to the present invention is according to general formula (IV)

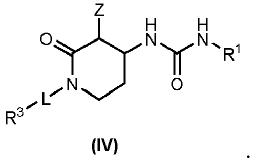

In another preferred embodiment, the compound according to the present invention is according to general formula (V)

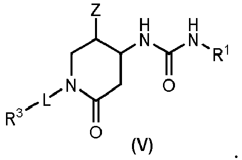

In a particularly preferred embodiment, the compound according to the present invention is according to general formula (III) or (V), most preferably (III).

According to the present invention, R¹ represents phenyl or 5 or 6-membered heteroaryl.

In a preferred embodiment, R¹ represents phenyl or 5 or 6-membered heteroaryl, wherein the 5 or 6-membered heteroaryl is selected from the group consisting of isothiazolyl, pyridyl, pyrimidinyl, pyridazinyl, pyrazinyl, pyrrolyl, imidazolyl, pyrazolyl, oxazolyl, isoxazolyl, thiazolyl, furanyl, thienyl (thiophenyl), triazolyl, thiadiazolyl, benzofuranyl, benzoimidazolyl, benzothienyl, benzothiadiazolyl, benzothiazolyl, benzotriazolyl, benzooxazolyl, benzooxadiazolyl, quinazolinyl, quinoxalinyl, carbazolyl, quinolinyl, dibenzofuranyl, dibenzothienyl, imidazothiazolyl, indazolyl, indolizinyl, indolyl, isoquinolinyl, naphthyridinyl, oxazolyl, oxadiazolyl, phenazinyl, phenothiazinyl, phthalazinyl, purinyl, phenazinyl, tetrazolyl and triazinyl; more preferably phenyl, isothiazolyl or pyridyl.

In a particularly preferred embodiment, R¹ represents phenyl, isothiazolyl, pyridyl, pyrimidinyl, thiophenyl, or thiazolyl, more preferably phenyl, isothiazolyl or pyridyl;
wherein said phenyl, isothiazolyl, pyridyl, pyrimidinyl, thiophenyl, and thiazolyl independently from one another are unsubstituted or monosubstituted with a substituent selected from the group consisting of F, Cl, Br, CN, unsubstituted C₁₋₆-alkyl and CF₃; more preferably Cl, CN and CH₃.

According to the present invention, Z represents a 5 or 6 membered heteroaryl which is monosubstituted, disubstituted or trisubstituted, more preferably monosubstituted or disubstituted, with R² which in each case independently is selected from the group consisting of O-C₁₋₆-alkyl, H, F, Cl, Br, CN, C₁₋₆-alkyl, C₃₋₆-cycloalkyl, CHF₂, CH₂F, CF₃, OH, OCHF₂, OCH2F, OCF₃, S-C₁₋₆-alkyl, S(O)-C₁₋₆-alkyl, S(O)₂-C₁₋₆-alkyl, O-C₃₋₆-cycloalkyl, S-C₃₋₆-cycloalkyl, S(O)-C₃₋₆-cycloalkyl, S(O)₂-C₃₋₆-cycloalkyl, NH₂, N(H)(C₁₋₆-alkyl), N(C₁₋₆-alkyl)₂, N(H)(C₃₋₆-cycloalkyl), N(C₁₋₆-alkyl)(C₃₋₆-cycloalkyl), NC(O)(C₁₋₆-alkyl), NC(O)(C₃₋₆-cycloalkyl), and NC(O)(3 to 6-membered heterocycloalkyl).

In a preferred embodiment, Z represents a 5 or 6 membered heteroaryl which is selected from the group consisting of pyridyl, thienyl (thiophenyl), thiazolyl, pyrimidinyl, pyridazinyl, pyrazinyl, pyrrolyl, imidazolyl, pyrazolyl, oxazolyl, isoxazolyl, isothiazolyl, furanyl, triazolyl, thiadiazolyl, benzofuranyl, benzoimidazolyl, benzothienyl, benzothiadiazolyl, benzothiazolyl, benzotriazolyl, benzooxazolyl, benzooxadiazolyl, quinazolinyl, quinoxalinyl, carbazolyl, quinolinyl, dibenzofuranyl, dibenzothienyl, imidazothiazolyl, indazolyl, indolizinyl, indolyl, isoquinolinyl, naphthyridinyl, oxazolyl, oxadiazolyl, phenazinyl, phenothiazinyl, phthalazinyl, purinyl, phenazinyl, tetrazolyl and triazinyl; more preferably pyridyl, thienyl and thiazolyl, most preferably pyridyl and thienyl;
preferably wherein said 5 or 6 membered heteroaryl is monosubstituted, disubstituted or trisubstituted, more preferably monosubstituted or disubstituted, with R² which in each case independently is selected from the group consisting of O-CH₃, F, Cl, Br and OH, more preferably O-CH₃ and Cl.

In a preferred embodiment, R² represents O-CH₃, F, Cl, Br, OH, O-CH₂CH₃, O-(CH₂)₂CH₃, O-CH(CH₃)₂ or OCF₃; more preferably O-CH₃, F, Cl, Br and OH; most preferably O-CH₃, F and Cl; and in particular O-CH₃ and Cl.

In another preferred embodiment, Z represents pyridyl, thienyl (thiophenyl), pyrimidinyl, pyridazinyl, pyrazinyl or pyrrolyl;
wherein said pyridyl, thienyl (thiophenyl), pyrimidinyl, pyridazinyl, pyrazinyl and pyrrolyl are monosubstituted or disubstituted with R² which in each case independently is selected from the group consisting of O-CH₃, Cl and F.

According to the present invention, L represents bond, C₁₋₆-alkylene, C(O), S(O)₂, C(CH₃)₂, phenyl, or pyridyl.

In a preferred embodiment, L represents bond, CH₂, CH₂CH₂, C(O), phenyl or pyridyl.

According to the present invention, R³ represents H, C₁₋₆-alkyl, C₃₋₆-cycloalkyl, 3 to 6-membered heterocycloalkyl, aryl, 5 or 6-membered heteroaryl, C₁₋₆-alkylene-OH, C₁₋₆-alkylene-O-C₁₋₆-alkyl, C₁₋₆-alkylene-NH₂, C₁₋₆-alkylene-N(H)-C₁₋₆-alkyl, C₁₋₆-alkylene-N(C₁₋₆-alkyl)₂, C₁₋₆-alkylene-C₃₋₆-cycloalkyl, C₁₋₆-alkylene-(3 to 6-membered heterocycloalkyl), C₁₋₆-alkylene-aryl, C₁₋₆-alkylene-(5 or 6-membered heteroaryl), C(O)NH₂, C(O)N(H)(C₁₋₆-alkyl), C(O)N(C₁₋₆-alkyl)₂, C(O)N(H)(C₃₋₆-cycloalkyl), C(O)N(H)(C₁₋₆-alkylene-C₃₋₆-cycloalkyl), C(O)N(H)(3 to 6-membered heterocycloalkyl), C(O)N(H)(C₁₋₆-alkylene-(3 to 6-membered heterocycloalkyl)), C(O)N(H)(aryl), C(O)N(H)(C₁₋₆-alkylene-aryl), C(O)N(H)(5 or 6-membered heteroaryl), C(O)N(H)(C₁₋₆-alkylene-(5 or 6-membered heteroaryl)), C(O)N(C₁₋₆-alkyl)(C₃₋₆-cycloalkyl), C(O)N(C₁₋₆-alkyl)(3 to 6-membered heterocycloalkyl), C(O)N(C₁₋₆-alkyl)(aryl), C(O)N(C₁₋₆-alkyl)(5 or 6-membered heteroaryl), C(O)N(C₃₋₆-cycloalkyl)(C₃₋₆-cycloalkyl), C(O)N(C₃₋₆-cycloalkyl)(3 to 6-membered heterocycloalkyl), C(O)N(C₃₋₆-cycloalkyl)(aryl), C(O)N(C₃₋₆-cycloalkyl)(5 or 6-membered heteroaryl), C(O)O-(C₁₋₆-alkyl), C(O)O-(C₃₋₆-cycloalkyl), C(O)O-(3 to 6-membered heterocycloalkyl), C(O)O-(aryl), C(O)O-(5 or 6-membered heteroaryl), S(O)-C₁₋₆-alkyl, S(O)₂-C₁₋₆-alkyl, S(O)-C₃₋₆-cycloalkyl, S(O)₂-C₃₋₆-cycloalkyl, S(O)-(3 to 6-membered heterocycloalkyl), S(O)₂-(3 to 6-membered heterocycloalkyl), S(O)-aryl; S(O)₂-aryl, S(O)-(5 or 6-membered heteroaryl), or S(O)₂-(5 or 6-membered heteroaryl).

Preferably, R³ represents H, C₁₋₆-alkyl, C₁₋₆-alkylene-OH, C₁₋₆-alkylene-O-C₁₋₆-alkyl, C₁₋₆-alkylene-NH₂, C₁₋₆-alkylene-N(H)-C₁₋₆-alkyl, C₁₋₆-alkylene-N(C₁₋₆-alkyl)₂, 3 to 6-membered cycloalkyl, 3 to 6-membered heterocycloalkyl, aryl, 5 or 6-membered heteroaryl, C(O)NH₂, C(O)N(H)(C₁₋₆-alkyl), C(O)N(C₁₋₆-alkyl)₂, C(O)N(H)(C₃₋₆-cycloalkyl), C(O)N(H)(C₁₋₆-alkylene-C₃₋₆-cycloalkyl), C(O)N(H)(3 to 6-membered heterocycloalkyl), C(O)N(H)(C₁₋₆-alkylene-(3 to 6-membered heterocycloalkyl)), C(O)N(H)(aryl), C(O)N(H)(C₁₋₆-alkylene-aryl), C(O)N(H)(5 or 6-membered heteroaryl), C(O)N(H)(C₁₋₆-alkylene-(5 or 6-membered heteroaryl)), or S(O)₂-C₁₋₆-alkyl.

In another preferred embodiment,
- L: represents bond, CH₂, CH₂CH₂, C(O), phenyl or pyridyl; and/or
- R³: represents H, C₁₋₆-alkyl, C₁₋₆-alkylene-OH, C₁₋₆-alkylene-O-C₁₋₆-alkyl, C₁₋₆-alkylene-NH₂, C₁₋₆-alkylene-N(H)-C₁₋₆-alkyl, C₁₋₆-alkylene-N(C₁₋₆-alkyl)₂, 3 to 6-membered cycloalkyl, 3 to 6-membered heterocycloalkyl, aryl, 5 or 6-membered heteroaryl, C(O)NH₂, C(O)N(H)(C₁₋₆-alkyl), C(O)N(C₁₋₆-alkyl)₂, C(O)N(H)(C₃₋₆-cycloalkyl), C(O)N(H)(C₁₋₆-alkylene-C₃₋₆-cycloalkyl), C(O)N(H)(3 to 6-membered heterocycloalkyl), C(O)N(H)(C₁₋₆-alkylene-(3 to 6-membered heterocycloalkyl)), C(O)N(H)(aryl), C(O)N(H)(C₁₋₆-alkylene-aryl), C(O)N(H)(5 or 6-membered heteroaryl), C(O)N(H)(C₁₋₆-alkylene-(5 or 6-membered heteroaryl)), or S(O)₂-C₁₋₆-alkyl.

In yet another preferred embodiment,
- R³: represents
H;
C₁₋₆-alkyl selected from the group consisting of methyl, ethyl, n-propyl, 2-propyl, n-butyl, isobutyl, sec-butyl, tert-butyl, n-pentyl, 2-pentyl, 3-pentyl, 2-methylbutyl, 3-methylbutyl, 3-methylbut-2-yl, 2-methylbut-2-yl, 2,2-dimethylpropyl and n-hexyl;
wherein C₁₋₆-alkyl is unsubstituted or mono- or polysubstituted with one or more substituents selected from the group consisting of F, Cl, Br, CN, CF₃, CHF₂, C(O)NH₂, OH, O-CH₃, O-CH₂CH₃, O-(CH₂)₂CH₃, O-CH(CH₃)₂, N(H)C(O)CH₃, N(H)S(O)₂CH₃, S(O)-CH₃, and S(O)₂-CH₃;
C₁₋₆-alkylene-OH selected from the group consisting of CH₂OH, CH₂CH₂OH, (CH₂)₃OH, (CH₂)₄OH, C(H)(OH)-CH₃, CH₂C(H)(OH)-CH₃, C(CH₃)₂-OH, C(H)(OH)-C(CH₃)₂, and CH₂C(CH₃)₂-OH,
wherein C₁₋₆-alkylene is unsubstituted or mono- or polysubstituted with one or more substituents selected from the group consisting of F, Cl, Br, CN, and OH;
C₁₋₆-alkylene-O-C₁₋₆-alkyl selected from the group consisting of CH₂OCH₃, CH₂CH₂OCH₃, (CH₂)₃OCH₃, (CH₂)₄OCH₃, (CH₂)₅OCH₃, and (CH₂)₆OCH₃,
wherein C₁₋₆-alkylene is unsubstituted or mono- or polysubstituted with one or more substituents selected from the group consisting of F, Cl, Br, CN, and OH;
C₁₋₆-alkylene-NH₂ selected from the group consisting of CH₂NH₂, CH₂CH₂NH₂, (CH₂)₃NH₂, (CH₂)₄NH₂, (CH₂)₅NH₂, and (CH₂)₆NH₂,
C₁₋₆-alkylene-N(H)-C₁₋₆-alkyl selected from the group consisting of CH₂N(H)CH₃, CH₂CH₂N(H)CH₃, (CH₂)₃N(H)CH₃, (CH₂)₄N(H)CH₃, (CH₂)₅N(H)CH₃, and (CH₂)₆N(H)CH₃, C₁₋₆-alkylene-N(C₁₋₆-alkyl)₂ selected from the group consisting of CH₂N(CH₃)₂, CH₂CH₂N(CH₃)₂, (CH₂)₃N(CH₃)₂, (CH₂)₄N(CH₃)₂, (CH₂)₅N(CH₃)₂, and (CH₂)₆N(CH₃)₂,
3 to 6-membered cycloalkyl selected from the group consisting of cyclopropyl, cyclobutyl, cyclopentyl and cyclohexyl;
wherein 3 to 6-membered cycloalkyl is unsubstituted or mono- or polysubstituted with one or more substituents selected from the group consisting of F, Cl, Br, CN, CH₃, CF₃, C(O)NH₂, C(O)N(H)(CH₃), C(O)N(CH₃)₂, OH, CH₂OH, OCH₃, CH₂OCH₃, N(H)C(O)CH₃, N(H)S(O)₂CH₃, and S(O)₂-CH₃;
3 to 6-membered heterocycloalkyl selected from the group consisting of tetrahydropyranyl, piperidinyl, oxetanyl, azetidinyl, tetrahydrothiopyran 1,1-dioxide, morpholinyl, pyrrolidinyl, 4-methylpiperazinyl, morpholinonyl, aziridinyl, dithiolanyl, dihydropyrrolyl, dioxanyl, dioxolanyl, dihydropyridinyl, dihydrofuranyl, dihydroisoxazolyl, dihydrooxazolyl, imidazolidinyl, isoxazolidinyl, oxazolidinyl, oxiranyl, piperazinyl, pyrazolidinyl, pyranyl and tetrahydropyrrolyl;
wherein 3 to 6-membered heterocycloalkyl is unsubstituted or mono- or polysubstituted with one or more substituents selected from the group consisting of F, Cl, Br, CN, CH₃, CF₃, C(O)NH₂, C(O)N(H)(CH₃), C(O)N(CH₃)₂, OH, CH₂OH, OCH₃, CH₂OCH₃, N(H)C(O)CH₃, N(H)S(O)₂CH₃, and S(O)₂-CH₃;
C(O)NH₂;
C(O)N(H)(C₁₋₆-alkyl) selected from the group consisting of C(O)N(H)(CH₃) and C(O)N(H)(CH₂CH₃);
wherein C₁₋₆-alkyl is unsubstituted or mono- or polysubstituted with one or more substituents selected from the group consisting of F, Cl, Br, CN, CF₃, CHF₂, OH, and O-CH₃;
C(O)N(C₁₋₆-alkyl)₂ selected from the group consisting of C(O)N(CH₃)₂ and C(O)N(CH₂CH₃)₂; C(O)N(H)(C₃₋₆-cycloalkyl) selected from the group consisting of C(O)N(H)(cyclopropyl), C(O)N(H)(cyclobutyl), C(O)N(H)(cyclopentyl) and C(O)N(H)(cyclohexyl);
C(O)N(H)(C₁₋₆-alkylene-C₃₋₆-cycloalkyl) selected from the group consisting of C(O)N(H)(CH₂-cyclopropyl), C(O)N(H)(CH₂-cyclobutyl), C(O)N(H)(CH₂CH₂-cyclopropyl), C(O)N(H)(CH₂CH₂-cyclobutyl), C(O)N(H)(CH₂-cyclopentyl) and C(O)N(H)(CH₂-cyclohexyl);
C(O)N(H)(3 to 6-membered heterocycloalkyl) selected from the group consisting of C(O)N(H)(tetrahydropyranyl), C(O)N(H)(piperidinyl), C(O)N(H)(oxetanyl), and C(O)N(H)(azetidinyl),
C(O)N(H)(C₁₋₆-alkylene-(3 to 6-membered heterocycloalkyl) selected from the group consisting of C(O)N(H)(CH₂-tetrahydropyranyl), C(O)N(H)(CH₂-piperidinyl), C(O)N(H)(CH₂-oxetanyl), and C(O)N (H)(CH₂-azetidinyl),
C(O)N(H)(aryl) selected from C(O)N(H)(phenyl);
C(O)N(H)(C₁₋₆-alkylene-aryl) selected from C(O)N(H)(CH₂-phenyl) and C(O)N(H)(CH₂CH₂-phenyl);
C(O)N(H)(5 or 6-membered heteroaryl) selected from the group consisting of C(O)N(H)(isoxazolyl), C(O)N(H)(pyridyl), C(O)N(H)(pyrazolyl), C(O)N(H)(oxazolyl), C(O)N(H)(thiazolyl), C(O)N(H)(triazolyl), C(O)N(H)(oxadiazolyl), C(O)N(H)(pyrimidinyl), C(O)N(H)(pyrazinyl), and C(O)N(H)(pyridazinyl);
wherein said 5- or 6-membered heteroaryl is unsubstituted or mono- or disubstituted with one or more substituents selected from the group consisting of F, Cl, Br, CN, CH₃, CF₃, cyclopropyl, OH, O-CH₃, and S(O)₂-CH₃;
C(O)N(H)(C₁₋₆-alkylene-5 or 6-membered heteroaryl) selected from the group consisting of C(O)N(H)(CH₂-isoxazolyl), C(O)N(H)(CH₂-pyridyl), C(O)N(H)(CH₂-pyrazolyl), C(O)N(H)(CH₂-oxazolyl), C(O)N(H)(CH₂-thiazolyl), C(O)N(H)(CH₂-triazolyl), C(O)N(H)(CH₂-oxadiazolyl), C(O)N(H)(CH₂-pyrimidinyl), C(O)N(H)(CH₂-pyrazinyl), and C(O)N(H)(CH₂-pyridazinyl);
wherein said 5- or 6-membered heteroaryl is unsubstituted or mono- or disubstituted with one or more substituents selected from the group consisting of F, Cl, Br, CN, CH₃, CF₃, cyclopropyl, OH, O-CH₃, and S(O)₂-CH₃;
S(O)₂-C₁₋₆-alkyl selected from the group consisting of S(O)₂-CH₃, S(O)₂-CH₂CH₃, S(O)₂-(CH₂)₂CH₃, and S(O)₂-CH(CH₃)₂;
phenyl, which is unsubstituted or mono- or disubstituted with one or more substituents selected from the group consisting of F, Cl, Br, CN, OH, CH₂OH, OCH₃, CH₂OCH₃, and CH₃; or
5- or 6-membered heteroaryl selected from the group consisting of pyrimidinyl, pyrazinyl, pyridazinyl, pyridyl, pyrazolyl, isoxazolyl, triazolyl, oxazolyl, oxadiazolyl, indazolyl, pyrazolopyridyl, isoindolinonyl, pyrrolyl, imidazolyl, thiazolyl, isothiazolyl, furanyl, thienyl, and thiadiazolyl;
wherein said 5- or 6-membered heteroaryl is unsubstituted or mono- or disubstituted with one or more substituents selected from the group consisting of F, Cl, Br, CN, CH₃, CF₃, cyclopropyl, N(H)CH₂CH₂OH, OH, CH₂OH, CH₂CH₂OH, OCH₃, CH₂OCH₃, CH₂CH₂OCH₃, and S(O)₂-CH₃.

In a particularly preferred embodiment,
- X₂: represents CH₂ or C(O) and X₁ represent CH₂;
- Z: represents pyridyl, thienyl (thiophenyl), pyrimidinyl, pyridazinyl, pyrazinyl or pyrrolyl;
wherein said pyridyl, thienyl (thiophenyl), pyrimidinyl, pyridazinyl, pyrazinyl and pyrrolyl are monosubstituted or disubstituted with R² which in each case independently is selected from the group consisting of O-CH₃, Cl and F;
- R¹: represents phenyl, isothiazolyl, pyridyl, pyrimidinyl, thiophenyl, or thiazolyl;
wherein said phenyl, isothiazolyl, pyridyl, pyrimidinyl, thiophenyl, and thiazolyl independently from one another are unsubstituted or monosubstituted with a substituents selected from the group consisting of F, Cl, Br, CN, unsubstituted C₁₋₆-alkyl and CF₃;
- L: represents bond, CH₂, CH₂CH₂, C(O), phenyl or pyridyl; and
- R³: represents
H;
C₁₋₆-alkyl selected from the group consisting of methyl, ethyl, n-propyl, 2-propyl, n-butyl, isobutyl, sec-butyl, tert-butyl, n-pentyl, 2-pentyl, 3-pentyl, 2-methylbutyl, 3-methylbutyl, 3-methylbut-2-yl, 2-methylbut-2-yl, 2,2-dimethylpropyl and n-hexyl;
wherein C₁₋₆-alkyl is unsubstituted or mono- or polysubstituted with one or more substituents selected from the group consisting of F, Cl, Br, CN, CF₃, CHF₂, C(O)NH₂, OH, O-CH₃, O-CH₂CH₃, O-(CH₂)₂CH₃, O-CH(CH₃)₂, N(H)C(O)CH₃, N(H)S(O)₂CH₃, S(O)-CH₃, and S(O)₂-CH₃;
C₁₋₆-alkylene-OH selected from the group consisting of CH₂OH, CH₂CH₂OH, (CH₂)₃OH, (CH₂)₄OH, C(H)(OH)-CH₃, CH₂C(H)(OH)-CH₃, C(CH₃)₂-OH, C(H)(OH)-C(CH₃)₂, and CH₂C(CH₃)₂-OH,
wherein C₁₋₆-alkylene is unsubstituted or mono- or polysubstituted with one or more substituents selected from the group consisting of F, Cl, Br, CN, and OH;
C₁₋₆-alkylene-O-C₁₋₆-alkyl selected from the group consisting of CH₂OCH₃, CH₂CH₂OCH₃, (CH₂)₃OCH₃, (CH₂)₄OCH₃, (CH₂)₅OCH₃, and (CH₂)₆OCH₃,
wherein C₁₋₆-alkylene is unsubstituted or mono- or polysubstituted with one or more substituents selected from the group consisting of F, Cl, Br, CN, and OH;
C₁₋₆-alkylene-NH₂ selected from the group consisting of CH₂NH₂, CH₂CH₂NH₂, (CH₂)₃NH₂, (CH₂)₄NH₂, (CH₂)₅NH₂, and (CH₂)₆NH₂,
C₁₋₆-alkylene-N(H)-C₁₋₆-alkyl selected from the group consisting of CH₂N(H)CH₃, CH₂CH₂N(H)CH₃, (CH₂)₃N(H)CH₃, (CH₂)₄N(H)CH₃, (CH₂)₅N(H)CH₃, and (CH₂)₆N(H)CH₃,
C₁₋₆-alkylene-N(C₁₋₆-alkyl)₂ selected from the group consisting of CH₂N(CH₃)₂, CH₂CH₂N(CH₃)₂, (CH₂)₃N(CH₃)₂, (CH₂)₄N(CH₃)₂, (CH₂)₅N(CH₃)₂, and (CH₂)₆N(CH₃)₂,
3 to 6-membered cycloalkyl selected from the group consisting of cyclopropyl, cyclobutyl, cyclopentyl and cyclohexyl;
wherein 3 to 6-membered cycloalkyl is unsubstituted or mono- or polysubstituted with one or more substituents selected from the group consisting of F, Cl, Br, CN, CH₃, CF₃, C(O)NH₂, C(O)N(H)(CH₃), C(O)N(CH₃)₂, OH, CH₂OH, OCH₃, CH₂OCH₃, N(H)C(O)CH₃, N(H)S(O)₂CH₃, and S(O)₂-CH₃;
3 to 6-membered heterocycloalkyl selected from the group consisting of tetrahydropyranyl, piperidinyl, oxetanyl, azetidinyl, tetrahydrothiopyran 1,1-dioxide, morpholinyl, pyrrolidinyl, 4-methylpiperazinyl, morpholinonyl, aziridinyl, dithiolanyl, dihydropyrrolyl, dioxanyl, dioxolanyl, dihydropyridinyl, dihydrofuranyl, dihydroisoxazolyl, dihydrooxazolyl, imidazolidinyl, isoxazolidinyl, oxazolidinyl, oxiranyl, piperazinyl, pyrazolidinyl, pyranyl and tetrahydropyrrolyl;
wherein 3 to 6-membered heterocycloalkyl is unsubstituted or mono- or polysubstituted with one or more substituents selected from the group consisting of F, Cl, Br, CN, CH₃, CF₃, C(O)NH₂, C(O)N(H)(CH₃), C(O)N(CH₃)₂, OH, CH₂OH, OCH₃, CH₂OCH₃, N(H)C(O)CH₃, N(H)S(O)₂CH₃, and S(O)₂-CH₃;
C(O)NH₂;
C(O)N(H)(C₁₋₆-alkyl) selected from the group consisting of C(O)N(H)(CH₃) and C(O)N(H)(CH₂CH₃);
wherein C₁₋₆-alkyl is unsubstituted or mono- or polysubstituted with one or more substituents selected from the group consisting of F, Cl, Br, CN, CF₃, CHF₂, OH, and O-CH₃;
C(O)N(C₁₋₆-alkyl)₂ selected from the group consisting of C(O)N(CH₃)₂ and C(O)N(CH₂CH₃)₂;
C(O)N(H)(C₃₋₆-cycloalkyl) selected from the group consisting of C(O)N(H)(cyclopropyl), C(O)N(H)(cyclobutyl), C(O)N(H)(cyclopentyl) and C(O)N(H)(cyclohexyl);
C(O)N(H)(C₁₋₆-alkylene-C₃₋₆-cycloalkyl) selected from the group consisting of C(O)N(H)(CH₂-cyclopropyl), C(O)N(H)(CH₂-cyclobutyl), C(O)N(H)(CH₂CH₂-cyclopropyl), C(O)N(H)(CH₂CH₂-cyclobutyl), C(O)N(H)(CH₂-cyclopentyl) and C(O)N(H)(CH₂-cyclohexyl);
C(O)N(H)(3 to 6-membered heterocycloalkyl) selected from the group consisting of C(O)N(H)(tetrahydropyranyl), C(O)N(H)(piperidinyl), C(O)N(H)(oxetanyl), and C(O)N(H)(azetidinyl),
C(O)N(H)(C₁₋₆-alkylene-(3 to 6-membered heterocycloalkyl) selected from the group consisting of C(O)N(H)(CH₂-tetrahydropyranyl), C(O)N(H)(CH₂-piperidinyl), C(O)N(H)(CH₂-oxetanyl), and C(O)N (H)(CH₂-azetidinyl),
C(O)N(H)(aryl) selected from C(O)N(H)(phenyl);
C(O)N(H)(C₁₋₆-alkylene-aryl) selected from C(O)N(H)(CH₂-phenyl) and C(O)N(H)(CH₂CH₂-phenyl);
C(O)N(H)(5 or 6-membered heteroaryl) selected from the group consisting of C(O)N(H)(isoxazolyl), C(O)N(H)(pyridyl), C(O)N(H)(pyrazolyl), C(O)N(H)(oxazolyl), C(O)N(H)(thiazolyl), C(O)N(H)(triazolyl), C(O)N(H)(oxadiazolyl), C(O)N(H)(pyrimidinyl), C(O)N(H)(pyrazinyl), and C(O)N (H) (pyridazinyl);
wherein said 5- or 6-membered heteroaryl is unsubstituted or mono- or disubstituted with one or more substituents selected from the group consisting of F, Cl, Br, CN, CH₃, CF₃, cyclopropyl, OH, O-CH₃, and S(O)₂-CH₃;
C(O)N(H)(C₁₋₆-alkylene-5 or 6-membered heteroaryl) selected from the group consisting of C(O)N(H)(CH₂-isoxazolyl), C(O)N(H)(CH₂-pyridyl), C(O)N(H)(CH₂-pyrazolyl), C(O)N(H)(CH₂-oxazolyl), C(O)N(H)(CH₂-thiazolyl), C(O)N(H)(CH₂-triazolyl), OC(O)N(H)(CH₂-oxadiazolyl), C(O)N(H)(CH₂-pyrimidinyl), C(O)N(H)(CH₂-pyrazinyl), and C(O)N(H)(CH₂-pyridazinyl);
wherein said 5- or 6-membered heteroaryl is unsubstituted or mono- or disubstituted with one or more substituents selected from the group consisting of F, Cl, Br, CN, CH₃, CF₃, cyclopropyl, OH, O-CH₃, and S(O)₂-CH₃;
S(O)₂-C₁₋₆-alkyl selected from the group consisting of S(O)₂-CH₃, S(O)₂-CH₂CH₃, S(O)₂-(CH₂)₂CH₃, and S(O)₂-CH(CH₃)₂;
phenyl, which is unsubstituted or mono- or disubstituted with one or more substituents selected from the group consisting of F, Cl, Br, CN, OH, CH₂OH, OCH₃, CH₂OCH₃, and CH₃; or
5- or 6-membered heteroaryl selected from the group consisting of pyrimidinyl, pyrazinyl, pyridazinyl, pyridyl, pyrazolyl, isoxazolyl, triazolyl, oxazolyl, oxadiazolyl, indazolyl, pyrazolopyridyl, isoindolinonyl, pyrrolyl, imidazolyl, thiazolyl, isothiazolyl, furanyl, thienyl, and thiadiazolyl;
wherein said 5- or 6-membered heteroaryl is unsubstituted or mono- or disubstituted with one or more substituents selected from the group consisting of F, Cl, Br, CN, CH₃, CF₃, cyclopropyl, N(H)CH₂CH₂OH, OH, CH₂OH, CH₂CH₂OH, OCH₃, CH₂OCH₃, CH₂CH₂OCH₃, and S(O)₂-CH₃.

In a particularly preferred embodiment, the compound according to the present invention is selected from the group consisting of
**Ex-1** trans-1-(4-Chlorophenyl)-3-[3-(6-methoxy-pyridin-3-yl)-1-pyrimidin-4-yl-piperidin-4-yl]-urea
**Ex-2** trans-1-(4-Chlorophenyl)-3-[1-(4-fluorophenyl)-3 -(6-methoxy-pyridin-3-yl)-piperidin-4-yl]-urea
**Ex-3** trans-1-[1-(4-Fluorophenyl)-3-(6-methoxy-pyridin-3-yl)-piperidin-4-yl]-3-(3-methyl-isothiazol-5-yl)-urea
**Ex-4** trans-1-[3-(6-Methoxy-pyridin-3-yl)-1-pyrimidin-4-yl-piperidin-4-yl]-3-(3-methyl-isothiazol-5-yl)-urea
**Ex-5** trans-1-(4-Chlorophenyl)-3-[1-(4-fluorophenyl)-5-(6-methoxy-pyridin-3-yl)-2-oxo-piperidin-4-yl]-urea
**Ex-6** cis-1-(4-Chlorophenyl)-3-[1-(4-fluorophenyl)-5-(6-methoxy-pyridin-3-yl)-2-oxo-piperidin-4-yl]-urea
**Ex-7** trans-1-(4-Chlorophenyl)-3-[3-(5-methoxy-pyridin-2-yl)-1-pyrimidin-4-yl-piperidin-4-yl]-urea
**Ex-8** trans-1-[3-(5-Methoxy-pyridin-2-yl)-1-pyrimidin-4-yl-piperidin-4-yl]-3-(3-methyl-isothiazol-5-yl)-urea
**Ex-9** trans-1-(4-Chlorophenyl)-3-[3-(5-chloro-pyridin-2-yl)-1-pyrimidin-4-yl-piperidin-4-yl]-urea
**Ex-10** trans-1-(4-Chlorophenyl)-3-[1-(4-fluorophenyl)-5-(5-methoxy-pyridin-2-yl)-2-oxo-piperidin-4-yl]-urea
**Ex-11** cis-1-(4-Chlorophenyl)-3-[1-(4-fluorophenyl)-5-(5-methoxy-pyridin-2-yl)-2-oxo-piperidin-4-yl]-urea
**Ex-12** trans-1-(4-Chlorophenyl)-3-[3-(5-chloro-pyridin-2-yl)-1-(4-fluorophenyl)-piperidin-4-yl]-urea
**Ex-13** trans-1-[3-(5-Chloro-pyridin-2-yl)-1-pyrimidin-4-yl-piperidin-4-yl]-3-(3-methyl-isothiazol-5-yl)-urea
**Ex-14** trans-1-(4-Chlorophenyl)-3-[5-(5-chloro-pyridin-2-yl)-1-(4-fluorophenyl)-2-oxo-piperidin-4-yl]-urea
**Ex-15** cis-1-(4-Chlorophenyl)-3-[5-(5-chloro-pyridin-2-yl)-1-(4-fluorophenyl)-2-oxo-piperidin-4-yl]-urea
**Ex-16** trans-1-[3-(5-Chloro-pyridin-2-yl)-1-pyrimidin-4-yl-piperidin-4-yl]-3-(3-methyl-isothiazol-5-yl)-urea ent-1
**Ex-17** trans-1-[3-(5-Chloro-pyridin-2-yl)-1-pyrimidin-4-yl-piperidin-4-yl]-3-(3-methyl-isothiazol-5-yl)-urea ent-2
**Ex-18** trans-1-(4-Chlorophenyl)-3-[1-(4-fluorophenyl)-3-(5-methoxy-pyridin-2-yl)-piperidin-4-yl]-urea
**Ex-19** trans-1-[3-(5-Chloro-pyridin-2-yl)-1-pyrazin-2-yl-piperidin-4-yl]-3-(3-methyl-isothiazol-5-yl)-urea
**Ex-20** trans-1-[1-(4-Fluorophenyl)-3-(5-methoxy-pyridin-2-yl)-piperidin-4-yl]-3-(3-methyl-isothiazol-5-yl)-urea
**Ex-21** trans-1-[3-(5-Chloro-pyridin-2-yl)-1-(2-methyl-pyrimidin-4-yl)-piperidin-4-yl]-3-(3-methyl-isothiazol-5-yl)-urea
**Ex-22** trans-1-(4-Chlorophenyl)-3-[3-(5-chloro-pyridin-2-yl)-1-[(5-methyl-isoxazol-3-yl)-methyl]-piperidin-4-yl]-urea ent-1
**Ex-23** trans-1-(4-Chlorophenyl)-3-[3-(5-chloro-pyridin-2-yl)-1-[(5-methyl-isoxazol-3-yl)-methyl]-piperidin-4-yl]-urea ent-2
**Ex-25** 1-(4-Chlorophenyl)-3-[5-(5-methoxy-pyridin-2-yl)-2-oxo-1-pyrimidin-4-yl-piperidin-4-yl]-urea dia-1
**Ex-26** trans-1-[3-(5-Chloro-pyridin-2-yl)-1-(4-fluorophenyl)-piperidin-4-yl]-3-(3-methyl-isothiazol-5-yl)-urea
**Ex-27** trans-1-[3-(5-Chloro-pyridin-2-yl)-1-[6-(trifluoromethyl)-pyrimidin-4-yl]-piperidin-4-yl]-3-(3-methyl-isothiazol-5-yl)-urea
**Ex-28** trans-1-[3-(5-Chloro-pyridin-2-yl)-1-[2-(trifluoromethyl)-pyrimidin-4-yl]-piperidin-4-yl]-3-(3-methyl-isothiazol-5-yl)-urea
**Ex-29** trans-1-(4-Chlorophenyl)-3-[3-(5-methoxy-pyridin-2-yl)-1-[(5-methyl-isoxazol-3-yl)-methyl]-piperidin-4-yl]-urea ent-1
**Ex-30** trans-1-(4-Chlorophenyl)-3-[3-(5-methoxy-pyridin-2-yl)-1-[(5-methyl-isoxazol-3-yl)-methyl]-piperidin-4-yl]-urea ent-2
**Ex-31** trans-1-[3-(5-Chloro-pyridin-2-yl)-1-(6-methyl-pyrimidin-4-yl)-piperidin-4-yl]-3-(3-methyl-isothiazol-5-yl)-urea
**Ex-32** trans-1-[3-(5-Chloro-pyridin-2-yl)-1-pyridazin-3-yl-piperidin-4-yl]-3-(3-methyl-isothiazol-5-yl)-urea
**Ex-33** trans-1-[3-(5-Chloro-pyridin-2-yl)-1-(6-methyl-pyridazin-3-yl)-piperidin-4-yl]-3-(3-methyl-isothiazol-5-yl)-urea
**Ex-34** trans-1-[3-(5-Chloro-pyridin-2-yl)-1-(6-methyl-pyrazin-2-yl)-piperidin-4-yl]-3-(3-methyl-isothiazol-5-yl)-urea
**Ex-35** trans-1-(4-Chlorophenyl)-3-[1-(2,2-difluoro-ethyl)-3-(5-methoxy-pyridin-2-yl)-piperidin-4-yl]-urea ent-1
**Ex-36** trans-1-(4-Chlorophenyl)-3-[1-(2,2-difluoro-ethyl)-3-(5-methoxy-pyridin-2-yl)-piperidin-4-yl]-urea ent-2
**Ex-37** trans-1-(4-Chlorophenyl)-3-[3-(5-methoxy-pyridin-2-yl)-1-[[5-(trifluoromethyl)-isoxazol-3-yl]-methyl]-piperidin-4-yl]-urea ent-2
**Ex-38** trans-1-(4-Chlorophenyl)-3-[3-(5-methoxy-pyridin-2-yl)-1-[[5-(trifluoromethyl)-isoxazol-3-yl]-methyl]-piperidin-4-yl]-urea ent-2
**Ex-39** trans-1-(4-Chlorophenyl)-3-[3-(5-methoxy-pyridin-2-yl)-1-[(1-methyl-1H-pyrazol-3-yl)-methyl]-piperidin-4-yl]-urea ent-1
**Ex-40** trans-1-(4-Chlorophenyl)-3-[3-(5-methoxy-pyridin-2-yl)-1-[(1-methyl-1H-pyrazol-3-yl)-methyl]-piperidin-4-yl]-urea ent-2
**Ex-41** trans-1-[3-(5-Chloro-pyridin-2-yl)-1-(5-methyl-pyrazin-2-yl)-piperidin-4-yl]-3-(3-methyl-isothiazol-5-yl)-urea
**Ex-42** trans-1-(4-Chlorophenyl)-3-[3-(5-methoxy-pyridin-2-yl)-1-[(1-methylsulfonyl-1H-pyrazol-3-yl)-methyl]-piperidin-4-yl]-urea ent-1
**Ex-43** trans-1-(4-Chlorophenyl)-3-[3-(5-methoxy-pyridin-2-yl)-1-[(1-methylsulfonyl-1H-pyrazol-3-yl)-methyl]-piperidin-4-yl]-urea ent-2
**Ex-44** trans-1-(4-Chlorophenyl)-3-[3-(5-chloro-thiophen-2-yl)-1-[(5-methyl-isoxazol-3-yl)-methyl]-piperidin-4-yl]-urea ent-1
**Ex-45** trans-1-(4-Chlorophenyl)-3-[3-(5-chloro-thiophen-2-yl)-1-[(5-methyl-isoxazol-3-yl)-methyl]-piperidin-4-yl]-urea ent-2
**Ex-46** trans-1-(4-Chlorophenyl)-3-[3-(5-chloro-thiophen-2-yl)-1-(pyrimidin-2-yl-methyl)-piperidin-4-yl]-urea ent-1
**Ex-47** trans-1-(4-Chlorophenyl)-3-[3-(5-chloro-thiophen-2-yl)-1-(pyrimidin-2-yl-methyl)-piperidin-4-yl]-urea ent-2
**Ex-48** trans-1-[3-(5-Chloro-thiophen-2-yl)-1-[(5-methyl-isoxazol-3-yl)-methyl]-piperidin-4-yl]-3-(3-methyl-isothiazol-5-yl)-urea ent-1
**Ex-49** trans-1-[3-(5-Chloro-thiophen-2-yl)-1-[(5-methyl-isoxazol-3-yl)-methyl]-piperidin-4-yl]-3-(3-methyl-isothiazol-5-yl)-urea ent-2
**Ex-50** trans-1-(4-Chlorophenyl)-3-[3-(5-methoxy-pyridin-2-yl)-1-(tetrahydro-pyran-4-yl-methyl)-piperidin-4-yl]-urea ent-2
**Ex-51** trans-1-(4-Chlorophenyl)-3-[3-(5-methoxy-pyridin-2-yl)-1-(tetrahydro-pyran-4-yl-methyl)-piperidin-4-yl]-urea ent-2
**Ex-52** trans-1-(4-Chlorophenyl)-3-[3-(5-methoxy-pyridin-2-yl)-1-(4-methyl-pyridine-3-carbonyl)-piperidin-4-yl]-urea ent-1
**Ex-53** trans-1-(4-Chlorophenyl)-3-[3-(5-methoxy-pyridin-2-yl)-1-(4-methyl-pyridine-3-carbonyl)-piperidin-4-yl]-urea ent-2
**Ex-54** trans-1-(4-Chlorophenyl)-3-[3-(5-methoxy-pyridin-2-yl)-1-(6-methyl-pyridine-3-carbonyl)-piperidin-4-yl]-urea ent-1
**Ex-55** trans-1-(4-Chlorophenyl)-3-[3-(5-methoxy-pyridin-2-yl)-1-(6-methyl-pyridine-3-carbonyl)-piperidin-4-yl]-urea ent-2
**Ex-56** trans-1-[3-(5-Chloro-thiophen-2-yl)-1-[(1-methylsulfonyl-1H-pyrazol-3-yl)-methyl]-piperidin-4-yl]-3-phenyl-urea ent-1
**Ex-57** trans-1-[3-(5-Chloro-thiophen-2-yl)-1-[(1-methylsulfonyl-1H-pyrazol-3-yl)-methyl]-piperidin-4-yl]-3-phenyl-urea ent-2
**Ex-58** trans-1-[3-(5-Methoxy-pyridin-2-yl)-1-[(1-methylsulfonyl-1H-pyrazol-3-yl)-methyl]-piperidin-4-yl]-3-phenyl-urea ent-1
**Ex-59** trans-1-[3-(5-Methoxy-pyridin-2-yl)-1-[(1-methylsulfonyl-1H-pyrazol-3-yl)-methyl]-piperidin-4-yl]-3-phenyl-urea ent-2
**Ex-60** trans-1-(4-Chlorophenyl)-3-[3-(5-methoxy-pyridin-2-yl)-1-[[6-(trifluoromethyl)-pyridin-3-yl]-methyl]-piperidin-4-yl]-urea
**Ex-61** trans-1-(4-Chlorophenyl)-3-[3-(5-methoxy-pyridin-2-yl)-1-[(2-methyl-pyrimidin-5-yl)-methyl]-piperidin-4-yl]-urea
**Ex-62** trans-1-[3-(5-Chloro-thiophen-2-yl)-1-(pyrimidin-2-yl-methyl)-piperidin-4-yl]-3-(3-methyl-isothiazol-5-yl)-urea ent-1
**Ex-63** trans-1-[3-(5-Chloro-thiophen-2-yl)-1-(pyrimidin-2-yl-methyl)-piperidin-4-yl]-3-(3-methyl-isothiazol-5-yl)-urea ent-2
**Ex-64** trans-1-(4-Chlorophenyl)-3-[3-(5-methoxy-pyridin-2-yl)-1-[(5-methyl-pyridin-3-yl)-methyl]-piperidin-4-yl]-urea
**Ex-65** trans-1-(4-Chlorophenyl)-3-[1-[(6-cyclopropyl-pyridin-3-yl)-methyl]-3-(5-methoxy-pyridin-2-yl)-piperidin-4-yl]-urea
**Ex-66** trans-1-(4-Chlorophenyl)-3-[3-(5-methoxy-pyridin-2-yl)-1-[(2-methoxy-pyrimidin-5-yl)-methyl]-piperidin-4-yl]-urea
**Ex-67** trans-1-(4-Chlorophenyl)-3-[3-(5-methoxy-pyridin-2-yl)-1-[(6-methyl-pyridin-3-yl)-methyl]-piperidin-4-yl]-urea
**Ex-68** trans-1-(4-Chlorophenyl)-3-[1-[(5-fluoro-pyridin-3-yl)-methyl]-3-(5-methoxy-pyridin-2-yl)-piperidin-4-yl]-urea
**Ex-69** trans-1-(4-Chlorophenyl)-3-[1-[(5-chloro-pyridin-3-yl)-methyl]-3-(5-methoxy-pyridin-2-yl)-piperidin-4-yl]-urea
**Ex-70** trans-1-(4-Chlorophenyl)-3-[3-(5-chloro-thiophen-2-yl)-1-[(5-fluoro-pyridin-3-yl)-methyl]-piperidin-4-yl]-urea
**Ex-71** trans-1-(4-Chlorophenyl)-3-[1-[(5-chloro-pyridin-3-yl)-methyl]-3-(5-chloro-thiophen-2-yl)-piperidin-4-yl]-urea
**Ex-72** trans-1-(4-Chlorophenyl)-3-[3-(5-chloro-thiophen-2-yl)-1-[(5-methyl-pyridin-3-yl)-methyl]-piperidin-4-yl]-urea
**Ex-73** trans-1-(4-Chlorophenyl)-3-[3-(5-chloro-thiophen-2-yl)-1-[(6-cyclopropyl-pyridin-3-yl)-methyl]-piperidin-4-yl]-urea
**Ex-74** trans-1-(4-Chlorophenyl)-3-[3-(5-chloro-thiophen-2-yl)-1-[[6-(trifluoromethyl)-pyridin-3-yl]-methyl]-piperidin-4-yl]-urea
**Ex-75** trans-1-(4-Chlorophenyl)-3-[3-(5-chloro-thiophen-2-yl)-1-[(2-methoxy-pyrimidin-5-yl)-methyl]-piperidin-4-yl]-urea
**Ex-76** trans-1-(4-Chlorophenyl)-3-[3-(5-chloro-thiophen-2-yl)-1-[(2-methyl-pyrimidin-5-yl)-methyl]-piperidin-4-yl]-urea
**Ex-77** trans-1-(4-Chlorophenyl)-3-[3-(5-chloro-thiophen-2-yl)-1-[(1-methylsulfonyl-1H-pyrazol-3-yl)-methyl]-piperidin-4-yl]-urea ent-1
**Ex-78** trans-1-(4-Chlorophenyl)-3-[3-(5-chloro-thiophen-2-yl)-1-[(1-methylsulfonyl-1H-pyrazol-3-yl)-methyl]-piperidin-4-yl]-urea ent-2
**Ex-79** trans-1-(4-Chlorophenyl)-3-[1-(3-methoxy-propanoyl)-3-(5-methoxy-pyridin-2-yl)-piperidin-4-yl]-urea
**Ex-80** trans-1-(4-Chlorophenyl)-3-[1-(3-methoxy-propyl)-3-(5-methoxy-pyridin-2-yl)-piperidin-4-yl]-urea
**Ex-81** trans-1-(4-Chlorophenyl)-3-[1-(4-methoxy-cyclohexanecarbonyl)-3-(5-methoxy-pyridin-2-yl)-piperidin-4-yl]-urea
**Ex-82** trans-1-(4-Chlorophenyl)-3-[3-(5-methoxy-pyridin-2-yl)-1-(piperidin-4-yl-methyl)-piperidin-4-yl]-urea ent-2
**Ex-83** trans-1-(4-Chlorophenyl)-3-[3-(5-chloro-thiophen-2-yl)-1-(piperidin-4-yl-methyl)-piperidin-4-yl]-urea dihydrochloride ent-1
**Ex-84** trans-1-(4-Chlorophenyl)-3-[3-(5-chloro-thiophen-2-yl)-1-(piperidin-4-yl-methyl)-piperidin-4-yl]-urea dihydrochloride ent-2
**Ex-85** trans-1-(4-Chlorophenyl)-3-[3-(5-chloro-thiophen-2-yl)-1-(tetrahydro-pyran-4-yl-methyl)-piperidin-4-yl]-urea ent-1
**Ex-86** trans-1-(4-Chlorophenyl)-3-[3-(5-chloro-thiophen-2-yl)-1-(tetrahydro-pyran-4-yl-methyl)-piperidin-4-yl]-urea ent-2
**Ex-87** trans-1-(4-Chlorophenyl)-3-[3-(5-chloro-thiophen-2-yl)-1-[(6-methyl-pyridin-3-yl)-methyl]-piperidin-4-yl]-urea
**Ex-88** trans-1-(4-Chlorophenyl)-3-[3-(5-methoxy-pyridin-2-yl)-1-(piperidine-4-carbonyl)-piperidin-4-yl]-urea
**Ex-89** trans-1-(4-Chlorophenyl)-3-[3-(5-methoxy-pyridin-2-yl)-1-(1-methyl-piperidine-4-carbonyl)-piperidin-4-yl]-urea
**Ex-90** trans-1-(4-Chlorophenyl)-3-[1-(4-hydroxy-cyclohexanecarbonyl)-3-(5-methoxy-piperidine-2-yl)-piperidin-4-yl]-urea
**Ex-91** trans-1-(4-Chlorophenyl)-3-[3-(5-chloro-thiophen-2-yl)-1-(3-methoxy-propyl)-piperidin-4-yl]-urea
**Ex-92** trans-1-(4-Chlorophenyl)-3-[3-(5-methoxy-pyridin-2-yl)-1-(oxetane-3-carbonyl)-piperidine-4-yl]-urea
**Ex-93** trans-1-(4-Chlorophenyl)-3-[3-(5-methoxy-pyridin-2-yl)-1-(2-methylsulfonyl-ethyl)-piperidin-4-yl]-urea
**Ex-94** trans-1-(4-Chlorophenyl)-3-[1-(2-dimethylamino-acetyl)-3-(5-methoxy-pyridin-2-yl)-piperidin-4-yl]-urea
**Ex-95** trans-1-(4-Chlorophenyl)-3-[1-(3-dimethylamino-propanoyl)-3-(5-methoxy-pyridin-2-yl)-piperidin-4-yl]-urea
**Ex-96** trans-1-(4-Chlorophenyl)-3-[3-(5-methoxy-pyridin-2-yl)-1-(2-methylamino-acetyl)-piperidin-4-yl]-urea
**Ex-97** trans-1-(4-Chlorophenyl)-3-[3-(5-methoxy-pyridin-2-yl)-1-(3-methylamino-propanoyl)-piperidin-4-yl]-urea
**Ex-98** trans-1-(4-Chlorophenyl)-3-[3-(5-methoxy-pyridin-2-yl)-1-[4-(trifluoromethyl)-pyridine-3-carbonyl]-piperidin-4-yl]-urea ent-1
**Ex-99** trans-1-(4-Chlorophenyl)-3-[3-(5-methoxy-pyridin-2-yl)-1-[4-(trifluoromethyl)-pyridine-3-carbonyl]-piperidin-4-yl]-urea ent-2
**Ex-100** trans-1-(4-Chlorophenyl)-3-[3-(5-methoxy-pyridin-2-yl)-1-[6-(trifluoromethyl)-pyridine-3-carbonyl]-piperidin-4-yl]-urea ent-1
**Ex-101** trans-1-(4-Chlorophenyl)-3-[3-(5-methoxy-pyridin-2-yl)-1-[6-(trifluoromethyl)-pyridine-3-carbonyl]-piperidin-4-yl]-urea ent-2
**Ex-102** trans-1-(4-Chlorophenyl)-3-[1-(3-hydroxy-propanoyl)-3-(5-methoxy-pyridin-2-yl)-piperidin-4-yl]-urea
**Ex-103** trans-1-(4-Chlorophenyl)-3-[1-(3-hydroxy-propyl)-3-(5-methoxy-pyridin-2-yl)-piperidin-4-yl]-urea
**Ex-104** trans-1-(4-Chlorophenyl)-3-[3-(5-chloro-thiophen-2-yl)-1-(1-methyl-piperidine-4-carbonyl)-piperidin-4-yl]-urea
**Ex-105** trans-1-(4-Chlorophenyl)-3-[3-(5-chloro-thiophen-2-yl)-1-(3-hydroxy-propyl)-piperidin-4-yl]-urea
**Ex-106** trans-1-(4-Chlorophenyl)-3-[3-(5-chloro-thiophen-2-yl)-1-(3-methoxy-propanoyl)-piperidin-4-yl]-urea
**Ex-107** trans-1-(4-Chlorophenyl)-3-[3-(5-chloro-thiophen-2-yl)-1-(4-methoxy-cyclohexanecarbonyl)-piperidin-4-yl]-urea
**Ex-108** trans-1-(4-Chlorophenyl)-3-[3-(5-methoxy-pyridin-2-yl)-1-(oxetan-3-yl)-piperidin-4-yl]-urea
**Ex-109** trans-1-(4-Chlorophenyl)-3-[3-(5-methoxy-pyridin-2-yl)-1-(oxetan-3-yl-methyl)-piperidin-4-yl]-urea
**Ex-110** trans-1-(4-Chlorophenyl)-3-[1-(1,1-dioxo-thian-4-yl)-3-(5-methoxy-pyridin-2-yl)-piperidin-4-yl]-urea
**Ex-111** trans-1-(4-Chlorophenyl)-3-[1-(3-dimethylamino-propyl)-3-(5-methoxy-pyridin-2-yl)-piperidin-4-yl]-urea
**Ex-112** trans-1-(4-Chlorophenyl)-3-[3-(5-methoxy-pyridin-2-yl)-piperidin-4-yl]-urea
**Ex-113** trans-1-(4-Cyano-phenyl)-3-[1-(2,2-difluoro-ethyl)-3-(5-methoxy-pyridin-2-yl)-piperidin-4-yl]-urea
**Ex-114** trans-1-(4-Chlorophenyl)-3-[1-[(4-hydroxy-cyclohexyl)-methyl]-3-(5-methoxy-pyridin-2-yl)-piperidin-4-yl]-urea
**Ex-115** trans-1-(4-Chlorophenyl)-3-[1-[(4-methoxy-cyclohexyl)-methyl]-3-(5-methoxy-pyridin-2-yl)-piperidin-4-yl]-urea
**Ex-116** trans-1-(4-Chlorophenyl)-3-[3-(5-chloro-thiophen-2-yl)-1-[(4-methoxy-cyclohexyl)-methyl]-piperidin-4-yl]-urea
**Ex-117** trans-1-(4-Chlorophenyl)-3-[3-(5-methoxy-pyridin-2-yl)-1-(2-methylamino-ethyl)-piperidin-4-yl]-urea
**Ex-118** trans-1-[1-(2-Dimethylamino-acetyl)-3-(5-methoxy-pyridin-2-yl)-piperidin-4-yl]-3-(3-methyl-isothiazol-5-yl)-urea
**Ex-119** trans-1-[3-(5-Methoxy-pyridin-2-yl)-1-(oxetane-3-carbonyl)-piperidin-4-yl]-3-(3-methyl-isothiazol-5-yl)-urea
**Ex-120** trans-1-(4-Chlorophenyl)-3-[1-(2-hydroxy-2-methyl-propyl)-3-(5-methoxy-pyridin-2-yl)-piperidin-4-yl]-urea
**Ex-121** trans-1-[1-(2-Hydroxy-2-methyl-propyl)-3-(5-methoxy-pyridin-2-yl)-piperidin-4-yl]-3-(3-methyl-isothiazol-5-yl)-urea
**Ex-122** trans-1-[1-(2-Hydroxy-ethyl)-3-(5-methoxy-pyridin-2-yl)-piperidin-4-yl]-3-(3-methyl-isothiazol-5-yl)-urea
**Ex-123** trans-1-[1-(2-Hydroxy-acetyl)-3-(5-methoxy-pyridin-2-yl)-piperidin-4-yl]-3-(3-methyl-isothiazol-5-yl)-urea
**Ex-124** trans-1-(4-Chlorophenyl)-3-[3-(5-methoxy-pyridin-2-yl)-1-(piperidin-4-yl-methyl)-piperidin-4-yl]-urea ent-1
**Ex-125** trans-1-(4-Chlorophenyl)-3-[3-(5-methoxy-pyridin-2-yl)-1-[(1-methylsulfonyl-1H-pyrazol-4-yl)-methyl]-piperidin-4-yl]-urea
**Ex-126** trans-1-(4-Chlorophenyl)-3-[3-(5-methoxy-pyridin-2-yl)-1-methyl-piperidin-4-yl]-urea
**Ex-127** trans-1-(4-Chlorophenyl)-3-[1-(cyclopropyl-methyl)-3-(5-methoxy-pyridin-2-yl)-piperidin-4-yl]-urea
**Ex-128** trans-1-(4-Cyano-phenyl)-3-[3-(5-methoxy-pyridin-2-yl)-1-(6-methyl-pyridine-3-carbonyl)-piperidin-4-yl]-urea
**Ex-129** trans-2-[4-[[(4-Chlorophenyl)-carbamoyl]amino]-3-(5-methoxy-pyridin-2-yl)-piperidin-1-yl]-acetamide
**Ex-130** trans-1-(4-Chlorophenyl)-3-[3-(5-chloro-thiophen-2-yl)-1-(piperidine-4-carbonyl)-piperidin-4-yl]-urea
**Ex-131** trans-1-(4-Chlorophenyl)-3-[3-(5-chloro-thiophen-2-yl)-1-(3-hydroxy-propanoyl)-piperidin-4-yl]-urea
**Ex-132** trans-1-(4-Chlorophenyl)-3-[3-(5-chloro-thiophen-2-yl)-1-(4-hydroxy-cyclohexanecarbonyl)-piperidin-4-yl]-urea
**Ex-133** trans-1-[3-(5-Methoxy-pyridin-2-yl)-1-(oxetan-3-yl)-piperidin-4-yl]-3-(3-methyl-isothiazol-5-yl)-urea
**Ex-134** trans-1-[3-(5-Methoxy-pyridin-2-yl)-1-(2-methylamino-ethyl)-piperidin-4-yl]-3-(3-methyl-isothiazol-5-yl)-urea
**Ex-135** trans-1-[3-(5-Methoxy-pyridin-2-yl)-1-(3-methylamino-propanoyl)-piperidin-4-yl]-3-(3-methyl-isothiazol-5-yl)-urea
**Ex-136** trans-1-(4-Chlorophenyl)-3-[1-[2-(dimethylamino)ethyl]-3-(5-methoxy-pyridin-2-yl)-piperidin-4-yl]-urea
**Ex-137** trans-1-(4-Chlorophenyl)-3-[3-(5-methoxy-pyridin-2-yl)-1-(3-methylamino-propyl)-piperidin-4-yl]-urea
**Ex-138** trans-1-[1-(3-Hydroxy-cyclobutanecarbonyl)-3-(5-methoxy-pyridin-2-yl)-piperidin-4-yl]-3-(3-methyl-isothiazol-5-yl)-urea
**Ex-139** trans-1-[1-(2-Hydroxy-acetyl)-3-(5-methoxy-pyridin-2-yl)-piperidin-4-yl]-3-phenyl-urea
**Ex-140** trans-1-(4-Cyano-phenyl)-3-[3-(5-methoxy-pyridin-2-yl)-1-[(1-methylsulfonyl-1H-pyrazol-4-yl)-methyl]-piperidin-4-yl]-urea
**Ex-141** trans-1-(4-Cyano-phenyl)-3-[3-(5-methoxy-pyridin-2-yl)-1-[6-(trifluoromethyl)-pyridine-3-carbonyl]-piperidin-4-yl]-urea
**Ex-142** trans-3-[4-[[(4-Chlorophenyl)-carbamoyl]amino]-3-(5-methoxy-pyridin-2-yl)-piperidin-1-yl]-propionamide
**Ex-143** trans-1-[[4-[[(4-Chlorophenyl)-carbamoyl]amino]-3-(5-methoxy-pyridin-2-yl)-piperidin-1-yl]-methyl]-cyclopropane-1-carboxyfic acid amide
**Ex-144** trans-1-(4-Chlorophenyl)-3-[1-(3-cyano-3-methyl-butyl)-3-(5-methoxy-pyridin-2-yl)-piperidin-4-yl]-urea
**Ex-145** trans-1-(4-Chlorophenyl)-3-[3-(5-methoxy-pyridin-2-yl)-1-[(1-methyl-1H-pyrazol-4-yl)-methyl]-piperidin-4-yl]-urea
**Ex-146** trans-1-(4-Cyano-phenyl)-3-[3-(5-methoxy-pyridin-2-yl)-1-[(1-methyl-1H-pyrazol-4-yl)-methyl]-piperidin-4-yl]-urea
**Ex-147** trans-1-(4-Chlorophenyl)-3-[3-(5-methoxy-pyridin-2-yl)-1-[(1-methyl-piperidin-4-yl)-methyl]-piperidin-4-yl]-urea
**Ex-148** trans-1-[3-(5-Methoxy-pyridin-2-yl)-1-(2-methylsulfonyl-ethyl)-piperidin-4-yl]-3-(3-methyl-isothiazol-5-yl)-urea
**Ex-149** trans-1-[1-(1,1-Dioxo-thian-4-yl)-3-(5-methoxy-pyridin-2-yl)-piperidin-4-yl]-3-(3-methyl-isothiazol-5-yl)-urea
**Ex-150** trans-1-[1-(2,2-Difluoro-ethyl)-3-(5-methoxy-pyridin-2-yl)-piperidin-4-yl]-3-phenyl-urea
**Ex-151** trans-1-[3-(5-Methoxy-pyridin-2-yl)-1-(piperidine-4-carbonyl)-piperidin-4-yl]-3-phenyl-urea
**Ex-152** trans-1-[3-(5-Chloro-pyridin-2-yl)-1-(2-hydroxy-ethyl)-piperidin-4-yl]-3-phenyl-urea
**Ex-153** trans-1-[3-(5-Chloro-pyridin-2-yl)-1-(2,2-difluoro-ethyl)-piperidin-4-yl]-3-phenyl-urea
**Ex-154** trans-1-(4-Cyano-phenyl)-3-[1-(2-methoxy-ethyl)-3-(5-methoxy-pyridin-2-yl)-piperidin-4-yl]-urea
**Ex-155** trans-1-(4-Chlorophenyl)-3-[1-[(1-cyano-cyclopropyl)-methyl]-3-(5-methoxy-pyridin-2-yl)-piperidin-4-yl]-urea
**Ex-156** trans-1-[3-(5-Methoxy-pyridin-2-yl)-1-(1-methyl-piperidine-4-carbonyl)-piperidin-4-yl]-3-phenyl-urea
**Ex-157** trans-1-[1-(2-Dimethylamino-acetyl)-3-(5-methoxy-pyridin-2-yl)-piperidin-4-yl]-3-phenyl-urea
**Ex-158** trans-1-[1-(3-Methoxy-propyl)-3-(5-methoxy-pyridin-2-yl)-piperidin-4-yl]-3-phenyl-urea
**Ex-159** trans-1-[1-(3-Hydroxy-propyl)-3-(5-methoxy-pyridin-2-yl)-piperidin-4-yl]-3-phenyl-urea
**Ex-160** trans-1-[3-(5-Methoxy-pyridin-2-yl)-1-(2-methylsulfonyl-ethyl)-piperidin-4-yl]-3-phenyl-urea
**Ex-161** trans-1-[3-(5-Methoxy-pyridin-2-yl)-1-(oxetan-3-yl-methyl)-piperidin-4-yl]-3-phenyl-urea
**Ex-162** trans-1-[3-(5-Methoxy-pyridin-2-yl)-1-(oxetan-3-yl)-piperidin-4-yl]-3-phenyl-urea
**Ex-163** trans-1-(4-Chlorophenyl)-3-[1-[(1-hydroxy-cyclopropyl)-methyl]-3-(5-methoxy-pyridin-2-yl)-piperidin-4-yl]-urea
**Ex-164** trans-1-[1-(2-Hydroxy-ethyl)-3-(5-methoxy-pyridin-2-yl)-piperidin-4-yl]-3-phenyl-urea
**Ex-165** trans-1-[1-(Azetidine-3-carbonyl)-3-(5-methoxy-pyridin-2-yl)-piperidin-4-yl]-3-phenyl-urea
**Ex-167** trans-1-[3-(5-Chloro-pyridin-2-yl)-1-(piperidine-4-carbonyl)-piperidin-4-yl]-3-phenyl-urea
**Ex-168** trans-1-(4-Chlorophenyl)-3-[3-(5-methoxy-pyridin-2-yl)-piperidin-4-yl]-urea ent-1
**Ex-169** trans-1-(4-Chlorophenyl)-3-[3-(5-methoxy-pyridin-2-yl)-piperidin-4-yl]-urea ent-2
**Ex-170** trans-2-[4-[[(4-Chlorophenyl)-carbamoyl]amino]-3-(5-methoxy-pyridin-2-yl)-piperidin-1-yl]-acetamide ent-1
**Ex-171** trans-2-[4-[[(4-Chlorophenyl)-carbamoyl]amino]-3-(5-methoxy-pyridin-2-yl)-piperidin-1-yl]-acetamide ent-2
**Ex-172** trans-1-(4-Chlorophenyl)-3-[1-(cyclopropyl-methyl)-3-(5-methoxy-pyridin-2-yl)-piperidin-4-yl]-urea ent-1
**Ex-173** trans-1-(4-Chlorophenyl)-3-[1-(cyclopropyl-methyl)-3-(5-methoxy-pyridin-2-yl)-piperidin-4-yl]-urea ent-2
**Ex-174** trans-1-(4-Cyano-phenyl)-3-[1-(2,2-difluoro-ethyl)-3-(5-methoxy-pyridin-2-yl)-piperidin-4-yl]-urea ent-2
**Ex-175** trans-1-(4-Cyano-phenyl)-3-[1-(2,2-difluoro-ethyl)-3-(5-methoxy-pyridin-2-yl)-piperidin-4-yl]-urea ent-2
**Ex-176** trans-1-[1-(2-Hydroxy-2-methyl-propyl)-3-(5-methoxy-pyridin-2-yl)-piperidin-4-yl]-3-(3-methyl-isothiazol-5-yl)-urea ent-1
**Ex-177** trans-1-[1-(2-Hydroxy-2-methyl-propyl)-3-(5-methoxy-pyridin-2-yl)-piperidin-4-yl]-3-(3-methyl-isothiazol-5-yl)-urea ent-2
**Ex-178** trans-1-(4-Chlorophenyl)-3-[1-(2-hydroxy-2-methyl-propyl)-3-(5-methoxy-pyridin-2-yl)-piperidin-4-yl]-urea ent-1
**Ex-179** trans-1-(4-Chlorophenyl)-3-[1-(2-hydroxy-2-methyl-propyl)-3-(5-methoxy-pyridin-2-yl)-piperidin-4-yl]-urea ent-2
**Ex-180** trans-1-(4-Chlorophenyl)-3-[1-(1-cyano-cyclopropyl)-3-(5-methoxy-pyridin-2-yl)-piperidin-4-yl]-urea
**Ex-181** trans-1-[4-[[(4-Chlorophenyl)-carbamoyl]amino]-3-(5-methoxy-pyridin-2-yl)-piperidin-1-yl]-cyclopropane-1-carboxylic acid amide
**Ex-182** trans-1-[1-[(1-Hydroxy-cyclopropyl)-methyl]-3-(5-methoxy-pyridin-2-yl)-piperidin-4-yl]-3-(3-methyl-isothiazol-5-yl)-urea
**Ex-183** trans-1-[1-(3-Hydroxy-cyclobutanecarbonyl)-3-(5-methoxy-pyridin-2-yl)-piperidin-4-yl]-3-(3-methyl-isothiazol-5-yl)-urea
**Ex-184** trans-1-[3-(5-Chloro-pyridin-2-yl)-1-[(1-methylsulfonyl-1H-pyrazol-3-yl)-methyl]-piperidin-4-yl]-3-phenyl-urea
**Ex-185** trans-1-[1-(Azetidin-3-yl)-3-(5-chloro-pyridin-2-yl)-piperidin-4-yl]-3-phenyl-urea
**Ex-186** trans-1-[1-(Azetidin-3-yl-methyl)-3-(5-chloro-pyridin-2-yl)-piperidin-4-yl]-3-phenyl-urea
**Ex-187** trans-1-(4-Chlorophenyl)-3-[3-(5-methoxy-pyridin-2-yl)-1-(1-methyl-piperidine-4-carbonyl)-piperidin-4-yl]-urea ent-1
**Ex-188** trans-1-(4-Chlorophenyl)-3-[3-(5-methoxy-pyridin-2-yl)-1-(1-methyl-piperidine-4-carbonyl)-piperidin-4-yl]-urea ent-2
**Ex-189** trans-1-(4-Chlorophenyl)-3-[1-(2-dimethylamino-acetyl)-3-(5-methoxy-pyridin-2-yl)-piperidin-4-yl]-urea ent-1
**Ex-190** trans-1-(4-Chlorophenyl)-3-[1-(2-dimethylamino-acetyl)-3-(5-methoxy-pyridin-2-yl)-piperidin-4-yl]-urea ent-2
**Ex-191** trans-1-(4-Chlorophenyl)-3-[1-(3-methoxy-propyl)-3-(5-methoxy-pyridin-2-yl)-piperidin-4-yl]-urea ent-1
**Ex-192** trans-1-(4-Chlorophenyl)-3-[1-(3-methoxy-propyl)-3-(5-methoxy-pyridin-2-yl)-piperidin-4-yl]-urea ent-2
**Ex-193** trans-1-(4-Chlorophenyl)-3-[3-(3-fluoro-5-methoxy-pyridin-2-yl)-1-(3-methoxy-propyl)-piperidin-4-yl]-urea
**Ex-194** trans-1-(4-Chlorophenyl)-3-[1-[(3-hydroxy-oxetan-3-yl)-methyl]-3-(5-methoxy-pyridin-2-yl)-piperidin-4-yl]-urea
**Ex-195** trans-1-(4-Chlorophenyl)-3-[1-[(3-methoxy-cyclobutyl)-methyl]-3-(5-methoxy-pyridin-2-yl)-piperidin-4-yl]-urea
**Ex-196** trans-1-(4-Chlorophenyl)-3-[3-(3-fluoro-5-methoxy-pyridin-2-yl)-1-(2-methylsulfonyl-ethyl)-piperidin-4-yl]-urea
**Ex-197** trans-1-(4-Chlorophenyl)-3-[3-(3-fluoro-5-methoxy-pyridin-2-yl)-1-(3-hydroxy-propyl)-piperidin-4-yl]-urea
**Ex-198** trans-1-(4-Chlorophenyl)-3-[3-(3-fluoro-5-methoxy-pyridin-2-yl)-1-(2-methoxy-ethyl)-piperidin-4-yl]-urea
**Ex-199** trans-1-(4-Chlorophenyl)-3-[3-(3-fluoro-5-methoxy-pyridin-2-yl)-1-(2-hydroxy-ethyl)-piperidin-4-yl]-urea
**Ex-200** trans-1-(4-Chlorophenyl)-3-[3-(3-fluoro-5-methoxy-pyridin-2-yl)-1-(oxetan-3-yl-methyl)-piperidin-4-yl]-urea
**Ex-201** trans-1-(4-Chlorophenyl)-3-[1-[(1-methoxy-cyclopropyl)-methyl]-3-(5-methoxy-pyridin-2-yl)-piperidin-4-yl]-urea
**Ex-202** trans-1-(4-Chlorophenyl)-3-[1-[(3-methoxy-cyclobutyl)-methyl]-3-(5-methoxy-pyridin-2-yl)-piperidin-4-yl]-urea
**Ex-203** trans-1-(4-Chlorophenyl)-3-[1-[(3-hydroxy-cyclobutyl)-methyl]-3-(5-methoxy-pyridin-2-yl)-piperidin-4-yl]-urea
**Ex-204** trans-1-[3-(5-Methoxy-pyridin-2-yl)-1-(oxetan-3-yl-methyl)-piperidin-4-yl]-3-(3-methyl-isothiazol-5-yl)-urea
**Ex-205** trans-1-(4-Chlorophenyl)-3-[1-[(3-hydroxy-cyclobutyl)-methyl]-3-(5-methoxy-pyridin-2-yl)-piperidin-4-yl]-urea
**Ex-206** trans-1-(4-Chlorophenyl)-3-[1-(2-hydroxy-ethyl)-3-(5-methoxy-pyridin-2-yl)-piperidin-4-yl]-urea
**Ex-207** trans-1-(4-Chlorophenyl)-3-[1-[(3-hydroxy-3-methyl-cyclobutyl)-methyl]-3-(5-methoxy-pyridin-2-yl)-piperidin-4-yl]-urea
**Ex-208** trans-1-(4-Chlorophenyl)-3-[3-(3-fluoro-5-methoxy-pyridin-2-yl)-1-(2-hydroxy-2-methylpropyl)-piperidin-4-yl]-urea
**Ex-209** trans-1-(4-Chlorophenyl)-3-[1-(cyclopropyl-methyl)-3-(3-fluoro-5-methoxy-pyridin-2-yl)-piperidin-4-yl]-urea
**Ex-210** trans-1-(4-Chlorophenyl)-3-[3-(5-methoxy-pyridin-2-yl)-1-(2-methyl-pyrimidin-4-yl)-piperidin-4-yl]-urea
**Ex-211** trans-1-[3-(5-Methoxy-pyridin-2-yl)-1-(2-methyl-pyrimidin-4-yl)-piperidin-4-yl]-3-(3-methyl-isothiazol-5-yl)-urea
**Ex-212** trans-1-(4-Chlorophenyl)-3-[1-(3-dimethylamino-propyl)-3-(3-fluoro-5-methoxy-pyridin-2-yl)-piperidin-4-yl]-urea
**Ex-213** trans-1-(4-Chlorophenyl)-3-[1-[2-(dimethylamino)ethyl]-3-(3-fluoro-5-methoxy-pyridin-2-yl)-piperidin-4-yl]-urea
**Ex-214** trans-1-[1-(2,2-Difluoro-ethyl)-3-(5-methoxy-pyridin-2-yl)-piperidin-4-yl]-3-(3-methyl-isothiazol-5-yl)-urea
**Ex-215** trans-2-[4-[[(4-Chlorophenyl)-carbamoyl]amino]-3-(5-methoxy-pyridin-2-yl)-piperidin-1-yl]-N-methyl-acetamide
**Ex-216** trans-2-[4-[[(4-Chlorophenyl)-carbamoyl]amino]-3-(5-methoxy-pyridin-2-yl)-piperidin-1-yl]-N,N-dimethyl-acetamide
**Ex-217** trans-1-[1-[(3-Hydroxy-oxetan-3-yl)-methyl]-3-(5-methoxy-pyridin-2-yl)-piperidin-4-yl]-3-(3-methyl-isothiazol-5-yl)-urea
**Ex-218** trans-1-(4-Chlorophenyl)-3-[3-(3-fluoro-5-methoxy-pyridin-2-yl)-1-(oxetan-3-yl)-piperidin-4-yl]-urea
**Ex-219** trans-1-(4-Chlorophenyl)-3-[1-(2-hydroxy-1,1-dimethyl-ethyl)-3-(5-methoxy-pyridin-2-yl)-piperidin-4-yl]-urea
**Ex-220** trans-1-(4-Chlorophenyl)-3-[1-[(1-hydroxy-cyclobutyl)-methyl]-3-(5-methoxy-pyridin-2-yl)-piperidin-4-yl]-urea
**Ex-221** trans-1-(4-Chlorophenyl)-3-[3-(3-fluoro-5-methoxy-pyridin-2-yl)-1-[(4-hydroxy-cyclohexyl)-methyl]-piperidin-4-yl]-urea
**Ex-222** trans-2-[4-[[(4-Chlorophenyl)-carbamoyl]amino]-3-(3-fluoro-5-methoxy-pyridin-2-yl)-piperidin-1-yl]-acetamide
**Ex-223** trans-1-(4-Chlorophenyl)-3-[3-(3-fluoro-5-methoxy-pyridin-2-yl)-1-[(4-methoxy-cyclohexyl)-methyl]-piperidin-4-yl]-urea
**Ex-224** trans-1-(4-Chlorophenyl)-3-[3-(5-methoxy-pyridin-2-yl)-1-(6-methyl-pyrazin-2-yl)-piperidin-4-yl]-urea
**Ex-225** trans-2-[4-[[(4-Chlorophenyl)-carbamoyl]amino]-3-(5-methoxy-pyridin-2-yl)-piperidin-1-yl]-2-methyl-propionamide
**Ex-226** trans-1-(4-Chlorophenyl)-3-[3-(5-methoxy-pyridin-2-yl)-1-pyrazin-2-yl-piperidin-4-yl]-urea
**Ex-227** trans-1-(4-Chlorophenyl)-3-[1-(2-methoxy-2-methyl-propyl)-3-(5-methoxy-pyridin-2-yl)-piperidin-4-yl]-urea
**Ex-228** trans-1-(4-Chlorophenyl)-3-[1-[(3-methoxy-3-methyl-cyclobutyl)-methyl]-3-(5-methoxy-pyridin-2-yl)-piperidin-4-yl]-urea
**Ex-229** trans-1-(4-Chlorophenyl)-3-[1-[(1-methoxy-cyclobutyl)-methyl]-3-(5-methoxy-pyridin-2-yl)-piperidin-4-yl]-urea
**Ex-230** trans-3-[4-[[(4-Chlorophenyl)-carbamoyl]amino]-3-(5-methoxy-pyridin-2-yl)-piperidin-1-yl]-2,2-dimethyl-propionamide
**Ex-231** trans-1-(4-Chlorophenyl)-3-[3-(5-methoxy-pyridin-2-yl)-1-pyridazin-3-yl-piperidin-4-yl]-urea
**Ex-232** trans-1-(4-Chlorophenyl)-3-[3-(5-methoxy-pyridin-2-yl)-1-(6-methyl-pyrimidin-4-yl)-piperidin-4-yl]-urea
**Ex-233** trans-1-(4-Chlorophenyl)-3-[1-(1-cyano-1-methyl-ethyl)-3-(5-methoxy-pyridin-2-yl)-piperidin-4-yl]-urea
**Ex-234** trans-1-(4-Chlorophenyl)-3-[1-(2-cyano-2-methyl-propyl)-3-(5-methoxy-pyridin-2-yl)-piperidin-4-yl]-urea
**Ex-235** trans-1-(4-Chlorophenyl)-3-[1-[2-(1-cyano-cyclopropyl)-ethyl]-3-(5-methoxy-pyridin-2-yl)-piperidin-4-yl]-urea
**Ex-236** trans-1-(4-Chlorophenyl)-3-[3-(5-methoxy-pyridin-2-yl)-l-(2,2,6,6-tetramethyl-piperidin-4-yl)-piperidin-4-yl]-urea ent-1
**Ex-237** trans-1-(4-Chlorophenyl)-3-[3-(5-methoxy-pyridin-2-yl)-1-(2,2,6,6-tetramethyl-piperidin-4-yl)-piperidin-4-yl]-urea ent-2
**Ex-238** trans-1-(4-Chlorophenyl)-3-[1-(3-dimethylamino-2,2-difluoro-propyl)-3-(5-methoxy-pyridin-2-yl)-piperidin-4-yl]-urea
**Ex-239** trans-1-[4-[[(4-Chlorophenyl)-carbamoyl]amino]-3-(5-methoxy-pyridin-2-yl)-piperidin-1-yl]-N-methyl-cyclopropane-1-carboxylic acid amide
**Ex-240** trans-1-[4-[[(4-Chlorophenyl)-carbamoyl]amino]-3-(5-methoxy-pyridin-2-yl)-piperidin-1-yl]-N,N-dimethyl-cyclopropane-1-carboxylic acid amide
**Ex-241** trans-1-[1-[(3-Hydroxy-cyclobutyl)-methyl]-3-(5-methoxy-pyridin-2-yl)-piperidin-4-yl]-3-(3-methyl-isothiazol-5-yl)-urea
**Ex-242** trans-1-[1-[(3-Methoxy-cyclobutyl)-methyl]-3-(5-methoxy-pyridin-2-yl)-piperidin-4-yl]-3-(3-methyl-isothiazol-5-yl)-urea
**Ex-243** trans-1-[1-[(3-Hydroxy-cyclobutyl)-methyl]-3-(5-methoxy-pyridin-2-yl)-piperidin-4-yl]-3-(3-methyl-isothiazol-5-yl)-urea
**Ex-244** trans-1-[1-[(3-Methoxy-cyclobutyl)-methyl]-3-(5-methoxy-pyridin-2-yl)-piperidin-4-yl]-3-(3-methyl-isothiazol-5-yl)-urea
**Ex-245** trans-1-[1-[(3-Hydroxy-3-methyl-cyclobutyl)-methyl]-3-(5-methoxy-pyridin-2-yl)-piperidin-4-yl]-3-(3-methyl-isothiazol-5-yl)-urea
**Ex-246** trans-1-[1-[(3-Methoxy-3-methyl-cyclobutyl)-methyl]-3-(5-methoxy-pyridin-2-yl)-piperidin-4-yl]-3-(3-methyl-isothiazol-5-yl)-urea
**Ex-247** trans-1-(4-Chlorophenyl)-3-[1-[1-(hydroxymethyl)-cyclopropyl]-3-(5-methoxy-pyridin-2-yl)-piperidin-4-yl]-urea
**Ex-248** trans-1-(4-Chlorophenyl)-3-[1-[1-(methoxymethyl)-cyclopropyl]-3-(5-methoxy-pyridin-2-yl)-piperidin-4-yl]-urea
**Ex-249** trans-1-(4-Chlorophenyl)-3-[1-(2-methoxy-1,1-dimethyl-ethyl)-3-(5-methoxy-pyridin-2-yl)-piperidin-4-yl]-urea
**Ex-250** trans-1-(4-Chlorophenyl)-3-[1-[3-(methoxymethyl)-oxetan-3-yl]-3-(5-methoxy-pyridin-2-yl)-piperidin-4-yl]-urea
**Ex-251** trans-1-(4-Chlorophenyl)-3-[1-[3-(hydroxymethyl)-oxetan-3-yl]-3-(5-methoxy-pyridm-2-yl)-piperidin-4-yl]-urea
**Ex-252** trans-1-(4-Chlorophenyl)-3-[1-(2,2-difluoro-3-hydroxy-propyl)-3-(5-methoxy-pyridin-2-yl)-piperidin-4-yl]-urea
**Ex-253** trans-1-(4-Chlorophenyl)-3-[1-(2,2-difluoro-3-methoxy-propyl)-3-(5-methoxy-pyridin-2-yl)-piperidin-4-yl]-urea
**Ex-254** trans-1-[1-(2,2-Difluoro-3-hydroxy-propyl)-3-(5-methoxy-pyridin-2-yl)-piperidin-4-yl]-3-(3-methyl-isothiazol-5-yl)-urea
**Ex-255** trans-1-[1-(2,2-Difluoro-3-methoxy-propyl)-3-(5-methoxy-pyridin-2-yl)-piperidin-4-yl]-3-(3-methyl-isothiazol-5-yl)-urea
**Ex-256** trans-1-(4-Chlorophenyl)-3-[3-(5-methoxy-pyridin-2-yl)-1-pyridazin-4-yl-piperidin-4-yl]-urea
**Ex-257** trans-1-(4-Chlorophenyl)-3-[3-(5-methoxy-pyridin-2-yl)-1-(2-methyl-pyrimidin-5-yl)-piperidin-4-yl]-urea
**Ex-258** trans-1-(4-Chlorophenyl)-3-[1-[2-(hydroxymethyl)-pyrimidin-5-yl]-3-(5-methoxy-pyridin-2-yl)-piperidin-4-yl]-urea
**Ex-259** trans-1-(4-Chlorophenyl)-3-[1-[2-(hydroxymethyl)-phenyl]-3-(5-methoxy-pyridin-2-yl)-piperidin-4-yl]-urea
**Ex-260** trans-1-(4-Chlorophenyl)-3-[1-[3-(hydroxymethyl)-phenyl]-3-(5-methoxy-pyridin-2-yl)-piperidin-4-yl]-urea
**Ex-261** trans-1-(4-Chlorophenyl)-3-[1-[4-(hydroxymethyl)-phenyl]-3-(5-methoxy-pyridin-2-yl)-piperidin-4-yl]-urea
**Ex-262** trans-1-(4-Chlorophenyl)-3-[1-[6-(hydroxymethyl)-pyridin-3-yl]-3-(5-methoxy-pyridin-2-yl)-piperidin-4-yl]-urea
**Ex-263** trans-1-(4-Chlorophenyl)-3-[1-[6-(2-hydroxy-ethylamino)-pyridin-2-yl]-3-(5-methoxy-pyridin-2-yl)-piperidin-4-yl]-urea
**Ex-264** trans-1-(4-Chlorophenyl)-3-[1-[5-(2-hydroxy-ethylamino)-pyridin-2-yl]-3-(5-methoxy-pyridin-2-yl)-piperidin-4-yl]-urea
**Ex-265** trans-1-(4-Chlorophenyl)-3-[1-[4-(2-hydroxy-ethylamino)-pyridin-2-yl]-3-(5-methoxy-pyridin-2-yl)-piperidin-4-yl]-urea
**Ex-266** trans-1-(4-Chlorophenyl)-3-[1-[(1-hydroxy-cyclopropyl)-methyl]-3-(5-methoxy-pyridin-2-yl)-piperidin-4-yl]-urea ent-1
**Ex-267** trans-1-(4-Chlorophenyl)-3-[1-[(1-hydroxy-cyclopropyl)-methyl]-3-(5-methoxy-pyridin-2-yl)-piperidin-4-yl]-urea ent-2
**Ex-268** trans-1-(4-Chlorophenyl)-3-[3-(5-methoxy-pyridin-2-yl)-1-(3-methyl-pyridazin-4-yl)-piperidin-4-yl]-urea
**Ex-269** trans-1-(4-Chlorophenyl)-3-[1-(6-hydroxy-pyridin-2-yl)-3-(5-methoxy-pyridin-2-yl)-piperidin-4-yl]-urea
**Ex-270** trans-1-(4-Chlorophenyl)-3-[1-(2-hydroxy-pyridin-4-yl)-3-(5-methoxy-pyridin-2-yl)-piperidin-4-yl]-urea
**Ex-271** trans-1-(4-Chlorophenyl)-3-[3-(5-methoxy-pyridin-2-yl)-1-(5-methyl-pyridazin-4-yl)-piperidin-4-yl]-urea
**Ex-272** trans-1-(4-Chlorophenyl)-3-[1-(2-hydroxy-pyridin-3-yl)-3-(5-methoxy-pyridin-2-yl)-piperidin-4-yl]-urea
**Ex-273** trans-1-(4-Chlorophenyl)-3-[1-(6-hydroxy-pyridin-3-yl)-3-(5-methoxy-pyridin-2-yl)-piperidin-4-yl]-urea
**Ex-274** trans-1-(4-Chlorophenyl)-3-[3-(5-methoxy-pyridin-2-yl)-1-(6-methyl-pyridazin-4-yl)-piperidin-4-yl]-urea
**Ex-275** trans-1-(4-Chlorophenyl)-3-[3-(5-methoxy-pyridin-2-yl)-1-(3-methyl-pyrazin-2-yl)-piperidin-4-yl]-urea
**Ex-276** trans-1-(4-Chlorophenyl)-3-[1-[(3,5-dimethyl-isoxazol-4-yl)-methyl]-3-(5-methoxy-pyridin-2-yl)-piperidin-4-yl]-urea
**Ex-277** trans-1-(4-Chlorophenyl)-3-[3-(5-methoxy-pyridin-2-yl)-1-[(5-methyl-[1,2,4]oxadiazol-3-yl)-methyl]-piperidin-4-yl]-urea
**Ex-278** trans-1-(4-Chlorophenyl)-3-[3-(5-methoxy-pyridin-2-yl)-1-[(1-methyl-1H-[1,2,4]triazol-3-yl)-methyl]-piperidin-4-yl]-urea
**Ex-279** trans-1-(4-Chlorophenyl)-3-[3-(5-methoxy-pyridin-2-yl)-1-[(5-methyl-1H-[1,2,4]triazol-3-yl)-methyl]-piperidin-4-yl]-urea
**Ex-280** trans-1-(4-Chlorophenyl)-3-[3-(5-methoxy-pyridin-2-yl)-1-[(3-methyl-[1,2,4]oxadiazol-5-yl)-methyl]-piperidin-4-yl]-urea
**Ex-281** trans-1-(4-Chlorophenyl)-3-[1-[[5-(hydroxymethyl)-isoxazol-3-yl]-methyl]-3-(5-methoxy-pyridin-2-yl)-piperidin-4-yl]-urea
**Ex-282** trans-1-(4-Chlorophenyl)-3-[1-[[1-(2-hydroxy-ethyl)-1H-pyrazol-3-yl]-methyl]-3-(5-methoxy-pyridin-2-yl)-piperidin-4-yl]-urea
**Ex-283** trans-1-(4-Chlorophenyl)-3-[3-(5-methoxy-pyridin-2-yl)-1-[(1-methylsulfonyl-1H-[1,2,4]triazol-3-yl)-methyl]-piperidin-4-yl]-urea
**Ex-284** trans-2-[4-[[(4-Chlorophenyl)-carbamoyl]amino]-3-(5-methoxy-pyridin-2-yl)-piperidin-1-yl]-N-ethyl-acetamide
**Ex-285** trans-2-[4-[[(4-Chlorophenyl)-carbamoyl]amino]-3-(5-methoxy-pyridin-2-yl)-piperidin-1-yl]-N-propyl-acetamide
**Ex-286** trans-2-[4-[[(4-Chlorophenyl)-carbamoyl]amino]-3-(5-methoxy-pyridin-2-yl)-piperidin-1-yl]-N-(2-fluoro-ethyl)-acetamide
**Ex-287** trans-2-[4-[[(4-Chlorophenyl)-carbamoyl]amino]-3-(5-methoxy-pyridin-2-yl)-piperidin-1-yl]-N-(2,2-difluoro-ethyl)-acetamide
**Ex-288** trans-2-[4-[[(4-Chlorophenyl)-carbamoyl]amino]-3-(5-methoxy-pyridin-2-yl)-piperidin-1-yl]-N-(2,2,2-trifluoro-ethyl)-acetamide
**Ex-289** trans-2-[4-[[(4-Chlorophenyl)-carbamoyl]amino]-3-(5-methoxy-pyridin-2-yl)-piperidin-1-yl]-N-isopropyl-acetamide
**Ex-290** trans-2-[4-[[(4-Chlorophenyl)-carbamoyl]amino]-3-(5-methoxy-pyridin-2-yl)-piperidin-1-yl]-N-cyclopropyl-acetamide
**Ex-291** trans-2-[4-[[(4-Chlorophenyl)-carbamoyl]amino]-3-(5-methoxy-pyridin-2-yl)-piperidin-1-yl]-N-cyclobutyl-acetamide
**Ex-292** trans-2-[4-[[(4-Chlorophenyl)-carbamoyl]amino]-3-(5-methoxy-pyridin-2-yl)-piperidin-1-yl]-N-(2-methyl-propyl)-acetamide
**Ex-293** trans-2-[4-[[(4-Chlorophenyl)-carbamoyl]amino]-3-(5-methoxy-pyridin-2-yl)-piperidin-1-yl]-N-(2-fluoro-2-methyl-propyl)-acetamide
**Ex-294** trans-2-[4-[[(4-Chlorophenyl)-carbamoyl]amino]-3-(5-methoxy-pyridin-2-yl)-piperidin-1-yl]-N-(cyclopropyl-methyl)-acetamide
**Ex-295** trans-2-[4-[[(4-Chlorophenyl)-carbamoyl]amino]-3-(5-methoxy-pyridin-2-yl)-piperidin-1-yl]-N-(cyclobutyl-methyl)-acetamide
**Ex-296** trans-2-[4-[[(4-Chlorophenyl)-carbamoyl]amino]-3-(5-methoxy-pyridin-2-yl)-piperidin-1-yl]-N-(2-hydroxy-ethyl)-acetamide
**Ex-297** trans-2-[4-[[(4-Chlorophenyl)-carbamoyl]amino]-3-(5-methoxy-pyridin-2-yl)-piperidin-1-yl]-N-(2-methoxy-ethyl)-acetamide
**Ex-298** trans-2-[4-[[(4-Chlorophenyl)-carbamoyl]amino]-3-(5-methoxy-pyridin-2-yl)-piperidin-1-yl]-N-(oxetan-3-yl)-acetamide
**Ex-299** trans-2-[4-[[(4-Chlorophenyl)-carbamoyl]amino]-3-(5-methoxy-pyridin-2-yl)-piperidin-1-yl]-N-tetrahydro-pyran-4-yl-acetamide
**Ex-300** trans-2-[4-[[(4-Chlorophenyl)-carbamoyl]amino]-3-(5-methoxy-pyridin-2-yl)-piperidin-1-yl]-N-isoxazol-3-yl-acetamide
**Ex-301** trans-2-[4-[[(4-Chlorophenyl)-carbamoyl]amino]-3-(5-methoxy-pyridin-2-yl)-piperidin-1-yl]-N-isoxazol-4-yl-acetamide
**Ex-302** trans-2-[4-[[(4-Chlorophenyl)-carbamoyl]amino]-3-(5-methoxy-pyridin-2-yl)-piperidin-1-yl]-N-isoxazol-5-yl-acetamide
**Ex-303** trans-2-[4-[[(4-Chlorophenyl)-carbamoyl]amino]-3-(5-methoxy-pyridin-2-yl)-piperidin-1-yl]-N-pyridin-2-yl-acetamide
**Ex-304** trans-2-[4-[[(4-Chlorophenyl)-carbamoyl]amino]-3-(5-methoxy-pyridin-2-yl)-piperidin-1-yl]-N-(1H-pyrazol-3-yl)-acetamide
**Ex-305** trans-2-[4-[[(4-Chlorophenyl)-carbamoyl]amino]-3-(5-methoxy-pyridin-2-yl)-piperidin-1-yl]-N-(1H-pyrazol-4-yl)-acetamide
**Ex-306** trans-2-[4-[[(4-Chlorophenyl)-carbamoyl]amino]-3-(5-methoxy-pyridin-2-yl)-piperidin-1-yl]-N-(1-methyl-1H-pyrazol-3-yl)-acetamide
Ex-307 trans-2-[4-[[(4-Chlorophenyl)-carbamoyl]amino]-3-(5-methoxy-pyridin-2-yl)-piperidin-1-yl]-N-(1-methyl-1H-pyrazol-4-yl)-acetamide
**Ex-308** trans-2-[4-[[(4-Chlorophenyl)-carbamoyl]amino]-3-(5-methoxy-pyridin-2-yl)-piperidin-1-yl]-N-(2-methyl-2H-pyrazol-3-yl)-acetamide
**Ex-309** trans-2-[4-[[(4-Chlorophenyl)-carbamoyl]amino]-3-(5-methoxy-pyridin-2-yl)-piperidin-1-yl]-N-oxazol-4-yl-acetamide
**Ex-310** trans-2-[4-[[(4-Chlorophenyl)-carbamoyl]amino]-3-(5-methoxy-pyridin-2-yl)-piperidin-1-yl]-N-oxazol-5-yl-acetamide
**Ex-311** trans-2-[4-[[(4-Chlorophenyl)-carbamoyl]amino]-3-(5-methoxy-pyridin-2-yl)-piperidin-1-yl]-N-oxazol-2-yl-acetamide
**Ex-312** trans-2-[4-[[(4-Chlorophenyl)-carbamoyl]amino]-3-(5-methoxy-pyridin-2-yl)-piperidin-1-yl]-N-thiazol-4-yl-acetamide
**Ex-313** trans-2-[4-[[(4-Chlorophenyl)-carbamoyl]amino]-3-(5-methoxy-pyridin-2-yl)-piperidin-1-yl]-N-thiazol-5-yl-acetamide
**Ex-314** trans-2-[4-[[(4-Chlorophenyl)-carbamoyl]amino]-3-(5-methoxy-pyridin-2-yl)-piperidin-1-yl]-N-thiazol-2-yl-acetamide
**Ex-315** trans-2-[4-[[(4-Chlorophenyl)-carbamoyl]amino]-3-(5-methoxy-pyridin-2-yl)-piperidin-1-yl]-N-(1H-pyrazol-3-yl-methyl)-acetamide
**Ex-316** trans-2-[4-[[(4-Chlorophenyl)-carbamoyl]amino]-3-(5-methoxy-pyridin-2-yl)-piperidin-1-yl]-N-(1H-pyrazol-4-yl-methyl)-acetamide
Ex-317 trans-2-[4-[[(4-Chlorophenyl)-carbamoyl]amino]-3-(5-methoxy-pyridin-2-yl)-piperidin-1-yl]-N-[(1-methyl-1H-pyrazol-3-yl)-methyl]-acetamide
**Ex-318** trans-2-[4-[[(4-Chlorophenyl)-carbamoyl]amino]-3-(5-methoxy-pyridin-2-yl)-piperidin-1-yl]-N-[(1-methyl-1H-pyrazol-4-yl)-methyl]-acetamide
**Ex-319** trans-2-[4-[[(4-Chlorophenyl)-carbamoyl]amino]-3-(5-methoxy-pyridin-2-yl)-piperidin-1-yl]-N-[(2-methyl-2H-pyrazol-3-yl)-methyl]-acetamide
**Ex-320** trans-2-[4-[[(4-Chlorophenyl)-carbamoyl]amino]-3-(5-methoxy-pyridin-2-yl)-piperidin-1-yl]-N-(1H-[1,2,3]triazol-4-yl)-acetamide
**Ex-321** trans-2-[4-[[(4-Chlorophenyl)-carbamoyl]amino]-3-(5-methoxy-pyridin-2-yl)-piperidin-1-yl]-N-(1-methyl-1H-[1,2,3]triazol-4-yl)-acetamide
**Ex-322** trans-2-[4-[[(4-Chlorophenyl)-carbamoyl]amino]-3-(5-methoxy-pyridin-2-yl)-piperidin-1-yl]-N-(3-methyl-3H-[1,2,3]triazol-4-yl)-acetamide
**Ex-323** trans-2-[4-[[(4-Chlorophenyl)-carbamoyl]amino]-3-(5-methoxy-pyridin-2-yl)-piperidin-1-yl]-N-(2H-[1,2,4]triazol-3-yl)-acetamide
**Ex-324** trans-2-[4-[[(4-Chlorophenyl)-carbamoyl]amino]-3-(5-methoxy-pyridin-2-yl)-piperidin-1-yl]-N-(4-methyl-4H-[1,2,4]triazol-3-yl)-acetamide
**Ex-325** trans-2-[4-[[(4-Chlorophenyl)-carbamoyl]amino]-3-(5-methoxy-pyridin-2-yl)-piperidin-1-yl]-N-(2-methyl-2H-[1,2,4]triazol-3-yl)-acetamide
**Ex-326** trans-2-[4-[[(4-Chlorophenyl)-carbamoyl]amino]-3-(5-methoxy-pyridin-2-yl)-piperidin-1-yl]-N-(1-methyl-1H-[1,2,4]triazol-3-yl)-acetamide
Ex-327 trans-2-[4-[[(4-Chlorophenyl)-carbamoyl]amino]-3-(5-methoxy-pyridin-2-yl)-piperidin-1-yl]-N-([1,2,4]oxadiazol-3-yl)-acetamide
**Ex-328** trans-2-[4-[[(4-Chlorophenyl)-carbamoyl]amino]-3-(5-methoxy-pyridin-2-yl)-piperidin-1-yl]-N-([1,2,4]oxadiazol-5-yl)-acetamide
**Ex-329** trans-2-[4-[[(4-Chlorophenyl)-carbamoyl]amino]-3-(5-methoxy-pyridin-2-yl)-piperidin-1-yl]-N-([1,3,4]oxadiazol-2-yl)-acetamide
**Ex-330** trans-1-(4-Chlorophenyl)-3-[1-[4-[1-(methanesulfonamido)-cyclopropyl]-phenyl]-3-(5-methoxy-pyridin-2-yl)-piperidin-4-yl]-urea
**Ex-331** trans-1-(4-Chlorophenyl)-3-[1-[4-[1-(methanesulfonamido)-1-methyl-ethyl]-phenyl]-3-(5-methoxy-pyridin-2-yl)-piperidin-4-yl]-urea
**Ex-332** trans-1-(4-Chlorophenyl)-3-[1-[4-(methanesulfonamido-methyl)-phenyl]-3-(5-methoxy-pyridin-2-yl)-piperidin-4-yl]-urea
**Ex-333** trans-1-(4-Chlorophenyl)-3-[1-[5-[1-(methanesulfonamido)-cyclopropyl]-pyridin-2-yl]-3-(5-methoxy-pyridin-2-yl)-piperidin-4-yl]-urea
**Ex-334** trans-1-(4-Chlorophenyl)-3-[1-[5-[1-(methanesulfonamido)-1-methyl-ethyl]-pyridin-2-yl]-3-(5-methoxy-pyridin-2-yl)-piperidin-4-yl]-urea
**Ex-335** trans-1-(4-Chlorophenyl)-3-[1-[5-(methanesulfonamido-methyl)-pyridin-2-yl]-3-(5-methoxy-pyridin-2-yl)-piperidin-4-yl]-urea
**Ex-336** trans-1-(4-Chlorophenyl)-3-[1-[6-[1-(methanesulfonamido)-cyclopropyl]-pyridin-3-yl]-3-(5-methoxy-pyridin-2-yl)-piperidin-4-yl]-urea
Ex-337 trans-1-(4-Chlorophenyl)-3-[1-[6-[1-(methanesulfonamido)-1-methyl-ethyl]-pyridin-3-yl]-3-(5-methoxy-pyridin-2-yl)-piperidin-4-yl]-urea
**Ex-338** trans-1-(4-Chlorophenyl)-3-[1-[6-(methanesulfonamido-methyl)-pyridin-3-yl]-3-(5-methoxy-pyridin-2-yl)-piperidin-4-yl]-urea
**Ex-339** trans-N-[1-[4-[4-[[(4-Chlorophenyl)-carbamoyl]amino]-3-(5-methoxy-pyridin-2-yl)-piperidin-1-yl]-phenyl]-cyclopropyl]-acetamide
**Ex-340** trans-N-[1-[4-[4-[[(4-Chlorophenyl)-carbamoyl]amino]-3-(5-methoxy-pyridin-2-yl)-piperidin-1-yl]-phenyl]-1-methyl-ethyl]-acetamide
**Ex-341** trans-N-[[4-[4-[[(4-Chlorophenyl)-carbamoyl]amino]-3-(5-methoxy-pyridin-2-yl)-piperidin-1-yl]-phenyl]-methyl]-acetamide
**Ex-342** trans-N-[1-[6-[4-[[(4-Chlorophenyl)-carbamoyl]amino]-3-(5-methoxy-pyridin-2-yl)-piperidin-1-yl]-pyridin-3-yl]-cyclopropyl]-acetamide
**Ex-343** trans-N-[1-[6-[4-[[(4-Chlorophenyl)-carbamoyl]amino]-3-(5-methoxy-pyridin-2-yl)-piperidin-1-yl]-pyridin-3-yl]-1-methyl-ethyl]-acetamide
**Ex-344** trans-N-[[6-[4-[[(4-Chlorophenyl)-carbamoyl]amino]-3-(5-methoxy-pyridin-2-yl)-piperidin-1-yl]-pyridin-3-yl]-methyl]-acetamide
**Ex-345** trans-N-[1-[5-[4-[[(4-Chlorophenyl)-carbamoyl]amino]-3-(5-methoxy-pyridin-2-yl)-piperidin-1-yl]-pyridin-2-yl]-cyclopropyl]-acetamide
**Ex-346** trans-N-[1-[5-[4-[[(4-Chlorophenyl)-carbamoyl]amino]-3-(5-methoxy-pyridin-2-yl)-piperidin-1-yl]-pyridin-2-yl]-1-methyl-ethyl]-acetamide
Ex-347 trans-N-[[5-[4-[[(4-Chlorophenyl)-carbamoyl]amino]-3-(5-methoxy-pyridin-2-yl)-piperidin-1-yl]-pyridin-2-yl]-methyl]-acetamide
**Ex-348** trans-1-(4-Chlorophenyl)-3-[3-(5-methoxy-pyridin-2-yl)-1-(2H-pyrazol-3-yl)-piperidin-4-yl]-urea
**Ex-349** trans-1-(4-Chlorophenyl)-3-[3-(5-methoxy-pyridin-2-yl)-1-(1H-pyrazol-4-yl)-piperidin-4-yl]-urea
Ex-350 trans-1-(4-Chlorophenyl)-3-[3-(5-methoxy-pyridin-2-yl)-1-(2-methyl-2H-pyrazol-3-yl)-piperidin-4-yl]-urea
Ex-351 trans-1-(4-Chlorophenyl)-3-[3-(5-methoxy-pyridin-2-yl)-1-(1-methyl-1H-pyrazol-4-yl)-piperidin-4-yl]-urea
Ex-352 trans-1-(4-Chlorophenyl)-3-[3-(5-methoxy-pyridin-2-yl)-1-(1-methyl-1H-pyrazol-3-yl)-piperidin-4-yl]-urea
Ex-353 trans-1-(4-Chlorophenyl)-3-[3-(5-methoxy-pyridin-2-yl)-1-(2-methylsulfonyl-2H-pyrazol-3-yl)-piperidin-4-yl]-urea
Ex-354 trans-1-(4-Chlorophenyl)-3-[3-(5-methoxy-pyridin-2-yl)-1-(1-methylsulfonyl-1H-pyrazol-4-yl)-piperidin-4-yl]-urea
**Ex-355** trans-1-(4-Chlorophenyl)-3-[3-(5-methoxy-pyridin-2-yl)-1-(1-methylsulfonyl-1H-pyrazol-3-yl)-piperidin-4-yl]-urea
Ex-356 trans-1-(4-Chlorophenyl)-3-[3-(5-methoxy-pyridin-2-yl)-1-(1H-[1,2,3]triazol-4-yl)-piperidin-4-yl]-urea
Ex-357 trans-1-(4-Chlorophenyl)-3-[3-(5-methoxy-pyridin-2-yl)-1-(1-methyl-1H-[1,2,3]triazol-4-yl)-piperidin-4-yl]-urea
Ex-358 trans-1-(4-Chlorophenyl)-3-[3-(5-methoxy-pyridin-2-yl)-1-(3-methyl-3H-[1,2,3]triazol-4-yl)-piperidin-4-yl]-urea
Ex-359 trans-1-(4-Chlorophenyl)-3-[3-(5-methoxy-pyridin-2-yl)-1-(1-methylsulfonyl-1H-[1,2,3]triazol-4-yl)-piperidin-4-yl]-urea
Ex-360 trans-1-(4-Chlorophenyl)-3-[1-isoxazol-5-yl-3-(5-methoxy-pyridin-2-yl)-piperidin-4-yl]-urea
Ex-361 trans-1-(4-Chlorophenyl)-3-[1-isoxazol-4-yl-3-(5-methoxy-pyridin-2-yl)-piperidin-4-yl]-urea
Ex-362 trans-1-(4-Chlorophenyl)-3-[1-isoxazol-3-yl-3-(5-methoxy-pyridin-2-yl)-piperidin-4-yl]-urea
Ex-363 trans-1-(4-Chlorophenyl)-3-[3-(5-methoxy-pyridin-2-yl)-1-oxazol-2-yl-piperidin-4-yl]-urea
Ex-364 trans-1-(4-Chlorophenyl)-3-[3-(5-methoxy-pyridin-2-yl)-1-oxazol-4-yl-piperidin-4-yl]-urea
**Ex-365** trans-1-(4-Chlorophenyl)-3-[3-(5-methoxy-pyridin-2-yl)-1-oxazol-5-yl-piperidin-4-yl]-urea
Ex-366 trans-1-(4-Chlorophenyl)-3-[3-(5-methoxy-pyridin-2-yl)-1-thiazol-2-yl-piperidin-4-yl]-urea
Ex-367 trans-1-(4-Chlorophenyl)-3-[3-(5-methoxy-pyridin-2-yl)-1-thiazol-4-yl-piperidin-4-yl]-urea
Ex-368 trans-1-(4-Chlorophenyl)-3-[3-(5-methoxy-pyridin-2-yl)-1-thiazol-5-yl-piperidin-4-yl]-urea
Ex-369 trans-1-(4-Chlorophenyl)-3-[3-(5-methoxy-pyridin-2-yl)-1-(2H-[1,2,4]triazol-3-yl)-piperidin-4-yl]-urea
Ex-370 trans-1-(4-Chlorophenyl)-3-[3-(5-methoxy-pyridin-2-yl)-1-(1-methyl-1H-[1,2,4]triazol-3-yl)-piperidin-4-yl]-urea
Ex-371 trans-1-(4-Chlorophenyl)-3-[3-(5-methoxy-pyridin-2-yl)-1-(1-methylsulfonyl-1H-[1,2,4]triazol-3-yl)-piperidin-4-yl]-urea
Ex-372 trans-1-(4-Chlorophenyl)-3-[3-(5-methoxy-pyridin-2-yl)-1-([1,2,4]oxadiazol-5-yl)-piperidin-4-yl]-urea
Ex-373 trans-1-(4-Chlorophenyl)-3-[3-(5-methoxy-pyridin-2-yl)-1-([1,2,4]oxadiazol-3-yl)-piperidin-4-yl]-urea
Ex-374 trans-1-(4-Chlorophenyl)-3-[3-(5-methoxy-pyridin-2-yl)-1-([1,3,4]oxadiazol-2-yl)-piperidin-4-yl]-urea
**Ex-375** trans-1-(4-Chlorophenyl)-3-[1-(1H-indazol-4-yl)-3-(5-methoxy-pyridin-2-yl)-piperidin-4-yl]-urea
Ex-376 trans-1-(4-Chlorophenyl)-3-[1-(1H-indazol-5-yl)-3-(5-methoxy-pyridin-2-yl)-piperidin-4-yl]-urea
**Ex-377** trans-1-(4-Chlorophenyl)-3-[1-(1H-indazol-7-yl)-3-(5-methoxy-pyridin-2-yl)-piperidin-4-yl]-urea
**Ex-378** trans-1-(4-Chlorophenyl)-3-[1-(1H-indazol-6-yl)-3-(5-methoxy-pyridin-2-yl)-piperidin-4-yl]-urea
**Ex-379** trans-1-(4-Chlorophenyl)-3-[3-(5-methoxy-pyridin-2-yl)-1-(1H-pyrazolo[3,4-b]pyridin-4-yl)-piperidin-4-yl]-urea
**Ex-380** trans-1-(4-Chlorophenyl)-3-[3-(5-methoxy-pyridin-2-yl)-1-(1H-pyrazolo[4,3-b]pyridin-7-yl)-piperidin-4-yl]-urea
**Ex-381** trans-1-(4-Chlorophenyl)-3-[3-(5-methoxy-pyridin-2-yl)-1-(1H-pyrazolo[3,4-b]pyridin-5-yl)-piperidin-4-yl]-urea
**Ex-382** trans-1-(4-Chlorophenyl)-3-[3-(5-methoxy-pyridin-2-yl)-1-(1H-pyrazolo[4,3 -b]pyridin-6-yl)-piperidin-4-yl]-urea
**Ex-383** trans-1-(4-Chlorophenyl)-3-[3-(5-methoxy-pyridin-2-yl)-1-(3-oxo-1,2-dihydro-isoindol-4-yl)-piperidin-4-yl]-urea
**Ex-384** trans-1-(4-Chlorophenyl)-3-[3-(5-methoxy-pyridin-2-yl)-1-(1-oxo-2,3 -dihydro-isoindol-4-yl)-piperidin-4-yl]-urea
**Ex-385** trans-1-(4-Chlorophenyl)-3-[3-(5-methoxy-pyridin-2-yl)-1-(3-oxo-1,2-dihydro-isoindol-5-yl)-piperidin-4-yl]-urea
**Ex-386** trans-1-(4-Chlorophenyl)-3-[3-(5-methoxy-pyridin-2-yl)-1-(1-oxo-2,3-dihydro-isoindol-5-yl)-piperidin-4-yl]-urea
**Ex-387** trans-1-(4-Chlorophenyl)-3-[3-(5-methoxy-pyridin-2-yl)-1-(2-oxo-1,3-dihydro-indol-7-yl)-piperidin-4-yl]-urea
**Ex-388** trans-1-(4-Chlorophenyl)-3-[3-(5-methoxy-pyridin-2-yl)-1-(2-oxo-1,3-dihydro-indol-4-yl)-piperidin-4-yl]-urea
**Ex-389** trans-1-(4-Chlorophenyl)-3-[3-(5-methoxy-pyridin-2-yl)-1-(2-oxo-1,3-dihydro-indol-6-yl)-piperidin-4-yl]-urea
**Ex-390** trans-1-(4-Chlorophenyl)-3-[3-(5-methoxy-pyridin-2-yl)-1-(2-oxo-1,3-dihydro-indol-5-yl)-piperidin-4-yl]-urea
in the form of the free compound or a physiologically acceptable salt thereof.

In a preferred embodiment, the compound according to the present invention is a modulator of FPR2, more preferably an agonist of FPR2. In the sense of the present invention, the term "modulator of FPR2 (FPR2 modulator)" preferably means that the respective compound exhibits in a target engagement assay an EC50 value on FPR2 of at most 10 µM (10·10⁻⁶ mol/L); more preferably at most 1 µM; still more preferably at most 500 nM (10⁻⁹ mol/L); yet more preferably at most 300 nM; even more preferably at most 100 nM; most preferably at most 10 nM; and in particular at most 1 nM.

A preferred target engagement assay for testing compounds for their potency (EC50) on human FPR2 is described herein below:
Cells (hFPR1-Gα15-CHO or hFPR2-Aq-CHO) are suspended in 10 mL of respective complete medium (F12(1X)HAM media; 10% HI-FBS; 0.4 mg/mL Geneticin and 0.25 mg/ml Zeocin) and viability is checked using Trypan Blue exclusion. After washing, the cells are plated at 10,000 cells per well in 40 µL complete medium in a 384-well sterile clear bottom black plate and incubated in a 5% CO₂ incubator at 37°C for 18 hours. Plating media is removed from each well by decanting and gentle tapping before 30 µL of 0.5X Calcium 5 dye solution (0.5X FLIPR Calcium 5 dye (Molecular devices, R8186)); HBSS; 20mM HEPES; 2.5mM Probenecid; 0.025% Pluronic F-127; pH adjusted to 7.4) is added to each well and the plate is then incubated at 37°C for 30 minutes. For the assay the plate is equilibrated at room temperature for 10 minutes before placing it in the FLIPR. Compounds are dissolved in DMSO and serially diluted over an 11 point half log (3.16 fold) dilution (2 mM to 20 nM). Compounds are then diluted 1:50 in assay buffer (HBSS; 20mM HEPES; 2.5mM Probenecid; 0.05% gelatin; 0.1% BSA; pH adjusted to 7.4) just before performing the assay. Compounds are finally added to the respective wells of the cell plate (final assay concentration 10 µM to 100 pM) using the FLIPR (e.g. FLIPR-Tetra, Molecular Devices) and fluorescence readings are captured for 5 minutes. The increase in fluorescence from the basal reading in the presence of the compounds is compared with that of the control wells (wells having no compound) to calculate the activity of the compounds. The EC₅₀ values of the compounds can be determined using e.g. Graph pad Prism software.

In a preferred embodiment, the compound according to the present invention exhibits in a target engagement assay an EC50 value on FPR2 of at most 1 µM (10⁻⁶ mol/L); still more preferably at most 500 nM (10⁻⁹ mol/L); yet more preferably at most 300 nM; even more preferably at most 100 nM; most preferably at most 10 nM; and in particular at most 1 nM or at most 100 pM (10⁻¹² mol/L) or at most 10 pM.

In a preferred embodiment, the compound according to the present invention exhibits in a target engagement assay an EC50 value on FPR2 in the range of from 0.1 nM (10⁻⁹ mol/L) to 1000 nM; still more preferably 0.1 nM to 800 nM; yet more preferably 0.1 nM to 500 nM; even more preferably 0.1 nM to 300 nM; most preferably 0.1 nM to 100 nM; and in particular 0.1 nM to 10 nM. In another preferred embodiment, the compound according to the present invention exhibits in a target engagement assay an EC50 value on FPR2 in the range of from 1 pM (10⁻¹² mol/L) to 1000 nM; still more preferably 1 pM to 800 nM; yet more preferably 1 pM to 500 nM; even more preferably 1 pM to 300 nM; most preferably 1 pM to 100 nM; and in particular 1 pM to 10 nM.

In a preferred embodiment, the compound according to the present invention does not activate FPR1.

Preferably, the compounds according to the present invention are useful as non-peptides modulators of the human FPR2 receptor. More preferably, the compounds according to the present invention are agonists of the human FPR2 receptor.

Therefore, the compounds according to the present invention are preferably useful for the in vivo treatment or prevention of diseases in which participation of FPR2 is implicated.

The present invention therefore further relates to a compound according to the present invention for use in the modulation of FPR2 activity.

Therefore, another aspect of the present invention relates to a compound according to the present invention for use in the treatment and/or prophylaxis of a disorder which is mediated at least in part by FPR2. Still another aspect of the present invention relates to a method of treatment of a disorder which is mediated at least in part by FPR2; comprising the administration of a therapeutically effective amount of a compound according to the present invention to a subject in need thereof, preferably a human.

A further aspect of the invention relates to a compound according to the present invention as medicament.

Another aspect of the present invention relates to a pharmaceutical dosage form comprising a compound according to the present invention. Preferably, the pharmaceutical dosage form comprises a compound according to the present invention and one or more pharmaceutical excipients such as physiologically acceptable carriers, additives and/or auxiliary substances; and optionally one or more further pharmacologically active ingredient. Examples of suitable physiologically acceptable carriers, additives and/or auxiliary substances are fillers, solvents, diluents, colorings and/or binders. These substances are known to the person skilled in the art (see H. P. Fiedler, Lexikon der Hilfsstoffe fur Pharmazie, Kosmetik und angrenzende Gebiete, Editio Cantor Aulendoff).

The pharmaceutical dosage form according to the present invention is preferably for systemic, topical or local administration, preferably for oral administration. Therefore, the pharmaceutical dosage form can be in form of a liquid, semisolid or solid, e.g. in the form of injection solutions, drops, juices, syrups, sprays, suspensions, tablets, patches, films, capsules, plasters, suppositories, ointments, creams, lotions, gels, emulsions, aerosols or in multiparticulate form, for example in the form of pellets or granules, if appropriate pressed into tablets, decanted in capsules or suspended in a liquid, and can also be administered as such.

The pharmaceutical dosage form according to the present invention is preferably prepared with the aid of conventional means, devices, methods and processes known in the art. The amount of the compound according to the present invention to be administered to the patient may vary and is e.g. dependent on the patient's weight or age and also on the type of administration, the indication and the severity of the disorder. Preferably 0.001 to 100 mg/kg, more preferably 0.05 to 75 mg/kg, most preferably 0.05 to 50 mg of a compound according to the present invention are administered per kg of the patient's body weight.

FPR2 is believed to have potential to modify a variety of diseases or disorders in mammals such as humans. These include inflammatory diseases, diabetes, obstructive airway diseases, autoimmune diseases, allergic conditions, rheumatological disorders, HIV-mediated retroviral 5 infections, infectious diseases, sepsis, cardiovascular disorders, fibrotic disorders, neuroinflammation, neurological disorders, pain, prion-mediated diseases, amyloid-mediated disorders, chronic obstructive pulmonary disease (COPD), asthma, idiopathic pulmonary fibrosis (IPF), inflammatory bowel disease (IBD), ulcerative colitis (UC), rheumatoid arthritis (RA), psoriatic arthritis (PsA), multiple sclerosis (MS). Further, FPR2 is believed to be involved in the modulation of immune responses, such as those elicited through Graft versus Host Disease (GvHD).

Therefore, another aspect of the present invention relates to a compound according to the present invention for use in the treatment and/or prophylaxis of a disorder selected from the group consisting of inflammatory diseases, diabetes, obstructive airway diseases, autoimmune diseases, allergic conditions, rheumatological disorders, HIV-mediated retroviral 5 infections, infectious diseases, sepsis, cardiovascular disorders, fibrotic disorders, neuroinflammation, neurological disorders, pain, prion-mediated diseases, amyloid-mediated disorders and Graft versus Host Disease (GvHD).

Still another aspect of the present invention relates to a compound according to the present invention for use in the treatment and/or prophylaxis of a disorder selected from the group consisting of chronic obstructive pulmonary disease (COPD), asthma, idiopathic pulmonary fibrosis (IPF), inflammatory bowel disease (IBD), ulcerative colitis (UC), rheumatoid arthritis (RA), psoriatic arthritis (PsA) and multiple sclerosis (MS).

A further aspect of the present invention relates to a method of treatment of a disorder selected from the group consisting of inflammatory diseases, diabetes, obstructive airway diseases, autoimmune diseases, allergic conditions, rheumatological disorders, HIV-mediated retroviral 5 infections, infectious diseases, sepsis, cardiovascular disorders, fibrotic disorders, neuroinflammation, neurological disorders, pain, prion-mediated diseases and amyloid-mediated disorders.

### EXAMPLES

### Abbreviations:

µL = microliter; ACN = acetonitrile; AcOH = acetic acid; anhyd. = anhydrous; BH₃-DMS = borane dimethylsulfide complex; Boc = tert.-butyloxy carbonyl; conc. = concentrated; Cu-TMEDA = di-µ-hydroxo-bis[(*N*,*N*,*N*'*.N*'-tetramethylethylenediamine)copper(II)] chloride; Da = Dalton; DCE = dichloro ethane; DCM = dichloro methane; DIBAL = diisobutyl aluminium hydride; DIPA = diisopropylamine; DIPEA = diisopropyl ethylamine; DMAP = 4-dimethylamino pyridine; DMF = dimethyl formamide; DMSO = dimethylsulfoxide; DMSO-d6 = hexadeutero dimethylsulfoxide; DPPA = diphenyl phosphorylazide; dppf = 1,1'-bis(diphenylphosphino)ferrocene; EDCI-HCl = 1-ethyl-3-(3-dimethylaminopropyl)carbodiimid hydrochloride; ent-1 = enantiomer 1; ent-2 = enantiomer 2; eq = equivalents; EtOAc/EA = ethyl acetate; EtOH = ethanol; HOBt = 1-Hydroxybenzotriazol; HPLC = high pressure liquid chromatography; IBCF = isobutyl chloroformiate; KOAc = potassium acetate; LC-MS = liquid chromatography - mass spectrometry; MeOH = methanol; MsCl =mesyl chloride; N = normal; NaOAc = sodium acetate; NaOEt = sodium ethoxide; nBuLi = n-butyl lithium; n-BuOH = n-butanol; NEt₃/Et₃N/TEA = triethylamine; PhMe = toluene; PPh₃ = triphenyl phosphine; prep-HPLC = preparative high pressure liquid chromatography; prep-TLC = preparative thin layer chromatography; quant. = quantitative; R_{f} = retention factor; Rₜ = retention time; RT/rt = room temperature; sat. = saturated; SFC = supercritical fluid chromatography; TBAF = tetrabutylammonium fluoride; tBuONO = tert.-butylnitrite; Tf₂O = trifluoromethanesulfonic acid anhydride; TFA = trifluoroacetic acid; THF = tetrahydrofuran; TLC = thin layer chromatography; TMSCN = trimethylsilyl cyanide; TsCl = tosyl chloride; Xantphos = 4,5-bis(diphenylphosphino)-9,9-dimethylxanthene.

### LC-MS method 1

Instrument Name: LCMS/MS API 2000; Instrument manufacturer: Applied Biosystem; HPLC: Shimadzu Prominence; Column (Name, Size, type): Zorbax Extend (C18 4.6 X 50 mm, 5 micron); Eluent (solvent): A channel: 10 mM Ammonium Acetate in water; B channel: Acetonitrile (Organic phase); Dual Wavelength: At 220 and 260 nm; Detector: UV; Gradient condition: A: Buffer 10 mM Ammonium Acetate in water; B: Acetonitrile; Flow rate: 1.2 ml/min; Column Temperature: 25 °C; Injection Volume: 2 µL; LC-MS gradient: mobile phase: from 90% [buffer] and 10% [CH₃CN] to 70% [buffer] and 30% [CH₃CN] in 1.5 min, further to 10% [buffer] and 90% [CH₃CN] in 3.0 min, held this mobile phase composition to 4 min and finally back to initial condition in 5 min). Mass conditions: Ionization technique: ESI (Electron Spray Ionization) using API (Atmospheric pressure Ionization) source; Declustering Potential: 10-70 V depending on the ionization of compound; Mass range: 100-800 amu; Scan type: Q1; Polarity: + Ve; Ion Source: Turbo spray; Ion spray voltage: +5500 for +ve mode; Mass Source temperature: 200 °C.

### LC-MS method 2

Instrument Name: LCMS/MS API 2000; Instrument manufacturer: Applied Biosystem; HPLC: Shimadzu Prominence; Column (Name, Size, type): Xbridge (C18 4.6 X 50 mm, 5 micron); Eluent (solvent): A channel: 10 mM Ammonium Acetate in water; B channel: Acetonitrile (Organic phase); Dual Wavelength: At 220 and 260 nm; Detector: UV; Gradient condition: A: Buffer 10 mM Ammonium Acetate in water; B: Acetonitrile; Flow rate: 1.2 ml/min; Column Temperature: 25 °C; Injection Volume: 2 µL; LC-MS gradient: mobile phase: from 90% [buffer] and 10% [CH₃CN] to 70% [buffer] and 30% [CH₃CN] in 1.5 min, further to 10% [buffer] and 90% [CH₃CN] in 3.0 min, held this mobile phase composition to 4 min and finally back to initial condition in 5 min). Mass conditions: Ionization technique: ESI (Electron Spray Ionization) using API (Atmospheric pressure Ionization) source; Declustering Potential: 10-70 V depending on the ionization of compound; Mass range: 100-800 amu; Scan type: Q1; Polarity: + Ve; Ion Source: Turbo spray; Ion spray voltage: +5500 for +ve mode; Mass Source temperature: 200 °C.

### LC-MS method 3

Column: Resteck/YMC; Column length: 30 mm; Internal diameter of column: 2.1 mm; Particle Size: 1.8 micron; Gradient condition: Flow rate: 1.5 ml/min; Column Temperature: 50 °C; Injection Volume: 2 µL; Waters ACQUITY UPLC with the following HPLC gradient conditions; (Solvent A: 0.05% Formic acid in water and Solvent B: Acetonitrile):

| TIME | MODULE | % A (Buffer) | % B (CH₃CN) |
|---|---|---|---|
| 0.00 | Pumps | 98 | 2 |
| 0.75 | Pumps | 98 | 2 |
| 1.00 | Pumps | 90 | 10 |
| 2.00 | Pumps | 2 | 98 |
| 2.25 | Pumps | 2 | 98 |
| 2.90 | Pumps | 98 | 2 |
| 3.00 | Pumps | 98 | 2 |

Mass conditions: ACQUITY SQD Mass Spectrometer from Waters (Single quadruple mass spectrometer); Ionisation method: Electro spray; Polarity: positive ions; Capillary (kV) 3.00, Cone (V) 40.00, Extractor (V) 3.00, Source Temperature (°C) 150, Desolvation Temperature (°C) 400, Cone Gas Flow (L/h) 50, Desolvation Gas Flow (L/h) 750; Mass range: 100 to 800 Da; DAD Wavelength range (nm): 210 to 400.

### LC-MS method 4

Column: Xtimate C18; Column length: 30 mm; Internal diameter of column: 2.1 mm; Particle Size: 3 micron; Temperature: 50 °C. Gradient condition: Method Waters ACQUITY UPLC with the following HPLC gradient conditions (Solvent A: 0.05% Formic acid in water and Solvent B: Acetonitrile,) Flow rate: 1.0 ml/min (mobile phase: 98% [0.05% HCOOH in water] and 2% [CH₃CN] held for 0.75 min, then to 90% [0.05% HCOOH in water] and 10% [CH₃CN] in 1.0 min, further to 2% [0.05% HCOOH in water] and 98% [CH₃CN] in 2.0 min, held this mobile phase composition up to 2.25 min and finally back to initial condition in 3.0 min).
Mass conditions: ACQUITY SQD Mass Spectrometer from Waters (Single quadruple mass spectrometer); Ionisation method: Electro spray; Polarity: positive ions; Capillary (kV) 3.00, Cone (V) 40.00, Extractor (V) 3.00, Source Temperature (°C) 150, Desolvation Temperature (°C) 400, Cone Gas Flow (L/h) 50, Desolvation Gas Flow (L/h) 750; Mass range: 100 to 800 Da; DAD Wavelength range (nm): 210 to 400.

### LC-MS method 5

Column: LUNA Omega Polar C18; Column length: 100 mm; Internal diameter of column: 4.6 mm; Particle Size: 3 micron; Gradient condition: Flow rate: 1.00 ml/min; Column Temperature: 50 °C; Injection Volume: 0.80 µL; Waters ACQUITY UPLC with the following HPLC gradient conditions; (Solvent A: 0.05% Formic acid in water and Solvent B: Acetonitrile); (mobile phase: 98% [0.05% HCOOH in water] and 2% [CH₃CN] held for 1.00 min, then to 50% [0.05% HCOOH in water] and 50% [CH₃CN] in 5.00 min, further to 10% [0.05% HCOOH in water] and 90% [CH₃CN] in 8.0 min, held this mobile phase composition up to 10.0 min and finally back to initial condition in 12.0 min).
Mass conditions: ACQUITY SQD Mass Spectrometer/Micromass ZQ Mass Spectrometer from Waters (Single quadruple mass spectrometer); Ionisation method: Electro spray; Polarity: positive ions; Capillary (kV) 3.50, Cone (V) 40.00, Extractor (V) 3.00, Source Temperature (°C) 150, Desolvation Temperature (°C) 400, Cone Gas Flow (L/h) 50, Desolvation Gas Flow (L/h) 750; Mass range: 130 to 1000 Da; DAD Wavelength range (nm): 210 to 400.

### LC-MS method 6

Column: Ultisil TM Polar RP; Column length: 30 mm; Internal diameter of column: 2.1 mm; Particle Size: 3 micron; Gradient condition: Flow rate: 1.0 ml/min; Column Temperature: 50 °C; Injection Volume: 2 µL; Waters ACQUITY UPLC with the following HPLC gradient conditions; (Solvent A: 5mM NH₄OAc in water and Solvent B: 5mM NH₄OAc in ACN/water 90:10); (mobile phase: 98% [5mM NH₄OAc in water] and 2% [5mM NH₄OAc in ACN/water 90:10] held for 0.75 min, then to 90% [5mM NH₄OAc in water] and 10% [5mM NH4OAc in ACN/water 90:10] in 1.0 min, further to 2% [5mM NH₄OAc in water] and 98% [5mM NH₄OAc in ACN/water 90:10] in 2.0 min, held this mobile phase composition up to 2.25 min and finally back to initial condition in 3.0 min and held the initial composition up to 3.10 min)
Mass conditions: ACQUITY SQD Mass Spectrometer from Waters (Single quadrupole mass spectrometer); Ionization method: Electro spray; Polarity: positive ions; Capillary (kV) 3.50, Cone (V) 40.00, Extractor (V) 3.00, Source Temperature (°C) 130, Desolvation Temperature (°C) 400, Cone Gas Flow (L/h) 80, Desolvation Gas Flow (L/h) 750; Mass range: 100 to 800 Da; DAD Wavelength range (nm): 210 to 400.

### LC-MS method 7

Column: YMC Triart C18; Column length: 33 mm; Internal diameter of column: 2.1 mm; Particle Size: 3 micron; Gradient condition: Flow rate: 1.2 ml/min; Column Temperature: 50 °C; Injection Volume: 0.80 µL; (Solvent A: 98% [10 mM NH₄OAc in water and Solvent B: Acetonitrile,); ((mobile phase: 98% [10 mM NH₄OAc in water] and 2% [CH₃CN] held for 0.50min then to 70% [10 mM NH₄OAc in water] and 30% [CH₃CN] in 1.0 min, further to 2% [10 mM NH₄OAc in water] and 98% [CH₃CN] in 2.0 min, held this mobile phase composition up to 2.25min, and finally back to initial condition in 3.0 min).
Mass conditions: SQD Mass Spectrometer from Agilent (Single quadrupole mass spectrometer); Ionization method: Electro spray; Polarity: positive ions; Capillary (kV) 4.00, Fragmentor (V) 150.00 , Threshold- 200, Drying gas Temperature (°C) 350, Drying Gas Flow (L/min) 12, Nebulizer pressure (bar) 3.4; Mass range: 100 to 800 Da.

### LC-MS method 8

Column: Gemini NX C18; Column length: 50 mm; Internal diameter of column: 4.6 mm; Particle Size: 5 micron; Gradient condition: Flow rate: 1.00 ml/min; Column Temperature: 50 °C; Injection Volume: 2 µL; Waters ACQUITY UPLC with the following HPLC gradient conditions; (Solvent A: 0.05% Formic acid in water and Solvent B: Acetonitrile)

| TIME | MODULE | % A (Buffer) | % B (CH₃CN) |
|---|---|---|---|
| 0.00 | Pumps | 98 | 2 |
| 1.00 | Pumps | 98 | 2 |
| 8.00 | Pumps | 60 | 40 |
| 9.00 | Pumps | 10 | 90 |
| 10.00 | Pumps | 10 | 90 |
| 12.00 | Pumps | 98 | 2 |

Mass conditions: ACQUITY SQD Mass Spectrometer/Micromass ZQ Mass Spectrometer from Waters (Single quadruple mass spectrometer); Ionisation method: Electro spray; Polarity: positive ions; Capillary (kV) 3.50, Cone (V) 40.00, Extractor (V) 3.00, Source Temperature (°C) 150, Desolvation Temperature (°C) 400, Cone Gas Flow (L/Hr) 50, Desolvation Gas Flow (L/Hr) 750; Mass range: 100 to 800 Da; DAD Wavelength range (nm): 210 to 400.

### Building Block Syntheses

The title compounds can be synthesized according to scheme 1. In brief, 1-(tert-butyl) 4-ethyl 3-oxopiperidine-1,4-dicarboxylate (**A**) is reacted with triflic anhydride to give 1-(tert-butyl) 4-ethyl 5-(((trifluoromethyl)sulfonyl)oxy)-3,6-dihydropyridine-1,4(2H)-dicarboxylate (**B**). This is converted to 1-(tert-butyl) 4-ethyl 5-(4,4,5,5-tetramethyl-1,3,2-dioxaborolan-2-yl)-3,6-dihydropyridine-1,4(2H)-dicarboxylate (**C**). coupling with various heteroaromatic halogenides results in esters **D.** Alternatively **D** can be obtained by Suzuki coupling of **B** with appropriate boronic acids or esters. Hydrogenation of **D** yields a mixture of cis/*trans*-piperidines **E.** Treatment with base causes isomerization and hydrolysis in a single step to give acids **F,** which are converted to the urease **G** by a Curtius rearrangement in the presence of (hetero)aromatic amines. Finally, deprotection under acidic conditions gives rise to the building blocks **BB.**

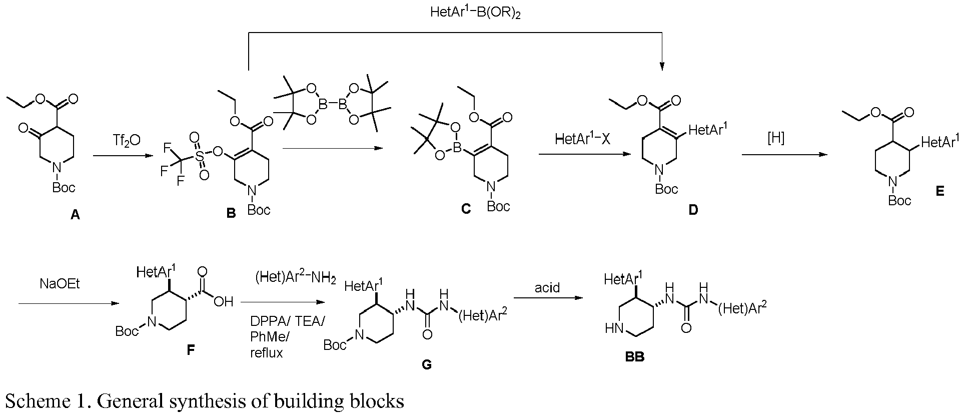

Final compounds can be synthesized from the building blocks by nucleophilic aromatic substitution, Chan-Lam coupling, Buchwald coupling, Ullmann coupling, reductive amination, nucleophilic substitution, amide coupling (eventually followed by reduction of the amide carbonyl), and any other C-N coupling reaction (Scheme 2).

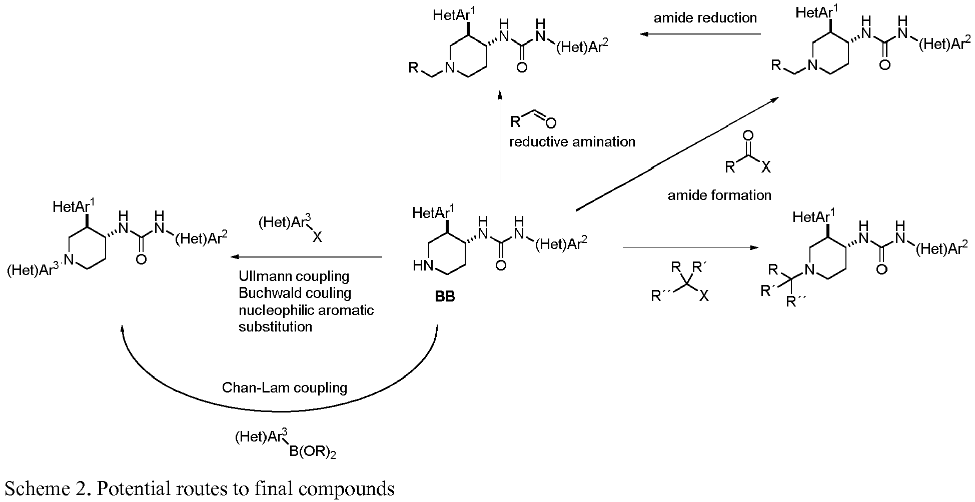

### Synthesis of trans-1-(4-chloro-phenyl)-3-(6'-methoxy-1,2,3,4,5,6-hexahydro-[3,3']bipyridiny1-4-yl)-urea hydrochloride (BB-I-HCl)

### Synthesis of 6'-methoxy-5,6-dihydro-2H-[3,3']bipyridinyl-1,4-dicarboxylic acid 1-tert-butyl ester 4-ethyl ester (D-I)

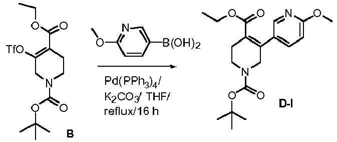

To a stirred solution of 5-trifluoromethanesulfonyloxy-3,6-dihydro-2H-pyridine-1,4-dicarboxylic acid 1-tert-butyl ester 4-ethyl ester (**B,** 10 g, 24.8 mmol, 1 eq) and 2-methoxy pyridine boronic acid (4.5 g, 29.4 mmol, 1.2 eq) in THF (100 ml) at RT was added K₂CO₃ (8.6 g, 62.03 mmol, 2.5 eq) and the reaction mixture was degassed with argon for a period of 15 minutes followed by the addition of Pd(PPh₃)₄ (860 mg, 0.74 mmol, 0.03 eq) and stirred under reflux for 16h. After completion of the reaction (monitored by TLC and LC-MS), the reaction mixture was diluted with EtOAc (600 ml) and washed with aq. NaHCO₃ solution and water. The organic layer was dried over anhyd. Na₂SO₄ and concentrated under reduced pressure to obtain crude product which was purified by silica gel (100-200 mesh) column chromatography (10-15% EtOAc/ Hexane) to afford 6'-methoxy-5,6-dihydro-2H-[3,3']bipyridinyl-1,4-dicarboxylic acid 1-tert-butyl ester 4-ethyl ester (**D-I,** 7.5 g, 73% yield) as brown oil.
LC-MS (Method 3): m/z [M+H]⁺ = 363.4 (exact mass calc. = 362.18).

### Synthesis of 6'-methoxy-3,4,5,6-tetrahydro-2H-[3,3']bipyridinyl-1,4-dicarboxylic acid 1-tert-butyl ester 4-ethyl ester (E-I)

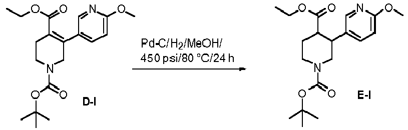

A stirred solution of 6'-methoxy-5,6-dihydro-2H-[3,3']bipyridinyl-1,4-dicarboxylic acid 1-tert-butyl ester 4-ethyl ester (**D-I,** 7 g, 19.33 mmol, 1 eq) in MeOH (150 ml) was degassed with argon for a period of 15 min followed by the addition of 10% moist Pd-C (3 g) and stirred at 80 °C under H₂ pressure (31 bar) in autoclave for a period of 24 h. After completion of the reaction, it was filtered through celite bed and washed thoroughly with 5% MeOH/ DCM. The solvent was evaporated to get the desired 6'-methoxy-3,4,5,6-tetrahydro-2H-[3,3']bipyridinyl-1,4-dicarboxylic acid 1-tert-butyl ester 4-ethyl ester (**E-I,** 7 g, 100% crude yield) as yellow sticky solid.
LC-MS (Method 1): m/z [M+H]⁺ = 365.0 (exact mass calc. = 364.20).

### Synthesis of trans-3-(4-methoxy-phenyl)-piperidine-1,4-dicarboxylic acid 1-tert-butyl ester (F-I)

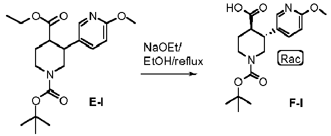

To a stirred solution of 6'-methoxy-3,4,5,6-tetrahydro-2H-[3,3']bipyridinyl-1,4-dicarboxylic acid 1-tert-butyl ester 4-ethyl ester (int-4, 7 g, 19.23 mmol, 1 eq) in EtOH (100 ml) was added 21% NaOEt in EtOH (6.5 ml, 20.19 mmol, 1.05 eq) and refluxed for a period of 6 h. After completion of the reaction, it was concentrated under reduced pressure and diluted with aq. NH4Cl solution and the organics were extracted with EtOAc. The solvent was evaporated and the residue was dissolved in in MeOH (25 ml), EtOH (50 ml) and H₂O (50 ml). KOH (2.15 g, 38.46 mmol, 2.0 eq) was added and refluxed for a period of 16 h. After completion of the reaction, it was concentrated under reduced pressure and diluted with water and washed with ethyl acetate. The aqueous part was acidified with IN HCl and the organic components were extracted with 5% MeOH in DCM. The combined organic layer was dried over anhyd. Na₂SO₄ and concentrated to get the desired *trans*-3-(4-methoxy-phenyl)-piperidine-1,4-dicarboxylic acid 1-tert-butyl ester (**F-I,** 5 g, 77% crude yield) as yellow solid.
LC-MS (Method 1): m/z [M+H]⁺ = 335.0 (exact mass calc. = 336.17).

### Synthesis of trans-4-[3-(4-chloro-phenyl)-ureido]-6'-methoxy-3,4,5,6-tetrahydro-2H-[3,3']bipyridinyl-1-carboxylic acid tert-butyl ester (G-I)

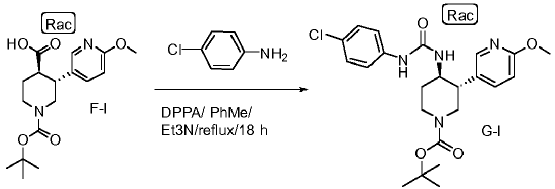

To a stirred solution of *trans*-6'-methoxy-3,4,5,6-tetrahydro-2H-[3,3']bipyridinyl-1,4-dicarboxylic acid 1-tert-butyl ester (**F-I,** 1.5 g, 4.47 mmol, 1 eq) in toluene (50 ml) was added Et₃N (1.25 ml, 8.94 mmol, 2 eq) followed by the addition of DPPA (1.9 ml, 8.92 mmol, 2 eq) and the reaction mass was refluxed for a period of 2 h. The reaction was cooled to RT and added 4-chloro-phenylamine (680 mg, 5.35 mmol, 1.2 eq) and refluxed for a period of 16 h. The reaction mixture was concentrated *in vacuo* and diluted with EtOAc (300 ml) and washed with water and brine. The combined organic layer was dried over anhyd. Na₂SO₄ and concentrated under reduced pressure to get the crude product which was purified by silica gel (100-200 mesh) column chromatography (25-40% EtOAc in Hexane) to obtain *trans-*4-[3-(4-chloro-phenyl)-ureido]-6'-methoxy-3,4,5,6-tetrahydro-2H-[3,3']bipyridinyl-1-carboxylic acid tert-butyl ester (**G-I**) (1.3 g, 63% yield) as yellowish solid.
LC-MS (Method 1): m/z [M+H]⁺ = 461.1 (exact mass calc. = 460.19).

### Synthesis of trans-1-(4-chloro-phenyl)-3-(6'-methoxy-1,2,3,4,5,6-hexahydro-[3,3']bipyridinyl-4-yl)-urea (BB-I-HCl)

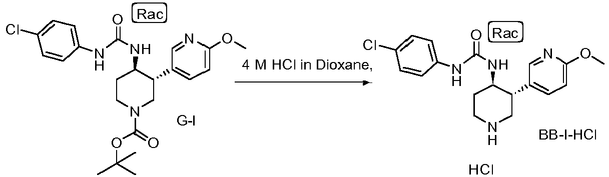

To a stirred solution of *trans*-4-[3-(4-hloro-phenyl)-ureido]-6'-methoxy-3,4,5,6-tetrahydro-2H-[3,3']bipyridinyl-1-carboxylic acid tert-butyl ester (**G-I**, 1.3 g, 2.82 mmol, 1 eq) in 1,4-dioxane (10 ml) was added 4M HCl in dioxane (10 ml) followed by stirring at RT for a period of 18 h. After completion of the reaction (monitored by TLC and LC-MS), the reaction mixture was concentrated under reduced pressure to get the crude material as HCl salt of *trans*-1*-*(4-chloro-phenyl)-3-(6'-methoxy-1,2,3,4,5,6-hexahydro-[3,3']bipyridinyl-4-yl)-urea (**BB-I-HCl,** 1 g, 90% crude yield) as brown solid.
LC-MS (Method 1): m/z [M+H]⁺ = 361.1 (exact mass calc. = 360.14).

### Synthesis of trans-1-(6'-methoxy-1,2,3,4,5,6-hexahydro-[3,3']bipyridinyl-4-yl)-3-(3-methyl-isothiazol-5-yl)-urea hydrochloride (BB-II-HCl)

### Synthesis of trans-6'-methoxy-4-[3-(3-methyl-isothiazol-5-yl)-ureido]-3,4,5,6-tetrahydro-2H-[3,3']bipyridinyl-1-carboxylic acid tert-butyl ester (G-II)

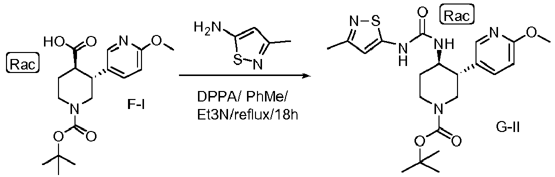

To a stirred solution of *trans*-6'-methoxy-3,4,5,6-tetrahydro-2H-[3,3']bipyridinyl-1,4-dicarboxylic acid 1-tert-butyl ester (**F-I,** 2 g, 5.95 mmol, 1 eq) in toluene (50 ml) was added Et₃N (1.7 ml, 11.9 mmol, 2 eq) followed by the addition of DPPA (2.6 ml, 11.9 mmol, 2.0 eq) and the reaction mixture was refluxed for a period of 2 h. The reaction was cooled to RT and added 3-methyl-isothiazol-5-ylamine (816 mg, 7.15 mmol, 1.2 eq) and refluxed for a period of 16 h. The reaction mixture was concentrated *in vacuo* and diluted with EtOAc (300 ml) and washed with water and brine. The combined organic layer was dried over anhyd. Na₂SO₄ and concentrated under reduced pressure to get the crude product which was purified by silica gel (100-200 mesh) column chromatography (25-40% EtOAc in Hexane) to obtain desired corresponding *trans*-6'-methoxy-4-[3-(3-methyl-isothiazol-5-yl)-ureido]-3,4,5,6-tetrahydro-2H-[3,3']bipyridinyl-1-carboxylic acid tert-butyl ester (**G-II,** 1.8 g, 68% yield) as yellowish solid.
LC-MS (Method 1): m/z [M+H]⁺ = 448.2 (exact mass calc. = 447.19).

### Synthesis of HCl salt of trans-1-(6'-methoxy-1,2,3,4,5,6-hexahydro-[3,3']bipyridinyl-4-yl)-3-(3-methyl-isothiazol-5-yl)-urea (BB-II-HCl)

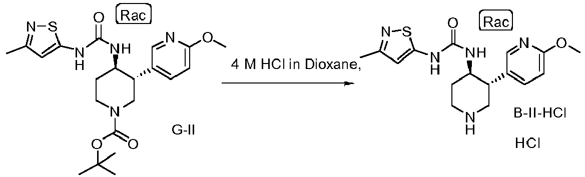

To a stirred solution of *trans*-6'-methoxy-4-[3-(3-methyl-isothiazol-5-yl)-ureido]-3,4,5,6-tetrahydro-2H-[3,3']bipyridinyl-1-carboxylic acid tert-butyl ester (**G-II,** 1.8 g, 4.02 mmol, 1 eq) in 1,4-dioxane (10 ml) was added 4M HCl in dioxane (10 ml) followed by stirring at RT for a period of 18 h. After completion of the reaction (monitored by TLC and LC-MS), the reaction mixture was concentrated under reduced pressure to get the crude material as HCl salt of *trans*-1-(6'-methoxy-1,2,3,4,5,6-hexahydro-[3,3']bipyridinyl-4-yl)-3-(3-methyl-isothiazol-5-yl)-urea (**BB-II-HCl,** 1.4 g, 90% crude yield) as brown solid.
LC-MS (Method 1): m/z [M+H]⁺ = 348.0 (exact mass calc. = 347.14).

### Synthesis of trans-1-(4-chloro-phenyl)-3-(5-methoxy-1',2',3',4',5',6'-hexahydro-[2,3']bipyridinyl-4'-yl)-urea (BB-III-HCl)

### Synthesis of 5-(4,4,5,5-tetramethyl-[1,3,2]dioxaborolan-2-yl)-3,6-dihydro-2H-pyridine-1,4-dicarboxylic acid 1-tert-butyl ester 4-ethyl ester (C):

To a stirred solution of 5-trifluoromethanesulfonyloxy-3,6-dihydro-2H-pyridine-1,4-dicarboxylic acid 1-tert-butyl ester 4-ethyl ester (**B,** 40.0 g, 99 mmol, 1 eq) in 1,4-dioxane (400 ml) was degassed with argon for a period of 15 min followed by the addition of 4,4,5,5,4',4',5',5'-octamethyl-[2,2']bi[[1,3,2]dioxaborolanyl] (30 g, 119.1 mmol, 1.2 eq), KOAc (4.3 g, 297 mmol, 3 eq) and PdCl₂(dppf).DCM (2.42 g, 2.97 mmol, 0.03 eq) and the reaction mixture was heated at reflux for a period of 16 h. After completion of the reaction (monitored by TLC and LC-MS), the reaction mixture was evaporated under reduced pressure and diluted with EA and washed with water and brine. The organic layer was dried over anhyd. Na₂SO₄ and concentrated under reduced pressure to obtain crude product which was purified by silica gel (100-200 mesh) column chromatography (5% EtOAc/Hexane) to afford 5-(4,4,5,5-tetramethyl-[1,3,2]dioxaborolan-2-yl)-3,6-dihydro-2H-pyridine-1,4-dicarboxylic acid 1-tert-butyl ester 4-ethyl ester (**C,** 32 g, 85% yield) as off white solid.
LC-MS (Method 2): m/z [M+H]⁺ = 382.2 (exact mass calc. = 381.23).

### Synthesis of 5-methoxy-5',6'-dihydro-2'H-[2,3']bipyridinyl-1',4'-dicarboxylic acid 1'-tert-butyl ester 4'-ethyl ester (D-III)

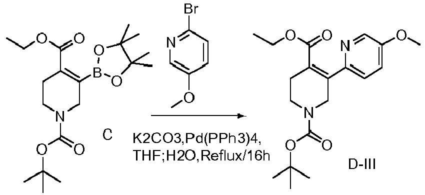

To a stirred solution of 5-(4,4,5,5-tetramethyl-[1,3,2]dioxaborolan-2-yl)-3,6-dihydro-2H-pyridine-1,4-dicarboxylic acid 1-tert-butyl ester 4-ethyl ester (**C,** 4 g, 10.49 mmol, 1 eq) and 2-bromo-5-methoxy-pyridine (2.3 g, 12.55 mmol, 1.2 eq) in THF:H₂O (4:1, 25 ml) at RT was added K₂CO₃ (3.6 g, 26.24 mmol, 2.5 eq) and the reaction mixture was degassed with argon for a period of 15 minutes followed by the addition of Pd(PPh₃)₄ (364 mg, 0.32 mmol, 0.03 eq) and stirred under reflux for 16h. After completion of the reaction (monitored by TLC and LC-MS), the reaction mixture was diluted with EtOAc (600 ml) and washed with aq. NaHCO₃ solution and water. The organic layer was dried over anhyd. Na₂SO₄ and concentrated under reduced pressure to obtain crude product which was purified by silica gel (100-200 mesh) column chromatography (15% EtOAc/Hexane) to afford 5-methoxy-5',6'-dihydro-2'H-[2,3']bipyridinyl-1,4'-dicarboxylic acid 1'-tert-butyl ester 4'-ethyl ester **(D-III,** 2 g, 53% yield) as yellow oil.
LC-MS (Method 3): m/z [M+H]⁺ = 363.4 (exact mass calc. = 362.18).

### Synthesis of 5-methoxy-3',4',5',6'-tetrahydro-2'H-[2,3']bipyridinyl-1',4'-dicarboxylic acid 1'-tert-butyl ester 4'-ethyl ester (E-III)

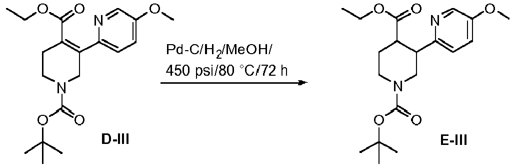

A stirred solution of 5-methoxy-5',6'-dihydro-2'H-[2,3']bipyridinyl-1',4'-dicarboxylic acid 1'-tert-butyl ester 4'-ethyl ester (**D-III,** 2.5 g, 6.90 mmol, 1 eq) in MeOH (50 ml) was degassed with argon for a period of 15 min followed by the addition of 10% moist Pd-C (1.2 g) and stirred at RT under H₂ pressure (31 bar) at 80 °C in autoclave for a period of 12 h. After completion of the reaction, it was filtered through celite bed and washed thoroughly with 5% MeOH/DCM. The solvent was evaporated to get the desired 5-methoxy-3',4',5',6'-tetrahydro-2'H-[2,3']bipyridinyl-1',4'-dicarboxylic acid 1'-tert-butyl ester 4'-ethyl ester (**E-III,** 2.5 g, 100% crude yield) as yellow sticky solid.
LC-MS (Method 3): m/z [M+H]⁺ = 365.3 (exact mass calc. = 364.20).

### Synthesis of trans-5-methoxy-3',4',5',6'-tetrahydro-2'H-[2,3']bipyridinyl-1',4'-dicarboxylic acid 1'-tert-butyl ester (G-III)

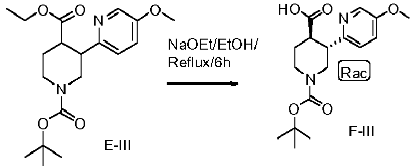

To a stirred solution of 5-methoxy-3',4',5',6'-tetrahydro-2'H-[2,3']bipyridinyl-1',4'-dicarboxylic acid 1'-tert-butyl ester 4'-ethyl ester (**E-III,** (2 g, 5.49 mmol, 1 eq) in EtOH (10 ml) was added 21% NaOEt in EtOH (1.8 ml, 5.78 mmol, 1.05 eq) and refluxed for a period of 6 h. After completion of the reaction, it was concentrated under reduced pressure and diluted with aq. NH₄Cl solution and the organics were extracted with DCM. The solvent was evaporated and the residue was dissolved in EtOH-Water (4:1, 25 ml) was added KOH (615 mg, 11 mmol, 2.0 eq) and refluxed for a period of 16 h. After completion of the reaction, it was concentrated under reduced pressure and diluted with water and washed with ethyl acetate. The aqueous part was acidified with IN HCl and the organic components were extracted with 5% MeOH in DCM. The combined organic layer was dried over anhyd. Na₂SO₄ and concentrated in vacuo to obtain *trans*-5-methoxy-3',4',5',6'-tetrahydro-2'H-[2,3']bipyridinyl-1',4'-dicarboxylic acid 1'-tert-butyl ester (**F-III,** 1.0 g, 54% crude yield) as off white solid.
LC-MS (Method 2): m/z [M+H]⁺ = 335.0 (exact mass calc. = 336.17).

### Synthesis of trans-4'-[3-(4-Chloro-phenyl)-ureido]-5-methoxy-3',4',5',6'-tetrahydro-2'H-[2,3']bipyridinyl-1'-carboxylic acid tert-butyl ester (G-III)

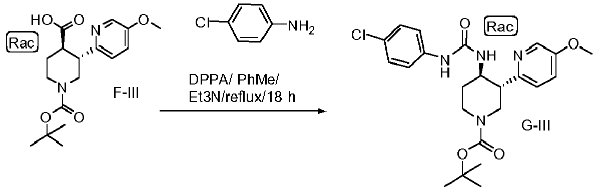

To a stirred solution of *trans*-5-methoxy-3',4',5',6'-tetrahydro-2'H-[2,3']bipyridinyl-1',4'-dicarboxylic acid 1'-tert-butyl ester (**F-III,** 500 mg, 1.48 mmol, 1eq) in Benzene:THF (1:2, 21 ml) was added Et₃N (0.4 ml, 2.97 mmol, 2.0 eq) followed by the addition of DPPA (0.42 ml, 1.93 mmol, 1.3 eq) and the reaction mass was stirred at RT for a period of 2 h, followed by the addition of 4-chloro phenylamine (226 mg, 1.78 mmol, 1.2 eq) and the reaction mixture was heated to reflux for a period of 16 h. The reaction mass was concentrated *in vacuo* and diluted with EtOAc (300 ml) and washed with water and brine. The combined organic layer was dried over anhyd. Na₂SO₄ and concentrated under reduced pressure to get the crude product which was purified by silica gel (100-200 mesh) column chromatography (25% EtOAc in Hexane) to obtain desired *trans*-4'-[3-(4-chloro-phenyl)-ureido]-5-methoxy-3',4',5',6'-tetrahydro-2'H-[2,3']bipyridinyl-1'-carboxylic acid tert-butyl ester (**G-III,** 250 mg, 37% yield) as off white solid.
LC-MS (Method 1): m/z [M+H]⁺ = 461.2 (exact mass calc. = 460.19).

### Synthesis of trans-1-(4-chloro-phenyl)-3-(5-methoxy-1',2',3',4',5',6'-hexahydro-[2,3']bipyridinyl-4'-yl)-urea (BB-III-HCl)

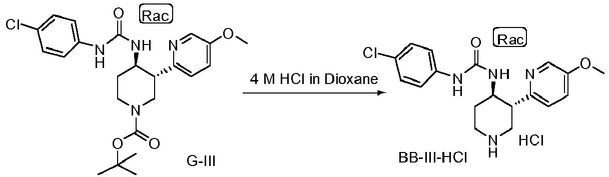

To a stirred solution of *trans*-4'-[3-(4-chloro-phenyl)-ureido]-5-methoxy-3',4',5',6'-tetrahydro-2'H-[2,3']bipyridinyl-1'-carboxylic acid tert-butyl ester (**G-III,** 250 mg, 0.54 mmol, 1 eq) in dioxane (5 ml) was added 4M HCl in dioxane (2 ml) followed by stirring at RT for a period of 4 h. After completion of the reaction (monitored by TLC and LC-MS), the reaction mixture was concentrated under reduced pressure to get the crude material as HCl salt of *trans*-1-(4-chloro-phenyl)-3-(5-methoxy-1',2',3',4',5',6'-hexahydro-[2,3']bipyridinyl-4'-yl)-urea (**BB-III-HCl,** 200 mg, 93% crude yield).
LC-MS (Method 1): m/z [M+H]⁺ = 361.2 (exact mass calc. = 360.14).

### Synthesis of 1-(4-chlorophenyl)-3-[3-(5-methoxypyridin-2-yl)piperidin-4-yl]urea (BB-III-TFA):

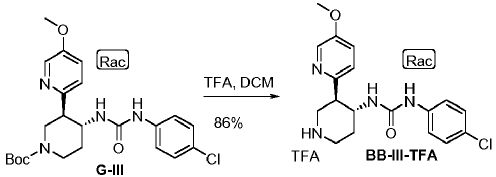

To the solution of **G-III** (0.45 g, 0.978 mmol, 1 eq) in DCM (6 ml), TFA (6 ml) was added drop wise at 0 °C. The reaction was allowed to stirr at room temperature for 2 hour. After completion of reaction (checked by TLC) DCM and excess TFA was removed under reduced pressure to get crude which was washed with diethyl ether and removed ether under reduced pressure to get 1-(4-chlorophenyl)-3-[3-(5-methoxypyridin-2-yl)piperidin-4-yl]urea (**BB-III-TFA,** 0.4 g, 86% as mono TFA salt).

### Synthesis of trans-1-(5-methoxy-1',2',3',4',5',6'-hexahydro-[2,3']bipyridinyl-4'-yl)-3-(3-methyl-isothiazol-5-yl)-urea hxdrochloride (BB-IV-HCl)

### Synthesis of trans-5-methoxy-4'-[3-(3-methyl-isothiazol-5-yl)-ureido]-3',4',5',6'-tetrahydro-2'H-[2,3']bipyridinyl-1'-carboxylic acid tert-butyl ester (G-IV)

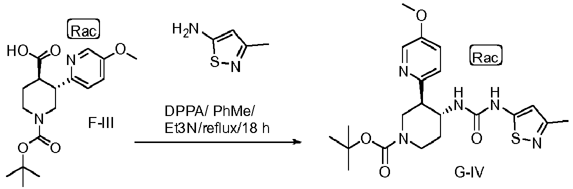

To a stirred solution of *trans*-5-methoxy-3',4',5',6'-tetrahydro-2'H-[2,3']bipyridinyl-1',4'-dicarboxylic acid 1'-tert-butyl (**F-III,** 500 mg, 1.48 mmol, 1eq) in toluene (10 ml) was added Et₃N (0.4 ml, 2.97 mmol, 2.0 eq) followed by the addition of DPPA (0.42 ml, 1.93 mmol, 1.3 eq) and the reaction mass was stirred under reflux for a period of 2 h, followed by the addition of 3-methyl-isothiazol-5-ylamine (205 mg, 1.78 mmol, 1.2 eq) and the reaction mixture was continued to reflux for another 16 h. The reaction mass was concentrated *in vacuo* and diluted with EtOAc (300 ml) and washed with water and brine. The combined organic layer was dried over anhyd. Na₂SO₄ and concentrated under reduced pressure to get the crude product which was purified by silica gel (100-200 mesh) column chromatography (25% EtOAc in Hexane) to obtain desired *trans*-5-methoxy-4'-[3-(3-methyl-isothiazol-5-yl)-ureido]-3',4',5',6'-tetrahydro-2'H-[2,3']bipyridinyl-1'-carboxylic acid tert-butyl ester (**G-IV,** 190 mg, 29% yield) as off white solid.
LC-MS (Method 1): m/z [M+H]⁺ = 448.2 (exact mass calc. = 447.19).

### Synthesis of f trans-1-(5-methoxy-1',2',3',4',5',6'-hexahydro-[2,3']bipyridinyl-4'-yl)-3-(3-methyl-isothiazol-5-yl)-urea (BB-IV-HCl)

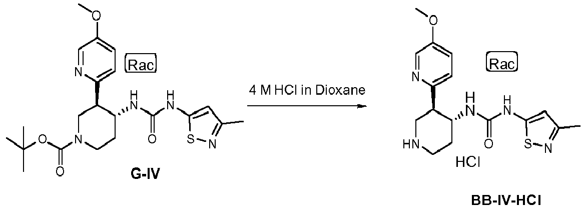

To a stirred solution of *trans*-5-methoxy-4'-[3-(3-methyl-isothiazol-5-yl)-ureido]-3',4',5',6'-tetrahydro-2'H-[2,3']bipyridinyl-1'-carboxylic acid tert-butyl ester (**G-IV,** 100 mg, 0.223 mmol, 1 eq) in 1,4-dioxane (2 ml) was added 4M HCl in dioxane (1 ml) followed by stirring at RT for a period of 4 h. After completion of the reaction (monitored by TLC and LC-MS), the reaction mixture was concentrated under reduced pressure to get the crude material as HCl salt of *trans*-1-(5-methoxy-1',2',3',4',5',6'-hexahydro-[2,3']bipyridinyl-4'-yl)-3-(3-methyl-isothiazol-5-yl)-urea (**BB-IV-HCl,** 85 mg, 99% crude yield).
LC-MS (Method 1): m/z [M+H]⁺ = 348.2 (exact mass calc. = 347.14).

### Synthesis of trans-1-(5-chloro-1',2',3',4',5',6'-hexahydro-[2,3']bipyridinyl-4'-yl)-3-(4-chloro-phenyl)-urea trifluoroacetate (BB-V-TFA)

### Synthesis of 5-nitro-5',6'-dihydro-2'H-[2,3']bipyridinyl-1',4'-dicarboxylic acid 1'-tert-butyl ester 4'-ethyl ester (D-V)

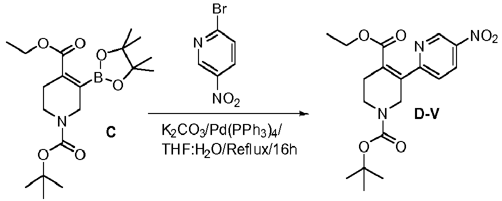

To a stirred solution of 5-(4,4,5,5-tetramethyl-[1,3,2]dioxaborolan-2-yl)-3,6-dihydro-2H-pyridine-1,4-dicarboxylic acid 1-tert-butyl ester 4-ethyl ester (**C,** 1 g, 2.6 mmol, 1 eq) and 2-bromo-5-nitro-pyridine (583 mg, 2.85 mmol, 1.1 eq) in THF:H₂O (7:1, 21 ml) at RT was added K₂CO₃ (897 mg, 6.5 mmol, 2.5 eq) and the reaction mixture was degassed with argon for a period of 15 minutes followed by the addition of Pd(PPh₃)₄ (90 mg, 0.08 mmol, 0.03 eq) and stirred under reflux for 16h. After completion of the reaction (monitored by TLC and LC-MS), the reaction mixture was diluted with EtOAc (600 ml) and washed with aq. NaHCO₃ solution and water. The organic layer was dried over anhyd. Na₂SO₄ and concentrated under reduced pressure to obtain crude product which was purified by silica gel (100-200 mesh) column chromatography (15% EtOAc/Hexane) to afford 5-nitro-5',6'-dihydro-2'H-[2,3']bipyridinyl-1',4'-dicarboxylic acid 1'-tert-butyl ester 4'-ethyl ester (**D-V,** 0.5 g, 51% yield) as yellow oil.
LC-MS (Method 1): m/z [M+H]⁺ = 378.1 (exact mass calc. = 377.16).

### Synthesis of 5-amino-3',4',5',6'-tetrahydro-2'H-[2,3']bipyridinyl-1',4'-dicarboxylic acid 1'-tert-butyl ester 4'-ethyl ester (E-Va):

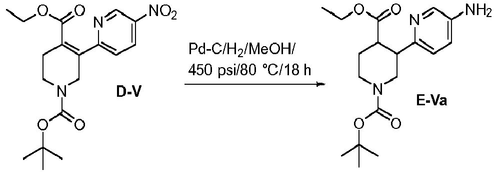

A stirred solution of 5-nitro-5',6'-dihydro-2'H-[2,3']bipyridinyl-r,4'-dicarboxylic acid 1'-tert-butyl ester 4'-ethyl ester (**D-V,** 2 g, 5.30 mmol, 1eq) in MeOH (200 ml) was degassed with argon for a period of 15 min followed by the addition of 10% moist Pd-C (1 g) and stirred at RT under H₂ pressure (31 bar) in autoclave at 80 °C for a period of 18 h. After completion of the reaction, it was filtered through celite bed and washed thoroughly with 5% MeOH/ DCM. The filtrate was concentrated to get 5-amino-3',4',5',6'-tetrahydro-2'H-[2,3']bipyridinyl-1',4'-dicarboxylic acid 1'-tert-butyl ester 4'-ethyl ester (**E-Va**) (1.8 g, 97% crude yield) as colorless gummy material.
LC-MS (Method 1): m/z [M+H]⁺ = 350.2 (exact mass calc. = 349.20).

### Synthesis of 1'-acetyl-5-chloro-1',2',3',4',5',6'-hexahydro-[2,3']bipyridinyl-4'-carboxylic acid ethyl ester (E-V)

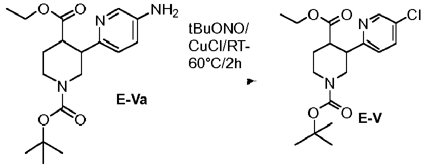

To a stirred solution of 5-amino-3',4',5',6'-tetrahydro-2'H-[2,3']bipyridinyl-1',4'-dicarboxylic acid 1'-tert-butyl ester 4'-ethyl ester (**E-Va,** 4.3 g, 12.2 mmol, 1 eq) in CH₃CN (100 ml) under inert atmosphere was added CuCl (1.8 g, 18.48 mmol, 1.5 eq) and stirred for 5 min at RT followed by the addition of *t*-BuONO (2.9 ml, 24.4 mmol, 2 eq) in CH₃CN (5 ml) drop wise and stirred at RT for another 2 h. The reaction mixture was heated at 60 °C for 2 h. The reaction mixture was quenched with saturated NH₄Cl (50 ml) and then evaporated under reduced pressure and the residue was diluted with EtOAc (300 ml) washed with water (50 ml) and brine (50 ml). The organic layer was dried over anhyd. Na₂SO₄ and concentrated under reduced pressure to obtain crude product which was purified by silica gel (100-200 mesh) column chromatography (10-15% EtOAc/Hexane) to afford 1'-acetyl-5-chloro-1',2',3',4',5',6'-hexahydro-[2,3']bipyridinyl-4'-carboxylic acid ethyl ester (**E-V,** 1.5 g, 33% yield) as colorless gummy material.
LC-MS (Method 1): m/z [M+H]⁺ = 368.9 (exact mass calc. = 368.15).

### Synthesis of trans-1-(tert-butyl) 4-ethyl 3-(5-chloropyridin-2-yl)piperidine-1,4-dicarboxylate (F-V)

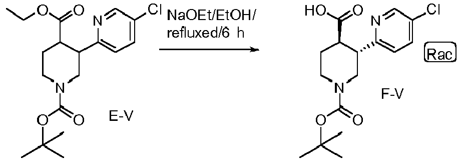

To a stirred solution of 1'-acetyl-5-chloro-1',2',3',4',5',6'-hexahydro-[2,3']bipyridinyl-4'-carboxylic acid ethyl ester (**E-V,** 700 mg, 1.90 mmol, 1eq) in EtOH (10 ml) was added 21% NaOEt in EtOH (700 µl, 5.78 mmol, 1.05 eq) and refluxed for a period of 6 h. After completion of the reaction, it was concentrated under reduced pressure and diluted with aq. NH₄Cl solution and the organics were extracted with DCM. The solvent was evaporated, the crude material was dissolved in EtOH-Water-MeOH (10:2:1, 13 ml) was added KOH (213 mg, 3.8 mmol, 2.0 eq) and refluxed for a period of 16 h. After completion of the reaction, it was concentrated under reduced pressure and diluted with water and washed with ethyl acetate. The aqueous part was acidified by IN HCl and the organic components were extracted with 5% MeOH in DCM. The combined organic layer was dried over anhyd. Na2SO4 and concentrated *in vacuo* to get *trans*-1-(tert-butyl) 4-ethyl 3-(5-chloropyridin-2-yl)piperidine-1,4-dicarboxylate (**F-V,** 400 mg, 62% crude yield) as off white solid.
LC-MS (Method 1): m/z [M+H]⁺ = 341.1 (exact mass calc. = 340.12).

### Synthesis of trans-5-chloro-4'-[3-(4-chloro-phenyl)-ureido]-3',4',5',6'-tetrahydro-2'H-[2,3']bipyridinyl-1'-carboxylic acid tert-butyl ester (G-V)

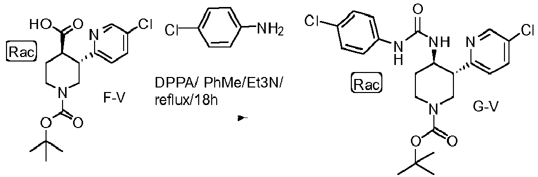

To a stirred solution of *trans*-1-(tert-butyl) 4-ethyl 3-(5-chloropyridin-2-yl)piperidine-1,4-dicarboxylate (**F-V,** 200 mg, 0.588 mmol, 1 eq) in benzene:THF (1:5, 18 ml) was added Et₃N (165 µl, 1.17 mmol, 2.0 eq) followed by the addition of DPPA (202 µl, 0.94 mmol, 1.6 eq) and the reaction mass was stirred at RT for a period of 2 h, followed by the addition of 4-chloro phenylamine (150 mg, 1.17 mmol, 2 eq) and the reaction mixture was heated to reflux for a period of 16 h. The reaction mass was concentrated *in vacuo* and diluted with EtOAc (150 ml) and washed with water and brine. The combined organic layer was dried over anhyd. Na₂SO₄ and concentrated under reduced pressure to get the crude product which was purified by silica gel (100-200 mesh) column chromatography (25% EtOAc in hexane) to obtain *trans*-5-chloro-4'-[3-(4-chloro-phenyl)-ureido]-3',4',5',6'-tetrahydro-2'H-[2,3']bipyridinyl-1'-carboxylic acid tert-butyl ester (**G-V,** 166 mg, 61% yield) as yellowish solid.
LC-MS (Method 1): m/z [M+H]⁺ = 465.4 (exact mass calc. = 464.14).

### Synthesis of trans-1-(5-chloro-1',2',3',4',5',6'-hexahydro-[2,3']bipyridinyl-4'-yl)-3-(4-chloro-phenyl)-urea trifluoroacetate (BB-V-TFA)

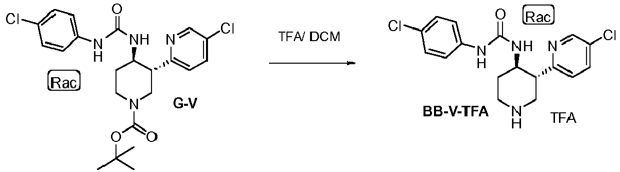

To a stirred solution of *trans*-5-chloro-4'-[3-(4-chloro-phenyl)-ureido]-3',4',5',6'-tetrahydro-2'H-[2,3']bipyridinyl-1'-carboxylic acid tert-butyl ester (**G-V,** 400 mg, 0.862 mmol, 1 eq) in DCM (15 ml) was added TFA (400 µl) followed by stirring at RT for a period of 6 h. After completion of the reaction (monitored by TLC and LC-MS), the reaction mixture was concentrated under reduced pressure to get the crude material as TFA salt of *trans*-1-(5-chloro-1',2',3',4',5',6'-hexahydro-[2,3']bipyridinyl-4'-yl)-3-(4-chloro-phenyl)-urea (**BB-V-TFA,** 350 mg, 85% crude yield).
LC-MS (Method 1): m/z [M+H]⁺ = 365.1 (exact mass calc. = 364.09).

### Synthesis of trans-1-(5-chloro-1',2',3',4',5',6'-hexahydro-[2,3']bipyridinyl-4'-yl)-3-(3-methyl-isothiazol-5-yl)-urea hydrochloride (BB-VI-HCl)

### Synthesis of trans-5-chloro-4'-[3-(3-methyl-isothiazol-5-yl)-ureido]-3',4',5',6'-tetrahydro-2'H-[2,3']bipyridinyl-1'-carboxylic acid tert-butyl ester (G-VI)

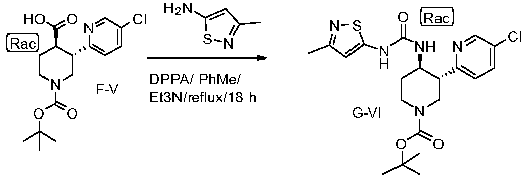

To a stirred solution of *trans*-5-chloro-3',4',5',6'-tetrahydro-2'H-[2,3']bipyridinyl-1',4'-dicarboxylic acid 1'-tert-butyl ester (**F-V,** 800 mg, 2.35 mmol, 1eq) in toluene (20 ml) was added Et₃N (655 µl, 4.70 mmol, 2.0 eq) followed by the addition of DPPA (660 µl, 3.05 mmol, 1.3 eq) and the reaction mass was stirred under reflux for a period of 2 h and allowed to cool to an ambient temperature, followed by the addition of 3-methyl-isothiazol-5-ylamine (590 mg, 5.17 mmol, 2.2 eq) was and refluxed for a period of 16h. The reaction mixture was concentrated *in vacuo* and diluted with EtOAc (150 ml) and washed with water and brine. The combined organic layer was dried over anhyd. Na₂SO₄ and concentrated under reduced pressure to get the crude product which was purified by silica gel (100-200 mesh) column chromatography (25% EtOAc in hexane) to obtain *trans*-5-chloro-4'-[3-(3-methyl-isothiazol-5-yl)-ureido]-3',4',5',6'-tetrahydro-2'H-[2,3']bipyridinyl-1'-carboxylic acid tert-butyl ester (**G-VI,** 400 mg, 38% yield) as brown solid.
LC-MS (Method 1): m/z [M+H]⁺ = 452.1 (exact mass calc. = 451.14).

### Synthesis of HCl salt of trans-1-(5-chloro-1',2',3',4',5',6'-hexahydro-[2,3']bipyridinyl-4'-yl)-3-(3-methyl-isothiazol-5-yl)-urea (BB-VI-HCl)

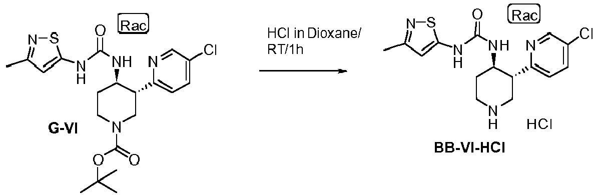

To a stirred solution of *trans*-5-chloro-4'-[3-(3-methyl-isothiazol-5-yl)-ureido]-3',4',5',6'-tetrahydro-2'H-[2,3']bipyridinyl-1'-carboxylic acid tert-butyl ester (**G-VI,** 200 mg, 0.443 mmol, 1 eq) in 1,4-dioxane (10 ml) was added 4 M Dioxane (2 ml) followed by stirring at RT for a period of 1 h. After completion of the reaction (monitored by TLC and LC-MS), the reaction mixture was concentrated under reduced pressure to get the crude material as HCl salt of *trans*-1-(5-chloro-1',2',3',4',5',6'-hexahydro-[2,3']bipyridinyl-4'-yl)-3-(3-methyl-isothiazol-5-yl)-urea (**BB-VI-HCl,** 160 mg, 93% crude yield).
LC-MS (Method 3): m/z [M+H]⁺ = 352.2 (exact mass calc. = 351.09).

### Synthesis of trans-1-(4-chlorophenyl)-3-[3-(5-chlorothiophen-2-yl)-piperidin-4-yl]urea trifluoroacetate (BB-VII-TFA)

### Synthesis of 1-[(tert-butoxy)carbonyl]-5-(5-chlorothiophen-2-yl)-1,2,3,6-tetrahydropyridine-4-carboxylic acid

To a solution of 1-(tert-butyl) 4-ethyl 5-(4,4,5,5-tetramethyl-1,3,2-dioxaborolan-2-yl)-3,6-dihydropyridine-1,4(2H)-dicarboxylate (**C,** 34.7 g, 91.3 mmol, 1.2 eq) in dioxane (200 ml), K₂CO₃ (31.5 g, 228.3 mmol, 3 eq) and 2-bromo-5-chlorothiophene (15.0 g, 76.1 mmol, 1 eq) was added at RT. The reaction mixture was then degassed for 30 min, followed by the addition of Pd(PPh₃)₄ (4.3 g, 3.8 mmol, 0.05 eq). The reaction mixture was then heated at 110 °C for 6h. After completion of reaction (monitored by TLC), reaction mixture was evaporated under reduced pressure to get the crude. Crude was purified by column chromatography to afford desired 1-[(tert-butoxy)carbonyl]-5-(5-chlorothiophen-2-yl)-1,2,3,6-tetrahydropyridine-4-carboxylic acid (8 g, 30.5%) as brown gum.

### Synthesis of 1-tert-butyl 4-ethyl 5-(5-chlorothiophen-2-yl)-1,2,3,6-tetrahydropyridine-1,4-dicarboxylate

To a solution of 1-[(tert-butoxy)carbonyl]-5-(5-chlorothiophen-2-yl)-1,2,3,6-tetrahydropyridine-4-carboxylic acid (30.0 g, 87.4 mmol, 1 eq) in Acetone (200 ml), K₂CO₃ (24 g, 177.8 mmol, 2 eq) and ethyl iodide (10.5 mL, 131.1 mmol, 1.5 eq) was added at RT for 16 h. After completion of reaction (monitored by TLC), reaction mixture was evaporated under reduced pressure to get the crude. Crude was purified by column chromatography to afford desired 1-tert-butyl 4-ethyl 5-(5-chlorothiophen-2-yl)-1,2,3,6-tetrahydropyridine-1,4-dicarboxylate (10 g, 30.76%) as brown gum.

### Synthesis of 1-tert-butyl 4-methyl 3-(5-chlorothiophen-2-yl)piperidine-1,4-dicarboxylate

To a solution of 1-tert-butyl 4-ethyl 5-(5-chlorothiophen-2-yl)-1,2,3,6-tetrahydropyridine-1,4-dicarboxylate (20.0 g, 53.9 mmol, 1 eq) in MeOH (100 ml), Mg (31.05 g, 1293 mmol, 24 eq) was added slowly to the reaction at RT. After 15 min reaction was initiated, cooled the reaction using ice water and stirred for 2 h at RT. After completion of reaction (monitored by TLC), reaction mixture was quenched by water and passing through celite bed using ethyl acetate. Reaction was extracted with ethyl acetate (3x500 ml). Organic part was collected, dried over Na₂SO₄ and evaporated to dryness to get crude, which was purified by column chromatography to get 1-tert-butyl 4-methyl 3-(5-chlorothiophen-2-yl)piperidine-1,4-dicarboxylate (7.8 g, 40.3%).

### Synthesis of trans-1-[(tert-butoxy)carbonyl]-3-(5-chlorothiophen-2-yl)piperidine-4-carboxylic acid

To the solution of 1-tert-butyl 4-methyl 3-(5-chlorothiophen-2-yl)piperidine-1,4-dicarboxylate (7.8 g, 21.6 mmol, 1 eq) in EtOH (100 ml), 21 % NaOEt solution (8.8 ml, 23.8 mmol, 1.1 eq) in EtOH was added at room temperature and allowed to stir at 90 °C for 16 hour. Then reaction mixture was cooled to room temperature and aqueous solution of KOH (3.6 g, 64.8 mmol, 3 eq) was added, again allowed to stir at 90 °C for 4 h. After completion of reaction EtOH was removed under reduced pressure, add water and aqueous part was washed with DCM for one time. Then aqueous part was acidified using sodium bisulfite solution and extracted with 10% MeOH in DCM (3 x 100 ml). Organic part was collected, dried over Na₂SO₄ and evaporated to dryness to get crude of *trans-*1-[(tert-butoxy)carbonyl]-3-(5-chlorothiophen-2-yl)piperidine-4-carboxylic acid (6 g, 80.3%) as a light yellowish gummy liquid, which was used for next step without further purification.

### Synthesis of trans-tert-butyl-4-{[(4-chlorophenyl)carbamoyl]amino}-3-(5-chlorothiophen-2-yl)piperidine-1-carboxylate

To the solution of of *trans*-1-[(tert-butoxy)carbonyl]-3-(5-chlorothiophen-2-yl)piperidine-4-carboxylic acid (6.5 g, 18.7 mmol, 1 eq) in tolune (50 ml), TEA (5.2 ml, 37.4 mmol, 2 eq) and DPPA (8.0 ml, 37.4 mmol, 2 eq) were added at room temperature and allowed to stir at 90 °C. After 2 hour reaction mixtures was cooled to room temperature and added 4-Chloro aniline (3.59 g, 28.1 mmol, 1.5 eq) and allowed to reflux for 16 hour. After completion of reaction cooled to room temperature, add water and extracted with ethyl acetate (3x50 ml). Organic part was collected, dried over Na₂SO₄ and evaporated to dryness to get crude, which was purified by column chromatography and submitted in prep-HPLC to get desired *trans*-tert-butyl -4-{[(4-chlorophenyl)carbamoyl]amino} -3-(5-chlorothiophen-2-yl)piperidine-1-carboxylate (1.9 g, 21.5%).

### Synthesis of trans-1-(4-chlorophenyl)-3-[(3-(5-chlorothiophen-2-yl)-piperidin-4-yl]urea trifluoroacetate (BB-VII-TFA)

To the solution of *trans*-tert-butyl-4-{[(4-chlorophenyl)carbamoyl]amino}-3-(5-chlorothiophen-2-yl)piperidine-1-carboxylate (1.37 g, 2.9 mmol, 1 eq) in DCM (20 ml), TFA (5 ml) was added drop wise at 0 °C. The reaction was allowed to stirr at room temperature for 2 hour. After completion of reaction (checked by TLC) DCM and excess TFA was removed under reduced pressure to get crude which was washed with diethyl ether and removed ether under reduced pressure to get *trans*-1-(4-chlorophenyl)-3-[3-(5-chlorothiophen-2-yl)-piperidin-4-yl]urea trifluoroacetate (**BB-VII-TFA,** 1.2 g, 88.5%) as TFA salt (as mono TFA salt).

### Synthesis of 1-[(3-(5-chlorothiophen-2-yl)-piperidin-4-yl]-3-(3-methyl-1,2-thiazol-5-yl)urea trifluoroacetate (BB-VIII-TFA)

### Preparation of trans-tert-butyl 4-{[(4-chlorophenyl)carbamoyl]amino}-3-(5-chlorothiophen-2-yl)piperidine-1-carboxylate

To the solution of *trans*-1-[(tert-butoxy)carbonyl]-3-(5-chlorothiophen-2-yl)piperidine-4-carboxylic acid (2.2 g, 6.361 mmol, 1 eq, see **BB-VII-TFA**) in toluene (20 ml), TEA (1.77 ml, 12.722 mmol, 2 eq) and DPPA (2.7 ml, 12.722 mmol, 2 eq) were added at room temperature and allowed to stir at 90 °C. After 2 hour reaction mixtures was cooled to room temperature and added 3-methyl-1,2-thiazol-5-amine (1.08 g, 9.54 mmol, 1.5 eq) and allowed to reflux for 16 hour. After completion of reaction cooled to room temperature, add water and extracted with ethyl acetate (3x50 ml). Organic part was collected, dried over Na₂SO₄ and evaporated to dryness to get crude, which was purified by column chromatography and submitted in prep-HPLC to get desired tert-butyl 4-{[(4-chlorophenyl)carbamoyl]amino} -3-(5-chlorothiophen-2-yl)piperidine-1-carboxylate (0.5g, 17%).

### Preparation of trans-1-[3-(5-chlorothiophen-2-yl)-piperidin-4-yl]-3-(3-methyl-1,2-thiazol-5-yl)urea trifluoroacetate (BB-VIII-TFA)

To the solution of tert-butyl 4-{[(4-chlorophenyl)carbamoyl]amino}-3-(5-chlorothiophen-2-yl)piperidine-1-carboxylate (0.850 g, 1.859 mmol, 1 eq) in DCM (20 ml), TFA (5 ml) was added drop wise at 0 °C. The reaction was allowed to stirr at room temperature for 2 hour. After completion of reaction (checked by TLC) DCM and excess TFA was removed under reduced pressure to get crude which was washed with diethyl ether and removed ether under reduced pressure to get *trans*-1-[3-(5-chlorothiophen-2-yl)-piperidin-4-yl]-3-(3-methyl-1,2-thiazol-5-yl)urea trifluoroacetate (**BB-VIII-TFA,** 0.880 g, quant. as mono TFA salt).

### Synthesis of 3-[3-(5-chlorothiophen-2-yl)piperidin-4-yl]-1-phenylurea (BB-IX-TFA)

### Synthesis of tert-butyl 3-(5-chlorothiophen-2-yl)-4-[(phenylcarbamoyl)amino]piperidine-1-carboxylate

To the solution of 1-[(tert-butoxy)carbonyl]-3-(5-chlorothiophen-2-yl)piperidine-4-carboxylic acid (1.5 g, 4.3 mmol, 1 eq, see **BB-VII-TFA**) in toluene (25 ml), TEA (0.9 ml, 6.51 mmol, 1.5 eq) and DPPA (1.4 ml, 6.51 mmol, 1.5 eq) were added at room temperature and allowed to stir at 90 °C. After 2 hour reaction mixtures was cooled to room temperature and added aniline (0.6 g, 6.51 mmol, 1.5 eq) and allowed to reflux for 16 hour. After completion of reaction cooled to room temperature, add water and extracted with ethyl acetate (3x50 ml). Organic part was collected, dried over Na₂SO₄ and evaporated to dryness to get crude, which was purified by column chromatography and submitted in prep-HPLC to get desired tert-butyl 3-(5-chlorothiophen-2-yl)-4-[(phenylcarbamoyl)amino]piperidine-1-carboxylate (0.6 g, 32%).
LC-MS (Method 3): m/z [M+H]⁺ = 436.0 (exact mass calc. = 435.14).

### Synthesis of 3-[3-(5-chlorothiophen-2-yl)piperidin-4-yl]-1-phenylurea trifluoroacetate (BB-IX-TFA)

To the solution of tert-butyl 3-(5-chlorothiophen-2-yl)-4-[(phenylcarbamoyl)amino]piperidine-1-carboxylate (0.6 g, 1.38 mmol, 1 eq) in DCM (10 ml), TFA (3 ml) was added drop wise at 0 °C. The reaction was allowed to stir at room temperature for 2 hour. After completion of reaction (checked by TLC, 5% MeOH in DCM, R*_{f}*=0.1). DCM and excess TFA was removed under reduced pressure to get crude which was washed with diethyl ether and removed ether under reduced pressure to get 3-[3-(5-chlorothiophen-2-yl)piperidin-4-yl]-1-phenylurea trifluoroacetate (**BB-IX-TFA,** 0.5 g, 81% as mono TFA salt).

### Synthesis of trans-1-(3-(5-methoxypyridin-2-yl)-1-methylpiperidin-4-yl)-3-phenylurea (BB-X-TFA)

### Synthesis of trans-tert-butyl 3-(5-methoxypyridin-2-yl)-4-(3-phenylureido)piperidine-1-carboxylate

To the solution of *trans*-5-methoxy-3',4',5',6'-tetrahydro-2'H-[2,3']bipyridinyl-1',4'-dicarboxylic acid 1'-tert-butyl ester (**F-III,** 0.65 g, 1.93 mmol, 1 eq) in toluene (10 ml), TEA (0.54 ml, 3.87 mmol, 2 eq) and DPPA (0.9 ml, 3.87 mmol, 2 eq) were added at room temperature and allowed to stir at 90 °C. After 2 hour reaction mixtures was cooled to room temperature and added aniline (0.26 ml, 2.90 mmol, 1.2 eq) and allowed to reflux for 4 hour. After completion of reaction cooled to room temperature, add water and extracted with ethyl acetate (3x50 ml). Organic part was collected, dried over Na₂SO₄ and evaporated to dryness to get crude, which was purified by column chromatography to get *trans-*tert-butyl 3-(5-methoxypyridin-2-yl)-4-(3-phenylureido)piperidine-1-carboxylate (0.5 g, 61%). LC-MS (Method 2): m/z [M+H]⁺ = 427.4 (exact mass calc. = 426.23).

### Synthesis of trans-1-(3-(5-methoxypyridin-2-yl)-1-methylpiperidin-4-yl)-3-phenylurea trifluoroacetate (BB-X-TFA)

To the solution of 3-(5-methoxypyridin-2-yl)-4-(3-phenylureido)piperidine-1-carboxylate (0.49 g, 1.15 mmol, 1 eq) in DCM (10 ml), TFA (4 ml) was added drop wise at 0 °C. The reaction was allowed to stir at room temperature for 2 hour. After completion of reaction (checked by TLC) DCM and excess TFA was removed under reduced pressure to get crude which was washed with diethyl ether and removed ether under reduced pressure to get *trans-*1-(3-(5-methoxypyridin-2-yl)-1-methylpiperidin-4-yl)-3-phenylurea (**BB-X-TFA,** 0.5 g, 99% as mono TFA salt).

### Synthesis of trans-1-(4-cyanophenyl)-3-[3-(5-methoxypyridin-2-yl)piperidin-4-yl]urea trifluoroacetate (BB-XI-TFA)

### Synthesis of trans-tert-butyl-4-{[(4-cyanophenyl)carbamoyl]amino}-3-(5-methoxypyridin-2-yl)piperidine-1-carboxylate

To the solution of *trans*-1-[(tert-butoxy)carbonyl]-3-(5-methoxypyridin-2-yl)piperidine-4-carboxylic acid (**F-III,** 1.8 g, 5.35 mmol, 1 eq) in toluene (50 ml), TEA (1.5 ml, 10.7 mmol, 2 eq) and DPPA (2.3 ml, 10.7 mmol, 2 eq) were added at room temperature and allowed to stir at 90 °C. After 2 hour reaction mixtures was cooled to room temperature and added 4-aminobenzonitrile (0.75 g, 6.42 mmol, 1.2 eq) and allowed to reflux for 16 hour. After completion of reaction cooled to room temperature, add water and extracted with ethyl acetate (3x50 ml). Organic part was collected, dried over Na₂SO₄ and evaporated to dryness to get crude, which was purified by column chromatography to get *trans*-tert-butyl-4-{[(4-cyanophenyl)carbamoyl]amino}-3-(5-methoxypyridin-2-yl)piperidine-1-carboxylate (1.0 g, 41%).
LC-MS (Method 2): m/z [M+H]⁺ = 452.3 (exact mass calc. = 451.22).

### Synthesis of trans-1-(4-cyanophenyl)-3-[3-(5-methoxypyridin-2-yl)piperidin-4-yl]urea trifluoroacetate (BB-XI-TFA)

To the solution of *trans*-tert-butyl-4-{[(4-cyanophenyl)carbamoyl]amino}-3-(5-methoxypyridin-2-yl)piperidine-1-carboxylate (1.0 g, 2.22 mmol, 1 eq) in DCM (20 ml), TFA (4 ml) was added drop wise at 0 °C. The reaction was allowed to stirr at room temperature for 2 hour. After completion of reaction (checked by TLC) DCM and excess TFA was removed under reduced pressure to get crude which was washed with diethyl ether and removed ether under reduced pressure to get *trans*-1-(4-cyanophenyl)-3-[-3-(5-methoxypyridin-2-yl)-piperidin-4-yl]urea trifluoroacetate (**BB-XI-TFA,** 0.75 g, 96%, as mono TFA salt).
LC-MS (Method 2): m/z [M+H]⁺ = 352.3 (exact mass calc. = 351.2).

### Synthesis of trans-1-(5-chloro-1',2',3',4',5',6'-hexahydro-[2,3']bipyridinyl-4'-yl)-3-phenyl-urea (BB-XII-HCl)

### Synthesis of trans-5-chloro-4'-(3-phenyl-ureido)-3',4',5',6'-tetrahydro-2'H-[2,3']bipyridinyl-1'-carboxylic acid tert-butyl ester (G-V)

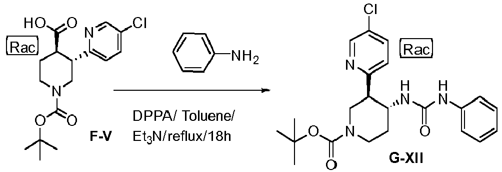

To a stirred solution of *trans*-5-chloro-3',4',5',6'-tetrahydro-2'H-[2,3']bipyridinyl-1',4'-dicarboxylic acid 1'-tert-butyl ester (**F-V,** 300 mg, 0.88 mmol, 1 eq) in toluene (10 ml) was added Et₃N (245 µl, 1.76 mmol, 2.0 eq) followed by the addition of DPPA (250 µl, 1.15 mmol, 1.3 eq) and the reaction mass was stirred at reflux for a period of 2 h, followed by the addition of phenylamine (98 µl, 1.056 mmol, 1.2 eq) and the reaction mixture was heated to reflux for a period of 16 h. The reaction mass was concentrated *in vacuo* and diluted with EtOAc (300 ml) and washed with water and brine. The combined organic layer was dried over anhyd. Na₂SO₄ and concentrated under reduced pressure to get the crude product which was purified by silica gel (100-200 mesh) column chromatography (60% EtOAc in hexane) to obtain desired *trans*-5-chloro-4'-(3-phenyl-ureido)-3',4',5',6'-tetrahydro-2'H-[2,3']bipyridinyl-1'-carboxylic acid tert-butyl ester (**G-XII,** 300 mg, 79% yield) as off white solid.
LC-MS (Method 7): m/z [M+H]⁺ = 431.1 (exact mass calc. = 430.18).

### Synthesis of trans-1-(5-chloro-1',2',3',4',5',6'-hexahydro-[2,3']bipyridinyl-4'-yl)-3-phenyl-urea (BB-XII-HCl)

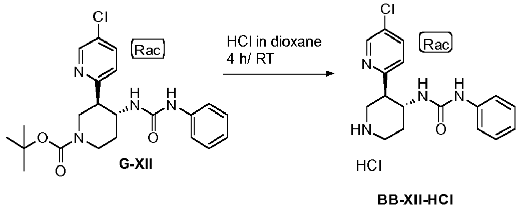

To a stirred solution of *trans*-5-chloro-4'-(3-phenyl-ureido)-3',4',5',6'-tetrahydro-2'H-[2,3']bipyridinyl-1'-carboxylic acid tert-butyl ester (**G-XII** 500 mg,1.16 mmol, 1 eq) in 1,4-dioxane (5 ml) was added 4M HCl in dioxane (5 ml) followed by stirring at RT for a period of 4 h. After completion of the reaction (monitored by TLC and LC-MS), the reaction mixture was concentrated under reduced pressure to get the crude material as HCl salt of *trans*-1-(5-chloro-1',2',3',4',5',6'-hexahydro-[2,3']bipyridinyl-4'-yl)-3-phenyl-urea (**BB-XII-HCl,** 400 mg, 74% crude yield).
LC-MS (Method 1): m/z [M+H]⁺ = 331.0 (exact mass calc. = 330.12).

### Synthesis of trans-1-(4-chloro-phenyl)-3-(3-fluoro-5-methoxy-1',2',3',4',5',6'-hexahydro-[2,3']bipyridinyl-4'-yl)-urea hydrochloride (BB-XIII-HCl)

### Synthesis of 2-chloro-3-fluoro-5-methoxy-pyridine

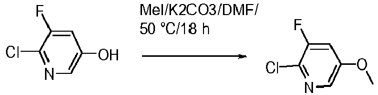

To a stirred solution of 6-chloro-5-fluoro-pyridin-3-ol (500 mg, 3.42 mmol, 1 eq) in dry DMF (10 ml) was added K2CO3 (945 mg, 6.84 mmol, 2 eq) followed by the addition of iodo methane (640 µl, 10.26 mmol, 3.0 eq) and the reaction mixture was heated at reflux for a period of 18 h in a sealed vessel. After completion of the reaction (monitored by TLC and LC-MS), the reaction mixture was diluted with EtOAc (70 ml) and washed the organic layer thoroughly with cold water and brine. The organic layer was dried over anhyd. Na₂SO₄ and concentrated under reduced pressure to obtain crude product which was purified by silica gel (100-200 mesh) column chromatography (10-15% EA/hexane) to afford 2-chloro-3-fluoro-5-methoxy-pyridine (300 mg, 55% yield) as colorless oil.

### Step-3: Synthesis of 3-fluoro-5-methoxy-5',6'-dihydro-2'H-[2,3']bipyridinyl-1',4'-dicarboxylic acid 1'-tert-butyl ester 4'-ethyl ester (D-XIII)

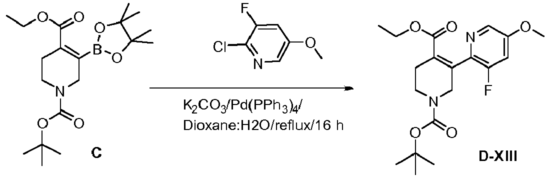

To a stirred solution of 5-(4,4,5,5-tetramethyl-[1,3,2]dioxaborolan-2-yl)-3,6-dihydro-2H-pyridine-1,4-dicarboxylic acid 1-tert-butyl ester 4-ethyl ester (C, 500 mg, 1.36 mmol, 1 eq) and 2-chloro-3-fluoro-5-methoxy-pyridine (253 mg, 1.57 mmol, 1.2 eq) in dioxane:Water (5:1, 12 ml) at RT was added K₂CO₃ (453 mg, 3.28 mmol, 2.5 eq) and the reaction mixture was degassed with argon for a period of 15 minutes followed by the addition of Pd(PPh₃)₄ (120 mg, 0.105 mmol, 0.08 eq) and stirred under reflux for 16 h. After completion of the reaction (monitored by TLC and LC-MS), the reaction mixture was diluted with EA (600 ml) and washed with aq. NaHCO₃ solution and water. The organic layer was dried over anhyd. Na₂SO₄ and concentrated under reduced pressure to obtain crude product which was purified by silica gel (100-200 mesh) column chromatography (15% EA/hexane) to afford 3-fluoro-5-methoxy-5',6'-dihydro-2'H-[2,3']bipyridinyl-1',4'-dicarboxylic acid 1'-tert-butyl ester 4'-ethyl ester (**D-XIII,** 350 mg, 68% yield) as gray gummy material. LC-MS (Method 2): m/z [M+H]⁺ = 381.3 (exact mass calc. = 380.17).

### Synthesis of 3-fluoro-5-methoxy-3',4',5',6'-tetrahydro-2'H-[2,3']bipyridinyl-1',4'-dicarboxylic acid 1'-tert-butyl ester 4'-ethyl ester (E-XIII):

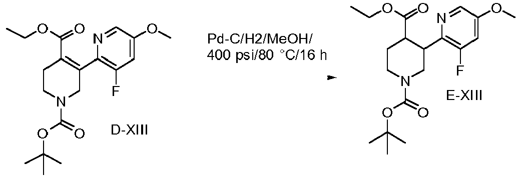

A stirred solution of 3-fluoro-5-methoxy-5',6'-dihydro-2'H-[2,3']bipyridinyl-1',4'-dicarboxylic acid 1'-tert-butyl ester 4'-ethyl ester (**D-XIII,** 500 mg, 1.36 mmol, 1 eq) in MeOH (50 ml) was degassed with argon for a period of 15 min followed by the addition of 10% moist Pd-C (200 mg) and stirred at RT under H₂ pressure (28 bar) for a period of 16 h. After completion of the reaction, it was filtered through celite bed and washed thoroughly with 5% MeOH/DCM. The solvent was evaporated to get the desired 3-fluoro-5-methoxy-3',4',5',6'-tetrahydro-2'H-[2,3']bipyridinyl-1',4'-dicarboxylic acid 1'-tert-butyl ester 4'-ethyl ester (**E-XIII,** 350 mg, 87% crude yield) as gray sticky solid. LC-MS (Method 3): m/z [M+H]⁺ = 383.3 (exact mass calc. = 382.19).

### Synthesis of trans-3-fluoro-5-methoxy-3',4',5',6'-tetrahydro-2'H-[2,3']bipyridinyl-1',4'-dicarboxylic acid 1'-tert-butyl ester (F-XIII)

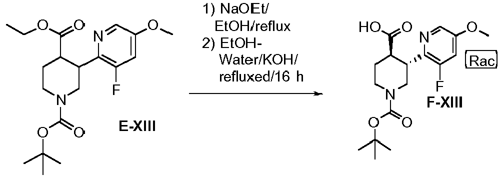

To a stirred solution of 3-fluoro-5-methoxy-3',4',5',6'-tetrahydro-2'H-[2,3']bipyridinyl-1',4'-dicarboxylic acid 1'-tert-butyl ester 4'-ethyl ester (**E-XIII,** 350 mg, 0.916 mmol, 1 eq) in EtOH (10 ml) was added 21% NaOEt in EtOH (310 µl, 0.962 mmol, 1.05 eq) and refluxed for a period of 6 h. After completion of the reaction, it was concentrated under reduced pressure and diluted with aq. NH₄Cl solution and the organics were extracted with DCM. The solvent was evaporated to get the desired *trans*-3-fluoro-5-methoxy-3',4',5',6'-tetrahydro-2'H-[2,3']bipyridinyl-1',4'-dicarboxylic acid 1'-tert-butyl ester 4'-ethyl ester (350 mg, 100% crude yield) as gray sticky solid, which was dissolved in EtOH (10 ml), MeOH (2 ml) and H2O (2 ml) was added KOH (103 mg, 1.83 mmol, 2.0 eq) was refluxed for a period of 16 h. After completion of the reaction, it was concentrated under reduced pressure and diluted with water and extracted with ethyl acetate. Then the aqs part was acidified by IN HCl and extracted with 5% MeOH in DCM. The combined organic layer was dried over anhyd. Na2SO4 and concentrated to get the desired *trans*-3-fluoro-5-methoxy-3',4',5',6'-tetrahydro-2'H-[2,3']bipyridinyl-1',4'-dicarboxylic acid 1'-tert-butyl ester (**F-XIII,** 220 mg, 68% crude yield) as light yellowish sticky solid. LC-MS (Method 1): m/z [M+H]⁺ = 355.0 (exact mass calc. = 354.16).

### Synthesis of trans-4'-[3-(4-Chloro-phenyl)-ureido]-3-fluoro-5-methoxy-3',4',5',6'-tetrahydro-2'H-[2,3']bipyridinyl-1'-carboxylic acid tert-butyl ester (G-XIII)

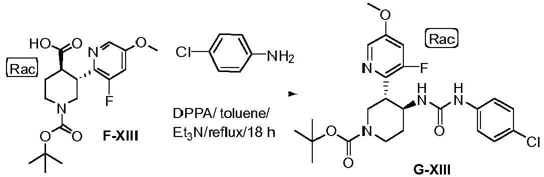

To a stirred solution of *trans*-3-fluoro-5-methoxy-3',4',5',6'-tetrahydro-2'H-[2,3']bipyridinyl-1',4'-dicarboxylic acid 1'-tert-butyl ester (**F-XIII,** 200 mg, 0.565 mmol, 1 eq) in toluene (10 ml) was added Et₃N (157 µl, 1.13 mmol, 2.0 eq) followed by the addition of DPPA (157 µl, 1.13 mmol, 2.0 eq) and the reaction mass was refluxed for a period of 2 h. The reaction was cooled to RT and 4-chloro-phenylamine (87 mg, 0.678 mmol, 1.2 eq) was added and the reaction mixture was heated at 120 °C for a period of 18 h. The reaction mass was concentrated *in vacuo* and diluted with EA (300 ml) and washed with water and brine. The combined organic layer was dried over anhyd. Na₂SO₄ and concentrated under reduced pressure to get the crude product which was purified by silica gel (100-200 mesh) column chromatography (25% EA in hexane) to obtain *trans*-4'-[3-(4-chloro-phenyl)-ureido]-3-fluoro-5-methoxy-3',4',5',6'-tetrahydro-2'H-[2,3']bipyridinyl-1'-carboxylic acid tert-butyl ester (**G-XIII,** 100 mg, 37%) as gray sticky solid. LC-MS (Method 1): m/z [M+H]⁺ = 479.2 (exact mass calc. = 478.18).

### Synthesis of trans-1-(4-chloro-phenyl)-3-(3-fluoro-5-methoxy-1',2',3',4',5',6'-hexahydro-[2,3']bipyridinyl-4'-yl)-urea hydrochloride (BB-XIII-HCl)

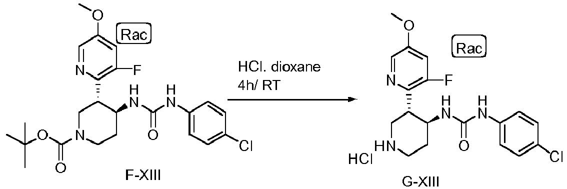

To a stirred solution of *trans*-4'-[3-(4-chloro-phenyl)-ureido]-3-fluoro-5-methoxy-3',4',5',6'-tetrahydro-2'H-[2,3']bipyridinyl-1'-carboxylic acid tert-butyl ester (**G-XIII,** 100 mg, 0.209 mmol, 1 eq) in 1,4-dioxane (5 ml) was added 4M HCl in dioxane (1 ml) followed by stirring at RT for a period of 4 h at room temperature. After completion of the reaction (monitored by TLC and LC-MS), the reaction mixture was concentrated under reduced pressure to get the crude material as HCl salt of *trans*-1-(4-chloro-phenyl)-3-(3-fluoro-5-methoxy-1',2',3',4',5',6'-hexahydro-[2,3']bipyridinyl-4'-yl)-urea (**BB-XIII-HCl,** 80 mg, 92% crude yield) as brown solid.
LC-MS (Method 2): m/z [M+H]⁺ = 379.3 (exact mass calc. = 378.13).

### Synthesis of final compounds (piperidines)

### Synthesis of trans-1-(4-chloro-phenyl)-3-(6'-methoxy-1-pyrimidin-4-yl-1,2,3,4,5,6-hexahydro-[3,3']bipyridinyl-4-yl)-urea (Ex-1)

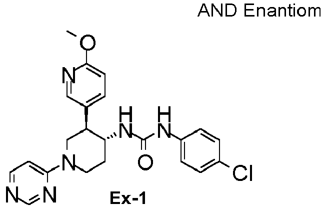

To a stirred solution of *trans*-1-(4-chloro-phenyl)-3-(6'-methoxy-1,2,3,4,5,6-hexahydro-[3,3']bipyridinyl-4-yl)-urea hydrochloride (**BB-I-HCl,** 100 mg, 0.252 mmol, 1 eq) and 4-iodo-pyrimidine (328 mg, 2.34 mmol, 3 eq), in DMSO (5 ml) was added K₂CO₃ (157 mg, 1.14 mmol, 4.5 eq) and degassed with argon for a period of 15 min followed by the addition of L-proline (1.5 mg, 0.012 mmol, 0.1 eq) and CuI (2.5 mg, 0.012 mmol, 0.1 eq) and the reaction mixture was allowed to stir at 90 °C for 18 h. After completion of the reaction, it was diluted with water and the organic components were extracted with EtOAc and the organic layer was dried over anhyd. Na₂SO₄, filtered and evaporated to get the crude product. The crude material was purified by column chromatography followed by prep TLC to obtain *trans*-1-(4-chloro-phenyl)-3-(6'-methoxy-1-pyrimidin-4-yl-1,2,3,4,5,6-hexahydro-[3,3']bipyridinyl-4-yl)-urea (**Ex-1**, 35 mg, yield 14%) as off white solid.
LC-MS (Method 1): m/z [M+H]⁺ = 439.1 (exact mass calc. = 438.16).
¹H-NMR (400 MHz, DMSO-d6): □ = 8.50 (s, 1 H), 8.38 (s, 1 H), 8.17 (d, J = 6 Hz, 1 H), 8.09 (s, 1 H), 7.72-7.70 (m, 1 H), 7.29 (d, J = 9 Hz, 2 H), 7.19 (d, J = 9 Hz, 2 H), 6.92 (d, J = 6 Hz, 1 H), 6.78 (d, J = 8 Hz, 1 H), 6.03 (d, J = 9 Hz, 1 H), 4.48-4.42 (m, 1 H), 4.39-4.37 (m, 1 H), 4.12-4.09 (m, 1 H), 3.80 (s, 3 H), 3.10 (t, J = 12 Hz, 2 H), 2.72-2.66 (m, 1 H), 2.07-2.04 (m, 1 H), 1.47-1.38 (m, 1 H).

### Synthesis of trans-1-(4-chloro-phenyl)-3-[1-(4-fluoro-phenyl)-6'-methoxy-1,2,3,4,5,6-hexahydro-[3,3']bipyridinyl-4-yl]-urea (Ex-2)

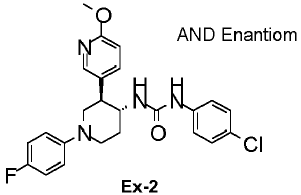

To a stirred solution of *trans*-1-(4-chloro-phenyl)-3-(6'-methoxy-1,2,3,4,5,6-hexahydro-[3,3']bipyridinyl-4-yl)-urea (**BB-I-HCl,** 300 mg, 0.76 mmol, 1 eq), 4-fluoro phenyl boronic acid (318 mg, 2.27 mmol, 3 eq), molecular sieves (150 mg) and TEA (0.53 ml, 3.78 mmol, 5 eq) in DCM (25 ml) was added Cu(OAc)₂ (344 mg, 1.89 mmol, 2.5 eq) and stirred at RT under oxygen atmosphere for a period of 24 h. After completion of the reaction, it was filtered and washed thoroughly with 5% MeOH-DCM and the organic layer was concentrated in vacuo to get crude product. The crude was purified by column chromatography followed by prep TLC to obtain *trans*-1-(4-chlorophenyl)-3-[1-(4-fluoro-phenyl)-6'-methoxy-1,2,3,4,5,6-hexahydro-[3,3']bipyridinyl-4-yl]-urea (**Ex-2**, 47 mg, yield 14%) as off white solid.
LC-MS (Method 1): m/z [M+H]⁺ = 455.4 (exact mass calc. = 454.16).
¹H-NMR (400 MHz, DMSO-d6): □ = 8.33 (s, 1 H), 8.11 (d, J = 2 Hz, 1 H), 7.71-7.68 (m, 1 H), 7.30 (d, J = 9 Hz, 2 H), 7.20 (d, J = 9 Hz, 2 H), 7.05-6.96 (m, 4 H), 6.76 (d, J = 9 Hz, 1 H), 6.08 (d, J = 8 Hz, 1 H), 4.04-3.95 (m, 1 H), 3.80 (s, 3 H), 3.69 (d, J = 13 Hz, 1 H), 3.59 (d, J = 8 Hz, 1 H), 2.89-2.84 (m, 3 H), 2.07 (d, J = 10 Hz, 1 H), 1.61-1.58 (m, 1H).

### Synthesis of trans-1-[1-(4-fluoro-phenyl)-6'-methoxy-1,2,3,4,5,6-hexahydro-[3,3']bipyridinyl-4-yl]-3-(3-methyl-isothiazol-5-yl)-urea (Ex-3)

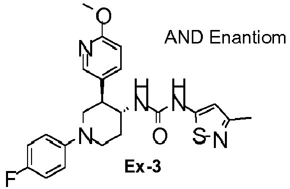

To a stirred solution of *trans*-1-(6'-methoxy-1,2,3,4,5,6-hexahydro-[3,3']bipyridinyl-4-yl)-3-(3-methyl-isothiazol-5-yl)-urea hydorchloride (**BB-II-HCl,** 300 mg, 0.78 mmol, 1 eq), 4-fluoro phenyl boronic acid (328 mg, 2.34 mmol, 3 eq), molecular sieves (100 mg), TEA (0.55 ml, 3.92 mmol, 5 eq) in DCM (30 ml) was added Cu(OAc)₂ (355 mg, 1.95 mmol, 2.5 eq) and stirred at RT under oxygen atmosphere for a period of 24 h. After completion of the reaction, it was filtered and washed thoroughly with 5% MeOH-DCM and the organic layer was concentrated and purified by column chromatography followed by prep TLC to obtain *trans*-1-[1-(4-fluoro-phenyl)-6'-methoxy-1,2,3,4,5,6-hexahydro-[3,3']bipyridinyl-4-yl]-3-(3-methyl-isothiazol-5-yl)-urea (Cpd-621, 30 mg, yield 14%) as off white solid.
LC-MS (Method 3): m/z [M+H]⁺ = 442.1 (exact mass calc. = 441.16).
¹H-NMR (400 MHz, DMSO-d6): □ = 9.91 (s, 1 H), 8.09 (s, 1 H), 7.68 (d, J = 9 Hz, 1 H), 7.05-6.99 (m, 4 H), 6.74 (d, J = 9 Hz, 1 H), 6.60-6.59 (m, 1 H), 6.40 (s, 1 H), 3.96-3.94 (m, 1 H), 3.80 (s, 3 H), 3.71 (d, J = 12 Hz, 1 H), 3.60 (d, J = 10 Hz, 1 H), 2.93-2.84 (m, 3 H), 2.19 (s, 3 H), 2.08-1.98 (m, 1 H), 1.70-1.62 (m, 1 H).

### Synthesis of trans-1-(6'-methoxy-1-pyrimidin-4-yl-1,2,3,4,5,6-hexahydro-[3,3']bipyridinyl-4-yl)-3-(3-methyl-isothiazol-5-yl)-urea (Ex-4)

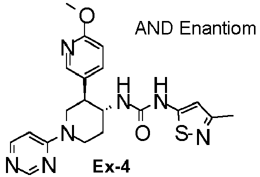

To a stirred solution of *trans*-1-(6'-methoxy-1,2,3,4,5,6-hexahydro-[3,3']bipyridinyl-4-yl)-3-(3-methyl-isothiazol-5-yl)-urea hydrochloride (**BB-II-HCl,** 150 mg, 0.391 mmol, 1 eq) and 4-iodo-pyrimidine (157 mg, 0.469 mmol, 1.2 eq), in DMSO (10 ml) was added K₂CO₃ (243 mg, 1.76 mmol, 4.5 eq) and degassed with argon for a period of 15 min followed by the addition of L-proline (4.5 mg, 0.04 mmol, 0.1 eq) and CuI (7.4 mg, 0.039 mmol, 0.1 eq) and the reaction mixture was allowed to stir at 90 °C for 18 h. After completion of the reaction, it was diluted with water and the organic components were extracted with EtOAc and the organic layer was dried over anhyd. Na₂SO₄, filtered and evaporated under reduced pressure to get the crude compound. The crude material was purified by column chromatography followed by prep TLC to obtain *trans*-1-(6'-methoxy-1-pyrimidin-4-yl-1,2,3,4,5,6-hexahydro-[3,3']bipyridinyl-4-yl)-3-(3-methyl-isothiazol-5-yl)-urea (**Ex-4**, 25 mg, yield 15%) as off white solid. LC-MS (Method 1): m/z [M+H]⁺ = 426.1 (exact mass calc. = 425.16).
¹H-NMR (400 MHz, DMSO-d6): □ = 9.96 (s, 1 H), 8.50 (s, 1 H), 8.18 (d, J = 6 Hz, 1 H), 8.08 (d, J = 2 Hz, 1 H), 7.70-7.68 (m, 1 H), 6.93 (d, J = 6 Hz, 1 H), 6.77 (d, J = 8 Hz, 1 H), 6.52 (d, J = 9 Hz, 1 H), 6.38 (s, 1 H), 4.56-4.52 (m, 1 H), 4.48-4.41 (m, 1 H), 4.12-4.06 (m, 1 H), 3.80 (s, 3 H), 3.12 (t, J = 12 Hz, 2 H), 2.76-2.71 (m, 1 H), 2.19 (s, 3 H), 2.07-2.02 (m, 1 H), 1.51-1.49 (m, 1 H).

### Synthesis trans-1-(4-chloro-phenyl)-3-(5-methoxy-1'-pyrimidin-4-yl-1',2',3',4',5',6'-hexahydro-[2,3']bipyridinyl-4'-yl)-urea a (Ex-7)

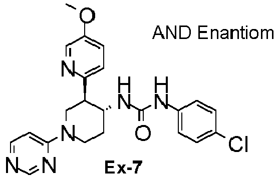

To a solution of *trans*-1-(4-chloro-phenyl)-3-(5-methoxy-1',2',3',4',5',6'-hexahydro-[2,3']bipyridinyl-4'-yl)-urea hydrochloride (**BB-III-HCl,** 50 mg, 0.12 mmol, 1 eq) and 4-iodopyrimidine (50 mg, 0.15 mmol, 1.2 eq) in dry DMSO (5 ml) was added K₂CO₃ (78 mg, 0.56 mmol, 4.5 eq) followed by the addition of L-proline (1.4 mg, 0.01 mmol, 0.1 eq) and CuI (2.4 mg, 0.12 mmol, 0.1 eq) and the reaction mixture was heated to 100 °C for 16h. The reaction mixture was cooled to ambient temperature, diluted with water (10 ml) and the organic components were extracted with ethyl acetate (2 x 40 ml). The organic layer was separated, dried over anhyd. Na₂SO₄ and concentrated under reduced pressure to obtain crude compound which was purified by preparative TLC (5% MeOH in DCM as eluent) to get *trans*-1-(4-chloro-phenyl)-3-(5-methoxy-1'-pyrimidin-4-yl-1',2',3',4',5',6'-hexahydro-[2,3']bipyridinyl-4'-yl)-urea (**Ex-7**, 30 mg, 45%) as off white solid.
LC-MS (Method 3): m/z [M+H]⁺ = 439.4 (exact mass calc. = 438.16).
¹H-NMR (400 MHz, DMSO-d6): □ 8.62 (br s, 1 H), 8.49 (s, 1 H), 8.24-8.22 (m, 1 H), 8.16 (d, J = 6 Hz, 1 H), (, H), 7.35-7.30 (m, 4 H), 7.19 (d, J = 9 Hz, 2 H), 6.88 (d, J = 6 Hz, 1 H), 6.33-6.28 (m, 1 H), 4.52-4.42 (m, 2 H), (, H), 4.14 (d, J = 12 Hz, 1 H), 3.79 (s, 3 H), 3.18-3.05 (m, 2 H), 2.84 (t, J = 4 Hz, 1 H), 2.06 (d, J = 9 Hz, 1 H), 1.47-1.38 (m, 1H).

### Synthesis of trans-1-(5-methoxy-1'-pyrimidin-4-yl-1',2',3',4',5',6'-hexahydro-[2,3']bipyridinyl-4'-yl)-3-(3 -methyl-isothiazol-5-yl)-urea (Ex-8)

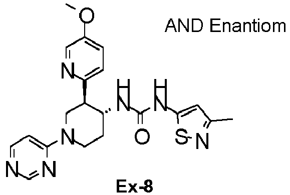

To a solution of HCl salt of *trans-*1-(5-methoxy-1',2',3',4',5',6'-hexahydro-[2,3']bipyridinyl-4'-yl)-3-(3-methyl-isothiazol-5-yl)-urea (**BB-IV-HCl,** 100 mg, 0.26 mmol, 1 eq) and 4-iodopyrimidine (104 mg, 0.31 mmol, 1.2 eq) in dry DMSO (10 ml) was added K₂CO₃ (128 mg, 0.56 mmol, 4.5 eq) followed by the addition of L-proline (3.0 mg, 0.026 mmol, 0.1 eq) and CuI (4.9 mg, 0.026 mmol, 0.1 eq) and the reaction mixture was heated to 100 °C for 16h. The reaction mixture was cooled to ambient temperature, diluted with water (10 ml) and the organic components were extracted with ethyl acetate (2 x 40 ml). The organic layer was separated, dried over anhyd. Na₂SO₄ and concentrated under reduced pressure to obtain crude compound which was purified by preparative TLC (5% MeOH in DCM as eluent) to get *trans*-1-(5-methoxy-1'-pyrimidin-4-yl-1',2',3',4',5',6'-hexahydro-[2,3']bipyridinyl-4'-yl)-3-(3-methyl-isothiazol-5-yl)-urea_(**Ex-8**, 18 mg, 16%) as off white solid.
LC-MS (Method 3): m/z [M+H]⁺ = 426.4 (exact mass calc. = 425.16).
¹H-NMR (400 MHz, DMSO-d6): δ 1.32-1.28 (br m, 1 H), 8.50 (s, 1 H), 8.23 (d, *J* = 3 Hz, 1 H), 8.17 (d*, J* = 6 Hz, 1 H), 7.34-7.31 (m, 1 H), 7.27 (d*, J* = 9 Hz, 1 H), 6.91-6.87 (m, 2 H), 6.37 (s, 1 H), 4.52-4.42 (m, 2 H), 4.18-4.11 (m, 1 H), 3.79 (s, 3 H), 3.19-3.08 (m, 2 H), 2.92-2.83 (m, 1 H), 2.19 (s, 3 H), 2.03 (d, *J* = 10 Hz, 1 H), 1.49-1.42 (m, 1 H).

### Synthesis of trans-1-(4-chloro-phenyl)-3-(5-chloro-1'-pyrimidin-4-yl-1',2',3',4',5',6'-hexahydro-[2,3']bipyridinyl-4'-yl)-urea (Ex-9)

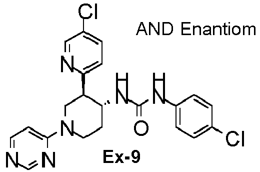

To a solution of TFA salt of of *trans*-1-(5-chloro-1',2',3',4',5',6'-hexahydro-[2,3']bipyridinyl-4'-yl)-3-(4-chlorophenyl)-urea (**BB-V-TFA**, 100 mg, 0.209 mmol, 1 eq) and 4-iodopyrimidine (84 mg, 0.251 mmol, 1.2 eq) in dry DMSO (5 ml) was added K₂CO₃ (130 mg, 0.94 mmol, 4.5 eq) followed by the addition of L-proline (4 mg, 0.029 mmol, 0.1 eq) and CuI (6 mg, 0.029 mmol, 0.1 eq) and the reaction mixture was heated to 100 °C for 16h. The reaction mixture was cooled to ambient temperature, diluted with water (10 ml) and the organic components were extracted with ethyl acetate (2 x 40 ml). The organic layer was separated, dried over anhyd. Na₂SO₄ and concentrated under reduced pressure to obtain crude compound which was purified by preparative TLC using 8% MeOH in DCM as eluent, to get *trans*--(4-chloro-phenyl)-3-(5-chloro-1'-pyrimidin-4-yl-1',2',3',4',5',6'-hexahydro-[2,3']bipyridinyl-4'-yl)-urea (**Ex-9**, 38 mg, 41%) as off white solid.
LC-MS (Method 2): m/z [M+H]⁺ = 443.1 (exact mass calc. = 442.11).
¹H-NMR (400 MHz, DMSO-d6): δ 8.58 (d*, J* = 2 Hz, 1 H), (, H), 8.50 (s, 1 H), 8.42 (s, 1 H), 8.18 (d*, J* = 6 Hz, 1 H), 7.90-7.87 (m, 1 H), 7.41 (d, *J* = 8 Hz, 1 H), 7.28 (d*, J* = 9 Hz, 2 H), 7.19 (d*, J* = 9 Hz, 2 H), 6.91 (d, *J* = 6 Hz, 1 H), 6.14 (d, *J* = 8 Hz, 1 H), 4.55-4.52 (m, 2 H), 4.17 (d, *J* = 12 Hz, 1 H), 3.31-3.08 (m, 2 H), 2.94-2.88 (m, 1 H), 2.04 (d*, J* = 9 Hz, 1 H), 1.48-1.44 (m, 1 H).

### Synthesis of trans-1-[5-chloro-1'-(4-fluoro-phenyl)-1',2',3',4',5',6'-hexahydro-[2,3']bipyridinyl-4'-yl]-3-(4-chlorophenyl)-urea (Ex-12)

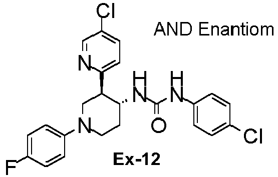

To a stirred solution *trans*-1-(5-chloro-1',2',3',4',5',6'-hexahydro-[2,3']bipyridinyl-4'-yl)-3-(4-chloro-phenyl)-urea triflate (**BB-V-TFA,** 300 mg, 0.627 mmol, 1 eq), 4-fluoro phenyl boronic acid (262 mg, 1.88 mmol, 3 eq), molecular sieves (300 mg) and TEA (420 µl, 3.135 mmol, 5 eq) in DCM (30 ml) was added Cu(OAc)₂ (285 mg, 1.57 mmol, 2.5 eq) and stirred at RT under oxygen atmosphere for a period of 3 days. After completion of the reaction, it was filtered and washed thoroughly with 5% MeOH-DCM, the filtrate was evaporated under reduced pressure and purified by silica gel (100-200 mesh) column chromatography followed by prep TLC purification to obtain *trans-*1-[5-chloro-1'-(4-fluoro-phenyl)-1',2',3',4',5',6'-hexahydro-[2,3']bipyridinyl-4'-yl]-3-(4-chlorophenyl)-urea (**Ex-12,** 28 mg, yield 10%) as off white solid.
LC-MS (Method 2): m/z [M+H]⁺ = 459.2 (exact mass calc. = 458.11).
¹H-NMR (400 MHz, DMSO-d6): δ 8.56 (s, 1 H), 8.40 (s, 1 H), 7.86 (d, *J* = 8 Hz, 1 H), 7.42 (d, *J* = 9 Hz, 1 H), 7.29 (d, *J* = 9 Hz, 2 H), 7.19 (d*, J* = 9 Hz, 2 H), 7.05-6.98 (m, 4 H), 6.21 (d, *J* = 8 Hz, 1 H), 4.11-3.99 (m, 1 H), 3.70 (t*, J* = 13 Hz, 2 H), 3.07-3.00 (m, 2 H), 2.90 (t*, J* = 13 Hz, 1 H), 2.03 (d*, J* = 12 Hz, 1 H), 1.67-1.58 (m, 1 H).

### Synthesis of trans-1-(5-chloro-1'-pyrimidin-4-yl-1',2',3',4',5',6'-hexahydro-[2,3']bipyridinyl-4'-yl)-3-(3-methyl-isothiazol-5-yl)-urea (Ex-13)

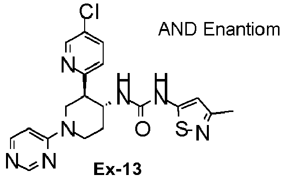

To a stirred solution of *trans*-1-(5-chloro-1',2',3',4',5',6'-hexahydro-[2,3']bipyridinyl-4'-yl)-3-(3-methyl-isothiazol-5-yl)-urea (**BB-VI-HCl,** 150 mg, 0.427 mmol, 1 eq) and 4-iodopyrimidine (170 mg, 0.512 mmol, 1.2 eq) in dry DMSO (10 ml) was added K₂CO₃ (265 mg, 1.92 mmol, 4.5 eq) followed by the addition of L-proline (5 mg, 0.042 mmol, 0.1 eq) and CuI (8 mg, 0.042 mmol, 0.1 eq) and the reaction mixture was heated to 100 °C for 16h. The reaction mixture was cooled to ambient temperature, diluted with water (10 ml) and the organic components were extracted with ethyl acetate (2 x 40 ml). The organic layer was separated, dried over anhyd. Na₂SO₄ and concentrated under reduced pressure to obtain crude compound which was purified by preparative TLC using 8% MeOH in DCM as eluent, to get *trans*-1-(5-chloro-1'-pyrimidin-4-yl-1',2',3',4',5',6'-hexahydro-[2,3']bipyridinyl-4'-yl)-3-(3-methyl-isothiazol-5-yl)-urea (**Ex-13,** 28 mg, 15%) as off white solid.
LC-MS (Method 3): m/z [M+H]⁺ = 430.0 (exact mass calc. = 429.11).
¹H-NMR (400 MHz, DMSO-d6): δ 10.12 (s, 1 H), 8.58 (s, 1 H), 8.50 (s, 1 H), 8.18 (d, *J* = 6 Hz, 1 H), 7.88-7.85 (m, 1 H), 7.38 (d, *J* = 8 Hz, 1 H), 6.92 (d, *J =* 6 Hz, 1 H), 6.71-6.66 (m, 1 H), 6.38 (s, 1 H), 4.52-4.42 (m, 2 H), 4.18-4.11 (m, 1 H), 3.30-3.10 (m, 2 H), 3.00-2.98 (m, 1 H), 2.19 (s, 3 H), 2.02 (d, *J* = 11 Hz, 1 H), 1.52-1.48 (m, 1H).
Chiral SFC afforded the two enantiomers **Ex-16** (enantiomer 1; first eluting) and Ex-17 (enantiomer 2, second eluting).

### Synthesis of trans-1-(4-chloro-phenyl)-3-[1'-(4-fluoro-phenyl)-5-methoxy-1',2',3',4',5',6'-hexahydro-[2,3]bipyridinyl-4'-yl]-urea (Ex-18)

### Synthesis of trans-5-methoxy-3',4',5',6'-tetrahydro-2'H-[2,3']bipyridinyl-1',4'-dicarboxylic acid 1'-tert-butyl ester 4'-methyl ester

To a stirred solution of 5-methoxy-3',4',5',6'-tetrahydro-2'H-[2,3']bipyridinyl-1',4'-dicarboxylic acid 1'-tert-butyl ester (**F-I,** 1 g, 2.98 mmol, 1 eq) in ACN (20 ml) at RT was added K₂CO₃ (1.2 g, 8.92 mmol, 3 eq) followed by the addition of MeI (370 µl, 5.95 mmol, 2.0 eq) and the reaction mixture was stirred at ambient temperature for 16 h. After completion of the reaction (monitored by TLC and LC-MS), the reaction mixture was concentrated *in vacuo* and the residue was diluted with EtOAc (600 ml) and washed with aq. NaHCO₃ solution and water. The organic layer was dried over anhyd. Na₂SO₄ and concentrated under reduced pressure to get the crude product, which was purified by silica gel (100-200 mesh) column chromatography (10-15% EtOAc/ Hexane) to afford *trans*-5-methoxy-3',4',5',6'-tetrahydro-2'H-[2,3']bipyridinyl-1',4'-dicarboxylic acid 1'-tert-butyl ester 4'-methyl ester (700 mg, 67% yield) as yellow sticky solid.
LC-MS (Method 2): m/z [M+H]⁺ = 351.3 (exact mass calc. = 350.18).

### Synthesis of trans-5-methoxy-1',2',3',4',5',6'-hexahydro-[2,3']bipyridinyl-4'-carboxylic acid methyl ester

To a stirred solution of *trans*-5-methoxy-3',4',5',6'-tetrahydro-2'H-[2,3']bipyridinyl-1',4'-dicarboxylic acid 1'-tert-butyl ester 4'-methyl ester 700 mg, 2.00 mmol, 1 eq) in 1,4-dioxane (15 ml) was added 4M HCl in dioxane (8 ml) followed by stirring at RT for a period of 3 h. After completion of the reaction (monitored by TLC and LC-MS), the reaction mixture was concentrated under reduced pressure to get the HCl salt of *trans*-5-methoxy-1',2',3',4',5',6'-hexahydro-[2,3']bipyridinyl-4'-carboxylic acid methyl ester (560 mg, 98% crude yield) as yellow sticky solid.
LC-MS (Method 1): m/z [M+H]⁺ = 251.0 (exact mass calc. = 250.13).

### Synthesis of trans-1'-(4-fluoro-phenyl)-5-methoxy-1',2',3',4',5',6'-hexahydro-[2,3']bipyridinyl-4'-carboxylic acid methyl ester

To a stirred solution of HCl salt of *trans*-5-methoxy-1',2',3',4',5',6'-hexahydro-[2,3']bipyridinyl-4'-carboxylic acid methyl ester (200 mg, 0.80 mmol, 1 eq), 4-fluoro phenyl boronic acid (444 mg, 3.20 mmol, 4 eq), molecular sieves (200 mg) and TEA (557 µl, 4.0 mmol, 5 eq) in DCM (10 ml) was added Cu(OAc)₂ (363 mg, 1.99 mmol, 2.5 eq) and stirred at RT under oxygen atmosphere for a period of 3 days. After completion of the reaction, it was filtered and washed thoroughly with 5% MeOH-DCM and the filtrate was evaporated under reduced pressure and purified by silica gel (100-200 mesh) column chromatography (10-15% EtOAc in Hexane) to obtain *trans*-1'-(4-fluorophenyl)-5-methoxy-1',2',3',4',5',6'-hexahydro-[2,3']bipyridinyl-4'-carboxylic acid methyl ester (50 mg, yield 18%) as yellow oil.
LC-MS (Method 2): m/z [M+H]⁺ = 344.7 (exact mass calc. = 344.15).

### Synthesis of trans-1'-(4-fluoro-phenyl)-5-methoxy-1',2',3',4',5',6'-hexahydro-[2,3']bipyridinyl-4'-carboxylic acid

To a stirred solution of *trans*-1'-(4-fluoro-phenyl)-5-methoxy-1',2',3',4',5',6'-hexahydro-[2,3']bipyridinyl-4'-carboxylic acid methyl ester (150 mg, 0.436 mmol, 1 eq) in THF-Water (2:1, 18 ml) was added KOH (73 mg, 1.31 mmol, 3.0 eq) and refluxed for a period of 3 h. After completion of the reaction, it was concentrated under reduced pressure and diluted with water and washed with ethyl acetate. The aqueous part was acidified with IN aq. HCl and the organic components were extracted with 10% MeOH in DCM. The combined organic layer was dried over anhyd. Na₂SO₄ and concentrated *in vacuo* to obtain *trans*-1'-(4-fluoro-phenyl)-5-methoxy-1',2',3',4',5',6'-hexahydro-[2,3']bipyridinyl-4'-carboxylic acid (90 mg, 62% yield) as pale yellow solid.
LC-MS (Method 2): m/z [M+H]⁺ = 330.9 (exact mass calc. = 330.14).

### Synthesis of trans-1-(4-chloro-phenyl)-3-[1'-(4-fluoro-phenyl)-5-methoxy-1',2',3',4',5',6'-hexahydro-[2,3']bipyridinyl-4'-yl]-urea (Ex-18)

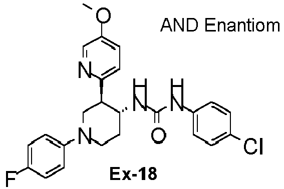

To a stirred solution of *trans*-1'-(4-fluoro-phenyl)-5-methoxy-1',2',3',4',5',6'-hexahydro-[2,3']bipyridinyl-4'-carboxylic acid (90 mg, 0.272 mmol, 1eq) in Benzene:THF (1:2, 21 ml) was added Et₃N (75 µl, 0.545 mmol, 2.0 eq) followed by the addition of DPPA (76 µl, 0.354 mmol, 1.3 eq) and the reaction mass was stirred at RT for a period of 2 h, followed by the addition of 4-chloro phenylamine (41 mg, 0.327 mmol, 1.2 eq) and the reaction mixture was heated to reflux for a period of 16 h. The reaction mass was concentrated *in vacuo* and diluted with water (20 ml) and extracted with 10% MeOH-DCM (2 x 50 ml) and the organic layer was washed with water and brine. The combined organic layer was dried over anhyd. Na₂SO₄ and concentrated under reduced pressure to get the crude product which was purified by silica gel (100-200 mesh) column chromatography (eluted with 2-3% MeOH in DCM) to obtain desired product which was repurified by trituration with DCM and diethyl ether to obtain pure *trans*-1-(4-chloro-phenyl)-3-[1'-(4-fluoro-phenyl)-5-methoxy-1',2',3',4',5',6'-hexahydro-[2,3']bipyridinyl-4'-yl]-urea (**Ex-18,** 18 mg, 14% yield) as off white solid. LC-MS (Method 2): m/z [M+H]⁺ = 454.8 (exact mass calc. = 454.16).
¹H-NMR (400 MHz, DMSO-d6): δ 8.43-7.96 (br m, 1 H), 8.22 (s, 1 H), 7.33 (d, *J* = 11 Hz, 4 H), 7.19 (d, *J* = 9 Hz, 2 H), 7.05-7.00 (m, 2 H), 6.98-6.94 (m, 2 H), 6.18-6.13 (br m, 1 H), 4.02 (d, *J* = 8 Hz, 1 H), 3.78 (s, 3 H), 3.76 (d, *J* = 13 Hz, 1 H), 3.67 (d, *J* = 14 Hz, 1 H), 2.99-2.85 (m, 3 H), 2.06 (d, *J* = 11 Hz, 1 H), 1.62-1.52 (m, 1 H).

### Synthesis of trans-1-(5-chloro-1'-pyrazin-2-yl-1',2',3',4',5',6'-hexahydro-[2,3']bipyridinyl-4'-yl)-3-(3-methyl-isothiazol-5-yl)-urea (Ex-19)

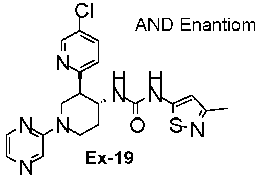

To a stirred solution of *trans*-1-(5-chloro-1',2',3',4',5',6'-hexahydro-[2,3']bipyridinyl-4'-yl)-3-(3-methyl-isothiazol-5-yl)-urea hydrochloride (**BB-VI-HCl,** 150 mg, 0.39 mmol, 1 eq) and 2-iodo-pyrazine (int-14, 104 mg, 0.503 mmol, 1.3 eq) in dry DMSO (6 ml) was added K₃PO₄ (246 mg, 1.16 mmol, 3.0 eq) followed by the addition of L-proline (9 mg, 0.077 mmol, 0.2 eq) and CuI (7 mg, 0.038 mmol, 0.1 eq) and the reaction mixture was heated to 90 °C for 16 h. The reaction mixture was cooled to ambient temperature, diluted with water (10 ml) and the organic components were extracted with ethyl acetate (2 x 40 ml). The organic layer was separated, dried over anhyd. Na₂SO₄ and concentrated under reduced pressure to obtain crude compound which was purified by preparative TLC using 2% MeOH in DCM as eluent, to get *trans*-1-(5-chloro-1'-pyrazin-2-yl-1',2',3',4',5',6'-hexahydro-[2,3']bipyridinyl-4'-yl)-3-(3-methyl-isothiazol-5-yl)-urea (**Ex-19,** 26 mg, 16%) as off white solid.
LC-MS (Method 1): m/z [M+H]⁺ = 430.2 (exact mass calc. = 429.11).
¹H-NMR (400 MHz, DMSO-d6): δ 10.23-10.01 (br s, 1 H), 8.57 (d, *J* = 2 Hz, 1 H), 8.40 (s, 1 H), 8.09 (s, 1 H), 7.88-7.82 (m, 2 H), 7.39 (d, *J* = 8 Hz, 1 H), 6.84-6.78 (m, 1 H), 6.38 (s, 1 H), 4.47 (d, *J* = 9 Hz, 2 H), 4.17 (d, *J* = 12 Hz, 1 H), 3.22-2.91 (m, 3 H), 2.19 (s, 3 H), 2.02 (d, *J* = 12 Hz, 1 H)1.54 (d, *J* = 9 Hz, 1 H).

### Synthesis of trans-1-[1'-(4-fluoro-phenyl)-5-methoxy-1',2',3',4',5',6'-hexahydro-[2,3']bipyridinyl-4'-yl]-3-(3-methyl-isothiazol-5-yl)-urea (Ex-20)

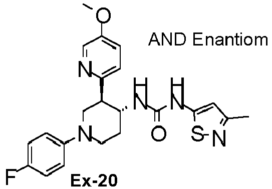

To a stirred solution of *trans*-1'-(4-fluoro-phenyl)-5-methoxy-1',2',3',4',5',6'-hexahydro-[2,3']bipyridinyl-4'-carboxylic acid (see: **Ex-18,** 100 mg, 0.303 mmol, 1eq) in toluene (20 ml) was added Et₃N (84 µl, 0.606 mmol, 2.0 eq) followed by the addition of DPPA (85 µl, 0.393 mmol, 1.3 eq) and the reaction mass was stirred under reflux for a period of 2 h, followed by the addition of 3-methyl-isothiazol-5-ylamine (69 mg, 0.606 mmol, 2 eq) and the reaction mixture was heated to reflux for a period of 18 h. The reaction mass was concentrated *in vacuo* and diluted with water (20 ml) and extracted with 10% MeOH-DCM (2 x 50 ml) and the organic layer was washed with water and brine. The combined organic layer was dried over anhyd. Na₂SO₄ and concentrated under reduced pressure to get the crude product which was purified by prep TLC plate (eluted with 3% MeOH in DCM) to obtain 1-[1'-(4-fluoro-phenyl)-5-methoxy-1',2',3',4',5',6'-hexahydro-[2,3']bipyridinyl-4'-yl]-3-(3-methyl-isothiazol-5-yl)-urea (**Ex-20**, 28 mg, 21% yield) as off white solid.
LC-MS (Method 3): m/z [M+H]⁺ = 442.2 (exact mass calc. = 441.16).
¹H-NMR (400 MHz, DMSO-d6): δ 10.14-10.06 (br s, 1 H), 8.22 (s, 1 H), 7.33-7.26 (m, 2 H), 7.05-6.95 (m, 4 H), 6.72-6.62 (m, 1 H), 6.39 (s, 1 H), 4.09-4.02 (m, 1 H), 3.78 (s, 3 H), 3.74-3.71 (m, 1 H), 3.67-3.63 (m, 1 H), 3.08-3.02 (m, 1 H), 2.98-2.92 (m, 1 H), 2.89-2.85 (m, 1 H), 2.19 (s, 3 H), 2.02 (d, *J* = 11 Hz, 1 H), 1.64 (d, *J* = 8 Hz, 1 H).

### Synthesis of trans-1-[5-chloro-1'-(2-methyl-pyrmidin-4-yl)-1',2',3',4',5',6'-hexahydro-[2,3']bipyridinyl-4'-yl]-3-(3-methyl-isothiazol-5-yl)-urea (Ex-21)

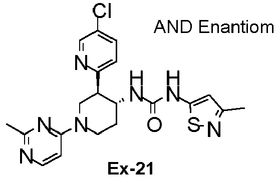

To a stirred solution of *trans*-1-(5-chloro-1',2',3',4',5',6'-hexahydro-[2,3']bipyridinyl-4'-yl)-3-(3-methyl-isothiazol-5-yl)-urea (**BB-VI-HCl,** 150 mg, 0.387mmol, 1 eq) and 4-chloro-2-methyl-pyrimidine (50 mg, 0.387 mmol, 1 eq) in n BuOH (4 ml) was heated to 100 °C for 16h. The reaction mixture was cooled to ambient temperature, diluted with EA and washed with water. The organic layer was separated, dried over anhyd. Na₂SO₄ and concentrated under reduced pressure to obtain crude compound which was purified by preparative TLC plate (8% MeOH in DCM as eluent) to get *trans*-1-[5-chloro-1'-(2-methyl-pyrimidin-4-yl)-1',2',3',4',5',6'-hexahydro-[2,3']bipyridinyl-4'-yl]-3-(3-methyl-isothiazol-5-yl)-urea (**Ex-21,** 35 mg, 20%) as off white solid.
LC-MS (Method 2): m/z [M+H]⁺ = 443.9 (exact mass calc. = 443.13).
¹H-NMR (400 MHz, DMSO-d6): δ 10.01 (s, 1 H), 8.59 (s, 1 H), 8.09 (d, *J* = 6 Hz, 1 H), 7.87 (d, *J* = 8 Hz, 1 H), 7.38 (d, *J* = 8 Hz, 1 H), 6.72 (d, *J* = 6 Hz, 1 H), 6.63 (br s, 1 H), 6.36 (s, 1 H), 4.53-4.50 (br m, 2 H), 4.17-4.14 (m, 1 H), 3.8-3.12 (m, 1 H), 3.14-2.97 (m, 2 H), 2.36 (s, 3 H), 2.19 (s, 3 H), 2.01 (d, *J* = 11 Hz, 1 H), 1.49 (d, *J* = 11 Hz, 1 H).

### Synthesis of trans-1-(4-chlorophenyl)-3-[3-(5-chloropyridin-2-yl)-1-[(5-methyl-1,2-oxazol-3-yl)methyl]piperidin-4-yl]urea ent-1 (Ex-22) and trans-1-(4-chlorophenyl)-3-[3-(5-chloropyridin-2-yl)-1-[(5-methyl-1,2-oxazol-3-yl)methyl]piperidin-4-yl]urea ent-2 (Ex-23)

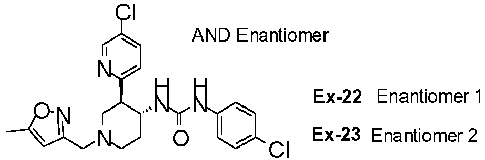

To the stirring solution of **BB-V-TFA** (0.26 g, 0.543 mmol, 1 eq) in acetonitrile (20 ml), K₂CO₃ (0.5 g, 3.559 mmol, 6.5 eq) was added at 0 °C and allowed to stir for 10 minutes, then 3-bromomethyl-5-methyl-isoxazole (0.150 g, 0.854 mmol, 1.6 eq) was added and again allowed to stir for 24 hours. After completion of reaction add water, extracted with ethyl acetate (3x100 ml). Ethyl acetate part was collected, dried over Na₂SO₄ and evaporated to dryness to get crude, which was purified by column chromatography followed by chiral prep-HPLC purification to afford compound **Ex-22** (0.0317 g, 12.7%) and **Ex-23** (0.0305 g, 12.2%).
¹H-NMR (400 MHz, DMSO-d6): δ 8.52 (s, 1H), 8.33 (s, 1H), 7.82-7.84 (m, 1H), 7.35 (d, *J* = 8.3 Hz, 1H), 7.28 (d, *J* = 8.7 Hz, 2H), 7.19 (d, *J* = 8.7 Hz, 2H), 6.17 (s, 1H), 6.06 (d, *J* = 8.1 Hz, 1H), 3.83-3.86 (m, 1H), 3.57 (s, 2H), 2.94-2.99 (m, 1H), 2.84-2.87 (m, 2H), 2.37 (s, 3H), 2.16-2.32 (m, 2H), 1.96-1.99 (m, 1H), 1.50-1.53 (m, 1H).

### Synthesis of trans-1-[5-chloro-1'-(4-fluoro-phenyl)-1',2',3',4',5',6'-hexahydro-[2,3']bipyridinyl-4'-yl]-3-(3-methyl-isothiazol-5-yl)-urea (Ex-26)

### Synthesis of HCl salt of 5-chloro-1',2',3',4',5',6'-hexahydro-[2,3']bipyridinyl-4'-carboxylic acid ethyl ester

To a stirred solution of 5-chloro-3',4',5',6'-tetrahydro-2'H-[2,3']bipyridinyl-1',4'-dicarboxylic acid 1'-tert-butyl ester 4'-ethyl ester (**E-V**, 1 g, 2.7 mmol, 1 eq) in 1,4-dioxane (20 ml) under ice cooled condition, was added 4 M HCl in dioxane (10 ml) followed by stirring at RT for a period of 2 h. After completion of the reaction (monitored by TLC and LC-MS), the reaction mixture was concentrated under reduced pressure to get the crude material as HCl salt of 5-chloro-1',2',3',4',5',6'-hexahydro-[2,3']bipyridinyl-4'-carboxylic acid ethyl ester (800 mg, 97% crude yield).
LC-MS (Method 2): m/z [M+H]⁺ = 268.8 (exact mass calc. = 268.10).

### Synthesis of 5-chloro-1'-(4-fluoro-phepyl)-1',2',3',4',5',6'-hexahydro-[2,3']bipyridinyl-4'-carboxylic acid ethyl ester

To a stirred solution of HCl salt of 5-chloro-1',2',3',4',5',6'-hexahydro-[2,3']bipyridinyl-4'-carboxylic acid ethyl ester (300 mg, 0.9865 mmol, 1 eq), 4-fluoro phenyl boronic acid (410 mg, 2.95 mmol, 3 eq), molecular sieves (300 mg) and TEA (700 µl, 4.925 mmol, 5 eq) in DCM (20 ml) was added Cu(OAc)₂ (268 mg, 1.47 mmol, 1.5 eq) and stirred at RT under oxygen atmosphere for a period of 2 days. After completion of the reaction, it was filtered and washed thoroughly with DCM, the filtrate was evaporated under reduced pressure and purified by silica gel (100-200 mesh) column chromatography 10-15% EA in hexane as an eluent to obtain 5-chloro-1'-(4-fluoro-phenyl)-1',2',3',4',5',6'-hexahydro-[2,3']bipyridinyl-4'-carboxylic acid ethyl ester (140 mg, yield 39%) as colorless gummy material.
LC-MS (Method 2): m/z [M+H]⁺ = 363.1 (exact mass calc. = 362.12).

### Synthesis of 5-chloro-1'-(4-fluoro-phenyl)-1',2',3',4',5',6'-hexahydro-[2,3']bipyridinyl-4'-carboxylic acid

To a stirred solution of 5-chloro-1'-(4-fluoro-phenyl)-1',2',3',4',5',6'-hexahydro-[2,3']bipyridinyl-4'-carboxylic acid ethyl ester (150 mg, 0.414 mmol, 1eq) in EtOH (5 ml) was added 21% NaOEt in EtOH (140 µl, 0.435 mmol, 1.05 eq) and refluxed for a period of 6 h. The reaction mixture was cooled to room temperature and added water-MeOH (1:1, 6 ml) and added KOH (46 mg, 0.82 mmol, 2.0 eq) and refluxed for a period of 16 h. After completion of the reaction, it was concentrated under reduced pressure and diluted with water and washed with ethyl acetate. The aqueous part was acidified by IN HCl and the organic components were extracted with 5% MeOH in DCM. The combined organic layer was then dried over anhyd. Na₂SO₄ and concentrated *in vacuo* to get *trans*-5-chloro-1'-(4-fluoro-phenyl)-1',2',3',4',5',6'-hexahydro-[2,3']bipyridinyl-4'-carboxylic acid (40 mg, 29% yield) as off white solid.
LC-MS (Method 2): m/z [M+H]⁺ = 335.1 (exact mass calc. = 334.09).

### Synthesis of trans-1-[5-chloro-1'-(4-fluoro-phenyl)-1',2',3',4',5',6'-hexahydro-[2,3']bipyridinyl-4'-yl]-3-(3-methyl-isothiazol-5-yl)-urea (Ex-26)

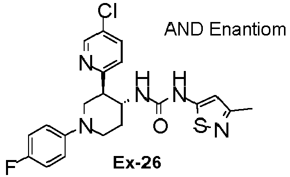

To a stirred solution of *trans*-5-chloro-1'-(4-fluoro-phenyl)-1',2',3',4',5',6'-hexahydro-[2,3']bipyridinyl-4'-carboxylic acid (20 mg, 0.06 mmol, 1eq) in Toluene (3 ml) was added Et₃N (17 µl, 0.12 mmol, 2.0 eq) followed by the addition of DPPA (21 µl, 0.097 mmol, 1.6 eq) and the reaction mass was stirred at reflux for a period of 2 h. The reaction mixture was then cooled to RT and added 3-methyl-isothiazol-5-ylamine (14 mg, 0.119 mmol, 2 eq) and the reaction mixture was heated to reflux for a period of 18 h. The reaction mass was concentrated *in vacuo* and diluted with EtOAc (150 ml) and washed with water and brine. The combined organic layer was dried over anhyd. Na₂SO₄ and concentrated under reduced pressure to get the crude product which was purified by silica gel (100-200 mesh) column chromatography (2-3% MeOH in DCM) followed by prep-TLC plate purification to obtain *trans*-1-[5-chloro-1'-(4-fluoro-phenyl)-1',2',3',4',5',6'-hexahydro-[2,3']bipyridinyl-4'-yl]-3-(3-methyl-isothiazol-5-yl)-urea (**Ex-26,** 16 mg, 60% yield) as off white solid.
LC-MS (Method 3): m/z [M+H]⁺ = 446.2 (exact mass calc. = 445.11).
¹H-NMR (400 MHz, DMSO-d6): δ 9.95 (s, 1 H), 8.56 (s, 1 H), 7.87-7.84 (m, 1 H), 7.40 (d, *J* = 8 Hz, 1 H), 7.06-6.96 (m, 4 H), 6.69-6.64 (br m, 1 H), 6.40 (s, 1 H), 4.06 (d, *J* = 12 Hz, 1 H), 3.76-3.68 (m, 2 H), 3.16-3.12 (m, 1 H), 3.03-2.88 (m, 2 H), 2.20 (s, 3 H), 1.99 (d, *J* = 13 Hz, 1 H), 1.68-1.64 (m, 1 H).

### Synthesis of trans-1-[5-chloro-1'-(6-trifluoromethyl-pyrimidin-4-yl)-1',2',3',4',5',6'-hexahydro-[2,3']bipyridinyl-4'-yl]-3-(3-methyl-isothiazol-5-yl)-urea (Ex-27)

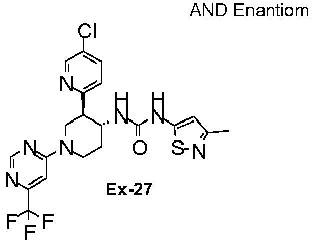

To a stirred solution of *trans*-1-(5-chloro-1',2',3',4',5',6'-hexahydro-[2,3']bipyridinyl-4'-yl)-3-(3-methyl-isothiazol-5-yl)-urea hydrochloride (**BB-VI-HCl,** 120 mg, 0.309 mmol, 1 eq) and 4-chloro-6-trifluoromethyl-pyrimidine (56 mg, 0.309 mmol, 1 eq) in *n*-BuOH (5 ml) was added DIPEA (162 µl, 0.927 mmol, 3.0 eq) and the reaction mixture was heated to 110 °C for 16h. The reaction mixture was cooled to ambient temperature, diluted with water (10 ml) and extracted with EA. The organic layer was separated, dried over anhyd. Na₂SO₄ and concentrated under reduced pressure to obtain crude compound which was purified by Silica gel (100-200 mesh) column chromatography (4% MeOH in DCM as eluenet) followed by preparative TLC using 8% MeOH in DCM as mobile phase to get *trans-*1-[5-chloro-1'-(6-trifluoromethyl-pyrimidin-4-yl)-1',2',3',4',5',6'-hexahydro-[2,3']bipyridinyl-4'-yl]-3-(3-methyl-isothiazol-5-yl)-urea (**Ex-27**, 38 mg, 24%) as off white solid.
LC-MS (Method 4): m/z [M+H]⁺ = 498.4 (exact mass calc. = 497.10).
¹H-NMR (400 MHz, DMSO-d6): δ 10.08 (s, 1 H), 8.64 (s, 1 H), 8.89 (s, 1 H), 7.89-7.86 (m, 1 H), 7.38 (d, *J* = 6 Hz, 2 H), 6.67-6.62 (m, 1 H), 6.40 (s, 1 H), 4.83-4.62 (m, 2 H), 4.26-4.16 (m, 1 H), 3.31-3.16 (m, 2 H), 3.04-2.98 (m, 1 H), 2.20 (s, 3 H), 2.06 (d, *J* = 11 Hz, 1 H), 1.59-1.51 (m, 1 H).

### Synthesis of trans-1-[5-chloro-1'-(2-trifluoromethyl-pyrimidm-4-yl)-1',2',3',4',5',6'-hexahydro-[2,3']bipyridinyl-4'-yl]-3-(3-methyl-isothiazol-5-yl)-urea (Ex-28)

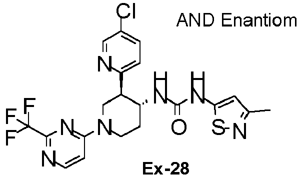

To a stirred solution of *trans*-1-(5-chloro-1',2',3',4',5',6'-hexahydro-[2,3']bipyridinyl-4'-yl)-3-(3-methyl-isothiazol-5-yl)-urea hydrochloride (**BB-VI-HCl,** 120 mg, 0.309 mmol, 1 eq) and 4-chloro-2-trifluoromethyl-pyrimidine (56 mg, 0.309 mmol, 1 eq) in *n*-BuOH (5 ml) was added DIPEA (162 µl, 0.927 mmol, 3.0 eq) and the reaction mixture was heated to 110 °C for 16h. The reaction mixture was cooled to ambient temperature, diluted with water (10 ml) and extracted with EA. The organic layer was separated, dried over anhyd. Na₂SO₄ and concentrated under reduced pressure to obtain crude compound which was purified by Silica gel (100-200 mesh) column chromatography (4% MeOH in DCM as eluent) followed by reverse phase prep preparative TLC using 8% MeOH in DCM as mobile phase to get *trans*-1-[5-chloro-1'-(2-trifluoromethyl-pyrimidin-4-yl)-1',2',3',4',5',6'-hexahydro-[2,3']bipyridinyl-4'-yl]-3-(3-methyl-isothiazol-5-yl)-urea (**Ex-28,** 20 mg, 13%) as off white solid.
LC-MS (Method 1): m/z [M+H]⁺ = 498.0 (exact mass calc. = 497.10).
¹H-NMR (400 MHz, DMSO-d6): δ 9.62 (s, 1 H), 8.55 (s, 1 H), 8.33 (d, *J* = 6 Hz, 1 H), 7.82-7.80 (m, 1 H), 7.39 (d, *J* = 8 Hz, 1 H), 7.06 (d, *J* = 6 Hz, 1 H), 6.39 (s, 1 H), 6.33 (d, *J* = 8 Hz, 1 H), 4.53-4.48 (m, 2 H), 4.21 (d, *J* = 8 Hz, 1 H), 3.41-3.35 (m, 1 H), 3.27-3.21 (m, 1 H), 3.10-3.04 (m, 1 H), 2.22 (s, 3 H), 2.13 (d, *J* = 13 Hz, 1 H), 1.65 (d, *J* = 8 Hz, 1 H).

### Synthesis of trans-1-(4-chlorophenyl)-3-[3-(5-methoxypyridin-2-yl)-1-[(5-methyl-1,2-oxazol-3-yl)methyl]piperidin-4-yl]urea ent-1 (Ex-29) and trans-1-(4-chlorophenyl)-3-[3-(5-methoxypyridin-2-yl)-1-[(5-methyl-1,2-oxazol-3-yl)methyl]piperidin-4-yl]urea ent-2 (Ex-30)

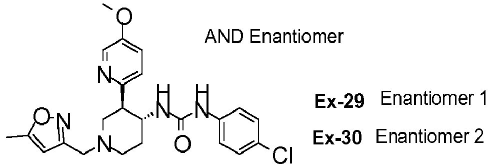

To the stirring solution of **BB-III-TFA** (0.4 g, 0.845 mmol, 0.76 eq) in acetonitrile (10 ml), K₂CO₃ (0.767 g, 5.55 mmol, 5 eq) was added at 0 °C and allowed to stir for 10 minutes, then 3-bromomethyl-5-methyl-isoxazole (0.195 g, 1.11 mmol, 1 eq) was added and again allowed to stir for 24 hours. After completion of reaction add water, extracted with ethyl acetate (3x100 ml). Ethyl acetate part was collected, dried over Na₂SO₄ and evaporated to dryness to get crude, which was purified by column chromatography followed by chiral prep-HPLC purification to afford compound **Ex-29** (0.055 g, 10.8%) and **Ex-30** (0.055 g, 10.8%).
**Ex-29:** ¹H-NMR (400 MHz, DMSO-d6): δ 8.33 (s, 1H), 8.18 (d, *J* = 2.5 Hz, 1H), 7.18-7.30 (m, 6H), 6.17 (s, 1H), 5.99 (d, *J* = 8.2 Hz, 1H), 3.80-3.82 (m, 1H), 3.76 (s, 3H), 3.51-3.52 (m, 2H), 2.81-2.89 (m, 3H), 2.37 (s, 3H), 2.17-2.25 (m, 2H), 1.99-2.01 (m, 1H), 1.47-1.50 (m, 1H).
Ex-30: ¹H-NMR (400 MHz, DMSO-d6): δ 8.33 (s, 1H), 8.18 (d, *J* = 2.6 Hz, 1H), 7.18-7.30 (m, 6H), 6.17 (s, 1H), 5.99 (d, *J* = 7.9 Hz, 1H), 3.76-3.83 (m, 4H), 3.51-3.52 (m, 2H), 2.81-2.86 (m, 3H), 2.37 (m, 3H), 2.17-2.25 (m, 2H), 1.98-2.01 (m, 1H), 1.47-1.50 (m, 1H).

### Synthesis of trans-1-[5-chloro-1'-(6-methyl-pyrimidin-4-yl)-1',2',3',4',5',6'-hexahydro-[2,3']bipyridinyl-4'-yl]-3-(3-methyl-isothiazol-5-yl)-urea (Ex-31)

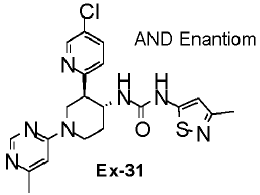

To a stirred solution of *trans*-1-(5-chloro-1',2',3',4',5',6'-hexahydro-[2,3']bipyridinyl-4'-yl)-3-(3-methyl-isothiazol-5-yl)-urea (**BB-VI-HCl,** 100 mg, 0.258 mmol, 1 eq) and 4-chloro-6-methyl-pyrimidine (33 mg, 0.258 mmol, 1 eq) in n BuOH (5 ml) was heated to 120 °C for 16 h. The reaction mixture was cooled to ambient temperature, diluted with EA and washed with water. The organic layer was separated, dried over anhyd. Na₂SO₄ and concentrated under reduced pressure to obtain crude compound which was purified by silica gel (100-200 mesh) column chromatography followed by reverse phase prep-HPLC purification to get *trans*-1-[5-chloro-1'-(6-methyl-pyrimidin-4-yl)-1',2',3',4',5',6'-hexahydro-[2,3']bipyridinyl-4'-yl]-3-(3-methyl-isothiazol-5-yl)-urea (**Ex-31,** 22 mg, 19%) as off white solid.
LC-MS (Method 2): m/z [M+H]⁺ = 444.3 (exact mass calc. = 443.13).
1H-NMR (400 MHz, DMSO-d6): δ 10.00 (s, 1 H), 8.58 (s, 1 H), 8.39 (s, 1 H), 7.88-7.86 (m, 1 H), 7.38 (d, *J* = 8 Hz, 1 H), 6.82 (s, 1 H), 6.62-6.59 (br m, 1 H), 6.39 (s, 1 H), 4.56-4.42 (m, 2 H), 4.17 (d, *J* = 12 Hz, 1 H), 3.27-3.15 (m, 1 H), 3.11-3.04 (m, 2 H), 2.26 (s, 3 H), 2.20 (s, 3 H), 2.01 (d, *J* = 10 Hz, 1 H), 1.51-1.48 (m, 1 H).

### Synthesis of trans-1-(5-chloro-1'-pyridazin-3-yl-1',2',3',4',5',6'-hexahydro-[2,3']bipyridinyl-4'-yl)-3-(3-methyl-isothiazol-5-yl)-urea (Ex-32)

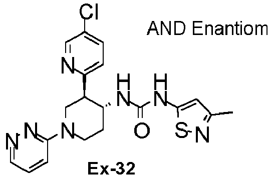

To a stirred solution of *trans*-1-(5-chloro-1',2',3',4',5',6'-hexahydro-[2,3']bipyridinyl-4'-yl)-3-(3-methyl-isothiazol-5-yl)-urea (**BB-VI-HCl,** 200 mg, 0.517 mmol, 1 eq) and 3-chloro-pyridazine (78 mg, 0.517 mmol, 1 eq) in DMSO (3 ml) was heated to 110 °C for 16h. The reaction mixture was cooled to ambient temperature, diluted with EA and washed with water. The organic layer was separated, dried over anhyd. Na₂SO₄ and concentrated under reduced pressure to obtain crude compound which was purified by reverse phase preparative HPLC to get *trans-*1-(5-chloro-1'-pyridazin-3-yl-1',2',3',4',5',6'-hexahydro-[2,3']bipyridinyl-4'-yl)-3-(3-methyl-isothiazol-5-yl)-urea (Ex-32, 14 mg, 6%) as off white solid.
LC-MS (Method 2): m/z [M+H]⁺ = 429.8 (exact mass calc. = 429.11).
¹H-NMR (400 MHz, DMSO-d6): δ); 9.93 (s, 1 H), 8.56 (s, 1 H), 8.51 (d, *J* = 4 Hz, 1 H), 7.86-7.83 (m, 1 H), 7.37-7.30 (m, 3 H), 7.62-7.58 (m, 1 H), 6.36 (s, 1 H), 4.53-4.48 (m, 2 H), 4.18-4.15 (m, 1 H), 3.27-3.16 (m, 1 H), 3.13-3.10 (m, 1 H), 3.07-3.0 (m, 1 H), 2.17 (s, 3 H), 1.99 (d, *J* = 12 Hz, 1 H), 1.58-1.49 (m, 1 H).

### Synthesis of trans-1-[5-chloro-1'-(6-methyl-pyridazin-3-yl)-1',2',3',4',5',6'-hexahydro-[2,3']bipyridinyl-4'-yl]-3-(3-methyl-isothiazol-5-yl)-urea (Ex-33)

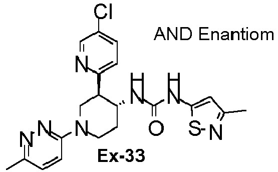

To a stirred solution of HCl salt of *trans*-1-(5-chloro-1',2',3',4',5',6'-hexahydro-[2,3']bipyridinyl-4'-yl)-3-(3-methyl-isothiazol-5-yl)-urea (**BB-VI-HCl,** 200 mg, 0.517 mmol, 1 eq) and 3-chloro-6-methyl-pyridazine (80 mg, 0.619 mmol, 1.2 eq) in DMSO (3 ml) was heated to 110 °C for 16 h. The reaction mixture was cooled to ambient temperature, diluted with EA and washed with water. The organic layer was separated, dried over anhyd. Na₂SO₄ and concentrated under reduced pressure to obtain crude compound which was purified by reverse phase preparative HPLC to get *trans*-1-[5-chloro-1'-(6-methyl-pyridazin-3-yl)-1',2',3',4',5',6'-hexahydro-[2,3']bipyridinyl-4'-yl]-3-(3-methyl-isothiazol-5-yl)-urea (**Ex-33,** 16 mg, 7%) as off white solid.
LC-MS (Method 1): m/z [M+H]⁺ = 444.0 (exact mass calc. = 443.13).
¹H-NMR (400 MHz, DMSO-d6): δ 9.94 (s, 1 H), 8.57 (d, *J* = 2 Hz, 1 H), 7.88-7.85 (m, 1 H), 7.37 (d, *J* = 8 Hz, 1 H), 7.2891 (s, 2 H), 6.62-6.58 (m, 1 H), 6.39 (s, 1 H), 4.43 (t, *J* = 15 Hz, 2 H), 4.19-4.16 (m, 1 H), 3.21-3.01 (m, 3 H), 2.44 (s, 3 H), 2.20 (s, 3 H), 2.01-1.98 (m, 1 H), 1.56-1.53 (m, 1 H).

### Synthesis of trans-1-[5-chloro-1'-(6-methyl-pyrazin-2-yl)-1',2',3',4',5',6'-hexahydro-[2,3']bipyridinyl-4'-yl]-3-(3-methyl-isothiazol-5-yl)-urea (Ex-34)

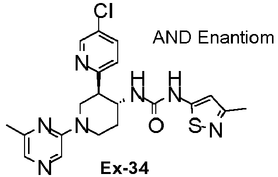

To a stirred solution of *trans*-1-(5-chloro-1',2',3',4',5',6'-hexahydro-[2,3']bipyridinyl-4'-yl)-3-(3-methyl-isothiazol-5-yl)-urea (**BB-VI-HCl,** 200 mg, 0.517 mmol, 1 eq) and 2-chloro-6-methyl-pyrazine (66 mg, 0.517 mmol, 1 eq) and DIPEA (270 µl, 1.55 mmol, 3 eq) in DMSO (3 ml) was heated to 110 °C for 16 h. The reaction mixture was cooled to ambient temperature, diluted with EA and washed with water. The organic layer was separated, dried over anhyd. Na₂SO₄ and concentrated under reduced pressure to obtain crude compound which was purified by reverse phase preparative HPLC to get *trans*-1-[5-chloro-1'-(6-methyl-pyrazin-2-yl)-1',2',3',4',5',6'-hexahydro-[2,3']bipyridinyl-4'-yl]-3-(3-methyl-isothiazol-5-yl)-urea (**Ex-34**, 19 mg, 8%) as off white solid.
LC-MS (Method 4): m/z [M+H]⁺ = 444.4 (exact mass calc. = 443.13).
¹H-NMR (400 MHz, DMSO-d6): δ 10.02 (s, 1 H), 8.57 (d, *J* = 2 Hz, 1 H), 8.18 (s, 1 H), 7.88-7.86 (m, 1 H), 7.73 (s, 1 H), 7.39 (d, *J* = 8 Hz, 1 H), 6.68-6.64 (br m, 1 H), 6.39 (s, 1 H), 4.45 (d, *J* = 13 Hz, 2 H), 4.20-4.15 (m, 1 H), 3.18-3.12 (m, 1 H), 3.08-2.99 (m, 2 H), 2.29 (s, 3 H), 2.20 (s, 3 H), 2.01 (d, *J* = 13 Hz, 1 H), 1.57-1.54 (m, 1 H).

### Synthesis of trans-1-(4-chlorophenyl)-3-[1-(2,2-difluoroethyl)-3-(5-methoxypyridin-2-yl)piperidin-4-yl]urea ent-1 (Ex-35) and trans-1-(4-chlorophenyl)-3-[1-(2,2-difluoroethyl)-3-(5-methoxypyridin-2-yl)piperidin-4-yl]urea ent-2 (Ex-36)

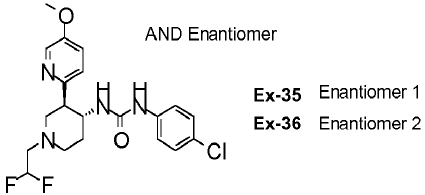

To a stirred solution **BB-III-TFA** (0.4 g, 0.843 mmol, 1 eq) in acetonitrile (20 ml), K₂CO₃ (0.764 g, 5.542 mmol, 6.6 eq) was added at 0 °C and allowed to stir for 10 minutes, then compound 2,2-difluoroethyl trifluoromethanesulfonate (0.284 g, 1.33 mmol, 1.6 eq) was added and again allowed to stir for 16 hours. After completion of reaction add water, extracted with ethyl acetate (3x100 ml). Ethyl acetate part was collected, dried over Na₂SO₄ and evaporated to dryness to get crude, which was purified by column chromatography followed by chiral prep-HPLC purification to afford *trans*-1-(4-chlorophenyl)-3-[1-(2,2-difluoroethyl)-3-(5-methoxypyridin-2-yl)piperidin-4-yl]urea ent-1 (**Ex-35**, 0.1 g, 28%) and *trans*-1-(4-chlorophenyl)-3-[1-(2,2-difluoroethyl)-3-(5-methoxypyridin-2-yl)piperidin-4-yl]urea ent-2 (**Ex-36**, 0.1 g, 28%).
**Ex-35:** ¹H-NMR (400 MHz, DMSO-d6): δ 8.34 (s, 1H), 8.19 (s, 1H), 7.32-7.18 (m, 6H), 6.27-5.97 (m, 2H), 3.84 -3.80 (m, 1H), 3.77 (s, 3H), 2.82-2.95 (m, 3H), 2.71-2.80 (m, 2H), 2.31-2.44 (m, 2H), 1.98 (d, J = 9.6 Hz, 1H), 1.47-1.51 (m, 1H).
**Ex-36:** ¹H-NMR (400 MHz, DMSO-d6): δ 8.32 (s, 1H), 8.19 (s, 1H), 7.18-7.31 (m, 6H), 5.98-6.26 (m, 2H), 3.80-3.84 (m, 1H), 3.77 (s, 3H), 2.85-2.94 (m, 3H), 2.71-2.80 (m, 2H), 2.30-2.44 (m, 2H), 1.98 (d, J = 10.4 Hz, 1H), 1.47-1.51 (m, 1H).

### Synthesis of trans-1-(4-chlorophenyl)-3-[3-(5-methoxypyridin-2-yl)-1-{[5-(trifluoromethyl)-1,2-oxazol-3-yl]methyl}piperidin-4-yl]urea ent-1 (Ex-37) and trans-1-(4-chlorophenyl)-3-[3-(5-methoxypyridin-2-yl)-1-{[5-(trifluoromethyl)-1,2-oxazol-3-yl]methyl}piperidin-4-yl]urea ent-2 (Ex-38)

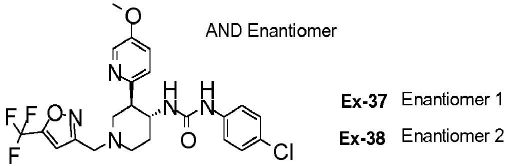

To the stirring solution of (5-Trifluoromethyl-isoxazol-3-yl)-methanol (0.15 g, 0.878 mmol, 1 eq) in DCM (10 ml), DMAP (0.022 g, 0.179 mmol, 0.2 eq) and TEA (0.25 ml, 1.796 mmol, 2 eq) was added at RT and allowed to stir for 10 min. After that TsCl (0.205 g, 1.077 mmol, 1.2 eq) was added at RT and the reaction was continued stirring at the same temperature for 3 h. Into the reaction mixture **BB-III-TFA** (0.35 g, 0.738 mmol, 0.8 eq) and K₂CO₃ (0.309 g, 2.245 mmol, 2.5 eq) were added at RT and stirred the reaction mixture for 16 h. After completion of reaction (monitored by TLC), reaction mixture was filtered and filtrate was evaporated under reduced pressure to get the crude. Crude was purified by column chromatography followed by chiral prep-HPLC purification to afford trans-1-(4-chlorophenyl)-3-[3-(5-methoxypyridin-2-yl)-1-{[5-(trifluoromethyl)-1,2-oxazol-3-yl]methyl}piperidin-4-yl]urea ent-1 (**Ex-37**, 0.075 g, 16.8%) and *trans*-1-(4-chlorophenyl)-3-[3-(5-methoxypyridin-2-yl)-1-{[5-(trifluoromethyl)-1,2-oxazol-3-yl]methyl}piperidin-4-yl]urea ent-2 (**Ex-38**, 0.07 g, 15.4%).
**Ex-37:** ¹H-NMR (400 MHz, DMSO-d6): δ 8.33 (s, 1H), 8.19 (s, 1H), 7.42 (s, 1H), 7.18-7.30 (m, 6H), 6.00 (d, J = 8.4 Hz, 1H), 3.83 (t, J = 4.8 Hz, 1H), 3.76 (s, 3H), 3.71 (s, 2H), 2.85-2.92 (m, 3H), 2.22-2.35 (m, 2H), 2.01 (d, J = 10.8 Hz, 1H), 1.50-1.52 (m, 1H).
**Ex-38:** ¹H-NMR (400 MHz, DMSO-d6): δ 8.33 (s, 1H), 8.19 (s, 1H), 7.42 (s, 1H), 7.18-7.30 (m, 5H), 6.00 (d, J = 8.0 Hz, 1H), 3.83 (bs, 1H), 3.71-3.76 (s, 5H), 2.85-2.89 (m, 3H), 2.24-2.32 (m, 2H), 2.01 (d, J = 10.8 Hz, 1H), 1.50-1.52 (m, 1H).

### Synthesis of trans-1-(4-chlorophenyl)-3-[3-(5-methoxypyridin-2-yl)-1-[(1-methyl-1H-pyrazol-3-yl)methyl]piperidin-4-yl]urea ent-1 (Ex-39) and trans-1-(4-chlorophenyl)-3-[3-(5-methoxypyridin-2-yl)-1-[(1-methyl-1H-pyrazol-3-yl)methyl]piperidin-4-yl]urea ent-2 (Ex-40)

### Synthesis of 3-(bromomethyl)-1-methyl-1H-pyrazole

To a stirred solution of (1-methyl-1H-pyrazol-3-yl)methanol (0.3 g, 2.678 mmol, 1 eq) in DCM (30 ml), PPh₃ (1.19 g, 4.535 mmol, 1.7 eq) and CBr₄ (1.59 g, 4.82 mmol, 1.8 eq) was added at RT. The reaction mixture was stirred at RT for 3 h. After completion of reaction (monitored by TLC), reaction mixture was evaporated under reduced pressure to get the crude. Crude was purified by column chromatography (30%EA-hexane) to afford 3-(bromomethyl)-1-methyl-1H-pyrazole (0.33 g, 70.9%) as brown gum.

### Synthesis of trans-1-(4-chlorophenyl)-3-[3-(5-methoxypyridin-2-yl)-1-[(1-methyl-1H-pyrazol-3 - yl)methyl]piperidin-4-yl]urea ent-1 (Ex-39) and trans-1-(4-chlorophenyl)-3-[3-(5-methoxypyridin-2-yl)-1-[(1-methyl-1H-pyrazol-3-yl)methyl]piperidin-4-yl]urea ent-2 (Ex-40)

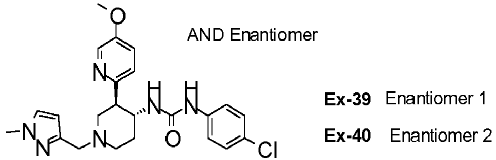

To the stirring solution of compound **BB-III-TFA** (0.75 g, 1.582 mmol, 1 eq) in acetonitrile (25 ml), K₂CO₃ (0.574 g, 4.166 mmol, 2.6 eq) was added at 0 °C and allowed to stir for 10 min, then 3-(bromomethyl)-1-methyl-1H-pyrazole (0.218 g, 1.249 mmol, 0.8 eq) was added and again allowed to stir for 2 h. After completion of reaction (monitored by TLC), reaction mixture was filtered and filtrate was evaporated under reduced pressure to get the crude. Crude was purified by column chromatography followed by chiral prep-HPLC purification to afford *trans-*1-(4-chlorophenyl)-3 -[3 -(5-methoxypyridin-2-yl)-1-[(1-methyl-1H-pyrazol-3 -yl)methyl]piperidin-4-yl]urea ent-1 (**Ex-39**, 0.045 g, 6.2%) and *trans*-1-(4-chlorophenyl)-3-[3-(5-methoxypyridin-2-yl)-1-[(1-methyl-1H-pyrazol-3-yl)methyl]piperidin-4-yl]urea ent-2 (**Ex-40**, 0.045 g, 6.2%).
**Ex-39:** ¹H-NMR (400 MHz, DMSO-d6): δ 8.37 (s, 1H), 8.17 (s, 1H), 7.56 (s, 1H), 7.27-7.30 (m, 3H), 7.17-7.22 (m, 3H), 6.10 (s, 1H), 6.02 (d, J = 8.0 Hz, 1H), 3.75-3.82 (m, 7H), 3.40 (q, J = 12.8 Hz & 13.2 Hz, 2H), 2.83-2.89 (m, 3H), 2.06-2.15 (m, 2H), 1.98 (d, J = 9.2 Hz, 1H), 1.45-1.50 (m, 1H).
**Ex-40:** ¹H-NMR (400 MHz, DMSO-d6): δ 8.33 (s, 1H), 8.17 (s, 1H), 7.56 (s, 1H), 7.27-7.29 (m, 3H), 7.17-7.22 (m, 3H), 6.10 (s, 1H), 5.98 (d, J = 8.0 Hz, 1H), 3.75-3.79 (m, 7H), 3.42 (q, J = 13.6 Hz & 12.4 Hz, 2H), 2.83-2.90 (m, 3H), 2.10-2.13 (m, 2H), 1.98 (d, J = 11.6 Hz, 1H), 1.45-1.50 (m, 1H).

### Synthesis of trans-1-[5-chloro-1'-(5-methyl-pyrazin-2-yl)-1',2',3',4',5',6'-hexahydro-[2,3']bipyridinyl-4'-yl]-3-(3-methyl-isothiazol-5-yl)-urea (Ex-41)

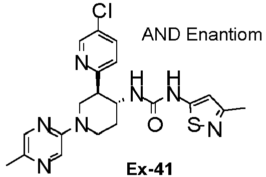

To a stirred solution of *trans*-1-(5-chloro-1',2',3',4',5',6'-hexahydro-[2,3']bipyridinyl-4'-yl)-3-(3-methyl-isothiazol-5-yl)-urea (**BB-VI-HCl,** 200 mg, 0.517 mmol, 1 eq) and 2-bromo-5-methyl-pyrazine (133 mg, 0.774 mmol, 1.5 eq) and DIPEA (270 µl, 1.548 mmol, 3 eq) in DMSO (3 ml) was heated to 110 °C for 16 h. The reaction mixture was cooled to ambient temperature, diluted with EA and washed with water. The organic layer was separated, dried over anhyd. Na₂SO₄ and concentrated under reduced pressure to obtain crude compound which was purified by reverse phase preparative HPLC to get *trans*-1-[5-chloro-1'-(5-methyl-pyrazin-2-yl)-1',2',3',4',5',6'-hexahydro-[2,3']bipyridinyl-4'-yl]-3-(3-methyl-isothiazol-5-yl)-urea (**Ex-41**, 18 mg, 8%) as light yellowish solid.
LC-MS (Method 2): m/z [M+H]⁺ = 443.9 (exact mass calc. = 443.13).
¹H-NMR (400 MHz, DMSO-d6): δ 9.95 (s, 1 H), 8.57 (d, *J* = 2 Hz, 1 H), 8.28 (s, 1 H), 7.99 (s, 1 H), 7.87-7.85 (m, 1 H), 7.37 (d, *J* = 8 Hz, 1 H), 6.60-6.59 (m, 1 H), 6.39 (s, 1 H), 4.41-4.38 (m, 2 H), 4.18-4.15 (m, 1 H), 3.18-3.00 (m, 3 H), 2.31 (s, 3 H), 2.19 (s, 3 H), 1.99 (d, *J* = 11 Hz, 1 H), 1.56-1.54 (m, 1 H).

### Synthesis of trans-1-(4-chlorophenyl)-3-[1-[(1-methanesulfonyl-1H-pyrazol-3-yl)methyl]-3-(5-methoxypyridin-2-yl)piperidin-4-yl]urea ent-1 (Ex-42) and trans-1-(4-chlorophenyl)-3-[1-[(1-methanesulfonyl-1H-pyrazol-3-yl)methyl]-3-(5-methoxypyridin-2-yl)piperidin-4-yl]urea ent-1 (Ex-43)

### Synthesis of 1-methanesulfonyl-1H-pyrazole-3-carbaldehyde

To a solution of 1H-pyrazole-3-carbaldehyde (2 g, 11.483 mmol, 1 eq) in pyridine (20 ml), MsCl (1.1 ml, 13.778 mmol, 1.2 eq) was added at rt. The reaction mixture was refluxed for 16 h. After completion of reaction (monitored by TLC), reaction mixture was evaporated under reduced pressure to get the crude. Crude was purified by column chromatography to afford 1-methanesulfonyl-1H-pyrazole-3-carbaldehyde (1.2 g, 60.3 %) as brown solid.

### Preparation of (1-methanesulfonyl-1H-pyrazol-3-yl)methanol

To a solution of 1-methanesulfonyl-1H-pyrazole-3-carbaldehyde (0.37 g, 2.124 mmol, 1 eq) in DCM (10 ml), DIBAL (25%, 3 ml, 4.248 mmol, 2 eq) was added drop wise at -78 °C. The reaction mixture was stirred at -78 °C for 1 h. After completion of reaction (monitored by TLC), reaction mixture was quenched with sodium potassium tertarate solution and extracted with EA. Wash the organic layer with water, brine, dried over Na₂SO₄, filtered and evaporated under reduced pressure to get the crude. Crude was purified by column chromatography to afford 1-methanesulfonyl-1H-pyrazol-3-yl)methanol (0.35 g, 93.8%) which was used for the next step without further purification.

### Preparation of 3-(bromomethyl)-1-methanesulfonyl-1H-pyrazole

To a stirred solution of 1-methanesulfonyl-1H-pyrazol-3-yl)methanol (0.37 g, 2.1 mmol, 1 eq) in DCM (10 ml), PPh₃ (0.826 g, 3.150 mmol, 1.5 eq) and CBr₄ (1.04 g, 3.15 mmol, 1.5 eq) was added at RT. The reaction mixture was stirred at RT for 3 h. After completion of reaction (monitored by TLC), reaction mixture was evaporated under reduced pressure to get the crude. Crude was purified by column chromatography (20%EA-hexane) to afford 3-(bromomethyl)-1-methanesulfonyl-1H-pyrazole (0.35 g, 70.4%) as brown gum.

### Synthesis of trans-1-(4-chlorophenyl)-3-[1-[(1-methanesulfonyl-1H-pyrazol-3-yl)methyl]-3-(5-methoxypyridin-2-yl)piperidin-4-yl]urea ent-1 (Ex-42) and trans-1-(4-chlorophenyl)-3-[(1-[(1-methanesulfonyl-1H-pyrazol-3-yl)methyl]-3-(5-methoxypyridin-2-yl)piperidin-4-yl]urea ent-1 (Ex-43)

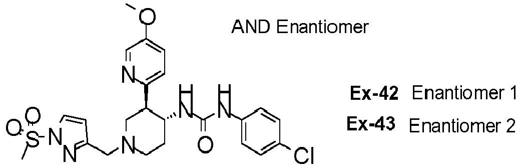

To the stirring solution of compound **BB-III-TFA** (0.367 g, 0.775 mmol, 1 eq) in acetonitrile (10 ml), K₂CO₃ (0.534 g, 3.875 mmol, 5 eq) was added at 0 °C and allowed to stir for 10 min, then 3-(bromomethyl)-1-methanesulfonyl-1H-pyrazole (0.185 g, 0.775 mmol, 1 eq) was added and again allowed to stir for 16 h. After completion of reaction (monitored by TLC), reaction mixture was filtered and filtrate was evaporated under reduced pressure to get the crude. Crude was purified by column chromatography followed by chiral prep-HPLC purification to afford *trans*-1-(4-chlorophenyl)-3-[1-[(1-methanesulfonyl-1H-pyrazol-3-yl)methyl]-3-(5-methoxypyridin-2-yl)piperidin-4-yl]urea ent-1 (**Ex-42**, 0.048 g, 11.9%) and *trans*_1-(4-chlorophenyl)-3-[1-[(1-methanesulfonyl-1H-pyrazol-3-yl)methyl]-3-(5-methoxypyridin-2-yl)piperidin-4-yl]urea ent-1 (**Ex-43**, 0.078 g, 19.3%) as white solid.
**Ex-42:** ¹H-NMR (400 MHz, DMSO-d6): δ 8.33 (s, 1H), 8.17-8.20 (m, 2H), 7.27-7.30 (m, 3H), 7.19-7.24 (m, 3H), 6.52 (d, J = 2.4 Hz, 1H), 5.98 (d, J = 8.0 Hz, 1H), 3.81-3.84 (m, 1H), 3.76 (s, 3H), 3.56 (s, 2H), 3.42 (s, 3H), 2.85-2.91 (m, 3H), 2.14-2.32 (m, 2H), 1.99 (d, J = 11.2 Hz, 1H), 1.46-1.54 (m, 1H).
**Ex-43:** ¹H-NMR (400 MHz, DMSO-d6): δ 8.34 (s, 1H), 8.17-8.20 (m, 2H), 7.17-7.30 (m, 6H), 6.52 (d, J = 2.8 Hz, 1H), 5.99 (d, J = 8.0 Hz, 1H), 3.81-3.84 (m, 1H), 3.76 (s, 3H), 3.56 (s, 2H), 3.42 (s, 3H), 2.57-2.91 (m, 3H), 2.14-2.28 (m, 2H), 1.99 (d, J = 12.4 Hz, 1H), 1.48-1.51 (m, 1H).

### Synthesis of trans-1-(4-chlorophenyl)-3-[3-(5-chlorothiophen-2-yl)-1-[(5-methyl-1,2-oxazol-3-yl)methyl]piperidin-4-yl]urea ent-1 (Ex-44) and trans-1-(4-chlorophenyl)-3-[3-(5-chlorothiophen-2-yl)-1-[(5-methyl-1,2-oxazol-3-yl)methyl]piperidin-4-yl]urea ent-2 (Ex-45)

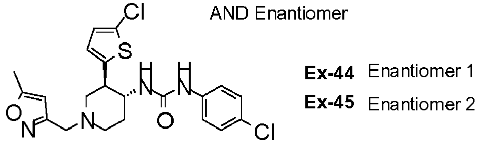

To the stirring solution of **BB-VII-TFA** (0.225 g, 0.465 mmol, 1.0 eq) in acetonitrile (20 ml), K₂CO₃ (0.192 g, 1.395 mmol, 3.0 eq) was added at 0 °C and allowed to stir for 10 minutes, then 3-bromomethyl-5-methyl-isoxazole (0.0819 g, 0.465 mmol, 1 eq) was added and again allowed to stir for 24 hours. After completion of reaction water was added, extracted with ethyl acetate (3x100 ml). Ethyl acetate part was collected, dried over Na₂SO₄ and evaporated to dryness to get crude, which was purified by column chromatography followed by chiral prep-HPLC purification to afford *trans*-1-(4-chlorophenyl)-3-[3-(5-chlorothiophen-2-yl)-1-[(5-methyl-1,2-oxazol-3-yl)methyl]piperidin-4-yl]urea ent-1 **(Ex-44,** 0.060 g, 27.8%) and *trans*-1-(4-chlorophenyl)-3-[3-(5-chlorothiophen-2-yl)-1-[(5-methyl-1,2-oxazol-3-yl)methyl]piperidin-4-yl]urea ent-2 (**Ex-45**, 0.062 g, 28.7%).
**Ex-44:** ¹H-NMR (400 MHz, DMSO-d6): δ 8.46 (s, 1H), 7.35 (d, *J* = 8.9 Hz, 2H), 7.22 (d, *J* = 8.8 Hz, 2H), 6.93 (d, *J* = 3.7 Hz, 1H), 6.80 (d, *J* = 3.7 Hz, 1H), 6.21 (d, *J* = 8.1 Hz, 1H), 6.18 (s, 1H), 3.50-3.70 (m, 3H), 3.04-3.09 (m, 1H), 2.94 (d, *J* = 9.5 Hz, 1H), 2.75-2.78 (m, 1H), 2.38 (s, 3H), 2.19-2.26 (m, 2H), 1.90-1.93 (m, 1H), 1.48-1.50 (m, 1H).
**Ex-45:** ¹H-NMR (400 MHz, DMSO-d6): δ 8.45 (s, 1H), 7.35 (d, *J* = 8.7 Hz, 2H), 7.22 (d, *J* = 8.7 Hz, 2H), 6.92 (d, *J=* 3.5 Hz, 1H), 6.80 (d, *J* = 3.6 Hz, 1H), 6.18-6.22 (m, 2H), 3.50-3.60 (m, 3H), 3.06 (t, *J*= 8.9 Hz, 1H), 2.94 (d, *J*= 10.7 Hz, 1H), 2.75-2.78 (m, 1H), 2.38 (s, 3H), 2.21-2.24 (m, 2H), 1.92 (d, *J=* 13.1 Hz, 1H), 1.50 (q, *J=* 10.8 Hz, 1H).

### Synthesis of trans-1-(4-chlorophenyl)-3-[3-(5-chlorothiophen-2-yl)-1-(pyrimidin-2-ylmethyl)piperidin-4 yl]urea (Ex-46) ent-1 and trans-1-(4-chlorophenyl)-3-[3-(5-chlorothiophen-2-yl)-1-(pyrimidin-2-ylmethyl)piperidin-4-yl]urea ent-2 (Ex-47)

### Preparation of 2-(bromomethyl)pyrimidine

To a stirred solution of pyrimidin-2-ylmethanol (1.0 g, 9.081 mmol, 1 eq) in DCM (10 mL) was added PBr₃ (5.408 g, 19.98 mmol, 2.2 eq) for 2 h at RT. After completion of the reaction (monitored by TLC, TLC system 20% ethyl acetate in hexane, R_{f}: 0.5), the reaction mixture was poured into sat. NaHCO₃ solution, extracted with ethyl acetate (2 x 100 mL). The combined organic layer was washed with water (2 x 100 mL) and brine (50 mL), dried over anhyd. Na₂SO₄ and concentrated under reduced pressure to afford 2-(bromomethyl)pyrimidine as brown liquid (0.800 g, 50.9%), which was used as such for next step.

### Synthesis of trans-1-(4-chlorophenyl)-3-[3-(5-chlorothiophen-2-yl)-1-(pyrimidin-2-ylmethyl)piperidin-4-yl]urea (Ex-46) ent-1 and trans-1-(4-chlorophenyl)-3-[3-(5-chlorothiophen-2-yl)-1-(pyrimidin-2-ylmethyl)piperidin-4-yllurea ent-2 (Ex-47)

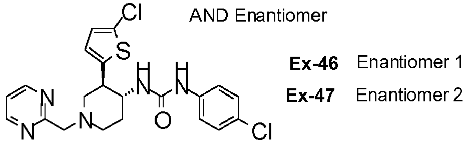

To the stirring solution of **BB-VII-TFA** (0.222 g, 0.459 mmol, 1.0 eq) in acetonitrile (20 ml), K₂CO₃ (0.190 g, 1.377 mmol, 3.0 eq) was added at 0 °C and allowed to stir for 10 minutes, then compound 2-(bromomethyl)pyrimidine (0.0795 g, 0.459 mmol, 1 eq) was added and again allowed to stir for 24 hours. After completion of reaction water was added, extracted with ethyl acetate (3x100 ml). Ethyl acetate part was collected, dried over Na₂SO₄ and evaporated to dryness to get crude, which was purified by column chromatography followed by chiral prep-HPLC purification to afford *trans*-1-(4-chlorophenyl)-3-[3-(5-chlorothiophen-2-yl)-1-(pyrimidin-2-ylmethyl)piperidin-4-yl]urea (**Ex-46,** 0.015g, 7.06%) and *trans*-1-(4-chlorophenyl)-3-[3-(5-chlorothiophen-2-yl)-1-(pyrimidin-2-ylmethyl)piperidin-4-yl]urea ent-2 (**Ex-47**, 0.398 g, 7.53%).
**Ex-46:** ¹H-NMR (400 MHz, DMSO-d6): δ 8.78 (d, *J* = 4.8 Hz, 2H), 8.45 (s, 1H), 7.40 (t, *J*= 4.8 Hz, 1H), 7.34 (d, *J*= 8.8 Hz, 2H), 7.22 (d, *J*= 8.8 Hz, 2H), 6.91 (d, *J*= 3.7 Hz, 1H), 6.80 (d, *J*= 3.7 Hz, 1H), 6.20 (d, *J*= 8.5 Hz, 1H), 3.77 (s, 2H), 3.54-3.56 (m, 1H), 3.0-3.10 (m, 2H), 2.89 (d, *J*= 11.0 Hz, 1H), 2.32-2.34 (m, 2H), 1.91 (d, *J*= 10.4 Hz, 1H), 1.51 (q, *J*= 10.6 Hz, 1H).
**Ex-47:** ¹H-NMR (400 MHz, DMSO-d6): δ 8.78 (d, *J*= 4.8 Hz, 2H), 8.44 (s, 1H), 7.40 (t, *J*= 5.0 Hz, 1H), 7.34 (d, *J*= 8.7 Hz, 2H), 7.22 (d, *J*= 8.8 Hz, 2H), 6.91 (d, *J*= 3.7 Hz, 1H), 6.80 (d, *J*= 3.7 Hz, 1H), 6.20 (d, *J*= 8.3 Hz, 1H), 3.77 (s, 2H), 3.51-3.56 (m, 1H), 3.0-3.10 (m, 2H), 2.80-2.90 (m, 1H), 2.32-2.35 (m, 2H), 1.91 (d, *J*= 10.4 Hz, 1H), 1.50-1.52 (m, 1H).

### Synthesis of trans-1-[3-(5-chlorothiophen-2-yl)-1-[(5-methyl-1,2-oxazol-3-yl)methyl]piperidin-4-yl]-3-(3-methyl-1,2-thiazol-5-yl)urea ent-1 (Ex-48) and trans-1-[3-(5-chlorothiophen-2-yl)-1-[(5-methyl-1,2-oxazol-3-yl)methyl]piperidin-4-yl]-3-(3-methyl-1,2-thiazol-5-yl)urea ent-2 (Ex-49)

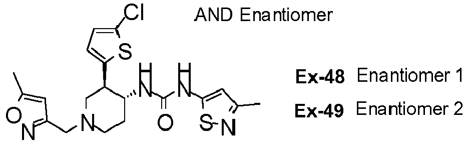

To the stirring solution of **BB-VIII-TFA** (0.350 g, 0.7446 mmol, 1.0 eq) in acetonitrile (20 ml), K₂CO₃ (0.308 g, 2.233 mmol, 3.0 eq) was added at 0 °C and allowed to stir for 10 minutes, then 3-bromomethyl-5-methyl-isoxazole (0.131 g, 0.7446 mmol, 1 eq) was added and again allowed to stir for 24 hours. After completion of reaction water was added, extracted with ethyl acetate (3x100 ml). Ethyl acetate part was collected, dried over Na₂SO₄ and evaporated to dryness to get crude, which was purified by column chromatography followed by chiral prep-HPLC purification to afford *trans*-1-[3-(5-chlorothiophen-2-yl)-1-[(5-methyl-1,2-oxazol-3-yl)methyl]piperidin-4-yl]-3-(3-methyl-1,2-thiazol-5-yl)urea ent-1 (**Ex-48,** 0.070 g, 20.8%) and *trans*-1-[3-(5-chlorothiophen-2-yl)-1-[(5-methyl-1,2-oxazol-3-yl)methyl]piperidin-4-yl]-3-(3-methyl-1,2-thiazol-5-yl)urea ent-2 (**Ex-49,** 0.072 g, 21.4%). **Ex-48:** ¹H-NMR (400 MHz, DMSO-d6): δ 10.01 (s, 1H), 6.92 (d, *J*= 3.7 Hz, 1H), 6.79 (d, *J*= 3.6 Hz, 1H), 3.60-3.70 (m, 1H), 6.44 (s, 1H), 6.18 (s, 1H), 3.50-3.60 (m, 3H), 3.13 (t, *J*= 8.9 Hz, 1H), 2.96 (d, *J* = 10.5 Hz, 1H), 2.79 (d, *J*= 11.3 Hz, 1H), 2.38 (s, 3H), 2.10-2.30 (m, 5H), 1.90 (d, *J*= 9.6 Hz, 1H), 1.54-1.59 (m, 1H).
**Ex-49:** ¹H-NMR (400 MHz, DMSO-d6): δ 10.01 (s, 1H), 6.92 (d, *J*= 3.7 Hz, 1H), 6.79 (d, *J*= 3.7 Hz, 1H), 3.60-3.70 (m, 1H), 6.44 (s, 1H), 6.18 (s, 1H), 3.50-3.60 (m, 3H), 3.13-3.15 (m, 1H), 2.95 (d, *J*= 9.6 Hz, 1H), 2.79 (d, *J*= 11.3 Hz, 1H), 2.38 (s, 3H), 2.10-2.30 (m, 5H), 1.90 (d, *J*= 10.5 Hz, 1H), 1.54-1.59 (m, 1H).

### Synthesis of trans-1-(4-chlorophenyl)-3-[(5-methoxypyridin-2-yl)-1-(oxan-4-ylmethyl)piperidin-4-yl]urea ent-1 (Ex-50) and trans-1-(4-chlorophoyl)-3-[3-(5-methoxypyridin-2-yl)-1-(oxan-4-ylmethyl)piperidin-4-yl]urea ent-2 (Ex-51)

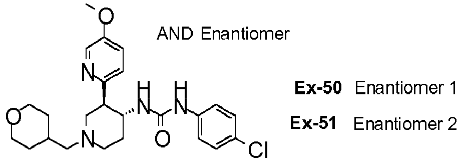

To the stirring solution of *trans*-1-(4-chlorophenyl)-3-(3-(5-methoxypyridin-2-yl)piperidin-4-yl)urea trifluoroacetate (**BB-III-**TFA, 0.4 g, 0.8438 mmol, 1.0 eq) in acetonitrile (20 ml), K₂CO₃ (0.582 g, 4.219 mmol, 5.0 eq) was added at 0 °C and allowed to stir for 10 minutes, then 4-(bromomethyl)oxane (0.166 g, 0.982 mmol, 1.2 eq) was added and again allowed to stir for 24 hours. After completion of reaction water was added, extracted with ethyl acetate (3x100 ml). Ethyl acetate part was collected, dried over Na₂SO₄ and evaporated to dryness to get crude, which was purified by column chromatography to get *trans*-1-(4-chlorophenyl)-3-[(5-methoxypyridin-2-yl)-1-(oxan-4-ylmethyl)piperidin-4-yl]urea (0.207g, 53%).
LC-MS (Method 2): m/z [M+H]⁺ = 459.2 (exact mass calc. = 458.21).
0.207g of *trans*-1-(4-chlorophenyl)-3-[(5-methoxypyridin-2-yl)-1-(oxan-4-ylmethyl)piperidin-4-yl]urea was separated by preparative chiral HPLC to afford *trans*-1-(4-chlorophenyl)-3-[(5-methoxypyridin-2-yl)-1-(oxan-4-ylmethyl)piperidin-4-yl]urea ent-1 (**Ex-50,** 0.051 g, 13.2%) *trans*-1-(4-chlorophenyl)-3-[3-(5-methoxypyridin-2-yl)-1-(oxan-4-ylmethyl)piperidin-4-yl]urea ent-2 (**Ex-51,** 0.045 g, 11.6%).
**Ex-50:**
LC-MS (Method 3): m/z [M+H]⁺ = 459.4 (exact mass calc. = 458.21).
¹H-NMR (400 MHz, DMSO-d6): δ 8.33 (s, 1H), 8.19 (d, *J*= 2.2 Hz, 1H), 7.18-7.30 (m, 6H), 5.96 (d, *J*= 8.0 Hz, 1H), 3.79-3.82 (m, 2H), 3.77 (s, 3H), 3.23-3.29 (m, 2H), 2.83-2.87 (m, 3H), 2.0-2.15 (m, 5H), 1.45-1.70 (m, 4H), 1.08-1.29 (m, 3H).
**Ex-51:**
LC-MS (Method 3): m/z [M+H]⁺ = 459.4 (exact mass calc. = 458.21)
¹H-NMR (400 MHz, DMSO-d6): δ 8.33 (s, 1H), 8.19 (s, 1H), 7.18-7.30 (m, 6H), 5.96 (d, *J*= 8.1 Hz, 1H), 3.77-3.82 (m, 5H), 3.23-3.31 (m, 2H), 2.85-2.90 (m, 3H), 2.02-2.13 (m, 5H), 1.45-1.70 (m, 4H), 1.08-1.23 (m, 3H).

### Synthesis of trans-1-(4-chlorophenyl)-3-[3-(5-methoxypyridin-2-yl)-1-[(4-methylpyridin-3-yl)carbopyl]piperidin-4-yl]urea ent-1 (Ex-52) and trans-1-(4-chlorophenyl)-3-[3-(5-methoxypyridin-2-yl)-1-[(4-methylpyridin-3-yl)carbonyl]piperidin-4-yl]urea ent-2 (Ex-53)

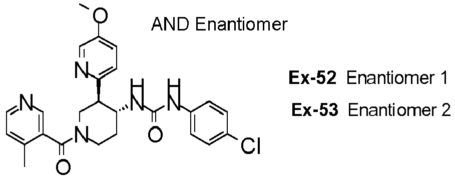

To a stirred solution 4-methylpyridine-3-carboxylic acid (0.223 g, 1.630 mmol, 1.5 eq) in DMF (10 ml) was added EDCI-HCl (0.3124 g, 1.630 mmol, 1.5 eq), HOBt (0.176 g, 1.303 mmol, 1.5 eq), DIPEA (0.9 ml, 8.43 mmol, 5.0 eq) and stirred at RT for 30 min followed by addition of a *trans*-1-(4-chlorophenyl)-3-(3-(5-methoxypyridin-2-yl)piperidin-4-yl)urea (**BB-III-TFA**, 0.45 g, 1.086 mmol, 1.0 eq). The reaction mixture was stirred for 16 hr at RT. After completion of the reaction (monitored by TLC, TLC system 5% MeOH-DCM, R_{f}: 0.4) reaction mixture was poured into cold water and extracted with EtOAc (2 x 25 ml). The organic layer was washed with water (2 x 25 ml) and brine (2 x 25 mL), dried over anhyd. Na₂SO₄, concentrated in vacuo. The crude material thus obtained was purified by flash chromatography (230-400 mesh silica gel; 5% MeOH-DCM as an elutent) to get *trans*-1-(4-chlorophenyl)-3-[3-(5-methoxypyridin-2-yl)-1-[(4-methylpyridin-3-yl)carbonyl]piperidin-4-yl]urea (0.21g,40.29%).
0.207g of *trans*-1-(4-chlorophenyl)-3-[3-(5-methoxypyridin-2-yl)-1-[(4-methylpyridin-3-yl)carbonyl]piperidin-4-yl]urea was separated by preparative chiral HPLC to afford *trans*-1-(4-chlorophenyl)-3-[3-(5-methoxypyridin-2-yl)-1-[(4-methylpyridin-3-yl)carbonyl]piperidin-4-yl]urea ent-1 (**Ex-52**, 0.1 g, 19%) and *trans*-1-(4-chlorophenyl)-3-[3-(5-methoxypyridin-2-yl)-1-[(4-methylpyridin-3-yl)carbonyl]piperidin-4-yl]urea ent-2 (**Ex-53**, 0.077 g, 15%).
**Ex-52:**
LC-MS (Method 3): m/z [M+H]⁺ = 480.4 (exact mass calc. = 479.17).
¹H-NMR (400 MHz, DMSO-d6 at 100 °C): δ 8.44 (s, 1H), 8.38 (s, 1H), 8.18-8.30 (m, 2H), 7.17-7.29 (m, 7H), 5.96 (d, *J=* 7.2 Hz, 1H), 4.61 (bs, 1H), 4.10-4.12 (m, 1H), 3.78 (s, 3H), 2.91-3.35 (m, 4H), 2.29 (s, 3H), 2.04-2.09 (m, 1H), 1.40-1.60 (m, 1H).
**Ex-53:**
LC-MS (Method 3): m/z [M+H]⁺ = 480.4 (exact mass calc. = 479.17).
¹H-NMR (400 MHz, DMSO-d6 at 100 °C): δ 8.44 (s, 1H), 8.38 (s, 1H), 8.19-8.30 (m, 2H), 7.17-7.29 (m, 7H), 5.97 (d, *J*= 7.2 Hz, 1H), 4.59 (bs, 1H), 4.10-4.12 (m, 1H), 3.78 (s, 3H), 2.91-3.34 (m, 4H), 2.29 (s, 3H), 2.04-2.19 (m, 1H), 1.40-1.60 (m, 1H).

### Synthesis of trans-1-(4-chlorophenyl)-3-[3-(5-methoxypyridin-2-yl)-1-[(6-methylpyridin-3-yl)carbonyl]piperidin-4-yl]urea ent-1 (Ex-54) and trans-1-(4-chlorophenyl)-3-[3-(5-methoxypyridin-2-yl)-1-[(6-methylpyridin-3-yl)carbonyl]piperidin-4-yl]urea (Ex-55)

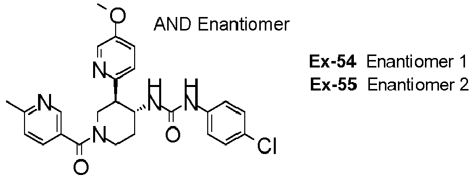

To a stirred solution 6-methylpyridine-3-carboxylic acid (0.223 g, 1.630 mmol, 1.5 eq) in DMF (10 ml) was added EDCI-HCl (0.312 g, 1.630 mmol, 1.5 eq), HOBt (0.176 g, 1.303 mmol, 1.2 eq), DIPEA (0.9 ml, 8.43 mmol, 5.0 eq) and stirred at RT for 30 min followed by addition of *trans*-1-(4-chlorophenyl)-3-(3-(5-methoxypyridin-2-yl)piperidin-4-yl)urea (**BB-III-TFA**, 0.45 g, 1.086 mmol, 1.0 eq). The reaction mixture was stirred for 16 hr at RT. After completion of the reaction (monitored by TLC, TLC system 5% MeOH-DCM, R_{f}: 0.4) reaction mixture was poured into cold water and extracted with EtOAc (2 x 25 ml). The organic layer was washed with water (2 x 25 ml) and brine (2 x 25 mL), dried over anhyd. Na₂SO₄, concentrated in vacuo. The crude material thus obtained was purified by flash chromatography (230-400 mesh silica gel; 5% MeOH-DCM as an elutent) to get *trans*-1-(4-chlorophenyl)-3-[3-(5-methoxypyridin-2-yl)-1-[(6-methylpyridin-3-yl)carbonyl]piperidin-4-yl]urea (0.22 g,42.2%).
0.207 g of *trans*-1-(4-chlorophenyl)-3-[3-(5-methoxypyridin-2-yl)-1-[(6-methylpyridin-3-yl)carbonyl]piperidin-4-yl]urea was separated by preparative chiral HPLC to afford *trans*-1-(4-chlorophenyl)-3-[3-(5-methoxypyridin-2-yl)-1-[(6-methylpyridin-3-yl)carbonyl]piperidin-4-yl]urea ent-1 (**Ex-54,** 0.099g, 19%) and *trans*-1-(4-chlorophenyl)-3-[3-(5-methoxypyridin-2-yl)-1-[(6-methylpyridin-3-yl)carbonyl]piperidin-4-yl]urea (**Ex-55,** 0.066 g, 13%).
**Ex-54:**
LC-MS (Method 3): m/z [M+H]⁺ = 480.4 (exact mass calc. = 479.17).
¹H-NMR (400 MHz, DMSO-d6 at 100 °C): δ 8.50 (s, 1H), 8.20 (s, 2H), 7.71 (d, *J*= 7.8 Hz, 1H), 7.24-7.31 (m, 5H), 7.18 (d, *J* = 8.7 Hz, 2H), 5.92 (d, *J* = 7.7 Hz, 1H), 4.0-4.12 (m, 3H), 3.79 (s, 3H), 3.17-3.25 (m, 2H), 2.93-2.95 (m, 1H), 2.08-2.11 (m, 1H), 1.51-1.60 (m, 1H).
**Ex-55:**
LC-MS (Method 3): m/z [M+H]⁺ = 480.4 (exact mass calc. = 479.17)
¹H-NMR (400 MHz, DMSO-d6 at 100 °C): δ 8.50 (s, 1H), 8.21 (s, 2H), 7.71 (d, *J=* 6.7 Hz, 1H), 7.24-7.30 (m, 5H), 7.18 (d, *J* = 8.5 Hz, 2H), 5.93 (d, *J* = 8.0 Hz, 1H), 4.0-4.11 (m, 3H), 3.79 (s, 3H), 3.17-3.25 (m, 2H), 2.91-2.96 (m, 1H), 2.04-2.11 (m, 1H), 1.54-1.1.57 (m, 1H).

### Synthesis of trans-1-(-3-(5-chlorothiophen-2-yl)-1-((1-(methylsulfonyl)-1H-pyrazol-3-yl)methyl)piperidin-4-yl)-3-phenylurea ent-1 (Ex-56) and trans-1-(-3-(5-chlorothiophen-2-yl)-1-((1-(methylsulfonyl)-1H-pyrazol-3-yl)methyl)piperidin-4-yl)-3-phenylurea ent-2 (Ex-57)

To the stirring solution of 3-[3-(5-chlorothiophen-2-yl)-piperidin-4-yl]-1-phenylurea trifluoroacetate (**BB-IX-TFA,** 0.450 g, 1.002 mmol, 1.0 eq) in acetonitrile (20 ml), K₂CO₃ (0.692 g, 5.011 mmol, 5.0 eq) was added at 0 °C and allowed to stir for 10 minutes, then compound 3-(bromomethyl)-1-methanesulfonyl-1H-pyrazole (0.288 g, 1.202 mmol, 1 eq, see **Ex-42**) was added and again allowed to stir for 24 hours. After completion of reaction water was added, extracted with ethyl acetate (3x100 ml). Ethyl acetate part was collected, dried over Na₂SO₄ and evaporated to dryness to get crude, which was purified by column chromatography to get *trans*-1-(-3-(5-chlorothiophen-2-yl)-1-((1-(methylsulfonyl)-1H-pyrazol-3-yl)methyl)piperidin-4-yl)-3-phenylurea (0.27 g, 55%), followed by chiral prep-HPLC purification to afford *trans*-1-(-3-(5-chlorothiophen-2-yl)-1-((1-(methylsulfonyl)-1H-pyrazol-3-yl)methyl)piperidin-4-yl)-3-phenylurea ent-1 (**Ex-56,** 0.107 g, 22%) *trans*-1-(-3-(5-chlorothiophen-2-yl)-1-((1-(methylsulfonyl)-1H-pyrazol-3-yl)methyl)piperidin-4-yl)-3-phenylurea ent-2 (**Ex-57,** 0.092 g, 19%).
**Ex-56:**
LC-MS (Method 2): m/z [M+H]⁺ = 494.2 (exact mass calc. = 493.1).
¹H-NMR (400 MHz, DMSO-d6): δ 8.30 (s, 1H), 8.21 (s, 1H), 7.31 (d, *J*= 7.9 Hz, 2H), 7.18 (t, *J*= 7.8 Hz, 2H), 6.92 (d, *J*= 3.6 Hz, 1H), 6.86 (t, *J*= 7.2 Hz, 1H), 6.81 (d, *J*= 3.5 Hz, 1H), 6.55 (d, *J*= 2.2 Hz, 1H), 6.15 (d, *J*= 7.9 Hz, 1H), 3.56-3.64 (m, 3H), 3.50 (s, 3H), 3.07 (t, *J*= 9.6 Hz, 1H), 2.98 (d, *J*= 9.7 Hz, 1H), 2.78 (d, *J*= 9.7 Hz, 1H), 2.23-2.29 (m, 2H), 1.94 (d, *J*= 10.9 Hz, 1H), 1.49-1.51 (m, 1H).
**Ex-57:**
LC-MS (Method 3): m/z [M+H]⁺ = 494.3 (exact mass calc. = 493.1).
¹H-NMR (400 MHz, DMSO-d6): δ 8.30 (s, 1H), 8.21 (s, 1H), 7.31 (d, *J*= 7.8 Hz, 2H), 7.18 (t, *J*= 7.6 Hz, 2H), 6.92 (d, *J*= 3.5 Hz, 1H), 6.86 (t, *J*= 7.2 Hz, 1H), 6.81 (d, *J*= 3.3 Hz, 1H), 6.55 (s, 1H), 6.15 (d, *J*= 7.9 Hz, 1H), 3.56-3.64 (m, 3H), 3.50 (s, 3H), 3.08 (t, *J*= 8.4 Hz, 1H), 2.98 (d, *J*= 10.5 Hz, 1H), 2.79 (d, *J*= 10.6 Hz, 1H), 2.23-2.32 (m, 2H), 1.94 (d, *J*= 11.6 Hz, 1H), 1.49-1.51 (m, 1H).

### Synthesis of trans-3-[1-[(1-methanesulfonyl-1H-pyrazol-3-yl)methyl]-3-(5-methoxypyridin-2-yl)piperidin-4-yl]-1-phenylurea ent-1 (Ex-58) and trans-3-[1-[(1-methanesulfonyl-1H-pyrazol-3-yl)methyl]-3-(5-methoxypyridin-2-yl)piperidin-4-yl]-1-phepylurea ent-2 (Ex-59)

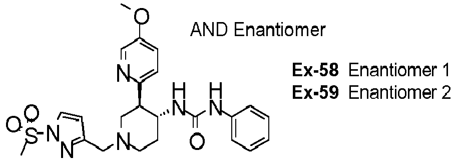

To the stirring solution of *trans*-3-[3-(5-methoxypyridin-2-yl)-1-piperidin-4-yl]-1-phenylurea **(BB-X-TFA,** 0.45 g, 1.0204 mmol, 1.0 eq) in acetonitrile (20 ml), K₂CO₃ (0.414 g, 3.061 mmol, 3.0 eq) was added at 0 °C and allowed to stir for 10 minutes, then compound 3-(bromomethyl)-1-methanesulfonyl-1H-pyrazole (0.243 g, 1.0204 mmol, 1.2 eq, see **Ex-42**) was added and again allowed to stir for 24 hours. After completion of reaction water was added, extracted with ethyl acetate (3x100 ml). Ethyl acetate part was collected, dried over Na₂SO₄ and evaporated to dryness to get crude, which was purified by column chromatography to get *trans*-3-[1-[(1-methanesulfonyl-1H-pyrazol-3-yl)methyl]-3-(5-methoxypyridin-2-yl)piperidin-4-yl]-1-phenylurea.
LC-MS (Method 2): m/z [M+H]⁺ = 485.0 (exact mass calc. = 484.19).
0.28 g of racemic *trans*-3-[1-[(1-methanesulfonyl-1H-pyrazol-3-yl)methyl]-3-(5-methoxypyridin-2-yl)piperidin-4-yl]-1-phenylurea was separated by preparative chiral HPLC to afford *trans*-3-[1-[(1-methanesulfonyl-1H-pyrazol-3-yl)methyl]-3-(5-methoxypyridin-2-yl)piperidin-4-yl]-1-phenylurea ent-1 (**Ex-58,** 0.06907 g, 14%) and *trans*-3-[1-[(1-methanesulfonyl-1H-pyrazol-3-yl)methyl]-3-(5-methoxypyridin-2-yl)piperidin-4-yl]-1-phenylurea ent-2 (**Ex-59,** 0.06546 g, 13%).
**Ex-58:** LC-MS (Method 2): m/z [M+H]⁺ = 485.3 (exact mass calc. = 484.19):
¹H-NMR (400 MHz, DMSO-d6): δ 8.17-8.20 (m, 3H), 7.22-7.30 (m, 4H), 7.14 (t, *J*= 8.1 Hz, 2H), 6.82 (t, *J*= 7.1 Hz, 1H), 6.53 (d, *J*= 2.5 Hz, 1H), 5.93 (d, *J*= 8.1 Hz, 1H), 3.82-3.84 (m, 1H), 3.76 (s, 3H), 3.57 (s, 2H), 3.48 (s, 3H), 2.86-2.88 (s, 3H), 2.15-2.32 (m, 2H), 1.99-2.03 (m, 1H), 1.47-1.53 (m, 1H).
**Ex-59:** LC-MS (Method 3): m/z [M+H]⁺ = 485.4 (exact mass calc. = 484.19).
¹H-NMR (400 MHz, DMSO-d6): δ 8.17-8.19 (m, 3H), 7.12-7.27 (m, 6H), 6.28 (t, *J*= 6.9 Hz, 1H), 6.53 (s, 1H), 5.93 (d, *J*= 7.9 Hz, 1H), 3.82-3.84 (m, 1H), 3.76 (s, 3H), 3.57 (s, 2H), 3.48 (s, 3H), 2.86-2.88 (m, 3H), 1.89-2.33 (m, 3H), 1.48-1.50 (m, 1H).

### Synthesis of trans-1-(4-chlorophenyl)-3-(3-(5-methoxypyridin-2-yl)-1-((6-(trifluoromethyl)pyridin-3-yl)methyl)piperidin-4-yl)urea (Ex-60)

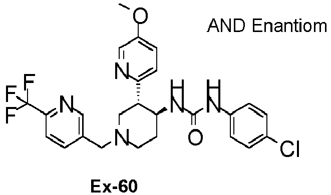

To the stirring solution of [6-(trifluoromethyl)pyridin-3-yl]methanol (0.067 g, 0.379 mmol, 1.2 eq) in DCM (20 ml), DMAP (0.008 g, 0.063 mmol, 0.2 eq) and TEA (0.13 ml, 0.949 mmol, 3 eq) was added at RT and allowed to stir for 10 min. After that TsCl (0.072 g, 0.379 mmol, 1.2 eq) was added at RT and the reaction was continued stirring at the same temperature for 3h. In to the reaction mixture *trans*-1-(4-chlorophenyl)-3-(3-(5-methoxypyridin-2-yl)piperidin-4-yl)urea (**BB-III-HCl,** 0.150 g, 0.316 mmol, 1.0 eq) and K₂CO₃ (0.087 g, 0.632 mmol, 2.0 eq) was added at RT and stirred the reaction mixture for 16h. After completion of reaction (monitored by TLC, 5% MeOH in DCM,Rf=0.3), reaction mixture was filtered and filtrate was evaporated under reduced pressure to get the crude. Crude was purified by column chromatography to afford *trans*-1-(4-chlorophenyl)-3-(3-(5-methoxypyridin-2-yl)-1-((6-(trifluoromethyl)pyridin-3-yl)methyl)piperidin-4-yl, (**Ex-60**, 0.04781 g, 29%). LC-MS (Method 2): m/z [M+H]⁺ = 520.2 (exact mass calc. = 519.16):
¹H-NMR (400 MHz, DMSO-d6): δ 8.69 (s, 1H), 8.34 (s, 1H), 8.17 (d, *J*= 2.7 Hz, 1H), 8.0 (d, *J*= 7.9 Hz, 1H), 7.86 (d, *J*= 8.0 Hz, 1H), 7.18-7.30 (m, 6H), 5.99 (d, *J*= 8.1 Hz, 1H), 3.84-3.87 (m, 1H), 3.75 (s, 3H), 3.61-3.69 (m, 2H), 2.81-2.93 (m, 3H), 2.16-2.32 (m, 2H), 1.98-2.02 (m, 1H), 1.48-1.57 (m, 1H).

### Synthesis of trans-1-(4-chlorophenyl)-3-[3-(5-methoxy-2-pyridyl)-1-[(2-methylpyrimidin-5-yl)methyl]-4-piperidyllurea (Ex-61)

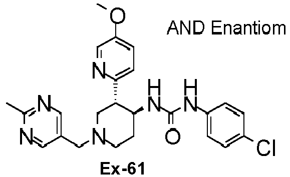

To the stirring solution of (2-methylpyrimidin-5-yl)methanol (0.046 g, 0.379 mmol, 1.2 eq) in DCM (20 ml), DMAP (0.008 g, 0.063 mmol, 0.2 eq) and TEA (0.13 ml, 0.949 mmol, 3 eq) was added at RT and allowed to stirr for 10 min. After that TsCl (0.072 g, 0.379 mmol, 1.2 eq) was added at RT and the reaction was continued stirring at the same temperature for 3 h. In to the reaction mixture 1-(4-chlorophenyl)-3-[3-(5-methoxypyridin-2-yl)piperidin-4-yl]urea trifluoroacetate (**BB-III-TFA,** 0.150 g, 0.316 mmol, 1.0 eq) and K₂CO₃ (0.087 g, 0.632 mmol, 2.0 eq) was added at RT and stirred the reaction mixture for 16 h. After completion of reaction (monitored by TLC), reaction mixture was filtered and filtrate was evaporated under reduced pressure to get the crude. Crude was purified by column chromatography to afford *trans*-1-(4-chlorophenyl)-3-[3-(5-methoxy-2-pyridyl)-1-[(2-methylpyrimidin-5-yl)methyl]-4-piperidyl]urea (**Ex-61,** 0.03342 g, 20%).
LC-MS (Method 2): m/z [M+H]⁺ = 467.5 (exact mass calc. = 466.19).
¹H-NMR (400 MHz, DMSO-d6): δ 8.59 (s, 2H), 8.33 (s, 1H), 8.17 (s, 1H), 7.17-7.30 (m, 6H), 5.97 (d, *J*= 8.3 Hz, 1H), 3.82-3.87 (m, 1H), 3.76 (s, 3H), 3.47-3.55 (m, 2H), 2.79-2.87 (m, 3H), 2.59 (s, 3H), 2.11-2.23 (m, 2H), 1.99-2.02 (m, 1H), 1.48-1.50 (m, 1H).

### Synthesis of trans-1-[3-(5-chlorothionhen-2-yl)-1-(pyrimidin-2-ylmethyl)piperidin-4-yl]-3-(3-methyl-1,2-thiazol-5-yl)urea ent-1 (Ex-62) and trans-1-[3-(5-chlorothiophen-2-yl)-1-(pyrimidin-2-ylmethyl)piperidin-4-yl]-3-(3-methyl-1,2-thiazol-5-yl)urea ent-2 (Ex-63)

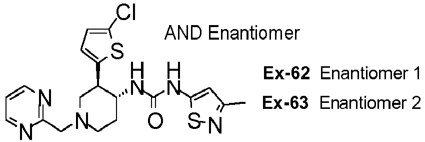

To the stirring solution of **BB-VIII-TFA** (0.400 g, 0.8509 mmol, 1.0 eq) in acetonitrile (20 ml), K₂CO₃ (0.352 g, 2.552 mmol, 3.0 eq) was added at 0 °C and allowed to stir for 10 minutes, then 2-(bromomethyl)pyrimidine (0.147 g, 0.8509 mmol, 1 eq) was added and again allowed to stir for 24 hours. After completion of reaction water was added, extracted with ethyl acetate (3x100 ml). Ethyl acetate part was collected, dried over Na₂SO₄ and evaporated to dryness to get crude, which was purified by column chromatography followed by chiral prep-HPLC purification to afford *trans*-1-[3-(5-chlorothiophen-2-yl)-1-(pyrimidin-2-ylmethyl)piperidin-4-yl]-3-(3-methyl-1,2-thiazol-5-yl)urea ent-1 (**Ex-62,** 0.079 g, 20%) and *trans-*1-[3-(5-chlorothiophen-2-yl)-1-(pyrimidin-2-ylmethyl)piperidin-4-yl]-3-(3-methyl-1,2-thiazol-5-yl)urea ent-2 (**Ex-63,** 0.082 g, 20.3%).
**Ex-62:**
¹H-NMR (400 MHz, DMSO-d6): δ 9.98 (s, 1H), 8.78 (d, *J*= 4.9 Hz, 2H), 7.40 (t, *J*= 4.9 Hz, 1H), 6.91 (d, *J*= 3.7 Hz, 1H), 6.78 (d, *J*= 3.7 Hz, 1H), 6.60-6.70 (m, 1H), 6.44 (s, 1H), 3.77 (s, 2H), 3.50-3.58 (m, 1H), 3.06-3.15 (m, 2H), 2.92 (d, *J*= 11.5 Hz, 1H), 2.30-2.37 (m, 2H), 2.21 (s, 3H), 1.89 (d, *J*= 9.5 Hz, 1H), 1.56 (q*, J*= 10.4 Hz, 1H).
**Ex-63:**
¹H-NMR (400 MHz, DMSO-d6): δ 9.99 (s, 1H), 8.78 (d, *J*= 4.8 Hz, 2H), 7.40 (t, *J*= 4.8 Hz, 1H), 6.91 (d, *J*= 3.7 Hz, 1H), 6.78 (d, *J*= 3.7 Hz, 1H), 6.60-6.70 (m, 1H), 6.44 (s, 1H), 3.77 (s, 2H), 3.54-3.56 (m, 1H), 3.06-3.13 (m, 2H), 2.92 (d, *J*= 9.8 Hz, 1H), 2.33-2.37 (m, 2H), 2.21 (s, 3H), 1.89 (d, *J*= 9.8 Hz, 1H), 1.55-1.57 (m, 1H).

### Synthesis of trans-1-(4-chlorophenyl)-3-(3-(5-methoxypyridin-2-yl)-1-((5-methylpyridin-3-yl)methyl)piperidin-4-yl)urea (Ex-64)

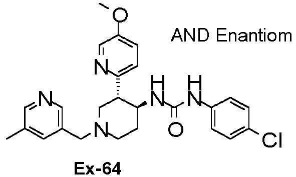

To the stirring solution of (5-methylpyridin-3-yl)methanol (0.046 g, 0.379 mmol, 1.2 eq) in DCM (20 ml), DMAP (0.008 g, 0.063 mmol, 0.2 eq) and TEA (0.13 ml, 0.949 mmol, 3 eq) was added at RT and allowed to stirr for 10 min. After that TsCl (0.072 g, 0.379 mmol, 1.2 eq) was added at RT and the reaction was continued stirring at the same temperature for 3 h. In to the reaction mixture *trans*-1-(4-chlorophenyl)-3-(3-(5-methoxypyridin-2-yl)piperidin-4-yl)urea trifluoroacetate (**BB-III-TFA,** 0.150 g, 0.316 mmol, 1.0 eq) and K2CO3 (0.087 g, 0.632 mmol, 2.0 eq) was added at RT and stirred the reaction mixture for 16 h. After completion of reaction (monitored by TLC), reaction mixture was filtered and filtrate was evaporated under reduced pressure to get the crude. Crude was purified by column chromatography to afford *trans*-1-(4-chlorophenyl)-3-(3-(5-methoxypyridin-2-yl)-1-((5-methylpyridin-3-yl)methyl)piperidin-4-yl)urea (**Ex-64,** 0.025 g, 17%).
LC-MS (Method 1): m/z [M+H]⁺ = 466.2 (exact mass calc. = 465.19).
¹H-NMR (400 MHz, DMSO-d6): δ 8.28-8.33 (m, 3H), 8.17 (s, 1H), 7.51 (s, 1H), 7.28-7.30 (m, 3H), 7.18-7.23 (m, 3H), 5.97 (d, *J=* 7.4 Hz, 1H), 3.85-3.90 (m, 1H), 3.76 (s, 3H), 3.44-3.53 (m, 2H), 2.79-2.87 (m, 3H), 2.28 (s, 3H), 1.98-2.21 (m, 3H), 1.49-1.51 (m, 1H).

### Synthesis of trans-1-(4-chlorophenyl)-3-[1-[(6-cyclopropylpyridin-3-yl)methyl]-3-(5-methoxypyridin-2-yl)piperidin-4-yl]urea (Ex-65)

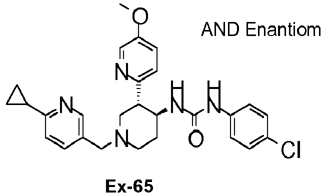

To the stirring solution of *trans*-1-(4-chlorophenyl)-3-(3-(5-methoxypyridin-2-yl)piperidin-4-yl)urea trifluoroacetate (**BB-III-TFA,** 0.12 g, 0.253 mmol, 1.0 eq) in acetonitrile (20 ml), K₂CO₃ (0.105 g, 0.759 mmol, 3.0 eq) was added at 0 °C and allowed to stir for 10 minutes, then 5-(bromomethyl)-2-cyclopropylpyridine (0.064 g, 0.3038 mmol, 1.2 eq) was added and again allowed to stir for 24 hours. After completion of reaction water was added, extracted with ethyl acetate (3x100 ml). Ethyl acetate part was collected, dried over Na₂SO₄ and evaporated to dryness to get crude, which was purified by column chromatography to afford *trans*-1-(4-chlorophenyl)-3-[1-[(6-cyclopropylpyridin-3-yl)methyl]-3-(5-methoxypyridin-2-yl)piperidin-4-yl]urea (**Ex-65,** 0.06286 g, 50.49%). LC-MS (Method 2): m/z [M+H]⁺ = 492.3 (exact mass calc. = 491.21).
¹H-NMR (400 MHz, DMSO-d6): δ 8.32 (s, 1H), 8.27 (s, 1H), 8.17 (s, 1H), 7.53 (d, *J*= 7.5 Hz, 1H), 7.28 (d, *J*= 8.7 Hz, 3H), 7.18-7.22 (m, 4H), 5.99 (d, *J*= 7.4 Hz, 1H), 3.82-3.90 (m, 1H), 3.76 (s, 3H), 3.40-3.50 (m, 2H), 2.77-2.85 (m, 3H), 1.98-2.17 (m, 4H), 1.44-1.53 (m, 1H), 0.88-0.91 (m, 4H).

### Synthesis of trans-1-(4-chlorophenyl)-3-[3-(5-methoxypyridin-2-yl)-1-[(2-methoxypyrimidin-5-yl)methyl]piperidin-4-yl]urea (Ex-66)

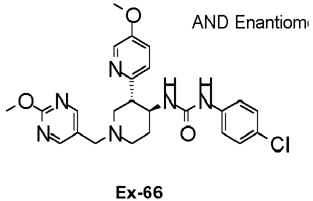

To the stirring solution of compound 2-methoxypyrimidin-5-yl)methanol (0.053 g, 0.379 mmol, 1.2 eq) in DCM (20 ml), DMAP (0.008 g, 0.063 mmol, 0.2 eq) and TEA (0.13 ml, 0.949 mmol, 3 eq) was added at RT and allowed to stir for 10 min. After that TsCl (0.072 g, 0.379 mmol, 1.2 eq) was added at RT and the reaction was continued stirring at the same temperature for 3 h. In to the reaction mixture *trans*-1-(4-chlorophenyl)-3-(3-(5-methoxypyridin-2-yl)piperidin-4-yl)urea trifluoroacetate (**BB-III-TFA,** 0.150 g, 0.316 mmol, 1.0 eq) and K₂CO₃ (0.087 g, 0.632 mmol, 2.0 eq) was added at RT and stirred the reaction mixture for 16 h. After completion of reaction (monitored by TLC), reaction mixture was filtered and filtrate was evaporated under reduced pressure to get the crude. Crude was purified by column chromatography to afford *trans*-1-(4-chlorophenyl)-3-[3-(5-methoxypyridin-2-yl)-1-[(2-methoxypyrimidin-5-yl)methyl]piperidin-4-yl]urea (**Ex-66,** 0.048 g, 31.5%).
LC-MS (Method 2): m/z [M+H]⁺ = 492.3 (exact mass calc. = 491.21).
¹H-NMR (400 MHz, DMSO-d6): δ 8.50 (s, 2H), 8.32 (s, 1H), 8.17 (s, 1H), 7.28 (d, *J*= 8.7 Hz, 3H), 7.13-7.23 (m, 3H), 5.97 (d, *J*= 7.3 Hz, 1H), 3.89 (s, 3H), 3.83-3.84 (m, 1H), 3.76 (s, 3H), 3.46-3.48 (m, 2H), 2.80-2.86 (m, 3H), 2.10-2.21 (m, 2H), 1.98-2.02 (m, 1H), 1.47-1.50 (m, 1H).

### Synthesis of trans-1-(4-chlorophenyl)-3-[-3-(5-methoxypyridin-2-yl)-1-[(6-methylpyridin-3-yl)methyl]piperidin-4-yl]urea (Ex-67)

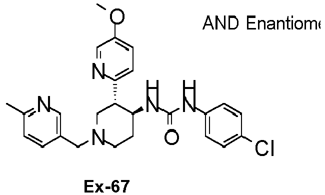

To the stirring solution of compound 6-methylpyridin-3-yl)methanol (0.046 g, 0.379 mmol, 1.2 eq) in DCM (20 ml), DMAP (0.008 g, 0.063 mmol, 0.2 eq) and TEA (0.13 ml, 0.949 mmol, 3 eq) was added at RT and allowed to stirr for 10 min. After that TsCl (0.072 g, 0.379 mmol, 1.2 eq) was added at RT and the reaction was continued stirring at the same temperature for 3 h. In to the reaction mixture *trans*-1-(4-chlorophenyl)-3-(3-(5-methoxypyridin-2-yl)piperidin-4-yl)urea trifluoroacetate (**BB-III-TFA,** 0.150 g, 0.316 mmol, 1.0 eq) and K₂CO₃ (0.087 g, 0.632 mmol, 2.0 eq) was added at RT and stirred the reaction mixture for 16 h. After completion of reaction (monitored by TLC), reaction mixture was filtered and filtrate was evaporated under reduced pressure to get the crude. Crude was purified by column chromatography to afford *trans*-1-(4-chlorophenyl)-3-[-3-(5-methoxypyridin-2-yl)-1-[(6-methylpyridin-3-yl)methyl]piperidin-4-yl]urea (**Ex-67,** 0.02546 g, 17.28%).
LC-MS (Method 2): m/z [M+H]⁺ = 466.2 (exact mass calc. = 465.1).
¹H-NMR (400 MHz, CD₃OD): δ 8.37 (d, *J*= 1.5 Hz, 1H), 8.13 (d, *J*= 2.5 Hz, 1H), 7.71-7.74 (m, 1H), 7.27-7.34 (m, 3H), 7.15-7.21 (m, 4H), 4.0-4.01 (m, 1H), 3.82 (s, 3H), 3.61 (q, *J*= 13.7 Hz, 2H), 2.95-3.02 (m, 3H), 2.52 (s, 3H), 2.32 (t, *J*= 11.5 Hz, 2H), 2.12-2.14 (m, 1H), 1.66-1.69 (m, 1H).

### Synthesis of trans-(4-chlorophenyl)-3-[1-[(5-fluoropyridin-3-yl)methyl]-3-(5-methoxypyridin-2-yl)piperidin-4-yllurea (Ex-68)

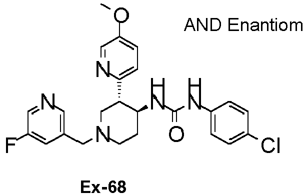

To the stirring solution of compound 1-(4-chlorophenyl)-3-[3-(5-methoxypyridin-2-yl)-piperidin-4-yl]urea trifluoroacetate (**BB-III-TFA,** 0.12 g, 0.253 mmol, 1.0 eq) in acetonitrile (20 ml), K₂CO₃ (0.177 g, 1.265 mmol, 5.0 eq) was added at 0 °C and allowed to stir for 10 minutes, then 3-(bromomethyl)-5-fluoropyridine (0.0573 g, 0.3037 mmol, 1.2 eq) was added and again allowed to stir for 24 hours. After completion of reaction water was added, extracted with ethyl acetate (3x100 ml). Ethyl acetate part was collected, dried over Na₂SO₄ and evaporated to dryness to get crude, which was purified by column chromatography to afford (±)*trans*-(4-chlorophenyl)-3-[1-[(5-fluoropyridin-3-yl)methyl]-3-(5-methoxypyridin-2-yl)piperidin-4-yl]urea (**Ex-68,** 0.03454 g, 29%).
LC-MS (Method3): m/z [M+H]⁺ = 470.4 (exact mass calc. = 469.17).
¹H-NMR (400 MHz, DMSO-d6): δ 8.46 (d, *J=* 2.1 Hz, 1H), 8.38 (s, 1H), 8.34 (s, 1H), 8.17 (d, *J=* 2.7 Hz, 1H), 7.64 (d, *J*= 9.6 Hz, 1H), 7.17-7.30 (m, 6H), 5.98 (d, *J*= 8.1 Hz, 1H), 3.83-3.86 (m, 1H), 3.75 (s, 3H), 3.54-3.62 (m, 2H), 2.81-2.92 (m, 3H), 2.14-2.26 (m, 2H), 2.01 (d, *J*= 9.6 Hz, 1H), 1.47-1.55 (m, 1H).

### Synthesis of trans-1-(4-chlorophenyl)-3-[1-[(5-chloropyridin-3-yl)methyl]-3-(5-methoxypyridin-2-yl)piperidin-4-yllurea (Ex-69)

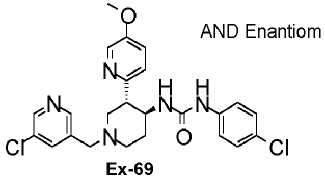

To the stirring solution of (5-chloropyridin-3-yl)methanol (0.054 g, 0.379 mmol, 1.2 eq) in DCM (20 ml), DMAP (0.008 g, 0.063 mmol, 0.2 eq) and TEA (0.13 ml, 0.949 mmol, 3 eq) was added at RT and allowed to stir for 10 min. After that TsCl (0.072 g, 0.379 mmol, 1.2 eq) was added at RT and the reaction was continued stirring at the same temperature for 3 h. In to the reaction mixture *trans*-1-(4-chlorophenyl)-3-(3-(5-methoxypyridin-2-yl)piperidin-4-yl)urea trifluoroacetate (**BB-III-TFA**, 0.150 g, 0.316 mmol, 1.0 eq) and K₂CO₃ (0.087 g, 0.632 mmol, 2.0 eq) was added at RT and stirred the reaction mixture for 16 h. After completion of reaction (monitored by TLC), reaction mixture was filtered and filtrate was evaporated under reduced pressure to get the crude. Crude was purified by column chromatography to afford compound *trans*-1-(4-chlorophenyl)-3-[1-[(5-chloropyridin-3-yl)methyl]-3-(5-methoxypyridin-2-yl)piperidin-4-yl]urea (**Ex-69,** 0.03039 g, 19.76%).
LC-MS (Method 2): m/z [M+H]⁺ = 486.3 (exact mass calc. = 485.14).
¹H-NMR (400 MHz, DMSO-d6): δ 8.51 (s, 1H), 8.46 (s, 1H), 8.34 (s, 1H), 8.17 (s, 1H), 7.84 (s, 1H), 7.17-7.30 (m, 6H), 5.97 (d, *J*= 7.8 Hz, 1H), 3.83-3.89 (m, 1H), 3.76 (s, 3H), 3.52-3.60 (m, 2H), 2.80-2.90 (m, 3H), 2.14-2.26 (m, 2H), 2.01 (d, *J***=** 9.6 Hz, 1H), 1.47-1.56 (m, 1H).

### Synthesis of trans-1-(4-chlorophenyl)-3-(3-(5-chlorothiophen-2-yl)-1-((5-fluoropyridin-3-yl)methyl)piperidin-4-yl)urea (Ex-70)

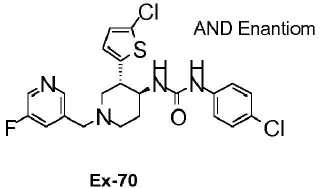

To the stirring solution of *trans*-1-(4-chlorophenyl)-3-(3-(5-chlorothiophen-2-yl)piperidin-4-yl)urea trifluoroacetate (**BB-VII-TFA,** 0.120 g, 0.2479 mmol, 1.0 eq) in acetonitrile (20 ml), K₂CO₃ (0.171 g, 1.24 mmol, 5.0 eq) was added at 0 °C and allowed to stir for 10 minutes, then 3-(bromomethyl)-5-fluoropyridine (0.0526 g, 0.2975 mmol, 1.2 eq) was added and again allowed to stir for 24 hours. After completion of reaction water was added, extracted with ethyl acetate (3x100 ml). Ethyl acetate part was collected, dried over Na₂SO₄ and evaporated to dryness to get crude, which was purified by column chromatography to afford compound *trans*-1-(4-chlorophenyl)-3-(3-(5-chlorothiophen-2-yl)-1-((5-fluoropyridin-3-yl)methyl)piperidin-4-yl)urea (**Ex-70,** 0.02525 g, 21%).
LC-MS (Method3): m/z [M+H]⁺ = 479.2 (exact mass calc. = 478.08).
¹H-NMR (400 MHz, DMSO-d6): δ 8.51 (s, 1H), 8.47 (d, *J*= 2.7 Hz, 1H), 8.41 (s, 1H), 7.66 (d, *J*= 9.9 Hz, 1H), 7.35 (d, *J*= 8.9 Hz, 2H), 7.22 (d, *J*= 8.8 Hz, 2H), 6.92 (d, *J*= 3.8 Hz, 1H), 6.80 (d, *J*= 3.7 Hz, 1H), 6.25 (d, *J*= 8.2 Hz, 1H), 3.56-3.65 (m, 3H), 3.07-3.13 (m, 1H), 2.94 (d, *J*= 11.2 Hz, 1H), 2.74-2.77 (m, 1H), 2.20-2.32 (m, 2H), 1.92-1.94 (m, 1H), 1.48-1.56 (m, 1H).

### Synthesis of trans-1-(4-chlorophenyl)-3-(1-((5-chloropyridin-3-yl)methyl)-3-(5-chlorothiophen-2-yl)piperidin-4-yl)urea (Ex-71)

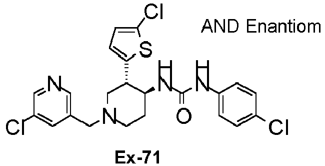

To the stirring solution of compound *trans*-1-(4-chlorophenyl)-3-(3-(5-chlorothiophen-2-yl)piperidin-4-yl)urea trifluoroacetate (**BB-VI-TFA,** 0.120 g, 0.248 mmol, 1.0 eq) in acetonitrile (20 ml), K₂CO₃ (0.103 g, 0.744 mmol, 3.0 eq) was added at 0 °C and allowed to stir for 10 minutes, then 3-(bromomethyl)-5-chloropyridine (0.061 g, 0.297 mmol, 1.2 eq) was added and again allowed to stir for 24 hours. After completion of reaction water was added, extracted with ethyl acetate (3x100 ml). Ethyl acetate part was collected, dried over Na₂SO₄ and evaporated to dryness to get crude, which was purified by column chromatography to afford compound *trans*-1-(4-chlorophenyl)-3-(1-((5-chloropyridin-3-yl)methyl)-3-(5-chlorothiophen-2-yl)piperidin-4-yl)urea (**Ex-71,** 0.01564 g, 13%).
LC-MS (Method 1): m/z [M+H]⁺ = 495.2 (exact mass calc. = 494.05).
¹H-NMR (400 MHz, DMSO-d6): δ 8.53 (d, *J*= 2.1 Hz, 1H), 8.48 (s, 2H), 7.86 (s, 1H), 7.35 (d, *J*= 8.7 Hz, 2H), 7.22 (d, *J*= 8.7 Hz, 2H), 6.92 (d, *J*= 3.7 Hz, 1H), 6.80 (d, *J*= 3.6 Hz, 1H), 6.20 (d, *J*= 8.1 Hz, 1H), 3.55-3.63 (m, 3H), 3.07-3.31 (m, 1H), 2.93 (d, *J*= 9.7 Hz, 1H), 2.73-2.76 (m, 1H), 2.22-2.27 (m, 2H), 1.92-1.94 (m, 1H), 1.48-1.55 (m, 1H).

### Synthesis of trans-1-(4-chlorophenyl)-3-(3-(5-chlorothiophen-2-yl)-1-((5-methylpyridin-3-yl)methyl)piperidin-4-yl)urea (Ex-72)

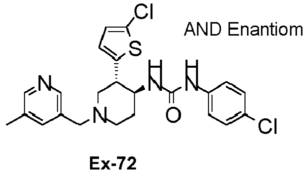

To the stirring solution of *trans-*1-(4-chlorophenyl)-3-(3-(5-chlorothiophen-2-yl)piperidin-4-yl)urea trifluoroacetate (**BB-VII-TFA,** 0.120 g, 0.248 mmol, 1.0 eq) in acetonitrile (20 ml), K₂CO₃ (0.103 g, 0.744 mmol, 3.0 eq) was added at 0 °C and allowed to stir for 10 minutes, then 3-(bromomethyl)-5-methylpyridine (0.055 g, 0.297 mmol, 1.2 eq) was added and again allowed to stir for 24 hours. After completion of reaction water was added, extracted with ethyl acetate (3x100 ml). Ethyl acetate part was collected, dried over Na₂SO₄ and evaporated to dryness to get crude, which was purified by column chromatography to afford compound of *trans*-1-(4-chlorophenyl)-3-(3-(5-chlorothiophen-2-yl)-1-((5-methylpyridin-3-yl)methyl)piperidin-4-yl)urea (**Ex-72,** 0.015 g, 13%)
LC-MS (Method 3): m/z [M+H]⁺ = 475.3 (exact mass calc. = 474.1).
¹H-NMR (400 MHz, DMSO-d6): δ 8.45 (s, 1H), 8.28 (s, 2H), 7.50 (s, 1H), 7.32 (d, *J*= 8.8 Hz, 2H), 7.19 (d, *J*= 8.8 Hz, 2H), 6.89 (d, *J*= 3.6 Hz, 1H), 6.77 (d, *J*= 3.7 Hz, 1H), 6.18 (d, *J*= 7.6 Hz, 1H), 3.49-3.53 (m, 3H), 3.0-3.10 (m, 1H), 2.88-2.90 (m, 1H), 2.70-2.71 (m, 1H), 2.26 (s, 3H), 2.0-2.18 (m, 2H), 1.89-1.92 (m, 1H), 1.47-1.49 (m, 1H).

### Synthesis of trans-1-(4-chlorophenyl)-3-[3-(5-chlorothiophen-2-yl)-1-[(6-cyclopropylpyridin-3-yl)methyl]piperidin-4-yl]urea (Ex-73)

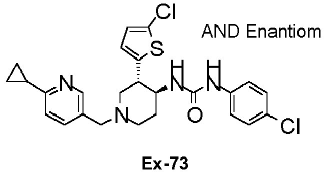

To the stirring solution of 1-(4-chlorophenyl)-3-[3-(5-chlorothiophen-2-yl)-piperidin-4-yl]urea trifluoroacetate (**BB-VII-TFA,** (0.120 g, 0.248 mmol, 1.0 eq) in acetonitrile (20 ml), K₂CO₃ (0.171 g, 1.24 mmol, 5.0 eq) was added at 0 °C and allowed to stir for 10 minutes, then 5-(bromomethyl)-2-cyclopropylpyridine (0.063 g, 0.2975 mmol, 1.2 eq) was added and again allowed to stir for 24 hours. After completion of reaction water was added, extracted with ethyl acetate (3x100 ml). Ethyl acetate part was collected, dried over Na₂SO₄ and evaporated to dryness to get crude, which was purified by column chromatography to afford compound *trans*-1-(4-chlorophenyl)-3-[3-(5-chlorothiophen-2-yl)-1-[(6-cyclopropylpyridin-3-yl)methyl]piperidin-4-yl]urea (**Ex-73,** 0.02435g, 19.61%).
LC-MS (Method 3): m/z [M+H]⁺ = 501.3 (exact mass calc. = 500.12).
¹H-NMR (400 MHz, DMSO-d6): δ 8.45 (s, 1H), 8.30 (s, 1H), 7.54-7.56 (m, 1H), 7.34 (d, *J*= 8.8 Hz, 2H), 7.23 (t, *J*= 7.8 Hz, 3H), 6.91 (d, *J*= 3.7 Hz, 1H), 6.79 (d, *J*= 3.7 Hz, 1H), 6.19 (d, *J*= 8.2 Hz, 1H), 3.52-3.60 (m, 1H), 3.48 (s, 2H), 3.04-3.08 (m, 1H), 2.88-2.91 (m, 1H), 2.71-2.74 (m, 1H), 2.13-2.20 (m, 2H), 2.02-2.09 (m, 1H), 1.90-1.93 (m, 1H), 1.44-1.53 (m, 1H), 0.80-0.94 (m, 4H).

### Synthesis of trans-1-(4-chlorophenyl)-3-[3-(5-chlorothiophen-2-yl)-1-{[6-(trifluoromethyl)pyridin-3-yl]methyl}piperidin-4-yl]urea (Ex-74)

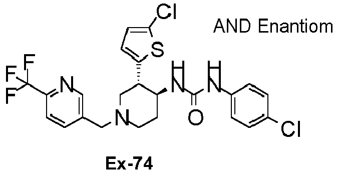

To the stirring solution of *trans*-1-(4-chlorophenyl)-3-[3-(5-chlorothiophen-2-yl)-piperidin-4-yl]urea trifluoroacetate **(BB-VII-TFA,** 0.120 g, 0.248 mmol, 1.0 eq) in acetonitrile (20 ml), K₂CO₃ (0.103 g, 0.744 mmol, 3.0 eq) was added at 0 °C and allowed to stir for 10 minutes, then 5-(bromomethyl)-2-(trifluoromethyl)pyridine (0.071 g, 0.297 mmol, 1.2 eq) was added and again allowed to stir for 24 hours. After completion of reaction water was added, extracted with ethyl acetate (3x100 ml). Ethyl acetate part was collected, dried over Na₂SO₄ and evaporated to dryness to get crude, which was purified by column chromatography to afford *trans*-1-(4-chlorophenyl)-3-[3-(5-chlorothiophen-2-yl)-1-{[6-(trifluoromethyl)pyridin-3-yl]methyl}piperidin-4-yl]urea (**Ex-74,** 0.028 g, 21%).
LC-MS (Method 3): m/z [M+H]⁺ = 529.3 (exact mass calc. = 528.08).
¹H-NMR (400 MHz, DMSO-d6): δ 8.71 (s, 1H), 8.47 (s, 1H), 8.01 (d, *J*= 7.1 Hz, 1H), 7.88 (d, *J*= 7.8 Hz, 1H), 7.35 (d, *J*= 8.3 Hz, 2H), 7.22 (d, *J*= 8.3 Hz, 2H), 6.91 (s, 1H), 6.81 (s, 1H), 6.22 (d, *J*= 7.6 Hz, 1H), 3.58-3.68 (m, 3H), 3.10-3.20 (m, 1H), 2.95 (d, *J*= 10.0 Hz, 1H), 2.70-2.80 (m, 1H), 2.24-2.29 (m, 2H), 1.94 (d, *J*= 10.3 Hz, 1H), 1.51-1.54 (m, 1H).

### Synthesis of trans-1-(4-chlorophenyl)-3-[3-(5-chlorothiophen-2-yl)-1-[(2-methoxypyrimidin-5-yl)methyl]piperidin-4-yl]urea (Ex-75)

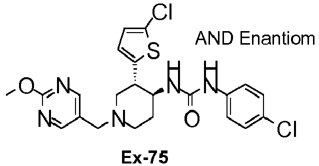

To the stirring solution of *trans*-1-(4-chlorophenyl)-3-[3-(5-chlorothiophen-2-yl)-1-piperidin-4-yl]urea trifluoroacetate **(BB-VII-TFA,** 0.120 g, 0.248 mmol, 1.0 eq) in acetonitrile (20 ml), K₂CO₃ (0.103 g, 0.744 mmol, 3.0 eq) was added at 0 °C and allowed to stir for 10 minutes, then (2-methoxypyrimidin-5-yl)methyl 4-methylbenzene-1-sulfonate (0.087 g, 0.297 mmol, 1.2 eq) was added and again allowed to stir for 24 hours. After completion of reaction water was added, extracted with ethyl acetate (3x100 ml). Ethyl acetate part was collected, dried over Na₂SO₄ and evaporated to dryness to get crude, which was purified by column chromatography to afford compound compound *trans*-1-(4-chlorophenyl)-3-[3-(5-chlorothiophen-2-yl)-1-[(2-methoxypyrimidin-5-yl)methyl]piperidin-4-yl]urea (**Ex-75,** 0.024g, 19.63%).
LC-MS (Method 3): m/z [M+H]⁺ = 492.4 (exact mass calc. = 491.09).
¹H-NMR (400 MHz, DMSO-d6): δ 8.46-8.52 (m, 3H), 7.35 (d, *J*= 8.8 Hz, 2H), 7.22 (d, *J*= 8.7 Hz, 2H), 6.92 (d, *J=* 3.6 Hz, 1H), 6.80 (d, *J*= 3.6 Hz, 1H), 6.21 (d, *J*= 7.8 Hz, 1H), 3.90 (s, 3H), 3.50-3.57 (m, 3H), 3.07 (t, *J*= 7.9 Hz, 1H), 2.91 (d, *J*= 9.7 Hz, 1H), 2.70-2.80 (m, 1H), 2.10-2.30 (m, 2H), 1.93 (d, *J*= 10.0 Hz, 1H), 1.48-1.50 (m, 1H).

### Synthesis of trans-1-(4-chloropheyl)-3-[3-(5-chlorothiophen-2-yl)-1-[(2-methylpyrimidin-5-yl)methyl]piperidin-4-yl]urea (Ex-76)

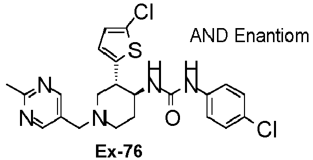

To the stirring solution of compound *trans*-1-(4-chlorophenyl)-3-[3-(5-chlorothiophen-2-yl)-piperidin-4-yl]urea trifluoroacetate (**Ex-76,** 0.120 g, 0.248 mmol, 1.0 eq) in acetonitrile (20 ml), K₂CO₃ (0.103 g, 0.744 mmol, 3.0 eq) was added at 0 °C and allowed to stir for 10 minutes, then 5-(bromomethyl)-2-methylpyrimidine (0.055 g, 0.297 mmol, 1.2 eq) was added and again allowed to stir for 24 hours. After completion of reaction water was added, extracted with ethyl acetate (3x100 ml). Ethyl acetate part was collected, dried over Na₂SO₄ and evaporated to dryness to get crude, which was purified by column chromatography to afford compound compound *trans*-1-(4-chlorophenyl)-3-[3-(5-chlorothiophen-2-yl)-1-[(2-methylpyrimidin-5-yl)methyl]piperidin-4-yl]urea (**Ex-76,** 0.05053 g, 39.43%).
LC-MS (Method 3): m/z [M+H]⁺ = 476.3 (exact mass calc. = 475.1).
¹H-NMR (400 MHz, DMSO-d6): δ 8.61 (s, 2H), 8.47 (s, 1H), 7.35 (d, *J*= 8.7 Hz, 2H), 7.22 (d, *J*= 8.7 Hz, 2H), 6.91 (d, *J*= 3.7 Hz, 1H), 6.80 (d, *J*= 3.6 Hz, 1H), 6.22 (d, *J*= 8.1 Hz, 1H), 3.49-3.57 (m, 3H), 3.05-3.10 (m, 1H), 2.91 (d, *J*= 9.7 Hz, 1H), 2.74 (d, *J*= 10.1 Hz, 1H), 2.59 (s, 3H), 2.20-2.24 (m, 2H), 1.93 (d, *J*= 10.1 Hz, 1H), 1.50 (q, *J*= 10.1 Hz, 1H).

### Synthesis of trans-1-(4-chlorophenyl)-3-[3-(5-chlorothiophen-2-yl)-1-[(1-methanesulfonyl-1H-pyrazol-3-yl)methyl]piperidin-4-yl]urea ent-1 (Ex-77) and trans-1-(4-chlorophenyl)-3-[3-(5-chlorothiophen-2-yl)-1-[(1-methanesulfonyl-1H-pyrazol-3-yl)methyl]piperidin-4-yl]urea ent-2 (Ex-78)

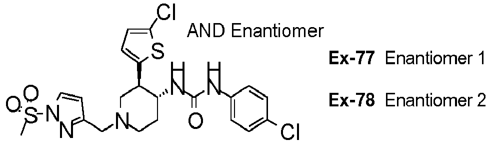

To the stirring solution of **BB-VII-TFA** (0.270 g, 0.5578 mmol, 1.0 eq) in acetonitrile (20 ml), K₂CO₃ (0.384 g, 2.789 mmol, 5.0 eq) was added at 0 °C and allowed to stir for 10 minutes, then 3-(bromomethyl)-1-methanesulfonyl-1H-pyrazole (0.160 g, 0.6694 mmol, 1.2 eq) was added and again allowed to stir for 24 hours. After completion of reaction water was added, extracted with ethyl acetate (3x100 ml). Ethyl acetate part was collected, dried over Na₂SO₄ and evaporated to dryness to get crude, which was purified by column chromatography to get *trans*-1-(4-chlorophenyl)-3-[3-(5-chlorothiophen-2-yl)-1-[(1-methanesulfonyl-1H-pyrazol-3-yl)methyl]piperidin-4-yl]urea (0.24g, 81.5%).
LC-MS (Method 2): m/z [M+H]⁺ = 527.9 (exact mass calc. = 527.06).
0.24g of racemic *trans-*1-(4-chlorophenyl)-3-[3-(5-chlorothiophen-2-yl)-1-[(1-methanesulfonyl-1H-pyrazol-3-yl)methyl]piperidin-4-yl]urea was separated by preparative chiral HPLC to afford both the enantiomers *trans-*1-(4-chlorophenyl)-3-[3-(5-chlorothiophen-2-yl)-1-[(1-methanesulfonyl-1H-pyrazol-3-yl)methyl]piperidin-4-yl]urea ent-1 (**Ex-77,** 0.05554g, 18.86%) and *trans*-1-(4-chlorophenyl)-3-[3-(5-chlorothiophen-2-yl)-1-[(1-methanesulfonyl-1H-pyrazol-3-yl)methyl]piperidin-4-yl]urea ent-2 (**Ex-78,** 0.05987g, 20.33%).
**Ex-77:** LC-MS (Method 3): m/z [M+H]⁺ = 528.2 (exact mass calc. = 527.06).
¹H-NMR (400 MHz, DMSO-d6): δ 8.45 (s, 1H), 8.21 (d, *J*= 2.4 Hz, 1H), 7.35 (d, *J*= 8.7 Hz, 2H), 7.22 (d, *J*= 8.7 Hz, 2H), 6.92 (d, *J*= 3.6 Hz, 1H), 6.80 (d, *J*= 3.6 Hz, 1H), 6.54 (d, *J*= 2.4 Hz, 1H), 6.20 (d, *J*= 8.3 Hz, 1H), 3.56-3.64 (m, 3H), 3.49 (s, 3H), 3.06-3.11 (m, 1H), 2.98 (d, *J*= 10.5 Hz,1H), 2.79 (d, *J*= 10.3 Hz, 1H), 2.20-2.32 (m, 2H), 1.91-2.07 (m, 1H), 1.47-1.55 (m, 1H).
**Ex-78:** LC-MS (Method 3): m/z [M+H]⁺ = 528.3 (exact mass calc. = 527.06).
¹H-NMR (400 MHz, DMSO-d6): δ 8.45 (s, 1H), 8.21 (d, *J*= 2.4 Hz, 1H), 7.35 (d, *J*= 8.7 Hz, 2H), 7.22 (d, *J*= 8.8 Hz, 2H), 6.92 (d, *J*= 3.6 Hz, 1H), 6.80 (d, *J*= 3.6 Hz, 1H), 6.54 (d, *J*= 2.4 Hz, 1H), 6.21 (d, *J*= 8.2 Hz, 1H), 3.56-3.64 (m, 3H), 3.49 (s, 3H), 3.06-3.11 (m, 1H), 2.98 (d, *J*= 10.3 Hz,1H), 2.79 (d, *J*= 10.3 Hz, 1H), 2.20-2.32 (m, 2H), 1.91-1.94 (m, 1H), 1.47-1.55 (m, 1H).

### Synthesis of trans-1-(4-chloro-phepyl)-3-[5-methoxy-1'-(3-methoxy-propionyl)-1',2',3',4',5',6'-hexahydro-[2,3']bipyridinyl-4'-yl]-urea (Ex-79)

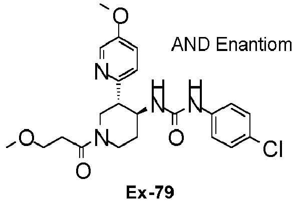

To a stirred solution of 3-methoxy-propionic acid (35 mg, 0.336 mmol, 1.0 eq) in dry THF (10 ml) were added EDCI-HCl (96 mg, 0.504 mmol, 1.5 eq) and HOBt (68 mg, 0.504 mmol, 1.5 eq) in ice-cold condition followed by addition of TEA (140 µl, 1.008 mmol, 3 eq) and 1-(4-chloro-phenyl)-3-(5-methoxy-1',2',3',4',5',6'-hexahydro-[2,3']bipyridinyl-4'-yl)-urea hydrochloride (**BB-III-HCl,** 160 mg, 0.403 mmol, 1.2 eq) and stirred at RT for 16 h. The reaction mixture was diluted with EtOAc and washed with water and brine. The organic layer was dried over anhyd. Na₂SO₄ and concentrated under reduced pressure to obtain crude product which was purified by combi flash column chromatography and followed by reverse phase prep-HPLC purification to afford *trans*-1-(4-chlorophenyl)-3-[5-methoxy-1'-(3-methoxy-propionyl)-1',2',3',4',5',6'-hexahydro-[2,3']bipyridinyl-4'-yl]-urea (**Ex-79,** 25 mg, 17% yield) as off white solid.
LC-MS (Method 1): m/z [M+H]⁺ = 447.2 (exact mass calc. = 446.17).
¹H-NMR (400 MHz, DMSO-d6, 100 °C): δ 8.22 (s, 1 H), 8.13 (s, 1 H), 7.28 (d, *J* = 8 Hz, 4 H), 7.17 (d, *J* = 8 Hz, 2 H), 5.88 (d, *J* = 8 Hz, 1 H), 4.36-4.31 (m, 1 H), 4.25-4.08 (m, 2 H), 3.80 (s, 3 H), 3.61-3.60 (m, 2 H), 3.26 (s, 3 H), 2.98 (s, 2 H), 2.81-2.71 (m, 1 H), 2.59 (s, 2 H), 2.07 (d, *J* = 12 Hz, 1 H), 1.44 (d, *J* = 8 Hz, 1 H).

### Synthesis of trans-1-(4-chloro-phenyl)-3-[5-methoxy-1'-(3-methoxy-propyl)-1',2',3',4',5',6'-hexahydro-[2,3']bipyridinyl-4'-yl]-urea (Ex-80)

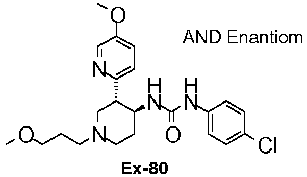

To a stirred solution of 1-(4-chloro-phenyl)-3-(5-methoxy-1',2',3',4',5',6'-hexahydro-[2,3']bipyridinyl-4'-yl)-urea hydrochloride (**BB-III-HCl,** 120 mg, 0.303 mmol, 1.0 eq) in dry ACN (7 ml) was added Cs₂CO₃ (395 mg, 1.21 mmol, 4 eq) followed by the addition of 1-bromo-3-methoxy-propane (92 mg, 0.606 mmol, 2.0 eq) at ice cooled condition and stirred at RT for a period of 18 h. After completion of the reaction it was evaporated under reduced pressure and diluted with ethyl acetate (100 ml) and washed with ice-cold water (50 ml) and brine (25 ml). The organic layer was then dried over anhyd. Na₂SO₄ and concentrated under reduced pressure to obtain crude product which was purified by reverse phase prep-HPLC purification to obtain *trans*-1-(4-chloro-phenyl)-3-[5-methoxy-1'-(3-methoxy-propyl)-1',2',3',4',5',6'-hexahydro-[2,3']bipyridinyl-4'-yl]-urea (**Ex-80,** 20 mg, 15%) as off white solid.
LC-MS (Method 1): m/z [M+H]⁺ = 433.0 (exact mass calc. = 432.19).
¹H-NMR (400 MHz, DMSO-d6): δ 8.38 (s, 1 H), 8.18 (d, *J* = 2 Hz, 1 H), 7.30-7.18 (m, 6 H), 6.02 (d, *J* = 8 Hz, 1 H), 3.81-3.80 (m, 1 H), 3.76 (s, 3 H), 3.34-3.30 (m, 2 H), 3.20 (s, 3 H), 2.90-2.80 (m, 3 H), 2.33 (t,J= 6 Hz, 2 H), 2.11 (t*, J* = 12 Hz, 1 H), 2.05-1.99 (m, 2 H), 1.64 (t, *J* = 7 Hz, 2 H), 1.47 (d, *J* = 12 Hz, 1 H);

### Synthesis of trans-1-(4-chloro-phenyl)-3-[5-methoxy-1'-(4-methoxy-cyclohexanecarbonyl)-1',2',3',4',5',6'-hexahydro-[2,3']bipyridinyl-4'-yl]-urea (Ex-81)

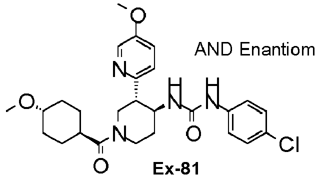

To a stirred solution of 4-methoxy-cyclohexanecarboxylic acid (40 mg, 0.25 mmol, 1.0 eq) in dry THF (10 ml) were added EDCI-HCl (72 mg, 0.375 mmol, 1.5 eq) and HOBt (51 mg, 0.375 mmol, 1.5 eq) in ice-cold condition followed by addition of TEA (104 µl, 0.75 mmol, 3 eq) and *trans*-1-(4-chloro-phenyl)-3-(5-methoxy-1',2',3',4',5',6'-hexahydro-[2,3']bipyridinyl-4'-yl)-urea hydrochloride (**BB-III-HCl,** 119 mg, 0.30 mmol, 1.2 eq) and stirred at RT for 16 h. The reaction mixture was diluted with EtOAc and washed with water and brine. The organic layer was dried over anhyd. Na₂SO₄ and concentrated under reduced pressure to obtain crude product which was purified by reverse phase prep-HPLC purification to afford *trans*-1-(4-chloro-phenyl)-3-[5-methoxy-1'-(4-methoxy-cyclohexanecarbonyl)-1',2',3',4',5',6'-hexahydro-[2,3']bipyridinyl-4'-yl]-urea (**Ex-81,** 18 mg, 14% yield) as off white solid.
LC-MS (Method 1): m/z [M+H]⁺ = 501.5 (exact mass calc. = 500.22).
¹H-NMR (400 MHz, DMSO-d6): δ 8.45 (s, 1 H), 8.22 (d, *J* = 7 Hz, 1 H), 7.31-7.25 (m, 4 H), 7.19 (d, *J* = 8 Hz, 2 H), 6.11 (d, *J* = 7 Hz, 1 H), 4.45-4.41 (m, 1 H), 4.09-4.07 (br m, 1 H), 3.78 (s, 3 H), 3.21 (d, *J* = 8 Hz, 3 H), 3.10-3.04 (m, 1 H), 2.78-2.56 (m, 4 H), 2.08-1.91 (m, 4 H), 1.68-1.58 (m, 2 H), 1.42-1.30 (m, 3 H), 1.20-1.12 (m, 1 H).

### Synthesis of trans-1-(4-chlorophenyl)-3-(3-(5-methoxypyridin-2-yl)-1-(piperidin-4-ylmethyl)piperidin-4-yl)urea ent-1 (Ex-124) and trans-1-(4-chlorophenyl)-3-(3-(5-methoxypyridin-2-yl)-1-(piperidin-4-ylmethyl)piperidin-4-yl)urea ent-2 (Ex-82)

### Synthesis of trans-tert-butyl 4-{[4-{[(4-chlorophenyl)carbamoyl]amino}-3-(5-methoxypyridin-2-yl)piperidin-1-yl]methyl}piperidine-1-carboxylate

To the stirring solution of *trans*-1-(4-chlorophenyl)-3-[3-(5-methoxypyridin-2-yl)-piperidin-4-yl]urea trifluoroacetate (**BB-III-TFA,** 0.5 g, 1.054 mmol, 1.0 eq) in acetonitrile (20 ml), K₂CO₃ (0.727 g, 5.27 mmol, 5.0 eq) was added at 0 °C and allowed to stir for 10 minutes, then compound tert-butyl 4-(bromomethyl)piperidine-1-carboxylate (0.323 g, 1.16 mmol, 1.1 eq) was added and again allowed to stir for 24 hours. After completion of reaction water was added, extracted with ethyl acetate (3x100 ml). Ethyl acetate part was collected, dried over Na₂SO₄ and evaporated to dryness to get crude, which was purified by column chromatography to afford *trans-*tert-butyl 4-{[4-{[(4-chlorophenyl)carbamoyl]amino}-3-(5-methoxypyridin-2-yl)piperidin-1-yl]methyl}piperidine-1-carboxylate (0.423 g, 72%).
LC-MS (Method 2): m/z [M+H]⁺ = 558.3 (exact mass calc. = 557.28).

### Synthesis of trans-1-(4-chlorophenyl)-3-(3-(5-methoxypyridin-2-yl)-1-(piperidin-4-ylmethyl)piperidin-4-yl)urea ent-1 (Ex-124) and trans-1-(4-chlorophenyl)-3-(3-(5-methoxypyridin-2-yl)-1-(piperidin-4-ylmethyl)piperidin-4-yl)urea ent-2 (Ex-82)

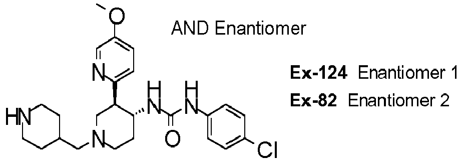

To the stirring solution of tert-butyl *trans*-4-{[4-{[(4-chlorophenyl)carbamoyl]amino}-3-(5-methoxypyridin-2-yl)piperidin-1-yl]methyl}piperidine-1-carboxylate (0.423 g, 0.7579 mmol, 1.0 eq) in DCM (20 ml), TFA (1.5 mL) was added at 0 °C and allowed to stir for 30 minutes at RT. After completion of reaction DCM was evaporated to dryness to get crude, which was purified by column chromatography using 0-20% MeOH in DCM and 230-400 silica gel to afford *trans*-1-(4-chlorophenyl)-3-(3-(5-methoxypyridin-2-yl)-1-(piperidin-4-ylmethyl)piperidin-4-yl)urea (0.23g , 53%).
0.23g of racemic *trans*-1-(4-chlorophenyl)-3-(3-(5-methoxypyridin-2-yl)-1-(piperidin-4-ylmethyl)piperidin-4-yl)urea was separated by preparative chiral HPLC to afford *trans*-1-(4-chlorophenyl)-3-(3-(5-methoxypyridin-2-yl)-1-(piperidin-4-ylmethyl)piperidin-4-yl)urea ent-1 (**Ex-124,** 0.01944g, 5.6%) and *trans*-1-(4-chlorophenyl)-3-(3-(5-methoxypyridin-2-yl)-1-(piperidin-4-ylmethyl)piperidin-4-yl)urea ent-2 (**Ex-82**, 0.01819 g, 5.24%).
**Ex-124:** LC-MS (Method 1): m/z [M+H]⁺ = 458.2 (exact mass calc. = 457.22).
¹H-NMR (400 MHz, DMSO-d6): δ 8.42 (s, 1H), 8.19 (d, *J*= 2.8 Hz, 1H), 7.17-7.31 (m, 6H), 6.04 (d, *J*= 8.1 Hz, 1H), 3.76-3.82 (m, 4H), 2.81-2.90 (m, 5H), 2.41 (t, *J*= 11.8 Hz, 2H), 1.97-2.11 (m, 5H), 1.45-1.61 (m, 4H), 1.20-1.30 (m, 1H), 0.92-0.98 (m, 2H).
**Ex-82:** LC-MS (Method 3): m/z [M+H]⁺ = 458.2 (exact mass calc. = 457.22).
¹H-NMR (400 MHz, DMSO-d6): δ 8.65 (s, 1H), 8.18 (s, 1H), 7.17-7.32 (m, 6H), 6.30 (d, *J*= 7.9 Hz, 1H), 3.76-3.90 (m, 4H), 2.95 (d, *J=* 11.1 Hz, 2H), 2.84-2.90 (m, 3H), 1.97-2.11 (m, 5H), 1.82 (s, 2H), 1.45-1.64 (m, 4H), 1.20-1.30 (m, 1H), 0.97-1.0 (m, 2H).

### Synthesis of trans-1-(4-chlorophenyl)-3-[3-(5-chlorothiophen-2-yl)-1-(piperidin-4-ylmethyl)piperidin-4 yl]urea dihydrochloride ent-1 (Ex-83) and trans-1-(4-chlorophenyl)-3-[3-(5-chlorothiophen-2-yl)-1-(piperidin-4-ylmethyl)piperidin-4-yl]urea dihydrochloride ent-2 (Ex-84)

### Synthesis of trans-tert-butyl 4-((4-(3-(4-chlorophenyl)ureido)-3-(5-chlorothiophen-2-yl)piperidin-1-yl)methyl)piperidine-1-carboxylate

To the stirring solution of *trans*-1-(4-chlorophenyl)-3-[3-(5-chlorothiophen-2-yl)-piperidin-4-yl]urea trifluoroacetate (**BB-VII-TFA,** 0.270 g, 0.5578 mmol, 1.0 eq) in acetonitrile (20 ml), K₂CO₃ (0.3848 g, 2.789 mmol, 5.0 eq) was added at 0 °C and allowed to stir for 10 minutes, then tert-butyl 4-(bromomethyl)piperidine-1-carboxylate (0.01862 g, 0.6694 mmol, 1.2 eq) was added and again allowed to stir for 24 hours. After completion of reaction water was added, extracted with ethyl acetate (3x100 ml). Ethyl acetate part was collected, dried over Na₂SO₄ and evaporated to dryness to get crude, which was purified by column chromatography get crude, which was purified by column chromatography to get racemic *trans-tert-butyl* 4-((4-(3-(4-chlorophenyl)ureido)-3-(5-chlorothiophen-2-yl)piperidin-1-yl)methyl)piperidine-1-carboxylate (0.18 g,56.85%).
LC-MS (Method 3): m/z [M+H]⁺ = 567.3 (exact mass calc. = 566.19).

### Synthesis of trans-tert-butyl 4-{[4-{[(4-chlorophenyl)carbamoyl]amino}-3-(5-chlorothiophen-2-yl)piperidin-1-yl]methyl}piperidine-1-carboxylate ent-1 and trans-tert-butyl 4-{[4-{[(4-chlorophenyl)carbamoyl]amino}-3-(5-chlorothiophen-2-yl)piperidin-1-yl]methyl}piperidine-1-carboxylate ent-2

0.18g of racemic *trans*-tert-butyl 4-{[4-{[(4-chlorophenyl)carbamoyl]amino}-3-(5-chlorothiophen-2-yl)piperidin-1-yl]methyl}piperidine-1-carboxylate was separated by preparative chiral HPLC to afford *trans*-tert-butyl 4-{[4-{[(4-chlorophenyl)carbamoyl]amino}-3-(5-chlorothiophen-2-yl)piperidin-1-yl]methyl}piperidine-1-carboxylate ent-1 (0.060 g, 18.95%) and *trans*-tert-butyl 4-{[4-{[(4-chlorophenyl)carbamoyl]amino}-3-(5-chlorothiophen-2-yl)piperidin-1-yl]methyl}piperidine-1-carboxylate ent-2 (0.063 g, 19.89%).
*trans*-tert-butyl 4-{[4-{[(4-chlorophenyl)carbamoyl]amino}-3-(5-chlorothiophen-2-yl)piperidin-1-yl]methyl}piperidine-1-carboxylate ent-1
LC-MS (Method 3): m/z [M+H]⁺ = 567.3 (exact mass calc. = 566.19).
*trans*-tert-butyl 4-{[4-{[(4-chlorophenyl)carbamoyl]amino}-3-(5-chlorothiophen-2-yl)piperidin-1-yl]methyl}piperidine-1-carboxylate ent-2
LC-MS (Method 2): m/z [M+H]⁺ = 567.3 (exact mass calc. = 566.19).

### Synthesis of trans-1-(4-chlorophenyl)-3-[3-(5-chlorothiophen-2-yl)-1-(piperidin-4-ylmethyl)piperidin-4-yl]urea dihydrochloride ent-1 (Ex-83)

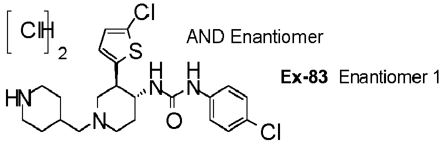

To the stirring solution of *trans*-tert-butyl 4-{[4-{[(4-chlorophenyl)carbamoyl]amino}-3-(5-chlorothiophen-2-yl)piperidin-1-yl]methyl}piperidine-1-carboxylate ent-1 (0.063 g, 0.1057 mmol, 1.0 eq) in dioxane (10 ml), HCl in dioxane (1 ml) was added at 0 °C and allowed to stir for 24 hours. After completion of reaction dioxane was evaporated to dryness to get crude, which was washed by diethyl ether to afford *trans*-1-(4-chlorophenyl)-3-[3-(5-chlorothiophen-2-yl)-1-(piperidin-4-ylmethyl)piperidin-4-yl]urea dihydrochloride ent-1 (**Ex-83,** 0.03094 g, 62.61%).
LC-MS (Method 2): m/z [M+H]⁺ = 466.9 (exact mass calc. = 466.14).
¹H-NMR (400 MHz, DMSO-d6, at 100 °C): δ 10.9 (bs, 1H), 8.43-8.84 (m, 3H), 7.34 (d, *J*= 8.3 Hz, 2H), 7.20 (d, *J=* 8.5 Hz, 2H), 6.92 (d, *J*= 22.1 Hz, 2H), 6.48 (s, 1H), 3.25-4.11 (m, 7H), 2.89-3.0 (m, 3H), 1.82-2.23 (m, 5H), 1.17-1.49 (m, 3H).

### Synthesis of trans-1-(4-chlorophenyl)-3-[3-(5-chlorothiophen-2-yl)-1-(piperidin-4-ylmethyl)piperidin-4-yl]urea dihydrochloride ent-2 (Ex-84)

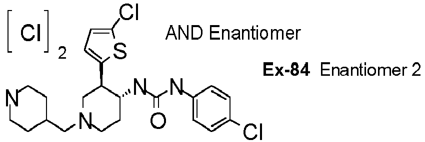

To the stirring solution *trans*-tert-butyl 4-{[4-{[(4-chlorophenyl)carbamoyl]amino}-3-(5-chlorothiophen-2-yl)piperidin-1-yl]methyl}piperidine-1-carboxylate ent-2 (0.060 g, 0.1109 mmol, 1.0 eq) in dioxane (10 ml), HCl in dioxane (1 ml) was added at 0 °C and allowed to stir for 24 hours. After completion of reaction dioxane was evaporated to dryness to get crude, which was washed by diethyl ether to afford *trans*-1-(4-chlorophenyl)-3-[3-(5-chlorothiophen-2-yl)-1-(piperidin-4-ylmethyl)piperidin-4-yl]urea dihydrochloride ent-2 (**Ex-84,** 0.00738 g, 14.23%).
LC-MS (Method 5): m/z [M+H]⁺ = 467.4 (exact mass calc. = 466.14).
¹H-NMR (400 MHz, DMSO-d6): δ 10.61 (s, 1H), 8.76-8.80 (m, 1H), 8.61 (s, 2H), 7.34 (d, *J*= 8.8 Hz, 2H), 7.23 (d, *J*= 8.7 Hz, 2H), 7.02 (d, *J*= 3.6 Hz, 1H), 6.89 (d, *J*= 3.7 Hz, 1H), 6.75 (d, *J*= 8.7 Hz, 1H), 3.90-3.94 (m, 1H), 3.38-3.67 (m, 3H), 2.84-3.27 (m, 8H), 1.95-2.16 (m, 5H), 1.37-1.40 (m, 2H).

### Synthesis of trans-1-(4-chlorophenyl)-3-(3-(5-chlorothiophen-2-yl)-1-((tetrahydro-2H-pyran-4-yl)methyl)piperidin-4-yl)urea ent-1 (Ex-85) and trans-1-(4-chlorophenyl)-3-(3-(5-chlorothiophen-2-yl)-1-((tetrahydro-2H-pyran-4-yl)methyl)piperidin-4-yl)urea ent-2 (Ex-86)

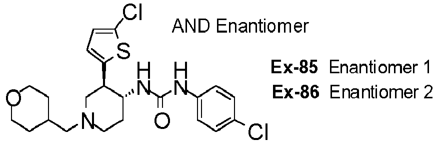

To the stirring solution of *trans*-1-(4-chlorophenyl)-3-[3-(5-chlorothiophen-2-yl)-piperidin-4-yl]urea trifluoroacetate **(BB-VII-TFA,** 0.270 g, 0.5578 mmol, 1.0 eq) in acetonitrile (20 ml), K₂CO₃ (0.384 g, 2.789 mmol, 5.0 eq) was added at 0 °C and allowed to stir for 10 minutes, then 4-(bromomethyl)oxane (0.1198 g, 0.6693 mmol, 1.2 eq) was added and again allowed to stir for 24 hours. After completion of reaction water was added, extracted with ethyl acetate (3x100 ml). Ethyl acetate part was collected, dried over Na₂SO₄ and evaporated to dryness to get crude, which was purified by column chromatography to get racemic *trans*-1-(4-chlorophenyl)-3-(3-(5-chlorothiophen-2-yl)-1-((tetrahydro-2H-pyran-4-yl)methyl)piperidin-4-yl)urea (0.18 g, 68.88%).
LC-MS (Method 3): m/z [M+H]⁺ = 468.2 (exact mass calc. = 467.12).
0.18g of racemic *trans*-1-(4-chlorophenyl)-3-(3-(5-chlorothiophen-2-yl)-1-((tetrahydro-2H-pyran-4-yl)methyl)piperidin-4-yl)urea was separated by preparative chiral HPLC to *trans*-1-(4-chlorophenyl)-3-(3-(5-chlorothiophen-2-yl)-1-((tetrahydro-2H-pyran-4-yl)methyl)piperidin-4-yl)urea ent-1 (**Ex-85,** 0.02926g, 11.19%) and *trans*-1-(4-chlorophenyl)-3-(3-(5-chlorothiophen-2-yl)-1-((tetrahydro-2H-pyran-4-yl)methyl)piperidin-4-yl)urea ent-2 (**Ex-86,** 0.02358g, 9.02%).
**Ex-85:** LC-MS (Method 3): m/z [M+H]⁺ = 468.2 (exact mass calc. = 467.12).
¹H-NMR (400 MHz, DMSO-d6): δ 8.46 (s, 1H), 7.35 (d, *J*= 8.6 Hz, 2H), 7.22 (d, *J*= 8.6 Hz, 2H), 6.92 (d, *J*= 3.4 Hz, 1H), 6.82 (d, *J*= 3.4 Hz, 1H), 6.19 (d, *J*= 7.9 Hz, 1H), 3.81 (d, *J*= 9.6 Hz, 2H), 3.53-3.55 (m, 1H), 3.24-3.27 (m, 2H), 3.04 (t, *J*= 8.5 Hz, 1H), 2.92 (d, *J*= 8.7 Hz, 1H), 2.76-2.80 (m, 1H), 2.12-2.17 (m, 4H), 1.92 (d, *J*= 12.8 Hz, 1H), 1.70-1.80 (m, 1H), 1.60 (d, *J*= 11.5 Hz, 2H), 1.44-1.49 (m, 1H), 1.07-1.14 (m, 2H).
**Ex-86:** LC-MS (Method 3): m/z [M+H]⁺ = 468.2 (exact mass calc. = 467.12).
¹H-NMR (400 MHz, DMSO-d6): δ 8.46 (s, 1H), 7.35 (d, *J*= 8.7 Hz, 2H), 7.22 (d, *J*= 8.7 Hz, 2H), 6.92 (d, *J*= 3.5 Hz, 1H), 6.82 (d, *J*= 3.5 Hz, 1H), 6.20 (d, *J*= 7.9 Hz, 1H), 3.82 (d, *J*= 10.5 Hz, 2H), 3.53-3.55 (m, 1H), 3.24-3.27 (m, 2H), 3.04 (t, *J*= 7.8 Hz, 1H), 2.92 (d, *J*= 9.9 Hz, 1H), 2.75-2.80 (m, 1H), 2.12-2.17 (m, 4H), 1.92 (d, *J*= 11.7 Hz, 1H), 1.70-1.80 (m, 1H), 1.60 (d, *J*= 11.9 Hz, 2H), 1.47-1.49 (m, 1H), 1.09-1.14 (m, 2H).

### Synthesis of trans-1-(4-chlorophenyl)-3-[3-(5-chlorothiophen-2-yl)-1-[(6-methylpyridin-3-yl)methyl]piperidin-4-yllurea (Ex-87)

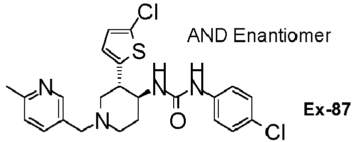

To the stirring solution of compound *trans-*1-(4-chlorophenyl)-3-[3-(5-chlorothiophen-2-yl)-piperidin-4-yl]urea trifluoroacetate (**BB-VII**-TFA, 0.120 g, 0.248 mmol, 1.0 eq) in acetonitrile (20 ml), K₂CO₃ (0.171 g, 1.24 mmol, 5.0 eq) was added at 0 °C and allowed to stir for 10 minutes, then 5-(bromomethyl)-2-methylpyridine (0.092 g, 0.496 mmol, 2 eq) was added and again allowed to stir for 24 hours. After completion of reaction water was added, extracted with ethyl acetate (3x100 ml). Ethyl acetate part was collected, dried over Na₂SO₄ and evaporated to dryness to get crude, which was purified by column chromatography to afford *trans*-1-(4-chlorophenyl)-3-[3-(5-chlorothiophen-2-yl)-1-[(6-methylpyridin-3-yl)methyl]piperidin-4-yl]urea (**Ex-87,** 0.02856 g, 24.22%).
LC-MS (Method 3): m/z [M+H]⁺ = 475.3 (exact mass calc. = 474.1).
¹H-NMR (400 MHz, DMSO-d6): δ 8.46 (s, 1H), 8.36 (s, 1H), 7.59 (d, *J*= 6.3 Hz, 1H), 7.35 (d, *J*= 8.9 Hz, 2H), 7.20-7.23 (m, 3H), 6.91 (d, *J*= 3.7 Hz, 1H), 6.79 (d, *J*= 3.7 Hz, 1H), 6.20 (d, *J*= 8.1 Hz, 1H), 3.56-3.61 (m, 1H), 3.50 (s, 2H), 3.05-3.09 (m, 1H), 2.90 (d, *J*= 9.5 Hz, 1H), 2.72-2.75 (m, 1H), 2.44 (s, 3H), 2.14-2.21 (m, 2H), 1.90-1.93 (m, 1H), 1.45-1.53 (m, 1H).

### Synthesis of trans-1-(4-chloro-phenyl)-3-[5-methoxy-1'-(piperidine-4-carbonyl)-1',2',3',4',5',6'-hexahydro-[2,3']bipyridinyl-4'-yl]-urea (Ex-88)

### Synthesis of trans-4-{4'-[3-(4-chloro-phenyl)-ureido]-5-methoxy-3',4',5',6'-tetrahydro-2'H-[2,3']bipyridinyl-1'-carbonyl}-piperidine-1-carboxylic acid tert-butyl ester

To a stirred solution of piperidine-1,4-dicarboxylic acid mono-tert-butyl ester (50 mg, 0.218 mmol, 1.0 eq) in dry THF (10 ml) were added EDCI-HCl (62 mg, 0.327 mmol, 1.5 eq) and HOBt (44 mg, 0.327 mmol, 1.5 eq) in ice-cold condition followed by addition of TEA (91 µl, 0.654 mmol, 3 eq) and HCl salt of 1-(4-chloro-phenyl)-3-(5-methoxy-1',2',3',4',5',6'-hexahydro-[2,3']bipyridinyl-4'-yl)-urea hydrochloride (**BB-III-HCl,** 86 mg, 0.218 mmol, 1.0 eq) and stirred at RT for 16 h. The reaction mixture was diluted with EtOAc and washed with water and brine. The organic layer was dried over anhyd. Na₂SO₄ and concentrated under reduced pressure to obtain crude *trans-*4-{4'-[3-(4-chloro-phenyl)-ureido]-5-methoxy-3',4',5',6'-tetrahydro-2'H-[2,3']bipyridinyl-1'-carbonyl}-piperidine-1-carboxylic acid tert-butyl ester (120 mg, 96% yield) as brownish solid.
LC-MS (Method 1): m/z [M+H]⁺ = 572.4 (exact mass calc. = 571.26).

### Synthesis of trans-1-(4-chloro-phenyl)-3-[5-methoxy-1'-(piperidine-4-carbonyl)-1',2',3',4',5',6'-hexahydro-[2,3']bipyridinyl-4'-yl]-urea (Ex-88)

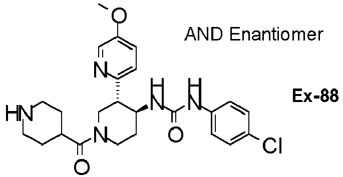

To a stirred solution of *trans*-4-{4'-[3-(4-chloro-phenyl)-ureido]-5-methoxy-3',4',5',6'-tetrahydro-2'H-[2,3']bipyridinyl-1'-carbonyl}-piperidine-1-carboxylic acid tert-butyl ester (250 mg, 0.44 mmol, 1 eq) in 1,4-dioxane (10 ml) was added 4M HCl in dioxane (2 ml) followed by stirring at RT for a period of 3 h. After completion of the reaction (monitored by TLC and LC-MS), the reaction mixture was concentrated under reduced pressure to get the crude material and it was purified by reverse phase prep-HPLC to obtain *trans-*1-(4-chlorophenyl)-3-[5-methoxy-1'-(piperidine-4-carbonyl)-1',2',3',4',5',6'-hexahydro-[2,3']bipyridinyl-4'-yl]-urea (**Ex-88,** 22 mg, 11% yield) as off white solid.
LC-MS (Method 3): m/z [M+H]⁺ = 472.2 (exact mass calc. = 471.20).
¹H-NMR (400 MHz, DMSO-d6): δ 8.40 (s, 1 H), 8.19 (s, 1 H), 7.28-7.22 (m, 4 H), 7.13 (d, *J* = 25 Hz, 2 H), 6.02 (br m, 1 H), 4.44-4.40 (m, 1 H), 4.01-3.93 (m, 2 H), 3.76 (s, 3 H), 3.20-3.12 (m, 1 H), 2.92-2.82 (m, 2 H), 2.72-2.62 (m, 2 H), 2.08-1.93 (m, 1 H), 1.51-1.38 (m, 5 H).

### Synthesis of trans-1-(4-chloro-phenyl)-3-[5-methoxy-1'-(1-methyl-piperidine-4-carbonyl)-1',2',3',4',5',6'-hexahydro-[2,3']bipyridinyl-4'-yl]-urea (Ex-89)

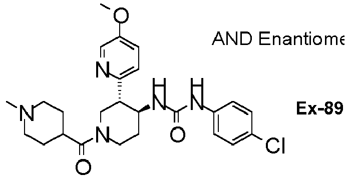

To a stirred solution of I -methyl-piperidine-4-carboxylic acid (40 mg, 0.28 mmol, 1.0 eq) in dry THF (10 ml) were added EDCI-HCl (80 mg, 0.42 mmol, 1.5 eq) and HOBt (56 mg, 0.42 mmol, 1.5 eq) in ice-cold condition followed by addition of TEA (117 µl, 0.84 mmol, 3 eq) and *trans*-1-(4-chloro-phenyl)-3-(5-methoxy-1',2',3',4',5',6'-hexahydro-[2,3']bipyridinyl-4'-yl)-urea hydrochloride (**BB-III-HCl,** 111 mg, 0.28 mmol, 1.0 eq) and stirred at RT for 16 h. The reaction mixture was diluted with EtOAc and washed with water and brine. The organic layer was dried over anhyd. Na₂SO₄ and concentrated under reduced pressure to obtain crude product which was purified by reverse phase prep-HPLC purification to obtain *trans*-1-(4-chloro-phenyl)-3-[5-methoxy-1'-(1-methyl-piperidine-4-carbonyl)-1',2',3',4',5',6'-hexahydro-[2,3']bipyridinyl-4'-yl]-urea (**Ex-89**, 35 mg, 25% yield) as off white solid.
LC-MS (Method 1): m/z [M+H]⁺ = 486.4 (exact mass calc. = 485.22).
1H-NMR (400 MHz, DMSO-d6): δ 8.40 (s, 1 H), 8.22 (s, 1 H), 7.31-7.24 (m, 4 H), 7.19 (d, J = 9 Hz, 2 H), 6.08-6.05 (m, 1 H), 4.46-4.42 (m, 1 H), 4.08-3.92 (m, 2 H), 3.78 (s, 3 H), 3.22-3.15 (m, 1 H), 2.75-2.62 (m, 4 H), 2.13-2.03 (m, 5 H), 1.92-1.74 (m, 3 H), 1.62-1.50 (4, 4 H), 1.42-1.28 (m, 1 H).

### Synthesis of trans-1-(4-chloro-phenyl)-3-[1'-(4-hydroxy-cyclohexanecarbonyl)-5-methoxy-1',2',3',4',5',6'-hexahydro-[2.3']bipyridinyl-4'-yl]-urea (Ex-90)

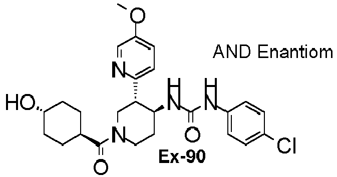

To a stirred solution of 4-hydroxy-cyclohexanecarboxylic acid (40 mg, 0.28 mmol, 1.0 eq) in dry THF (10 ml) were added EDCI-HCl (80 mg, 0.42 mmol, 1.5 eq) and HOBt (56 mg, 0.42 mmol, 1.5 eq) in ice-cold condition followed by addition of TEA (117 µl, 0.84 mmol, 3 eq) and 1-(4-chloro-phenyl)-3-(5-methoxy-1',2',3',4',5',6'-hexahydro-[2,3']bipyridinyl-4'-yl)-urea hydrochloride (**BB-III-HCl,** 111 mg, 0.84 mmol, 1.0 eq) and stirred at RT for 16 h. The reaction mixture was diluted with EtOAc and washed with water and brine. The organic layer was dried over anhyd. Na₂SO₄ and concentrated under reduced pressure to obtain crude product which was purified by reverse phase purification to obtain *trans*-1-(4-chloro-phenyl)-3-[1'-(4-hydroxy-cyclohexanecarbonyl)-5-methoxy-1',2',3',4',5',6'-hexahydro-[2,3']bipyridinyl-4'-yl]-urea (**Ex-90,** 26 mg, 19% yield) as off white solid
LC-MS (Method 1): m/z [M+H]⁺ = 487.4 (exact mass calc. = 486.20).
¹H-NMR (400 MHz, DMSO-d6): δ 8.37 (s, 1 H), 8.21 (t, *J* = 8 Hz, 1 H), 7.35-7.24 (m, 4 H), 7.19 (d, *J* = 9 Hz, 2 H), 6.03 (t, *J* = 8 Hz, 1 H), 4.55-4.42 (m, 2 H), 4.06-3.99 (m, 2 H), 3.92 (d, *J* = 12 Hz, 1 H), 3.78 (s, 3 H), 3.32-3.15 (m, 2 H), 2.80-2.63 (m, 3 H), 2.53 (s, 1 H), 2.08-1.99 (m, 1 H), 1.83-1.75 (m, 2 H), 1.67-1.54 (m, 2 H), 1.40-1.30 (m, 3 H), 1.27-1.09 (m, 3 H).

### Synthesis of trans-1-(4-chloro-phenyl)-3-[3-(5-chloro-thiophen-2-yl)-1-(3-methoxy-propyl)-piperidin-4-yl]-urea (Ex-91)

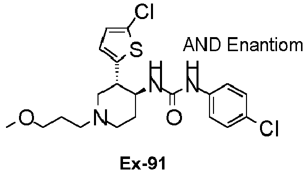

To a stirred solution of *trans*-1-(4-chloro-phenyl)-3-[3-(5-chloro-thiophen-2-yl)-piperidin-4-yl]-urea hydrochloride (**BB-VII-HCl,** 100 mg, 0.246 mmol, 1.0 eq) in dry ACN (7 ml) was added Cs₂CO₃ (320 mg, 0.984 mmol, 4 eq) followed by the addition of 1-bromo-3-methoxy-propane (75 mg, 0.493 mmol, 2 eq) and stirred at RT for a period of 18 h. After completion of the reaction it was evaporated under reduced pressure and diluted with ethyl acetate (50 ml) and washed with water (25 ml) and brine (25 ml). The organic layer was then dried over anhyd. Na₂SO₄ and concentrated under reduced pressure to obtain crude product which was purified by reverse phase prep-HPLC purification to obtain *trans*-1-(4-chloro-phenyl)-3-[3-(5-chloro-thiophen-2-yl)-1-(3-methoxypropyl)-piperidin-4-yl]-urea (**Ex-91**, 26 mg, 24% yield) as off white solid.
LC-MS (Method 2): m/z [M+H]⁺ = 442.2 (exact mass calc. = 441.10).
¹H-NMR (400 MHz, DMSO-d6): δ 8.46 (s, 1 H), 7.35 (d, *J* = 9 Hz, 2 H), 7.22 (d, *J* = 9 Hz, 2 H), 6.92 (d, *J* = 4 Hz, 1 H), 6.80 (d, *J* = 4 Hz, 1 H), 6.22 (d, *J* = 8 Hz, 1 H), 3.57-3.53 (m, 1 H), 3.54-3.34 (m, 2 H), 3.21 (s, 3 H), 3.07-3.03 (m, 1 H), 2.92 (d*, J* = 10 Hz, 1 H), 2.78-2.74 (m, 1 H), 2.53 (t, *J* = 13 Hz, 2 H), 2.13-2.09 (m, 2 H), 1.92 (d, *J* = 9 Hz, 1 H), 1.65 (t, *J* = 7 Hz, 2 H), 1.50-1.42 (m, 1 H).

### Synthesis of trans-1-(4-chloro-phenyl)-3-[5-methoxy-1'-(oxetane-3-carbonyl)-1',2',3',4',5',6'-hexahydro-[2,3']bipyridinyl-4'-yl]-urea (Ex-92)

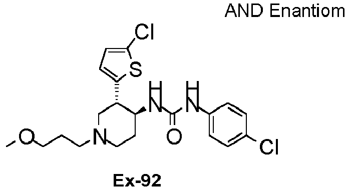

To a stirred solution of oxetane-3-carboxylic acid (38 mg, 0.38 mmol, 1 eq) in dry THF (10 ml) were added N-methyl morpholine (48 µl, 0.45 mmol, 1.2 eq) followed by the addition of IBCF (60 µl, 0.45 mmol, 1.2 eq) at RT then stirred for 15 min then *trans*-1-(4-chloro-phenyl)-3-(5-methoxy-1',2',3',4',5',6'-hexahydro-[2,3']bipyridinyl-4'-yl)-urea hydrochloride (**BB-III-HCl,** 150 mg, 0.38 mmol, 1 eq) was added and continued for 16 h at RT. The reaction mixture was diluted with 10% MeOH-DCM and washed with water and brine. The organic layer was dried over anhyd. Na₂SO₄ and concentrated under reduced pressure to obtain crude product which was purified by reverse phase purification to obtain *trans*-1-(4-chloro-phenyl)-3-[5-methoxy-1'-(oxetane-3-carbonyl)-1',2',3',4',5',6'-hexahydro-[2,3']bipyridinyl-4'-yl]-urea (**Ex-92**, 22 mg, 13% yield) as off white solid
LC-MS (Method 3): m/z [M+H]⁺ = 445.3 (exact mass calc. = 444.16).
¹H-NMR (400 MHz, DMSO-d6): δ 8.38 (s, 1 H), 8.22 (s, 1 H), 7.34-7.25 (m, 4 H), 7.19 (d, *J* = 9 Hz, 2 H), 5.99 (d, *J* = 8 Hz, 1 H), 4.74-4.62 (m, 3 H), 4.46-4.42 (m, 1 H), 4.14 (d, *J* = 5 Hz, 1 H), 4.04 (d, *J* = 11 Hz, 1 H), 3.78 (s, 3 H), 3.47 (d, *J* = 6 Hz, 1 H), 3.26-3.21 (m, 1 H), 3.13 (m, 1 H), 2.84-2.61 (m, 2 H), 2.06-1.91 (m, 1 H), 1.36 (d, *J* = 12 Hz, 1 H).

### Synthesis of trans-1-(4-chloro-phenyl)-3-[1'-(2-methanesulfonyl-ethyl)-5-methoxy-1',2',3',4',5',6'-hexahydro-[2,3']bipyridinyl-4'-yl]-urea (Ex-93)

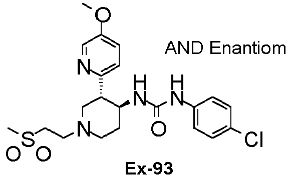

To a stirred solution of 1-(4-chloro-phenyl)-3-(5-methoxy-1',2',3',4',5',6'-hexahydro-[2,3']bipyridinyl-4'-yl)-urea hydrochloride (**BB-III-HCl,** 100 mg, 0.25 mmol, 1 eq) in ACN (5 ml) were added Cs₂CO₃ (246 mg, 0.75 mmol, 3 eq) followed by the addition of 1-bromo-2-methanesulfonyl-ethane (56 mg, 0.30 mmol, 1.2 eq) and stirred at RT for 18 h. The reaction mixture was diluted with 10% MeOH-DCM and washed with water and brine. The organic layer was dried over anhyd. Na₂SO₄ and concentrated under reduced pressure to obtain crude product which was purified by reverse phase purification to obtain *trans*-1-(4-chloro-phenyl)-3-[1'-(2-methanesulfonyl-ethyl)-5-methoxy-1',2',3',4',5',6'-hexahydro-[2,3']bipyridinyl-4'-yl]-urea (**Ex-93**, 36 mg, 30% yield) as off white solid.
LC-MS (Method 3): m/z [M+H]⁺ = 467.5 (exact mass calc. = 466.14).
¹H-NMR (400 MHz, DMSO-d6): δ 8.32 (s, 1 H), 8.19 (d, *J* = 3 Hz, 1 H), 7.32-7.23 (m, 4 H), 7.19 (d, *J* = 9 Hz, 2 H), 5.98 (d, *J* = 8 Hz, 1 H), 3.86-3.82 (m, 1 H), 3.77 (s, 3 H), 3.29-3.26 (m, 2 H), 3.02 (s, 3 H), 2.93-2.83 (m, 3 H), 2.74 (t, *J* = 7 Hz, 2 H), 2.25 (t, *J* = 11 Hz, 1 H), 2.15 (t, *J* = 11 Hz, 1 H), 2.01 (d, *J* = 9 Hz, 1 H), 1.5-1.46 (m, 1 H).

### Synthesis of trans-1-(4-chloro-phenyl)-3-[1'-(2-dimethylamino-acetyl)-5-methoxy-1',2',3',4',5',6'-hexahydro-[2,3']bipyridinyl-4'-yl]-urea (Ex-94)

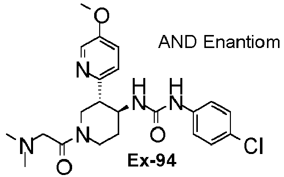

To a stirred solution of dimethylamino-acetic acid (50 mg, 0.485 mmol, 1.0 eq) in dry THF (7 ml) were added EDCI-HCl (139 mg, 0.728 mmol, 1.5 eq) and HOBt (99 mg, 0.728 mmol, 1.5 eq) in ice-cold condition followed by addition of TEA (200 µl, 1.45 mmol, 3 eq) and *trans*-1-(4-chloro-phenyl)-3-(5-methoxy-1',2',3',4',5',6'-hexahydro-[2,3']bipyridinyl-4'-yl)-urea hydrochloride (**BB-III-HCl,** 192 mg, 0.485 mmol, 1.0 eq) and stirred at RT for 18 h. The reaction mixture was diluted with EtOAc and washed with water and brine. The organic layer was dried over anhyd. Na₂SO₄ and concentrated under reduced pressure and obtained crude was purified by reverse phase prep-HPLC purification to obtain *trans*-1-(4-chloro-phenyl)-3-[1'-(2-dimethylamino-acetyl)-5-methoxy-1',2',3',4',5',6'-hexahydro-[2,3']bipyridinyl-4'-yl]-urea (**Ex-94,** 30 mg, 13% yield) as off white solid.
LC-MS (Method 5): m/z [M+H]⁺ = 446.4 (exact mass calc. = 445.19).
¹H-NMR (400 MHz, DMSO-d6): δ 8.40 (d, *J* = 9 Hz, 1 H), 8.22 (s, 1 H), 7.34-7.18 (m, 6 H), 6.12 (d, *J* = 6 Hz, 1 H), 4.41 (t, *J* = 12 Hz, 1 H), 4.05 (d*, J* = 12 Hz, 2 H), 3.78 (s, 3 H), 3.22-2.63 (m, 5 H), 2.19 (s, 3 H), 2.16 (s, 3 H), 2.03 (d, *J* = 11 Hz, 1 H), 1.23-1.45 (m, 1 H).

### Synthesis of trans-1-(4-chloro-phenyl)-3-[1'-(3-dimethylamino-propiopyl)-5-methoxy-1',2',3',4',5',6'-hexahydro-[2,3']bipyridinyl-4'-yl]-urea (Ex-95)

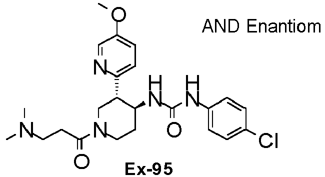

To a stirred solution of 3-dimethylamino-propionic acid (24 mg, 0.20 mmol, 1 eq) in dry THF (10 ml) were added EDCI-HCl (58 mg, 0.30 mmol, 1.5 eq) and HOBt (41 mg, 0.30 mmol, 1.5 eq) and TEA (84 µl, 0.60 mmol, 3 eq) followed by the addition of f *trans*-1-(4-Chloro-phenyl)-3-(5-methoxy-1',2',3',4',5',6'-hexahydro-[2,3']bipyridinyl-4'-yl)-urea hydrochloride (**BB-III-HCl,** 80 mg, 0.20 mmol, 1 eq) and stirred at RT for 16 h. The reaction mixture was diluted with 10% MeOH-DCM and washed with water and brine. The organic layer was dried over anhyd. Na₂SO₄ and concentrated under reduced pressure to obtain crude product which was purified by reverse phase purification to obtain trans-1-(4-chloro-phenyl)-3-[1'-(3-dimethylamino-propionyl)-5-methoxy-1',2',3',4',5',6'-hexahydro-[2,3']bipyridinyl-4'-yl]-urea (**Ex-95**, 22 mg, 24% yield) as off white solid.
LC-MS (Method 3): m/z [M+H]⁺ = 460.3 (exact mass calc. = 459.20).
¹H-NMR (400 MHz, DMSO-d6): δ 8.38 (s, 1 H), 8.20-8.19 (m, 1 H), 7.30-7.22 (m, 4 H), 7.17 (d, *J* = 9 Hz, 2 H), 6.05 (t, *J* = 6 Hz, 1H), 4.40 (d, *J* = 7 Hz, 1 H), 4.02 (d, *J* = 11 Hz, 1 H), 3.95-3.83 (m, 1 H), 3.75 (s, 3 H), 3.22-3.11 (m, 1 H), 2.93-2.65 (m, 2 H), 2.11-1.85 (m, 7 H), 1.401.20 (m, 1 H).

### Synthesis of trans-1-(4-chloro-phenyl)-3-[5-methoxy-1'-(2-methylamino-acetyl)-1',2',3',4',5',6'-hexahydro-[2,3']bipyridinyl-4'-yl]-urea (Ex-96)

### Synthesis of trans-(2-{4'-[3-(4-chloro-phenyl)-ureido]-5-methoxy-3',4',5',6'-tetrahydro-2'H-[2,3']bipyridinyl-1'-yl}-2-oxo-ethyl)-methyl-carbamic acid tert-butyl ester

To a stirred solution of N-(tert-butoxycarbonyl)-N-methylglycine (47 mg, 0.25 mmol, 1 eq) in dry THF (10 ml) were added EDCI-HCl (72 mg, 0.37 mmol, 1.5 eq) and HOBt (51 mg, 0.37 mmol, 1.5 eq) in RT condition followed by addition of TEA (105 µl, 0.75 mmol, 3 eq) and *trans*-1-(4-chloro-phenyl)-3-(5-methoxy-1',2',3',4',5',6'-hexahydro-[2,3']bipyridinyl-4'-yl)-urea hydrochloride (**BB-III-HCl,** 100 mg, 0.25 mmol, 1 eq) and stirred at RT for 16 h. The reaction mixture was diluted with 10% MeOH-DCM and washed with water and brine. The organic layer was dried over anhyd. Na₂SO₄ and concentrated under reduced pressure to obtain crude *trans-*(2-{4'-[3-(4-chloro-phenyl)-ureido]-5-methoxy-3',4',5',6'-tetrahydro-2'H-[2,3']bipyridinyl-1'-yl}-2-oxo-ethyl)-methyl-carbamic acid tert-butyl ester (200mg crude weight) as yellow sticky solid.
LC-MS (Method 2): m/z [M+H]⁺ = 532.2 (exact mass calc. = 531.22).

### Synthesis of trans-1-(4-chloro-phenyl)-3-[5-methoxy-1'-(2-methylamino-acetyl)-1',2',3',4',5',6'-hexahydro-[2,3']bipyridinyl-4'-yl]-urea (Ex-96)

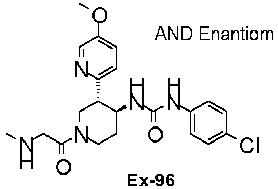

To a stirred solution of *trans*-(2-{4'-[3-(4-chloro-phenyl)-ureido]-5-methoxy-3',4',5',6'-tetrahydro-2'H-[2,3']bipyridinyl-1'-yl}-2-oxo-ethyl)-methyl-carbamic acid tert-butyl ester (200 mg, 0.376 mmol, 1 eq) in dioxane (3 ml) was added 4M HCL in dioxane (2 ml) and stirred at RT for 3 h. The reaction mixture was concentrated under reduced pressure to obtain crude product which was purified by reverse phase purification using NaHCO₃ as buffer to obtain *trans*-1-(4-chloro-phenyl)-3-[5-methoxy-1'-(2-methylamino-acetyl)-1',2',3',4',5',6'-hexahydro-[2,3']bipyridinyl-4'-yl]-urea (**Ex-96**, 34 mg, 21% yield) as off white solid.
LC-MS (Method 1): m/z [M+H]⁺ = 432.2 (exact mass calc. = 431.17).
¹H-NMR (400 MHz, DMSO-d6): δ 8.42 (s, 1 H), 8.22 (s, 1 H), 7.48-7.25 (m, 4 H), 7.20-7.18 (m, 2 H), 6.10 (d, *J* = 8 Hz, 1 H), 4.43 (d, *J* = 7 Hz, 1 H), 4.07-4.04 (m, 1 H), 3.96-3.78 (m, 4 H), 3.39-3.10 (m, 3 H), 2.97-2.67 (m, 3 H), 2.32-2.26 (m, 3 H), 2.04-2.01 (m, 1 H), 1.43-1.29 (m, 1 H).

### Synthesis of trans-1-(4-chloro-phenyl)-3-[5-methoxy-1'-(3-methylamino-propionyl)-1',2',3',4',5',6' hexahydro-[2,3']bipyridinyl-4'-yl]-urea (Ex-97)

### Synthesis of trans-(3-{4'-[3-(4-chloro-phenyl)-ureido]-5-methoxy-3',4',5',6'-tetrahydro-2'H-[2,3']bipyridinyl-1'-yl}-3-oxo-propyl)-methyl-carbamic acid tert-butyl ester

To a stirred solution of 3-(tert-butoxycarbonyl-methyl-amino)-propionic acid (77 mg, 0.38 mmol, 1 eq) in dry THF (10 ml) were added N-methyl morpholine (48 µl, 0.45 mmol, 1.2 eq) and IBCF (60 µl, 0.45 mmol, 1.2 eq) at RT and stirred for 15 min followed by the addition of *trans*-1-(4-chloro-phenyl)-3-(5-methoxy-1',2',3',4',5',6'-hexahydro-[2,3']bipyridinyl-4'-yl)-urea hydrochloride (**BB-III-HCl,** 150 mg, 0.38 mmol, 1 eq) was added and continued stirring at Reflux for 16 h. The reaction mixture was diluted with 10% MeOH-DCM and washed with water and brine. The organic layer was dried over anhyd. Na₂SO₄ and concentrated under reduced pressure to obtain crude product which was purified by reverse phase purification to obtain *trans-*(3-{4'-[3-(4-chloro-phenyl)-ureido]-5-methoxy-3',4',5',6'-tetrahydro-2'H-[2,3']bipyridinyl-1'-yl}-3-oxo-propyl)-methyl-carbamic acid tert-butyl ester (200 mg, 96% crude yield) as yellow sticky solid.
LC-MS (Method 1): m/z [M+H]⁺ = 546.2 (exact mass calc. = 545.24).

### Synthesis of trans-1-(4-chloro-phenyl)-3-[5-methoxy-1'-(3-methylamino-propionyl)-1',2',3',4',5',6'-hexahydro-[2,3']bipyridinyl-4'-yl]-urea (Ex-97)

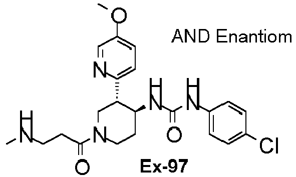

To a stirred solution of *trans*-(3-{4'-[3-(4-chloro-phenyl)-ureido]-5-methoxy-3',4',5',6'-tetrahydro-2'H-[2,3']bipyridinyl-1'-yl}-3-oxo-propyl)-methyl-carbamic acid tert-butyl ester (200 mg, 0.36 mmol, 1 eq) in 1,4-dioxane (2 ml) was added 4M HCl in dioxane was added and stirred at RT for 3 h. The reaction mixture was concentrated under reduced pressure to obtain crude product which was purified by reverse phase prep-HPLC purification to obtain *trans*-1-(4-chloro-phenyl)-3-[5-methoxy-1',2',3',4',5',6'-hexahydro-[2,3']bipyridinyl-4'-yl]-urea (**Ex-97**, 80 mg, 50% yield) as off white solid.
LC-MS (Method 3): m/z [M+H]⁺ = 446.5 (exact mass calc. = 445.19).
¹H-NMR (400 MHz, DMSO-d6): δ 8.52 (s, 1 H), 8.23-8.21 (m, 1 H), 7.33-7.18 (m, 6 H), 6.21-6.18 (br m, 1 H), 4.45-4.42 (m, 1 H), 4.08-4.02 (m, 1 H), 3.96-3.82 (m, 1 H), 3.78 (s, 3 H), 3.21-3.13 (m, 1 H), 2.81-2.71 (m, 1 H), 2.70-2.62 (m, 3 H), 2.54-2.42 (m, 2 H), 2.26 (d, *J* = 8 Hz, 3 H), 2.09-1.98 (m, 1 H), 1.42-1.31 (m, 1 H).

### Synthesis of trans-1-(4-chlorophenyl)-3-[3-(5-methoxypyridin-2-yl)-1-{[4-(trifluoromethyl)pyridin-3-yl]carbonyl}piperidin-4-yl]urea ent-1 (Ex-98) and trans-1-(4-chlorophenyl)-3-[3-(5-methoxypyridin-2-yl)-1-{[4-(trifluoromethyl)pyridin-3-yl]carbonyl}piperidin-4-yl]urea ent-2 (EX-99)

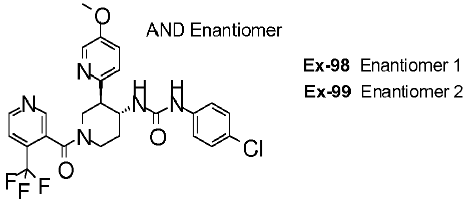

To a stirred solution 4-(trifluoromethyl)pyridine-3-carboxylic acid (0.242 g, 1.2658 mmol, 1.5 eq) in DMF (10 ml) was added EDCI-HCl (0.243 g, 1.2657 mmol, 1.5 eq), HOBt (0.137 g, 1.0125 mmol, 1.2 eq), DIPEA (0.7 ml, 4.219 mmol, 5.0 eq) and stirred at RT for 30 min followed by addition of a *trans*-1-(4-chlorophenyl)-3-(3-(5-methoxypyridin-2-yl)piperidin-4-yl)urea trifluoroacetate (**BB-III-TFA,** 0.40 g, 0.8438 mmol, 1.0 eq). The reaction mixture was stirred for 16 hr at RT. After completion of the reaction (monitored by TLC, TLC system 5% MeOH-DCM, Rf-0.4) reaction mixture was poured into cold water and extracted with EtOAc (2 x 25 ml). The organic layer was washed with water (2 x 25 ml) and brine (2 x 25 mL), dried over anhyd. Na₂SO₄, concentrated in vacuo. The crude material thus obtained was purified by flash chromatography (230-400 mesh silica gel; 5% MeOH-DCM as an elutent) to get *trans*-1-(4-chlorophenyl)-3-[3-(5-methoxypyridin-2-yl)-1-1{[4-(trifluoromethyl)pyridin-3-yl]carbonyl}piperidin-4-yl]urea (0.205g,45%).
LC-MS (Method 3): m/z [M+H]⁺ = 534.4 (exact mass calc. = 533.14).
0.205g of racemic *trans*-1-(4-chlorophenyl)-3-[3-(5-methoxypyridin-2-yl)-1-{[4-(trifluoromethyl)pyridin-3-yl]carbonyl}piperidin-4-yl]urea was separated by preparative chiral HPLC to afford both the enantiomers as *trans-*1-(4-chlorophenyl)-3-[3-(5-methoxypyridin-2-yl)-1-{[4-(trifluoromethyl)pyridin-3-yl]carbonyl}piperidin-4-yl]urea ent-1 (**Ex-98,** 0.06801g, 18.98%) and *trans*-1-(4-chlorophenyl)-3-[3-(5-methoxypyridin-2-yl)-1-{[4-(trifluoromethyl)pyridin-3-yl]carbonyl}piperidin-4-yl]urea ent-2 (**EX-99,** 0.06991 g, 12.66%).
**Ex-98:** LC-MS (Method 3): m/z [M+H]⁺ = 534.4 (exact mass calc. = 533.14).
¹H-NMR (400 MHz, DMSO-d6 at 100 °C): δ 8.77-8.88 (m, 2H), 8.10-8.25 (m, 2H), 7.78 (s, 1H), 7.16-7.32 (m, 6H), 5.95 (s, 1H), 4.59 (bs, 1H), 4.11 (s, 1H), 3.76-3.81 (m, 3H), 3.01-3.40 (m, 3H), 2.02-2.21 (m, 1H), 1.49-1.56 (m, 1H).
**Ex-99:** LC-MS (Method 3): m/z [M+H]⁺ = 534.4 (exact mass calc. = 533.17)
¹H-NMR (400 MHz, DMSO-d6 at 100 °C): δ 8.77-8.89 (m, 2H), 8.10-8.25 (m, 2H), 7.78 (s, 1H), 7.16-7.32 (m, 6H), 5.95 (s, 1H), 4.59 (bs, 1H), 4.11 (s, 1H), 3.76-3.81 (m, 3H), 3.06-3.31 (m, 3H), 2.03-2.18 (m, 1H), 1.51-1.60 (m, 1H).

### Synthesis of trans-1-(4-chlorophenyl)-3 -[3-(5-methoxypyridin-2-yl)-1-{[6-(trifluoromethyl)pyridin-3-yl]carbonyl}piperidin-4-yl]urea ent-1 (Ex-100) and trans-1-(4-chlorophenyl)-3-[(5-methoxypyridin-2-yl)-1-{[6-(trifluoromethyl)pyridin-3-yl]carbonyl}piperidin-4-yl]urea ent-2 (Ex-101)

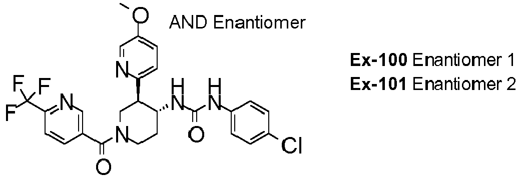

To a stirred solution 6-(trifluoromethyl)pyridine-3-carboxylic acid (0.242 g, 1.2658 mmol, 1.5 eq) in DMF (10 ml) was added EDCI-HCl (0.243 g, 1.2657 mmol, 1.5 eq), HOBt (0.137 g, 1.0125 mmol, 1.2 eq), DIPEA (0.7 ml, 4.219 mmol, 5.0 eq) and stirred at RT for 30 min followed by addition of a of *trans*-1-(4-chlorophenyl)-3-(3-(5-methoxypyridin-2-yl)piperidin-4-yl)urea trifluoroacetate (**BB-III-TFA,** 0.40 g, 0.8438 mmol, 1.0 eq). The reaction mixture was stirred for 16 hr at RT. After completion of the reaction (monitored by TLC, TLC system 5% MeOH-DCM, R_{f}: 0.4) reaction mixture was poured into cold water and extracted with EtOAc (2 x 25 ml). The organic layer was washed with water (2 x 25 ml) and brine (2 x 25 mL), dried over anhyd. Na₂SO₄, concentrated in vacuo. The crude material thus obtained was purified by flash chromatography (230-400 mesh silica gel; 5% MeOH-DCM as an elutent) to get *trans*-1-(4-chlorophenyl)-3-[3-(5-methoxypyridin-2-yl)-1-{[6-(trifluoromethyl)pyridin-3-yl]carbonyl}piperidin-4-yl]urea (0.23g, 51%).
LC-MS (Method 3): m/z [M+H]⁺ = 534.4 (exact mass calc. = 533.14).
0.23 g of racemic *trans*-1-(4-chlorophenyl)-3-[3-(5-methoxypyridin-2-yl)-1-{[6-(trifluoromethyl)pyridin-3-yl]carbonyl}piperidin-4-yl]urea was separated by preparative chiral HPLC to afford *trans*-1-(4-chlorophenyl)-3-[3-(5-methoxypyridin-2-yl)-1-{[6-(trifluoromethyl)pyridin-3-yl]carbonyl}piperidin-4-yl]urea ent-1 (**Ex-100,** 0.09121g, 20.24%) and *trans*-1-(4-chlorophenyl)-3-[(5-methoxypyridin-2-yl)-1-{[6-(trifluoromethyl)pyridin-3-yl]carbonyl}piperidin-4-yl]urea ent-2 (**Ex-101,** 0.09632 g, 21.37%).
**Ex-100:** LC-MS (Method 3): m/z [M+H]⁺ = 534.4 (exact mass calc. = 533.14).
¹H-NMR (400 MHz, DMSO-d6 at 100 °C): δ 8.81 (s, 1H), 8.11-8.21 (m, 3H), 7.93 (d, *J*= 7.7 Hz, 1H), 7.17-7.30 (m, 6H), 5.91 (d, *J*= 7.7 Hz, 1H), 4.0-4.12 (m, 2H), 3.80 (s, 3H), 3.24-3.32 (m, 2H), 2.97-2.99 (m, 1H), 2.10-2.13 (m, 1H), 1.59-1.62 (m, 1H).
**Ex-101:** LC-MS (Method 3): m/z [M+H]⁺ = 534.4 (exact mass calc. = 533.14).
¹H-NMR (400 MHz, DMSO-d6 at 100 °C): δ 8.80 (s, 1H), 8.11-8.21 (m, 3H), 7.93 (d, *J*= 7.8 Hz, 1H), 7.17-7.36 (m, 6H), 5.90 (d, *J*= 7.5 Hz, 1H), 4.12-4.35 (m, 2H), 3.80 (s, 3H), 3.24-3.33 (m, 2H), 2.98-3.10 (m, 1H), 2.10-2.23 (m, 1H), 1.59-1.62 (m, 1H).

### Synthesis of trans-1-(4-chloro-phenyl)-3-[1'-(3-hydroxy-propionyl)-5-methoxy-1',2',3',4',5',6'-hexahydro-[2,3']bipyridinyl-4'-yl]-urea (Ex-102)

### Synthesis of trans-1-{1'-[3-(tert-butyl-dimethyl-silanyloxy-propionyl]-5-methoxy-1',2',3',4',5',6'-hexahydro-[2,3']bipyridinyl-4'-yl}-3-(4-chloro-phenyl)-urea

To a stirred solution of 3-(tert-butyl-dimethyl-silanyloxy)-propionic acid (50 mg, 0.24 mmol, 1.0 eq) in dry THF (10 ml) were added EDCI-HCl (68 mg, 0.36 mmol, 1.5 eq) and HOBt (49 mg, 0.36 mmol, 1.5 eq) in ice-cold condition followed by addition of TEA (100 µl, 0.72 mmol, 3 eq) and *trans*-1-(4-chloro-phenyl)-3-(5-methoxy-1',2',3',4',5',6'-hexahydro-[2,3']bipyridinyl-4'-yl)-urea hydrochloride (**BB-III-HCl,** 95 mg, 0.24 mmol, 1.0 eq) and stirred at RT for 18 h. The reaction mixture was diluted with EtOAc and washed with water and brine. The organic layer was dried over anhyd. Na₂SO₄ and concentrated under reduced pressure to obtain *trans*-1-{1'-[3-(tert-butyldimethyl-silanyloxy)-propionyl]-5-methoxy-1',2',3',4',5',6'-hexahydro-[2,3']bipyridinyl-4'-yl}-3-(4-chlorophenyl)-urea (120 mg, 98% crude yield) as brownish sticky solid.
LC-MS (Method 2): m/z [M+H]⁺ = 547.1 (exact mass calc. = 546.24).

### Synthesis of trans-1-(4-chloro-phenyl)-3-[1'-(3-hydroxy-propionyl)-5-methoxy-1',2',3',4',5',6'-hexahydro-[2,3']bipyridinyl-4'-yl]-urea (Ex-102)

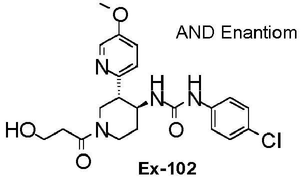

To a stirred solution of *trans*-1-{1'-[3-(tert-butyl-dimethyl-silanyloxy)-propionyl]-5-methoxy-1',2',3',4',5',6'-hexahydro-[2,3']bipyridinyl-4'-yl}-3-(4-chloro-phenyl)-urea (200 mg, 0.366 mmol, 1.0 eq) in dry THF (8 ml) were added TBAF (1 M in THF, 1.5 ml) dropwise in ice-cold condition and stirred at RT for 4 h. The reaction mixture was quenched with MeOH (1 ml). The reaction mixture was diluted with EtOAc and washed with water and brine. The organic layer was dried over anhyd. Na₂SO₄ and concentrated under reduced pressure to obtain crude product which was purified by reverse phase purification to obtain *trans*-1-(4-chloro-phenyl)-3-[1'-(3-hydroxy-propionyl)-5-methoxy-1',2',3',4',5',6'-hexahydro-[2,3']bipyridinyl-4'-yl]-urea (**Ex-102,** 16 mg, 10% crude yield) as off white solid.
LC-MS (Method 1): m/z [M+H]⁺ = 433.2 (exact mass calc. = 432.16).
¹H-NMR (400 MHz, DMSO-d6, 100 °C): δ 8.22 (d, *J=* 2 Hz, 1 H), 8.13 (br s, 1 H), 7.30-7.24 (m, 4 H), 7.17 (d, *J* = 9 Hz, 2 H), 5.90-5.88 (br s, 1 H), 4.39-4.29 (br m, 2 H), 4.22-4.08 (br m, 2 H), 3.81 (s, 3 H), 3.71-3.68 (m, 2 H), 3.53-3.49 (m, 1 H), 3.04-2.91 (m, 1 H), 2.84-2.78 (m, 1 H), 2.54-2.50 (m, 2 H), 2.10-2.07 (m, 1 H), 1.48-1.46 (m, 1 H).

### Synthesis of trans-1-(4-chloro-phenyl)-3-[1'-(3-hydroxy-propyl)-5-methoxy-1',2',3',4',5',6'-hexahydro-[2,3']bipyridinyl-4'-yl]-urea

### Synthesis of trans-1-{1'-[3-(tert-butyl-dimethyl-silanyloxy)-propyl]-5-methoxy-1',2',3',4',5',6'-hexahydro-[2,3']bipyridinyl-4'-yl}-3-(4-chloro-phenyl)-urea (

To a stirred solution of *trans*-1-(4-chloro-phenyl)-3-(5-methoxy-1',2',3',4',5',6'-hexahydro-[2,3']bipyridinyl-4'-yl)-urea hydrochloride (**BB-III-HCl,** 200 mg, 0.504 mmol, 1.0 eq) in dry ACN (10 ml) was added Cs₂CO₃ (657 mg, 2.02 mmol, 4 eq) followed by the addition of (3-bromo-propoxy)-tert-butyl-dimethyl-silane (255 mg, 1.01 mmol, 2 eq) and stirred at RT for a period of 18 h. After completion of the reaction it was evaporated under reduced pressure and diluted with ethyl acetate (50 ml) and washed with water (25 ml) and brine (25 ml). The organic layer was then dried over anhyd. Na₂SO₄ and concentrated under reduced pressure to obtain crude product *trans-*1-{1'-[3-(tert-butyl-dimethyl-silanyloxy)-propyl]-5-methoxy-1',2',3',4',5',6'-hexahydro-[2,3']bipyridinyl-4'-yl}-3-(4-chloro-phenyl)-urea (**int.14**) (230 mg, 85% crude yield) as brownish gummy.
LC-MS (Method 2): m/z [M+H]⁺ = 533.3 (exact mass calc. = 532.26).

### Synthesis of trans-1-(4-chloro-phenyl)-3-[1'-(3-hydroxy-propyl)-5-methoxy-1',2',3',4',5',6'-hexahydro-[2,3']bipyridinyl-4'-yl]-urea (Ex-103)

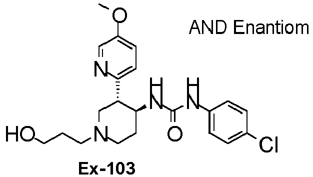

To a stirred solution of *trans*-1-{1'-[3-(tert-butyl-dimethyl-silanyloxy)-propyl]-5-methoxy-1',2',3',4',5',6'-hexahydro-[2,3']bipyridinyl-4'-yl}-3-(4-chloro-phenyl)-urea (240 mg, 0.451 mmol, 1.0 eq) in dry THF (7 ml) were added TBAF (1 M in THF, 0.5 ml) drop wise in ice-cold condition and stirred at RT for 4 h. The reaction mixture was quenched with MeOH (1 ml). The reaction mixture was diluted with EtOAc and washed with water and brine. The organic layer was dried over anhyd. Na₂SO₄ and concentrated under reduced pressure to obtain crude product which was purified by reverse phase prep-HPLC purification to *trans*-1-(4-chloro-phenyl)-3-[1'-(3-hydroxypropyl)-5-methoxy-1',2',3',4',5',6'-hexahydro-[2,3']bipyridinyl-4'-yl]-urea (**Ex-103,** 30 mg, 16% yield) as off white solid.
LC-MS (Method 1): m/z [M+H]⁺ = 419.4 (exact mass calc. = 418.18).
¹H-NMR (400 MHz, DMSO-d6): δ 8.45 (s, 1 H), 8.19 (d, *J* = 3 Hz, 1 H), 7.31-7.18 (m, 6 H), 6.10 (d, *J* = 8 Hz, 1 H), 4.48-4.41 (m, 1 H), 3.86-3.81 (m, 1 H), 3.76 (s, 3 H), 3.42 (t, *J* = 6 Hz, 2 H), 2.91-2.82 (m, 3 H), 2.34 (t, *J* = 7 Hz, 2 H), 2.10 (t*, J* = 11 Hz, 1 H), 2.00 (d, *J* = 11 Hz, 2 H), 1.57 (t, *J* = 6 Hz, 2 H), 1.48-1.45 (m, 1 H).

### Synthesis of trans-1-(4-chloro-phenyl)-3 -[3-(5-chloro-thiophen-2-yl)-1-(1-methyl-piperidine-4-carbonyl)-piperidⁱn-4-yll-urea (Ex-104)

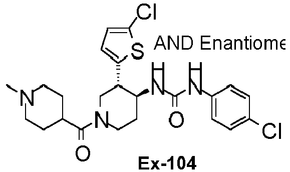

To a stirred solution of 1-methyl-piperidine-4-carboxylic acid (40 mg, 0.279 mmol, 1.0 eq) in dry THF (10 ml) were added EDCI-HCl (80 mg, 0.418 mmol, 1.5 eq) and HOBt (57 mg, 0.418 mmol, 1.5 eq) in ice-cold condition followed by addition of TEA (117 µl, 0.837 mmol, 3 eq) and *trans*-1-(4-chloro-phenyl)-3-[3-(5-chloro-thiophen-2-yl)-piperidin-4-yl]-urea hydrochloride (114 mg, 0.279 mmol, 1.0 eq) and stirred at RT for 18 h. The reaction mixture was diluted with EtOAc and washed with water and brine. The organic layer was dried over anhyd. Na₂SO₄ and concentrated under reduced pressure to obtain crude product which was purified by reverse phase purification to obtain *trans*-1-(4-chloro-phenyl)-3-[3-(5-chloro-thiophen-2-yl)-1-(1-methyl-piperidine-4-carbonyl)-piperidin-4-yl]-urea (**Ex-104,** 45 mg, 32% yield) as off white solid.
LC-MS (Method 3): m/z [M+H]⁺ = 495.3 (exact mass calc. = 494.13).
1H-NMR (400 MHz, DMSO-d6): δ 8.56 (s, 1 H), 7.40-7.34 (m, 2 H), 7.26-7.22 (m, 2 H), 6.97-6.96 (m, 1 H), 7.00-6.98 (m, 1 H), 6.27-6.26 (m, 1 H), 4.48-4.42 (m, 1 H), 4.08-3.82 (m, 3 H), 3.21-3.14 (m, 1 H), 3.08-2.91 (m, 1 H), 2.85-2.72 (m, 2 H), 2.62-2.56 (m, 1 H), 2.13 (s, 3 H), 1.98-1.82 (m, 3 H), 1.69-1.51 (m, 4 H), 1.40-1.31 (m, 1 H).

### Synthesis of trans-1-(4-chloro-phenyl)-3-[3-(5-chloro-thiophen-2-yl)-1-(3-hydroxy-propyl)-piperidin-4-yl]-urea (Ex-105)

### Synthesis of trans-1-[1-[3-(tert-butyl-dimethyl-silanyloxy)-propyl]-3-(5-chloro-thiophen-2-yl)-piperidin-4-yl]-3-(4-chloro-phenyl)-urea

To a stirred solution of *trans*-1-(4-chloro-phenyl)-3-[3-(5-chloro-thiophen-2-yl)-piperidin-4-yl]-urea (**BB-VII-HCl,** 100 mg, 0.246 mmol, 1.0 eq) in dry ACN (10 ml) was added Cs2CO3 (320 mg, 0.984 mmol, 4 eq) followed by the addition of (3-bromo-propoxy)-tert-butyl-dimethyl-silane (124 mg, 0.493 mmol, 2 eq) and stirred at RT for a period of 18 h. After completion of the reaction it was evaporated under reduced pressure and diluted with ethyl acetate (50 ml) and washed with water (25 ml) and brine (25 ml). The organic layer was then dried over anhyd. Na₂SO₄ and concentrated under reduced pressure to obtain crude product *trans*-1-[1-[3-(tert-butyl-dimethyl-silanyloxy)-propyl]-3-(5-chloro-thiophen-2-yl)-piperidin-4-yl]-3-(4-chloro-phenyl)-urea (130 mg, 97% crude yield) as brownish gummy.
LC-MS (Method 2): m/z [M+H]⁺ = 542.2 (exact mass calc. = 541.18).

### Synthesis of trans-1-(4-chloro-phenyl)-3-[3-(5-chloro-thiophen-2-yl)-1-(3-hydroxy-propyl)-piperidin-4-yl]-urea (Ex-105)

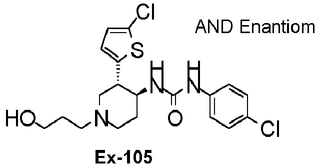

To a stirred solution of *trans-*1-[1-[3-(tert-butyl-dimethyl-silanyloxy)-propyl]-3-(5-chloro-thiophen-2-yl)-piperidin-4-yl]-3-(4-chloro-phenyl)-urea (200 mg, 0.369 mmol, 1.0 eq) in dry THF (5 ml) were added TBAF (1 M in THF, 0.36 ml) drop wise in ice-cold condition and stirred at RT for 4 h. The reaction mixture was then quenched with MeOH (1 ml). The reaction mixture was diluted with EtOAc and washed with water and brine. The organic layer was dried over anhyd. Na₂SO₄ and concentrated under reduced pressure to obtain crude product which was purified by reverse phase prep-HPLC purification to obtain *trans*-1-(4-chloro-phenyl)-3-[3-(5-chlorothiophen-2-yl)-1-(3-hydroxy-propyl)-piperidin-4-yl]-urea (**Ex-105,** 26 mg, 16% yield) as off white solid.
LC-MS (Method 1): m/z [M+H]⁺ = 428.0 (exact mass calc. = 427.09).
¹H-NMR (400 MHz, DMSO-d6): δ 8.46 (s, 1 H), 7.35 (d, *J* = 9 Hz, 2 H), 7.22 (d, *J* = 9 Hz, 2 H), 6.92 (d, *J* = 4 Hz, 1 H), 6.80 (d, *J* = 4 Hz, 1 H), 6.22 (d, *J* = 9 Hz, 1 H), 4.43-4.39 (m, 1 H), 3.58-3.52 (m, 1 H), 3.43 (t, *J* = 6 Hz, 2 H), 3.07-3.01 (m, 1 H), 2.93 (d, *J* = 11 Hz, 1 H), 2.80 (d, *J* = 10 Hz, 1 H), 2.39-2.35 (m, 2 H), 2.17-2.07 (m, 2 H),1.94-1.91 (m, 1H), 1.59 (t, *J* = 7 Hz, 2 H), 1.51-1.46 (m, 1 H).

### Synthesis of trans-1-(4-chloro-phenyl)-3-[3-(5-chloro-thiophen-2-yl)-1-(3-methoxy-propiopyl)-piperidin-4-yl]-urea (Ex-106)

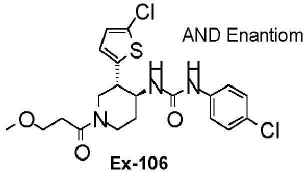

To a stirred solution of 3-methoxy-propionic acid (40 mg, 0.384 mmol, 1.0 eq) in dry THF (10 ml) were added EDCI-HCl (110 mg, 0.576 mmol, 1.5 eq) and HOBt (77 mg, 0.576 mmol, 1.5 eq) in ice-cold condition followed by addition of TEA (160 µl, 1.152 mmol, 3 eq) and *trans*-1-(4-chloro-phenyl)-3-[3-(5-chloro-t$hiophen-2-yl)-piperidin-4-yl]-urea hydrochloride (**BB-VII-HCl,** 155 mg, 0.384 mmol, 1.0 eq) and stirred at RT for 16 h. The reaction mixture was diluted with EtOAc and washed with water and brine. The organic layer was dried over anhyd. Na₂SO₄ and concentrated under reduced pressure to obtain crude product which was purified by reverse phase prep-HPLC purification to obtain *trans*-1-(4-chloro-phenyl)-3-[3-(5-chloro-thiophen-2-yl)-1-(3-methoxy-propionyl)-piperidin-4-yl]-urea (**Ex-106,** 30 mg, 17% yield) as off white solid
LC-MS (Method 2): m/z [M+H]⁺ = 456.2 (exact mass calc. = 455.08).
¹H-NMR (400 MHz, DMSO-d6, 100 °C): δ 8.28 (s, 1 H), 7.34 (d, *J* = 9 Hz, 2 H), 7.20 (d, *J* = 9 Hz, 2 H), 6.90 (d, *J* = 4 Hz, 1 H), 6.84 (d, *J* = 4 Hz, 1 H), 6.14 (d, *J* = 8 Hz, 1 H), 4.40-4.39 (m, 1 H), 4.21-4.13 (m, 1 H), 3.84-3.82 (m, 1 H), 3.62-3.60 (m, 2 H), 3.04-2.92 (m, 6 H), 2.66-2.58 (m, 2 H), 2.03-1.99 (m, 1 H), 1.49-1.46 (m, 1 H).

### Synthesis of trans-1-(4-chloro-phenyl)-3-[3-(5-chloro-thiophen-2-yl)-1-(4-methoxy-cyclohexanecarbonyl)-piperidin-4-yl]-urea (Ex-107)

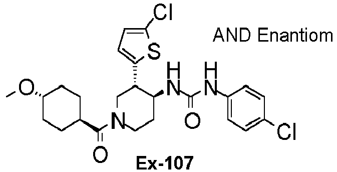

To a stirred solution of 4-methoxy-cyclohexanecarboxylic acid (40 mg, 0.25 mmol, 1.0 eq) in dry THF (10 ml) were added EDCI-HCl (72 mg, 0.375 mmol, 1.5 eq) and HOBt (51 mg, 0.375 mmol, 1.5 eq) in ice-cold condition followed by addition of TEA (104 µl, 0.75 mmol, 3 eq) and *trans*-1-(4-chloro-phenyl)-3-[3-(5-chloro-thiophen-2-yl)-piperidin-4-yl]-urea hydrochloride (**BB-VII-HCl,** 102 mg, 0.25 mmol, 1.0 eq) and stirred at RT for 18 h. The reaction mixture was diluted with EtOAc and washed with water and brine. The organic layer was dried over anhyd. Na₂SO₄ and concentrated under reduced pressure to obtain crude product which was purified by reverse phase prep-HPLC purification to obtain *trans*-1-(4-chloro-phenyl)-3-[3-(5-chloro-thiophen-2-yl)-1-(4-methoxy-cyclohexanecarbonyl)-piperidin-4-yl]-urea (**Ex-107,** 48 mg, 38% yield) as off white solid.
LC-MS (Method 2): m/z [M+H]⁺ = 510.3 (exact mass calc. = 509.13).
¹H-NMR (400 MHz, DMSO-d6): δ 8.32 (s, 1 H), 7.39-7.34 (m, 2 H), 7.23-7.19 (m, 2 H), 6.93-6.90 (m, 1 H), 6.85-6.76 (m, 1 H), 6.20 (s, 1 H), 4.29 (d, *J* = 7 Hz, 1 H), 4.13 (s, 1 H), 3.84 (s, 1 H), 3.25 (s, 3 H), 3.14-2.95 (m, 2 H), 2.62-2.57 (m, 1 H), 2.02 (d, *J* = 12 Hz, 3 H), 1.74-1.65 (m, 3 H) 1.46-140 (m, 3 H)1.2-1.17 (m, 3 H).

### Synthesis of trans-1-(4-chloro-phoyl)-3-(5-methoxy-1'-oxetan-3-yl-1',2',3',4',5',6'-hexahydro-[2,3']bipyridinyl-4'-yl)-urea (Ex-108)

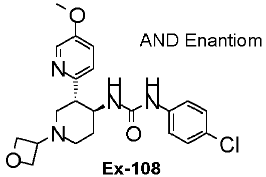

To a stirred solution of 1-(4-chloro-phenyl)-3-(5-methoxy-1',2',3',4',5',6'-hexahydro-[2,3']bipyridinyl-4'-yl)-urea hydrochloride (**BB-III-HCl,** 100 mg, 0.25 mmol, 1 eq) in MeOH (10 ml) were added oxetan-3-one (36 mg, 0.50 mmol, 2 eq) and stirred at RT for 16 h. NaBH₃N (47 mg, 0.76 mmol, 3 eq) was added at RT and the reaction was continued for 3 h. The reaction mixture was diluted with 10% MeOH-DCM and washed with water and brine. The organic layer was dried over anhyd. Na₂SO₄ and concentrated under reduced pressure to obtain crude product which was purified by reverse phase purification to obtain *trans*-1-(4-chloro-phenyl)-3-(5-methoxy-1'-oxetan-3-yl-1',2',3',4',5',6'-hexahydro-[2,3']bipyridinyl-4'-yl)-urea (**Ex-108,** 55 mg, 53% yield) as off white solid.
LC-MS (Method 6): m/z [M+H]⁺ = 417.3 (exact mass calc. = 416.16).
¹H-NMR (400 MHz, DMSO-d6): δ 8.338 (s, 1 H), 8.186 (d, *J* = 3 Hz, 1 H), 7.316-7.183 (m, 6 H), 6.018 (d, *J* = 8 Hz, 1 H), 4.546-4.378 (m, 4 H), 3.854-3.796 (m, 1 H), 3.765 (s, 3 H), 3.434-3.402 (m, 1 H), 2.9208-2.856 (m, 1 H), 2.763-2.69 (m, 2 H), 2.062-1.903 (m, 3 H), 1.5206-1.482 (m, 1 H).

### Synthesis of trans-1-(4-chloro-phenyl)-3-(5-methoxy-1'-oxetan-3-ylmethyl-1',2',3',4',5',6'-hexahydro-[2,3']bipyridinyl-4'-yl)-urea (Ex-109)

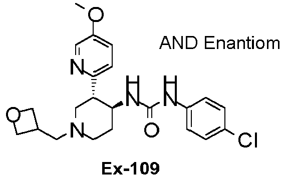

To a stirred solution of 1-(4-chloro-phenyl)-3-(5-methoxy-1',2',3',4',5',6'-hexahydro-[2,3']bipyridinyl-4'-yl)-urea hydrochloride (**BB-III-HCl,** 100 mg, 0.25 mmol, 1 eq) in ACN (4 ml) were added Cs₂CO₃ (246 mg, 0.75 mmol, 3 eq) followed by the addition of 3-(iodomethyl)oxetane (48 mg, 0.25 mmol, 1 eq) and stirred at RT for 16 h. The reaction mixture was diluted with 10% MeOH-DCM and washed with water and brine. The organic layer was dried over anhyd. Na₂SO₄ and concentrated under reduced pressure to obtain crude product which was purified by reverse phase purification to *trans*-1-(4-chloro-phenyl)-3-(5-methoxy-1'-oxetan-3-ylmethyl-1',2',3',4',5',6'-hexahydro-[2,3']bipyridinyl-4'-yl)-urea (**Ex-109,** 42 mg, 39% yield) as off white solid.
LC-MS (Method 2): m/z [M+H]⁺ = 431.2 (exact mass calc. = 430.18).
1H-NMR (400 MHz, DMSO-d6, 100 °C): δ 8.22 (d, *J* = 3 Hz, 1 H), 8.14 (s, 1 H), 7.34-7.25 (m, 4 H), 7.18 (d, *J* = 9 Hz, 2 H), 6.05-6.01 (m, 1 H), 4.72-4.67 (m, 2 H), 4.38-4.34 (m, 2 H), 4.03-3.96 (m, 1 H), 3.82 (s, 3 H), 3.42-3.32 (m, 1 H), 3.28-3.12 (m, 5 H), 2.13 (d, *J* = 15 Hz, 1 H), 1.84-1.82 (m, 1 H).

### Synthesis of trans-1-(4-chlorophenyl)-3-(1-(1,1-dioxidotetrahydro-2H-thiopyran-4-yl)-3-(5-methoxypyridin-2-yl)piperidin-4-yl)urea (Ex-110)

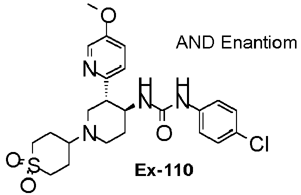

To a stirred solution of 1-(4-chloro-phenyl)-3-(5-methoxy-1',2',3',4',5',6'-hexahydro-[2,3']bipyridinyl-4'-yl)-urea hydrochloride (**BB-III-HCl,** 100 mg, 0.25 mmol, 1 eq) in MeOH (10 ml) were added tetrahydro-4H-thiopyran-4-one 1,1-dioxide (145 mg, 1.0 mmol, 4 eq) and stirred at RT for 16 h. Then NaBH₃N (47 mg, 0.76 mmol, 3 eq) was added at RT and reaction was continued for 3 h. The reaction mixture was then diluted with 10% MeOH-DCM and washed with water and brine. The organic layer was dried over anhyd. Na₂SO₄ and concentrated under reduced pressure to obtain crude product which was purified by reverse phase prep-HPLC purification to obtain trans-1-(4-chlorophenyl)-3-(1-(1,1-dioxidotetrahydro-2H-thiopyran-4-yl)-3-(5-methoxypyridin-2-yl)piperidin-4-yl)urea (**Ex-110,** 25 mg, 20% yield) as off white solid.
LC-MS (Method 6): m/z [M+H]⁺ = 493.3 (exact mass calc. = 492.16).
¹H-NMR (400 MHz, DMSO-d6): δ 8.38 (s, 1 H), 8.19 (d, *J* = 3 Hz, 1 H), 7.32-7.28 (m, 3 H), 7.24 (d, *J* = 9 Hz, 1 H), 7.19 (d, *J* = 9 Hz, 2 H), 6.02 (d, *J* = 8 Hz, 1 H), 3.83-3.80 (m, 1 H), 3.76 (s, 3 H), 3.11-3.08 (m, 4 H), 2.86-2.80 (m, 3 H), 2.68 (d, *J* = 9 Hz, 1 H), 2.42-2.29 (m, 2 H), 2.03-1.97 (m, 5 H), 1.42 (d, *J* = 8 Hz, 1 H).

### Synthesis of trans-1-(4-chloro-phoyl)-3-[1'-(3-dimethylamino-propyl)-5-methoxy-1',2',3',4',5',6'-hexahydro-[2,3']bipyridinyl-4'-yl]-urea (Ex-111)

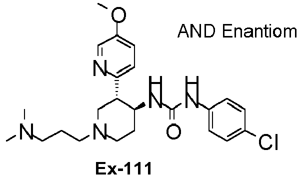

To a stirred solution of 1-(4-chloro-phenyl)-3-(5-methoxy-1',2',3',4',5',6'-hexahydro-[2,3']bipyridinyl-4'-yl)-urea hydrochloride (**BB-III-HCl,** 100 mg, 0.25 mmol, 1 eq) in ACN (5 ml) were added Cs₂CO₃ (246 mg, 0.75 mmol, 3 eq) followed by the addition of (3-chloro-propyl)-dimethyl-amine (37 mg, 0.30 mmol, 1.2 eq) and stirred at RT for 16 h. The reaction mixture was diluted with 10% MeOH-DCM and washed with water and brine. The organic layer was dried over anhyd. Na₂SO₄ and concentrated under reduced pressure to obtain crude product which was purified by reverse phase prep-HPLC purification to obtain *trans*-1-(4-chloro-phenyl)-3-[1'-(3-dimethylaminopropyl)-5-methoxy-1',2',3',4',5',6'-hexahydro-[2,3']bipyridinyl-4'-yl]-urea (**Ex-111,** 28 mg, 25% yield) as off white solid.
LC-MS (Method 1): m/z [M+H]⁺ = 446.4 (exact mass calc. = 445.22).
¹H-NMR (400 MHz, DMSO-d6): δ 8.53 (s, 1 H), 8.18 (d, *J* = 3 Hz, 1 H), 7.31-7.17 (m, 6 H), 6.18 (d, *J* = 8 Hz, 1 H), 3.82-3.80 (m, 1 H), 3.76 (s, 3 H), 2.90-2.83 (m, 3 H), 2.32-2.28 (m, 2 H), 2.20-2.16 (m, 2 H), 2.13-2.08 (m, 1 H), 2.04 (s, 6 H), 1.98-1.78 (m, 2 H), 1.57-1.39 (m, 3 H).

### Synthesis of trans-1-(4-chlorophenyl)-3-[3-(5-methoxypyridin-2-yl)piperidin-4-yl]urea (Ex-112)

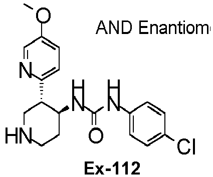

0.15 g of **BB-III-TFA** were purified by preparative HPLC to yield *trans*-1-(4-chlorophenyl)-3-[3-(5-methoxypyridin-2-yl)piperidin-4-yl]urea (**Ex-112**, 0.0556 , 47%)
LC-MS (Method 3): m/z [M+H]⁺ = 361.4 (exact mass calc. = 360.14).
¹H-NMR (400 MHz, DMSO-d6): δ 8.29 (s, 1H), 8.18 (d, *J*= 2.9 Hz, 1H), 7.27-7.30 (m, 3H), 7.18-7.21 (m, 3H), 5.98 (d, *J*= 8.2 Hz, 1H), 3.85-3.93 (m, 1H), 3.76 (s, 3H), 2.94-2.97 (m, 2H), 2.53-2.70 (m, 3H), 1.94-1.97 (m, 1H), 1.23-1.34 (m, 1H).

### Synthesis of trans-1-(4-cyanophenyl)-3-[1-(2,2-difluoroethyl)-3-(5-methoxy-2-pyridyl)-4-piperidyl]urea (Ex-113)

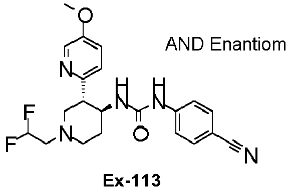

To the stirring solution of compound *trans*-1-(4-cyanophenyl)-3-[-3-(5-methoxypyridin-2-yl)-piperidin-4-yl]urea trifluoroacetate (**BB-XI-TFA**, 0.10 g, 0.214 mmol, 1.0 eq) in acetonitrile (10 ml), K₂CO₃ (0.148 g, 1.074 mmol, 5.0 eq) was added at 0 °C and allowed to stir for 10 minutes, then compound 2,2-difluoroethyl trifluoromethanesulfonate (0.055 g, 0.257 mmol, 1.2 eq) was added and again allowed to stir for 24 hours. After completion of reaction water was added, extracted with ethyl acetate (3x100 ml). Ethyl acetate part was collected, dried over Na₂SO₄ and evaporated to dryness to get crude, which was purified by column chromatography to afford *trans-*1-(4-cyanophenyl)-3-[1-(2,2-difluoroethyl)-3-(5-methoxy-2-pyridyl)-4-piperidyl]urea (**Ex-113,** 0.04216 g, 47%).
LC-MS (Method 3): m/z [M+H]⁺ = 416.4 (exact mass calc. = 415.18).
¹H-NMR (400 MHz, DMSO-d6): δ 8.74 (s, 1H), 8.19 (d, *J* = 2.7 Hz, 1H), 7.60 (d, *J* = 8.7 Hz, 2H), 7.44 (d, *J* = 8.7 Hz, 2H), 7.29-7.32 (m, 1H), 7.24 (d, *J* = 8.5 Hz, 1H), 5.98-6.28 (m, 2H), 3.81-3.87 (m, 1H), 3.76 (s, 3H), 2.71-2.94 (m, 5H), 2.31-2.45 (m, 2H), 1.97-1.99 (m, 1H), 1.50-1.56 (m, 1H).

### Synthesis of trans-1-(4-chloro-phenyl)-3-[1'-(4-hydroxy-cyclohexylmethyl)-5-methoxy-1',2',3',4',5',6'-hexahydro-[2,3']bipyridinyl-4'-yl]-urea (Ex-114)

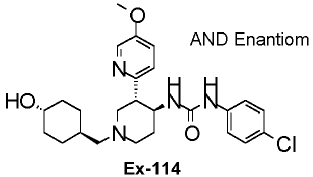

To a stirred solution of *trans*-1-(4-chloro-phenyl)-3-[1'-(4-hydroxy-cyclohexanecarbonyl)-5-methoxy-1',2',3',4',5',6'-hexahydro-[2,3']bipyridinyl-4'-yl]-urea (**Ex-90**, 130 mg, 0.267 mmol, 1.0 eq) in dry THF (5 ml) at ice-cold condition were added BH₃-DMS (1 ml) and stirred at RT for 40 min. The reaction mixture was diluted with EtOAc and the organic layer was then washed with water and brine. The organic layer was dried over anhyd. Na₂SO₄ and concentrated under reduced pressure to obtain crude product which was purified by reverse phase prep-HPLC purification to obtain *trans*-1-(4-chloro-phenyl)-3-[1'-(4-hydroxy-cyclohexylmethyl)-5-methoxy-1',2',3',4',5',6'-hexahydro-[2,3']bipyridinyl-4'-yl]-urea (**Ex-114**, 27 mg, 21% yield) as off white solid.
LC-MS (Method 1): m/z [M+H]⁺ = 473.4 (exact mass calc. = 472.22).
¹H-NMR (400 MHz, DMSO-d6): δ 8.34 (s, 1 H), 8.18 (d, *J* = 3 Hz, 1 H), 7.31-7.18 (m, 6 H), 5.96 (d, *J* = 8 Hz, 1 H), 4.44 (d, *J=* 4 Hz, 1 H), 3.80-3.78 (m, 1 H), 3.76 (s, 3 H), 2.87-2.80 (m, 3 H), 2.08-1.96 (m, 5 H), 1.80-1.70 (m, 4 H), 1.68-1.38 (m, 1 H), 1.37-1.11 (m, 1 H), 1.10-1.07 (m, 2 H), 0.84-0.810 (m, 2 H).

### Synthesis of trans-1-(4-chloro-phenyl)-3-[5-methoxy-1'-(4-methoxy-cyclohexylmethyl)-1',2',3',4',5',6'-hexahydro-[2,3']bipyridinyl-4'-yl]-urea (Ex-115)

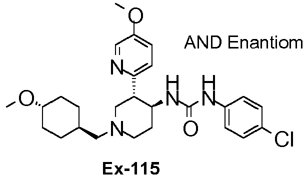

To a stirred solution of *trans*-1-(4-chloro-phenyl)-3-[5-methoxy-1'-(4-methoxy-cyclohexanecarbonyl)-1',2',3',4',5',6'-hexahydro-[2,3']bipyridinyl-4'-yl]-urea (**Ex-81**, 100 mg, 0.2 mmol, 1.0 eq) in dry THF (5 ml) at ice-cold condition were added BH₃-DMS (0.7 ml) and stirred at RT for 40 min. The reaction mixture was diluted with EtOAc and washed with water and brine. The organic layer was dried over anhyd. Na₂SO₄ and concentrated under reduced pressure to obtain crude product which was purified by reverse phase prep-HPLC purification to obtain *trans*-1-(4-chloro-phenyl)-3-[5-methoxy-1'-(4-methoxy-cyclohexylmethyl)-1',2',3',4',5',6'-hexahydro-[2,3']bipyridinyl-4'-yl]-urea (**Ex-115,** 24 mg, 24% yield) as off white solid.
LC-MS (Method 2): m/z [M+H]⁺ = 487.5 (exact mass calc. = 486.24).
¹H-NMR (400 MHz, DMSO-d6): δ 8.39 (s, 1 H), 8.18 (s, 1 H), 7.30-7.20 (m, 4 H), 7.19 (d*, J* = 12 Hz, 2 H), 6.01 (d, *J* = 8 Hz, 1 H), 3.81 (d, *J* = 12 Hz, 1 H), 3.76 (s, 3 H), 3.32 (s, 3 H), 3.05-3.03 (m, 1 H), 2.99-2.84 (m, 1 H), 2.81-2.67 (m, 4 H), 2.13-2.11 (m, 3 H), 2.08-1.96 (m, 4 H), 1.76 (d, *J* = 13 Hz, 2 H), 1.62-1.42 (m, 2 H), 1.08-0.99 (m, 2 H), 0.89-0.72 (m, 2 H).

### Synthesis of trans-1-(4-chloro-phenyl)-3-[3-(5-chloro-thiophen-2-yl)-1-(4-methoxy-cyclohexylmethyl)-piperidin-4-yl]-urea (Ex-116)

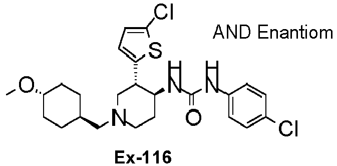

To a stirred solution of *trans*-1-(4-chloro-phenyl)-3-[3-(5-chloro-thiophen-2-yl)-1-(4-methoxy-cyclohexanecarbonyl)-piperidin-4-yl]-urea (**Ex-107**, 130 mg, 0.255 mmol, 1.0 eq) in dry THF (10 ml) at ice-cold condition were added BH₃-DMS (1 M in THF, 1 ml) and stirred at RT for 40 min. After that the reaction mixture was quenched with MeOH (1 ml). The reaction mixture was diluted with EtOAc and washed with water and brine. The organic layer was dried over anhyd. Na₂SO₄ and concentrated under reduced pressure to obtain crude product which was purified by reverse phase prep-HPLC purification to obtain *trans*-1-(4-chloro-phenyl)-3-[3-(5-chlorothiophen-2-yl)-1-(4-methoxy-cyclohexylmethyl)-piperidin-4-yl]-urea (**Ex-116,** 18 mg, 14% yield) as off white solid.
LC-MS (Method 3): m/z [M+H]⁺ = 496.4 (exact mass calc. = 495.15).
¹H-NMR (400 MHz, DMSO-d6): δ 8.46 (s, 1 H), 7.36-7.34 (m, 2 H), 7.23-7.11 (m, 2 H), 6.92 (d, *J* = 4 Hz, 1 H), 6.82 (d, *J* = 4 Hz, 1 H), 6.19 (d, *J* = 8 Hz, 1 H), 3.56-3.52 (m, 1 H), 3.25 (s, 3 H), 3.19-2.99 (m, 2 H), 2.98-2.88 (m, 1 H), 2.87-2.56 (m, 1 H), 2.26-2.08 (m, 4 H), 2.04-1.95 (m, 3 H), 1.79-1.76 (m, 2 H), 1.49-1.37 (m, 2 H), 1.19-0.98 (m, 2 H), 0.91-0.78 (m, 2 H).

### Synthesis of trans-1-(4-chloro-phenyl)-3-[5-methoxy-1'-(2-methylamino-ethyl)-1',2',3',4',5',6'-hexahydro-[2,3']bipyridinyl-4'-yl]-urea (Ex-117)

### Synthesis of trans-(2-{4'-[3-(4-chloro-phenyl)-ureido]-5-methoxy-3',4',5',6'-tetrahydro-2'H-[2,3']bipyridinyl-1'-yl}-ethyl)-methyl-carbamic acid tert-butyl ester

To a stirred solution of 1-(4-chloro-phenyl)-3-(5-methoxy-1',2',3',4',5',6'-hexahydro-[2,3']bipyridinyl-4'-yl)-urea hydrochloride (**BB-III-HCl,** 100 mg, 0.25 mmol, 1 eq) in MeOH (10 ml) were added *tert*-methyl-(2-oxo-ethyl)-carbamic acid tert-butyl ester (130 mg, 0.75 mmol, 3 eq) and stirred at RT for 16 h. After 16 h NaBH₃N (47 mg, 0.76 mmol, 3 eq) was added at RT and reaction was continued for 3 h. The reaction mixture was diluted with 10% MeOH-DCM and washed with water and brine. The organic layer was dried over anhyd. Na₂SO₄ and concentrated under reduced pressure to obtain crude product *trans*-(2-{4'-[3-(4-chloro-phenyl)-ureido]-5-methoxy-3',4',5',6'-tetrahydro-2'H-[2,3']bipyridinyl-1'-yl}-ethyl)-methyl-carbamic acid tert-butyl ester (100 mg, 77% crude yield) as yellow sticky which was used without further purification.
LC-MS (Method 1): m/z [M+H]⁺ = 518.4 (exact mass calc. = 517.25).

### Synthesis of trans-1-(4-chloro-phenyl)-3-[5-methoxy-1'-(2-methylamino-ethyl)-1',2',3',4',5',6'-hexahydro-[2,3']bipyridinyl-4'-yl]-urea (Ex-117)

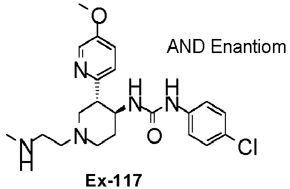

To a stirred solution of *trans*-(2-{4'-[3-(4-chloro-phenyl)-ureido]-5-methoxy-3',4',5',6'-tetrahydro-2'H-[2,3']bipyridinyl-1'-yl}-ethyl)-methyl-carbamic acid tert-butyl ester (100 mg, 0.193 mmol, 1 eq) in dioxane (2 ml) was added 2 ml HC in dioxane (4 M) and stirred at RT for 3 h. The reaction mixture was concentrated under reduced pressure to obtain crude product which was purified by reverse phase prep-HPLC purification to obtain *trans*-1-(4-chloro-phenyl)-3-[5-methoxy-1'-(2-methylamino-ethyl)-1',2',3',4',5',6'-hexahydro-[2,3']bipyridinyl-4'-yl]-urea (**Ex-117**), 12 mg, 15% yield) as off white solid
LC-MS (Method 2): m/z [M+H]⁺ = 418.2 (exact mass calc. = 417.19).
¹H-NMR (400 MHz, DMSO-d6): δ 8.49 (s, 1 H), 8.18 (d, *J* = 4 Hz, 1 H), 7.31-7.17 (m, 6 H), 6.13 (d, *J* = 8 Hz, 1 H), 3.98-3.91 (m, 1 H), 3.76 (s, 3 H), 2.97-2.79 (m, 3 H), 2.58-2.49 (m, 2 H), 2.39-2.36 (m, 2 H), 2.26 (s, 3 H), 2.18-1.93 (m, 3 H), 1.56-1.39 (m, 1 H).

### Synthesis of trans-1-[1'-(2-dimethylamino-acetyl)-5-methoxy-1',2',3',4',5',6'-hexahydro-[2,3']bipyridinyl-4'-yl]-3-(3-methyl-isothiazol-5-yl)-urea (Ex-118)

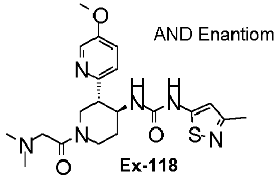

To a stirred solution of dimethylamino-acetic acid (50 mg, 0.49 mmol, 1.0 eq) in dry THF (10 ml) were added EDCI-HCl (140 mg, 0.73 mmol, 1.5 eq) and HOBt (99 mg, 0.73 mmol, 1.5 eq) in ice-cold condition followed by addition of TEA (202 µl, 1.46 mmol, 3 eq) and *trans-*1-(5-methoxy-1',2',3',4',5',6'-hexahydro-[2,3']bipyridinyl-4'-yl)-3-(3-methyl-isothiazol-5-yl)-urea hydrochloride (**BB-IV-HCl,** 194 mg, 0.49 mmol, 1.0 eq) and stirred at RT for 18 h. The reaction mixture was diluted with EtOAc and washed with water and brine. The organic layer was dried over anhyd. Na₂SO₄ and concentrated under reduced pressure to obtain crude product which was purified by reverse phase prep-HPLC purification to obtain *trans*-1-[1'-(2-dimethylamino-acetyl)-5-methoxy-1',2',3',4',5',6'-hexahydro-[2,3']bipyridinyl-4'-yl]-3-(3-methyl-isothiazol-5-yl)-urea (**Ex-118,** 34 mg, 16% yield) as off white solid LC-MS (Method 3): m/z [M+H]⁺ = 433.4 (exact mass calc. = 432.19).
¹H-NMR (400 MHz, DMSO-d6, 100 °C): δ 9.55 (s, 1 H), 8.22 (s, 1 H), 7.29-7.22 (m, 2 H), 6.38 (s, 1 H), 6.24 (d, *J=* 12 Hz, 1 H), 4.28-4.19 (m, 2 H), 4.10-4.07 (m, 1 H), 3.80 (s, 3 H), 3.11 (s, 2 H), 3.09-2.93 (m, 2 H),2.89-2.69 (m, 2 H), 2.23-2.21 (m, 9 H), 2.08-2.05 (m, 1 H).

### Synthesis of trans-1-[5-methoxy-1'-(oxetane-3-carbonyl)-1',2',3',4',5',6'-hexahydro-[2,3']bipyridinyl-4'-yl]-3-(3-methyl-isothiazol-5-yl)-urea (Ex-119)

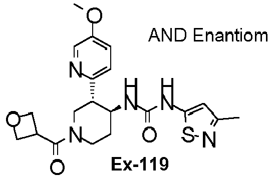

To a stirred solution of oxetane-3-carboxylic acid (40 mg, 0.4mmol, 1.0 eq) in dry THF (10 ml) were added EDCI-HCl (114 mg, 0.6 mmol, 1.5 eq) and HOBt (81mg, 0.6 mmol, 1.5 eq) in ice-cold condition followed by addition of TEA (167 µl, 1.2 mmol, 3 eq) and *trans*-1-(5-methoxy-1',2',3',4',5',6'-hexahydro-[2,3']bipyridinyl-4'-yl)-3-(3-methyl-isothiazol-5-yl)-urea hydrochloride (**BB-IV-HCl,** 153 mg, 0.4 mmol, 1.0 eq) and stirred at RT for 18 h. The reaction mixture was diluted with EtOAc and washed with water and brine. The organic layer was dried over anhyd. Na₂SO₄ and concentrated under reduced pressure to obtain crude product which was purified by reverse phase prep-HPLC purification to obtain *trans*-1-[5-methoxy-1'-(oxetane-3-carbonyl)-1',2',3',4',5',6'-hexahydro-[2,3']bipyridinyl-4'-yl]-3-(3-methyl-isothiazol-5-yl)-urea (**Ex-119,** 18 mg, 11% yield) as off white solid.
LC-MS (Method 2): m/z [M+H]⁺ = 432.3 (exact mass calc. = 431.16).
¹H-NMR (400 MHz, DMSO-d6): δ 9.92 (s, 1 H), 8.19 (d, *J* = 72 Hz, 1 H), 7.31-7.28 (m, 1 H), 7.22-7.19 (m, 1 H), 6.47 (d, *J* = 72 Hz, 1 H), 6.36 (m, 1 H), 4.69-4.58 (m, 4 H), 4.42-4.39 (m, 1 H), 4.15-4.10 (m, 1 H), 4.04-4.01 (m, 1 H), 3.75 (s, 3 H), 3.44-3.40 (m, 1 H), 3.20-3.09 (m, 1 H), 2.83-2.76 (m, 2 H), 2.16 (s, 3 H), 1.99-1.96 (m, 1 H), 1.39-1.36 (m, 1 H).

### Synthesis of trans-1-(4-chloro-phenyl)-3-[1'-(2-hydroxy-2-methyl-propyl)-5-methoxy-1',2',3',4',5',6'-hexahydro-[2,3']bipyridinyl-4'-yl]-urea (Ex-120)

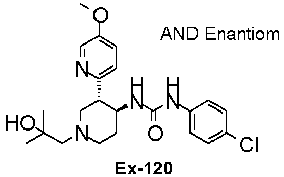

To a stirred solution of 1-(4-chloro-phenyl)-3-(5-methoxy-1',2',3',4',5',6'-hexahydro-[2,3']bipyridinyl-4'-yl)-urea hydrochloride (**BB-III-HCl,** 100 mg, 0.25 mmol, 1 eq) in EtOH (5 ml) were added 2,2-dimethyl-oxirane (36 mg, 0.50 mmol, 2 eq) and stirred at 90 °C for 18 h. The reaction mixture was diluted with 10% MeOH-DCM and washed with water and brine. The organic layer was dried over anhyd. Na₂SO₄ and concentrated under reduced pressure to obtain crude product which was purified by reverse phase purification to obtain *trans*-1-(4-chlorophenyl)-3-[1'-(2-hydroxy-2-methyl-propyl)-5-methoxy-1',2',3',4',5',6'-hexahydro-[2,3']bipyridinyl-4'-yl]-urea
(Ex-120, 15 mg, 14% yield) as off white solid
LC-MS (Method 2): m/z [M+H]⁺ = 433.2 (exact mass calc. = 432.19).
¹H-NMR (DMSO-d6): δ 8.32 (s, 1 H), 8.18 (s, 1 H), 7.30 (d, *J* = 5 Hz, 3 H), 7.24-7.18 (m, 3 H), 5.94 (d, *J* = 8 Hz, 1 H), 4.04 (s, 1 H), 2.88-2.82 (m, 1 H), 3.76 (s, 3 H), 3.01-2.95 (t, *J* = 12 Hz, 2 H), 2.88 (t, *J* = 11 Hz, 1 H), 2.34-2.18 (m, 4 H), 1.94 (d, *J* = 11 Hz, 1 H), 1.50 (d, *J* = 12 Hz, 1 H), 1.08 (s, 6 H).

### Synthesis of trans-1-[1'-(2-hydroxy-2-methyl-propyl)-5-methoxy-1',2',3',4',5',6'-hexahydro-[2,3']bipyridinyl-4'-yl]-3-(3-methyl-isothiazol-5-yl)-urea (Ex-121)

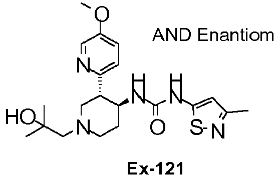

To a stirred solution of *trans*-1-(5-methoxy-1',2',3',4',5',6'-hexahydro-[2,3']bipyridinyl-4'-yl)-3-(3-methylisothiazol-5-yl)-urea hydrochloride (**BB-IV-HCl,** 150 mg, 0.39 mmol, 1 eq) in EtOH (8 ml) were added 2,2-dimethyl-oxirane (56 mg, 0.78 mmol,2 eq) and stirred at 90 °C for 16 h. The reaction mixture was diluted with 10% MeOH-DCM and washed with water and brine. The organic layer was dried over anhyd. Na₂SO₄ and concentrated under reduced pressure to obtain crude product which was purified by reverse phase purification to obtain *trans*-1-[1'-(2-hydroxy-2-methyl-propyl)-5-methoxy-1',2',3',4',5',6'-hexahydro-[2,3']bipyridinyl-4'-yl]-3-(3-methyl-isothiazol-5-yl)-urea (**Ex-121,** 24 mg, 14% yield) as off white solid.
LC-MS (Method 2): m/z [M+H]⁺ = 420.4 (exact mass calc. = 419.20).
¹H-NMR (400 MHz, DMSO-d6): δ 9.85 (s, 1 H), 8.18 (d, *J* = 3 Hz, 1 H), 7.29-7.26 (m, 1 H), 7.21 (d, *J* = 9 Hz, 1 H), 6.42-6.39 (m, 2 H), 4.04 (s, 1 H), 3.86-3.81 (m, 1 H), 3.76 (s, 1 H), 2.99-2.94 (m, 3 H), 2.35-2.27 (m, 2 H), 2.23-2.19 (m, 4 H), 1.91 (d, *J* = 7 Hz, 1 H), 1.56 (d, *J* = 11 Hz, 1 H), 1.08 (s, 6 H).

### Synthesis of trans-1-[1'-(2-hydroxy-ethyl)-5-methoxy-1',2',3',4',5',6'-hexyhydro-[2,3']bipyridinyl-4'-yl]-3-(3-methyl-isothiazol-5-yl)-urea (Ex-122)

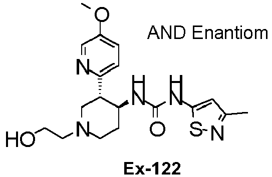

To a stirred solution of *trans-*1-(5-methoxy-1',2',3',4',5',6'-hexahydro-[2,3']bipyridinyl-4'-yl)-3-(3-methylisothiazol-5-yl)-ureahydrochloride (**BB-IV-HCl,** 100 mg, 0.26 mmol, 1 eq) in ACN (5 ml) were added Cs₂CO₃ (254 mg, 0.78 mmol, 3 eq) followed by the addition of 2-bromo-ethanol (39 mg, 0.31 mmol, 1.2 eq) and stirred at RT for 16 h. The reaction mixture was diluted with 10% MeOH-DCM and washed with water and brine. The organic layer was dried over anhyd. Na₂SO₄ and concentrated under reduced pressure to obtain crude product which was purified by reverse phase purification to obtain *trans-*1-[1'-(2-hydroxy-ethyl)-5-methoxy-1',2',3',4',5',6'-hexahydro-[2,3']bipyridinyl-4'-yl]-3-(3-methyl-isothiazol-5-yl)-urea (**Ex-122,** 14 mg, 14% yield) as off white solid.
LC-MS (Method 2): m/z [M+H]⁺ = 392.1 (exact mass calc. = 391.17).
¹H-NMR (400 MHz, DMSO-d6): δ 9.85 (s, 1 H), 8.19 (d, *J* = 3 Hz, 1 H), 7.30-7.27 (m, 1 H), 7.21 (d, *J* = 9 Hz, 1 H), 6.49 (br s, 1 H), 6.39 (s, 1 H), 4.36 (t, *J* = 5 Hz, 1 H), 3.84-3.81 (m, 1 H), 3.76 (s, 3 H), 3.48 (d, *J* = 34 Hz, 2 H), 2.95-2.90 (m, 3 H), 2.42-2.39 (m, 2 H), 2.22-2.14 (m, 3 H), 2.11-1.93 (m, 2 H), 1.57-1.48 (m, 1 H).

### Synthesis of trans-1-[1'-(2-hydroxy-acetyl)-5-methoxy-1',2',3',4',5',6'-hexahydro-[2,3']bipyridinyl-4'-yl]-3-(3-methyl-isothiazol-5-yl)-urea (Ex-123)

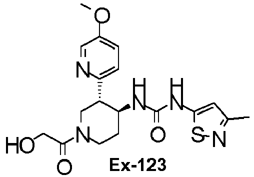

To a stirred solution of hydroxy-acetic acid (24 mg, 0.31 mmol, 1.2 eq) in dry THF (10 ml) were added EDCI-HCl (75 mg, 0.39 mmol, 1.5 eq) and HOBt (52 mg, 0.39 mmol, 1.5 eq) at RT followed by the addition of TEA (108 µl, 0.78 mmol, 3 eq) and 1-(5-methoxy-1',2',3',4',5',6'-hexahydro-[2,3']bipyridinyl-4'-yl)-3-(3-methylisothiazol-5-yl)-ureahydrochloride (**BB-IV-HCl,** 100 mg, 0.26 mmol, 1 eq) and stirred at RT for 16 h. The reaction mixture was diluted with 10% MeOH-DCM and washed with water and brine. The organic layer was dried over anhyd. Na₂SO₄ and concentrated under reduced pressure to obtain crude product which was purified by reverse phase purification to *trans*-1-[1'-(2-hydroxy-acetyl)-5-methoxy-1',2',3',4',5',6'-hexahydro-[2,3']bipyridinyl-4'-yl]-3-(3-methyl-isothiazol-5-yl)-urea (**Ex-123,** 17 mg, 16% yield) as off white solid.
LC-MS (Method 2): m/z [M+H]⁺ = 406.4 (exact mass calc. = 405.15).
¹H-NMR (400 MHz, DMSO-d6): δ 9.94 (s, 1 H), 8.22 (d, *J* = 2 Hz, 1 H), 7.33-7.32 (m, 1 H), 7.27-7.23 (m, 1 H), 6.59-6.52 (br s, 1 H), 6.40 (s, 1 H), 4.58 (d, *J* = 5 Hz, 1 H), 4.45-4.39 (m, 1 H), 4.12-4.08 (m, 3 H), 3.78 (s, 3 H), 3.75-3.71 (m, 1 H), 3.27-3.09 (m, 1 H), 2.93-2.67 (m, 2 H), 2.20 (s, 3 H), 1.99 (d, *J=* 13 Hz, 1 H), 1.51-1.38 (m, 1 H).

### Synthesis of trans-1-(4-chlorophenyl)-3-(3-(5-methoxypyridin-2-yl)-1-((1-(methylsulfonyl)-1H-pyrazol-4-yl)methyl)piperidin-4-yl)urea (Ex-125)

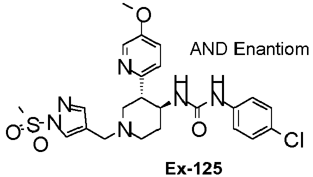

To the solution of *trans*-1-(4-chlorophenyl)-3-(3-(5-methoxypyridin-2-yl)piperidin-4-yl)urea trifluoroacetate (**BB-III-TFA,** 0.15 g, 0.3163 mmol, 1 eq) in ACN (10 ml), K₂CO₃ (0.218g,1.5815mmol,5eq), 4-(bromomethyl)-1-methanesulfonyl-1H-pyrazole (0.09 g, 0.3796 mmol, 1.2 eq) followed by was added at RT. The reaction was allowed to stir at room temperature for 16 hour. After completion of reaction (checked by TLC, 5% MeOH in DCM, R_{f}: 0.3), the reaction mixture was passed through celite bed and concentrated to get crude which was purified by column chromatography using 0-10% MeOH in DCM and 230-400 silica gel to get *trans*-1-(4-chlorophenyl)-3-(3-(5-methoxypyridin-2-yl)-1-((1-(methylsulfonyl)-1H-pyrazol-4-yl)methyl)piperidin-4-yl)urea (**Ex-125,** 0.07409 g, 45.13%).
LC-MS (Method 3): m/z [M+H]⁺ = 519.4 (exact mass calc. = 518.15).
¹H-NMR (400 MHz, DMSO-d6): δ 8.33 (s, 1H), 8.18 (d, *J* = 2.1 Hz, 1H), 8.10-8.14 (m, 1H), 7.88 (s, 1H), 7.17-7.29 (m, 6H), 5.98 (d, *J*= 6.5 Hz, 1H), 3.80-3.90 (m, 1H), 3.76 (s, 3H), 3.45-3.51 (m, 5H), 2.80-2.90 (m, 3H), 1.99-2.16 (m, 3H), 1.48-1.50 (m, 1H).

### Synthesis of trans-1-(4-chlorophenyl)-3-[3-(5-methoxy-2-pyridyl)-1-methyl-4-piperidyl]urea (Ex-126)

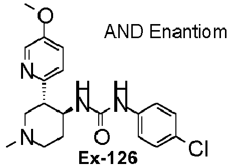

To the solution of *trans*1-(4-chlorophenyl)-3-[3-(5-methoxypyridin-2-yl)-piperidin-4-yl]urea trifluoroacetate (**BB-III-TFA,** 0.05 g, 0.1054 mmol, 1 eq) in MeOH (10 ml), NaOAc (0.043 g, 0.5272 mmol, 5 eq), formalin solution (0.2 ml), AcOH (0.2 ml) was added at RT. The reaction was allowed to stir at room temperature for 2 hour. After completion of reaction (checked by TLC, 5% MeOH in DCM, R_{f}: 0.3), the reaction mixture was concentrated to get crude which was purified by column chromatography using 0-10% MeOH in DCM and 230-400 silica gel to get *trans*-1-(4-chlorophenyl)-3-[3-(5-methoxy-2-pyridyl)-1-methyl-4-piperidyl]urea (**Ex-126,** 0.02356 g, 59.63%).
LC-MS (Method 3): m/z [M+H]⁺ = 375.3 (exact mass calc. = 374.15).
¹H-NMR (400 MHz, DMSO-d6): δ 8.32 (s, 1H), 8.19 (d, *J*= 2.9 Hz, 1H), 7.28-7.32 (m, 3H), 7.18-7.24 (m, 3H), 6.02 (d, *J*= 7.7 Hz, 1H), 3.80-3.83 (m, 1H), 3.77 (s, 3H), 2.87-2.93 (m, 3H), 1.99-2.32 (m, 6H), 1.47-1.56 (m, 1H).

### Synthesis of trans-1-(4-chlorophenyl)-3-(1-(cyclopropylmethyl)-3-(5-methoxypyridin-2-yl)piperidin-4-yl)urea (Ex-127)

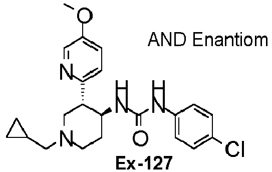

To the solution of *trans*1-(4-chlorophenyl)-3-[3-(5-methoxypyridin-2-yl)-piperidin-4-yl]urea trifluoroacetate **(BB-III-TFA,** 0.12 g, 0.2530 mmol, 1 eq) in ACN (10 ml), K₂CO₃ (0.1745 g, 1.265mmol, 5 eq), followed by (bromomethyl)cyclopropane (0.0372 g, 0.2783 mmol, 1.1 eq), was added at RT. The reaction was allowed to stir at room temperature for 16 hour. After completion of reaction (checked by TLC, 5% MeOH in DCM, R_{f}: 0.3), the reaction mixture was concentrated to get crude which was purified by column chromatography using 0-10% MeOH in DCM and 230-400 silica gel to get *trans*-1-(4-chlorophenyl)-3-(1-(cyclopropylmethyl)-3-(5-methoxypyridin-2-yl)piperidin-4-yl)urea (**Ex-127,** 0.03215 g, 30.62%).
LC-MS (Method 3): m/z [M+H]⁺ = 415.4 (exact mass calc. = 414.18).
¹H-NMR (400 MHz, DMSO-d6): δ 8.32 (s, 1H), 8.19 (d, *J*= 2.3 Hz, 1H), 7.18-7.30 (m, 6H), 5.96 (d, *J*= 8.0 Hz, 1H), 3.81-3.84 (m, 1H), 3.77 (s, 3H), 2.85-3.01 (m, 3H), 1.99-2.23 (m, 5H), 1.44-1.52 (m, 1H), 0.80-0.90 (m, 1H), 0.42-0.44 (m, 2H), 0.03-0.10 (m, 2H).

### Synthesis of trans-1-(4-cyanophenyl)-3-[3-(5-methoxypyridin-2-yl)-1-[(6-methylpyridin-3-yl)carbonyl]piperidin-4-yl]urea (Ex-128)

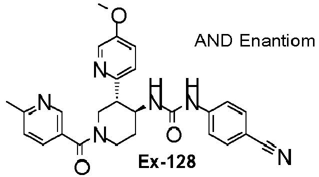

To a stirred solution 6-methylpyridine-3-carboxylic acid (0.0486 g, 0.3545 mmol, 1.5 eq) in DMF (10 ml) was added EDCI-HCl (0.068 g, 0.3545 mmol, 1.5 eq), HOBt (0.038 g, 0.2835 mmol, 1.2 eq), DIPEA (0.19 ml, 1.1815 mmol, 5.0 eq) and stirred at RT for 30 min followed by addition of a *trans*-1-(4-cyanophenyl)-3-[3-(5-methoxypyridin-2-yl)piperidin-4-yl]urea trifluoroacetate (**BB-XI-TFA,** 0.110 g, 0.2363 mmol, 1.0 eq). The reaction mixture was stirred for 16 hr at RT. After completion of the reaction (monitored by TLC, TLC system 5% MeOH-DCM, _{Rf}: 0.4) reaction mixture was poured into cold water and extracted with EtOAc (2 x 25 ml). The organic layer was washed with water (2 x 25 ml) and brine (2 x 25 mL), dried over anhyd. Na₂SO₄, concentrated in vacuo. The crude material thus obtained was purified by flash chromatography (230-400 mesh silica gel; 5% MeOH-DCM as an elutent) to get *trans*-1-(4-cyanophenyl)-3-[3-(5-methoxypyridin-2-yl)-1-[(6-methylpyridin-3-yl)carbonyl]piperidin-4-yl]urea (**Ex-128,** 0.03118g,28.04%).
LC-MS (Method3): m/z [M+H]⁺ = 471.4(exact mass calc. = 470.21).
¹H-NMR (400 MHz, DMSO-d6 at 100 °C): δ 8.50-8.51 (m, 2H), 8.21 (d, *J* = 2.5 Hz, 1H), 7.68-7.71 (m, 1H), 7.54 (d, *J* = 8.7 Hz, 2H), 7.43-7.45 (m, 2H), 7.23-7.30 (m, 3H), 6.06 (d, *J* = 7.7 Hz, 1H), 4.10-4.16 (m, 3H), 3.79 (s, 3H), 3.19-3.31 (m, 2H), 2.97 (t, *J* = 5.4 Hz, 1H), 2.49-2.51 (m, 2H), 2.09-2.13 (m, 1H), 1.58-1.62 (m, 1H).

### Synthesis of trans-2-4-{[(4-chlorophenyl)carbamoyl]amino}-3-(5-methoxypyridin-2-yl)piperidin-1-yl acetamide (Ex-129)

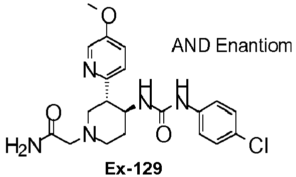

To the solution of *trans*-1-(4-chlorophenyl)-3-(3-(5-methoxypyridin-2-yl)piperidin-4-yl)urea trifluoroacetate **(BB-III-TFA,** 0.1 g, 0.2109 mmol, 1 eq) in ACN (10 ml), K₂CO₃ (0.145 g, 1.054 mmol, 5 eq), followed by 2-bromoacetamide (0.0349 g,0.2530 mmol,1.2 eq), was added at RT. The reaction was allowed to stir at room temperature for 16 hour. After completion of reaction (checked by TLC, 5% MeOH in DCM, R_{f}: 0.3), the reaction mixture was concentrated to get crude which was purified by column chromatography using 0-10% MeOH in DCM and 230-400 silica gel to get *trans*-2-4-{[(4-chlorophenyl)carbamoyl]amino}-3-(5-methoxypyridin-2-yl)piperidin-1-yl acetamide (**Ex-129,** 0.048 g, 54.46 %).
LC-MS (Method 3): m/z [M+H]⁺ = 418.4 (exact mass calc. = 417.02).
¹H-NMR (400 MHz, DMSO-d6): δ 8.33 (s, 1H), 8.19 (d, J=2.84 Hz, 1H), 7.32- 7.30(m, 3H), 7.29-7.18 (m, 4H), 7.11 (bs, 1H), 6.00-5.99 (m, 1H),3.82-3.80(m,1H) 3.77 (s, 3H), 2.95-2.89 (m, 5H), 2.32-2.24(m, 2H), 1.99(d, J= 10.08, 1H), 1.58-1.55 (m, 1H).

### Synthesis of trans-1-(4-chloro-phenyl)-3-[3-(5-chloro-thiophen-2-yl)-1-(piperidine-4-carbonyl)-piperidin-4-yl]-urea (Ex-130)

### Synthesis of trans-4-[4-[3-(4-chloro-phenyl)-ureido]-3-(5-chloro-thiophen-2-yl)-piperidine-1-carbonyl]-piperidine-1-carboxylic acid tert-butyl ester

To a stirred solution of piperidine-1,4-dicarboxylic acid mono-tert-butyl ester (50 mg, 0.218 mmol, 1.0 eq) in dry THF (10 ml) were added EDCI-HCl (63 mg, 0.327 mmol, 1.5 eq) and HOBt (45 mg, 0.327 mmol, 1.5 eq) in ice-cold condition followed by addition of TEA (92 µl, 0.654 mmol, 3 eq) and *trans*-1-(4-chloro-phenyl)-3-[3-(5-chloro-thiophen-2-yl)-piperidin-4-yl]-urea hydrochloride (**BB-VII-HCl,** 90 mg, 0.218 mmol, 1.0 eq) and stirred at RT for 18 h. The reaction mixture was diluted with EtOAc and washed with water and brine. The organic layer was dried over anhyd. Na₂SO₄ and concentrated under reduced pressure to obtain crude *trans*-4-[4-[3-(4-chlorophenyl)-ureido]-3-(5-chloro-thiophen-2-yl)-piperidine-1-carbonyl]-piperidine-1-carboxylic acid tert-butyl ester (120 mg, 95% crude yield) as brownish solid.
LC-MS (Method 2): m/z [M+H]⁺ = 580.9 (exact mass calc. = 580.17).

### Synthesis of trans-1-(4-chloro-phenyl)-3-[3-(5-chloro-thiophen-2-yl)-1-(piperidine-4-carbonyl)-piperidin-4-yl]-urea (Ex-130)

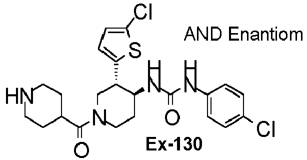

To a stirred solution of *trans*-4-[4-[3-(4-chloro-phenyl)-ureido]-3-(5-chloro-thiophen-2-yl)-piperidine-1-carbonyl]-piperidine-1-carboxylic acid tert-butyl ester (200 mg, 0.344 mmol, 1 eq) in 1,4-dioxane (5 ml) was added 4M HCl in dioxane (2 ml) followed by stirring at RT for a period of 4 h. After completion of the reaction (monitored by TLC and LC-MS), the reaction mixture was concentrated under reduced pressure to get the crude material and it was purified by reverse phase prep-HPLC to obtain *trans*-1-(4-chloro-phenyl)-3-[3-(5-chloro-thiophen-2-yl)-1-(piperidine-4-carbonyl)-piperidin-4-yl]-urea (**Ex-130,** 22 mg, 13% yield) as off white solid.
LC-MS (Method 3): m/z [M+H]⁺ = 481.1 (exact mass calc. = 480.12).
¹H-NMR (400 MHz, DMSO-d6, 100 °C): δ 8.20 (s, 1 H), 7.38-7.33 (m, 2 H), 7.24-7.19 (m, 2 H), 6.93-6.91 (m, 1 H), 6.83 (m, 1 H), 6.06 (d, *J* = 8 Hz, 1 H), 4.45-4.35 (m, 1 H), 4.18-4.08 (m, 1 H), 3.95-3.82 (m, 2 H), 3.62-3.51 (m, 1 H), 3.02-2.87 (m, 4 H), 2.92-2.72 (m, 1 H), 2.62-2.52 (m, 2 H), 2.03 (d, *J* = 8 Hz, 1 H), 1.61-1.42 (m, 6 H).

### Synthesis of trans-1-(4-chloro-phenyl)-3-[3-(5-chloro-thiophen-2-yl)-1-(3-hydroxy-propionyl)-piperidin-4-yl]-urea (Ex-131)

### Synthesis of trans-1-[1-[3-(tert-butyl-dimethyl-silanyloxy)-propionyl]-3-(5-chloro-thiophen-2-yl)-piperidin-4-yl]-3-(4-chloro-phenyl)-urea

To a stirred solution of 3-(tert-butyl-dimethyl-silanyloxy)-propionic acid (40 mg, 0.196 mmol, 1.0 eq) in dry THF (10 ml) were added EDCI-HCl (56 mg, 0.294 mmol, 1.5 eq) and HOBt (40 mg, 0.294 mmol, 1.5 eq) in ice-cold condition followed by addition of TEA (82 µl, 0.588 mmol, 3 eq) and *trans*-1-(4-chloro-phenyl)-3-[3-(5-chloro-thiophen-2-yl)-piperidin-4-yl]-urea hydrochloride (**BB-VII-HCl,** 80 mg, 0.218 mmol, 1.0 eq) and stirred at RT for 18 h. The reaction mixture was diluted with EtOAc and washed with water and brine. The organic layer was dried over anhyd. Na₂SO₄ and concentrated under reduced pressure to obtain crude *trans*-1-[1-[3-(tert-butyl-dimethyl-silanyloxy)-propionyl]-3-(5-chloro-thiophen-2-yl)-piperidin-4-yl]-3-(4-chloro-phenyl)-urea (100 mg, 92% crude yield) as brownish sticky.
LC-MS (Method 2): m/z [M+H]⁺ = 556.3 (exact mass calc. = 555.15).

### Synthesis of trans-1-(4-chloro-phepyl)-3-[3-(5-chloro-thiophen-2-yl)-1-(3-hydroxy-propiopyl)-piperidin-4-yl]-urea (Ex-131)

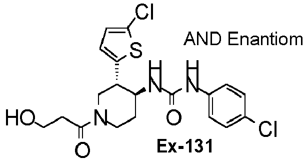

To a stirred solution of crude *trans*-1-[1-[3-(tert-butyl-dimethyl-silanyloxy)-propionyl]-3-(5-chloro-thiophen-2-yl)-piperidin-4-yl]-3-(4-chloro-phenyl)-urea (100 mg, 0.180 mmol, 1.0 eq) in dioxane (5 ml) were added HCl in 1,4-dioxane (4 M, 1.5 ml) drop wise in ice-cold condition and stirred at RT for 4 h. The reaction mixture was then evaporated under reduced pressure to obtain crude product which was purified by reverse phase prep-HPLC purification to obtain *trans*-1-(4-chloro-phenyl)-3-[3-(5-chloro-thiophen-2-yl)-1-(3-hydroxy-propionyl)-piperidin-4-yl]-urea (Ex-131, 15 mg, 18% yield) as off white solid.
LC-MS (Method 2): m/z [M+H]⁺ = 442.3 (exact mass calc. = 441.07).
¹H-NMR (400 MHz, DMSO-d6, 100 °C): δ 8.20 (s, 1 H), 7.38-7.33 (m, 2 H), 7.32-7.19 (m, 2 H), 6.94-6.91 (m, 1 H), 6.84 (d, 1 H), 6.04 (d, *J* = 8 Hz, 1 H), 4.29-4.13 (m, 3 H), 3.89-3.84 (m, 2 H), 3.71-3.60 (m, 2 H), 3.12-3.98 (m, 2 H), 2.66-2.46 (m, 2 H), 2.04-1.94 (m, 1 H), 1.59-1.39 (m, 1 H).

### Synthesis of trans-1-(4-chloro-phenyl)-3-[3-(5-chloro-thiophen-2-yl)-1-(4-hydroxy-cyclohexanecarbonyl)-piperidin-4-yl]-urea (Ex-132)

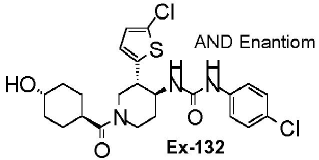

To a stirred solution of 4-hydroxy-cyclohexanecarboxylic acid (40 mg, 0.277 mmol, 1.0 eq) in dry THF (10 ml) were added EDCI-HCl (80 mg, 0.415 mmol, 1.5 eq) and HOBt (57 mg, 0.415 mmol, 1.5 eq) in ice-cold condition followed by addition of TEA (115 µl, 0.831 mmol, 3 eq) and *trans*-1-(4-chloro-phenyl)-3-[3-(5-chloro-thiophen-2-yl)-piperidin-4-yl]-urea hydrochloride (**BB-VII-HCl,** 112 mg, 0.277 mmol, 1.0 eq) and stirred at RT for 18 h. The reaction mixture was diluted with EtOAc and washed with water and brine. The organic layer was dried over anhyd. Na₂SO₄ and concentrated under reduced pressure to get the crude material and it was purified by reverse phase prep-HPLC to obtain *trans-*1-(4-chloro-phenyl)-3-[3-(5-chloro-thiophen-2-yl)-1-(4-hydroxy-cyclohexanecarbonyl)-piperidin-4-yl]-urea (**Ex-132,** 25 mg, 18% crude yield) as off white solid.
LC-MS (Method 3): m/z [M+H]⁺ = 496.4 (exact mass calc. = 495.12).
¹H-NMR (400 MHz, DMSO-d6, 100 °C): δ 8.22 (s, 1 H), 7.38-7.33 (m, 2 H), 7.23-7.19 (m, 2 H), 6.84 (d, *J=* 4 Hz, 1 H), 6.76 (d, *J* = 4 Hz, 1 H), 6.09 (d, *J* = 8 Hz, 2 H), 4.38-4.21 (m, 1 H), 4.22-4.05 (m, 2 H), 3.91-3.86 (m, 1 H), 3.40-3.36 (m, 1 H), 3.15-2.95 (m, 2 H), 2.05-1.98 (m, 1 H), 1.90 (d,J= 36 Hz, 2 H), 1.79-1.63 (m, 2 H), 1.55-1.42 (m, 3 H), 1.32-1.18 (m, 3 H).

### Synthesis of trans-1-(5-methoxy-1'-oxetan-3-yl-1',2',3',4',5',6'-hexahydro-[2,3']bipyridinyl-4'-yl)-3-(3-methyl-isothiazol-5-yl)-urea (Ex-133)

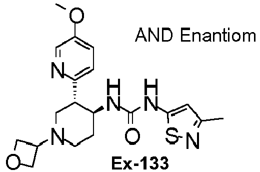

To a stirred solution of 1-(5-methoxy-1',2',3',4',5',6'-hexahydro-[2,3']bipyridinyl-4'-yl)-3-(3-methyl-isothiazol-5-yl)-urea hydrochloride (**BB-IV-HCl,** 100 mg, 0.26 mmol, 1 eq) in MeOH (5 ml) were added oxetan-3-one (36 mg, 0.50 mmol, 2 eq) and stirred at RT for 16 h. NaBH₃CN (49 mg, 0.78 mmol, 3 eq) was added at RT and the reaction was continued for 3 h. The reaction mixture was diluted with 10% MeOH-DCM and washed with water and brine. The organic layer was dried over anhyd. Na₂SO₄ and concentrated under reduced pressure to obtain crude product which was purified by reverse phase purification to obtain *trans-*1-(5-methoxy-1'-oxetan-3-yl-1',2',3',4',5',6'-hexahydro-[2,3']bipyridinyl-4'-yl)-3-(3-methyl-isothiazol-5-yl)-urea (**Ex-133,** 32 mg, 31% yield) as off white solid.
LC-MS (Method 2): m/z [M+H]⁺ = 404.4 (exact mass calc. = 403.17).
¹H-NMR (400 MHz, DMSO-d6, 100 °C): δ 9.53 (s, 1 H), 8.18 (d, *J* = 4 Hz, 1 H), 7.27-7.21 (m, 2 H), 6.37 (s, 1 H), 6.17 (d, *J* = 8 Hz, 1 H), 4.57-4.43 (m, 4 H), 3.89-3.81 (m, 1 H), 3.78 (s, 3 H), 3.56-3.52 (m, 1 H), 3.01-2.95 (m, 1 H), 2.85-2.76 (m, 2 H), 2.25 (s, 3 H), 2.15-1.95 (m, 2 H), 1.91-1.76 (m, 1 H), 1.74-1.61 (m, 1 H).

### Synthesis of trans-1-[5-methoxy-1'-(2-methylamino-ethyl)-1',2',3',4',5',6'-hexahydro-[2,3']bipyridinyl-4' yl]-3-(3-methyl-isothiazol-5-yl)-urea (Ex-134)

### Synthesis of trans-(2-{5-methoxy-4'-[3-(3-methyl-isothiazol-5-yl)-ureido]-3',4',5',6'-tetrahydro-2'H-[2,3']bipyridinyl-1'-yl}-ethyl)-methyl-carbamic acid tert-butyl ester

To a stirred solution of 1-(5-methoxy-1',2',3',4',5',6'-hexahydro-[2,3']bipyridinyl-4'-yl)-3-(3-methyl-isothiazol-5-yl)-urea hydrochloride (**BB-IV-HCl,** 100 mg, 0.26 mmol, 1 eq) in MeOH (7 ml) were added methyl-(2-oxo-ethyl)-carbamic acid tert-butyl ester (90 mg, 0.52 mmol, 2 eq) and stirred at RT for 16 h. NaBH₃CN (50 mg, 0.78 mmol, 3 eq) was added at RT and the reaction was continued for 3 h. The reaction mixture was diluted with 10% MeOH-DCM and washed with water and brine. The organic layer was dried over anhyd. Na₂SO₄ and concentrated under reduced pressure to obtain crude *trans*-(2-{5-methoxy-4'-[3-(3-methyl-isothiazol-5-yl)-ureido]-3',4',5',6'-tetrahydro-2'H-[2,3']bipyridinyl-1'-yl}-ethyl)-methyl-carbamic acid tert-butyl ester (125 mg, 95% crude yield) as brownish solid.
LC-MS (Method 2): m/z [M+H]⁺ = 505.5 (exact mass calc. = 504.25).

### Synthesis of trans-1-[5-methoxy-1'-(2-methylamino-ethyl)-1',2',3',4',5',6'-hexahydro-[2,3']bipyridinyl-4'-yl]-3-(3-methyl-isothiazol-5-yl)-urea (Ex-134)

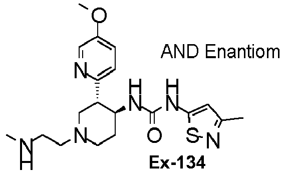

To a stirred solution of *trans*-(2-{5-methoxy-4'-[3-(3-methyl-isothiazol-5-yl)-ureido]-3',4',5',6'-tetrahydro-2'H-[2,3']bipyridinyl-1'-yl}-ethyl)-methyl-carbamic acid tert-butyl ester (150 mg, 0.297 mmol, 1 eq) in dioxane (2 ml) was added 5 ml of HCl in dioxane (4 M) and stirred at RT for 3 h. The reaction mixture was concentrated under reduced pressure to obtain crude product which was purified by reverse phase prep-HPLC purification to obtain *trans*-1-[5-methoxy-1'-(2-methylamino-ethyl)-1',2',3',4',5',6'-hexahydro-[2,3']bipyridinyl-4'-yl]-3-(3-methyl-isothiazol-5-yl)-urea (**Ex-134**, 35 mg, 45% yield) as off white solid.
LC-MS (Method 2): m/z [M+H]⁺ = 404.8 (exact mass calc. = 404.20).
¹H-NMR (400 MHz, DMSO-d6, 100 °C): δ 9.56 (br s, 1 H), 8.19 (s, 1 H), 7.27-7.20 (m, 2 H), 6.36 (s, 1 H), 6.14 (d, *J* = 8 Hz, 1 H), 3.87-3.81 (m, 1 H), 3.79 (s, 3 H), 2.98-2.94 (m, 3 H), 2.71-2.69 (m, 2 H), 2.55-2.41 (m, 2 H), 2.35 (s, 3 H), 2.31-2.25 (m, 1 H), 2.23 (s, 3 H), 2.21-2.18 (m, 1 H), 2.05 (d, *J* = 32 Hz, 1 H), 1.69-1.52 (m, 1 H);

### Synthesis of trans-1-[5-methoxy-1'-(3-methylamino-propionyl)-1',2',3',4',5',6'-hexahydro-[2,3']bipyridinyl-4'-yl]-3-(3-methyl-isothiazol-5-yl)-urea (Ex-135)

### Synthesis of trans-(3-{5-methoxy-4'-[3-(3-methyl-isothiazol-5-yl)-ureido]-3',4',5',6'-tetrahydro-2'H-[2,3']bipyridinyl-1'-yl}-3-oxo-propyl)-methyl-carbamic acid tert-butyl ester

To a stirred solution of 3-(tert-butoxycarbonyl-methyl-amino)-propionic acid (30 mg, 0.15 mmol, 1 eq) in dry THF (10 ml) were added EDCI-HCl (42 mg, 0.225 mmol, 1.5 eq),HOBt (30 mg, 0.225 mmol, 1.5 eq) and TEA (60 µl, 0.45 mmol, 1.5 eq) at RT followed by the addition of *trans*-1-(5-methoxy-1',2',3',4',5',6'-hexahydro-[2,3']bipyridinyl-4'-yl)-3-(3-methyl-isothiazol-5-yl)-urea hydrochloride (**BB-IV-HCl**, 57 mg, 0.15 mmol, 1 eq) was added and continued stirring for 18 h at RT. The reaction mixture was diluted with 10% MeOH-DCM and washed with water and brine. The organic layer was dried over anhyd. Na₂SO₄ and concentrated under reduced pressure to obtain crude *trans*-(3-{5-methoxy-4'-[3-(3-methyl-isothiazol-5-yl)-ureido]-3',4',5',6'-tetrahydro-2'H-[2,3']bipyridinyl-1'-yl}-3-oxo-propyl)-methyl-carbamic acid tert-butyl ester (80 mg, 100% crude yield) as yellow sticky.
LC-MS (Method 1): m/z [M+H]⁺ = 533.2 (exact mass calc. = 532.25).

### Synthesis of trans-1-[5-methoxy-1'-(3-methylamino-propionyl)-1',2',3',4',5',6'-hexahydro-[2,3']bipyridinyl-4'-yl]-3-(3-methyl-isothiazol-5-yl)-urea (Ex-135)

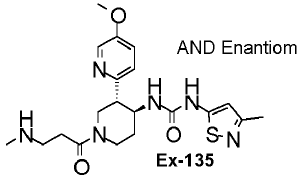

To a stirred solution of *trans*-(3-{5-methoxy-4'-[3-(3-methyl-isothiazol-5-yl)-ureido]-3',4',5',6'-tetrahydro-2'H-[2,3']bipyridinyl-1'-yl}-3-oxo-propyl)-methyl-carbamic acid tert-butyl ester (150 mg, 0.28 mmol, 1 eq) in 1,4-dioxane (3 ml) was added 4M HCl in dioxane (1 ml) was added and stirred at RT for 3 h. The reaction mixture was concentrated under reduced pressure to obtain crude product which was purified by reverse phase prep-HPLC purification to obtain *trans*-1-[5-methoxy-1'-(3-methylamino-propionyl)-1',2',3',4',5',6'-hexahydro-[2,3']bipyridinyl-4'-yl]-3-(3-methyl-isothiazol-5-yl)-urea (**Ex-135**, 20 mg, 16% yield) as off white solid.
LC-MS (Method 1): m/z [M+H]⁺ = 433.3 (exact mass calc. = 432.19).
¹H-NMR (400 MHz, DMSO-d6, 100 °C): δ 8.22 (d, *J* = 4 Hz, 1 H), 7.30-7.23 (m, 3 H), 6.38 (s, 1 H), 6.23 (d, *J* = 8 Hz, 1 H), 4.22-4.03 (m, 4 H), 3.80 (s, 3 H), 2.91-2.75 (m, 2 H), 2.74-2.67 (m, 2 H), 2.38 (s, 3 H), 2.25 (s, 3 H), 2.15-2.09 (m, 2 H), 1.59-1.45 (m, 2 H).

### Synthesis of trans-1-(4-chloro-phepyl)-3-[1'-(2-dimethylamino-ethyl)-5-methoxy-1',2',3',4',5',6'-hexahydro-[2,3']bipyridinyl-4'-yl]-urea (Ex-136)

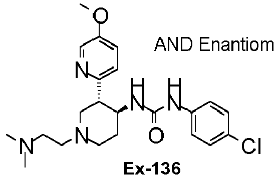

To a stirred solution of 1-(4-chloro-phenyl)-3-(5-methoxy-1',2',3',4',5',6'-hexahydro-[2,3']bipyridinyl-4'-yl)-urea hydrochloride (**BB-III-HCl,** 100 mg, 0.25 mmol, 1 eq) in ACN (5 ml) were added Cs₂CO₃ (246 mg, 0.75 mmol, 3 eq) followed by the addition of (2-chloro-ethyl)-dimethyl-amine (32 mg, 0.30 mmol, 1.2 eq) and stirred at RT for 18 h. The reaction mixture was diluted with 10% MeOH-DCM and washed with water and brine. The organic layer was dried over anhyd. Na₂SO₄ and concentrated under reduced pressure to obtain crude product which was purified by reverse phase purification to obtain *trans*-1-(4-chloro-phenyl)-3-[1'-(2-dimethylamino-ethyl)-5-methoxy-1',2',3',4',5',6'-hexahydro-[2,3']bipyridinyl-4'-yl]-urea (**Ex-136,** 15 mg, 14% yield) as off white solid.
LC-MS (Method 7): m/z [M+H]⁺ = 432.2 (exact mass calc. = 431.21).
¹H-NMR (400 MHz, DMSO-d6): δ 8.34 (s, 1 H), 8.14 (d, *J* = 4 Hz, 1 H), 7.26-7.13 (m, 6 H), 5.99 (d, *J* = 8 Hz, 1 H), 3.78-3.75 (m, 2 H), 3.72 (s, 3 H), 2.88-2.80 (m, 3 H), 2.36-2.33 (m, 2 H), 2.28-2.25 (m, 2 H), 2.14-2.11 (m, 1 H), 2.06 (s, 6 H), 1.91-1.83 (m, 1 H), 1.49-1.35 (m, 1 H).

### Synthesis of trans-1-(4-chloro-phenyl)-3-[5-methoxy-1'-(3-methylamino-propyl)-1',2',3',4',5',6'-hexahydro-[2,3']bipyridinyl-4'-yl]-urea (Ex-137)

### Synthesis of trans-(3-{4'-[3-(4-chloro-phenyl)-ureido]-5-methoxy-3',4',5',6'-tetrahydro-2'H-[2,3']bipyridinyl-1'-yl}-3-oxo-propyl)-methyl-carbamic acid tert-butyl ester

To a stirred solution of 3-(tert-butoxycarbonyl-methyl-amino)-propionic acid (123 mg, 0.60 mmol, 1.2 eq) in dry THF (10 ml) were added EDCI-HCl (144 mg, 0.75 mmol, 1.5 eq) and HOBt (102 mg, 0.75 mmol, 1.5 eq) in RT condition followed by addition of TEA (210 µl, 1.51 mmol, 3 eq) and 1-(4-chloro-phenyl)-3-(5-methoxy-1',2',3',4',5',6'-hexahydro-[2,3']bipyridinyl-4'-yl)-urea hydrochloride (**BB-III-HCl,** 200 mg, 0.50 mmol, 1 eq) and stirred at RT for 18 h. The reaction mixture was diluted with 10% MeOH-DCM and washed with water and brine. The organic layer was dried over anhyd. Na₂SO₄ and concentrated under reduced pressure to obtain crude product which was purified by 100-200 silioca gel using 1%MeOH-DCM as mobile phnase to obtained *trans*-(3-{4'-[3-(4-chloro-phenyl)-ureido]-5-methoxy-3',4',5',6'-tetrahydro-2'H-[2,3']bipyridinyl-1'-yl}-3-oxo-propyl)-methyl-carbamic acid tert-butyl ester (120 mg, 44% yield) as yellow sticky.
LC-MS (Method): m/z [M+H]⁺ = 546.4 (exact mass calc. = 545.24).

### Synthesis of trans-(3-{4'-[3-(4-chloro-phenyl)-ureido]-5-methoxy-3',4',5',6'-tetrahydro-2'H-[2,3']bipyridinyl-1'-yl}-propyl)-methyl-carbamic acid tert-butyl ester

To a stirred solution of *trans*-(3-{4'-[3-(4-chloro-phenyl)-ureido]-5-methoxy-3',4',5',6'-tetrahydro-2'H-[2,3']bipyridinyl-1'-yl}-3-oxo-propyl)-methyl-carbamic acid tert-butyl ester (200 mg, 0.36 mmol, 1 eq) in THF (2 ml) was added BH₃-DMS (500 µl) and stirred at RT for 30 min. The reaction mixture was quenched with water and extracted with 10% MeOH-DCM. The organic layer was separate out, dried over anhyd. Na₂SO₄, filtered and concentrated under reduced pressure to obtain crude product which was purified by 100-200 mesh silica gel using 2% MeOH in DCM as an eluent to obtain *trans*-(3-{4'-[3-(4-chloro-phenyl)-ureido]-5-methoxy-3',4',5',6'-tetrahydro-2'H-[2,3']bipyridinyl-1'-yl}-propyl)-methyl-carbamic acid tert-butyl ester (100 mg, 51%) as yellow sticky.
LC-MS (Method 2): m/z [M+H]⁺ = 532.4 (exact mass calc. = 531.26).

### Synthesis of trans-1-(4-chloro-phenyl)-3-[5-methoxy-1'-(3-methylamino-propyl)-1',2',3',4',5',6'-hexahydro-[2,3']bipyridinyl-4'-yl]-urea (Ex-137)

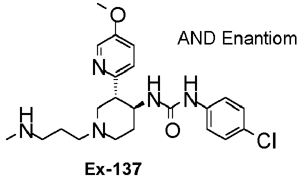

To a stirred solution of *trans*-(3-{4'-[3-(4-chloro-phenyl)-ureido]-5-methoxy-3',4',5',6'-tetrahydro-2'H-[2,3']bipyridinyl-1'-yl}-propyl)-methyl-carbamic acid tert-butyl ester (100mg, 0.18mmol, 1 eq) in 1,4-dioxane (2 ml) was added 4M HCl in dioxane followed by stirring at RT for 3 h. The reaction mixture was concentrated under reduced pressure to obtain crude product which was purified by reverse phase prep-HPLC purification to obtain *trans*-1-(4-chloro-phenyl)-3-[5-methoxy-1'-(3-methylamino-propyl)-1',2',3',4',5',6'-hexahydro-[2,3']bipyridinyl-4'-yl]-urea (**Ex-137**, 37mg, 48% yield) as off white solid.
LC-MS (Method 7): m/z [M+H]⁺ = 432.2 (exact mass calc. = 431.21).
¹H-NMR (400 MHz, DMSO-d6): δ 8.38 (s, 1 H), 8.18 (d, *J* = 4 Hz, 1 H), 7.30-7.18 (m, 6 H), 6.03 (d, *J* = 8 Hz, 1 H), 3.89-3.74 (m, 1 H), 3.76 (s, 3 H), 2.87-2.82 (m, 3 H), 2.32-2.26 (m, 4 H), 2.12-1.98 (m, 3 H), 1.54-1.45 (m, 3 H).

### Synthesis of trans-1-[1'-(3-hydroxy-cyclobutanecarbonyl)-5-methoxy-1',2',3',4',5',6'-hexahydro-[2,3']bipyridipyl-4'-yl]-3-(3-methyl-isothiazol-5-yl)-urea (Ex-138)

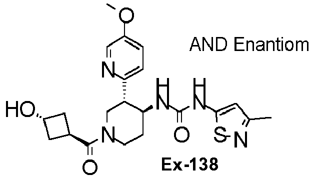

To a stirred solution of 3-hydroxy-cyclobutanecarboxylic acid (54 mg, 0.47 mmol, 1.2 eq) in dry THF (5 ml) were added EDCI-HCl (112 mg, 0.58 mmol, 1.5 eq) and HOBt (79 mg, 0.58 mmol, 1.5 eq) in RT condition followed by addition of TEA (163 µl, 1.17 mmol, 3 eq) and *trans*-1-(5-methoxy-1',2',3',4',5',6'-hexahydro-[2,3']bipyridinyl-4'-yl)-3-(3-methyl-isothiazol-5-yl)-urea hydrochloride (**BB-III-HCl,** 150 mg, 0.39 mmol, 1 eq) and stirred at RT for 16 h. The reaction mixture was diluted with 10% MeOH-DCM and washed with water and brine. The organic layer was dried over anhyd. Na₂SO₄ and concentrated under reduced pressure to obtain crude product which was purified by reverse phase purification to obtain *trans*-1-[1'-(3-hydroxy-cyclobutanecarbonyl)-5-methoxy-1',2',3',4',5',6'-hexahydro-[2,3']bipyridinyl-4'-yl]-3-(3-methyl-isothiazol-5-yl)-urea (**Ex-138**, 25 mg, 14% yield) as off white solid.
LC-MS (Method 7): m/z [M+H]⁺ = 446.1 (exact mass calc. = 445.18).
¹H-NMR (400 MHz, DMSO-d6, 100 °C): δ 9.75-9.52 (br m, 1 H), 8.21 (s, 1 H), 7.27-7.23 (m, 2 H), 6.37 (s, 1 H), 6.23 (br s, 1 H), 4.67 (br s, 1 H), 4.15-4.06 (m, 4 H), 3.79 (s, 3 H), 3.25 (br s, 1 H), 2.95-2.74 (m, 2 H), 2.65-2.34 (m, 3 H), 2.21 (s, 3 H), 2.05 (br s, 3 H), 1.47-1.45 (m, 1 H).

### Synthesis of trans-1-[1'-(2-hydroxy-acetyl)-5-methoxy-1',2',3',4',5',6'-hexahydro-[2,3']bipyridinyl-4'-yl]-3-phenyl-urea (Ex-139)

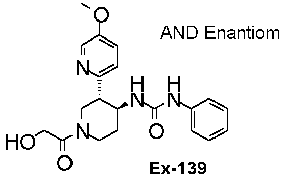

To a stirred solution of hydroxy-acetic acid(25 mg, 0.33 mmol, 1.2 eq) in dry THF (10 ml) were added EDCI-HCl (78 mg, 0.41 mmol, 1.5 eq) and HOBt (55 mg, 0.41 mmol, 1.5 eq) and TEA (115 µl, 0.825 mmol, 3.0 eq) at RT followed by the addition of *trans*-1-(5-methoxy-1',2',3',4',5',6'-hexahydro-[2,3']bipyridinyl-4'-yl)-3-phenyl-urea (**BB-C-HCl**, 100 mg, 0.275 mmol, 1 eq) was added and continued stirring for 18 h at RT. The reaction mixture was diluted with 10% MeOH-DCM and washed with water and brine. The organic layer was dried over anhyd. Na₂SO₄ and concentrated under reduced pressure to obtain crude *trans*-1-[1'-(2-hydroxy-acetyl)-5-methoxy-1',2',3',4',5',6'-hexahydro-[2,3']bipyridinyl-4'-yl]-3-phenyl-urea (**Ex-139,** 42 mg, 40% yield) as off white solid.
LC-MS (Method 7): m/z [M+H]⁺ = 385.2 (exact mass calc. = 384.18).
¹H-NMR (400 MHz, DMSO-d6): δ 8.27-8.19 (m, 2 H), 7.34-7.25 (m, 4 H), 7.17-7.13 (m, 2 H), 6.85-6.81 (m, 1 H), 6.05 (d, *J* = 8 Hz, 1 H), 4.55 (br s, 1 H), 4.42-4.40 (m, 1 H), 4.26-4.02 (m, 3 H), 3.78 (s, 3 H), 3.75-3.96 (m, 1 H), 3.16-3.10 (m, 1 H), 2.88-2.72 (m, 2 H), 2.03 (d, *J* = 12 Hz, 1 H), 1.49-1.32 (m, 1 H).

### Synthesis of trans-1-(4-cyanophenyl)-3-(-3-(5-methoxypyridin-2-yl)-1-((1-(methylsulfonyl)-1H-pyrazol-4-yl)methyl)piperidin-4-yl)urea (Ex-140)

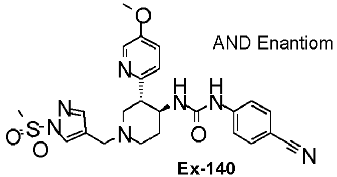

To the stirring solution of compound *trans*-1-(4-cyanophenyl)-3-[-3-(5-methoxypyridin-2-yl)-1-piperidin-4-yl]urea trifluoroacetate (**BB-XI-TFA,** 0.15 g, 0.3223 mmol, 1.0 eq) in acetonitrile (10 ml), K₂CO₃ (0.222 g, 1.6115 mmol, 5.0 eq) was added at 0 °C and allowed to stir for 10 minutes, then compound 4-(bromomethyl)-1-methanesulfonyl-1H-pyrazole (0.0924 g, 0.3867 mmol, 1.2 eq) was added and again allowed to stir for 24 hours. After completion of reaction water was added, extracted with ethyl acetate (3x100 ml). Ethyl acetate part was collected, dried over Na₂SO₄ and evaporated to dryness to get crude, which was purified by column chromatography to afford trans-1-(4-cyanophenyl)-3-(-3-(5-methoxypyridin-2-yl)-1-((1-(methylsulfonyl)-1H-pyrazol-4-yl)methyl)piperidin-4-yl)urea (**Ex-140,** 0.08567 g, 52.16%).
LC-MS (Method 3): m/z [M+H]⁺ = 510.5 (exact mass calc. = 509.18).
¹H-NMR (400 MHz, DMSO-d6): δ 8.73 (s, 1H), 8.18 (d, *J* = 2.8 Hz, 1H), 8.14 (s, 1H), 7.87 (s, 1H), 7.59 (d, *J*= 8.7 Hz, 2H), 7.43 (d, *J* = 8.8 Hz, 2H), 7.27-7.30 (m, 1H), 7.23 (*d, J* = 8.5 Hz, 1H), 6.18 (d, *J* = 8.2 Hz, 1H), 3.79-3.85 (m, 1H), 3.76 (s, 3H), 3.51 (s, 3H), 3.45 (s, 2H), 2.84-2.92 (m, 3H), 1.98-2.20 (m, 3H), 1.47-1.49 (m, 1H).

### Synthesis of trans-1-(4-cyanophenyl)-3-[3-(5-methoxypyridin-2-yl)-1-{[6-(trifluoromethyl)pyridin-3-yl]carbonyl}piperidin-4-yl]urea (Ex-141)

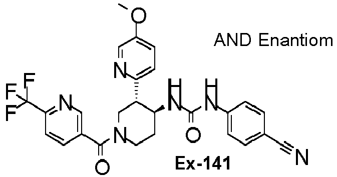

To a stirred solution 6-(trifluoromethyl)pyridine-3-carboxylic acid (0.0732 g, 0.3867 mmol, 1.5 eq) in DMF (10 ml) was added EDCI-HCl (0.0741 g, 0.3867 mmol, 1.5 eq), HOBt (0.0418 g, 0.3093 mmol, 1.2 eq), DIPEA (0.2 ml, 0.3093 mmol, 5.0 eq) and stirred at RT for 30 min followed by addition of a of *trans*-1-(4-cyanophenyl)-3-[3-(5-methoxypyridin-2-yl)-piperidin-4-yl]urea trifluoroacetate (**BB-XI-TFA,** 0.12 g, 0.2578 mmol, 1.0 eq). The reaction mixture was stirred for 16 h at RT. After completion of the reaction (monitored by TLC, TLC system 5% MeOH-DCM, R_{f}: 0.4) reaction mixture was poured into cold water and extracted with EtOAc (2 x 25 ml). The organic layer was washed with water (2 x 25 ml) and brine (2 x 25 mL), dried over anhyd. Na₂SO₄, concentrated in vacuo. The crude material thus obtained was purified by flash chromatography (230-400 mesh silica gel; 5% MeOH-DCM as an elutent) to get *trans*-1-(4-cyanophenyl)-3-[3-(5-methoxypyridin-2-yl)-1-{[6-(trifluoromethyl)pyridin-3-yl]carbonyl}piperidin-4-yl]urea (**Ex-141,** 0.03298 g, 24.39%).
LC-MS (Method 3): m/z [M+H]⁺ = 525.6 (exact mass calc. = 524.18).
¹H-NMR (400 MHz, DMSO-d6 at 100 °C): δ 8.81 (s, 1H), 8.56 (s, 1H), 8.21 (s, 1H), 8.12 (d, *J* = 8.3 Hz, 1H), 7.93 (d, *J* = 7.8 Hz, 1H), 7.55 (d, *J* = 8.1 Hz, 2H), 7.44 (d, *J* = 8.3 Hz, 2H), 7.24-7.29 (m, 2H), 6.09 (d, *J* = 8.2 Hz, 1H), 4.0-4.20 (m, 3H), 3.79 (s, 3H), 3.22-3.33 (m, 2H), 2.97-3.03 (m, 1H), 2.13 (d, *J* = 12.2 Hz, 1H), 1.61-1.65 (m, 1H).

### Synthesis of trans-3-4-(4-chlorophenyl)carbamoyl amino -3-(5-methoxypyridin-2-yl)piperidin-1-yl propanamide (Ex-142)

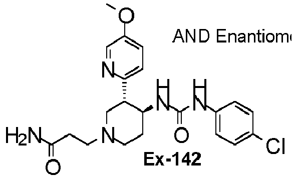

To the solution of -1-(4-chlorophenyl)-3-(3-(5-methoxypyridin-2-yl)piperidin-4-yl)urea trifluoroacetate (**BB-III-TFA,** 0.06 g, 0.1265 mmol, 1 eq) in ACN (10 ml), K₂CO₃ (0.087 g, 0.6325 mmol, 5 eq), followed by 3-bromopropanamide (0.023 g, 0.1518 mmol, 1.2 eq), was added at RT. The reaction was allowed to stir at room temperature for 16 hour. After completion of reaction (checked by TLC, 5% MeOH in DCM, R_{f}: 0.3), the reaction mixture was concentrated to get crude which was purified by column chromatography using 0-10% MeOH in DCM and 230-400 silica gel to get *trans*-3-4-(4-chlorophenyl)carbamoyl amino-3-(5-methoxypyridin-2-yl)piperidin-1-yl propanamide (**Ex-142,** 0.033 g, 60.39 %).
LC-MS (Method 3): m/z [M+H]⁺ = 432.4 (exact mass calc. = 431.06).
¹H-NMR (400 MHz, DMSO-d6): δ 8.33 (s, 1H), 8.19 (d, J=2.68 Hz, 1H), 7.33- 7.18(m, 6H), 6.75 (s, 1H), 5.99-5.97 (m, 1H),3.82-3.81(m,1H), 3.77 (s, 3H), 2.88-2.85 (m, 3H), 2.33-2.07(m, 4H), 1.99(d, J= 9.16, 1H), 1.48-1.45 (m, 1H).

### Synthesis of trans-1-(4-chlorophenyl)-3-1-[(1-cyanocyclopropyl)methyl]-3-(5-methoxypyridin-2-yl)piperidin-4-yl urea (Ex-155)

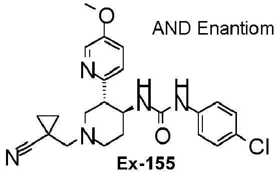

To the solution of *trans*-1-(4-chlorophenyl)-3-(3-(5-methoxypyridin-2-yl)piperidin-4-yl)urea trifluoroacetate (**BB-III-TFA,** 0.1 g, 0.2109 mmol, 1 eq) in ACN (10 ml), K₂CO₃ (0.087 g, 0.6329 mmol, 3 eq), followed by 1-(bromomethyl)cyclopropane-1-carbonitrile (0.041 g, 0.2532 mmol, 1.2 eq), was added at RT. The reaction was allowed to stir at room temperature for 16 hour. After completion of reaction (checked by TLC, 5% MeOH in DCM, R_{f}: 0.3), the reaction mixture was concentrated to get crude which was purified by column chromatography using 0-10% MeOH in DCM and 230-400 silica gel to *trans*-1-(4-chlorophenyl)-3-1-[(1-cyanocyclopropyl)methyl]-3-(5-methoxypyridin-2-yl)piperidin-4-yl urea (**Ex-155**, 0.033 g, 35.56 %).
LC-MS (Method 7): m/z [M+H]⁺ = 440.1 (exact mass calc. = 439.02).
¹H-NMR (400 MHz, DMSO-d6): δ 8.34 (s, 1H), 8.20 (d, J=2.4 Hz, 1H), 7.20- 7.18(m, 2H), 6.00-5.98 (m, 1H),3.85-3.82(m,1H), 3.77 (s, 3H),3.00 (t, J=11.36 Hz, 2H), 2.92 (t, J=7.8 Hz, 1H), 2.45-2.38 (m, 2H), 2.29 (t, J=14.2 Hz, 1H), 2.19 (t, J=15.28 Hz, 1H), 2.07-2.00 (m, 1H), 1.56-1.48 (m, 1H), 1.22-1.19(m, 2H), 0.93-0.83 (m, 2H).

### Synthesis of trans-1-4-{[(4-chlorophenyl)carbamoyl]amino}-3-(5-methoxypyridin-2-yl)piperidin-1-yl]methyl}cyclopropane-1-carboxamide: (Ex-143)

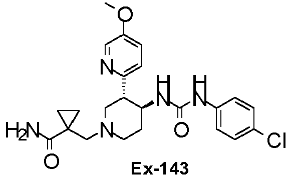

To the solution of *trans*-1-(4-chlorophenyl)-3-1-[(1-cyanocyclopropyl)methyl]-3-(5-methoxypyridin-2-yl)piperidin-4-yl urea (**Ex-155,** 0.04 g, 0.0909 mmol, 1 eq) in H₂SO₄ (0.4 ml). The reaction was allowed to stir at 90 °C for 3 hour. After completion of reaction (checked by TLC, 5% MeOH in DCM, R_{f}: 0.3), the reaction mixture was quenched by NaHCO₃ solution and extracted by ethyl acetate, dried by Na₂SO₄ and concentrated to get crude which was purified by column chromatography using 0-10% MeOH in DCM and 230-400 silica gel to *trans*-1-4-{[(4-chlorophenyl)carbamoyl]amino}-3-(5-methoxypyridin-2-yl)piperidin-1-yl]methyl}cyclopropane-1-carboxamide: (**Ex-143,** 0.034 g, 81.67 %).
LC-MS (Method 3): m/z [M+H]⁺ = 458.5 (exact mass calc. = 457.02).
¹H-NMR (400 MHz, DMSO-d6): δ 8.47 (s,1H),8.35 (s, 1H), 8.20 (d, J=2.44 Hz, 1H), 7.31- 7.29(m, 4H), 7.26-7.20 (m, 2H), 7.09 (bs, 1H), 6.02-6.00 (m, 1H),3.86-3.83(m,1H), 3.77(s,3H) ,3.06-3.04 (m, 1H), 2.95-2.89 (m, 1H), 2.50-2.42 (m, 2H), 2.19-2.04 (m, 4H), 1.55-1.47 (m, 1H), 0.95-0.91 (m, 2H), 0.49-0.41 (m, 2H).

### Synthesis of trans-1-(4-chlorophenyl)-3-1-(3-cyano-3,3-dimethylpropyl)-3-(5-methoxypyridin-2-yl)piperidin-4-yl urea (Ex-144)

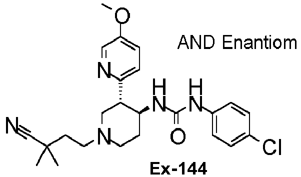

To the solution of *trans*-1-(4-chlorophenyl)-3-(3-(5-methoxypyridin-2-yl)piperidin-4-yl)urea trifluroacetate (**BB-III-TFA,** 0.15 g, 0.3163 mmol, 1 eq) in ACN (10 ml), K₂CO₃ (0.218 g, 1.5815 mmol, 5 eq), followed by 4-chloro-2,2-dimethylbutanenitrile (0.05 g,0.3796 mmol,1.2 eq), was added at RT. The reaction was allowed to stir at 90 °C for 16 hour. After completion of reaction (checked by TLC, 5% MeOH in DCM, R_{f}: 0.3), the reaction mixture was concentrated to get crude which was purified by column chromatography using 0-10% MeOH in DCM and 230-400 silica gel to *trans*-1-(4-chlorophenyl)-3-1-(3-cyano-3,3-dimethylpropyl)-3-(5-methoxypyridin-2-yl)piperidin-4-yl urea (**Ex-144,** 0.032 g, 22.18 %).
LC-MS (Method 3): m/z [M+H]⁺ = 456.4 (exact mass calc. = 455.06).
¹H-NMR (400 MHz, DMSO-d6): δ 8.31 (s, 1H), 8.19 (d, J=2.76 Hz, 1H), 7.32- 7.18(m, 6H), 6.01-5.99 (m, 1H),3.84-3.80(m,1H), 3.77(s,3H) ,2.89-2.87 (m, 3H), 2.50-2.43 (m, 2H), 2.14-2.02 (m, 2H), 1.74-1.47 (m, 4H), 1.29 (s, 6H).

### Synthesis of trans-1-(4-chlorophenyl)-3-(3 -(5-methoxypyridin-2-yl)-1-((1-methyl-1H-pyrazol-4-yl)methyl)piperidin-4-yl)urea (Ex-145)

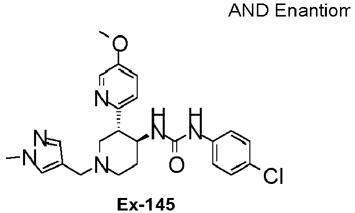

To the solution of *trans*-1-(4-chlorophenyl)-3-(3-(5-methoxypyridin-2-yl)piperidin-4-yl)urea trifluoroacetate **(BB-III-TFA,** 0.12 g, 0.2530 mmol, 1 eq) in ACN (10 ml), K₂CO₃ (0.1745g, 1.265 mmol, 5eq), followed by 4-(bromomethyl)-1-methyl-1H-pyrazole (0.11 g, 0.6327 mmol, 2.5 eq), was added at RT. The reaction was allowed to stir at room temperature for 16 hour. After completion of reaction (checked by TLC, 5% MeOH in DCM, R_{f}: 0.3), the reaction mixture was concentrated to get crude which was purified by column chromatography using 0-10% MeOH in DCM and 230-400 silica gel to get *trans*-1-(4-chlorophenyl)-3-(3-(5-methoxypyridin-2-yl)-1-((1-methyl-1H-pyrazol-4-yl)methyl)piperidin-4-yl)urea(**Ex-145**, 0.01855 g, 16.11%).
LC-MS (Method 3): m/z [M+H]⁺ = 455.4 (exact mass calc. = 454.19).
¹H-NMR (400 MHz, DMSO-d6): δ 8.31 (s, 1H), 8.17 (d, *J=* 2.8 Hz, 1H), 7.53 (s, 1H), 7.26-7.29 (m, 4H), 7.19 (t, *J=* 9.2 Hz, 3H), 5.96 (d, *J=* 8.1 Hz, 1H), 3.80-3.83 (m, 1H), 3.77 (s, 3H), 3.76 (s, 3H), 3.35-3.38 (m, 1H), 3.28-3.32 (m, 1H), 2.80-2.88 (m, 3H), 1.98-2.09 (m, 3H), 1.43-1.49 (m, 1H).

### Synthesis of trans-1-(4-cyanophenyl)-3-[3-(5-methoxypyridin-2-yl)-1-[(1-methyl-1H-pyrazol-4-yl)methyl]piperidin-4-yl]urea (Ex-146)

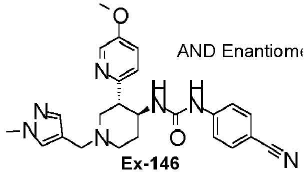

To the solution of *trans*-1-(4-cyanophenyl)-3-(3-(5-methoxypyridin-2-yl)piperidin-4-yl)urea trifluoroacetate **(BB-XI-TFA,** 0.3 g, 0.0.6446 mmol, 1 eq) in ACN (10 ml), K₂CO₃ (0.622 g, 4.5122 mmol, 5 eq), followed by 4-(bromomethyl)-1-methyl-1H-pyrazole (0.338 g, 1.933 mmol, 3.0 eq), was added at RT. The reaction was allowed to stir at room temperature for 16 hour. After completion of reaction (checked by TLC, 5% MeOH in DCM, R_{f}: 0.3), the reaction mixture was concentrated to get crude which was purified by column chromatography using 0-10% MeOH in DCM and 230-400 silica gel to get *trans*-1-(4-cyanophenyl)-3-[3-(5-methoxypyridin-2-yl)-1-[(1-methyl-1H-pyrazol-4-yl)methyl]piperidin-4-yl]urea (**Ex-146,** 0.01513 g, 5.26 %).
LC-MS (Method 3): m/z [M+H]⁺ = 446.4 (exact mass calc. = 445.22).
¹H-NMR (400 MHz, DMSO-d6): δ 8.72 (s, 1H), 8.17 (d, *J* = 2.5 Hz, 1H), 7.59 (d, *J* = 8.7 Hz, 2H), 7.53 (s, 1H), 7.43 (d, *J* = 8.6 Hz, 2H), 7.26-7.29 (m, 2H), 7.21 (d, *J* = 8.6 Hz, 1H), 6.17 (d, *J* = 7.3 Hz, 1H), 3.75-3.90 (m, 7H), 3.35-3.39 (m, 2H), 2.82-2.90 (m, 3H), 1.98-2.07 (m, 3H), 1.46-1.49 (m, 1H).

### Synthesis of trans-1-(4-chloro-phenyl)-3-[5-methoxy-1'-(-methyl-piperidin-4-ylmethyl)-1',2',3',4',5',6'-hexahydro-[2,3']bipyridinyl-4'-yl]-urea (Ex-147)

### Synthesis of trans-4-{4'-[3-(4-chloro-phenyl)-ureido]-5-methoxy-3',4',5',6'-tetrahydro-2'H-[2,3']bipyridinyl-1'-ylmethyl}-piperidine-1-carboxylic acid tert-butyl ester

To a stirred solution of *trans*-1-(4-chloro-phenyl)-3-(5-methoxy-1',2',3',4',5',6'-hexahydro-[2,3']bipyridinyl-4'-yl)-urea hydrochloride (**BB-III-HCl,** 200 mg, 0.504 mmol, 1.0 eq) in dry ACN (10 ml) was added Cs₂CO₃ (656 mg, 2.016 mmol, 4 eq) followed by the addition of 4-bromomethyl-piperidine-1-carboxylic acid tert-butyl ester (210 mg, 0.756 mmol, 1.5 eq) at ice cooled condition and stirred at RT for a period of 16 h. After completion of the reaction it was evaporated under reduced pressure and diluted with ethyl acetate (100 ml) and washed with ice-cold water (50 ml) and brine (25 ml). The organic layer was then dried over anhyd. Na₂SO₄ and concentrated under reduced pressure to obtain crude product which was purified by silica gel (100-200 mesh) column chromatography (3-4% MeOH in DCM as an eluent) to obtain *trans*-4-{4'-[3-(4-chloro-phenyl)-ureido]-5-methoxy-3',4',5',6'-tetrahydro-2'H-[2,3']bipyridinyl-1'-ylmethyl}-piperidine-1-carboxylic acid tert-butyl ester (180 mg, 64%) as brownish gummy.
LC-MS (Method 2): m/z [M+H]⁺ = 558.4 (exact mass calc. = 557.28).

### Synthesis of trans-1-(4-chloro-phenyl)-3-(5-methoxy-1'-piperidin-4-ylmethyl-1',2',3',4',5',6'-hexahydro-[2,3']bipyridinyl-4'-yl)-urea hydrochloride

To a stirred solution of *trans*-4-{4'-[3-(4-chloro-phenyl)-ureido]-5-methoxy-3',4',5',6'-tetrahydro-2'H-[2,3']bipyridinyl-1'-ylmethyl}-piperidine-1-carboxylic acid tert-butyl ester (180 mg, 0.323 mmol, 1.0 eq) in 1,4-dioxane (5 ml) was added 4M HCl in dioxane (2 ml) followed by stirring at RT for a period of 6 h. After completion of the reaction (monitored by TLC and LC-MS), the reaction mixture was concentrated under reduced pressure to get the crude *trans*-1-(4-chloro-phenyl)-3-(5-methoxy-1'-piperidin-4-ylmethyl-1',2',3',4',5',6'-hexahydro-[2,3']bipyridinyl-4'-yl)-urea hydrochloride (150 mg, 94% crude yield).
LC-MS (Method 1): m/z [M+H]⁺ = 458.4 (exact mass calc. = 457.22).

### Synthesis of trans-1-(4-chloro-phenyl)-3-[5-methoxy-1'-(1-methyl-piperidin-4-ylmethyl)-1',2',3',4',5',6'-hexahydro-[2,3']bipyridinyl-4'-yl]-urea (Ex-147)

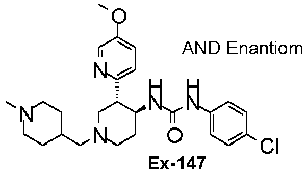

To a stirred solution of *trans*-1-(4-chloro-phenyl)-3-(5-methoxy-1'-piperidin-4-ylmethyl-1',2',3',4',5',6'-hexahydro-[2,3']bipyridinyl-4'-yl)-urea hydrochloride (100 mg, 0.202 mmol, leq) and formaldehyde solution (30% Aqs, 40 µl, 0.405 mmol, 2.0 eq) in DCE (10 ml) at RT was added Et₃N (84 µl, 0.60 mmol, 3.0 eq) and the reaction mixture was stirred at RT for a period of 30 min followed by the addition of NaCNBH₃ (50 mg, 0.808 mmol, 1 eq) at ice cooled condition and the reaction mixture was stirred at RT for a period of 18 h. The reaction mixture was then diluted with DCM and washed with water and brine. The combined organic layer was dried over anhyd. Na₂SO₄ and concentrated under reduced pressure. The obtained crude was purified by by reverse phase purification to obtain *trans*-1-(4-chloro-phenyl)-3-[5-methoxy-1'-(1-methyl-piperidin-4-ylmethyl)-1',2',3',4',5',6'-hexahydro-[2,3']bipyridinyl-4'-yl]-urea (Ex-147, 20 mg, % yield) as off white solid.
LC-MS (Method 7): m/z [M+H]⁺ = 472.2 (exact mass calc. = 471.24).
¹H-NMR (400 MHz, DMSO-d6): δ 8.48 (s, 1 H), 8.18 (s, 1 H), 7.31-7.17 (m, 6 H), 6.11 (br s, 1 H), 3.89-3.79 (m, 1 H), 3.76 (s, 3 H), 2.84-2.81 (m, 3 H), 2.71-2.69 (m, 2 H), 2.14-2.09 (m, 5 H), 1.99-1.97 (m, 2 H), 1.77 (t, *J* = 12 Hz, 2 H), 1.63-1.61 (m, 2 H), 1.47-1.22 (m, 2 H), 1.11-1.02 (m, 2 H).

### Synthesis of trans-1-[1'-(2-methanesulfonyl-ethyl)-5-methoxy-1',2',3',4',5',6'-hexahydro-[2,3']bipyridinyl-4'-yl]-3-(3-methyl-isothiazol-5-yl)-urea (Ex-148)

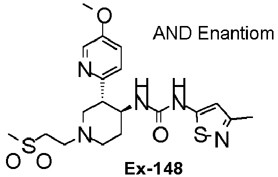

To a stirred solution of *trans-*1-(5-methoxy-1',2',3',4',5',6'-hexahydro-[2,3']bipyridinyl-4'-yl)-3-(3-methyl-isothiazol-5-yl)-urea hydrochloride (**BB-IV-HCl,** 100 mg, 0.26 mmol, 1 eq) in ACN (5 ml) were added Cs₂CO₃ (339 mg, 1.06 mmol, 4 eq) followed by the addition of 1-bromo-2-methanesulfonyl-ethane (49 mg, 0.26 mmol, 1.0 eq) and stirred at RT for 18 h. The reaction mixture was diluted with 10% MeOH-DCM and washed with water and brine. The organic layer was dried over anhyd. Na₂SO₄ and concentrated under reduced pressure to obtain crude product which was purified by reverse phase purification to obtain *trans*-1-[1'-(2-methanesulfonyl-ethyl)-5-methoxy-1',2',3',4',5',6'-hexahydro-[2,3']bipyridinyl-4'-yl]-3-(3-methyl-isothiazol-5-yl)-urea **(Ex-148,** 20 mg, 17% yield) as off white solid.
LC-MS (Method 7): m/z [M+H]⁺ = 454.1 (exact mass calc. = 453.15).
¹H-NMR (400 MHz, DMSO-d6): δ 11.01 (br s, 1 H), 8.18 (s, 1 H), 7.58-7.31 (br m, 1 H), 7.29-7.22 (m, 2 H), 6.34 (s, 1 H), 3.83-3.81 (m, 1 H), 3.76 (s, 3 H), 3.32-3.27 (m, 2 H), 3.02 (s, 3 H), 3.01-2.89 (m, 3 H), 2.74-2.66 (m, 2 H), 2.32-2.26 (m, 1 H), 2.18 (s, 3 H), 2.12-2.09 (m, 1 H), 1.94 (d, *J* = 12 Hz, 1 H), 1.62-1.49 (m, 1 H).

### Synthesis of trans-1-(3-methylisothiazol-5-yl)-3-[1-(1,1-dioxothian-4-yl)-3-(5-methoxy-2-pyridyl)-4-piperidyllurea (Ex-149)

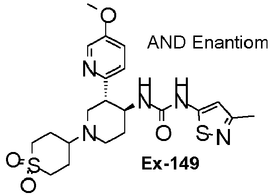

To a stirred solution of *trans*-1-(5-methoxy-1',2',3',4',5',6'-hexahydro-[2,3']bipyridinyl-4'-yl)-3-(3-methyl-isothiazol-5-yl)-urea trifluoroacetate (**BB-IV-HCl,** 100 mg, 0.26 mmol, 1 eq) in MeOH (7 ml) was added 1,1-dioxo-tetrahydro-116-thiopyran-4-one (38 mg, 0.26 mmol, 1 eq) and the reaction mixture was stirred at room temperature for a period of 12 h followed by the addition of sodium cyano borohydride (50 mg, 0.78 mmol, 3.0 eq) and stirred at RT for another 4 h. The reaction mixture was then evaporated under reduced pressure to obtain crude product which was purified by silica gel column chromatography (100-200 mesh) and eluted by 2-3% MeOH in DCM to obtain *trans*-1-(3-methylisothiazol-5-yl)-3-[1-(1,1-dioxothian-4-yl)-3-(5-methoxy-2-pyridyl)-4-piperidyl]urea (**Ex-149,** 15 mg, 12% yield) as off white solid.
LC-MS (Method 7): m/z [M+H]⁺ = 480.2 (exact mass calc. = 479.17).
¹H-NMR (400 MHz, DMSO-d6): δ 10.27 (br s, 1 H), 8.18 (d, *J* = 4 Hz, 1 H), 7.30-7.21 (m, 2 H), 6.83 (br s, 1 H), 6.37 (s, 1 H), 4.55 (br s, 1 H), 3.82-3.77 (m, 1 H), 3.76 (s, 3 H), 3.55-3.49 (m, 2 H), 3.42-3.38 (m, 2 H), 3.18-3.02 (m, 1 H), 2.98-8.03 (m, 2 H), 2.68 (d, *J* = 8 Hz, 1 H), 2.42-2.28 (m, 2 H), 2.20 (s, 3 H), 2.08-1.97 (m, 4 H), 1.54-1.49 (m, 1 H).

### Synthesis of trans-1-[1'-(2,2-difluoro-ethyl)-5-methoxy-1',2',3',4',5',6'-hexahydro-[2,3']bipyridinyl-4'-yl]-3-phenyl-urea (Ex-150)

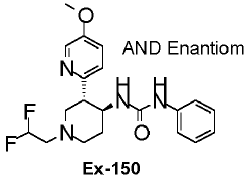

To a stirred solution of *trans*-1-(5-methoxy-1',2',3',4',5',6'-hexahydro-[2,3']bipyridinyl-4'-yl)-3-phenyl-urea hydrochloride (**BB-X-HCl,** 100 mg, 0.27 mmol, 1 eq) in ACN (5 ml) were added Cs₂CO₃ (268 mg, 0.82 mmol, 3 eq) followed by the addition of2,2-difluoroethyl methanesulfonate (70 mg, 0.33 mmol, 1.2 eq) and stirred at RT for 16 h. The reaction mixture was diluted with 10% MeOH-DCM and washed with water and brine. The organic layer was dried over anhyd. Na₂SO₄ and concentrated under reduced pressure to obtain crude product which was purified by reverse phase purification to obtain *trans*-1-[1'-(2,2-difluoro-ethyl)-5-methoxy-1',2',3',4',5',6'-hexahydro-[2,3']bipyridinyl-4'-yl]-3-phenyl-urea (**Ex-150,** 32 mg, 30% yield) as off white solid.
LC-MS (Method 7): m/z [M+H]⁺ = 391.2 (exact mass calc. = 390.19).
¹H-NMR (400 MHz, DMSO-d6): δ 8.20-8.17 (m, 2 H), 7.32-7.24 (m, 4 H), 7.16-7.12 (m, 2 H), 6.83 (t, *J* = 4 Hz, 1 H), 5.92 (d, *J* = 8 Hz, 1 H), 3.84-3.81 (m, 1 H), 3.77 (s, 3 H), 2.95-2.88 (m, 3 H), 2.87-2.80 (m, 2 H), 2.49-2.31 (m, 3 H), 1.99 (d, *J* = 8 Hz, 1 H), 1.50-1.47 (m, 1 H).

### Synthesis of trans-1-[5-methoxy-1'-(piperidine-4-carbonyl)-1',2',3',4',5',6'-hexahydro-[2,3']bipyridinyl-4'-yl]-3-phenyl-urea (Ex-151)

### Synthesis of trans-4-[5-methoxy-4'-(3-phenyl-ureido)-3',4',5',6'-tetrahydro-2'H-[2,3']bipyridinyl-1'-carbonyl]-piperidine-1-carboxylic acid tert-butyl ester

To a stirred solution of piperidine-1,4-dicarboxylic acid mono-tert-butyl ester (75.5 mg, 0.33 mmol, 1.2 eq) in dry THF (10 ml) were added EDCI-HCl (78 mg, 0.41 mmol, 1.5 eq) and HOBt (55 mg, 0.41 mmol, 1.5 eq) and TEA (115 µl, 0.825 mmol, 3.0 eq) at RT followed by the addition of *trans*-1-(5-methoxy-1',2',3',4',5',6'-hexahydro-[2,3']bipyridinyl-4'-yl)-3-phenyl-urea hydrochloride (**BB-X-HCl,** 100 mg, 0.275 mmol, 1 eq) was added and continued stirring for 18 h at RT. The reaction mixture was diluted with 10% MeOH-DCM and washed with water and brine. The organic layer was dried over anhyd. Na₂SO₄ and concentrated under reduced pressure to obtain crude *trans*-4-[5-methoxy-4'-(3-phenyl-ureido)-3',4',5',6'-tetrahydro-2'H-[2,3']bipyridinyl-1'-carbonyl]-piperidine-1-carboxylic acid tert-butyl ester (140 mg, 95% crude yield) as yellow sticky.
LC-MS (Method 2): m/z [M+H]⁺ = 538.4 (exact mass calc. = 537.30).

### Synthesis of trans-1-[5-methoxy-1'-(piperidine-4-carbonyl)-1',2',3',4',5',6'-hexahydro-[2,3']bipyridinyl-4'-yl]-3-phenyl-urea (Ex-151)

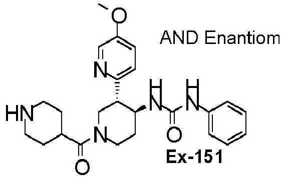

To a stirred solution of *trans*-4-[5-methoxy-4'-(3-phenyl-ureido)-3',4',5',6'-tetrahydro-2'H-[2,3']bipyridinyl-1'-carbonyl]-piperidine-1-carboxylic acid tert-butyl ester (150 mg, 0.279 mmol, 1 eq) in 1,4-dioxane (2 ml) was added 4M HCl in dioxane (2 ml) followed by stirring at RT for a period of 4 h. the reaction mixture was concentrated under reduced pressure to obtain crude product which was purified by reverse phase purification to obtain *trans-*1-[5-methoxy-1'-(piperidine-4-carbonyl)-1',2',3',4',5',6'-hexahydro-[2,3']bipyridinyl-4'-yl]-3-phenyl-urea (**Ex-151,** 47 mg, 38% yield).
LC-MS (Method 7): m/z [M+H]⁺ = 438.4 (exact mass calc. = 437.24).
¹H-NMR (400 MHz, DMSO-d6): δ 8.42 (s, 1 H), 8.23-8.20 (m, 1 H), 7.43-7.28 (m, 4 H), 7.18-7.09 (m, 2 H), 6.85-6.77 (m, 1 H), 6.21-6.19 (m, 1 H), 4.55-4.38 (m, 1 H), 4.13-3.95 (m, 2 H), 3.76 (s, 3 H), 3.29-3.18 (m, 2 H), 3.01-2.85 (m, 2 H), 2.76-2.58 (m, 3 H), 2.58-2.49 (m, 1 H), 2.09-2.00 (m, 1 H), 1.53-1.23 (m, 6 H).

### Synthesis of trans-1-[5-chloro-1'-(2-hydroxy-ethyl)-1',2',3',4',5',6'-hexahydro-[2,3']bipyridinyl-4'-yl]-3-phenyl-urea (Ex-152)

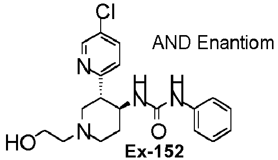

To a stirred solution of *trans*-1-(5-chloro-1',2',3',4',5',6'-hexahydro-[2,3']bipyridinyl-4'-yl)-3-phenyl-urea hydrochloride (**BB-XII-HCl,** 100 mg, 0.303 mmol, 1 eq) in ACN (5 ml) were added Cs₂CO₃ (394 mg, 1.21 mmol, 4 eq) followed by the addition of 2-bromo-ethanol (60 mg, 0.454 mmol, 1.5 eq) and stirred at RT for 16 h. The reaction mixture was diluted with 10% MeOH-DCM and washed with water and brine. The organic layer was dried over anhyd. Na₂SO₄ and concentrated under reduced pressure to obtain crude product which was purified by reverse phase phase prep-HPLC purification to obtain *trans*-1-[5-chloro-1'-(2-hydroxy-ethyl)-1',2',3',4',5',6'-hexahydro-[2,3']bipyridinyl-4'-yl]-3-phenyl-urea (**Ex-152,** 24 mg, 21% yield) as off white solid.
LC-MS (Method 7): m/z [M+H]⁺ = 375.2 (exact mass calc. = 374.15).
¹H-NMR (400 MHz, DMSO-d6): δ 8.52 (s, 1 H), 8.29 (s, 1 H), 7.83 (d, *J* = 8 Hz, 1 H), 7.37 (d, *J* = 8 Hz, 1 H), 7.26-7.24 (m, 2 H), 7.14 (t, *J* = 8 Hz, 2 H), 6.82 (t, *J* = 8 Hz, 1 H), 6.14 (d, *J* = 8 Hz, 1 H), 4.38-4.37 (m, 1 H), 3.86-3.84 (m, 1 H), 3.48-3.47 (m, 2 H), 2.96-2.91 (m, 3 H), 2.43-2.40 (m, 2 H), 2.25 (t, *J* = 8 Hz, 1 H), 2.13 (t, *J* = 8 Hz, 1 H), 1.96 (d, *J* = 4 Hz, 1 H), 1.59-1.48 (m, 1 H).

### Synthesis of trans-1-[5-chloro-1'-(2,2-difluoro-ethyl)-1',2',3',4',5',6'-hexahydro-[2,3']bipyridinyl-4'-yl]-3-phenyl-urea (Ex-153)

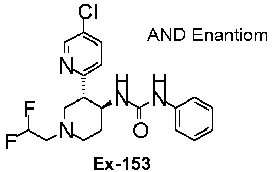

To a stirred solution of *trans-*1-(5-chloro-1',2',3',4',5',6'-hexahydro-[2,3']bipyridinyl-4'-yl)-3-phenyl-urea **(BB-XII_HCl,** 100 mg, 0.272 mmol, 1 eq) in ACN (10 ml) were added Cs₂CO₃ (354 mg, 1.08 mmol, 4 eq) followed by the addition of methanesulfonic acid 2,2-difluoro-ethyl ester (77 mg, 0.327 mmol, 1.2 eq) and stirred at RT for 16 h. The reaction mixture was diluted with 10% MeOH-DCM and washed with water and brine. The organic layer was dried over anhyd. Na₂SO₄ and concentrated under reduced pressure to obtain crude product which was purified by reverse phase phase prep-HPLC purification to obtain *trans*-1-[5-chloro-1'-(2,2-difluoro-ethyl)-1',2',3',4',5',6'-hexahydro-[2,3']bipyridinyl-4'-yl]-3-phenyl-urea (**Ex-153,** 22 mg, 20% yield) as off white solid.
LC-MS (Method 7): m/z [M+H]⁺ = 395.2 (exact mass calc. = 394.14).
¹H-NMR (400 MHz, DMSO-d6): δ 8.54 (s, 1 H), 8.25 (s, 1 H), 7.84 (d, *J* = 8 Hz, 1 H), 7.38 (d, *J* = 8 Hz, 1 H), 7.25-6.81 (m, 4 H), 6.83 (t, *J* = 4 Hz, 1 H), 6.13-6.07 (m, 2 H), 3.86-3.84 (m, 1 H), 2.97-2.95 (m, 3 H), 2.82-2.74 (m, 2 H), 2.39-2.33 (m, 2 H), 1.96 (d, *J* = 12 Hz, 1 H), 1.53-1.51 (m, 1 H).

### Synthesis of trans-1-(4-cyanophenyl)-3-[1-(2-methoxyethyl)-3-(5-methoxypyridin-2-yl)piperidin-4-yl]urea (Ex-154)

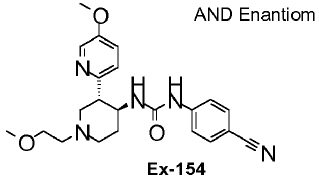

To the stirring solution of compound *trans*-1-(4-cyanophenyl)-3-[-3-(5-methoxypyridin-2-yl)-1-piperidin-4-yl]urea trifluoroacetate (**BB-XII-TFA**, 0.10 g, 0.214 mmol, 1.0 eq) in acetonitrile (10 ml), K₂CO₃ (0.148 g, 1.074 mmol, 5.0 eq) was added at 0 °C and allowed to stir for 10 minutes, then compound 1-bromo-2-methoxyethane (0.036 g, 0.2578 mmol, 1.2 eq) was added and again allowed to stir for 24 hours. After completion of reaction water was added, extracted with ethyl acetate (3x100 ml). Ethyl acetate part was collected, dried over Na₂SO₄ and evaporated to dryness to get crude, which was purified by column chromatography to afford *trans*-1-(4-cyanophenyl)-3-[1-(2-methoxyethyl)-3-(5-methoxypyridin-2-yl)piperidin-4-yl]urea (**Ex-154,** 0.01181 g, 13.47%).
LC-MS (Method 3): m/z [M+H]⁺ = 410.2 (exact mass calc. = 409.21).
¹H-NMR (400 MHz, DMSO-d6): δ 8.72 (s, 1H), 8.19 (d, *J* = 2.7 Hz, 1H), 7.59 (d, *J* = 8.7 Hz, 2H), 7.43 (d, *J* = 8.7 Hz, 2H), 7.28-7.31 (m, 1H), 7.23 (d, *J* = 8.5 Hz, 1H), 6.17 (d, *J=* 8.1 Hz, 1H), 3.82-3.85 (m, 1H), 3.76 (s, 3H), 3.41 (t, *J* = 5.7 Hz, 2H), 3.21 (s, 3H), 2.84-2.90 (m, 3H), 2.49-2.50 (m, 2H), 2.10-2.23 (m, 2H), 1.98 (d, *J* = 9.2 Hz, 1H), 1.44-1.54 (m, 1H).

### Synthesis of trans-3-[3-(5-methoxypyridin-2-yl)-1-[(1-methylpiperidin-4-yl)carbonyl]piperidin-4-yl]-1-phenylurea (Ex-156)

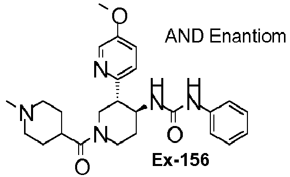

To a stirred solution 1-methylpiperidine-4-carboxylic acid (0.0487 g, 0.3409 mmol, 1.5 eq) in DMF (10 ml) was added EDCI-HCl (0.065 g, 0.3409 mmol, 1.5 eq), HOBt (0.0368 g, 0.2726 mmol, 1.2 eq), DIPEA (0.18 ml, 1.136 mmol, 5.0 eq) and stirred at RT for 30 min followed by addition of a *trans*-3-[3-(5-methoxypyridin-2-yl)piperidin-4-yl]-1-phenylurea trifluoroacetate (**BB-X-TFA,** 0.100 g, 0.2272 mmol, 1.0 eq). The reaction mixture was stirred for 16 h at RT. After completion of the reaction (monitored by TLC, TLC system 5% MeOH-DCM, R_{f}: 0.4) reaction mixture was poured into cold water and extracted with EtOAc (2 x 25 ml). The organic layer was washed with water (2 x 25 ml) and brine (2 x 25 mL), dried over anhyd. Na₂SO₄, concentrated in vacuo. The crude material thus obtained was purified by flash chromatography (230-400 mesh silica gel; 5% MeOH-DCM as an elutent) to *trans*-3-[3-(5-methoxypyridin-2-yl)-1-[(1-methylpiperidin-4-yl)carbonyl]piperidin-4-yl]-1-phenylurea (**Ex-156,** 0.01798g,17.52%).
LC-MS (Method 3): m/z [M+H]⁺ = 452.5(exact mass calc. = 451.26).
¹H-NMR (400 MHz, DMSO-d6): δ 8.22 (s, 2H), 7.25-7.33 (m, 4H), 7.15 (t, *J*= 7.7 Hz, 2H), 6.83 (t, *J*= 7.1 Hz, 1H), 5.97-5.99 (m, 1H), 4.40-4.50 (m, 1H), 3.99-4.04 (m, 2H), 3.78 (s, 3H), 3.16-3.22 (m, 1H), 2.67-2.78 (m, 4H), 1.90-2.15 (m, 7H), 1.33-1.61 (m, 5H).

### Synthesis of trans-3-1-[2-(dimethylamino)acetyl-3-(5-methoxypyridin-2-yl)piperidin-4-yl]-1-phenylurea (Ex-157)

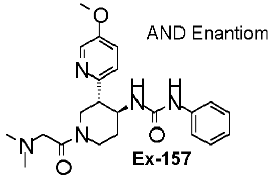

To a stirred solution 2-(dimethylamino)acetic acid (0.035 g, 0.3409 mmol, 1.5 eq) in DMF (10 ml) was added EDCI-HCl (0.065 g, 0.3409 mmol, 1.5 eq), HOBt (0.0368 g, 0.2726 mmol, 1.2 eq), DIPEA (0.18 ml, 1.136 mmol, 5.0 eq) and stirred at RT for 30 min followed by addition of a *trans*-3-[3-(5-methoxypyridin-2-yl)piperidin-4-yl]-1-phenylurea trifluoroacetate (**BB-X-TFA,** 0.100 g, 0.2272 mmol, 1.0 eq). The reaction mixture was stirred for 16 hr at RT. After completion of the reaction (monitored by TLC, TLC system 5% MeOH-DCM, R_{f}: 0.4) reaction mixture was poured into cold water and extracted with EtOAc (2 x 25 ml). The organic layer was washed with water (2 x 25 ml) and brine (2 x 25 mL), dried over anhyd. Na₂SO₄, concentrated in vacuo. The crude material thus obtained was purified by flash chromatography (230-400 mesh silica gel; 5% MeOH-DCM as an elutent) to get *trans*-3-1-[2-(dimethylamino)acetyl-3-(5-methoxypyridin-2-yl)piperidin-4-yl]-1-phenylurea (**Ex-157,** 0.017g,18.18%).
LC-MS (Method 3): m/z [M+H]⁺ = 412.5 (exact mass calc. = 411.23).
¹H-NMR (400 MHz, DMSO-d6): δ 8.16-8.22 (m, 2H), 7.24-7.32 (m, 4H), 7.15 (t, *J=* 8.0 Hz, 2H), 6.83 (t, *J=* 7.0 Hz, 1H), 5.99 (d, *J=* 8.4 Hz, 1H), 4.38-4.41 (m, 1H), 4.04-4.12 (m, 2H), 3.78 (s, 3H), 3.15-3.25 (m, 2H), 3.02 (t, *J=* 13.0 Hz, 1H), 2.67-2.86 (m, 2H), 2.17-2.20 (m, 6H), 2.03-2.06 (m, 1H), 1.23-1.45 (m, 2H).

### Synthesis of trans-3-1-(3-methoxypropyl)-3-(5-methoxypyridin-2-yl)piperidin-4-yl-1-phenylurea (Ex-158)

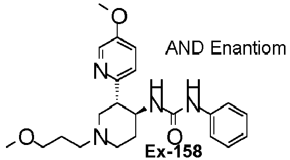

To the solution of *trans*-3-[3-(5-methoxypyridin-2-yl)piperidin-4-yl]-1-phenylurea trifluoroacetate **(BB-X-TFA,** 0.1 g, 0.2272 mmol, 1 eq) in ACN (10 ml), K₂CO₃ (0.156 g, 1.136 mmol, 5 eq), followed by 1-bromo-3-methoxypropane (0.0417 g, 0.2727 mmol, 1.2 eq), was added at RT. The reaction was allowed to stir at room temperature for 16 hour. After completion of reaction (checked by TLC, 5% MeOH in DCM, R_{f}: 0.3), the reaction mixture was concentrated to get crude which was purified by column chromatography using 0-10% MeOH in DCM and 230-400 silica gel to get *trans*-3-1-(3-methoxypropyl)-3-(5-methoxypyridin-2-yl)piperidin-4-yl-1-phenylurea (**Ex-158,** 0.01848 g, 20.41 %).
LC-MS (Method 7): m/z [M+H]⁺ = 399.2 (exact mass calc. = 398.23).
¹H-NMR (400 MHz, CD₃OD): δ 8.16 (s, 1H), 7.30-7.35 (m, 2H), 7.17-7.18 (m, 4H), 6.89-6.94 (m, 1H), 3.98-4.04 (m, 1H), 3.81 (s, 3H), 3.44 (t, *J*= 6.0 Hz, 2H), 3.29-3.31 (m, 3H), 3.17 (d, *J*= 11.2 Hz, 2H), 3.0-3.10 (m, 1H), 2.55-2.68 (m, 4H), 2.15-2.18 (m, 1H), 1.70-1.84 (m, 3H).

### Synthesis of trans-3-1-(3-hydroxypropyl)-3-(5-methoxypyridin-2-yl)piperidin-4-yl-1-phenylurea (Ex-159)

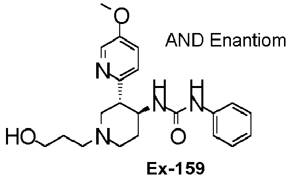

To the solution of *trans*-3-[3-(5-methoxypyridin-2-yl)piperidin-4-yl]-1-phenylurea trifluoroacetate (**BB-X-TFA,** 0.1 g, 0.2272 mmol, 1 eq) in ACN (10 ml), K₂CO₃ (0.156 g, 1.136 mmol, 5 eq), followed by 3-bromopropan-1-ol (0.038 g, 0.2727 mmol, 1.2 eq), was added at RT. The reaction was allowed to stir at room temperature for 16 hour. After completion of reaction (checked by TLC, 5% MeOH in DCM, R_{f}: 0.3), the reaction mixture was concentrated to get crude which was purified by column chromatography using 0-10% MeOH in DCM and 230-400 silica gel to get *trans*-3-1-(3-hydroxypropyl)-3-(5-methoxypyridin-2-yl)piperidin-4-yl-1-phenylurea (**Ex-159,** 0.02015 g, 23.06 %).
LC-MS (Method 7): m/z [M+H]⁺ = 385.2 (exact mass calc. = 384.22).
¹H-NMR (400 MHz, DMSO-d6 at 100 °C): δ 8.20 (s, 1H), 7.94-7.96 (m, 1H), 7.13-7.26 (m, 6H), 6.85-6.90 (m, 1H), 5.84-5.90 (m, 1H), 3.88-3.90 (m, 1H), 3.80 (s, 3H), 3.31-3.48 (m, 3H), 3.21-3.24 (m, 1H), 2.93-3.02 (m, 2H), 2.36-2.49 (m, 1H), 1.77-2.06 (m, 2H), 1.51-1.67 (m, 5H).

### Synthesis of trans-3-1-(2-methanesulfonylethyl)-3-(5-methoxypyridin-2-yl)piperidin-4-yl-1-phenylurea (Ex-160)

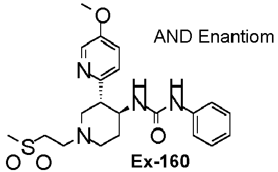

To the solution of *trans*-3-[3-(5-methoxypyridin-2-yl)piperidin-4-yl]-1-phenylurea trifluoroacetate (**BB-X-TFA,** 0.1 g, 0.2272 mmol, 1 eq) in ACN (10 ml), K₂CO₃ (0.156 g, 1.136 mmol, 5 eq), followed by 1-bromo-2-methanesulfonylethane (0.051 g, 0.2727 mmol, 1.2 eq), was added at RT. The reaction was allowed to stir at room temperature for 16 h. After completion of reaction (checked by TLC, 5% MeOH in DCM, R_{f}: 0.3), the reaction mixture was concentrated to get crude which was purified by column chromatography using 0-10% MeOH in DCM and 230-400 silica gel to get *trans*-3-1-(2-methanesulfonylethyl)-3-(5-methoxypyridin-2-yl)piperidin-4-yl-1-phenylurea (**Ex-160,** 0.03677 g, 37.41 %).
LC-MS (Method 3): m/z [M+H]⁺ = 433.4 (exact mass calc. = 432.18).
¹H-NMR (400 MHz, DMSO-d6): δ 8.18 (d, *J* = 12.9 Hz, 2H), 7.24-7.32 (m, 4H), 7.15 (t, *J* = 7.5 Hz, 2H), 6.83 (t, *J=* 6.9 Hz, 1H), 5.94 (d, *J* = 7.9 Hz, 1H), 3.77-3.82 (m, 4H), 3.20-3.29 (m, 2H), 2.72-3.02 (m, 8H), 2.0-2.27 (m, 3H), 1.45-1.48 (m, 1H).

### Synthesis of trans-3-3-(5-methoxypyridin-2-yl)-1-(oxetan-3-ylmethyl)piperidin-4-yl-1-phenylurea (Ex-161)

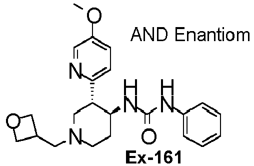

To the solution of *trans*-3-[3-(5-methoxypyridin-2-yl)piperidin-4-yl]-1-phenylurea trifluoroacetate (**BB-X-TFA**, 0.1 g, 0.2272 mmol, 1 eq) in ACN (10 ml), K₂CO₃ (0.156 g, 1.136 mmol, 5 eq), followed by 3-(iodomethyl)oxetane (0.0494 g, 0.2499 mmol, 1.1 eq), was added at RT. The reaction was allowed to stir at room temperature for 16 h. After completion of reaction (checked by TLC, 5% MeOH in DCM, R_{f}: 0.3), the reaction mixture was concentrated to get crude which was purified by column chromatography using 0-10% MeOH in DCM and 230-400 silica gel to get *trans*-3-3-(5-methoxypyridin-2-yl)-1-(oxetan-3-ylmethyl)piperidin-4-yl-1-phenylurea (**Ex-161,** 0.03372 g, 37.43 %).
LC-MS (Method 7): m/z [M+H]⁺ = 397.2 (exact mass calc. = 396.22.
¹H-NMR (400 MHz, DMSO-d6): δ 8.17-8.19 (m, 2H), 7.22-7.31 (m, 4H), 7.14 (t, *J* = 7.7 Hz, 2H), 6.82 (t, *J* = 7.2 Hz, 1H), 5.90 (d, *J* = 7.8 Hz, 1H), 4.58-4.64 (m, 2H), 4.21-4.25 (m, 2H), 3.76-3.90 (m, 4H), 3.14-3.19 (m, 1H), 2.79-2.82 (m, 3H), 2.62-2.64 (m, 2H), 1.96-2.17 (m, 3H), 1.42-1.45 (m, 1H).

### Synthesis of trans-3-3-(5-methoxypyridin-2-yl)-1-(oxetan-3-yl)piperidin-4-yl-1-phenylurea (Ex-162)

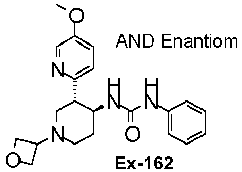

To the solution of *trans*-3-[3-(5-methoxypyridin-2-yl)piperidin-4-yl]-1-phenylurea trifluoroacetate (**BB-X-TFA,** 0.1 g, 0.2272 mmol, 1 eq) in MeOH (10 ml), NaBH₃CN (0.021 g, 0.3109 mmol, 1.5 eq), followed by oxetan-3-one (0.02456 g, 0.3409 mmol, 1.5 eq), was added at RT. The reaction was allowed to stir at room temperature for 16 hour. After completion of reaction (checked by TLC, 5% MeOH in DCM, R_{f}: 0.3), the reaction mixture was concentrated, diluted with water, extracted with 10% MeOH/DCM, dried over Na₂SO₄ and concentred to get crude which was purified by column chromatography using 0-10% MeOH in DCM and 230-400 silica gel to get *trans-*3-3-(5-methoxypyridin-2-yl)-1-(oxetan-3-yl)piperidin-4-yl-1-phenylurea (**Ex-162,** 0.03183 g, 36.63 %).
LC-MS (Method 7): m/z [M+H]⁺ = 383.2 (exact mass calc. = 382.20).
¹H-NMR (400 MHz, DMSO-d6): δ 8.17-8.19 (m, 2H), 7.24-7.31 (m, 4H), 7.15 (t, *J*= 7.8 Hz, 2H), 6.83 (t, *J*= 7.2 Hz, 1H), 5.96 (d, *J*= 8.2 Hz, 1H), 4.38-4.54 (m, 4H), 3.80-3.85 (m, 1H), 3.76 (s, 3H), 3.40-3.43 (m, 1H), 2.70-2.91 (m, 3H), 2.01-2.06 (m, 2H), 1.93 (t, *J*= 11.1 Hz, 1H), 1.47-1.51 (m, 1H).

### Synthesis of trans-1-(4-chloro-phenyl)-3-[1'-(1-hydroxy-cyclopropylmethyl)-5-methoxy-1',2',3',4',5',6'-hexahydro-[2,3']bipyridinyl-4'-yl]-urea (Ex-163)

### Synthesis of trans-1-(4-chloro-phenyl)-3-[1'-(1-hydroxy-cyclopropanecarbonyl)-5-methoxy-1',2',3',4',5',6'-hexahydro-[2,3']bipyridinyl-4'-yl]-urea

To a stirred solution of 1-hydroxy-cyclopropanecarboxylic acid (26 mg, 0.25 mmol, 1.0 eq) in dry THF (6 ml) were added EDCI-HCl (72 mg, 0.375 mmol, 1.5 eq) and HOBt (51 mg, 0.375 mmol, 1.5 eq) in ice-cold condition followed by addition of TEA (106 µl, 0.75 mmol, 3 eq) and *trans*-1-(4-chloro-phenyl)-3-(5-methoxy-1',2',3',4',5',6'-hexahydro-[2,3']bipyridinyl-4'-yl)-urea hydrochloride (**BB-III-HCl,** 100 mg, 0.25 mmol, 1.0 eq) and stirred at RT for 18 h. The reaction mixture was diluted with EtOAc and washed with water and brine. The organic layer was dried over anhyd. Na2SO4 and concentrated under reduced pressure to obtain *trans*-1-(4-chlorophenyl)-3-[1'-(1-hydroxy-cyclopropanecarbonyl)-5-methoxy-1',2',3',4',5',6'-hexahydro-[2,3']bipyridinyl-4'-yl]-urea (100 mg, 90% crude yield) as reddish sticky.
LC-MS (Method 2): m/z [M+H]⁺ = 445.2 (exact mass calc. = 444.16).

### Synthesis of trans-1-(4-chloro-phenyl)-3-[1'-(1-hydroxy-cyclopropylmethyl)-5-methoxy-1',2',3',4',5',6'-hexahydro-[2,3']bipyridinyl-4'-yl]-urea (Ex-163)

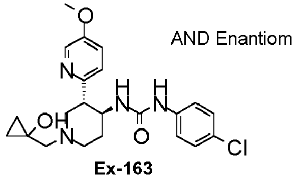

To a stirred solution of *trans*-1-(4-chloro-phenyl)-3-[1'-(1-hydroxy-cyclopropanecarbonyl)-5-methoxy-1',2',3',4',5',6'-hexahydro-[2,3']bipyridinyl-4'-yl]-urea (100 mg, 0.225 mmol, 1.0 eq) in dry THF (5 ml) at ice-cold condition was added BHₐ-DMS (1 M in THF, 1 ml) and stirred at RT for 18 h. After that the reaction mixture was quenched with MeOH (1 ml). The reaction mixture was diluted with EtOAc and washed with water and brine. The organic layer was dried over anhyd. Na₂SO₄ and concentrated under reduced pressure to obtain crude product which was purified by reverse phase prep-HPLC purification to obtain *trans*-1-(4-chloro-phenyl)-3-[1'-(1-hydroxy-cyclopropylmethyl)-5-methoxy-1',2',3',4',5',6'-hexahydro-[2,3']bipyridinyl-4'-yl]-urea (**Ex-163,** 24 mg, 25% yield) as off white solid.
LC-MS (Method 7): m/z [M+H]⁺ = 431.2 (exact mass calc. = 430.18).
¹H-NMR (400 MHz, DMSO-d6): δ 8.35 (s, 1 H), 8.19 (s, 1 H), 7.30-7.17 (m, 6 H), 6.01 (d, *J=* 8 Hz, 1 H), 4.88 (s, 1 H), 3.84-3.81 (m, 1 H), 3.77 (s, 3 H), 3.06-3.04 (m, 2 H), 2.91-2.87 (m, 1 H), 2.41 (s, 2H), 2.27-2.14 (m, 2 H), 2.00-1.97 (m, 1 H), 1.53-1.49 (m, 1 H), 0.54-0.52 (m, 2 H), 0.38-0.29 (m, 2 H).

### Synthesis of trans-1-[1'-(2-hydroxy-ethyl)-5-methoxy-1,2',3',4',5',6'-hexahydro-[2,3']bipyridinyl-4'-yl]-3-phenyl-urea (Ex-164)

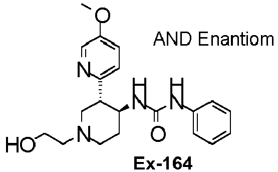

To a stirred solution of 1-(5-methoxy-1',2',3',4',5',6'-hexahydro-[2,3']bipyridinyl-4'-yl)-3-phenyl-urea hydrochloride (**BB-X-HCl,** 100 mg, 0.27 mmol, 1 eq) in ACN (5 ml) were added Cs₂CO₃ (268 mg, 0.82 mmol, 3 eq) followed by the addition of 2-bromo-ethanol (41 mg, 0.33 mmol, 1.2 eq) and stirred at RT for 16 h. The reaction mixture was diluted with 10% MeOH-DCM and washed with water and brine. The organic layer was dried over anhyd. Na₂SO₄ and concentrated under reduced pressure to obtain crude product which was purified by reverse phase prep-HPLC purification to obtain *trans*-1-[1'-(2-hydroxy-ethyl)-5-methoxy-1',2',3',4',5',6'-hexahydro-[2,3']bipyridinyl-4'-yl]-3-phenyl-urea (**Ex-164**, 18 mg, 18% yield) as an off white solid.
LC-MS (Method 7): m/z [M+H]⁺ = 371.3 (exact mass calc. = 370.20).
¹H-NMR (400 MHz, DMSO-d6): δ 8.24 (s, 1 H), 8.18 (d, *J* = 4 Hz, 1 H), 7.31-7.23 (m, 4 H), 7.16-7.12 (m, 2 H), 6.84-6.80 (m, 1 H), 6.01 (d, *J* = 8 Hz, 1 H), 4.37-4.35 (m, 1 H), 3.83-3.80 (m, 1 H), 3.76 (s, 3 H), 3.48-3.45 (m, 2 H), 2.92-2.81 (m, 3 H), 2.43-2.38 (m, 2 H), 2.22-2.08 (m, 2 H), 1.99-1.97 (m, 1 H), 1.48-1.44 (m, 1 H).

### Synthesis of trans-rac 1-[1'-(azetidine-3-carbonyl)-5-methoxy-1',2',3',4',5',6'-hexahydro-[2,3']bipyridinyl 4'-yl]-3-phenyl-urea (Ex-165)

### Synthesis of trans-3-[5-methoxy-4'-(3-phenyl-ureido)-3',4',5',6'-tetrahydro-2'H-[2,3']bipyridinyl-1'-carbonyl]-azetidine-1-carboxylic acid tert-butyl ester

To a stirred solution of azetidine-1,3-dicarboxylic acid mono-tert-butyl ester (66 mg, 0.33 mmol, 1.2 eq) in dry THF (10 ml) were added EDCI-HCl (79 mg, 0.41 mmol, 1.5 eq) and HOBt (55 mg, 0.41 mmol, 1.5 eq) and TEA (115 µl, 0.825 mmol, 3.0 eq) at RT followed by the addition of HCl salt of *trans*-1-(5-methoxy-1',2',3',4',5',6'-hexahydro-[2,3']bipyridinyl-4'-yl)-3-phenyl-urea hydrochloride (**BB-X-HCl,** 100 mg, 0.275 mmol, 1 eq) was added and continued stirring for 18 h at RT. The reaction mixture was diluted with 10% MeOH-DCM and washed with water and brine. The organic layer was dried over anhyd. Na₂SO₄ and concentrated under reduced pressure to obtain crude *trans*-3-[5-methoxy-4'-(3-phenyl-ureido)-3',4',5',6'-tetrahydro-2'H-[2,3']bipyridinyl-1'-carbonyl]-azetidine-1-carboxylic acid tert-butyl ester (130 mg crude, 93% crude yield) as yellow sticky.
LC-MS (Method 2): m/z [M+H]⁺ = 510.4 (exact mass calc. = 509.26).

### Synthesis of trans-1-[1'-(azetidine-3-carbonyl)-5-methoxy-1',2',3',4',5',6'-hexahydro-[2,3']bipyridinyl-4'-yl]-3-phenyl-urea (Ex-165)

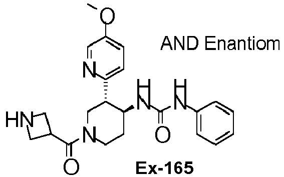

To a stirred solution of crude *trans*-3-[5-methoxy-4'-(3-phenyl-ureido)-3',4',5',6'-tetrahydro-2'H-[2,3']bipyridinyl-1'-carbonyl]-azetidine-1-carboxylic acid tert-butyl ester (150 mg, 0.311 mmol, 1 eq) in 1,4-dioxane (2 ml) was added 4M HCl in dioxane (2 ml) followed by stirring at RT for a period of 4 h. the reaction mixture was concentrated under reduced pressure to obtain crude product which was purified by reverse phase purification to obtain *trans*-1-[1'-(azetidine-3-carbonyl)-5-methoxy-1',2',3',4',5',6'-hexahydro-[2,3']bipyridinyl-4'-yl]-3-phenyl-urea (**Ex-165**, 24 mg, 19% yield) as an off white solid.
LC-MS (Method 3): m/z [M+H]⁺ = 410.2 (exact mass calc. = 409.21).
¹H-NMR (400 MHz, DMSO-d6): δ 8.22 (s, 2 H), 7.34-7.32 (m, 1 H), 7.27-7.24 (m, 3 H), 7.17-7.13 (m, 2 H), 6.85-6.81 (m, 1 H), 6.00 (br s, 1 H), 4.43-4.41 (m, 1 H), 4.19-4.03 (m, 1 H), 3.78 (s, 3 H), 3.73-3.66 (m, 3 H), 3.58-3.48 (m, 3 H), 3.43-3.41 (m, 1 H), 3.16-3.08 (m, 2 H), 2.80-2.66 (m, 2 H), 2.03 (d, *J* = 12 Hz, 1 H), 1.39-1.28 (m, 1H).

### Synthesis of trans-1-[5-chloro-1'-(piperidine-4-carbonyl)-1',2',3',4',5',6'-hexahydro-[2,3']bipyridinyl-4'-yl]-3-phenyl-urea (Ex-167)

### Synthesis of trans-4-[5-chloro-4'-(3-phenyl-ureido)-3',4',5',6'-tetrahydro-2'H-[2,3']bipyridinyl-1'-carbonyl]-piperidine-1-carboxylic acid tert-butyl ester

To a stirred solution of piperidine-1,4-dicarboxylic acid mono-tert-butyl ester (60 mg, 0.26 mmol, 1.0 eq) in dry THF (10 ml) were added EDCI-HCl (74 mg, 0.39 mmol, 1.5 eq) and HOBt (53 mg, 0.39 mmol, 1.5 eq) and TEA (108 µl, 0.78 mmol, 3.0 eq) at RT followed by the addition of *trans*-1-(5-chloro-1',2',3',4',5',6'-hexahydro-[2,3']bipyridinyl-4'-yl)-3-phenyl-urea hydrochloride (**BB-XII-HCl,** 95 mg, 0.26 mmol, 1 eq) was added and continued stirring for 18 h at RT. The reaction mixture was diluted with 10% MeOH-DCM and washed with water and brine. The organic layer was dried over anhyd. Na₂SO₄ and concentrated under reduced pressure to obtain crude *trans*-4-[5-chloro-4'-(3-phenyl-ureido)-3',4',5',6'-tetrahydro-2'H-[2,3']bipyridinyl-1'-carbonyl]-piperidine-1-carboxylic acid tert-butyl ester (135 mg, 96% crude yield) as a yellow sticky material.
LC-MS (Method 2): m/z [M+H]⁺ = 542.6 (exact mass calc. = 541.25).

### Synthesis of trans-1-[5-chloro-1'-(piperidine-4-carbonyl)-1',2',3',4',5',6'-hexahydro-[2,3']bipiridinyl-4'-yl]-3-phenyl-urea (Ex-167)

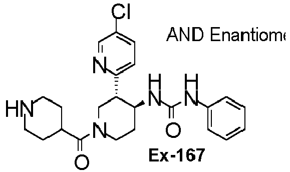

To a stirred solution of *trans*-4-[5-chloro-4'-(3-phenyl-ureido)-3',4',5',6'-tetrahydro-2'H-[2,3']bipyridinyl-1'-carbonyl]-piperidine-1-carboxylic acid tert-butyl ester (200 mg, 0.37 mmol, 1 eq) in 1,4-dioxane (5 ml) was added 4M HCl in dioxane (2 ml) followed by stirring at RT for a period of 4 h. the reaction mixture was concentrated under reduced pressure to obtain crude product which was purified by reverse phase purification to obtain *trans-*1-[5-chloro-1'-(piperidine-4-carbonyl)-1',2',3',4',5',6'-hexahydro-[2,3']bipyridinyl-4'-yl]-3-phenyl-urea (**Ex-167**, 18 mg, 38% yield) as an off white solid.
LC-MS (Method 2): m/z [M+H]⁺ = 442.2 (exact mass calc. = 441.19).
¹H-NMR (400 MHz, DMSO-d6): δ 8.53 (s, 1 H), 8.23 (s, 1 H), 7.84-7.83 (m, 1 H), 7.37 (t, *J* = 12 Hz, 1 H), 7.23-7.07 (m, 4 H), 6.81 (t, *J* = 8 Hz, 1H), 6.06 (d, *J* = 8 Hz, 1 H), 4.58-4.43 (m, 2 H), 4.02-3.95 (m, 2 H), 3.46-3.37 (m, 3 H), 3.18-3.14 (m, 1 H), 2.93-2.64 (m, 4 H), 2.52-2.47 (m, 1 H), 2.05-1.95 (m, 1 H), 1.52-1.37 (m, 5 H).

### Synthesis of trans-1-(4-chlorophoyl)-3-(3-(5-methoxypyridin-2-yl)piperidin-4-yl)urea ent-1(Ex-168) and trans-1-(4-chlorophenyl)-3-(3-(5-methoxypyridin-2-yl)piperidin-4-yl)urea ent-2 (Ex-169)

200 mg of racemic **BB-III-HCl** was separated by preparative chiral SFC to afford *trans*-1-(4-chlorophenyl)-3-(3-(5-methoxypyridin-2-yl)piperidin-4-yl)urea ent-1 (**Ex-168;** 22 mg) *trans*-1-(4-chlorophenyl)-3-(3-(5-methoxypyridin-2-yl)piperidin-4-yl)urea ent-2 (**Ex-169,** 30 mg).
**Ex-168:** LC-MS (Method 3): m/z [M+H]⁺ = 361.2 (exact mass calc. = 360.14).
¹H-NMR (400 MHz, DMSO-d6): δ 8.28 (s, 1 H), 8.18 (br s, 1 H), 7.307.27 (m, 3 H), 7.21-7.18 (m, 3 H), 5.99 (d, *J* = 8 Hz, 1 H), 3.91-3.88 (m, 1 H), 3.76 (s, 3 H), 2.97 (d, *J* = 8 Hz, 2 H), 2.66-2.62 (m, 3 H), 1.99-1.95 (m, 1 H), 1.34-1.30 (m, 1 H).
**Ex-169:** LC-MS (Method 3): m/z [M+H]⁺ = 361.2 (exact mass calc. = 360.14).
¹H-NMR (400 MHz, DMSO-d6): δ 8.28 (s, 1 H), 8.18 (br s, 1 H), 7.307.27 (m, 3 H), 7.21-7.18 (m, 3 H), 5.99 (d, *J* = 8 Hz, 1 H), 3.91-3.88 (m, 1 H), 3.76 (s, 3 H), 2.97 (d, *J* = 8 Hz, 2 H), 2.66-2.62 (m, 3 H), 1.99-1.95 (m, 1 H), 1.34-1.30 (m, 1 H).

### Synthesis of trans-2-[4-[(4-chlorophenyl)carbamoylamino]-3-(5-methoxypyridin-2-yl)piperidin-1-yl]acetamide ent-1 (Ex-170) and trans-2-[4-[(4-chlorophenyl)carbamoylamino]-3-(5-methoxypyridin-2-yl)piperidin-1-yl]acetamide ent-2 (Ex-171)

0.28 g of racemic **Ex-129** was separated by preparative chiral HPLC to afford *trans*-2-[4-[(4-chlorophenyl)carbamoylamino]-3-(5-*trans*-2-[4-[(4-chlorophenyl)carbamoylamino]-3-(5-methoxypyridin-2-yl)piperidin-1-yl]acetamide ent-2 (**Ex-170**, 0.056 g, 15.88 %).
**Ex-170:** LC-MS (Method 4): m/z [M+H]⁺ = 418.2 (exact mass calc. = 417.16).
¹H-NMR (400 MHz, DMSO-d6): δ 8.32 (s, 1H), 8.19 (d, *J*= 2.8 Hz, 1H), 7.18-7.32 (m, 7H), 7.09 (s, 1H), 5.98 (d, *J* = 8.1 Hz, 1H), 3.81-3.85 (m, 1H), 3.77 (s, 3H), 2.84-2.97 (m, 5H), 2.31 (t, *J*= 11.1 Hz, 1H), 2.23 (t, *J*= 11.2 Hz, 1H), 1.97-1.99 (m, 1H), 1.51-1.59 (m, 1H).
**Ex-171:** LC-MS (Method 7): m/z [M+H]⁺ = 418.1 (exact mass calc. = 417.16).
¹H-NMR (400 MHz, DMSO-d6): δ 8.32 (s, 1H), 8.19 (d, *J*= 2.8 Hz, 1H), 7.18-7.32 (m, 7H), 7.09 (s, 1H), 5.98 (d, *J*= 8.1 Hz, 1H), 3.81-3.84 (m, 1H), 3.77 (s, 3H), 2.84-2.96 (m, 5H), 2.31 (t, *J*= 11.1 Hz, 1H), 2.23 (t, *J*= 11.2 Hz, 1H), 1.97-1.99 (m, 1H), 1.54-1.59 (m, 1H).

### Synthesis of trans-1-(4-chlorophenyl)-3-[1-(cyclopropylmethyl)-3-(5-methoxypyridin-2-yl)piperidin-4-yl]urea ent-1 (Ex-172) and trans-1-(4-chlorophenyl)-3-[1-(cyclopropylmethyl)-3-(5-methoxypyridin-2-yl)piperidin-4-yllurea ent-1 (Ex-173)

0.265 g of racemic **Ex-127** was separated by preparative chiral HPLC to afford *trans*-1-(4-chlorophenyl)-3-[1-(cyclopropylmethyl)-3-(5-methoxypyridin-2-yl)piperidin-4-yl]urea ent-1 (**Ex-172,** 0.070 g, 22.26 %) and *trans-*1-(4-chlorophenyl)-3-[1-(cyclopropylmethyl)-3-(5-methoxypyridin-2-yl)piperidin-4-yl]urea ent-2 (**Ex-173,** 0.055 g, 17.49 %).
**Ex-172:** LC-MS (Method 3): m/z [M+H]⁺ = 415.4 (exact mass calc. = 414.18).
¹H-NMR (400 MHz, DMSO-d6): δ 8.32 (s, 1H), 8.19 (d, *J*= 2.8 Hz, 1H), 7.18-7.32 (m, 7H), 7.09 (s, 1H), 5.98 (d, *J=* 8.1 Hz, 1H), 3.81-3.85 (m, 1H), 3.77 (s, 3H), 2.84-2.97 (m, 5H), 2.31 (t, *J*= 11.1 Hz, 1H), 2.23 (t, *J*= 11.2 Hz, 1H), 1.97-1.99 (m, 1H), 1.51-1.59 (m, 1H).
**Ex-173:** LC-MS (Method 7): m/z [M+H]⁺ = 415.2 (exact mass calc. = 414.18).
¹H-NMR (400 MHz, DMSO-d6): δ 8.32 (s, 1H), 8.19 (d, *J*= 2.8 Hz, 1H), 7.18-7.32 (m, 7H), 7.09 (s, 1H), 5.98 (d, *J=* 8.1 Hz, 1H), 3.81-3.84 (m, 1H), 3.77 (s, 3H), 2.84-2.96 (m, 5H), 2.31 (t, *J*= 11.1 Hz, 1H), 2.23 (t, *J*= 11.2 Hz, 1H), 1.97-1.99 (m, 1H), 1.54-1.59 (m, 1H).

### Synthesis of trans-1-(4-cyanophenyl)-3-[1-(2,2-difluoroethyl)-3-(5-methoxypyridin-2-yl)piperidin-4-yl]urea ent-1 (Ex-174) and trans-1-(4-cyanophenyl)-3-[1-(2,2-difluoroethyl)-3-(5-methoxypyridin-2-yl)piperidin-4-yl]urea ent-2 (Ex-175)

0.265g of racemic **Ex-113** was separated by preparative chiral HPLC to afford *trans*-1-(4-cyanophenyl)-3-[1-(2,2-difluoroethyl)-3-(5-methoxypyridin-2-yl)piperidin-4-yl]urea ent-1 (**Ex-174,** 0.03817 g, 14.25 %) and *trans-*1*-*(4-cyanophenyl)-3-[1-(2,2-difluoroethyl)-3-(5-methoxypyridin-2-yl)piperidin-4-yl]urea ent-2 (**Ex-175,** 0.035 g, 13.06 %).
**Ex-174:** LC-MS (Method 7): m/z [M+H]⁺ = 416.2 (exact mass calc. = 415.18).
¹H-NMR (400 MHz, DMSO-d6): δ 8.76 (s, 1H), 8.19 (d, *J*= 2.7 Hz, 1H), 7.59 (d, *J*= 8.7 Hz, 2H), 7.44 (d, *J*= 8.7 Hz, 2H), 7.30 (dd, *J*= 2.9, 8.5 Hz, 1H), 7.24 (d, *J*= 8.5 Hz, 1H), 6.0-6.28 (m, 2H), 3.84-3.87 (m, 1H), 3.76 (s, 3H), 2.87-2.94 (m, 3H), 2.76 (dt, *J*= 4.2, 15.7 Hz, 2H), 2.32-2.45 (m, 2H), 1.96-1.99 (m, 1H), 1.48-1.56 (m, 1H). **Ex-175:** LC-MS (Method 7): m/z [M+H]⁺ = 416.2 (exact mass calc. = 415.18).
¹H-NMR (400 MHz, DMSO-d6): δ 8.75 (s, 1H), 8.19 (d, *J*= 2.7 Hz, 1H), 7.60 (d, *J*= 8.7 Hz, 2H), 7.44 (d, *J*= 8.7 Hz, 2H), 7.30 (dd, *J*= 2.9, 8.5 Hz, 1H), 7.24 (d, *J*= 8.6 Hz, 1H), 5.98-6.28 (m, 2H), 3.81-3.87 (m, 1H), 3.76 (s, 3H), 2.87-2.94 (m, 3H), 2.76 (dt, *J*= 4.2, 15.7 Hz, 2H), 2.31-2.45 (m, 2H), 1.97-1.99 (m, 1H), 1.48-1.56 (m, 1H).

### Synthesis of trans-1-[1-(2-hydroxy-2-methylpropyl)-3-(5-methoxypyridin-2-yl)piperidin-4-yl]-3-(3-methyl-1,2-thiazol-5-yl)urea ent-1 (Ex-176) and trans-1-[1-(2-hydroxy-2-methylpropyl)-3-(5-methoxypyridin-2-yl)piperidin-4-yl]-3-(3-methyl-1,2-thiazol-5-yl)urea ent-2 (EX-177)

0.2 g of racemic **Ex-121** was separated by preparative chiral HPLC to *trans*-1-[1-(2-hydroxy-2-methylpropyl)-3-(5-methoxypyridin-2-yl)piperidin-4-yl]-3-(3-methyl-1,2-thiazol-5-yl)urea ent-1 (**Ex-176**), 0.01923 g, 6.03 %) and *trans*-1-[1-(2-hydroxy-2-methylpropyl)-3-(5-methoxypyridin-2-yl)piperidin-4-yl]-3-(3-methyl-1,2-thiazol-5-yl)urea ent-2 (**EX-177**) 0.01733 g, 5.44 %).
**Ex-176:** LC-MS (Method 7): m/z [M+H]⁺ = 420.2 (exact mass calc. = 419.2).
¹H-NMR (400 MHz, DMSO-d6): δ 9.85 (s, 1H), 8.18 (d, *J*= 2.8 Hz, 1H), 7.28 (dd, *J*= 2.9, 8.5 Hz, 1H), 7.21 (d, *J*= 8.5 Hz, 1H), 6.40-6.50 (m, 1H), 6.39 (s, 1H), 4.03 (s, 1H), 3.79-3.82 (m, 1H), 3.76 (s, 3H), 2.92-3.01 (m, 3H), 2.21-2.35 (m, 4H), 2.19 (s, 3H), 1.89-1.96 (m, 1H), 1.52-1.61 (m, 1H), 1.08 (s, 6H).
**Ex-177:** LC-MS (Method 6): m/z [M+H]⁺ = 420.2 (exact mass calc. = 419.2).
¹H-NMR (400 MHz, DMSO-d6): δ 9.85 (s, 1H), 8.18 (d, *J*= 2.8 Hz, 1H), 7.28 (dd, *J*= 2.9, 8.5 Hz, 1H), 7.21 (d, *J*= 8.5 Hz, 1H), 6.40-6.50 (m, 1H), 6.39 (s, 1H), 4.03 (s, 1H), 3.79-3.82 (m, 1H), 3.76 (s, 3H), 2.92-3.01 (m, 3H), 2.19-2.35 (m, 7H), 1.89-1.92 (m, 1H), 1.55-1.58 (m, 1H), 1.08 (s, 6H).

### Synthesis of trans-1-(4-chlorophenyl)-3-[1-(2-hydroxy-2-methylpropyl)-3-(5-methoxypyridin-2-yl)piperidin-4-yl]urea ent-1 (Ex-178) and trans-1-(4-chlorophenyl)-3-[1-(2-hydroxy-2-methylpropyl)-3-(5-methoxypyridin-2-yl)piperidin-4-yl]urea ent-2 (Ex-179)

0.22 g of racemic **Ex-120** was separated by preparative chiral HPLC to *trans*-1-(4-chlorophenyl)-3-[1-(2-hydroxy-2-methylpropyl)-3-(5-methoxypyridin-2-yl)piperidin-4-yl]urea ent-1 (**Ex-178**, 0.06274 g, 17.17 %) *trans*-1-(4-chlorophenyl)-3-[1-(2-hydroxy-2-methylpropyl)-3-(5-methoxypyridin-2-yl)piperidin-4-yl]urea ent-2 (**Ex-179**, 0.05364 g, 14.68 %).
**Ex-178:** LC-MS (Method 7): m/z [M+H]⁺ = 433.2 (exact mass calc. = 432.19).
¹H-NMR (400 MHz, DMSO-d6): δ 8.32 (s, 1H), 8.18 (d, *J*= 2.7 Hz, 1H), 7.27-7.30 (m, 3H), 7.18-7.24 (m, 3H), 5.94 (d, *J*= 8.1 Hz, 1H), 4.03 (s, 1H), 3.79-3.81 (m, 1H), 3.76 (s, 3H), 2.85-3.01 (m, 3H), 2.18-2.35 (m, 4H), 1.94 (d, *J*= 9.8 Hz, 1H), 1.48-1.51 (m, 1H), 1.07 (s, 6H).
**Ex-179:** LC-MS (Method 7): m/z [M+H]⁺ = 433.2 (exact mass calc. = 432.19).
¹H-NMR (400 MHz, DMSO-d6): δ 8.32 (s, 1H), 8.18 (d, *J*= 2.6 Hz, 1H), 7.27-7.30 (m, 3H), 7.18-7.24 (m, 3H), 5.94 (d, *J*= 8.1 Hz, 1H), 4.03 (s, 1H), 3.78-3.81 (m, 1H), 3.76 (s, 3H), 2.84-3.01 (m, 3H), 2.18-2.34 (m, 4H), 1.94 (d, *J*= 9.9 Hz, 1H), 1.49-1.51 (m, 1H), 1.07 (s, 6H).

### Synthesis of trans-1-(4-chlorophenyl)-3-1-(1-cyanocyclopropyl)-3-(5-methoxypyridin-2-yl)piperidin-4-yl urea (Ex-180)

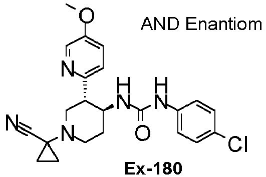

To the solution of *trans*-1-(4-chlorophenyl)-3-(3-(5-methoxypyridin-2-yl)piperidin-4-yl)urea trifluoroacetate (**BB-III-TFA,** 0.2 g, 0.4219 mmol, 1 eq) in ACN (10 ml), K₂CO₃ (0.291 g, 2.10 mmol, 5 eq) was stirred for 30 min in RT, filtered and evaporated to get salt free crude. Crude was taken in AcOH (1 ml) followed by (1-ethoxycyclopropoxy)trimethylsilane (0.073 g,0.4219 mmol,1.0 eq), was added at 0 °C.After 20 min TMSCN (0.104 g, 1.054 mmol, 2.5 eq) was added and stirred in RT for 16 hour. After completion of reaction (checked by TLC, 5% MeOH in DCM, R_{f}: 0.3), the reaction mixture was diluted by DCM, adjust pH∼10 by 10% NaOH solution and extracted by 10% MeOH in DCM concentrated to get crude which was purified by column chromatography using 0-10% MeOH in DCM and 230-400 silica gel to get *trans*-1-(4-chlorophenyl)-3-1-(1-cyanocyclopropyl)-3-(5-methoxypyridin-2-yl)piperidin-4-yl urea (**Ex-180,** 0.032 g, 17.8 %).
LC-MS (Method 7): m/z [M+H]⁺ = 426.2 (exact mass calc. = 425.06).
¹H-NMR (400 MHz, DMSO-d6): δ 8.30 (s, 1H), 8.19 (d, J=2.72 Hz, 1H), 7.30- 7.21(m, 4H), 7.18-7.15 (m, 2H), 5.99-5.97 (m, 1H),3.83-3.80(m,1H) 3.75 (s, 3H), 2.91-2.90 (m, 2H), 2.80(t, J= 7.24, 1H), 2.59(t, J= 10.92, 1H), 2.56-2.47 (m, 1H), 2.05-1.96 (m, 1H), 1.47-1.39 (m, 1H), 1.22-1.14 (m, 2H), 1.06-1.02 (m, 2H).

### Synthesis of trans-1-4-{[4-chlorophenyl)carbamoyl]amino}-3-(5-methoxypyridin-2-yl)piperidin-1-ylcyclopropane-1-carboxamide: (Ex-181)

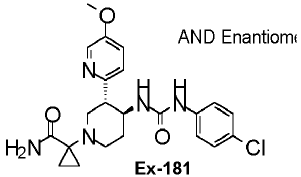

In 0.4 mL of H₂SO₄, *trans*-1-(4-hlorophenyl)-3-1-(1-cyanocyclopropyl)-3-(5-methoxypyridin-2-yl)piperidin-4-yl urea (**Ex-180**, 0.05 g, 0.1173 mmol, 1 eq) was stirred at 90 °C for 3 hour. After completion of reaction (checked by TLC, 5% MeOH in DCM, R_{f}: 0.3), the reaction mixture was quenched by NaHCO₃ solution and extracted by ethyl acetate, dried by Na₂SO₄ and concentrated to get crude which was purified by column chromatography using 0-10% MeOH in DCM and 230-400 silica gel to get *trans*-1-4-{[(4-chlorophenyl)carbamoyl]amino}-3-(5-methoxypyridin-2-yl)piperidin-1-ylcyclopropane-1-carboxamide: (**Ex-181,** 0.018 g, 34.56 %).
LC-MS (Method 7): m/z [M+H]⁺ = 444.2 (exact mass calc. = 443.01).
¹H-NMR (400 MHz, DMSO-d6): δ 8.51 (s, 1H), 8.18 (d, J=2.68 Hz, 1H), 7.42 (bs, 1H), 7.31- 7.29(m, 3H), 7.23-7.18 (m, 3H), 7.14 (bs, 1H), 6.04-6.02 (m, 1H),3.81-3.80(m,1H),3.76(s,3H) 2.95 (t, J=7.72 Hz, 1H), 2.73-2.66 (m, 2H), 2.50-2.32 (m, 2H), 1.98-1.95 (m, 1H), 1.59-1.51 (m, 1H), 0.92-0.89 (m, 4H).

### Synthesis of trans-1-[1'-(1-hydroxy-cyclopropylmethyl)-5-methoxy-1',2',3',4',5',6'-hexahydro-[2,3']bipyridinyl-4'-yl]-3-(3-methyl-isothiazol-5-yl)-urea (Ex-182)

### Synthesis of trans-1-[1'-(1-hydroxy-cyclopropanecarbonyl)-5-methoxy-1',2',3',4',5',6'-hexahydro-[2,3']bipyridinyl-4'-yl]-3-(3-methyl-isothiazol-5-yl)-urea

To a stirred solution of 1-hydroxy-cyclopropanecarboxylic acid (5 mg, 0.052 mmol, 1.0 eq) in dry THF (3 ml) were added EDCI-HCl (15 mg, 0.078 mmol, 1.5 eq) and HOBt (11 mg, 0.078 mmol, 1.5 eq) in ice-cold condition followed by addition of TEA (20 µl, 0.156 mmol, 3 eq) and *trans*-1-(5-methoxy-1',2',3',4',5',6'-hexahydro-[2,3']bipyridinyl-4'-yl)-3-(3-methyl-isothiazol-5-yl)-urea hydrochloride (**BB-IV-HCl,** 20 mg, 0.052 mmol, 1.0 eq) and stirred at RT for 18 h. The reaction mixture was diluted with EtOAc and washed with water and brine. The organic layer was dried over anhyd. Na₂SO₄ and concentrated under reduced pressure to obtain *trans*-1-[1'-(1-hydroxy-cyclopropanecarbonyl)-5-methoxy-1',2',3',4',5',6'-hexahydro-[2,3']bipyridinyl-4'-yl]-3-(3-methyl-isothiazol-5-yl)-urea (22 mg, 98% crude yield) as reddish sticky.
LC-MS (Method 2): m/z [M+H]⁺ = 432.2 (exact mass calc. = 431.16).

### Synthesis of trans-1-[1'-(1-hydroxy-cyclopropylmethyl)-5-methoxy-1',2',3',4',5',6'-hexahydro-[2,3']bipyridinyl-4'-yl]-3-(3-methyl-isothiazol-5-yl)-urea (Ex-182)

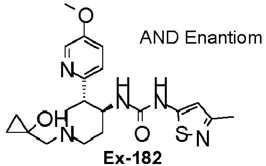

To a stirred solution of *trans*-1-[1'-(1-hydroxy-cyclopropanecarbonyl)-5-methoxy-1',2',3',4',5',6'-hexahydro-[2,3']bipyridinyl-4'-yl]-3-(3-methyl-isothiazol-5-yl)-urea (60 mg, 0.139 mmol, 1.0 eq) in dry THF (4 ml) at ice-cold condition was added BH₃-DMS (1 M in THF, 0.5 ml) and stirred at RT for 18 h. After that the reaction mixture was quenched with MeOH (1 ml). The reaction mixture was diluted with EtOAc and washed with water and brine. The organic layer was dried over anhyd. Na₂SO₄ and concentrated under reduced pressure to obtain crude product which was purified by reverse phase prep-HPLC purification to obtain *trans*-1-[1'-(1-hydroxy-cyclopropylmethyl)-5-methoxy-1',2',3',4',5',6'-hexahydro-[2,3']bipyridinyl-4'-yl]-3-(3-methyl-isothiazol-5-yl)-urea (**Ex-182**, 12 mg, 21% yield) as an off white solid.
LC-MS (Method 3): m/z [M+H]⁺ = 418.2 (exact mass calc. = 417.18).
¹H-NMR (400 MHz, DMSO-d6): δ 10.37 (br s, 1 H), 8.18 (s, 1 H), 7.28-7.21 (m, 2 H), 6.91 (br s, 1 H), 6.36 (s, 1 H), 4.92 (s, 1 H), 3.83-3.81 (m, 1 H), 3.76 (s, 3 H), 3.48-3.40 (m, 2 H), 3.05-2.93 (m, 3 H), 2.41 (s, 2 H), 2.28-2.22 (m, 1 H), 2.19 (s, 3 H), 1.93 (d, *J* = 12 Hz, 1 H), 1.57-1.54 (m, 1 H), 0.53-0.51 (m, 2 H), 0.38-0.31 (m, 2 H):

### Synthesis of trans-1-[1'-(3-hydroxy-cyclobutanecarbonyl)-5-methoxy-1',2',3',4',5',6'-hexahydro-[2,3']bipyridinyl-4'-yl]-3-(3-methyl-isothiazol-5-yl)-urea (Ex-183)

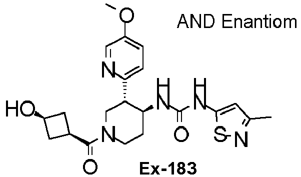

To a stirred solution of 3-hydroxy-cyclobutanecarboxylic acid 36 mg, 0.312 mmol, 1.2 eq) in dry THF (5 ml) were added EDCI-HCl (74 mg, 0.39 mmol, 1.5 eq) and HOBt (52 mg, 0.39 mmol, 1.5 eq) at RT followed by the addition of TEA (108 µl, 0.78 mmol, 3 eq) and 1-(5-methoxy-1',2',3',4',5',6'-hexahydro-[2,3']bipyridinyl-4'-yl)-3-(3-methyl-isothiazol-5-yl)-urea hydrochloride (**BB-IV-HCl,** 100 mg, 0.26 mmol, 1 eq) and stirred at RT for 16 h. The reaction mixture was diluted with 10% MeOH-DCM and washed with water and brine. The organic layer was dried over anhyd. Na₂SO₄ and concentrated under reduced pressure to obtain crude product which was purified by reverse phase purification to obtain *trans*-1-[1'-(3-hydroxy-cyclobutanecarbonyl)-5-methoxy-1',2',3',4',5',6'-hexahydro-[2,3']bipyridinyl-4'-yl]-3-(3-methyl-isothiazol-5-yl)-urea (**Ex-183,** 13 mg, 11% yield) as an off white sticky solid.
LC-MS (Method 3): [M+H]⁺ = 446.2 (exact mass calc. = 445.18):
¹H-NMR (400 MHz, DMSO-d6): δ 10.02 (br s, 1 H), 8.22-8.20 (m, 1 H), 7.32-7.21 (m, 2 H), 6.61 (br s, 1 H), 6.38 (s, 1 H), 5.05 (t, *J* = 4 Hz, 1 H), 4.45-4.39 (m, 1 H), 4.08-3.92 (m, 2 H), 3.85-3.79 (m, 1 H), 3.77 (s, 3 H), 3.22-3.11 (m, 2 H), 2.79-2.66 (m, 2 H), 2.37-2.30 (m, 1 H), 2.19 (s, 4 H), 2.02-1.95 (m, 3 H), 1.38-1.32 (m, 1H):

### Synthesis of trans-1-[5-chloro-1'-(1-methanesulfonyl-1H-pyrazol-3-ylmethyl)-1',2',3',4',5',6'-hexahydro-[2,3']bipyridinyl-4'-yl]-3-phenyl-urea (Ex-184)

### Synthesis of 1-methanesulfonyl-1H-pyrazole-3-carbaldehyde

To a stirred solution 1H-pyrazole-3-carbaldehyde (1 g, 10.4 mmol, 1 eq) in pyridine (10 ml) was added methanesulfonyl chloride (1.2 ml, 15.6 mmmol, 1.5 eq) and the reaction mixture was stirred at room temperature for a period of 3 h. The reaction mixture was evaporated under reduced pressure to obtain crude product which was purified by silica gel column chromatography (100-200 mesh) and eluted by 10-15% EtOAc in hexane to obtain 1-methanesulfonyl-1H-pyrazole-3-carbaldehyde (150 mg, 8% yield) as an off white solid.

### Synthesis of trans-1-[5-chloro-1'-(1-methanesulfonyl-1H-pyrazol-3-ylmethyl)-1',2',3 ',4',5',6'-hexahydro-[2,3']bipyridinyl-4'-yl]-3-phenyl-urea (Ex-184)

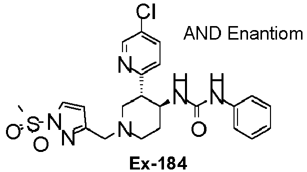

To a stirred solution of *trans*-1-(5-chloro-1',2',3',4',5',6'-hexahydro-[2,3']bipyridinyl-4'-yl)-3-phenyl-urea hydrochloride (**BB-X-HCl,** 100 mg, 0.272 mmol, 1 eq) in MeOH (5 ml) was added 1-methanesulfonyl-1H-pyrazole-3-carbaldehyde (47 mg, 0.272 mmmol, 1 eq) and the reaction mixture was stirred at room temperature for a period of 18 h followed by the addition of sodium cyano borohydride (68 mg, 1.08 mmol, 4.0 eq) and stirred at RT for another 3 h. The reaction mixture was then evaporated under reduced pressure to obtain crude product which was purified by silica gel column chromatography (100-200 mesh) and eluted by 2-3% MeOH in DCM to obtain *trans-*1*-*[5-chloro-5-chloro-1'-(1-methanesulfonyl-1H-pyrazol-3 -ylmethyl)-1 ',2',3 ',4',5',6'-hexahydro-[2,3']bipyridinyl-4'-yl]-3-phenyl-urea (**Ex-184,** 34 mg, 26% yield) as an off white solid.
LC-MS (Method 3): m/z [M+H]⁺ = 489.1 (exact mass calc. = 488.14).
¹H-NMR (400 MHz, DMSO-d6): δ 8.52 (s, 1 H), 8.20 (s, 1 H), 8.16 (s, 1 H), 7.83 (d, *J* = 8 Hz, 1 H), 7.36 (d, *J* = 12 Hz, 1 H), 7.24 (d, *J* = 8 Hz, 2 H), 7.14 (t, *J* = 8 Hz, 2 H), 6.83 (t, *J* = 8 Hz, 1 H), 6.53 (s, 1 H), 5.99 (d, *J* = 8 Hz, 1 H), 3.91-3.82 (m, 1 H), 3.58 (s, 2 H), 3.48 (s, 3 H), 3.00-2.88 (m, 3 H), 2.32 (t, *J* = 12 Hz, 1H), 2.19 (t, *J* = 12 Hz, 1 H), 2.00-1.97 (m, 1 H), 1.57-1.48 (m, 1 H).

### Synthesis of trans-1-(1'-azetidin-3-yl-5-chloro-1',2',3',4',5',6'-hexahydro-[2,3']bipyridinyl-4'-yl)-3-phenyl-urea (Ex-185)

### Synthesis of trans-3-[5-chloro-4'-(3-phenyl-ureido)-3',4',5',6'-tetrahydro-2'H-[2,3']bipyridinyl-1'-yl]-azetidine-1-carboxylic acid tert-butyl ester

To a stirred solution of *trans*-1-(5-chloro-1',2',3',4',5',6'-hexahydro-[2,3']bipyridinyl-4'-yl)-3-phenyl-urea hydrochloride (**BB-XII-HCl,** 100 mg, 0.27 mmol, 1 eq) in ACN (5 ml) were added Cs₂CO₃ (175 mg, 0.54 mmol, 2 eq) followed by the addition of 3-iodo-azetidine-1-carboxylic acid tert-butyl ester (382 mg, 1.35 mmol, 5 eq) and stirred at reflux for 16 h. The reaction mixture was diluted with 10% MeOH-DCM and washed with water and brine. The organic layer was dried over anhyd. Na₂SO₄ and concentrated under reduced pressure to obtain crude product which was purified by silica gel (100-200 mesh) column chromatography and eluted by 2-3% MeOH in DCM solution to obtain *trans*-3-[5-chloro-4'-(3-phenyl-ureido)-3',4',5',6'-tetrahydro-2'H-[2,3']bipyridinyl-1'-yl]-azetidine-1-carboxylic acid tert-butyl ester (100 mg, 76% yield) as off white solid.
LC-MS (Method 2): m/z [M+H]⁺ = 486.5 (exact mass calc. = 485.22).

### Synthesis of trans-1-(1'-azetidin-3-yl-5-chloro-1',2',3',4',5',6'-hexahydro-[2,3']bipyridinyl-4'-yl)-3-phenyl-urea (Ex-185)

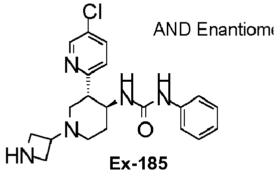

To a stirred solution of *trans*-3-[5-chloro-4'-(3-phenyl-ureido)-3',4',5',6'-tetrahydro-2'H-[2,3']bipyridinyl-1'-yl]-azetidine-1-carboxylic acid tert-butyl ester (100 mg, 0.206 mmol, 1 eq) in 1,4-dioxane (5 ml) was added 4M HCl in 1,4-dioxane (1 ml) followed by stirring at RT for a period of 4 h. the reaction mixture was concentrated under reduced pressure to obtain crude product which was purified by reverse phase prep-HPLC purification to obtain *trans*-1-(1'-azetidin-3-yl-5-chloro-1',2',3',4',5',6'-hexahydro-[2,3']bipyridinyl-4'-yl)-3-phenyl-urea (**Ex-185,** 24 mg, 30% yield) as an off white solid.
LC-MS (Method 3): m/z [M+H]⁺ = 386.2 (exact mass calc. = 385.17).
¹H-NMR (400 MHz, DMSO-d6): δ 8.52 (s, 1 H), 8.18 (s, 1 H), 7.84-7.82 (m, 1 H), 7.37 (d, *J=* 8 Hz, 1 H), 7.24 (d, *J* = 8 Hz, 2 H), 7.14 (t, *J* = 8 Hz, 2 H), 6.83 (t, *J* = 8 Hz, 1 H), 6.04 (d, *J* = 8 Hz, 1 H), 3.87-3.84 (m, 1 H), 3.47-3.37 (m, 4 H), 3.18-3.12 (m, 1 H), 2.95-2.90 (m, 1 H), 2.76 (d, *J* = 12 Hz, 2 H), 2.09-1.90 (m, 3 H), 1.53-1.45 (m, 1 H).

### Synthesis of trans-1-(1'-azetidin-3-ylmethyl-5-chloro-1',2',3',4',5',6'-hexahydro-[2,3']bipyridinyl-4'-yl)-3-phenyl-urea (Ex-186)

### Synthesis of trans-3-[5-chloro-4'-(3-phenyl-ureido)-3',4',5',6'-tetrahydro-2'H-[2,3']bipyridinyl-1'-carbonyl]-azetidine-1-carboxylic acid tert-butyl ester

To a stirred solution of azetidine-1,3-dicarboxylic acid mono-tert-butyl ester (80 mg, 0.40 mmol, 1.0 eq) in dry THF (10 ml) were added EDCI-HCl (114 mg, 0.60 mmol, 1.5 eq) and HOBt (81 mg, 0.60 mmol, 1.5 eq) and TEA (167 µl, 1.2 mmol, 3.0 eq) at RT followed by the addition of HCl salt of *trans*-1-(5-chloro-1',2',3',4',5',6'-hexahydro-[2,3']bipyridinyl-4'-yl)-3-phenyl-urea hydrochloride (**BB-XII-HCl,** 146 mg, 0.40 mmol, 1 eq) was added and continued stirring for 18 h at RT. The reaction mixture was diluted with 10% MeOH-DCM and washed with water and brine. The organic layer was dried over anhyd. Na₂SO₄ and concentrated under reduced pressure to obtain crude which was then purified by silica gel (100-200 mesh) column chromatography (3-5% MeOH in DCM as an eluent) to obatain *trans*-3-[5-chloro-4'-(3-phenyl-ureido)-3',4',5',6'-tetrahydro-2'H-[2,3']bipyridinyl-1'-carbonyl]-azetidine-1-carboxylic acid tert-butyl ester (175 mg, 85% yield) as an off white solid.
LC-MS (Method 2): m/z [M+H]⁺ = 514.3 (exact mass calc. = 513.21).

### Synthesis of trans-3-[5-chloro-4'-(3-phenyl-ureido)-3',4',5',6'-tetrahydro-2'H-[2,3']bipyridinyl-1'-ylmethyl]-azetidine-1-carboxylic acid tert-butyl ester

To a stirred solution of *trans*-3-[5-chloro-4'-(3-phenyl-ureido)-3',4',5',6'-tetrahydro-2'H-[2,3']bipyridinyl-1'-carbonyl]-azetidine-1-carboxylic acid tert-butyl ester (160 mg, 0.31 mmol, 1.0 eq) in dry THF (5 ml) at ice-cold condition was added BH₃-DMS (1 M in THF, 1.5 ml) and stirred at RT for 90 min. After that the reaction mixture was quenched with MeOH (1 ml). The reaction mixture was diluted with EtOAc and washed with water and brine. The organic layer was dried over anhyd. Na₂SO₄ and concentrated under reduced pressure to obtain crude product which was purified by reverse phase prep-HPLC purification to obtain *trans*-3-[5-Chloro-4'-(3-phenyl-ureido)-3',4',5',6'-tetrahydro-2'H-[2,3']bipyridinyl-1-ylmethyl]-azetidine-1-carboxylic acid tert-butyl ester (140 mg, 28% yield) as an off white solid.
LC-MS (Method 2): m/z [M+H]⁺ = 500.2 (exact mass calc. = 499.24).

### Synthesis of trans-1-(1'-azetidin-3-ylmethyl-5-chloro-1',2',3 ',4',5',6'-hexahydro-[2,3']bipyridinyl-4'-yl)-3-phenyl-urea (Ex-186)

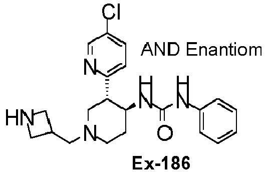

To a stirred solution of *trans*-3-[5-Chloro-4'-(3-phenyl-ureido)-3',4',5',6'-tetrahydro-2'H-[2,3']bipyridinyl-1'-ylmethyl]-azetidine-1-carboxylic acid tert-butyl ester (150 mg, 0.30 mmol, 1 eq) in 1,4-dioxane (3 ml) was added 4M HCl in 1,4-dioxane (1.2 ml) followed by stirring at RT for a period of 4 h. The reaction mixture was concentrated under reduced pressure to obtain crude product which was purified by reverse phase prep-HPLC purification to obtain *trans*-1-(1'-azetidin-3-ylmethyl-5-chloro-1',2',3',4',5',6'-hexahydro-[2,3']bipyridinyl-4'-yl)-3-phenyl-urea (**Ex-186,** 26 mg, 22% yield) as an off white solid.
LC-MS (Method 3): m/z [M+H]⁺ = 400.4 (exact mass calc. = 399.18).
¹H-NMR (400 MHz, DMSO-d6): δ 8.52 (s, 1 H), 8.28 (s, 1 H), 7.84-7.81 (m, 1 H), 7.37 (d, *J* = 8 Hz, 1 H), 7.24 (d, *J* = 8 Hz, 2 H), 7.14 (t, *J* = 8 Hz, 2 H), 6.82 (t, *J* = 8 Hz, 1H), 6.10 (d, *J* = 8 Hz, 1H), 3.85-3.82 (m, 1H), 3.53-3.40 (m, 5 H), 2.94-2.89 (m, 1 H), 2.82-2.79 (m, 3 H), 2.53-2.49 (m, 2 H), 2.21 (t, *J* = 12 Hz, 1 H), 2.08 (t, *J* = 12 Hz, 1 H), 1.95 (d, *J* = 8 Hz, 1 H), 1.52-1.43 (m, 1 H);

### Synthesis of trans-1-(4-chlorophenyl)-3-[3-(5-methoxypyridin-2-yl)-1-(1-methylpiperidine-4-carbonyl)piperidin-4-yl]urea ent-1 (Ex-187) and trans-1-(4-chlorophenyl)-3-[3-(5-methoxypyridin-2-yl)-1-(1-methylpiperidine-4-carbonyl)piperidin-4-yl]urea ent-2 (Ex-188)

170 mg of racemic *trans*-1-(4-chlorophenyl)-3-[3-(5-methoxypyridin-2-yl)-1-(1-methylpiperidine-4-carbonyl)piperidin-4-yl]urea (**Ex-89**) was separated by preparative chiral SFC to afford *trans*-1-(4-chlorophenyl)-3-[3-(5-methoxypyridin-2-yl)-1-(1-methylpiperidine-4-carbonyl)piperidin-4-yl]urea ent-1 (**Ex-187,** 15 mg) and *trans*-1-(4-chlorophenyl)-3-[3-(5-methoxypyridin-2-yl)-1-(1-methylpiperidine-4-carbonyl)piperidin-4-yl]urea ent-2 (**Ex-188**, 17 mg).
**Ex-187:** LC-MS (Method 3): m/z [M+H]⁺ = 486.2 (exact mass calc. = 485.22).
¹H-NMR (400 MHz, DMSO-d6): δ); 8.37 (s, 1H), 8.23-8.22 (d, *J* = 4 Hz, 1H), 7.33-7.25 (m, 4H), 7.20-7.18 *(d, J* = 8 Hz, 2 H), 6.06-6.01 (m, 1H), 4.46-4.43 (d, *J* = 12 Hz, 1H), 4.07-3.98 (m, 2H), 3.92-3.89 (d, *J* = 12 Hz, 1H), 3.78 (s, 3H), 3.22-3.16 (m, 1H), 2.74-2.67 (m, 4H), 2.56 (s, 1H), 2.14-1.99 (m, 5 H), 1.90 (s, 3H), 1.60-1.51 (m, 5H).
**Ex-188:** LC-MS (Method 3): m/z [M+H]⁺ = 486.2 (exact mass calc. = 485.22).
¹H-NMR (400 MHz, DMSO-d6): δ); 8.37 (s, 1H), 8.23-8.22 (d, *J* = 4 Hz, 1H), 7.33-7.25 (m, 4H), 7.20-7.18 (d, *J* = 8 Hz, 2 H), 6.06-6.01 (m, 1H), 4.46-4.43 (d, *J* = 12 Hz, 1H), 4.07-3.98 (m, 2H), 3.92-3.89 (d, *J* = 12 Hz, 1H), 3.78 (s, 3H), 3.22-3.16 (m, 1H), 2.74-2.67 (m, 4H), 2.56 (s, 1H), 2.14-1.99 (m, 5 H), 1.90 (s, 3H), 1.60-1.51 (m, 5H).

### Synthesis of trans-1-(4-chlorophenyl)-3-[1-[2-(dimethylamino)acetyl]-3-(5-methoxypyridin-2-yl)piperidin-4-yllurea ent-1 (Ex-189) and trans-1-(4-chlorophenyl)-3-[1-[2-(dimethylamino)acetyl]-3-(5-methoxypyridin-2-yl)piperidin-4-yl]urea ent-2 (Ex-190)

160 mg of racemic *trans*-1-(4-chlorophenyl)-3-[1-[2-(dimethylamino)acetyl]-3-(5-methoxypyridin-2-yl)piperidin-4-yl]urea (**Ex-94**) was separated by preparative chiral SFC to afford *trans*-1-(4-chlorophenyl)-3-[1-[2-(dimethylamino)acetyl]-3-(5-methoxypyridin-2-yl)piperidin-4-yl]urea ent-1 (**Ex-189,** 25 mg) and *trans*-1-(4-chlorophenyl)-3-[1-[2-(dimethylamino)acetyl]-3-(5-methoxypyridin-2-yl)piperidin-4-yl]urea ent-2 (**Ex-190,** 12 mg).
**Ex-189:** LC-MS (Method 3): m/z [M+H]⁺ = 446.4 (exact mass calc. = 445.19).
¹H-NMR (400 MHz, DMSO-d6): δ 8.34-8.33 (d, *J* = 4 Hz, 1H), 8.22 (s, 1H), 7.30-7.28 (d, *J* = 8 Hz, 4H), 7.20-7.18 (d, *J* = 8 Hz, 2H), 6.07-6.05 (d, *J* = 8 Hz, 1H), 4.41-4.38 (d, *J* = 12 Hz, 1H), 4.13-4.05 (m, 2 H), 3.78 (s, 3 H), 3.16-3.14 (d, *J* = 8 Hz, 1 H), 3.04-2.97 (m, 1 H), 2.82-2.76 (m, 1 H), 2.66-2.62 (d, *J* = 16 Hz, 1H), 2.19-2.16 *(d, J* = 12 Hz, 6H), 2.05-2.01 (d, 1H), 1.46-1.33 (d, *J* = 52 Hz, 1H).
**Ex-190:** LC-MS (Method 3): m/z [M+H]⁺ = 446.4 (exact mass calc. = 445.19).
¹H-NMR (400 MHz, DMSO-d6): δ 8.34-8.33 (d, *J* = 4 Hz, 1H), 8.22 (s, 1H), 7.30-7.28 (d, *J* = 8 Hz, 4H), 7.20-7.18 (d, *J* = 8 Hz, 2H), 6.07-6.05 (d, *J* = 8 Hz, 1H), 4.41-4.38 (d, *J* = 12 Hz, 1H), 4.13-4.05 (m, 2 H), 3.78 (s, 3 H), 3.16-3.14 (d, *J* = 8 Hz, 1 H), 3.04-2.97 (m, 1 H), 2.82-2.76 (m, 1 H), 2.66-2.62 (d, *J* = 16 Hz, 1H), 2.19-2.16 *(d, J* = 12 Hz, 6H), 2.05-2.01 (d, 1H), 1.46-1.33 (d, *J* = 52 Hz, 1H).

### Synthesis of trans-1-(4-chlorophenyl)-3-[1-(3-methoxypropyl)-3-(5-methoxypyridin-2-yl)piperidin-4-yl]urea ent-1 (Ex-191) and trans-1-(4-chlorophenyl)-3-[1-(3-methoxypropyl)-3-(5-methoxypyridin-2-yl)piperidin-4-yl]urea ent-2 (Ex-192)

180 mg of racemic *trans*-1-(4-chlorophenyl)-3-[1-(3-methoxypropyl)-3-(5-methoxypyridin-2-yl)piperidin-4-yl]urea (**Ex-80**) was separated by preparative chiral SFC to afford *trans*-1-(4-chlorophenyl)-3-[1-(3-methoxypropyl)-3-(5-methoxypyridin-2-yl)piperidin-4-yl]urea ent-1 (**Ex-191,** 31 mg) and *trans*-1-(4-chlorophenyl)-3-[1-(3-methoxypropyl)-3-(5-methoxypyridin-2-yl)piperidin-4-yl]urea ent-2 (**Ex-192,** 30 mg). **Ex-191:** LC-MS (Method 3): m/z [M+H]⁺ = 433.2 (exact mass calc. = 432.19).
¹H-NMR (400 MHz, DMSO-d6): δ 8.33 (s, 1H), 8.19-8.18 (d, *J* = 4 Hz, 1H), 7.30-7.28 (dd, 3H), 7.24-7.22 (d, *J* = 20 Hz, 1 H), 7.20-7.17 (m, 2H), 5.98-5.96 (d, *J* = 8 Hz, 1H), 3.83-3.79 (m, 1H), 3.76 (s, 3H), 3.34-3.27 (m, 2H), 3.20 (s, 3H), 2.89-2.83 (m, 3H), 2.34-2.30 (t, *J* = 16 Hz, 2H), 2.14-2.08 (t, *J* = 24 Hz, 1H), 2.05-1.99 (m, 2H), 1.66-1.61 (m, 2H), 1.48-1.44 (dd, *J* = 4, 4 Hz, 1H).
**Ex-192:** LC-MS (Method 3): m/z [M+H]⁺ = 433.2 (exact mass calc. = 432.19).
¹H-NMR (400 MHz, DMSO-d6): δ 8.33 (s, 1H), 8.19-8.18 (d, *J* = 4 Hz, 1H), 7.30-7.28 (dd, 3H), 7.24-7.22 (d, *J* = 20 Hz, 1 H), 7.20-7.17 (m, 2H), 5.98-5.96 (d, *J* = 8 Hz, 1H), 3.83-3.79 (m, 1H), 3.76 (s, 3H), 3.34-3.27 (m, 2H), 3.20 (s, 3H), 2.89-2.83 (m, 3H), 2.34-2.30 (t, *J* = 16 Hz, 2H), 2.14-2.08 (t, *J* = 24 Hz, 1H), 2.05-1.99 (m, 2H), 1.66-1.61 (m, 2H), 1.48-1.44 (dd, *J* = 4, 4 Hz, 1H).

### Synthesis of trans-1-(4-chloro-phenyl)-3-[3-fluoro-5-methoxy-1'-(3-methoxy-propyl)-1',2',3 ',4',5',6'-hexahydro-[2,3']bipyridinyl-4'-yl]-urea (Ex-193)

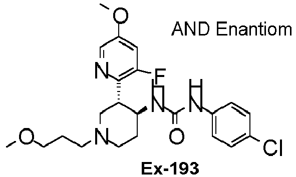

To a stirred solution of *trans*-1-(4-chloro-phenyl)-3-(3-fluoro-5-methoxy-1',2',3',4',5',6'-hexahydro-[2,3']bipyridinyl-4'-yl)-urea hydrochloride (**BB-XIII-HCl,** 80 mg, 0.193 mmol, 1 eq) in ACN (5 ml) were added Cs₂CO₃ (125 mg, 0.386 mmol, 2 eq) followed by the addition of 1-bromo-3-methoxy-propane (36 mg, 0.231 mmol, 1.2 eq) and stirred at RT for 16 h. The reaction mixture was diluted with 10% MeOH-DCM and washed with water and brine. The organic layer was dried over anhyd. Na₂SO₄ and concentrated under reduced pressure to obtain crude product which was purified by reverse phase phase prep-HPLC purification to obtain *trans-*1-(4-chlorophenyl)-3-[3-fluoro-5-methoxy-1'-(3-methoxy-propyl)-1',2',3',4',5',6'-hexahydro-[2,3']bipyridinyl-4'-yl]-urea
(**Ex-193,** 18 mg, 21% yield) as off white solid.
LC-MS (Method 3): m/z [M+H]⁺ = 451.2 (exact mass calc. = 450.18).
¹H-NMR (400 MHz, DMSO-d6): δ 8.34 (s, 1 H), 8.12 (s, 1 H), 7.35-7.26 (m, 3 H), 7.18 (d, *J=* 8 Hz, 2 H), 5.96 (d, *J=* 8 Hz, 1 H), 4.02-3.88 (m, 1 H), 3.79 (s, 3 H), 3.02 (s, 3 H), 3.14-3.08 (m, 1 H), 2.91-2.89 (m, 1 H), 2.82-2.79 (m, 1 H), 2.35-2.31 (m, 2 H), 2.21 (t, *J* = 12 Hz, 1 H), 2.09-1.91 (m, 2 H), 1.75-1.62 (m, 2 H), 1.69-1.41 (m, 1 H).

### Synthesis of trans-1-(4-chloro-phenyl)-3-[1'-(3-hydroxy-oxetan-3-ylmethyl)-5-methoxy-1',2',3',4',5',6'-hexahydro-[2,3']bipyridinyl-4'-yl]-urea (Ex-194)

### Synthesis of 3-vinyl-oxetan-3-ol

To a stirred solution of oxetan-3-one (1.0 g, 13.88 mmol, 1 eq) in dry THF (50 ml) was degassed with argon for a period of 15 min followed by the addition of vinyl magnesium bromide (1 M in THF, 23.6 ml, 23.61 mmol, 1.7 eq) at 0 °C in such a rate that internal temperature was below 10 °C and the reaction temperature was slowly increase to come to an ambient temperature over 2 h. The reaction mixture was then slowly stirred at RT for a period of 18 H. After completion of the reaction it was quenched with saturated aqueous NH₄Cl solution and extracted with ethyl acetate. The organic layer was dried over anhyd. Na₂SO₄ and concentrated under reduced pressure to obtain to afford 3-vinyl-oxetan-3-ol (1.1 g, 79% yield) as brown oil.

### Synthesis of 3-hydroxy-oxetane-3-carbaldehyde

To a stirred solution of 3-vinyl-oxetan-3-ol (1 g, 10.00 mmol, 1 eq) in MeOH at -78 °C was purged O₃ gas over a period of 1 h followed by the addition of triphenyl phosphine resin (1-1.5 eq/g, 100-200 mesh, 785 mg) was added at same temperature and the reaction mixture was stirred at RT over a period of 16 h. After completion of the reaction (monitored by TLC and LC-MS), the reaction mixture was diluted with EA (600 ml) and washed with water and brine. The organic layer was dried over anhyd. Na₂SO₄ and concentrated under reduced pressure to obtain crude product which was purified by silica gel (100-200 mesh) column chromatography (2-3% MeOH in DCM) to afford 3-hydroxy-oxetane-3-carbaldehyde (965 mg, 95% yield) as brown oil.

### Synthesis of trans-1-(4-chloro-phenyl)-3-[1'-(3-hydroxy-oxetan-3-ylmethyl)-5-methoxy-1',2',3',4',5',6'-hexahydro-[2,3']bipyridinyl-4'-yl]-urea (Ex-194)

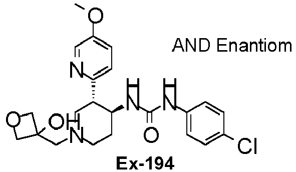

To a stirred solution of *trans*-1-(4-chloro-phenyl)-3-(5-methoxy-1',2',3',4',5',6'-hexahydro-[2,3']bipyridinyl-4'-yl)-urea (**BB-III-HCl,** 70 mg, 0.176 mmol, 1eq) in MeOH (5 ml) was added 3-hydroxy-oxetane-3-carbaldehyde (22 mg, 0.212 mmol, 1.2 eq) and the reaction mixture was stirred at RT over a period of 16 h, followed by the addition of NaCNBH₃ (33 mg, 0.528, 3 eq) and the reaction mixture was stirred at RT for another 2 h and quenched with a pinch of ice. The reaction mixture was evaporated under reduced pressure and submitted for reverse phase prep-HPLC chromatography to obtain *trans*-1-(4-chloro-phenyl)-3-[1'-(3-hydroxy-oxetan-3-ylmethyl)-5-methoxy-1',2',3',4',5',6'-hexahydro-[2,3']bipyridinyl-4'-yl]-urea (**Ex-194,** 12 mg, 15% yield) as white solid.
LC-MS (Method 3): m/z [M+H]⁺ = 447.2 (exact mass calc. = 446.17).
¹H-NMR (400 MHz, DMSO-d6): δ 8.34 (s, 1 H), 8.18 (d, *J* = 4 Hz, 1 H), 7.30-7.24 (m, 3 H), 7.24-7.18 (m, 3 H), 5.96 (d, *J* = 8 Hz, 1 H), 5.47 (s, 1 H), 4.40-4.33 (m, 4 H), 3.82-3.77 (m, 1 H), 3.74 (s, 3 H), 2.95-2.85 (m, 4 H), 2.69-2.60 (m, 2 H), 2.36-2.24 (m, 2 H), 1.96-1.93 (m, 1 H), 1.55-1.45 (m, 1 H).

### Synthesis of trans-1-(4-chloro-phenyl)-3-[5-methoxy-1'-(3-methoxy-cyclobutylmethyl)-1',2',3',4',5',6'-hexahydro-[2,3']bipyridinyl-4'-yl]-urea (Ex-195)

### Synthesis of trans-1-(4-chloro-phenyl)-3-[5-methoxy-1'-(3-methoxy-cyclobutanecarbonyl)-1',2',3',4',5',6'-hexahydro-[2,3']bipyridinyl-4'-yl]-urea

To a stirred solution of *trans*-3-methoxy-cyclobutanecarboxylic acid (16 mg, 0.126 mmol, 1.0 eq) in dry THF (5 ml) were added EDCI-HCl (36 mg, 0.189 mmol, 1.5 eq) and HOBt (25 mg, 0.189 mmol, 1.5 eq) in ice-cold condition followed by addition of TEA (53 µl, 0.378 mmol, 3 eq) and 1-(4-chloro-phenyl)-3-(5-methoxy-1',2',3',4',5',6'-hexahydro-[2,3']bipyridinyl-4'-yl)-urea hydrochloride (**BB-III-HCl,** 50 mg, 0.126 mmol, 1.0 eq) and stirred at RT for 16 h. The reaction mixture was diluted with EtOAc and washed with water and brine. The organic layer was dried over anhyd. Na₂SO₄ and concentrated under reduced pressure to obtain *trans-*1-(4-chlorophenyl)-3-[5-methoxy-1'-(3-methoxy-cyclobutanecarbonyl)-1',2',3',4',5',6'-hexahydro-[2,3']bipyridinyl-4'-yl]-urea (55 mg, 92% crude yield) as red sticky which was directly used for the next step.
LC-MS (Method 2): m/z [M+H]⁺ = 473.6 (exact mass calc. = 472.19).

### Synthesis of trans-1-(4-chloro-phenyl)-3-[5-methoxy-1'-(3-methoxy-cyclobutylmethyl)-1',2',3',4',5',6'-hexahydro-[2,3']bipyridinyl-4'-yl]-urea (Ex-195)

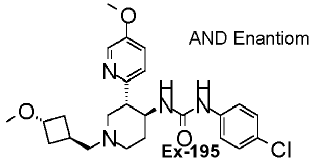

To a stirred solution of *trans*-1-(4-chloro-phenyl)-3-[5-methoxy-1'-(3-methoxy-cyclobutanecarbonyl)-1',2',3',4',5',6'-hexahydro-[2,3']bipyridinyl-4'-yl]-urea (55 mg, 0.116 mmol, 1.0 eq) in dry THF (2 ml) at ice-cold condition were added BH₃-DMS (200 µl, 2.1 mmol, 18 eq) and stirred at RT for 16 h. The reaction mixture was diluted with EtOAc and washed with water and brine. The organic layer was dried over anhyd. Na₂SO₄ and concentrated under reduced pressure to obtain crude product which was purified by reverse phase prep-HPLC purification to obtain *trans*-1-(4-chloro-phenyl)-3-[5-methoxy-1'-(3-methoxy-cyclobutylmethyl)-1',2',3',4',5',6'-hexahydro-[2,3']bipyridinyl-4'-yl]-urea (**Ex-195,** 12 mg, 22% yield) as white solid.
LC-MS (Method 3): m/z [M+H]⁺ = 459.2 (exact mass calc. = 458.21).
¹H-NMR (400 MHz, DMSO-d6): δ 8.33 (s, 1 H), 8.18 (d, *J* = 4 Hz, 1 H), 7.30-7.18 (m, 6 H), 5.96 (br s, 1 H), 3.90-3.81 (m, 2 H), 3.76 (s, 3 H), 3.09 (s, 3 H), 2.89-2.75 (m, 3 H), 2.33-2.29 (m, 2 H), 2.05-1.90 (m, 8 H), 1.51-1.42 (m, 1H).

### Synthesis of trans-1-(4-chloro-phepyl)-3-[3-fluoro-1'-(2-methanesulfonyl-ethyl)-5-methoxy-1',2',3',4',5',6'-hexahydro-[2,3']bipyridinyl-4'-yl]-urea (Ex-196)

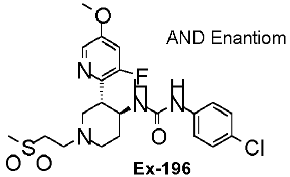

To a stirred solution of *trans*-1-(4-chloro-phenyl)-3-(3-fluoro-5-methoxy-1',2',3',4',5',6'-hexahydro-[2,3']bipyridinyl-4'-yl)-urea hydrochloride (**BB-XIII-HCl,** 100 mg, 0.241 mmol, 1 eq) in ACN (5 ml) were added Cs₂CO₃ (236 mg, 0.74 mmol, 3 eq) followed by the addition of 1-bromo-2-methanesulfonyl-ethane (90 mg, 0.483 mmol, 2 eq) and stirred at RT for 2 days. The reaction mixture was diluted with 10% MeOH-DCM and washed with water and brine. The organic layer was dried over anhyd. Na₂SO₄ and concentrated under reduced pressure to obtain crude product which was purified by reverse phase phase prep-HPLC purification to obtain *trans*-1-(4-chloro-phenyl)-3-[3-fluoro-1'-(2-methanesulfonyl-ethyl)-5-methoxy-1',2',3',4',5',6'-hexahydro-[2,3']bipyridinyl-4'-yl]-urea (**Ex-196**, 44 mg, 37% yield) as off white solid.
LC-MS (Method 3): m/z [M+H]⁺ = 485.1 (exact mass calc. = 484.13).
¹H-NMR (400 MHz, DMSO-d6): δ 8.31 (s, 1 H), 8.13 (s, 1 H), 7.34 (d, *j* = 12 Hz, 1 H), 7.27 (d, *j* = 8 Hz, 2 H), 7.18 (d, *J* = 12 Hz, 2 H), 5.96 (d, *J* = 8 Hz, 1 H), 3.95-3.86 (m, 1 H), 3.80 (s, 3 H), 3.32-3.25 (m, 2 H), 3.18-3.09 (m, 1 H), 3.02 (s, 3 H), 2.99-2.87 (m, 2 H), 2.79-2.72 (m, 2 H), 2.33 (t, *J* = 8 Hz, 1 H), 2.13 (t, *J* = 12 Hz, 1 H), 2.05-1.98 (m, 1 H), 1.59-1.48 (m, 1 H).

### Synthesis of trans-1-(4-chloro-phenyl)-3-[3-fluoro-1'-(3-hydroxy-propyl)-5-methoxy-1',2',3',4',5',6-hexahydro-[2,3']bipyridinyl-4'-yl]-urea (Ex-197)

### Synthesis of trans-1-{1'-[3-(tert-butyl-dimethyl-silanyloxy)-propyl]-3-fluoro-5-methoxy-1',2',3',4',5',6'-hexahydro-[2,3']bipyridinyl-4'-yl}-3-(4-chloro-phenyl)-urea

To a stirred solution of *trans*-1-(4-chloro-phenyl)-3-(3-fluoro-5-methoxy-1',2',3',4',5',6'-hexahydro-[2,3']bipyridinyl-4'-yl)-urea (**BB-XIII-HCl**, 80 mg, 0.193 mmol, 1 eq) in ACN (7 ml) were added Cs₂CO₃ (125 mg, 0.386 mmol, 2.0 eq) followed by the addition of (3-bromo-propoxy)-tert-butyl-dimethyl-silane (59 mg, 0.232 mmol, 1.2 eq) and stirred at RT for 18 h. The eaction mixture was diluted with 10% MeOH-DCM and washed with water and brine. The organic layer was dried over anhyd. Na₂SO₄ and concentrated under reduced pressure to obtain crude product which was purified by combi flash column chromatography (2-3% MeOH in DCM) to obtain *trans*-1-{1'-[3-(tert-butyl-dimethyl-silanyloxy)-propyl]-3-fluoro-5-methoxy-1',2',3',4',5',6'-hexahydro-[2,3']bipyridinyl-4'-yl}-3-(4-chloro-phenyl)-urea (90 mg, 85% yield) as yellowish sticky.
LC-MS (Method 2): m/z [M+H]⁺ = 550.7 (exact mass calc. = 550.25).

### Synthesis of trans-1-(4-chloro-phenyl)-3-[3-fluoro-1'-(3-hydroxy-propyl)-5-methoxy-1',2',3',4',5',6'-hexahydro-[2,3']bipyridinyl-4'-yl]-urea (Ex-197)

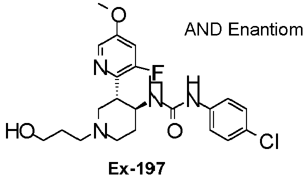

To a stirred solution of *trans*-1-{1'-[3-(tert-butyl-dimethyl-silanyloxy)-propyl]-3-fluoro-5-methoxy-1',2',3',4',5',6'-hexahydro-[2,3']bipyridinyl-4'-yl}-3-(4-chloro-phenyl)-urea (90 mg, 0.163 mmol, 1 eq) in THF (5 ml) was added TBAF (1 M soln in THF, 160 µl, 0.16 mmol, 1 eq) at ice cooled condition followed by the stirring at RT for a period of 6 h. The reaction mixture was evaporated under reduced pressure and diluted with 10% MeOH-DCM and washed with water, NaHCO₃, and brine. The organic layer was dried over anhyd. Na₂SO₄ and concentrated under reduced pressure to obtain crude product which was purified by reverse phase prep-HPLC purification to obtain *trans*-1-(4-chloro-phenyl)-3-[3-fluoro-1'-(3-hydroxy-propyl)-5-methoxy-1',2',3',4',5',6'-hexahydro-[2,3']bipyridinyl-4'-yl]-urea (**Ex-197**, 25 mg, 35% yield) as off white solid.
LC-MS (Method 3): m/z [M+H]⁺ = 437.2 (exact mass calc. = 436.17).
¹H-NMR (400 MHz, DMSO-d6): δ 8.31 (s, 1 H), 8.12 (s, 1 H), 7.33 (d, *J* = 12 Hz, 1 H), 7.27 (d, *j* = 8 Hz, 2 H), 7.18 (d, *j* = 8 Hz, 2 H), 5.93 (d, *J* = 12 Hz, 1 H), 4.43 (br s, 1 H), 3.95-3.89 (m, 1 H), 3.80 (s, 3 H), 3.42 (t, *J* = 8 Hz, 2 H), 3.13-3.08 (m, 1 H), 2.93-2.90 (m, 1 H), 2.83-2.81 (m, 1 H), 2.36 (t, *J* = 4 Hz, 2 H), 2.21 (t*, J* = 12 Hz, 1 H), 2.04-1.98 (m, 2 H), 1.59-1.48 (m, 3 H).

### Synthesis of trans-1-(4-chloro-phenyl)-3-[3-fluoro-5-methoxy-1'-(2-methoxy-ethyl)-1',2',3',4',5',6'-hexahydro-[2,3']bipyridinyl-4'-yl]-urea (Ex-198)

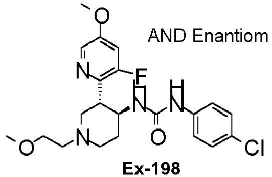

To a stirred solution of *trans*-1-(4-chloro-phenyl)-3-(3-fluoro-5-methoxy-1',2',3',4',5',6'-hexahydro-[2,3']bipyridinyl-4'-yl)-urea (**BB-XIII-HCl** (80 mg, 0.193 mmol, 1 eq) in ACN (7 ml) were added Cs₂CO₃ (125 mg, 0.386 mmol, 2.0 eq) followed by the addition of 1-bromo-2-methoxy-ethane (32 mg, 0.236 mmol, 1.2 eq) and stirred at RT for 18 h. The reaction mixture was diluted with 10% MeOH-DCM and washed with water and brine. The organic layer was dried over anhyd. Na₂SO₄ and concentrated under reduced pressure to obtain crude product which was purified by reverse phase prep-HPLC purification to obtain *trans*-1-(4-chloro-phenyl)-3-[3-fluoro-5-methoxy-1'-(2-methoxy-ethyl)-1',2',3',4',5',6'-hexahydro-[2,3']bipyridinyl-4'-yl]-urea (**Ex-198,** 14 mg, 17% yield) as off white solid.
LC-MS (Method 3): m/z [M+H]⁺ = 437.2 (exact mass calc. = 436.17).
¹H-NMR (400 MHz, DMSO-d6): δ 8.31 (s, 1 H), 8.12 (s, 1 H), 7.33 (d, *J* = 12 Hz, 1 H), 7.27 (d, *J* = 8 Hz, 2 H), 7.18 (d, *J* = 8 Hz, 2 H), 5.93 (d, *J* **=** 8 Hz, 1 H), 3.94-3.85 (m, 1 H), 3.79 (s, 3 H), 3.41 (t, *J* = 4 Hz, 2 H), 3.21 (s, 3 H), 3.13-3.09 (m, 1 H), 2.94-2.91 (m, 1 H), 2.86-2.84 (m, 1 H), 2.30 (t*, J* = 12 Hz, 1 H), 2.10 (t*, J* = 8 Hz, 1 H), 1.99-1.97 (m, 1 H), 1.55-1.46 (m, 1 H).

### Synthesis of trans-1-(4-chloro-phepyl)-3-[3-fluoro-1'-(2-hydroxy-ethyl)-5-methoxy-1',2',3',4',5',6'-hexahydro-[2,3']bipyridinyl-4'-yl]-urea (Ex-199)

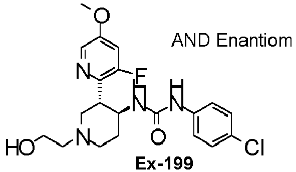

To a stirred solution of *trans*-1-(4-chloro-phenyl)-3-(3-fluoro-5-methoxy-1',2',3',4',5',6'-hexahydro-[2,3']bipyridinyl-4'-yl)-urea hydrochloride (**BB-XIII-HCl**, 80 mg, 0.193 mmol, 1 eq) in ACN (7 ml) were added Cs₂CO₃ (125 mg, 0.386 mmol, 2.0 eq) followed by the addition of 2-bromo-ethanol (29 mg, 0.236 mmol, 1.2 eq) and stirred at RT for 18 h. The reaction mixture was diluted with 10% MeOH-DCM and washed with water and brine. The organic layer was dried over anhyd. Na₂SO₄ and concentrated under reduced pressure to obtain crude product which was purified by reverse phase prep-HPLC purification to obtain *trans*-1-(4-chloro-phenyl)-3-[3-fluoro-1'-(2-hydroxy-ethyl)-5-methoxy-1',2',3',4',5',6'-hexahydro-[2,3']bipyridinyl-4'-yl]-urea (**Ex-199,** 25 mg, 31% yield) as off white solid.
LC-MS (Method 3): m/z [M+H]⁺ = 423.2 (exact mass calc. = 422.15).
¹H-NMR (400 MHz, DMSO-d6): δ 8.29 (s, 1 H), 8.12 (s, 1 H), 7.32 (d, *J* = 12 Hz, 1 H), 7.27 (d, *J* = 8 Hz, 2 H), 7.18 (d, *J* = 8 Hz, 2 H), 5.92 (d, *J* = 8 Hz, 1 H), 4.42-4.29 (m, 1 H), 3.93-3.87 (m, 1 H), 3.80 (s, 3 H), 3.50-3.46 (m, 2 H), 3.15-3.10 (m, 1 H), 2.94-2.85 (m, 2 H), 2.43-2.40 (m, 2 H), 2.34-2.28 (m, 1 H), 2.13-2.08 (m, 1 H), 2.04-1.98 (m, 1 H), 1.55-1.50 (m, 1 H).

### Synthesis of trans-1-(4-chloro-phenyl)-3-(3-fluoro-5-methoxy-1'-oxetan-3-ylmethyl-1-1',2',3',4',5',6'-hexahydro-[2,3']bipyridinyl-4'-yl)-urea (Ex-200)

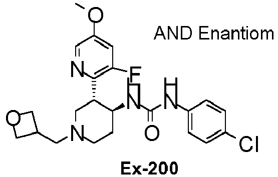

To a stirred solution of *trans*-1-(4-chloro-phenyl)-3-(3-fluoro-5-methoxy-1',2',3',4',5',6'-hexahydro-[2,3']bipyridinyl-4'-yl)-urea hydrochloride (**BB-XIII-HCl**, 80 mg, 0.193 mmol, 1 eq) in ACN (5 ml) were added DIPEA (200 µl, 1.158 mmol, 6.0 eq) followed by the addition of 3-iodomethyl-oxetane (38 mg, 0.193 mmol, 1.0 eq) and stirred at 70 °C for 18 h. The reaction mixture was diluted with 10% MeOH-DCM and washed with water and brine. The organic layer was dried over anhyd. Na₂SO₄ and concentrated under reduced pressure to obtain crude product which was purified by reverse phase prep-HPLC purification to obtain *trans*-1-(4-chloro-phenyl)-3-(3-fluoro-5-methoxy-1'-oxetan-3-ylmethyl-1',2',3',4',5',6'-hexahydro-[2,3']bipyridinyl-4'-yl)-urea (**Ex-200,** 18 mg, 21% yield) as off white solid.
LC-MS (Method 1): m/z [M+H]⁺ = 449.2 (exact mass calc. = 448.17).
¹H-NMR (400 MHz, DMSO-d6): δ 8.31 (s, 1 H), 8.12 (s, 1 H), 7.33 (d, *J* = 12 Hz, 1 H), 7.26 (d, *J* = 12 Hz, 2 H), 7.18 (d, *J* = 8 Hz, 2 H), 5.91 (d, *J* = 8 Hz, 1 H), 4.64-4.58 (m, 2 H), 4.25-4.21 (m, 2 H), 3.92-3.84 (m, 1 H), 3.79 (s, 3 H), 3.19-3.07 (m, 2 H), 2.81-2.71 (m, 2 H), 2.64 (d, *J* = 8 Hz, 2 H), 2.27 (t, *J* = 12 Hz, 1 H), 2.07-1.95 (m, 2 H), 1.52-1.44 (m, 1 H).

### Synthesis of trans-1-(4-chloro-phenyl)-3-[5-methoxy-1'-(1-methoxy-cyclopropylmethyl)-1',2',3 ',4',5',6'-hexahydro-[2,3']bipyridinyl-4'-yl]-urea (Ex-201)

### Synthesis of trans-1-(4-chloro-phenyl)-3-[5-methoxy-1'-(1-methoxy-cyclopropanecarbonyl)-1',2',3',4',5',6'-hexahydro-[2,3']bipyridinyl-4'-yl]-urea

To a stirred solution of 1-methoxy-cyclopropanecarboxylic acid (36 mg, 0.25 mmol, 1.0 eq) in dry THF (8 ml) were added EDCI-HCl (72 mg, 0.375 mmol, 1.5 eq) and HOBt (51 mg, 0.375 mmol, 1.5 eq) in ice-cold condition followed by addition of TEA (106 µl, 0.75 mmol, 3 eq) and HCl salt of *trans*-1-(4-chloro-phenyl)-3-(5-methoxy-1',2',3',4',5',6'-hexahydro-[2,3']bipyridinyl-4'-yl)-urea hydrochloride **(BB-III-HCl,** 100 mg, 0.25 mmol, 1.0 eq) and stirred at RT for 18 h. The reaction mixture was diluted with EtOAc and washed with water and brine. The organic layer was dried over anhyd. Na₂SO₄ and concentrated under reduced pressure to obtain *trans*-1-(4-chlorophenyl)-3-[5-methoxy-1'-(1-methoxy-cyclopropanecarbonyl)-1',2',3',4',5',6'-hexahydro-[2,3']bipyridinyl-4'-yl]-urea (110 mg, 65% crude yield) as yellowish sticky.
LC-MS (Method 7): m/z [M+H]⁺ = 459.0 (exact mass calc. = 458.17).

### Synthesis of trans-1-(4-chloro-phenyl)-3-[5-methoxy-1'-(1-methoxy-cyclopropylmethyl)-1',2',3',4',5',6'-hexahydro-[2,3']bipyridinyl-4'-yl]-urea (Ex-201)

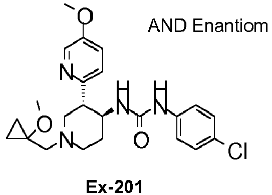

To a stirred solution of *trans*-1-(4-chloro-phenyl)-3-[5-methoxy-1'-(1-methoxy-cyclopropanecarbonyl)-1',2',3',4',5',6'-hexahydro-[2,3']bipyridinyl-4'-yl]-urea (100 mg, 0.218 mmol, 1.0 eq) in dry THF (5 ml) at ice-cold condition was added BH₃-DMS (517 µl, 4.36 mmol, 20.0 eq) and stirred at RT for 18 h. After that the reaction mixture was quenched with MeOH (1 ml). The reaction mixture was diluted with EtOAc and washed with water and brine. The organic layer was dried over anhyd. Na₂SO₄ and concentrated under reduced pressure to obtain crude product which was purified by reverse phase prep-HPLC purification to obtain *trans*-1-(4-chloro-phenyl)-3-[5-methoxy-1'-(1-methoxy-cyclopropylmethyl)-1',2',3',4',5',6'-hexahydro-[2,3']bipyridinyl-4'-yl]-urea (**Ex-201**, 11 mg, 11% yield) as off white solid
LC-MS (Method 3): m/z [M+H]⁺ = 445.2 (exact mass calc. = 444.19).
¹H-NMR (400 MHz, DMSO-d6): δ 8.32 (s, 1 H), 8.17 (br s, 1 H), 7.28-7.15 (m, 6 H), 5.94 (d, *J* = 8 Hz, 1 H), 3.81-3.78 (m, 1 H), 3.74 (s, 3 H), 3.17 (s, 3 H), 3.02 (d, *J* = 8 Hz, 2 H), 2.88-2.83 (m, 1 H), 2.22-2.10 (m, 2 H), 1.95 (d, *J* = 12 Hz, 1 H), 1.51-1.42 (m, 1 H), 0.62 (s, 2 H), 0.42-0.34 (m, 2 H).

### trans-1-(4-chloro-phenyl)-3-[5-methoxy-1'-(3-methoxy-cyclobutylmethyl)-1',2',3',4',5',6'-hexahydro-[2,3']bipyridinyl-4'-yl]-urea (Ex-202)

### Synthesis of trans-1-(4-chloro-phenyl)-3-[5-methoxy-1'-(3-methoxy-cyclobutanecarbonyl)-1',2',3',4',5',6'-hexahydro-[2,3']bipyridinyl-4'-yl]-urea

To a stirred solution of 3-methoxy-cyclobutanecarboxylic acid (33 mg, 0.25 mmol, 1.0 eq) in dry THF (5 ml) were added EDCI-HCl (72 mg, 0.375 mmol, 1.5 eq) and HOBt (51 mg, 0.375 mmol, 1.5 eq) in ice-cold condition followed by addition of TEA (106 µl, 0.75 mmol, 3 eq) and *trans*-1-(4-chloro-phenyl)-3-(5-methoxy-1',2',3',4',5',6'-hexahydro-[2,3']bipyridinyl-4'-yl)-urea hydrochloride (**BB-III-HCl,** 100 mg, 0.25 mmol, 1.0 eq) and stirred at RT for 18 h. The reaction mixture was diluted with EtOAc and washed with water and brine. The organic layer was dried over anhyd. Na₂SO₄ and concentrated under reduced pressure to obtain *trans*-1-(4-chlorophenyl)-3-[5-methoxy-1'-(1-methoxy-cyclopropanecarbonyl)-1',2',3',4',5',6'-hexahydro-[2,3']bipyridinyl-4'-yl]-urea (140 mg, 100% crude yield) as reddish sticky.
LC-MS (Method 2): m/z [M+H]⁺ = 473.6 (exact mass calc. = 472.19).

### Synthesis of trans-1-(4-chloro-phenyl)-3-[5-methoxy-1'-(3-methoxy-cyclobutylmethyl)-1',2',3',4',5',6'-hexahydro-[2,3']bipyridinyl-4'-yl]-urea (Ex-202)

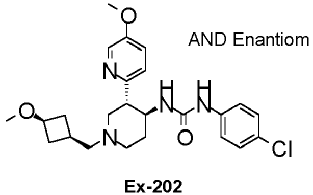

To a stirred solution of *trans*-1-(4-chloro-phenyl)-3-[5-methoxy-1'-(1-methoxy-cyclopropanecarbonyl)-1',2',3',4',5',6'-hexahydro-[2,3']bipyridinyl-4'-yl]-urea (140 mg, 0.296 mmol, 1.0 eq) in dry THF (5 ml) at ice-cold condition was added BH₃-DMS (1.5 ml, 15.79 mmol, 53 eq) and stirred at RT for 18 h. After that the reaction mixture was quenched with MeOH (1 ml). The reaction mixture was diluted with EtOAc and washed with water and brine. The organic layer was dried over anhyd. Na₂SO₄ and concentrated under reduced pressure to obtain crude product which was purified by reverse phase prep-HPLC purification to obtain *trans*-1-(4-chloro-phenyl)-3-[5-methoxy-1'-(3-methoxy-cyclobutylmethyl)-1',2',3',4',5',6'-hexahydro-[2,3']bipyridinyl-4'-yl]-urea (**Ex-202,** 15 mg, 11% yield) as white solid.
LC-MS (Method 3): m/z [M+H]⁺ = 459.2 (exact mass calc. = 458.21).
¹H-NMR (400 MHz, DMSO-d6): δ 8.31 (s, 1 H), 8.19-8.18 (m, 1 H), 7.30-7.17 (m, 6 H), 5.93 (d, *J* = 8 Hz, 1 H), 3.89-3.80 (m, 1 H), 3.77 (s, 3 H), 3.67-3.63 (m, 1 H), 3.08 (s, 3 H), 2.89-2.79 (m, 3 H), 2.39-2.28 (m, 4 H), 2.23-1.86 (m, 4 H), 1.49-1.36 (m, 3 H).

### Synthesis of trans-1-(4-chloro-phenyl)-3-[1'-(3-hydroxy-cyclobutylmethyl)-5-methoxy-1',2',3',4',5',6'-hexahydro-[2,3']bipyridinyl-4'-yl]-urea (Ex-203)

### Synthesis of trans-1-(4-chloro-phenyl)-3-[1'-(3-hydroxy-cyclobutanecarbonyl)-5-methoxy-1',2',3',4',5',6'-hexahydro-[2,3']bipyridinyl-4'-yl]-urea

To a stirred solution of 3-hydroxy-cyclobutanecarboxylic acid (52 mg, 0.45 mmol, 1.0 eq) in dry THF (10 ml) were added EDCI-HCl (108 mg, 0.567 mmol, 1.5 eq) and HOBt (77 mg, 0.567 mmol, 1.5 eq) in ice-cold condition followed by addition of TEA (158 µl, 1.134 mmol, 3 eq) and HCl salt of *trans*-1-(4-chloro-phenyl)-3-(5-methoxy-1',2',3',4',5',6'-hexahydro-[2,3']bipyridinyl-4'-yl)-urea hydrochloride **(BB-III-HCl,** 150 mg, 0.378 mmol, 1.0 eq) and stirred at RT for 18 h. The reaction mixture was diluted with EtOAc and washed with water and brine. The organic layer was dried over anhyd. Na₂SO₄ and concentrated under reduced pressure to obtain crude *trans*-1-(4-chloro-phenyl)-3-[1'-(3-hydroxy-cyclobutanecarbonyl)-5-methoxy-1',2',3',4',5',6'-hexahydro-[2,3']bipyridinyl-4'-yl]-urea (120 mg, 69% crude yield) as reddish sticky.
LC-MS (Method 1): m/z [M+H]⁺ = 459.2 (exact mass calc. = 458.17).

### Synthesis of trans-1-(4-chloro-phenyl)-3-[1'-(3-hydroxy-cyclobutylmethyl)-5-methoxy-1',2',3',4',5',6'-hexahydro-[2,3']bipyridinyl-4'-yl]-urea (Ex-203)

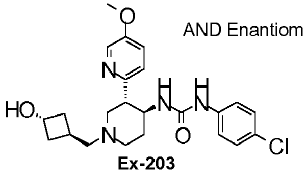

To a stirred solution of *trans*-1-(4-chloro-phenyl)-3-[1'-(3-hydroxy-cyclobutanecarbonyl)-5-methoxy-1',2',3',4',5',6'-hexahydro-[2,3']bipyridinyl-4'-yl]-urea (100 mg, 0.218 mmol, 1.0 eq) in dry THF (10 ml) at ice-cold condition was added BH₃-DMS (327 mg, 4.366 mmol, 20 eq) and stirred at RT for 18 h. After that the reaction mixture was quenched with MeOH (1 ml). The reaction mixture was diluted with EtOAc and washed with water and brine. The organic layer was dried over anhyd. Na₂SO₄ and concentrated under reduced pressure to obtain crude product which was purified by reverse phase prep-HPLC purification to obtain *trans*-1-(4-chloro-phenyl)-3-[1'-(3-hydroxy-cyclobutylmethyl)-5-methoxy-1',2',3',4',5',6'-hexahydro-[2,3']bipyridinyl-4'-yl]-urea (**Ex-203,** 15 mg, 15% yield) as white solid.
LC-MS (Method 3): m/z [M+H]⁺ = 445.2 (exact mass calc. = 444.19).
¹H-NMR (400 MHz, DMSO-d6): δ 8.33 (s, 1 H), 8.18 (d, *J* = 2 Hz, 1 H), 7.30-7.18 (m, 6 H), 5.95 (d, *J* = 8 Hz, 1 H), 4.87 (d, *J* = 8 Hz, 1 H), 4.18-4.13 (m, 1 H), 3.82-3.79 (m, 1 H), 3.76 (s, 3 H), 2.91-2.83 (m, 3 H), 2.42-2.26 (m, 3 H), 2.14-1.88 (m, 7 H), 1.50-1.40 (m, 1 H).

### Synthesis of trans-1-(5-methoxy-1'-oxetan-3-ylmethyl-1',2',3',4',5',6'-hexahydro-[2,3']bipyridinyl-4'-yl)-3-(3-methyl-isothiazol-5-yl)-urea (Ex-204)

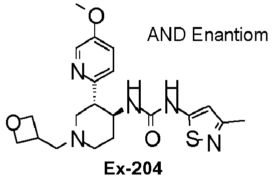

To a stirred solution of *trans*-1-(5-methoxy-1',2',3',4',5',6'-hexahydro-[2,3']bipyridinyl-4'-yl)-3-(3-methyl-isothiazol-5-yl)-urea hydrochloride (**BB-IV-HCl,** 130 mg, 0.338 mmol, 1 eq) in ACN (5 ml) were added DIPEA (351 µl, 2.02 mmol, 6 eq) followed by the addition of 3-iodomethyl-oxetane (67 mg, 0.338 mmol, 1.0 eq) and stirred at reflux for 16 h. The reaction mixture was diluted with 10% MeOH-DCM and washed with water and brine. The organic layer was dried over anhyd. Na₂SO₄ and concentrated under reduced pressure to obtain crude product which was purified by reverse phase purification to obtain *trans*-1-(5-methoxy-1'-oxetan-3-ylmethyl-1',2',3',4',5',6'-hexahydro-[2,3']bipyridinyl-4'-yl)-3-(3-methyl-isothiazol-5-yl)-urea (**Ex-204,** 12 mg, 9% yield) as off white solid.
LC-MS (Method 3): m/z [M+H]⁺ = 418.2 (exact mass calc. = 417.18).
¹H-NMR (400 MHz, DMSO-d6): δ 9.85 (s, 1 H), 8.18 (s, 1 H), 7.35-7.27 (m, 1 H), 7.25-7.19 (m, 1 H), 6.48-6.35 (m, 2 H), 4.62-4.58 (m, 2 H), 4.28-4.22 (m, 2 H), 3.88-3.78 (m, 1 H), 3.76 (s, 3 H), 3.33-3.25 (m, 1 H), 3.21-3.13 (m, 1 H), 2.98-2.86 (m, 1 H), 2.82-2.74 (m, 2 H), 2.68-2.58 (m, 2 H), 2.19 (s, 3 H), 2.12-2.05 (m, 1 H), 1.98-1.90 (m, 1 H), 1.55-1.45 (m, 1 H).

### Synthesis of trans-1-(4-chloro-phenyl)-3-[1'-(3-hydroxy-cyclobutylmethyl)-5-methoxy-1',2',3',4',5',6'-hexahydro-[2,3']bipyridinyl-4'-yl]-urea (Ex-205)

### Synthesis of trans-1-(4-chloro-phenyl)-3-[1'-(3-hydroxy-cyclobutanecarbonyl)-5-methoxy-1',2',3',4',5',6'-hexahydro-[2,3']bipyridinyl-4'-yl]-urea

To a stirred solution of *cis*-3-hydroxy-cyclobutanecarboxylic acid (52 mg, 0.45 mmol, 1.0 eq) in dry THF (10 ml) were added EDCI-HCl (108 mg, 0.567 mmol, 1.5 eq) and HOBt (77 mg, 0.567 mmol, 1.5 eq) in ice-cold condition followed by addition of TEA (158 µl, 1.134 mmol, 3 eq) and *trans*-1-(4-chloro-phenyl)-3-(5-methoxy-1',2',3',4',5',6'-hexahydro-[2,3']bipyridinyl-4'-yl)-urea hydrochloride (**BB-III-HCl,** 150 mg, 0.378 mmol, 1.0 eq) and stirred at RT for 18 h. The reaction mixture was diluted with EtOAc and washed with water and brine. The organic layer was dried over anhyd. Na₂SO₄ and concentrated under reduced pressure to obtain crude material which was purified by combi flash column chromatography to obtain *trans*-1-(4-chloro-phenyl)-3-[1'-(3-hydroxy-cyclobutanecarbonyl)-5-methoxy-1',2',3',4',5',6'-hexahydro-[2,3']bipyridinyl-4'-yl]-urea (110 mg, 63% crude yield) as off white solid.
LC-MS (Method 2): m/z [M+H]⁺ = 459.4 (exact mass calc. = 458.17).

### Synthesis of trans-1-(4-chloro-phenyl)-3-[1'-(3-hydroxy-cyclobutylmethyl)-5-methoxy-1',2',3',4',5',6'-hexahydro-[2,3']bipyridinyl-4'-yl]-urea (Ex-205)

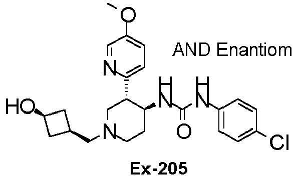

To a stirred solution of *trans*-1-(4-chloro-phenyl)-3-[1'-(3-hydroxy-cyclobutanecarbonyl)-5-methoxy-1',2',3',4',5',6'-hexahydro-[2,3']bipyridinyl-4'-yl]-urea (110 mg, 0.23 mmol, 1.0 eq) in dry THF (15 ml) at ice-cold condition was added BH₃-DMS (445 µl, 4.79 mmol, 20 eq) and stirred at RT for 18 h. After that the reaction mixture was quenched with MeOH (1 ml). The reaction mixture was diluted with EtOAc and washed with water and brine. The organic layer was dried over anhyd. Na₂SO₄ and concentrated under reduced pressure to obtain crude product which was purified by reverse phase prep-HPLC purification to obtain *trans*-1-(4-chloro-phenyl)-3-[1'-(3-hydroxy-cyclobutylmethyl)-5-methoxy-1',2',3',4',5',6'-hexahydro-[2,3']bipyridinyl-4'-yl]-urea (**Ex-205,** 16 mg, 15% yield) as off white solid.
LC-MS (Method 7): m/z [M+H]⁺ = 445.2 (exact mass calc. = 444.19).
¹H-NMR (400 MHz, DMSO-d6): δ 8.35 (s, 1 H), 8.18 (s, 1 H), 7.30-7.18 (m, 6 H), 5.97 (d, *J* = 4 Hz, 1 H), 4.87 (d, *J* = 4 Hz, 1 H), 3.88-3.80 (m, 2 H), 3.76 (s, 3 H), 2.92-2.74 (m, 3 H), 2.39-2.22 (m, 4 H), 2.15-1.95 (m, 3 H), 1.88-1.74 (m, 1 H), 1.51-1.35 (m, 3 H).

### Synthesis of trans-1-(4-chlorophenyl)-3-1-(2-hydroxyethyl)-3-(5-methoxypyridin-2-yl)piperidin-4-yl]urea (Ex-206)

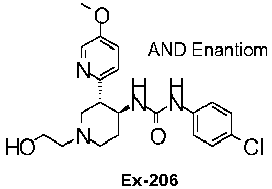

To the solution of *trans*-1-(4-chlorophenyl)-3-(3-(5-methoxypyridin-2-yl)piperidin-4-yl)urea trifluoroacetate (**BB-III-TFA**, 0.1 g, 0.2109 mmol, 1 eq) in ACN (10 ml), K₂CO₃ (0.145 g,1.054 mmol,5 eq), followed by 2-bromoethan-1-ol (0.031 g,0.2531 mmol, 1.2 eq), was added at RT. The reaction was allowed to stir at 90 °C for 16 h. After completion of reaction (checked by TLC, 5% MeOH in DCM, R_{f}:0.3), the reaction mixture was concentrated to get crude which was purified by column chromatography using 0-10% MeOH in DCM and 230-400 silica gel to get *trans*-1-(4-chlorophenyl)-3-1-(2-hydroxyethyl)-3-(5-methoxypyridin-2-yl)piperidin-4-yl]urea (**Ex-206,** 0.015 g, 17.56 %).
LC-MS (Method 7): m/z [M+H]⁺ = 405.2 (exact mass calc. = 404.06).
¹H-NMR (400 MHz, DMSO-d6): δ 8.31 (s, 1H), 8.19 (s, 1H), 7.31- 7.28(m, 3H), 7.24-7.18 (m, 2H), 5.99-5.97 (d,J=8.12, 1H),4.38(s,1H),3.84-3.81(m,1H), 3.78-3.77 (s, 3H), 3.50-3.41 (m, 2H), 2.92-2.84 (m, 3H),2.43(s,2H), 2.23-2.15 (m, 1H), 2.00-1.97 (d, J=9.6, 1H), 1.50-1.44 (m, 1H).

### Synthesis of trans-1-(4-chloro-phenyl)-3-[1'-(3-hydroxy-3-methyl-cyclobutylmethyl)-5-methoxy-1',2',3',4',5',6'-hexahydro-[2,3']bipyridinyl-4'-yl]-urea (Ex-207)

### Synthesis of trans-1-(4-chloro-phenyl)-3-[1'-(3-hydroxy-3-methyl-cyclobutanecarbonyl)-5-methoxy-1',2',3',4',5',6'-hexahydro-[2,3']bipyridinyl-4'-yl]-urea

To a stirred solution of 3-hydroxy-3-methyl-cyclobutanecarboxylic acid (78 mg, 0.605 mmol, 1.2 eq) in dry THF (15 ml) were added EDCI-HCl (145 mg, 0.756 mmol, 1.5 eq) and HOBt (102 mg, 0.756 mmol, 1.5 eq) in ice-cold condition followed by addition of TEA (210 µl, 1.513 mmol, 3 eq) and *trans*-1-(4-chloro-phenyl)-3-(5-methoxy-1',2',3',4',5',6'-hexahydro-[2,3']bipyridinyl-4'-yl)-urea hydrochloride (**BB-III-HCl,** 200 mg, 0.504 mmol, 1.0 eq) and stirred at RT for 18 h. The reaction mixture was diluted with EtOAc and washed with water and brine. The organic layer was dried over anhyd. Na₂SO₄ and concentrated under reduced pressure to obtain crude which was purified by silica gel (100-200 mesh) column chromatography (2-3% MeOH in DCM) to obtain *trans*-1-(4-chlorophenyl)-3-[1'-(3-hydroxy-3-methyl-cyclobutanecarbonyl)-5-methoxy-1',2',3',4',5',6'-hexahydro-[2,3']bipyridinyl-4'-yl]-urea (150 mg, 63% crude yield) as yellow solid.
LC-MS (Method 7): m/z [M+H]⁺ = 473.1 (exact mass calc. = 472.19).

### Synthesis of trans-1-(4-chloro-phenyl)-3-[1'-(3-hydroxy-3-methyl-cyclobutylmethyl)-5-methoxy-1',2',3',4',5',6'-hexahydro-[2,3']bipyridinyl-4'-yl]-urea (Ex-207)

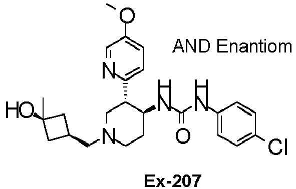

To a stirred solution of *trans*-1-(4-chloro-phenyl)-3-[1'-(3-hydroxy-3-methyl-cyclobutanecarbonyl)-5-methoxy-1',2',3',4',5',6'-hexahydro-[2,3']bipyridinyl-4'-yl]-urea (150 mg, 0.317 mmol, 1.0 eq) in dry THF (50 ml) at ice-cold condition was added BH₃-DMS (595 µl, 6.34 mmol, 20 eq) and stirred at RT for 18 h. After that the reaction mixture was quenched with MeOH (1 ml). The reaction mixture was diluted with EtOAc and washed with water and brine. The organic layer was dried over anhyd. Na₂SO₄ and concentrated under reduced pressure to obtain crude product which was purified by reverse phase prep-HPLC purification to obtain *trans*-1-(4-chloro-phenyl)-3-[1'-(3-hydroxy-3-methyl-cyclobutylmethyl)-5-methoxy-1',2',3',4',5',6'-hexahydro-[2,3']bipyridinyl-4'-yl]-urea
(**Ex-207,** 40 mg, 28% yield) as white solid.
LC-MS (Method 7): m/z [M+H]⁺ = 459.2 (exact mass calc. = 458.21).
¹H-NMR (DMSO-d6): δ 8.38 (s, 1 H), 8.18 (s, 2 H), 7.30-7.17 (m, 5 H), 6.03-6.01 (d, *J* = 8 Hz, 1 H), 4.80-4.56 (br m, 1 H), 3.78-3.78 (m, a H), 3.76 (s, 3 H), 3.40-3.39 (m, 1 H), 2.86-2.82 (m, 3 H), 2.37 (d, *J* = 6 Hz, 2 H), 2.20-2.18 (m, 1 H), 2.06-1.92 (m, 3 H), 1.61 (d, *J* = 8 Hz, 2 H), 1.47 (d, *J* = 6 Hz, 1 H), 1.20 (s, 3 H).

### Synthesis of trans-1-(4-chloro-phepyl)-3-[3-fluoro-1'-(2-hydroxy-2-methyl-propyl)-5-methoxy-1',2',3',4',5',6'-hexahydro-[2,3']bipyridinyl-4'-yl]-urea (Ex-208)

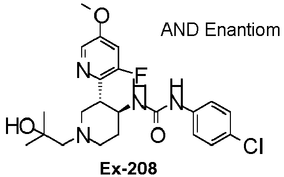

To a stirred solution of *trans*-1-(4-chloro-phenyl)-3-(3-fluoro-5-methoxy-1',2',3',4',5',6'-hexahydro-[2,3']bipyridinyl-4'-yl)-urea (**BB-XIII-HCl**, 70 mg, 0.168 mmol, 1 eq) in EtOH (3 ml) were added 2,2-dimethyloxirane (40 mg, 0.506 mmol, 3 eq) and stirred at 90 °C for 16 h. The reaction mixture was diluted with 10% MeOH-DCM and washed with water and brine. The organic layer was dried over anhyd. Na₂SO₄ and concentrated under reduced pressure to obtain crude product which was purified by reverse phase purification to obtain *trans-*1-(4-chloro-phenyl)-3-[3-fluoro-1'-(2-hydroxy-2-methyl-propyl)-5-methoxy-1',2',3',4',5',6'-hexahydro-[2,3']bipyridinyl-4'-yl]-urea (**Ex-208,** 40 mg, 53% yield) as off white solid.
LC-MS (Method 7): m/z [M+H]⁺ = 451.4 (exact mass calc. = 450.18).
¹H-NMR (DMSO-d6): δ 8.31 (s, 1 H), 8.11 (d, *J* = 4 Hz, 1 H), 7.34-7.29 (m, 3 H), 7.18 (d, *J* = 8 Hz, 2 H), 5.91 (d, *J* = 8 Hz, 1 H), 4.05 (s, 1 H), 3.89 (d, *J* = 8 Hz, 1 H), 3.79 (s, 3 H), 3.16 (d, *J* = 5 Hz, 1 H), 2.96 (d, *J* = 12 Hz, 2 H), 2.44-2.39 (m, 1 H), 2.28-2.19 (m, 3 H), 1.94 (d, *J* = 8 Hz, 1 H), 1.53 (d, *J* = 10 Hz, 1 H), 1.07 (s, 6 H).

### Synthesis of trans-1-(4-chloro-phenyl)-3-(1'-cyclopropylmethyl-3-fluoro-5-methoxy-1',2',3',4',5',6'-hexahydro-[2,3']bipyridinyl-4'-yl)-urea (Ex-209)

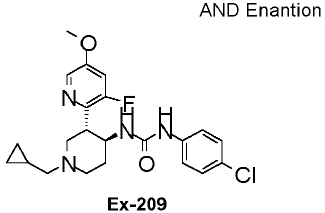

To a stirred solution of *trans*-1-(4-chloro-phenyl)-3-(3-fluoro-5-methoxy-1',2',3',4',5',6'-hexahydro-[2,3']bipyridinyl-4'-yl)-urea hydrochloride (**BB-XIII-HCl**, 70 mg, 0.166 mmol, 1 eq) in ACN (5 ml) were added Cs₂CO₃ (110 mg, 0.336 mmol, 2.0 eq) followed by the addition of bromomethyl-cyclopropane (28 mg, 0.202 mmol, 1.2 eq) and stirred at RT for 18 h. The reaction mixture was diluted with 10% MeOH-DCM and washed with water and brine. The organic layer was dried over anhyd. Na₂SO₄ and concentrated under reduced pressure to obtain crude product which was purified by reverse phase prep HPLC purification to obtain *trans*-1-(4-chlorophenyl)-3-(1'-cyclopropylmethyl-3-fluoro-5-methoxy-1',2',3',4',5',6'-hexahydro-[2,3']bipyridinyl-4'-yl)-urea (Ex-**209**, 32 mg, 44% yield) as off white solid.
LC-MS (Method 7): m/z [M+H]⁺ = 433.4 (exact mass calc. = 432.17).
¹H-NMR (DMSO-d6): δ 8.32 (s, 1 H), 8.12 (d, *J* = 4 Hz, 1 H), 7.33 (dd, *J* = 2,2 Hz, 1 H), 7.27 (d, *J* = 8 Hz, 2 H), 7.18 (d, *J* = 8 Hz, 2 H), 5.94 (d, *J* = 8 Hz, 1 H), 3.90 (d, *J* = 12 Hz, 1 H), 3.80 (s, 3 H), 3.13 (t, *J* = 12 Hz, 1 H), 3.02 (d, *J* = 8 Hz, 1 H), 2.94 (d, *J* = 8 Hz, 1 H), 2.32-2.20 (m, 3 H), 2.08-1.99 (m, 2 H), 1.54-1.50 (m, 1 H), 0.81 (d, *J* = 4 Hz, 1 H), 0.43 (d, *J* = 8 Hz, 2 H), 0.05-0.03 (m, 2 H).

### Synthesis of trans-1-(4-chloro-phenyl)-3-[5-methoxy-1'-(2-methyl-pyrimidm-4-yl)-1',2',3',4',5',6'-hexahydro-[2,3']bipyridinyl-4'-yl]-urea (Ex-210)

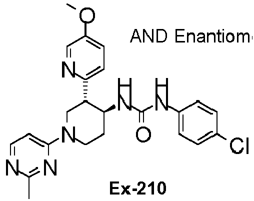

To a stirred solution of *trans*-1-(4-chloro-phenyl)-3-(5-methoxy-1',2',3',4',5',6'-hexahydro-[2,3']bipyridinyl-4'-yl)-urea hydrochloride **(BB-III-HCl,** 100 mg, 0.2522 mmol, 1 eq) in *n*-BuOH (3 ml) were added DIPEA (130 µl, 0.756 mmol, 3.0 eq) followed by the addition of 4-chloro-2-methyl-pyrimidine (39 mg, 0.302 mmol, 1.2 eq) and stirred at 130 °C for a period of 18 h. The reaction mixture was diluted with EA and washed with water and brine. The organic layer was dried over anhyd. Na₂SO₄ and concentrated under reduced pressure to obtain crude product which was purified by reverse phase prep HPLC purification to obtain *trans*-1-(4-chloro-phenyl)-3-[5-methoxy-1'-(2-methyl-pyrimidin-4-yl)-1',2',3',4',5',6'-hexahydro-[2,3']bipyridinyl-4'-yl]-urea (**Ex-210**) 16 mg, 14% yield) as off white solid.
LC-MS (Method 7): m/z [M+H]⁺ = 453.2 (exact mass calc. = 452.17).
¹H-NMR (DMSO-d6): δ 8.37 (s, 1 H), 8.24 (s, 1 H), 8.07 (d, *J* = 8 Hz, 1 H), 7.40-7.29 (m, 4 H), 7.19 (d, *J* = 8 Hz, 2 H), 6.68 (d, *J* = 8 Hz, 1 H), 6.03 (d, *J* = 12 Hz, 1 H), 4.51-4.43 (m, 2 H), 4.12 (d, *J* = 12 Hz, 1 H), 3.79 (s, 3 H), 3.14-3.01 (m, 2 H), 2.84-2.78 (m, 1 H), 2.34 (s, 3 H), 2.07 (d, *J* = 8 Hz, 1 H), 1.46-1.38 (m, 1 H).

### Synthesis of trans-1-[5-methoxy-1'-(2-methyl-pyrimidin-4-yl)-1',2',3',4',5',6'-hexahydro-[2,3']bipyridinyl-4'-yl]-3-(3-methyl-isothiazol-5-yl)-urea (Ex-211)

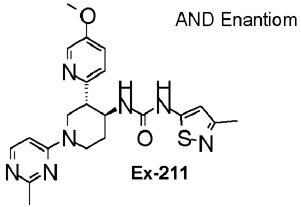

To a stirred solution of *trans*-1-(5-Methoxy-1',2',3',4',5',6'-hexahydro-[2,3']bipyridinyl-4'-yl)-3-(3-methyl-isothiazol-5-yl)-urea hydrochloride (**BB-IV-HCl,** 150 mg, 0.391 mmol, 1 eq) in *n*-BuOH (3 ml) were added DIPEA (200 µl, 1.173 mmol, 3.0 eq) followed by the addition of 4-chloro-2-methyl-pyrimidine (60 mg, 0.469 mmol, 1.2 eq) and stirred at 130 °C for a period of 18 h. The reaction mixture was diluted with EA and washed with water and brine. The organic layer was dried over anhyd. Na₂SO₄ and concentrated under reduced pressure to obtain crude product which was purified by reverse phase prep-HPLC purification to obtain *trans*-1-[5-methoxy-1'-(2-methyl-pyrimidin-4-yl)-1',2',3',4',5',6'-hexahydro-[2,3']bipyridinyl-4'-yl]-3-(3-methyl-isothiazol-5-yl)-urea (**Ex-211**, 12 mg, 7% yield) as off white solid.
LC-MS (Method 3): m/z [M+H]⁺ = 440.2 (exact mass calc. = 439.18).
¹H-NMR (DMSO-d6): δ 9.94 (s, 1 H), 8.23 (d, *J* = 4 Hz, 1 H), 8.08 (d, *J* = 8 Hz, 1 H), 7.34-7.26 (m, 2 H), 6.71-6.69 (d, *J* = 8 Hz, 1 H), 6.70 (d, *J* = 4 Hz, 1 H), 6.59-5.57 (br m, 1 H), 6.39 (s, 1 H), 4.51-4.42 (s, 2 H), 4.13 (d, *J* = 8 Hz, 1 H), 3.78 (s, 3 H), 3.15-3.0 (m, 2 H), 2.91-2.86 (m, 1 H), 2.35 (m, 3 H), 2.19 (s, 3 H), 2.03 (m, 1 H), 1.51-1.45 (m, 1 H), 1.33 (s, 1 H).

### Synthesis of trans-1-(4-chloro-phenyl)-3-[1'-(3-dimethylamino-propyl)-3-fluoro-5-methoxy-1',2',3',4',5',6'-hexahydro-[2,3']bipyridinyl-4'-yl]-urea (Ex-212)

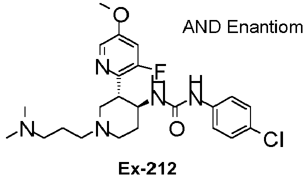

To a stirred solution of *trans*-1-(4-chloro-phenyl)-3-(3-fluoro-5-methoxy-1',2',3',4',5',6'-hexahydro-[2,3']bipyridinyl-4'-yl)-urea hydrochloride (**BB-XIII-HCl**, 90 mg, 0.217 mmol, 1 eq) in ACN (5 ml) were added DIPEA (227 µl, 1.30 mmol, 6 eq) followed by the addition of (3-chloro-propyl)-dimethyl-amine (32 mg, 0.26 mmol, 1.2 eq) and stirred at RT for 24 h. The reaction mixture was diluted with 10% MeOH-DCM and washed with water and brine. The organic layer was dried over anhyd. Na₂SO₄ and concentrated under reduced pressure to obtain crude product which was purified by reverse phase prep-HPLC purification to obtain *trans*-1-(4-chlorophenyl)-3-[1'-(3-dimethylamino-propyl)-3-fluoro-5-methoxy-1',2',3',4',5',6'-hexahydro-[2,3']bipyridinyl-4'-yl]-urea (**Ex-212,** 10 mg, 10% yield) as off white solid.
LC-MS (Method 7): m/z [M+H]⁺ = 464.3 (exact mass calc. = 463.22).
¹H-NMR (DMSO-d6): δ 8.30 (s, 1 H), 8.12 (s, 1 H), 7.33 (d, *J* = 8 Hz, 1 H), 7.27 (d, *J* = 8 Hz, 2 H), 7.18 (d, *J* = 8 Hz, 2 H), 5.93 (d, *J* = 8 Hz, 1 H), 3.90 (d, *J* = 12 Hz, 1 H), 3.80 (s, 3 H), 3.12 (t, *J* = 8 Hz, 1 H), 2.90 (d, *J* = 12 Hz, 1 H), 2.81 (d, *J* = 12 Hz, 1 H), 2.30 (t, *J* = 8 Hz, 2 H), 2.25-2.16 (m, 2 H), 2.09 (s, 6 H), 2.03-1.97 (m, 2 H), 1.54-1.42 (m, 4 H).

### Synthesis of trans-1-(4-chloro-phenyl)-3-[1'-(2-dimethylamino-ethyl)-3-fluoro-5-methoxy-1',2',3',4',5',6'-hexahydro-[2,3']bipyridinyl-4'-yl]-urea (Ex-213)

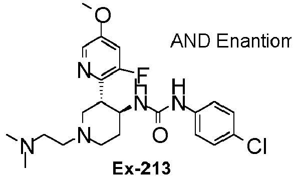

To a stirred solution of *trans*-1-(4-chloro-phenyl)-3-(3-fluoro-5-methoxy-1',2',3',4',5',6'-hexahydro-[2,3']bipyridinyl-4'-yl)-urea hydrochloride (**BB-XIII-HCl**, 70 mg, 0.168 mmol, 1 eq) in ACN (5 ml) were added DIPEA (174 µl, 1.008 mmol, 6 eq) followed by the addition of (2-chloro-ethyl)-dimethyl-amine (18 mg, 0.168 mmol, 1 eq) and stirred at RT for 24 h. The reaction mixture was diluted with 10% MeOH-DCM and washed with water and brine. The organic layer was dried over anhyd. Na₂SO₄ and concentrated under reduced pressure to obtain crude product which was purified by reverse phase prep-HPLC purification to obtain *trans*-1-(4-chlorophenyl)-3-[1'-(2-dimethylamino-ethyl)-3-fluoro-5-methoxy-1',2',3',4',5',6'-hexahydro-[2,3']bipyridinyl-4'-yl]-urea **(Ex-213,** 10 mg, 13% yield) as off white solid.
LC-MS (Method 8): m/z [M+H]⁺ = 450.5 (exact mass calc. = 449.20).
¹H-NMR (DMSO-d6): δ 8.32 (s, 1 H), 8.12 (s, 1 H), 7.32 (d, *J* = 12 Hz, 1 H), 7.27 (d, *J* = 8 Hz, 2 H), 7.18 (d, *J* = 8 Hz, 2 H), 5.95 (d, *J* = 8 Hz, 1 H), 3.90 (d, *J* = 12 Hz, 1 H), 3.80 (s, 3 H), 3.10 (t, *J* = 10 Hz, 1 H), 2.92 (d, *J* = 12 Hz, 1 H), 2.84 (d, *J* = 12 Hz, 1 H), 2.42-2.38 (m, 2 H), 2.32-2.23 (m, 4 H), 2.11-2.03 (m, 6 H), 1.98 (d, *J* = 12 Hz, 1 H), 1.54-1.45 (m, 1 H).

### Synthesis of trans-1-1-(2,2-difluoroethyl)-3-(5-methoxypyridin-2-yl)piperidin-4-yl]-3-(3-methyl-1,2-thiazol-5-yl)urea (Ex-214)

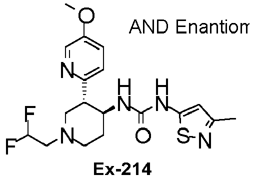

To the stirring solution of *trans*-1-[3-(5-methoxypyridin-2-yl)piperidin-4-yl]-3-(3-methyl-1,2-thiazol-5-yl)urea trifluoroacetate (**BB-IV-TFA,** 0.14g, 0.3033 mmol, 1.0 eq) in acetonitrile (20 ml), K₂CO₃ (0.209 g, 1.516 mmol, 5.0 eq) was added at 0 °C and allowed to stir for 10 minutes, then compound 2,2-difluoroethyl trifluoromethanesulfonate (0.0779g, 0.3640 mmol, 1.2 eq) was added and again allowed to stir for 24 hours. After completion of reaction water was added, extracted with ethyl acetate (3x50 ml). Ethyl acetate part was collected, dried over Na₂SO₄ and evaporated to dryness to get crude, which was purified by column chromatography to get *trans*-1-1-(2,2-difluoroethyl)-3-(5-methoxypyridin-2-yl)piperidin-4-yl]-3-(3-methyl-1,2-thiazol-5-yl)urea (**Ex-214**, 0.036g, 28.84 %).
LC-MS (Method 3): m/z [M+H]⁺ = 412.3 (exact mass calc. = 411.2).
¹H-NMR (400 MHz, DMSO-d6): δ 9.85 (s, 1H), 8.19-8.18 (d, 1H), 7.30-7.28 (m, 1H), 7.23-7.20 (d, *J=* 8.56 Hz, 1H), 6.47 (m, 1H), 6.14-5.98 (s, 1H), 3.85-3.82 (m, 1H), 3.79 (s, 1H), 2.98-2.92 (m, 3H), 2.80-2.71 (m, 2H), 2.42-2.31 (m, 2H), 2.19 (s, 3H), 1.96-1.94 (m, 1H), 1.59-1.53 (m, 1H).

### Synthesis of trans-2-4-{[(4-chlorophenyl)carbamoyl]amino}-3-(5-methoxypyridin-2-yl)piperidin-1-yl]-N-methylacetamide (Ex-215)

### Synthesis of trans-tert-butyl 2-4-{[(4-chlorophenyl)carbamoyl]amino}-3-(5-methoxypyridin-2-yl)piperidin-1-yl]acetate

To the solution of *trans*-1-(4-chlorophenyl)-3-(3-(5-methoxypyridin-2-yl)piperidin-4-yl)urea trifluoroacetate (**BB-III-TFA**, 0.2 g, 0.4219 mmol, 1 eq) in ACN (10 ml), K₂CO₃ (0.291 g, 2.109 mmol, 5 eq), followed by tert-butyl 2-bromoacetate (0.099 g, 0.5063 mmol, 1.2 eq), was added at RT. The reaction was allowed to stir at RT for 16 hour. After completion of reaction (checked by TLC, 5% MeOH in DCM, R_{f}: 0.3), the reaction mixture was concentrated to get crude which was purified by column chromatography using 0-10% MeOH in DCM and 230-400 silica gel to get *trans*-tert-butyl 2-4-{[(4-chlorophenyl)carbamoyl]amino}-3-(5-methoxypyridin-2-yl)piperidin-1-yl]acetate (0.120 g, 59.8 %).
LC-MS (Method 7): m/z [M+H]⁺ = 475.3 (exact mass calc. = 474.02).

### Synthesis of trans-2-4-{[(4-chlorophenyl)carbamoyl]amino}-3-(5-methoxypyridin-2-yl)piperidin-1-yl]acetic acid:

To the solution of *trans*-tert-butyl 2-4-{[(4-chlorophenyl)carbamoyl]amino}-3-(5-methoxypyridin-2-yl)piperidin-1-yl]acetate (0.25 g, 0.526 mmol, 1 eq) in DCM (20 ml), TFA (2 ml) was added drop wise at 0 °C. The reaction was allowed to stirr at room temperature for 16 hour. After completion of reaction (checked by TLC) DCM and excess TFA was removed under reduced pressure to get crude which was quenched by NaHCO₃ solution, extracted by MeOH-DCM and washed with diethyl ether and removed ether under reduced pressure to get *trans*-2-4-{[(4-chlorophenyl)carbamoyl]amino}-3-(5-methoxypyridin-2-yl)piperidin-1-yl]acetic acid (0.2 g, 90.77%) as TFA salt.
LC-MS (Method 7): m/z [M+H]⁺ = 419.35 (exact mass calc. = 418.02).

### Synthesis of trans-2-4-{[(4-chlorophenyl)carbamoyl]amino}-3-(5-methoxypyridin-2-yl)piperidin-1-yl]-N-methylacetamide (Ex-215)

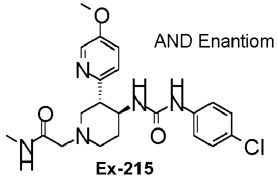

To a stirred solution *trans*-2-4-{[(4-chlorophenyl)carbamoyl]amino}-3-(5-methoxypyridin-2-yl)piperidin-1-yl]acetic acid trifluoroacetate (0.25 g, 0.5968 mmol, 1 eq) in DMF (10 ml) was added EDCI-HCl (0.171 g, 0.8952 mmol, 1.5 eq), HOBt (0.097 g, 0.716 mmol, 1.2 eq), DIPEA (0.49 ml, 2.98 mmol, 5.0 eq) and stirred at RT for 30 min followed by addition of a methanamine (0.12 ml, 0.895 mmol, 1.5 eq). The reaction mixture was stirred for 16 h at RT. After completion of the reaction (monitored by TLC, TLC system 5% MeOH-DCM, R_{f}: 0.4) reaction mixture was poured into cold water and extracted with EtOAc (2 x 25 ml). The organic layer was washed with water (2 x 25 ml) and brine (2 x 25 mL), dried over anhyd. Na₂SO₄, concentrated in vacuo. The crude material thus obtained was purified by flash chromatography (230-400 mesh silica gel; 5% MeOH-DCM as an elutent) to get *trans*-2-4-{[(4-chlorophenyl)carbamoyl]amino}-3-(5-methoxypyridin-2-yl)piperidin-1-yl]-N-methylacetamide (**Ex-215,** 0.01 g, 3.8%).
LC-MS (Method 3): m/z [M+H]⁺ = 432.3 (exact mass calc. = 431.2).
¹H-NMR (400 MHz, DMSO-d6): δ 8.34 (s, 1H), 8.19 (d, 1H), 7.73(s, 1H), 7.32-7.18 (m, 6H), 6.00-5.98 (d, J = 7.64,1H),3.81(s,1H) 3.77 (s, 3H), 2.93 (m, 3H), 2.81(m, 2H), 2.61-2.60(m, 3H), 2.24 (m, 1H), 1.99-1.96 (m, 1H), 1.61-1.53 (m, 1H).

### Synthesis of trans-2-4-{[(4-chlorophenyl)carbamoyl]amino}-3-(5-methoxypyridin-2-yl)piperidin-1-yl]-N,N-dimethylacetamide (Ex-216)

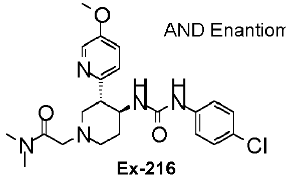

To a stirred solution *trans*-2-4-{[(4-chlorophenyl)carbamoyl]amino}-3-(5-methoxypyridin-2-yl)piperidin-1-yl]acetic acid (0.25 g, 0.5968 mmol, 1 eq) in DMF (10 ml) was added EDCI-HCl (0.171 g, 0.8952 mmol, 1.5 eq), HOBt (0.097 g, 0.716 mmol, 1.2 eq), DIPEA (0.49 ml, 2.98 mmol, 5.0 eq) and stirred at RT for 30 min followed by addition of a dimethylamine (2M, 0.4 ml, 0.895 mmol, 1.5 eq). The reaction mixture was stirred for 16 hr at RT. After completion of the reaction (monitored by TLC, TLC system 5% MeOH-DCM, R_{f}: 0.4) reaction mixture was poured into cold water and extracted with EtOAc (2 x 25 ml). The organic layer was washed with water (2 x 25 ml) and brine (2 x 25 mL), dried over anhyd. Na₂SO₄, concentrated in vacuo. The crude material thus obtained was purified by flash chromatography (230-400 mesh silica gel; 5% MeOH-DCM as an elutent) to get *trans*-2-4-{[(4-chlorophenyl)carbamoyl]amino}-3-(5-methoxypyridin-2-yl)piperidin-1-yl]-N,N-dimethylacetamide (**Ex-216**, 0.03 g, 11.2%).
LC-MS (Method 3): m/z [M+H]⁺ = 446.4 (exact mass calc. = 445.2).
¹H-NMR (400 MHz, DMSO-d6): δ 8.34 (s, 1H), 8.19-8.18 (s, 1H), 7.31- 7.18(m, 6H), 6.01-5.99 (d, J=7.96, 1H),3.84-3.81(m,1H) 3.76 (s, 3H), 3.01 (m, 2H), 2.88(s, 3H), 2.79(s, 3H), 2.32-2.22 (m, 2H), 2.01-1.97 (m, 1H), 1.54-1.46 (m, 1H).

### Synthesis of trans-1-[1'-(3-hydroxy-oxetan-3-ylmethyl)-5-methoxy-1',2',3',4',5',6'-hexahydro-[2,3']bipyridinyl-4'-yl]-3-(3-methyl-isothiazol-5-yl)-urea (Ex-217)

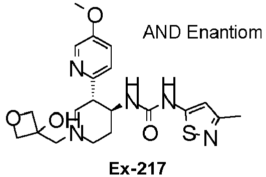

To a stirred solution of *trans-*1-(5-methoxy-1',2',3',4',5',6'-hexahydro-[2,3']bipyridinyl-4'-yl)-3-(3-methyl-isothiazol-5-yl)-urea hydrochloride (**BB-IV-HCl,** 50 mg, 0.130 mmol, 1eq) in MeOH (5 ml) was added 3-hydroxy-oxetane-3-carbaldehyde (53 mg, 0.524 mmol, 4 eq) and the reaction mixture was stirred at RT over a period of 16 h, followed by the addition of NaCNBH₃ (25 mg, 0.390 mmol, 3 eq) and the reaction mixture was stirred at RT for another 2 h and quenched with a pinch of ice. The reaction mixture was rota evaporated and submitted for reverse phase prep-HPLC chromatography to obtain *trans*-1-[1'-(3-hydroxy-oxetan-3-ylmethyl)-5-methoxy-1',2',3',4',5',6'-hexahydro-[2,3']bipyridinyl-4'-yl]-3-(3-methyl-isothiazol-5-yl)-urea (**Ex-217**)9 mg, 16% yield) as off white solid.
LC-MS (Method 3): m/z [M+H]⁺ = 434.4 (exact mass calc. = 433.18).
¹H-NMR (400 MHz, DMSO-d6): δ 9.93 (s, 1 H), 8.18 (d, *J* = 4 Hz, 1 H), 7.29-7.27 (m, 1 H), 7.21 (d, *J* = 12 Hz, 1 H), 6.50 (br s, 1 H), 6.38 (s, 1 H), 5.48 (s, 1 H), 4.40-4.35 (m, 4 H), 3.83-3.80 (br s, 1 H), 3.76 (s, 3 H), 2.94 (d, *J* = 12 Hz, 3 H), 2.69-2.60 (m, 2 H), 2.36-2.26 (m, 2 H), 2.23 (s, 3 H), 1.92 (d, *J* = 12 Hz, 1 H), 1.59-1.51 (m, 1 H).

### Synthesis of trans-1-(4-chloro-phenyl)-3-(3 -fluoro-5-methoxy-1'-oxetan-3 -yl-1',2',3',4',5',6'-hexahydro-[2,3']bipyridinyl-4'-yl)-urea (Ex-218)

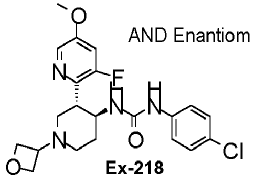

To a stirred solution of *trans*-1-(4-chloro-phenyl)-3-(3-fluoro-5-methoxy-1',2',3',4',5',6'-hexahydro-[2,3']bipyridinyl-4'-yl)-ureahydrochloride (**BB-XIII-HCl**, 100 mg, 0.241 mmol, 1 eq) in DCE (10ml) were added TEA (67 µl, 0.482 mmol, 2 eq) followed by the addition of oxetan-3-one (26 mg, 0.36 mmol, 1.5 eq) and stirred at RT for 2 h followed by the addition of NaCNBH₃ (30 mg, 0.482 mmol, 2 eq) and the reaction mixture was stirred at rt for another 18 h. The reaction mixture was then quenched with a pinch of ice and diluted with 5% MeOH-DCM (40 ml) and the organic part was then washed with saturated NaHCO₃ solution, water and brine. The combined organic layer was dried over Na₂SO₄ filtered and evaporated to get the crude which was purified by reverse phase prep-HPLC purification to obtain *trans*-1-(4-chloro-phenyl)-3-(3-fluoro-5-methoxy-1'-oxetan-3-yl-1',2',3',4',5',6'-hexahydro-[2,3']bipyridinyl-4'-yl)-urea (**Ex-218,** 14 mg, 13% yield) as off white solid.
LC-MS (Method 7): m/z [M+H]⁺ = 435.1 (exact mass calc. = 434.15).
¹H-NMR (400 MHz, DMSO-d6): δ 8.36 (s, 1 H), 8.11 (s, 1 H), 7.35-7.31 (m, 1 H), 7.27 (d, *J* = 8 Hz, 2 H), 7.18 (d, *J* = 8 Hz, 2 H), 6.00 (d, *J* = 8 Hz, 1 H), 4.53-4.37 (m, 4 H), 3.93-3.86 (m, 1 H), 3.79 (s, 3 H), 3.47-3.42 (m, 1 H), 3.15 (d, *J* = 12 Hz, 1 H), 2.77 (d, *J* = 12 Hz, 1 H), 2.66 (s, 1 H), 2.15 (t, *J* = 12 Hz, 1 H), 2.01 (d, *J* = 12 Hz, 1 H), 1.92 (t, *J* = 8 Hz, 1 H), 1.59-1.50 (m, 1 H).

### Synthesis of trans-1-(4-chloro-phenyl)-3-[1'-(2-hydroxy-1,1-dimethyl-ethyl)-5-methoxy-1',2',3',4',5',6'-hexahydro-[2,3']bipyridinyl-4'-yl]-urea (Ex-219)

### Synthesis of trans-2-{4'-[3-(4-chloro-phoyl)-ureido]-5-methoxy-3',4',5',6'-tetrahydro-2'H-[2,3']bipyridinyl-1'-yl}-2-methyl-propionic acid methyl ester

To a stirred solution of *trans*-1-(4-chloro-phenyl)-3-(5-methoxy-1',2',3',4',5',6'-hexahydro-[2,3']bipyridinyl-4'-yl)-urea hydrochloride (**BB-III-HCl,** 150 mg, 0.378 mmol, 1 eq) in ACN (7 ml) were added Cs₂CO₃ (370 mg, 1.134 mmol, 3 eq) followed by the addition of 2-bromo-2-methyl-propionic acid methyl ester (137 mg, 0.756 mmol, 2.0 eq) and stirred at reflux for 48 h. The reaction mixture was diluted with 10% MeOH-DCM and washed with water and brine. The organic layer was dried over anhyd. Na₂SO₄ and concentrated under reduced pressure to obtain crude *trans*-2-{4'-[3-(4-chloro-phenyl)-ureido]-5-methoxy-3',4',5',6'-tetrahydro-2'H-[2,3']bipyridinyl-1'-yl}-2-methyl-propionic acid methyl ester (100 mg, 58% yield) as reddish sticky.
LC-MS (Method 7): m/z [M+H]⁺ = 461.1 (exact mass calc. = 460.19).

### Synthesis of trans-1-(4-chloro-phenyl)-3-[1'-(2-hydroxy-1,1-dimethyl-ethyl)-5-methoxy-1',2',3',4',5',6'-hexahydro-[2,3']bipyridinyl-4'-yl]-urea (Ex-219)

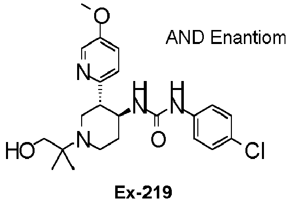

To a stirred solution of *trans*-2-{4'-[3-(4-chloro-phenyl)-ureido]-5-methoxy-3',4',5',6'-tetrahydro-2'H-[2,3']bipyridinyl-1'-yl}-2-methyl-propionic acid methyl ester (130 mg, 0.282 mmol, 0.282 mmol, 1.0 eq) in dry THF (5 ml) at ice-cold condition was added BH₃-DMS (500 µl) and stirred at reflux for 18 h. After that the reaction mixture was quenched with MeOH (1 ml). The reaction mixture was diluted with EtOAc and washed with water and brine. The organic layer was dried over anhyd. Na₂SO₄ and concentrated under reduced pressure to obtain crude product which was purified by reverse phase prep-HPLC purification to obtain *trans*-1-(4-chloro-phenyl)-3-[1'-(2-hydroxy-1,1-dimethyl-ethyl)-5-methoxy-1',2',3',4',5',6'-hexahydro-[2,3']bipyridinyl-4'-yl]-urea (**Ex-219,** 10 mg, 8% yield) as white solid.
LC-MS (Method 7): m/z [M+H]⁺ = 433.2 (exact mass calc. = 432.19).
¹H-NMR (400 MHz, DMSO-d6): δ 8.34 (s, 1 H), 8.19 (d, *J* = 4 Hz, 1 H), 7.30-7.17 (m, 6 H), 5.94 (d, *J* = 8 Hz, 1 H), 4.28-4.23 (br s, 1 H), 3.91-3.89 (m, 1 H), 3.65 (s, 3 H), 3.05-2.92 (m, 3 H), 2.78 (t, *J* = 8 Hz, 1 H), 2.62-2.59 (m, 1 H), 2.36-2.25 (m, 2 H), 2.01 (d, *J* = 8 Hz, 1 H), 1.40-1.33 (m, 1 H), 0.94 (s, 3 H), 0.93 (s, 3 H).

### Synthesis of trans-1-(4-chloro-phenyl)-3-[1'-(1-hydroxy-cyclobutanecarbonyl)-5-methoxy-1',2',3',4',5',6'-hexahydro-[2,3']bipyridinyl-4'-yl]-urea

To a stirred solution of 1-hydroxy-cyclobutanecarboxylic acid (70 mg, 0.605 mmol, 1.0 eq) in dry THF (15 ml) were added EDCI-HCl (144 mg, 0.756 mmol, 1.5 eq) and HOBt (102 mg, 0.756 mmol, 1.5 eq) in ice-cold condition followed by addition of TEA (210 µl, 1.513 mmol, 3 eq) and *trans*-1-(4-chloro-phenyl)-3-(5-methoxy-1',2',3',4',5',6'-hexahydro-[2,3']bipyridinyl-4'-yl)-urea hydrochloride (**BB-III-HCl,** 200 mg, 0.504 mmol, 1.0 eq) and stirred at RT for 18 h. The reaction mixture was diluted with EtOAc and washed with water and brine. The organic layer was dried over anhyd. Na₂SO₄ and concentrated under reduced pressure to obtain crude material which was purified by combi flash column chromatography to obtain *trans*-1-(4-chloro-phenyl)-3-[1'-(1-hydroxy-cyclobutanecarbonyl)-5-methoxy-1',2',3',4',5',6'-hexahydro-[2,3']bipyridinyl-4'-yl]-urea (150 mg, 65% crude yield) as yellow sticky.
LC-MS (Method 7): m/z [M+H]⁺ = 459.3 (exact mass calc. = 458.17).

### Synthesis of trans-1-(4-chloro-phenyl)-3-[1'-(1-hydroxy-cyclobutylmethyl)-5-methoxy-1',2',3',4',5',6'-hexahydro-[2,3']bipyridinyl-4'-yl]-urea (Ex-220)

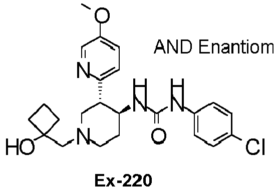

To a stirred solution of *trans*-1-(4-chloro-phenyl)-3-[1'-(1-hydroxy-cyclobutanecarbonyl)-5-methoxy-1',2',3',4',5',6'-hexahydro-[2,3']bipyridinyl-4'-yl]-urea (150 mg, 0.327 mmol, 1.0 eq) in dry THF (15 ml) at ice-cold condition was added BH₃-DMS (608 µl, 6.55 mmol, 20 eq) and stirred at RT for 18 h. After that the reaction mixture was quenched with MeOH (1 ml). The reaction mixture was diluted with EtOAc and washed with water and brine. The organic layer was dried over anhyd. Na₂SO₄ and concentrated under reduced pressure to obtain crude product which was purified by reverse phase prep-HPLC purification to obtain *trans*-1-(4-chloro-phenyl)-3-[1'-(1-hydroxy-cyclobutylmethyl)-5-methoxy-1',2',3',4',5',6'-hexahydro-[2,3']bipyridinyl-4'-yl]-urea (**Ex-220**, 26 mg, 18% yield) as off white solid.
LC-MS (Method 3): m/z [M+H]⁺ = 445.4 (exact mass calc. = 444.19).
¹H-NMR (400 MHz, DMSO-d6): δ 8.34 (s, 1 H), 8.18 (s, 1 H), 7.30-7.18 (m, 6 H), 5.95 (d, *J* = 8 Hz, 1 H), 4.74 (s, s), 3.89-3.8 (m, 1 H), 3.76 (s, 3 H), 3.09-2.94 (m, 2 H), 2.90-2.86 (m, 1 H), 2.42-2.35 (m, 2 H), 2.32-2.19 (m, 2 H), 2.02-1.96 (m, 3 H), 1.89-1.84 (m, 2 H), 1.61 (d, *J* = 8 Hz, 1 H), 1.50-1.41 (m, 2 H).

### Synthesis of trans-1-(4-chloro-phenyl)-3-[3-fluoro-1'-(4-hydroxy-cyclohexylmethyl)-5-methoxy-1',2',3',4',5',6'-hexahydro-[2,3']bipyridinyl-4'-yl]-urea (Ex-221)

### Synthesis of trans-1-(4-chloro-phenyl)-3-[3-fluoro-1'-(4-hydroxy-cyclohexanecarbonyl)-5-methoxy-1',2',3',4',5',6'-hexahydro-[2,3']bipyridinyl-4'-yl]-urea

To a stirred solution of *trans*-4-hydroxy-cyclohexanecarboxylic acid (50 mg, 0.35 mmol, 1.0 eq) in dry THF (10 ml) were added EDCI.HCl (100 mg, 0.525 mmol, 1.5 eq) and HOBT (71 mg, 0.525 mmol, 1.5 eq) in ice-cold condition followed by addition of TEA (146 µl, 1.05 mmol, 3 eq) and of *trans*-1-(4-chloro-phenyl)-3-(3-fluoro-5-methoxy-1',2',3',4',5',6'-hexahydro-[2,3']bipyridinyl-4'-yl)-urea hydrochloride (**BB-XIII-HCl**, 145 mg, 0.35 mmol, 1.0 eq) and stirred at RT for 18 h. The reaction mixture was diluted with EtOAc and washed with water and brine. The organic layer was dried over anhyd. Na₂SO₄ and concentrated under reduced pressure to obtain crude material which was purified by combi flash column chromatography to obtain *trans*-1-(4-chloro-phenyl)-3-[3-fluoro-1'-(4-hydroxy-cyclohexanecarbonyl)-5-methoxy-1',2',3',4',5',6'-hexahydro-[2,3']bipyridinyl-4'-yl]-urea (120 mg, 70%yield) as yellow sticky.
LC-MS (Method 2): m/z [M+H]⁺ = 505.2 (exact mass calc. = 504.19).

### Synthesis of trans-1-(4-chloro-phenyl)-3-[3-fluoro-1'-(4-hydroxy-cyclohexylmethyl)-5-methoxy-1',2',3',4',5',6'-hexahydro-[2,3']bipyridinyl-4'-yl]-urea (Ex-221)

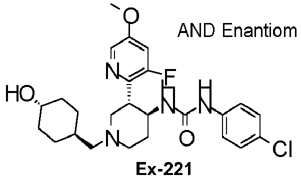

To a stirred solution of *trans*-1-(4-chloro-phenyl)-3-[3-fluoro-1'-(4-hydroxy-cyclohexanecarbonyl)-5-methoxy-1',2',3',4',5',6'-hexahydro-[2,3']bipyridinyl-4'-yl]-urea (100 mg, 0.20 mmol, 1.0 eq) in dry THF (5 ml) at ice-cold condition was added BH₃-DMS (2.5 ml) and stirred at RT for 18 h. After that the reaction mixture was quenched with MeOH (2 ml). The reaction mixture was diluted with EtOAc and washed with water and brine. The organic layer was dried over anhyd. Na₂SO₄ and concentrated under reduced pressure to obtain crude product which was purified by reverse phase prep-HPLC purification to obtain *trans*-1-(4-chloro-phenyl)-3-[3-fluoro-1'-(4-hydroxy-cyclohexylmethyl)-5-methoxy-1',2',3',4',5',6'-hexahydro-[2,3']bipyridinyl-4'-yl]-urea (**Ex-221,** 20 mg, 20% yield) as off white solid.
LC-MS (Method 3): m/z [M+H]⁺ = 491.2 (exact mass calc. = 490.21).
¹H-NMR (400 MHz, DMSO-d6): δ 8.32 (s, 1 H), 8.12 (s, 1 H), 7.34 (d, *J* = 2 Hz, 1 H), 7.31 (d, *J* = 2 Hz, 2 H), 7.18 (d, *J* = 8 Hz, 2 H), 5.92 (d, *J* = 8 Hz, 1 H), 4.44 (d, *J* = 4 Hz, 1 H), 3.92-3.89 (m, 1 H), 3.79 (s, 3 H), 3.10 (t, *J* = 8 Hz, 1 H), 2.86 (d, *J* = 8 Hz, 1 H), 2.76 (d, *J* = 8 Hz, 1 H), 2.19 (t, *J* = 12 Hz, 1 H), 2.08 (d, *J* = 8 Hz, 2 H), 1.96 (t, *J* = 12 Hz, 2 H), 1.79 (d, *J* = 12 Hz, 2 H), 1.71 (d, *J* = 12 Hz, 2 H), 1.49 (t, *J* = 8 Hz, 1 H), 1.33 (s, 1 H), 1.13-1.04 (m, 2 H), 0.87-0.77 (m, 2 H).

### Synthesis of trans-2-{4'-[3-(4-chloro-phenyl)-ureido]-3 -fluoro-5-methoxy-3',4',5',6'-tetrahydro-2'H-[2,3']bipyridinyl-1'-yl}-acetamide (Ex-222)

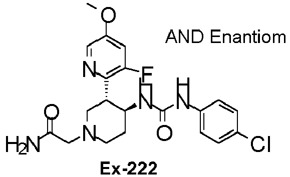

To a stirred solution of *trans*-1-(4-chloro-phenyl)-3-(3-fluoro-5-methoxy-1',2',3',4',5',6'-hexahydro-[2,3']bipyridinyl-4'-yl)-urea hydrochloride (**BB-XIII-HCl**, 100 mg, 0.241 mmol, 1 eq) in ACN (10 ml) were added K₂CO₃ (100 mg, 0.723 mmol, 3.0 eq) followed by the addition of 2-bromo-acetamide (40 mg, 0.289 mmol, 1.2 eq) and stirred at RT for 18 h. The reaction mixture was diluted with 10% MeOH-DCM and washed with water and brine. The organic layer was dried over anhyd. Na₂SO₄ and concentrated under reduced pressure to obtain crude product which was purified by reverse phase prep-HPLC purification to obtain *trans*-2-{4'-[3-(4-chlorophenyl)-ureido]-3-fluoro-5-methoxy-3',4',5',6'-tetrahydro-2'H-[2,3']bipyridinyl-1'-yl}-acetamide (**Ex-222,** 45 mg, 43% yield) as off white solid.
LC-MS (Method 7): m/z [M+H]⁺ = 436.2 (exact mass calc. = 435.15).
¹H-NMR (400 MHz, DMSO-d6): δ 8.34 (s, 1 H), 8.13 (s, 1 H), 7.34 (d, *J* = 2 Hz, 1 H), 7.33-7.28 (m, 3 H), 7.19 (d, *J* = 8 Hz, 2 H), 7.09 (s, 1 H), 5.94 (d, *J* = 8 Hz, 1 H), 3.93-3.85 (m, 1 H), 3.79 (s, 3 H), 3.23-3.18 (m, 1 H), 2.90 (d, *J* = 4 Hz, 3 H), 2.80 (d, *J* = 8 Hz, 1 H), 2.42 (d, *J* = 12 Hz, 1 H), 2.21 (t, *J* = 12 Hz, 1 H), 1.98 (d, *J* = 8 Hz, 1 H), 1.63-1.55 (m, 1H).

### Synthesis of trans-1-(4-Chloro-phenyl)-3-[3-fluoro-5-methoxy-1'-(4-methoxy-cyclohexylmethyl)-1',2',3',4',5',6'-hexahydro-[2,3']bipyridinyl-4'-yl]-urea (Ex-223)

### Synthesis of trans-1-(4-chloro-phenyl)-3-[3-fluoro-5-methoxy-1'-(4-methoxy-cyclohexanecarbonyl)-1',2',3',4',5',6'-hexahydro-[2,3']bipyridinyl-4'-yl]-urea

To a stirred solution of *trans*-4-methoxy-cyclohexanecarboxylic acid (50 mg, 0.316 mmol, 1.0 eq) in dry THF (10 ml) were added EDCI-HCl (91 mg, 0.474 mmol, 1.5 eq) and HOBt (64 mg, 0.474 mmol, 1.5 eq) in ice-cold condition followed by addition of TEA (132 µl, 0.948 mmol, 3 eq) and *trans*-1-(4-chloro-phenyl)-3-(3-fluoro-5-methoxy-1',2',3',4',5',6'-hexahydro-[2,3']bipyridinyl-4'-yl)-urea hydrochloride (**BB-XIII-HCl**, 130 mg, 0.316 mmol, 1.0 eq) and stirred at RT for 18 h. The reaction mixture was diluted with EtOAc and washed with water and brine. The organic layer was dried over anhyd. Na₂SO₄ and concentrated under reduced pressure to obtain crude material which was purified by combi flash column chromatography to obtain *trans*-1-(4-chloro-phenyl)-3-[3-fluoro-5-methoxy-1'-(4-methoxy-cyclohexanecarbonyl)-1',2',3',4',5',6'-hexahydro-[2,3']bipyridinyl-4'-yl]-urea (120 mg, 73%yield) as yellow sticky.
LC-MS (Method 2): m/z [M+H]⁺ = 519.2 (exact mass calc. = 518.21).

### Synthesis of trans-1-(4-Chloro-phenyl)-3-[3-fluoro-5-methoxy-1'-(4-methoxy-cyclohexylmethyl)-1',2',3',4',5',6',-hexahydro-[2,3']bipyridinyl-4'-yl]-urea (Ex-223)

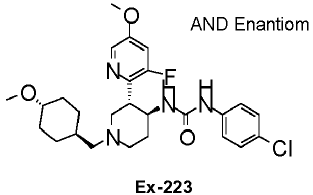

To a stirred solution of *trans*-1-(4-chloro-phenyl)-3-[3-fluoro-5-methoxy-1'-(4-methoxy-cyclohexanecarbonyl)-1',2',3',4',5',6'-hexahydro-[2,3']bipyridinyl-4'-yl]-urea (100 mg, 0.20 mmol, 1.0 eq) in dry THF (5 ml) at ice-cold condition was added BH₃-DMS (3 ml) and stirred at RT for 18 h. After that the reaction mixture was quenched with MeOH (2 ml). The reaction mixture was diluted with EtOAc and washed with water and brine. The organic layer was dried over anhyd. Na₂SO₄ and concentrated under reduced pressure to obtain crude product which was purified by reverse phase prep-HPLC purification to obtain *trans*-1-(4-Chloro-phenyl)-3-[3-fluoro-5-methoxy-1'-(4-methoxy-cyclohexylmethyl)-1',2',3',4',5',6'-hexahydro-[2,3']bipyridinyl-4'-yl]-urea (**Ex-223,** 12 mg, 12% yield) as off white solid.
LC-MS (Method 3): m/z [M+H]⁺ = 505.2 (exact mass calc. = 504.23).
¹H-NMR (400 MHz, DMSO-d6): δ 8.32 (s, 1 H), 8.12 (s, 1 H), 7.33 (t, *J* = 8 Hz, 1 H), 7.27 (d, *J* = 16 Hz, 2 H), 7.19 (d, *J* = 8 Hz, 2 H), 5.92 (d, *J* = 4 Hz, 1 H), 3.92-3.89 (m, 1 H), 3.80 (s, 3 H), 3.21 (s, 3 H), 3.10 (s, 1 H), 3.01 (d, *J* = 8 Hz, 1 H), 2.87 (d, *J* = 8 Hz, 1 H), 2.77 (d, *J* = 8 Hz, 1 H), 2.28-2.17 (m, 1 H), 2.09 (d, *J* = 8 Hz, 2 H), 1.97 (s, 4 H), 1.76 (d, *J* = 12 Hz, 2 H), 1.54-1.45 (m, 1 H), 1.39 (d, *J* = 8 Hz, 1 H), 1.14-1.03 (m, 2 H), 0.87 (d, *J* = 12 Hz, 2 H).

### Synthesis of trans-1-(4-chloro-phenyl)-3-[5-methoxy-1'-(6-methyl-pyrazin-2-yl)-1',2',3',4',5',6'-hexahydro-[2,3']bipyridinyl-4'-yl]-urea (Ex-224)

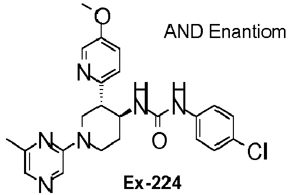

To a stirred solution of *trans*-1-(4-chloro-phenyl)-3-(5-methoxy-1',2',3',4',5',6'-hexahydro-[2,3']bipyridinyl-4'-yl)-urea hydrochloride (**BB-III-HCl,** 150 mg, 0.38 mmol, 1 eq) in DMSO (5 ml) were added DIPEA (200 µl, 1.134 mmol, 3 eq) followed by the addition of 2-chloro-6-methyl-pyrazine (**Int-13**) (76 mg, 0.567 mmol, 3.0 eq) and stirred at 100 °C for 18 h. The reaction mixture was diluted with 10% MeOH-DCM and washed with water and brine. The organic layer was dried over anhyd. Na₂SO₄ and concentrated under reduced pressure to obtain crude product which was purified by reverse phase purification to obtain *trans*-1-(4-chloro-phenyl)-3-[5-methoxy-1'-(6-methyl-pyrazin-2-yl)-1',2',3',4',5',6'-hexahydro-[2,3']bipyridinyl-4'-yl]-urea (**Ex-224,** 10 mg, 6% yield) as off white solid.
LC-MS (Method 7): m/z [M+H]⁺ = 453.5 (exact mass calc. = 452.17).
¹H-NMR (400 MHz, DMSO-d6): δ 8.348 (s, 1 H), 8.24 (d, *J* = 4 Hz, 1 H), 8.14 (s, 1 H), 7.71 (s, 1 H), 7.36-7.28 (m, 4 H), 7.20 (d, *J* = 8 Hz, 2 H), 6.04 (d, *J* = 8 Hz, 1 H), 4.42 (d, *J* = 12 Hz, 2 H), 4.12 (s, 1 H), 3.79 (s, 3 H), 3.12-3.01 (m, 3 H), 2.87 (t*, J* = 8 Hz, 1 H), 2.31 (s, 3 H), 2.08 (d, *J* = 8 Hz, 1 H), 1.47 (t, *J* = 12 Hz, 1 H).

### Synthesis of trans-2-4-{[(4-chlorophenyl)carbamoyl]amino}-3-(5-methoxypyridin-2-yl)piperidin-1-yl]-2-methylpropanamide (Ex-225)

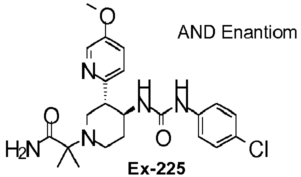

To the solution of *trans*-1-(4-chlorophenyl)-3-(3-(5-methoxypyridin-2-yl)piperidin-4-yl)urea trifluoroacetate (**BB-III-TFA**, 0.2 g, 0.4219 mmol, 1 eq) in ACN (10 ml), K₂CO₃ (0.291 g, 2.109 mmol, 5 eq), followed by 2-bromo-2-methylpropanamide (0.084 g, 0.5063 mmol, 1.2 eq), was added at RT. The reaction was allowed to stir at 90 °C for 16 h. After completion of reaction (checked by TLC, 5% MeOH in DCM, R_{f}: 0.3), the reaction mixture was concentrated to get crude which was purified by column chromatography using 0-10% MeOH in DCM and 230-400 silica gel to get *trans*-2-4-{[(4-chlorophenyl)carbamoyl]amino}-3-(5-methoxypyridin-2-yl)piperidin-1-yl]-2-methylpropanamide (**Ex-225,** 0.045 g, 23.91 %).
LC-MS (Method 7): m/z [M+H]⁺ = 446.2 (exact mass calc. = 445.02).
¹H-NMR (400 MHz, DMSO-d6): δ 8.36 (s, 1H), 8.20 (s, 1H), 7.31- 7280(m, 3H), 7.23-7.18 (m, 4H), 6.97 (bs, 1H), 5.94-5.92 (d, J=8.04, 1H),3.83-3.80(m,1H) 3.76 (s, 3H), 2.93-2.88 (m, 1H), 2.81-2.75(m, 2H), 2.30-2.22(m, 2H), 2.07-2.02 (m, 1H), 1.54-1.51 (m, 1H), 1.09-1.04 (m, 6H).

### Synthesis of trans-1-(4-chloro-phoyl)-3-(5-methoxy-1'-pyrazin-2-yl-1',2',3',4',5',6'-hexahydro-[2,3']bipyridinyl-4'-yl)-urea (Ex-226)

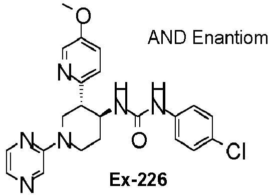

To a stirred solution of 1-(4-chloro-phenyl)-3-(5-methoxy-1',2',3',4',5',6'-hexahydro-[2,3']bipyridinyl-4'-yl)-urea hydrochloride (**BB-III-HCl,** 120 mg, 0.3 mmol, 1 eq) in *n*-BuOH (3 ml) were added DIPEA (157 mg, 0.9 mmol, 3 eq) followed by the addition of 2-chloro-pyrazine (32 µl, 0.36 mmol, 1.2 eq) and stirred at 130 °C for 18 h. The reaction mixture was diluted with 10% MeOH-DCM and washed with water and brine. The organic layer was dried over anhyd. Na₂SO₄ and concentrated under reduced pressure to obtain crude product which was purified by reverse phase purification to obtain *trans*-1-(4-chloro-phenyl)-3-(5-methoxy-1'-pyrazin-2-yl-1',2',3',4',5',6'-hexahydro-[2,3']bipyridinyl-4'-yl)-urea (**Ex-226,** 12 mg, 9% yield) as off white solid.
LC-MS (Method 7): m/z [M-H]⁺ = 439.1 (exact mass calc. = 438.16).
¹H-NMR (400 MHz, DMSO-d6): δ 8.48-8.46 (br s, 1 H), 8.36 (s, 1 H), 8.24 (s, 1 H), 8.07 (s, 1 H), 7.81 (s, 1 H), 7.34-7.29 (m, 4 H), 7.19 (d*, J* = 8 Hz, 2 H), 6.17 (s, 1 H), 4.43 (d, *J* = 12 Hz, 2 H), 4.15 (d, *J* = 12 Hz, 1 H), 3.79 (s, 3 H), 3.17-3.06 (m, 2 H), 2.89-2.84 (m, 1 H), 2.07 (d*, J* = 8 Hz, 1 H), 1.52-1.44 (m, 1 H).

### Synthesis of final compounds (piperidones)

### Synthesis of trans-1-(4-chlorophenyl)-3-[5-(5-methoxypyridin-2-yl)-2-oxo-1-(pyrimidin-4-yl)piperidin-4-yl]urea diastereomer-1 (Ex-25)

### Synthesis of 5-bromo-2-methoxypyridine-4-carbaldehyde

To a stirred solution of DIPA (20.87 mL, 148.92 mmol, 2.0 eq) in THF (500 ml), nBuLi (44.67 mL, 111.69 mmol, 1.5 eq) was added at -78 °C and stirred for 1 h at this temperature. Then 5-bromo-2-methoxypyridine (14 g, 74.46 mmol, 1 eq) in THF was added to it at the same temperature. After stirring the reaction mixture for 30 min at same temperature DMF (8 mL, 111.69 mmol, 1.5 eq) was added. The reaction was then continued for 30 min at RT. After completion of reaction (monitored by TLC, 5% ethyl acetate in hexane, R_{f}: 0.3), reaction mixture was quenched with saturated NH₄Cl solution (500 ml) and extracted with EA (3x500 ml). Wash the organic layer with water (500 ml), brine (500 ml), dried over Na₂SO₄, filtered and concentrated under reduced pressure to get the crude. Crude was purified by column chromatography using 100-200 silica gel and 2% ethyl acetate in hexane as eluting solvent to afford 5-bromo-2-methoxyisonicotinaldehyde (15 g, 94%) as off white solid.

### Synthesis of 5-bromo-2-methoxypyridine-4-carboxylic acid

NaH₂PO₄ (245 g, 2.036 mol, 4.04 eq), NaClO2 (185 g, 2.036 mol, 4.44 eq) was 5-bromo-2-methoxyisonicotinaldehyde (110 g, 0.509 mol, 1 eq) in THF (1000 ml): n-BuOH (1000 ml): H₂O (300 ml) at 0 °C. Then 2-methyl-2-butene (200 ml, 2.036 mol, 4.44 eq) was added to it. The reaction mixture was stirred at RT for 1 h. After completion of the reaction monitor by TLC (5% MeOH in DCM, R_{f}: 0.1), organic solvent was separated, dried over Na₂SO₄ and concentrated, finally compound was purified by triturated with hexane to afford 5-bromo-2-methoxyisonicotinic acid (93 g, 79%).

### Synthesis of methyl 5-bromo-2-methoxypyridine-4-carboxylate

To a solution of 5-bromo-2-methoxyisonicotinic acid (13.0 g, 56.027 mmol, 1.0 eq) in MeOH (100 ml), conc H₂SO₄ (15 ml) was added at RT. The reaction mixture was then heated at 80 °C for 16h. After completion of reaction (monitored by TLC, 5% MeOH in DCM, R_{f}: 0.1), reaction mixture was evaporated under reduced pressure, diluted with ice cold water (100 ml), neutralized by saturated NaHCO₃ solution. A solid precipitate was formed filtered to get the crude which was purified by triturating with hexane to afford methyl 5-bromo-2-methoxyisonicotinate (10 g, 73%) as off white solid.

### Synthesis of methyl 2-methoxy-5-(tetramethyl-1,3,2-dioxaborolan-2-yl)pyridine-4-carboxylate

To a stirred solution of 5-bromo-2-methoxyisonicotinate (9.0 g, 36.5 mmol, 1 eq) in dioxane (100 ml), KOAc (7.0 g, 54.0 mmol, 2 eq) and bis-pinacolatodiborane (14 g, 54.0 mmol, 1.5 eq) was added at RT. The reaction mixture was then degassed for 30 min, followed by the addition of PdC12(dppf).DCM (1.5 g, 1.825 mmol, 0.05 eq). The reaction mixture was then heated at 110 °C for 16 h. After completion of reaction (monitored by TLC,10% ethyl acetate in hexane, R_{f}: 0.4), reaction mixture was passed through celite bed, evaporated under reduced pressure to get the crude. Crude was purified by column chromatography to afford desired methyl 2-methoxy-5-(tetramethyl-1,3,2-dioxaborolan-2-yl)pyridine-4-carboxylate (10.1 g, 94%) as brown gum.

### Synthesis of methyl 2-methoxy-5-(5-methoxypyridin-2-yl)pyridine-4-carboxylate

To a solution of desired methyl 2-methoxy-5-(tetramethyl-1,3,2-dioxaborolan-2-yl)pyridine-4-carboxylate (10.0 g, 53.185 mmol, 1.0 eq) in dioxane (100 ml), K₂CO₃ (15 g, 106.371 mmol, 2 eq) and 2-bromo-5-methoxy-pyridine (17 g, 58.504 mmol, 1.1 eq) was added at RT. The reaction mixture was then degassed for 30 min, followed by the addition of Pd(PPh₃)₄ (3.0 g, 2.659 mmol, 0.05 eq). The reaction mixture was then heated at 110 °C for 6 h. After completion of reaction (monitored by TLC), reaction mixture was evaporated under reduced pressure to get the crude. Crude was purified by column chromatography to afford methyl 2-methoxy-5-(5-methoxypyridin-2-yl)pyridine-4-carboxylate (5.6 g, 19%) as brown gum.

### Synthesis of methyl 5-(5-methoxypyridin-2-yl)-2-oxo-1,2-dihydropyridine-4-carboxylate

To a stirred solution of 2-methoxy-5-(5-methoxypyridin-2-yl)pyridine-4-carboxylate (2.5 g, 9.124 mmol, 1 eq) in MeOH (50 ml), conc. HCl (10 ml) was added at RT and the reaction mixture was heated at 80 °C for 16 h. After completion of the reaction, monitored by TLC (R_{f}: 0.2, mobile phase, 5% MeOH in DCM), MeOH was removed by distillation, neutralised by saturated NaHCO₃ solution, extracted with 10% MeOH in DCM, dried over Na₂SO₄ and concentrated. Finally compound was purified by column chromatography using 230-400 silica gel and 0-3% MeOH in DCM to afford methyl 5-(5-methoxypyridin-2-yl)-2-oxo-1,2-dihydropyridine-4-carboxylate (0.8 g, 34%) as off white solid.

### Synthesis of methyl 5-(5-methoxypyridin-2-yl)-2-oxopiperidine-4-carboxylate

To a stirred solution of methyl 5-(5-methoxypyridin-2-yl)-2-oxo-1,2-dihydropyridine-4-carboxylate (0.5 g, 1.923 mmol, 1.0 eq) in Methanol (50 mL), Pd-C (0.15 g, 10%, moist) was added and the reaction was stirred under H₂ atmosphere at autoclave 17 bar) for 16 h at 70 °C. After completion, (monitored by TLC, TLC system 5% methanol in DCM, R_{f}: 0.2), the reaction mixture was filtered through celite bed and washed 2-3 times with MeOH. The filtrate was concentrated to get the methyl 5-(5-methoxypyridin-2-yl)-2-oxopiperidine-4-carboxylate as white gum (0.4 g, 79%).

### Synthesis of methyl 5-(5-methoxypyridin-2-yl)-2-oxo-1-(pyrimidin-4-yl)piperidine-4-carboxylate

A stirred solution of methyl 5-(5-methoxypyridin-2-yl)-2-oxopiperidine-4-carboxylate (0.5 g, 1.893 mmol, 1.0 eq), compound 4-chloropyrimidine hydrochloride (0.259 g, 2.271 mmol, 1.2 eq), Cs₂CO₃ (1.8 g, 5.679 mmol, 3.0 eq) in 1,4-dioxane (20 ml) was de gassed with argon for 30 min. Xantphos (0.109 g, 0.189 mmol, 0.1 eq) and PdCl₂(dppf)-DCM (0.077 g, 0.094 mmol, 0.05 eq) was added and the reaction was stirred for 16 h at 90 °C in sealed tube. After completion of the reaction, (monitored by TLC, TLC system 5% methanol in DCM, R_{f}: 0.5), the reaction mixture was filtered through celite bed and washed 2-3 times with dioxane. The combined organic layer was concentrated to get the crude product which was purified by column chromatography (230-400 mesh silica gel; 0 to 2% MeOH in DCM) to afford methyl 5-(5-methoxypyridin-2-yl)-2-oxo-1-(pyrimidin-4-yl)piperidine-4-carboxylate (0.11 g, 17%).

### Synthesis of 5-(5-methoxypyridin-2-yl)-2-oxo-1-(pyrimidin-4-yl)piperidine-4-carboxylic acid

To a stirred solution of methyl 5-(5-methoxypyridin-2-yl)-2-oxo-1-(pyrimidin-4-yl)piperidine-4-carboxylate (0.6 g, 1.754 mmol, 1.0 eq) in THF:H₂O=3:1 (40 ml) was LiOH·H₂O (0.4 g, 8.77 mmol, 5 eq) and the reaction mixture was stirred at RT for 3 h. After completion of the reaction (monitored by TLC, TLC system 5% methanol in DCM, R_{f}: 0.1), reaction mixture was concentrated and acidified with NaHSO₄ solution, removed water by rotary evaporation, triturated with 20% MeOH in DCM, dried over Na₂SO₄ and concentrated to afford 5-(5-methoxypyridin-2-yl)-2-oxo-1-(pyrimidin-4-yl)piperidine-4-carboxylic acid (0.477 g, 82.83%) as a white solid.

### Synthesis of trans-1-(4-chlorophenyl)-3-[5-(5-methoxypyridin-2-yl)-2-oxo-1-(pyrimidin-4-yl)piperidin-4-yl]urea diastereomer-1 (Ex-25)

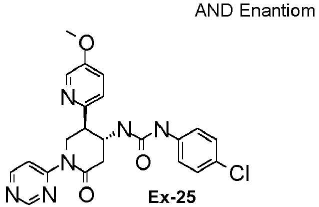

To a stirred solution of afford 5-(5-methoxypyridin-2-yl)-2-oxo-1-(pyrimidin-4-yl)piperidine-4-carboxylic acid (0.3 g, 0.886 mmol, 1.0 eq) in toluene:THF (30:10 mL) was added TEA (0.3 mL, 1.772 mmol, 2.0 eq) and DPPA (0.488 g, 1.772 mmol, 2.0 eq) and the reaction mixture was stirred at 90 °C for 30 min. After 30 min, 4-chloroaniline (0.17 g, 1.33 mmol, 1.5 eq) was added to the reaction mixture and heated to reflux for 16 h. After completion, (monitored by TLC, TLC system 5% methanol in DCM, R_{f}: 0.3), the reaction mixture was concentrated under reduced pressure, diluted with ethyl acetate (150 mL) washed with water (2x100 mL), dried over anhyd. Na₂SO₄ and concentrated under reduced pressure to get the crude product which was purified by column chromatography (230-400 mesh silica gel; 0-3% MeOH in DCM) and again separated by prep-HPLC to afford *trans*-1-(4-chlorophenyl)-3-[5-(5-methoxypyridin-2-yl)-2-oxo-1-(pyrimidin-4-yl)piperidin-4-yl]urea diastereomer-1 (**Ex-25,** 0.03 g, 7%).
¹H-NMR (400 MHz, DMSO-d6): δ 8.96 (s, 1H), 8.71 (d*, J* = 5.9 Hz, 1H), 8.68 (s, 1H), 8.35 (d*, J* = 5.6 Hz, 1H), 8.27 (d, *J* = 2.6 Hz, 1H), 7.44 (d, *J* = 8.6 Hz, 1H), 7.34-7.39 (m, 3H), 7.24 (d, *J* = 8.8 Hz, 2H), 6.33 (t, *J* = 5.9 Hz, 1H), 4.42 (t, *J* = 9.7 Hz, 1H), 3.93 (t, *J* = 9.9 Hz, 1H), 3.80 (s, 3H), 3.42-3.64 (m, 3H), 3.26-3.29 (m, 1H).

### Synthesis of trans-1-(4-chlorophenyl)-3-[1-(4-fluorophenyl)-5-(6-methoxypyridin-3-yl)-2-oxopiperidin-4-yl]urea (Ex-5) and cis-1-(4-chlorophenyl)-3-[1-(4-fluorophenyl)-5-(6-methoxypyridin-3-yl)-2-oxopiperidin-4-yl]urea (Ex-6)

### Synthesis of methyl 5-bromo-1-(4-fluorophenyl)-2-oxo-1,2-dihydropyridine-4-carboxylate

To a stirred solution of methyl 5-bromo-2-oxo-1,2-dihydropyridine-4-carboxylate (2.5 g, 10.775 mmol, 1.0 eq), (4-fluorophenyl) boronic acid (3.01 g, 21.55 mmol, 2.0 eq), in DCM (10 mL), degassed the reaction by oxygen for 15 min. Then Cu-TMEDA (1.50 g, 3.232 mmol, 0.3 eq) was added and the reaction was stirred for 16 h at RT. After completion of the reaction (monitored by TLC, TLC system 40% ethyl acetate in hexane, R_{f}: 0.4), the solvent was removed under reduced pressure to get the crude. Crude was diluted with water. The aqueous layer was extracted twice with ethyl acetate (2x50 mL). The combined organic was washed with brine, dried over Na₂SO₄, filtered, and concentrated. Crude was purified by column chromatography to afford methyl 5-bromo-1-(4-fluorophenyl)-2-oxo-1,2-dihydropyridine-4-carboxylate (2 g, 54.75%).

### Synthesis of methyl 1-(4-fluorophenyl)-5-(6-methoxypyridin-3-yl)-2-oxo-1,2-dihydropyridine-4-carboxylate

To a solution of methyl 5-bromo-1-(4-fluorophenyl)-2-oxo-1,2-dihydropyridine-4-carboxylate (0.7 g, 2.147 mmol, 1.0 eq) in Dioxane (50 ml), K₂CO₃ (0.890 g, 6.441 mmol, 3 eq), (6-methoxypyridin-3-yl)boronic acid (0.656 g, 4.294 mmol, 2.0 eq) was added at RT. The solution was degassed with argon for 20 min followed by addition of Pd(PPh₃)₄ (0.124 g, 0.107 mmol, 0.05 eq). The reaction mixture was then placed to pre-heated oil bath at 90 °C for 16 h. After completion of reaction (monitored by TLC and LC-MS), reaction mixture was diluted with water (100 mL) and extracted with Ethyl acetate (2x250 mL). Wash the organic layer with brine (75 mL), dried over Na₂SO₄, filtered and evaporated under reduced pressure to get the crude. Crude was initially purified by column chromatography using EtOAC-hexane as elutent. The product was then washed with ether to afford methyl 1-(4-fluorophenyl)-5-(6-methoxypyridin-3-yl)-2-oxo-1,2-dihydropyridine-4-carboxylate (0.500 g, 63%) as white solid.

### Synthesis of methyl 1-(4-fluorophenyl)-5-(6-methoxypyridin-3-yl)-2-oxopiperidine-4-carboxylate

To a stirred solution of methyl 1-(4-fluorophenyl)-5-(6-methoxypyridin-3-yl)-2-oxo-1,2-dihydropyridine-4-carboxylate (0.5 g, 1.358 mmol, 1.0 eq) in methanol (50 mL) Pd-C (0.3 g, 10%, moist) was added and the reaction was stirred in a Parr aparatus (17 bar) for 16 h at 70 °C. After completion, (monitored by TLC, TLC system 5% methanol in DCM, R_{f}: 0.2), the reaction mixture was filtered through celite bed and washed 2-3 times with MeOH. The filtrate was concentrated to get methyl 1-(4-fluorophenyl)-5-(6-methoxypyridin-3-yl)-2-oxopiperidine-4-carboxylate as white gum (0.4 g, 79%).

### Synthesis of 1-(4-fluorophenyl)-5-(6-methoxypyridin-3-yl)-2-oxopiperidine-4-carboxylic acid

To a stirred solution of methyl 1-(4-fluorophenyl)-5-(6-methoxypyridin-3-yl)-2-oxopiperidine-4-carboxylate (0.4 g, 1.074 mmol, 1.0 eq) in MeOH (50 ml) was added 2MNaOH solution (4 mL) and the reaction mixture was stirred at 100 °C for 16 h. After completion of the reaction (monitored by TLC, TLC system 5% methanol in DCM, R_{f}: 0.1), reaction mixture was concentrated and acidified with NaHSO₄ solution to get solid, filtered and washed with diethyl ether, dried under vacuum to afford 1-(4-fluorophenyl)-5-(6-methoxypyridin-3-yl)-2-oxopiperidine-4-carboxylic acid (0.35 g, 94.64%) as a white solid.

### Synthesis of trans-1-(4-chlorophenyl)-3-[-1-(4-fluorophenyl)-5-(6-methoxypyridin-3-yl)-2-oxopiperidin-4-yl]urea (Ex-5) and cis-1-(4-chlorophenyl)-3-[1-(4-fluorophenyl)-5-(6-methoxypyridin-3-yl)-2-oxopiperidin-4-yl]urea (Ex-6)

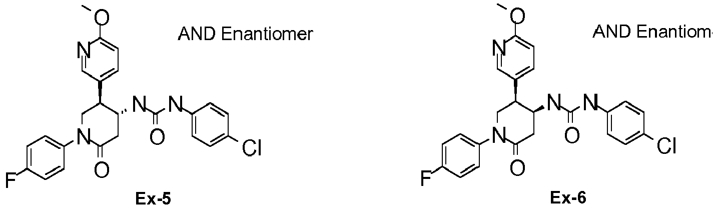

To a stirred solution 1-(4-fluorophenyl)-5-(6-methoxypyridin-3-yl)-2-oxopiperidine-4-carboxylic acid (0.25 g, 0.726 mmol, 1.0 eq) in toluene:THF (30:10 mL) was added TEA (0.2 mL, 1.452 mmol, 2.0 eq) and DPPA (0.4 g, 1.452 mmol, 2.0 eq) and the reaction mixture was stirred at 90 °C for 30 min. After 30 min, 4-chloroaniline (0.139 g, 1.089 mmol, 1.5 eq) was added to the reaction mixture and heated to reflux for 16 h. After completion, (monitored by TLC, TLC system 5% methanol in DCM, R_{f}: 0.3), the reaction mixture was concentrated under reduced pressure diluted with ethyl acetate (150 mL) washed with water (2x100 mL), dried over anhyd. Na₂SO₄ and concentrated under reduced pressure to get the crude product which was purified by column chromatography (230-400 mesh silica gel; 0-3% MeOH in DCM) and again separated by prep-HPLC to afford *trans*-1-(4-chlorophenyl)-3-[-1-(4-fluorophenyl)-5-(6-methoxypyridin-3-yl)-2-oxopiperidin-4-yl]urea (**Ex-5,** 0.025 g, 7.46%) and cis-1-(4-chlorophenyl)-3-[-1-(4-fluorophenyl)-5-(6-methoxypyridin-3-yl)-2-oxopiperidin-4-yl]urea (**Ex-6,** 0.015 g, 4.37%).
**Ex-5:** 1H-NMR (400 MHz, DMSO-d6): δ 8.48 (s, 1H), 8.13 (d, *J* = 1.7 Hz, 1H), 7.73 (dd, *J* = 2.0, 8.5 Hz, 1H), 7.31-7.38 (m, 4H), 7.19-7.23 (m, 4H), 6.78 (d*, J* = 8.5 Hz, 1H), 6.24 (d*, J* = 8.3 Hz, 1H), 4.46-4.49 (m, 1H), 3.89 (t*, J* = 11.4 Hz, 1H), 3.79 (s, 3H), 3.51-3.55 (m, 1H), 3.34-3.39 (m, 1H), 2.82 (dd, *J* = 5.5, 17.0 Hz, 1H), 2.54-2.61 (m, 1H).
**Ex-6:** 1H-NMR (400 MHz, DMSO-d6): δ 8.51 (s, 1H), 8.10 (s, 1H), 7.69 (d*, J* = 7.2 Hz, 1H), 7.43-7.46 (m, 2H), 7.38 (d, *J* = 8.6 Hz, 2H), 7.24-7.28 (m, 4H), 6.80 (d, *J* = 8.6 Hz, 1H), 6.57 (d, *J* = 8.0 Hz, 1H), 4.42-4.43 (m, 1H), 3.91-3.92 (m, 2H), 3.81 (s, 3H), 3.50-3.60 (m, 1H), 2.86 (dd, *J* = 4.8, 17.5 Hz, 1H), 2.34-2.39 (m, 1H).

### Synthesis of trans-1-(4-chlorophenyl)-3-[1-(4-fluorophenyl)-5-(5-methoxypyridin-2-yl)-2-oxopiperidin-4-yl]urea (Ex-10) and cis-1-(4-chlorophenyl)-3-[1-(4-fluorophenyl)-5-(5-methoxypyridin-2-yl)-2-oxopiperidin-4-yl]urea (Ex-10)

### Synthesis of methyl 1-(4-fluorophenyl)-5-(6-methoxypyridin-3-yl)-2-oxo-1,2-dihydropyridine-4-carboxylate

To a solution of methyl 5-bromo-1-(4-fluorophenyl)-2-oxo-1,2-dihydropyridine-4-carboxylate (4.7 g, 14.4 mmol, 1.0 eq) in Dioxane (50 ml), K₂CO₃ (5.9 g, 43.2 mmol, 3 eq), 5-methoxy-2-(tributylstannyl)pyridine (17.2 g, 43.2 mmol, 3.0 eq) was added at RT. The solution was degassed with argon for 20 min followed by addition of Pd(PPh₃)₄ (0.83 g, 0.72 mmol, 0.05 eq). The reaction mixture was then placed to pre-heated oil bath at 90 °C for 16 h. After completion of reaction (monitored by TLC and LC-MS), reaction mixture was diluted with water (100 mL) and extracted with ethyl acetate (2x250 mL). Wash the organic layer with brine (75 mL), dried over Na₂SO₄, filtered and evaporated under reduced pressure to get the crude. Crude was initially purified by column chromatography using EtOAC-hexane as elutent. The product was then washed with ether to afford methyl 1-(4-fluorophenyl)-5-(6-methoxypyridin-3-yl)-2-oxo-1,2-dihydropyridine-4-carboxylate (1.5 g, 28.27%) as white solid.

### Synthesis of methyl 1-(4-fluorophenyl)-5-(5-methoxypyridin-2-yl)-2-oxopiperidine-4-carboxylate

To a stirred solution of methyl 1-(4-fluorophenyl)-5-(6-methoxypyridin-3-yl)-2-oxo-1,2-dihydropyridine-4-carboxylate (0.8 g, 2.259 mmol, 1.0 eq) in Methanol (50 mL), Pd-C (0.4 g, 10%, moist) was added and the reaction was stirred in a Parr apparatus (17 bar) for 16 h at 70 °C. After completion, (monitored by TLC, TLC system 5% methanol in DCM, R_{f}: 0.2), the reaction mixture was filtered through celite bed and washed 2-3 times with MeOH. The filtrate was concentrated to get the 1-(4-fluorophenyl)-5-(5-methoxypyridin-2-yl)-2-oxopiperidine-4-carboxylate as white gum (0.4 g, 48%).

### Synthesis of 1-(4-fluorophenyl)-5-(5-methoxypyridin-2-yl)-2-oxopiperidine-4-carboxylic acid

To a stirred solution of 1-(4-fluorophenyl)-5-(5-methoxypyridin-2-yl)-2-oxopiperidine-4-carboxylate (0.6 g, 1.611 mmol, 1.0 eq) in MeOH (50 ml) was added 2 N NaOH solution (4 mL) and the reaction mixture was stirred at 100 °C for 16 h. After completion of the reaction (monitored by TLC, TLC system 5% methanol in DCM, R_{f}: 0.1), reaction mixture was concentrated and acidified with NaHSO₄ solution to get solid, filtered and washed with diethyl ether, dried under vacuum to afford of 1-(4-fluorophenyl)-5-(5-methoxypyridin-2-yl)-2-oxopiperidine-4-carboxylic acid (0.5 g, 90%) as a white solid.

### Synthesis of trans-1-(4-chlorophenyl)-3-[1-(4-fluorophenyl)-5-(5-methoxypyridin-2-yl)-2-oxopiperidin-4-yl]urea (Ex-10) and cis-1-(4-chlorophenyl)-3-[1-(4-fluorophenyl)-5-(5-methoxypyridin-2-yl)-2-oxopiperidin-4-yl]urea (Ex-11)

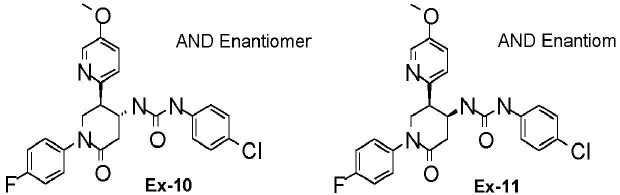

To a stirred solution of compound 1-(4-fluorophenyl)-5-(5-methoxypyridin-2-yl)-2-oxopiperidine-4-carboxylic acid (0.7 g, 2.03 mmol, 1.0 eq) in toluene:THF (30:10 mL) was added TEA (0.56 mL, 4.06 mmol, 2.0 eq) and DPPA (1.1 g, 4.06 mmol, 2.0 eq) and the reaction mixture was stirred at 90 °C for 30 min. After 30 min, 4-chloroaniline (0.389 g, 3.05 mmol, 1.5 eq) was added to the reaction mixture and heated to reflux for 16 h. After completion, (monitored by TLC, TLC system 5% methanol in DCM, R_{f}: 0.3), the reaction mixture was concentrated under reduced pressure diluted with ethyl acetate (150 mL) washed with water (2x100 mL), dried over anhyd. Na₂SO₄ and concentrated under reduced pressure to get the crude product which was purified by column chromatography (230-400 mesh silica gel; 0-3% MeOH in DCM) and again separated by prep-HPLC to afford compound *trans*-1-(4-chlorophenyl)-3-[1-(4-fluorophenyl)-5-(5-methoxypyridin-2-yl)-2-oxopiperidin-4-yl]urea (**Ex-10,** 0.0358 g, 4%) and cis-1-(4-chlorophenyl)-3-[1-(4-fluorophenyl)-5-(5-methoxypyridin-2-yl)-2-oxopiperidin-4-yl]urea (**Ex-11,** 0.0151 g, 2%).
**Ex-10:** 1H-NMR (400 MHz, DMSO-d6): δ 8.48 (s, 1H), 8.24 (s, 1H), 7.33-7.38 (m, 6H), 7.19-7.24 (m, 4H), 6.34 (d*, J* = 7.9 Hz, 1H), 4.45-4.49 (m, 1H), 3.98 (t*, J* = 10.9 Hz, 1H), 3.79 (s, 3H), 3.62-3.67 (m, 1H), 3.48-3.52 (m, 1H), 2.79 (dd, *J* = 5.7, 17.0 Hz, 1H), 2.54-2.58 (m, 1H).
**Ex-11:** 1H-NMR (400 MHz, DMSO-d6): δ 8.62 (s, 1H), 8.23 (d*, J* = 2.4 Hz, 1H), 7.35-7.41 (m, 6H), 7.23-7.27 (m, 4H), 6.54 (d*, J* = 7.7 Hz, 1H), 4.40-4.60 (m, 1H), 3.98-4.06 (m, 2H), 3.80 (s, 3H), 3.70-3.80 (m, 1H), 2.80-2.84 (m, 1H), 2.54-2.55 (m, 1H).

### Synthesis of trans-1-(4-chlorophenyl)-3-[5-(5-chloropyridin-2-yl)-1-(4-fluorophenyl)-2-oxopiperidin-4 yl]urea (Ex-14) and 1-(4-chlorophenyl)-3-[5-(5-chloropyridin-2-yl)-1-(4-fluorophenyl)-2-oxopiperidin-4 yl]urea (Ex-15)

### Synthesis of methyl 2-methoxy-5-(5-nitropyridin-2-yl)pyridine-4-carboxylate

To a solution of methyl 2-methoxy-5-(tetramethyl-1,3,2-dioxaborolan-2-yl)pyridine-4-carboxylate (2.0 g, 7.38 mmol, 1.5 eq) in dioxane (20 ml), K₂CO₃ (1.4 g, 9.84 mmol, 2 eq) and 2-bromo-5-nitropyridine (1.0 g, 4.92 mmol, 1.0 eq) was added at RT. The reaction mixture was then degassed for 30 min, followed by the addition of Pd(PPh₃)₄ (0.2 g, 0.246 mmol, 0.05 eq). The reaction mixture was then heated at 110 °C for 6 h. After completion of reaction (monitored by TLC), reaction mixture was evaporated under reduced pressure to get the crude. Crude was purified by column chromatography to afford methyl 2-methoxy-5-(5-nitropyridin-2-yl)pyridine-4-carboxylate (0.85 g, 60%) as brown gum.

### Synthesis of methyl 5-(5-nitropyridin-2-yl)-2-oxo-1,2-dihydropyridine-4-carboxylate

To a stirred solution of methyl 2-methoxy-5-(5-nitropyridin-2-yl)pyridine-4-carboxylate (0.85 g, 2.94 mmol, 1 eq) in MeOH (20 ml), conc. HCl (4 ml) was added at RT and the reaction mixture was heated at 80 °C for 16 h. After completion of the reaction, monitored by TLC (R_{f}: 0.2, mobile phase, 5% MeOH in DCM), MeOH was removed by distillation, neutralised by saturated NaHCO₃ solution, extracted with 10% MeOH in DCM, dried over Na₂SO₄ and concentrated. Finally compound was purified by column chromatography using 230-400 silica gel and 0-3% MeOH in DCM to afford methyl 5-(5-nitropyridin-2-yl)-2-oxo-1,2-dihydropyridine-4-carboxylate (0.375 g, 46%) as off white solid.

### Synthesis of methyl 1-(4-fluorophenyl)-5-(5-nitropyridin-2-yl)-2-oxo-1,2-dihydropyridine-4-carboxylate

To a stirred solution of methyl 5-(5-nitropyridin-2-yl)-2-oxo-1,2-dihydropyridine-4-carboxylate (0.375 g, 1.36 mmol, 1.0 eq), (4-fluorophenyl) boronic acid (0.381 g, 2.725 mmol, 2 eq), in DCM (10 mL) was added, and the reaction was flushed with oxygen for 15 min. Then Cu-TMEDA (0.19 g, 0.408 mmol, 0.3 eq) was added and the reaction was stirred for 16 h at RT. After completion of the reaction (monitored by TLC, TLC system 40% ethyl acetate in hexane, R_{f}: 0.4), the solvent was removed under reduced pressure to get the crude. Crude was diluted with water. The aqueous layer was extracted twice with Ethyl acetate (2x50 mL). The combined organic was washed with brine, dried over Na₂SO₄, filtered, and concentrated. Crude was purified by column chromatography to afford methyl 1-(4-fluorophenyl)-5-(5-nitropyridin-2-yl)-2-oxo-1,2-dihydropyridine-4-carboxylate (0.3 g, 60%).

### Synthesis of methyl 5-(5-aminopyridin-2-yl)-1-(4-fluorophenyl)-2-oxopiperidine-4-carboxylate

To a stirred solution of methyl 1-(4-fluorophenyl)-5-(5-nitropyridin-2-yl)-2-oxo-1,2-dihydropyridine-4-carboxylate (0.3 g, 0.812 mmol, 1.0 eq) in methanol (25 mL), Pd-C (0.3 g, 10%, moist) was added and the reaction was stirred in a Parr apparatus (17 bar) for 16 h at 70 °C. After completion, (monitored by TLC, TLC system 5% methanol in DCM, R_{f}: 0.2), the reaction mixture was filtered through celite bed and washed 2-3 times with MeOH. The filtrate was concentrated to get methyl 5-(5-aminopyridin-2-yl)-l-(4-fluorophenyl)-2-oxopiperidine-4-carboxylate as white gum (0.21 g, 75%).

### Synthesis of methyl 5-(5-chloropyridin-2-yl)-1-(4-fluorophenyl)-2-oxopiperidine-4-carboxylate

To a stirred solution of 5-(5-aminopyridin-2-yl)-1-(4-fluorophenyl)-2-oxopiperidine-4-carboxylate (1.5 g, 4.368 mmol, 1.0 eq) in ACN (20 ml), tert-butylnitrite (0.902 g, 8.737 mmol, 2 eq) was added and degassed with argon for 30 min. CuCl (0.649 g, 6.553 mmol, 1.5 eq) was added and the reaction was stirred for 16 h at RT. After completion of the reaction, (monitored by TLC, TLC system 5% methanol in DCM, R_{f}: 0.3), the reaction mixture was diluted with ethyl acetate (50 ml) washed with water (2 x 50 ml), dried over Na₂SO₄ and concentrated to get the crude product which was purified by column chromatography (230-400 mesh silica gel; 0 to 2% MeOH in DCM) to afford 5-(5-chloropyridin-2-yl)-1-(4-fluorophenyl)-2-oxopiperidine-4-carboxylate (0.91 g, 57%).

### Synthesis of 5-(5-chloropyridin-2-yl)-1-(4-fluorophenyl)-2-oxopiperidine-4-carboxylic acid

To a stirred solution of 5-(5-chloropyridin-2-yl)-1-(4-fluorophenyl)-2-oxopiperidine-4-carboxylate (0.91 g, 2.508 mmol, 1.0 eq) in THF: H₂O=3:1 (20 ml) was LiOH·H₂O (0.527 g, 12.542 mmol, 5 eq) and the reaction mixture was stirred at RT for 3 h. After completion of the reaction (monitored by TLC, TLC system 5% methanol in DCM, R_{f}: 0.1), reaction mixture was concentrated and acidified with NaHSO₄ solution, removed water by rotary evaporation, triturated with 20% MeOH in DCM, dried over Na₂SO₄ and concentrated to afford 5-(5-chloropyridin-2-yl)-1-(4-fluorophenyl)-2-oxopiperidine-4-carboxylic acid (0.78 g, 89.16%) as a white solid.

### Synthesis of trans-1-(4-chlorophenyl)-3-[5-(5-chloropyridin-2-yl)-1-(4-fluorophenyl)-2-oxopiperidin-4-yl]urea (Ex-14) and 1-(4-chlorophenyl)-3-[5-(5-chloropyridin-2-yl)-1-(4-fluorophenyl)-2-oxopiperidin-4-yl]urea (Ex-15)

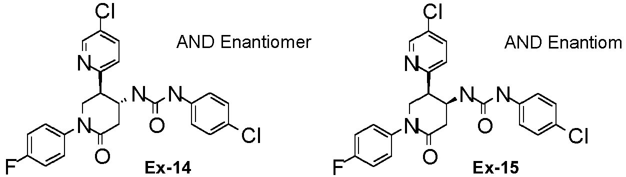

To a stirred solution of 5-(5-chloropyridin-2-yl)-1-(4-fluorophenyl)-2-oxopiperidine-4-carboxylic acid (1.0 g, 2.872 mmol, 1.0 eq) in toluene:THF (30:10 mL) was added TEA (0.8 mL, 5.744 mmol, 2.0 eq) and DPPA (1.58 g, 5.744 mmol, 2.0 eq) and the reaction mixture was stirred at 90 °C for 30 min. After 30 min, 4-chloroaniline (0.55 g, 4.309 mmol, 1.5 eq) was added to the reaction mixture and heated to reflux for 16 h. After completion, (monitored by TLC, TLC system 5% methanol in DCM, R_{f}: 0.3), the reaction mixture was concentrated under reduced pressure, diluted with ethyl acetate (150 mL) washed with water (2x100 mL), dried over anhyd. Na₂SO₄ and concentrated under reduced pressure to get the crude product which was purified by column chromatography (230-400 mesh silica gel; 0-3% MeOH in DCM) and again separated by prep-HPLC to afford compound **Ex-14** (0.144 g, 10.59%) and **Ex-15** (0.128 g, 9.41%).
**Ex-14:** ¹H-NMR (400 MHz, DMSO- d6): δ 8.59 (s, 1H), 8.49 (s, 1H), 7.91 (d, J = 8.4 Hz, 1H), 7.47 (d, J = 8.4 Hz, 1H), 7.31-7.38 (m, 4H), 7.19-7.23 (m, 4H), 6.39 (d, J = 8.2 Hz, 1H), 4.47-4.51 (m, 1H), 4.01 (t, J = 10.8 Hz, 1H), 3.56-3.70 (m, 2H), 2.79 (dd, J = 5.7, 17.1 Hz, 1H), 2.55-2.62 (m, 1H).
**Ex-15:** ¹H-NMR (400 MHz, DMSO-d6): δ 8.56 (s, 1H), 8.51 (s, 1H), 7.93 (d*, J* = 8.6 Hz, 1H), 7.49 (d*, J* = 8.4 Hz, 1H), 7.33-7.39 (m, 4H), 7.23-7.28 (m, 4H), 6.55 (d*, J* = 7.6 Hz, 1H), 4.55-4.60 (m, 1H), 3.95-4.09 (m, 2H), 3.80-3.85 (m, 1H), 2.88 (dd, *J* = 5.2, 17.2 Hz, 1H).

**Preparative HPLC and SFC conditions**

| Chromatographic conditions | Example # or name first eluting | Example # or name second eluting |
|---|---|---|
| Column ID: EXTENDC-18 (150X4.6) 5u,Diluent:DMSO; A:ACN B: 10mM NH₄OAc in water;Flow: 1.0ml/min | **Ex-5** | **Ex-6** |
| | Rₜ: 9.729 min | Rₜ: 9.908 min |
| Column name: Kinetex EVO C18 (250 x 21.2 mm, 5µ) ambient temperature flow rate of 16 mL/min. Mobile phase: A = 10 Mm Ammonium acetate in water, B=Acetonitrile; Gradient Profile: Mobile phase initial composition of 80% A and 20% B, then 65% A and 35% B in 1 min, then to 40% A and 60% B in 24 min., then to 100% B in 24.5 min., held this composition up to 27.5 min. for column washing, then returned to initial composition in 28 min. and held till 32 min. | **Ex-14** | **Ex-15** |
| | Rₜ: 20.95 min | Rₜ: 23.08 min |
| Column Name : Chiralpak IG (4.6 x 250 mm),5µ; ARD/K/; Mobile Phase : Hexane/DCM/EtOH : 50/25/25; Flow Rate : 1.0 ml/min; Solubility : MeOH | **Ex-22** | **Ex-23** |
| | Rₜ: 4.88 min | Rₜ: 8.91 min |
| Column Name : Chiralpak IE (4.6 x 250 mm),5µ; ARD/K/7868; Mobile Phase : Hexane/EA/EtOH/isopropylamine: 70/15/15/0.1; Flow Rate : 1.0 ml/min; Solubility : MeOH | **Ex-29** | **Ex-30** |
| | Rₜ: 11.14 min | Rₜ: 14.79 min |
| *Column Name : Chiralpak IG (4.6 x 250 mm), 5µ; ARD*/*K*/*; Mobile phase : Hexane*/*EA*/*EtOH*/*Isopropylamine : 50*/*25*/*25*/*0.1; Flow Rate : 1.0 ml*/*min; Solubility :* MeOH | **Ex-35** | **Ex-36** |
| | Rₜ: 3.98 min | Rₜ: 6.01 min |
| *Column Name : Chiralpak IG (4.6 x 250 mm), 5µ; ARD*/*K*/*; Mobile phase : Hexane*/*DCM*/*EtOH: 60*/*20*/*20; Flow Rate : 1.0 ml*/*min; Solubility :* MeOH | **Ex-37** | **Ex-38** |
| | Rₜ: 4.67 min | Rₜ: 7.55 min |
| Column Name : Chiralpak IG (4.6 x 250 mm), 5µ; ARD/K/; Mobile phase : Hexane/EA/EtOH/ isopropylamine: 50/25/25/0.1; Flow Rate : 1.0 ml/min; Solubility : MeOH | **Ex-39** | **Ex-40** |
| | Rₜ: 7.46 min | Rₜ: 14.08 min |
| Instrument Method M-2-50; Inj. Vol. 10; Solvent 0.5% isopropylamine in MeOH; Column (R,R)-WHELK-01; Temp. 34.9; Flow 2; % Modifier 50; Pressure 100 | **Ex-42** | **Ex-43** |
| | Rₜ: 4.19 min | Rₜ: 5.62 min |
| Column Name : Chiralpak IG (4.6 x 250 mm), 5µ; ARD/K/; Mobile phase : Hexane/EA/EtOH/Isopropylamine: 70/15/15/0.1; Flow Rate : 1.0 ml/min; Solubility : MeOH | **Ex-44** | **Ex-45** |
| | Rₜ: 5.50 min | Rₜ: 7.09 min |
| Column Name : Chiralpak IG (4.6 x 250 mm),5µ; ARD/K/; Mobile Phase : Hexane/EA/EtOH/Isopropylamine : 50/25/25/0.1; Flow Rate : 1.0 ml/min; Solubility : MeOH | **Ex-46** | **Ex-47** |
| | Rₜ: 4.23 min | Rₜ: 7.49 min |
| Column Name : Chiralpak IA (4.6 x 250 mm),5µ; ARD/K/C-93; Mobile Phase : Hexane/EA/EtOH/Isopropylamine: 50/25/25/0.1; Flow Rate : 1.0 ml/min; Solubility : MeOH | **Ex-48** | **Ex-49** |
| | Rₜ: 3.93 min | Rₜ: 14.43 min |
| Column Name : Chiralpak IA (4.6 x 250 mm), 5µ; ARD/K/C-93; Mobile phase : Hexane/EA/EtOH/Isopropylamine : 70/15/15/0.1; Flow Rate : 1.0 ml/min; Solubility : MeOH | **Ex-50** | **Ex-51** |
| | Rₜ: 12.29 min | Rₜ: 14.88 min |
| Column Name : Chiralpak IC (4.6 x 250 mm), 5µ; ARD/K/7788; Mobile phase : Hexane/DCM/EtOH : 60/20/20; Flow Rate : 1.0 ml/min; Solubility : MeOH | **Ex-52** | **Ex-53** |
| | Rₜ: 11.43 min | Rₜ: 16.44 min |
| Column Name : Chiralpak IC (4.6 x 250 mm), 5µ; ARD/K/7788; Mobile phase : Hexane/DCM/EtOH : 60/20/20; Flow Rate : 1.0 ml/min; Solubility : MeOH | **Ex-54** | **Ex-55** |
| | Rₜ: 9.44 min | Rₜ: 11.10 min |
| Column Name : Chiralpak IC (4.6 x 250 mm), 5µ; ARD/K/7788; Mobile phase : Hexane/DCM/EtOH/DEA : 80/10/10/0.1; Flow Rate : 1.0 ml/min; Solubility : MeOH | **Ex-56** | **Ex-57** |
| | Rₜ: 9.81 min | Rₜ: 11.54 min |
| Column Name : Chiralpak IG (4.6 x 250 mm), 5µ; ARD/K/; Mobile phase : Hexane/EA/EtOH/ isopropylamine: 50/25/25/0.1; Flow Rate : 1.0 ml/min; Solubility : MeOH | **Ex-58** | **Ex-59** |
| | Rₜ: 6.70 min | Rₜ: 9.66 min |
| Column Name : Chiralpak IG (4.6 x 250 mm), 5µ; ARD/K/; Mobile phase : Hexane/ETOH/DCM : 60/20/20; Flow Rate : 1.0 ml/min; Solubility : MeOH | **Ex-62** | **Ex-63** |
| | Rₜ: 4.76 min | Rₜ: 6.42 min |
| Column Name : Chiralpak IG (4.6 x 250 mm),5µ; ARD/K/; Mobile Phase : Hexane/EA/EtOH/ isopropylamine: 50/25/25/0.1; Flow Rate : 1.0 ml/min; Solubility : MeOH | **Ex-77** | **Ex-78** |
| | Rₜ: 3.93 min | Rₜ: 4.70 min |
| Instrument Method M-2-40F; Inj. Vol. 10; Solvent 0.5% isopropylamine in MeOH; Column IC; Flow 2; % Modifier 40; Pressure 100 | **Ex-124** | **Ex-125** |
| | Rₜ: 10.45 min | Rₜ: 14.16 min |
| Column Name : Chiralpak IG (4.6 x 250 mm),5µ; ARD/K/; Mobile Phase : Hexane/EA/EtOH/Isopropylamine: 70/15/15/0.1; Flow Rate : 1.0 ml/min; Solubility : MeOH | trans-tert-butyl 4-{[4-{[(4-chlorophenyl) carbamoyl]am ino}-3-(5-chlorothiophe n-2-yl)piperidin-1-yl]methyl}pip eridine-1-carboxylate ent-1 | trans-tert-butyl 4-{[4-{[(4-chlorophenyl) carbamoyl]am ino}-3-(5-chlorothiophe n-2-yl)piperidin-1-yl]methyl}pip eridine-1-carboxylate ent-2 |
| | Rₜ: 5.34 min | Rₜ: 8.03 min |
| Column Name : Chiralpak IC (4.6 x 250 mm),5µ; ARD/K/7788; Mobile Phase : Hexane/EtOH/Isopropylamine: 80/20/0.1; Flow Rate : 1.0 ml/min; Solubility : MeOH | **Ex-85** | **Ex-86** |
| | Rₜ: 4.81 min | Rₜ: 5.39 min |
| Inj. Vol. 10; Solvent 0.5% Isopropylamine in MeOH; Column (R,R)-WHELK-01; Temp. 34.9; Flow 2; % Modifier 40; Pressure 100 | **Ex-98** | **Ex-99** |
| | Rt: 5.12 min | Rₜ: 6.39 min |
| Inj. Vol. 10; Solvent 0.5% Isopropylamine in MeOH; Column IE; Temp. 34.9; Flow 2; % Modifier 40; Pressure 100 | **Ex-100** | **Ex-101** |
| | Rₜ: 6.07 min | Rₜ: 9.62 min |
| Column Name : Chiralpak IG (4.6 x 250 mm), 5µ; ARD/K/; Mobile phase : Hexane/DCM/EtOH : 30/30/40; Flow Rate : 1.0 ml/min; Solubility : MeOH | **Ex-170** | **Ex-171** |
| | Rₜ: 3.80 min | Rₜ: 4.66 min |
| Column Name : Chiralpak IC (4.6 x 250 mm), 5µ; ARD/K/7788; Mobile phase :Hexane/EtOH/Isopropylamine: 80/20/0.1; Flow Rate : 1.0 ml/min; Solubility : MeOH | **Ex-172** | **Ex-173** |
| | Rₜ: 4.83 min | Rₜ: 5.09 min |
| Column Name : Chiralpak IG (4.6 x 250 mm),5µ; ARD/K/; Mobile Phase : Hexane/EA/EtOH/Isopropylamine: 50/25/25/0.1; Flow Rate : 1.0 ml/min; Solubility : MeOH | **Ex-174** | **Ex-175** |
| | Rₜ: 4.18 min | Rₜ: 7.28 min |
| Column Name : Chiralpak IA (4.6 x 250 mm), 5µ; ARD/K/C-93; Mobile phase : Hexane/EA/EtOH/Isopropylamine: 50/25/25/0.1; Flow Rate : 1.0 ml/min; Solubility : MeOH | **Ex-176** | **Ex-177** |
| | Rₜ: 5.33 min | Rₜ: 9.07 min |
| Column Name : Chiralpak IA (4.6 x 250 mm), 5µ; ARD/K/C-93; Mobile phase : Hexane/EA/EtOH/Isopropylamine: 50/25/25/0.1; Flow Rate : 1.0 ml/min; Solubility : MeOH | **Ex-178** | **Ex-179** |
| | Rₜ: 6.03 min | Rₜ: 7.56 min |
| Column :Gemini C-18 (100x 4.6 mm ; 5µ) Diluent: DMSO; Mobile Phase : A: 10 mM NH₄OAC in WATER, B:ACN | **Ex-112** | |
| | Rₜ: 7.62 min | |
| Column: Chiralpak IG (250x20 mm) 5µ; Flow : 25 g/min; Mobile Phase: 50 % CO₂ + 50 % (0.5% Isopropylamine in MeOH); ABPR: 100 bar; Temp.: 35° C | **Ex-16** | **Ex-17** |
| | Rₜ: 3.52 min | Rₜ: 7.23 min |
| Column: Chiralpak IG (250x20 mm) 5µ; Flow : 25 g/min; Mobile Phase: 45 % CO₂ + 55 % (0.5% Isopropylamine in MeOH); ABPR: 100 bar; Temp.: 35° C | **Ex-168** | **Ex-169** |
| | Rₜ: 2.1 min | Rₜ: 3.31 min |
| Column: Chiralpak IC (250x20 mm) 5µ ; Flow : 25 g/min; Mobile Phase: 70 % CO₂ + 30 % (0.5% Isopropylamine in MeOH); ABPR: 115 bar; Temp.: 35° C | **Ex-187** | **Ex-188** |
| | Rₜ: 9.54 min | ₜ: 12.12 min |
| Column: Amylose SA (250x20 mm) 5µ ; Flow : 25 g/min; Mobile Phase: 60 % CO₂ + 40 % (0.5% Isopropylamine in MeOH); ABPR: 100 bar; Temp.: 35° C | **Ex-189** | **Ex-190** |
| | Rₜ: 3.31 min | Rₜ: 5.32 min |
| Column: Chiralpak IG (250x20 mm) 5µ; Flow : 25 g/min; Mobile Phase: 45 % CO₂ + 55 % (0.5% Isopropylamine in MeOH); ABPR: 130 bar; Temp.: 35° C | **Ex-191** | **Ex-192** |
| | Rₜ: 6.92 min | Rₜ: 15.28 min |

### Biological data

### Assay method for measuring agonistic activity of compounds in hFPR2-Aq-CHO cell

Cryo-vial containing 6x10⁶ cells (hFPR1-Gα15-CHO or hFPR2-Aq-CHO) was thawed in a 37°C water bath. Cells were suspended in 10ml of respective complete growth media (F12(1X)HAM media (Gibco; Cat#11765); 10% HI-FBS (Gibco; Cat#10082); 0.4 mg/ml Geneticin (Gibco; Cat#10131); 0.25 mg/ml Zeocin (Invitrogen; Cat#R25001)) in a 15ml centrifuge tube. The cell viability was checked with the help of Trypan Blue dye. Upon washing the cells, those were plated in a 384-well sterile clear bottom black plate (Greiner Bioone Cat# 781091) so that, each well contained 10,000 cells in 40µl complete growth media. The plate was incubated in a 5% CO₂ incubator at 37°C for 18 hours.
Before assay on the next day, the cell plating media was removed from each well of the plate by decanting and gentle tapping. 30µl of 0.5X Calcium 5 dye solution (0.5X FLIPR Calcium 5 dye (Molecular devices Cat# R8186); HBSS (Invitrogen; Cat#14025); 20mM HEPES (Sigma; Cat#H0887); 2.5mM Probenecid (Sigma; Cat#P8761); 0.025% Pluronic F-127 (Sigma; Cat#P2443); pH adjusted to 7.4) was added to each well. The plate was then incubated at 37°C for 30 minutes. Following which the plate was equilibrated at room temperature for 10 minutes before placing it in a 384 well FLIPR for assay. Compounds were dissolved in DMSO and serially diluted following 11 point half log (3.16 fold) dilution with a starting concentration of 2mM (Final assay concentration 10µM). Aliquots of above mentioned each dilution was mixed with assay buffer (HBSS (Invitrogen; Cat#14025); 20mM HEPES (Sigma; Cat#H0887); 2.5mM Probenecid (Sigma; Cat#P8761); 0.05% gelatin (Sigma; Cat#G1890); 0.1% BSA (Sigma; Cat#A3059); pH adjusted to 7.4) just before performing the assay. Compounds were added to the respective wells of the assay ready cell plate with the help of the FLIPR (FLIPR Tetra) and fluorescence readings were captured for 5 minutes to measure any agonistic response of the compounds. The increase in fluorescence readings from the basal reading in presence of the compounds were compared with that of the control wells (wells having no compound) to calculate the agonistic activity of the compounds. The EC₅₀ values of the compounds were determined using the Graph pad Prism software. The results are summarized in the table below.

| **Ex-#** | h FPR2 FLIPR EC₅₀ [µM] | **Ex-#** | h FPR2 FLIPR EC₅₀ [µM] |
|---|---|---|---|
| **Ex-1** | 0.00600 | **Ex-201** | 0.00060 |
| **Ex-2** | 0.01100 | **Ex-202** | 0.00140 |
| **Ex-3** | 0.01000 | **Ex-203** | 0.00070 |
| **Ex-4** | 0.01100 | **Ex-204** | 0.00400 |
| **Ex-5** | 0.00016 | **Ex-205** | 0.00400 |
| **Ex-6** | 0.00600 | **Ex-206** | 0.00200 |
| **Ex-7** | 0.00040 | **Ex-207** | 0.00900 |
| **Ex-8** | 0.00100 | **Ex-208** | 0.00070 |
| **Ex-9** | 0.00200 | **Ex-209** | 0.00030 |
| **Ex-10** | 0.00005 | **Ex-210** | 0.00500 |
| **Ex-11** | 0.00600 | **Ex-211** | 0.00700 |
| **Ex-12** | 0.00900 | **Ex-212** | 0.00030 |
| **Ex-13** | 0.00200 | **Ex-213** | 0.00105 |
| **Ex-14** | 0.00040 | **Ex-214** | 0.00080 |
| **Ex-15** | 0.00700 | **Ex-215** | 0.00030 |
| **Ex-16** | 0.00090 | **Ex-216** | 0.00200 |
| **Ex-17** | 30%@0.1µM | **Ex-217** | 0.00600 |
| **Ex-18** | 0.00080 | **Ex-218** | 0.00400 |
| **Ex-19** | 0.00080 | **Ex-43** | 0.00003 |
| **Ex-20** | 0.00100 | **Ex-44** | 0.00100 |
| **Ex-21** | 0.00700 | **Ex-45** | 10%@0.1µM |
| **Ex-22** | 0.00020 | **Ex-46** | 0.00040 |
| **Ex-23** | 28%@0.1µM | **Ex-47** | 0.06500 |
| | | **Ex-48** | 0.07200 |
| **Ex-25** | 17%@0.1µM | **Ex-49** | 0.00030 |
| **Ex-26** | 0.00100 | **Ex-50** | 40%@0.1µM |
| **Ex-27** | 0.00600 | **Ex-51** | 0.00040 |
| **Ex-28** | 0.01700 | **Ex-52** | 0.00050 |
| **Ex-29** | 0.01100 | **Ex-53** | 0.05000 |
| **Ex-30** | 0.00009 | **Ex-54** | 0.00060 |
| **Ex-31** | 0.00500 | **Ex-55** | 0.04000 |
| **Ex-32** | 0.00300 | **Ex-56** | 0.00090 |
| **Ex-33** | 0.01100 | **Ex-57** | 0.05500 |
| **Ex-34** | 0.00400 | **Ex-58** | 0.00040 |
| **Ex-35** | 0.00006 | **Ex-59** | 26%@0.1µM |
| **Ex-36** | 0.08500 | **Ex-60** | 0.00004 |
| **Ex-37** | 0.00013 | **Ex-61** | 0.00060 |
| **Ex-38** | 0.05400 | **Ex-62** | 0.00020 |
| **Ex-39** | 0.00030 | **Ex-63** | 44%@0.1µM |
| **Ex-40** | 10%@0.1M | **Ex-64** | 0.00006 |
| **Ex-41** | 0.00300 | **Ex-65** | 0.00009 |
| **Ex-42** | 0.00100 | **Ex-66** | 0.00020 |
| **Ex-85** | 0.01400 | **Ex-67** | 0.00008 |
| **Ex-86** | 19%@0.1µM | **Ex-68** | 0.00009 |
| **Ex-87** | 0.00100 | **Ex-69** | 0.00007 |
| **Ex-88** | 0.00120 | **Ex-70** | 0.00200 |
| **Ex-89** | 0.00200 | **Ex-71** | 0.00200 |
| **Ex-90** | 0.03200 | **Ex-72** | 0.00200 |
| **Ex-91** | 0.01100 | **Ex-73** | 0.00400 |
| **Ex-92** | 0.00400 | **Ex-74** | 0.00200 |
| **Ex-93** | 0.00300 | **Ex-75** | 0.00300 |
| **Ex-94** | 0.00200 | **Ex-76** | 0.00200 |
| **Ex-95** | 0.01200 | **Ex-77** | 0.00006 |
| **Ex-96** | 0.00400 | **Ex-78** | 0.05200 |
| **Ex-97** | 0.00400 | **Ex-79** | 0.02300 |
| **Ex-98** | 26%@0.1µM | **Ex-80** | 0.00090 |
| **Ex-99** | 0.00040 | **Ex-81** | 0.01300 |
| **Ex-100** | 0.09300 | **Ex-82** | 0.03700 |
| **Ex-101** | 0.00011 | **Ex-83** | 0.00020 |
| **Ex-102** | 0.02700 | **Ex-84** | 0.03300 |
| **Ex-103** | 0.00200 | **Ex-130** | 0.00400 |
| **Ex-104** | 0.00600 | **Ex-131** | 38%@0.1µM |
| **Ex-105** | 0.01100 | **Ex-132** | 0.03900 |
| **Ex-106** | 41%@0.1µM | **Ex-133** | 0.01300 |
| **Ex-107** | 0.04600 | **Ex-134** | 0.00200 |
| **Ex-108** | 0.00700 | **Ex-135** | 0.01700 |
| **Ex-109** | 0.00120 | **Ex-136** | 0.00140 |
| **Ex-110** | 0.04900 | **Ex-137** | 0.00020 |
| **Ex-111** | 0.00020 | **Ex-138** | 0.00700 |
| **Ex-112** | 0.00120 | **Ex-139** | 34%@0.1µM |
| **Ex-113** | 0.00120 | **Ex-140** | 0.00090 |
| **Ex-114** | 0.00060 | **Ex-141** | 0.00120 |
| **Ex-115** | 0.00050 | **Ex-142** | 0.00110 |
| **Ex-116** | 0.00900 | **Ex-143** | 0.00012 |
| **Ex-117** | 0.00300 | **Ex-144** | 0.00100 |
| **Ex-118** | 0.00500 | **Ex-145** | 0.00030 |
| **Ex-119** | 0.00900 | **Ex-146** | 0.00200 |
| **Ex-120** | 0.00070 | **Ex-147** | 0.00040 |
| **Ex-121** | 0.00120 | **Ex-148** | 0.00600 |
| **Ex-122** | 0.00300 | **Ex-149** | 25%@0.1µM |
| **Ex-123** | 0.01800 | **Ex-150** | 0.00300 |
| **Ex-124** | 0.00003 | **Ex-151** | 0.03500 |
| **Ex-125** | 0.00009 | **Ex-152** | 25%@0.1µM |
| **Ex-126** | 0.00120 | **Ex-153** | 0.01200 |
| **Ex-127** | 0.00016 | **Ex-154** | 0.01000 |
| **Ex-128** | 0.00500 | **Ex-155** | 0.00009 |
| **Ex-129** | 0.00030 | **Ex-156** | 0.05800 |
| **Ex-175** | 17%@0.1µM | **Ex-157** | 0.08100 |
| **Ex-176** | 5%@0.1µM | **Ex-158** | 0.03100 |
| **Ex-177** | 0.00140 | **Ex-159** | 0.05700 |
| **Ex-178** | 0.2%@0,1µM (dystomer) | **Ex-160** | 0.08800 |
| **Ex-179** | 0.00070 | **Ex-161** | 0.02800 |
| **Ex-180** | 0.00050 | **Ex-162** | 34%@0.1µM |
| **Ex-181** | 0.00090 | **Ex-163** | 0.00080 |
| **Ex-182** | 0.00200 | **Ex-164** | 0.05600 |
| **Ex-183** | 0.08900 | **Ex-165** | 0.01100 |
| **Ex-184** | 0.00140 | **Ex-167** | 0.08500 |
| **Ex-185** | 19%@0.1µM | **Ex-168** | 0.00090 |
| **Ex-186** | 0.02700 | **Ex-169** | 23%@0.1µM |
| **Ex-187** | 0.00140 | **Ex-170** | 0.00030 |
| **Ex-188** | 0.02900 | **Ex-171** | 3%@0.1µM (dystomer) |
| **Ex-189** | 0.00170 | **Ex-172** | 0.00018 |
| **Ex-190** | 8%@0.1µM | **Ex-173** | 44%@0.1µM |
| **Ex-191** | 0.00070 | **Ex-174** | 0.00140 |
| **Ex-192** | 0.05800 | **Ex-219** | 0.03800 |
| **Ex-193** | 0.00100 | **Ex-220** | 0.00040 |
| **Ex-194** | 0.00130 | **Ex-221** | 0.00090 |
| **Ex-195** | 0.00050 | **Ex-222** | 0.00030 |
| **Ex-196** | 0.00070 | **Ex-223** | 0.00080 |
| **Ex-197** | 0.00090 | **Ex-224** | 0.00500 |
| **Ex-198** | 0.00040 | **Ex-225** | 0.00800 |
| **Ex-199** | 0.00050 | **Ex-226** | 0.00090 |
| **Ex-200** | 0.00040 | | |

### Prophetic examples

Using the appropriate building blocks the following compounds could be synthesized in analogy to the procedures described above.

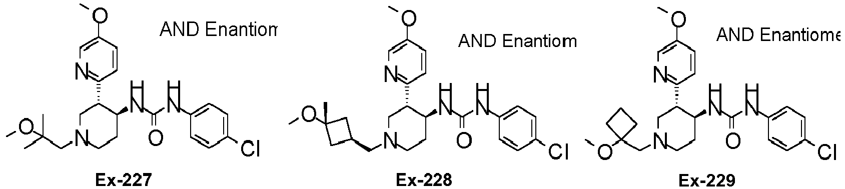

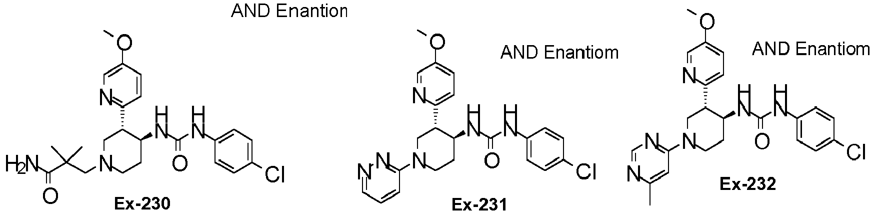

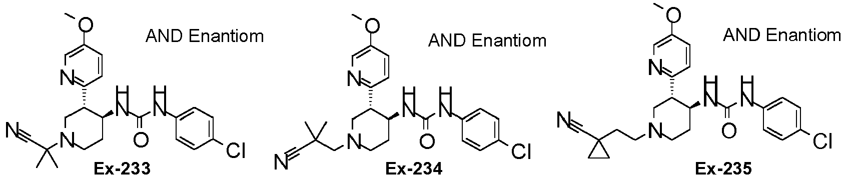

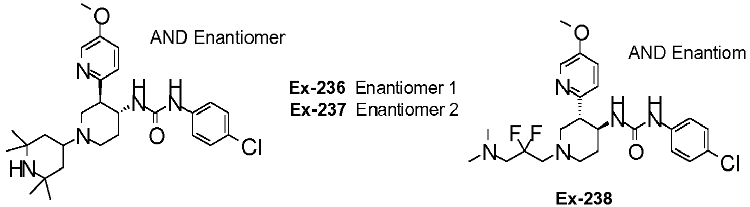

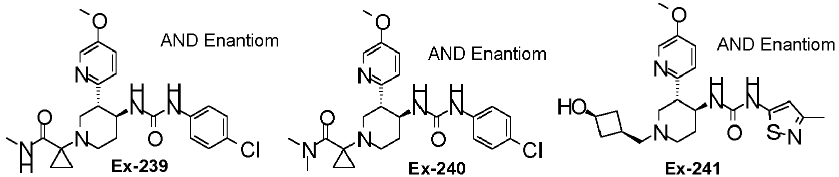

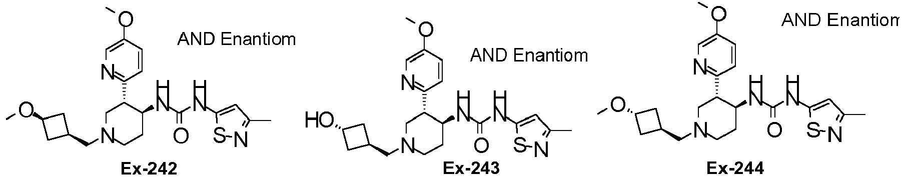

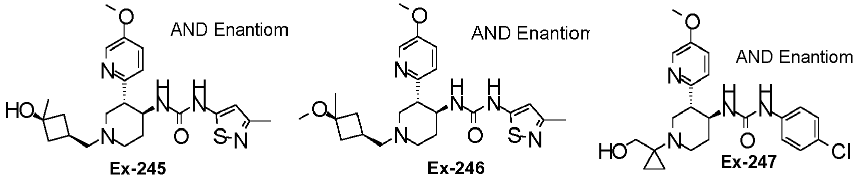

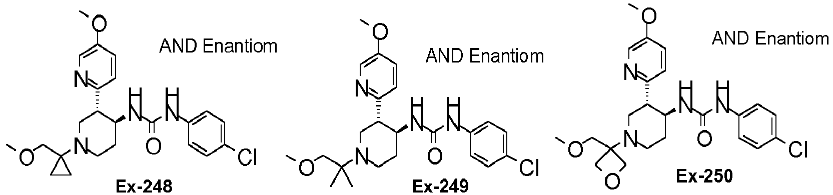

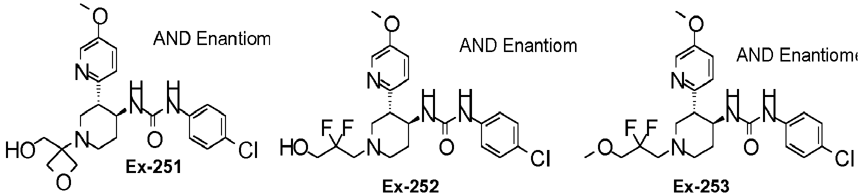

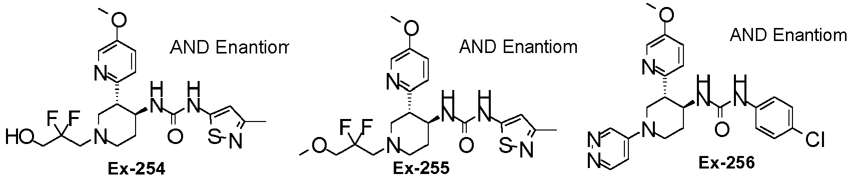

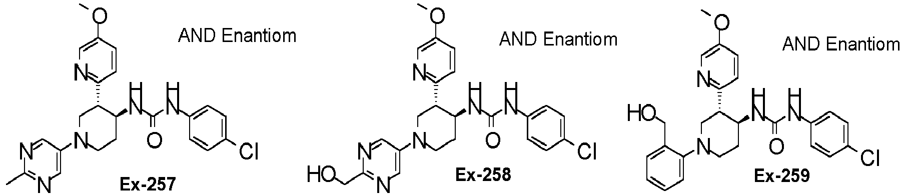

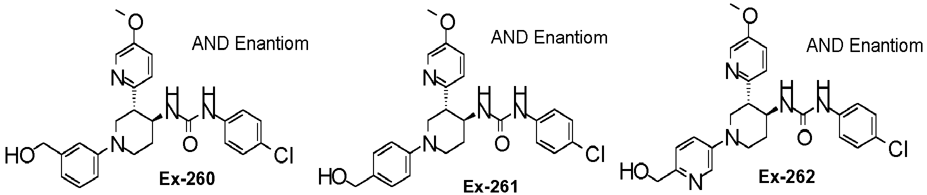

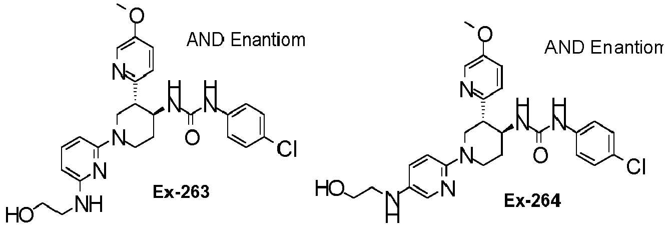

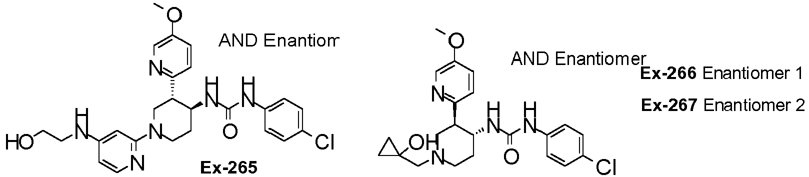

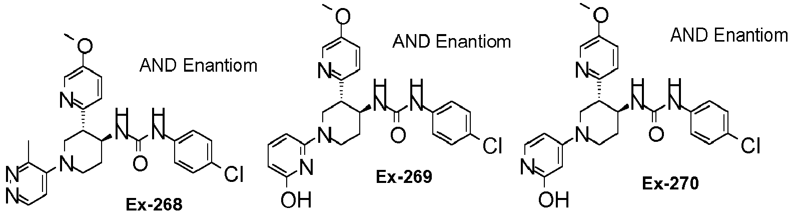

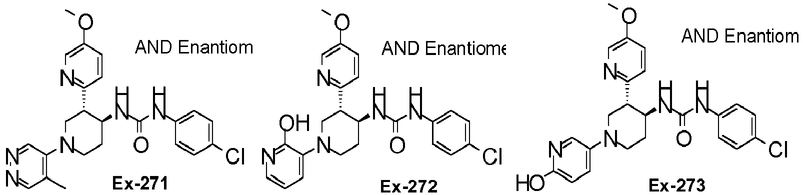

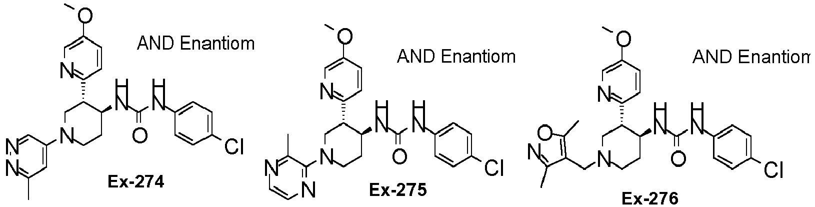

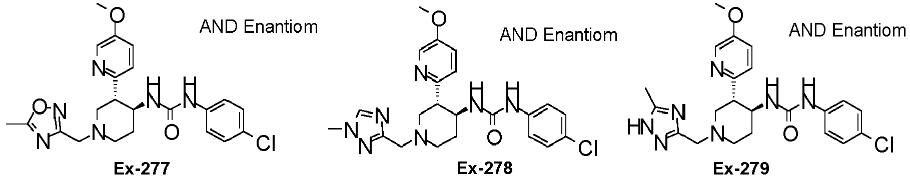

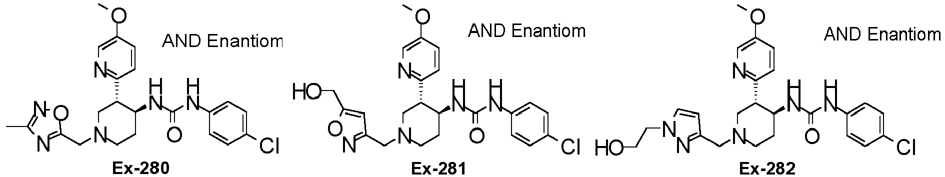

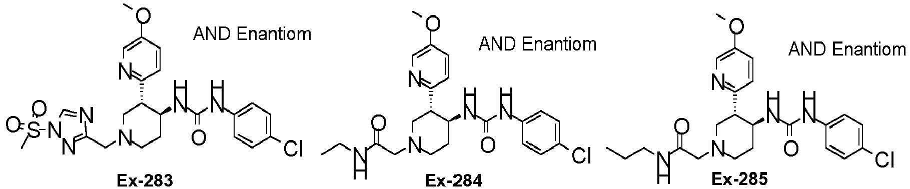

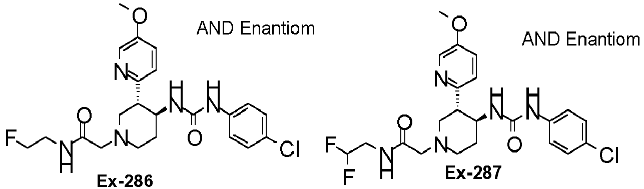

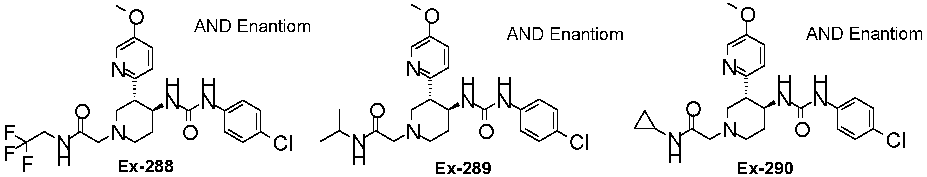

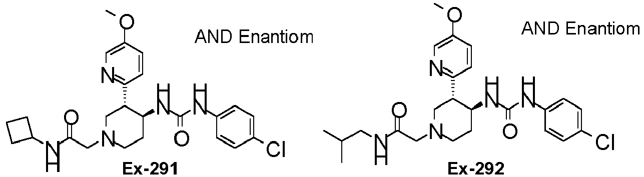

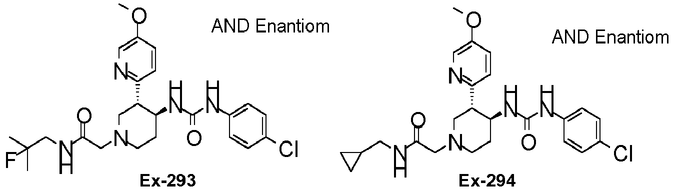

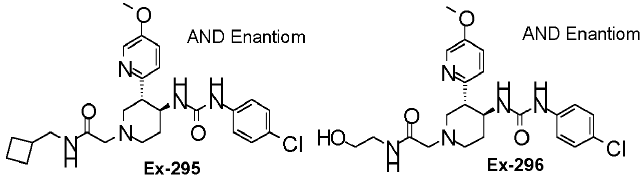

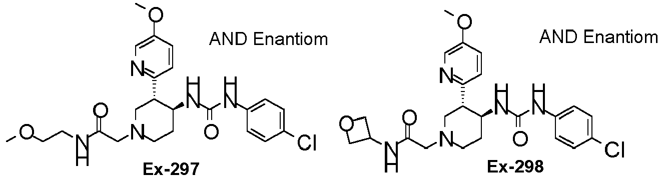

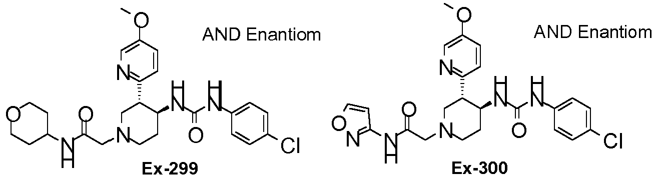

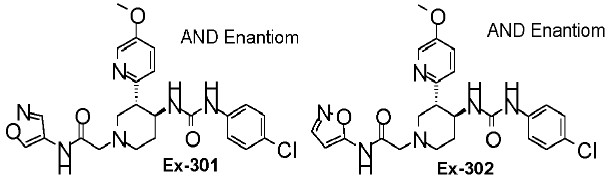

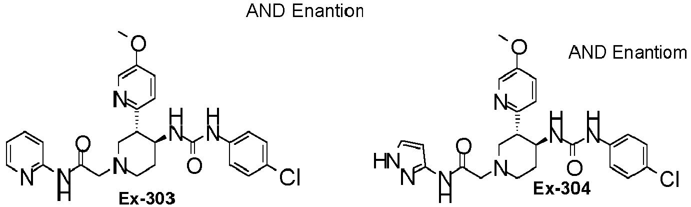

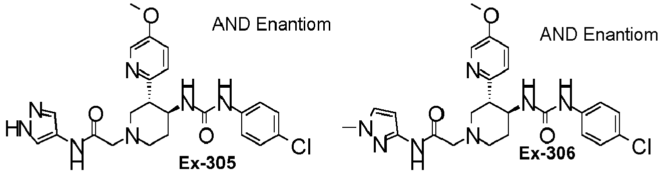

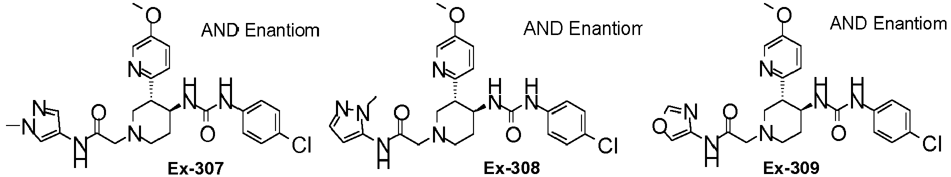

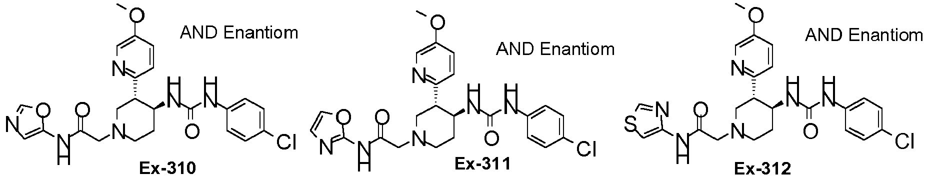

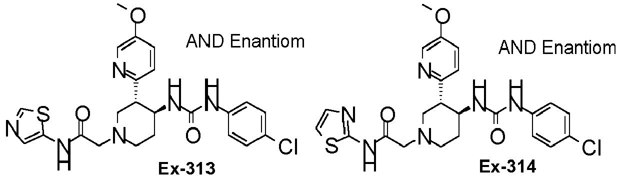

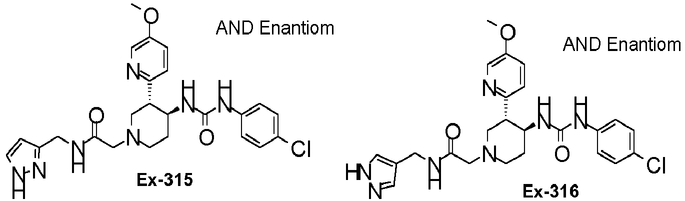

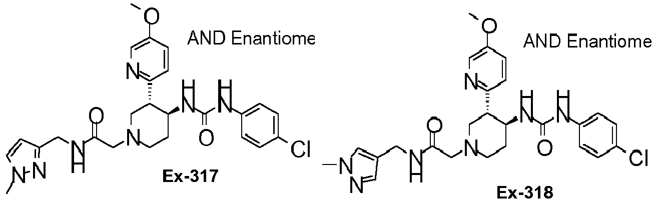

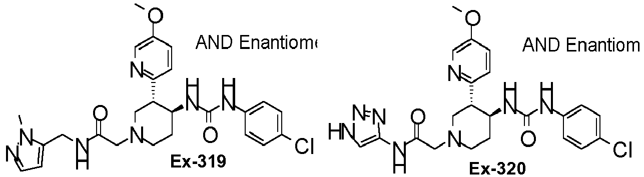

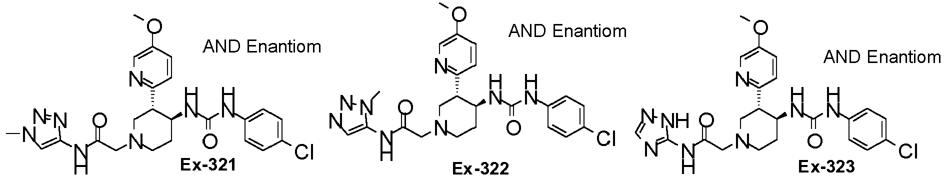

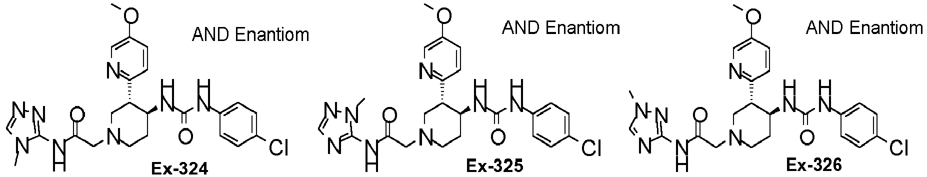

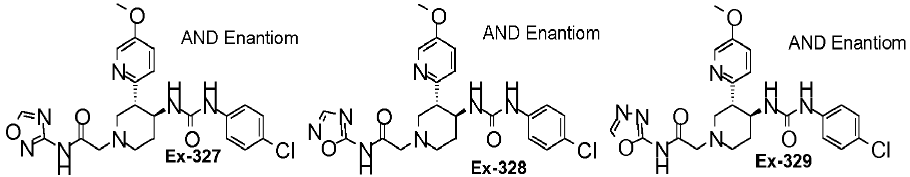

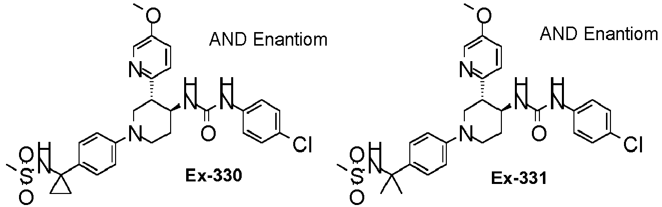

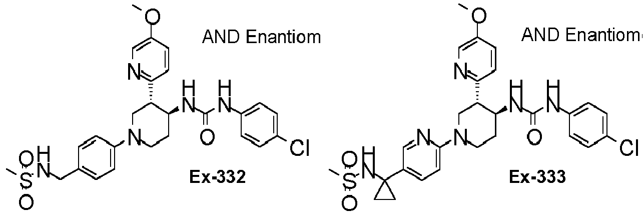

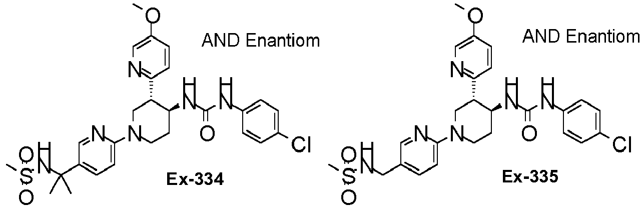

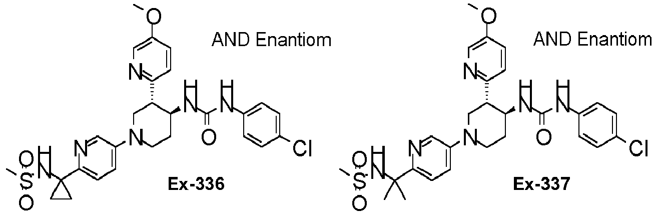

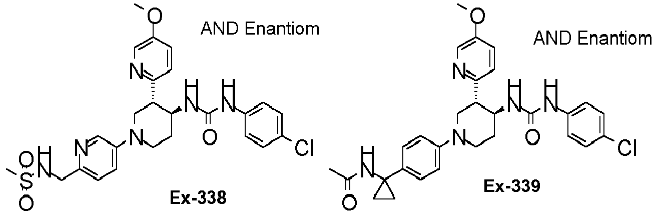

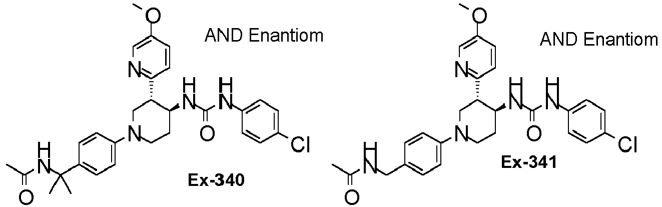

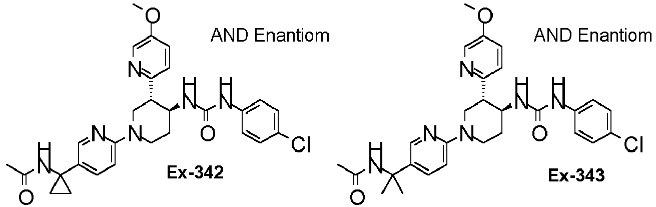

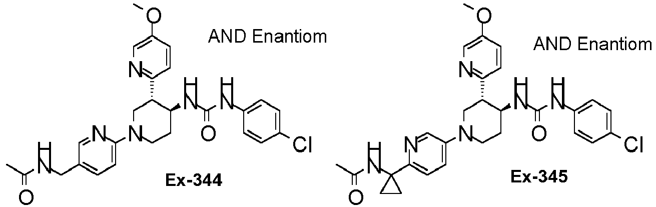

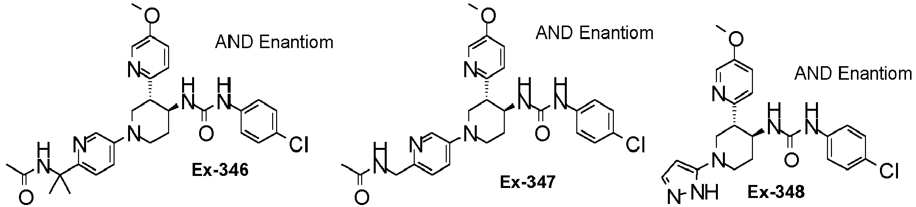

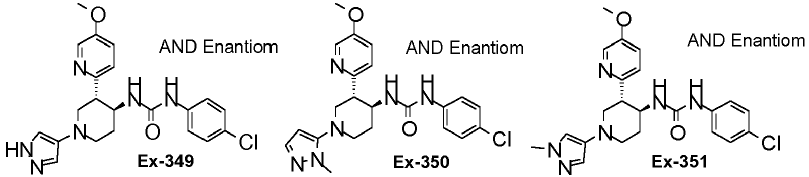

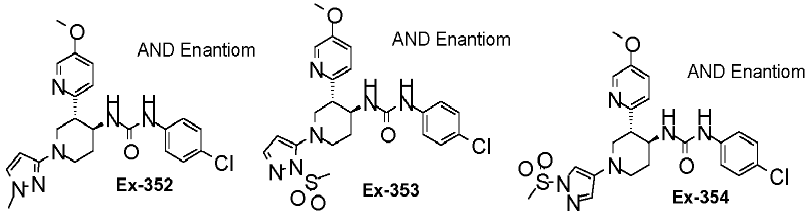

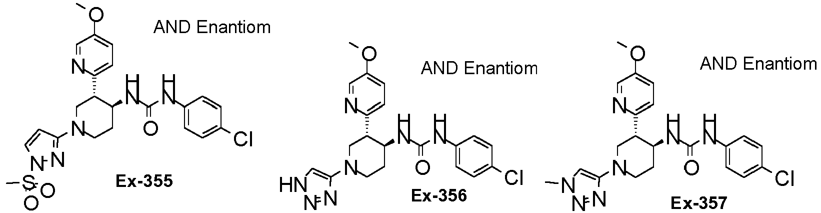

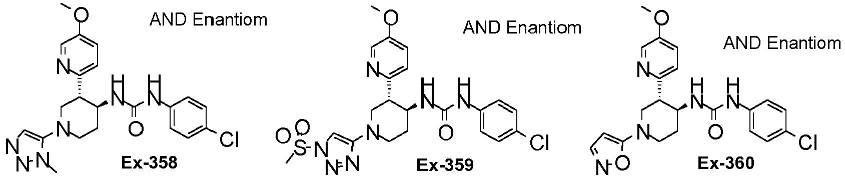

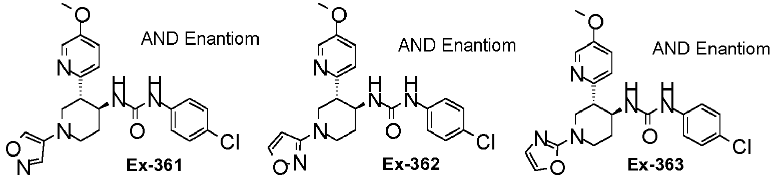

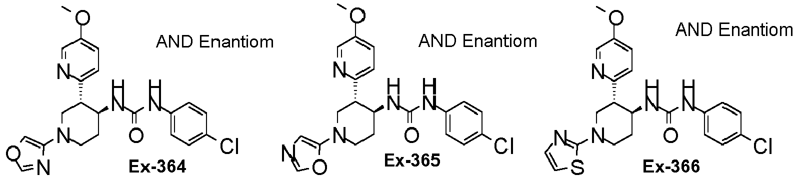

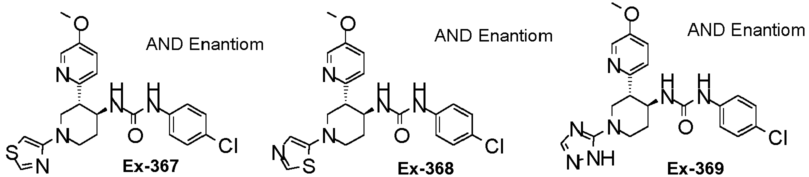

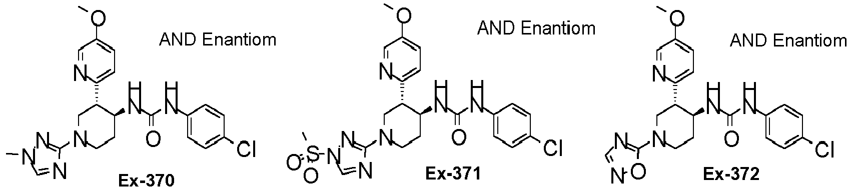

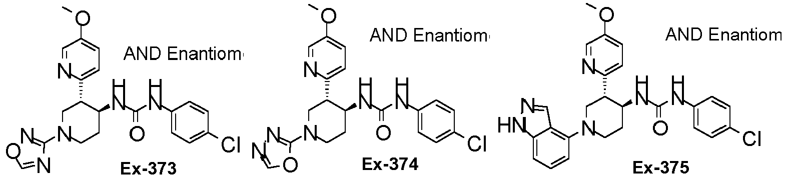

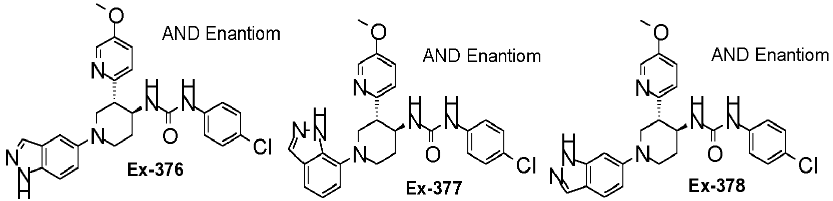

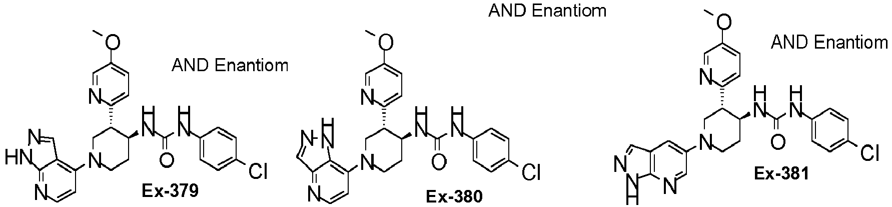

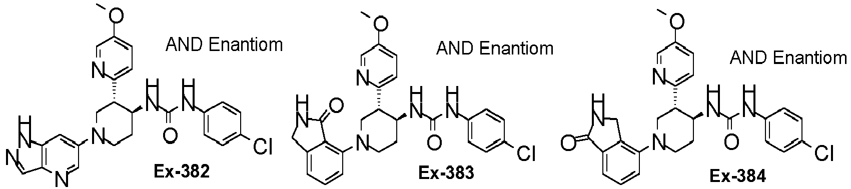

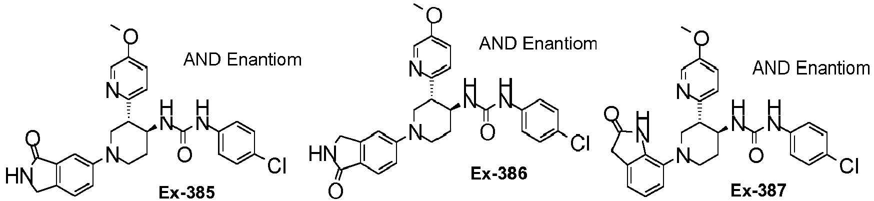

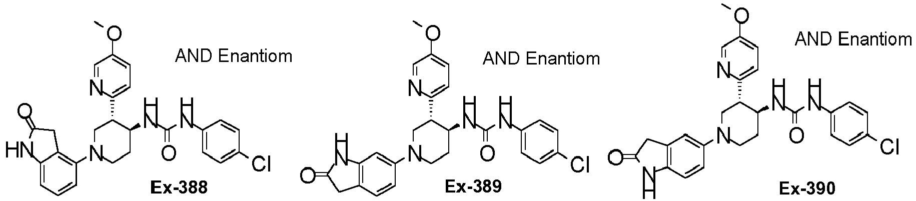

## Claims

1. A compound according to general formula (I) 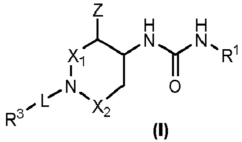 wherein
- X₂ represents CH₂ or C(O) and X₁ represents CH₂; or
- X₁ represents CH₂ or C(O) and X₂ represents CH₂;
and
R¹ represents phenyl or 5 or 6-membered heteroaryl,
Z represents a 5 or 6 membered heteroaryl which is monosubstituted, disubstituted or trisubstituted with R² which in each case independently is selected from the group consisting of O-C₁₋₆-alkyl, H, F, Cl, Br, CN, C₁₋₆-alkyl, C₃₋₆-cycloalkyl, CHF₂, CH₂F, CF₃, OH, OCHF₂, OCH2F, OCF₃, S-C₁₋₆-alkyl, S(O)-C₁₋₆-alkyl, S(O)₂-C₁₋₆-alkyl, O-C₃₋₆-cycloalkyl, S-C₃₋₆-cycloalkyl, S(O)-C₃₋₆-cycloalkyl, S(O)₂-C₃₋₆-cycloalkyl, NH₂, N(H)(C₁₋₆-alkyl), N(C₁₋₆-alkyl)₂, N(H)(C₃₋₆-cycloalkyl), N(C₁₋₆-alkyl)(C₃₋₆-cycloalkyl), NC(O)(C₁₋₆-alkyl), NC(O)(C₃₋₆-cycloalkyl), and NC(O)(3 to 6-membered heterocycloalkyl);
L represents bond, C₁₋₆-alkylene, C(O), S(O)₂, C(CH₃)₂, phenyl, or pyridyl; and
R³ represents H, C₁₋₆-alkyl, C₃₋₆-cycloalkyl, 3 to 6-membered heterocycloalkyl, aryl, 5 or 6-membered heteroaryl, C₁₋₆-alkylene-OH, C₁₋₆-alkylene-O-C₁₋₆-alkyl, C₁₋₆-alkylene-NH₂, C₁₋₆-alkylene-N(H)-C₁₋₆-alkyl, C₁₋₆-alkylene-N(C₁₋₆-alkyl)₂, C₁₋₆-alkylene-C₃₋₆-cycloalkyl, C₁₋₆-alkylene-(3 to 6-membered heterocycloalkyl), C₁₋₆-alkylene-aryl, C₁₋₆-alkylene-(5 or 6-membered heteroaryl), C(O)NH₂, C(O)N(H)(C₁₋₆-alkyl), C(O)N(C₁₋₆-alkyl)₂, C(O)N(H)(C₃₋₆-cycloalkyl), C(O)N(H)(C₁₋₆-alkylene-C₃₋₆-cycloalkyl), C(O)N(H)(3 to 6-membered heterocycloalkyl), C(O)N(H)(C₁₋₆-alkylene-(3 to 6-membered heterocycloalkyl)), C(O)N(H)(aryl), C(O)N(H)(C₁₋₆-alkylene-aryl), C(O)N(H)(5 or 6-membered heteroaryl), C(O)N(H)(C₁₋₆-alkylene-(5 or 6-membered heteroaryl)), C(O)N(C₁₋₆-alkyl)(C₃₋₆-cycloalkyl), C(O)N(C₁₋₆-alkyl)(3 to 6-membered heterocycloalkyl), C(O)N(C₁₋₆-alkyl)(aryl), C(O)N(C₁₋₆-alkyl)(5 or 6-membered heteroaryl), C(O)N(C₃₋₆-cycloalkyl)(C₃₋₆-cycloalkyl), C(O)N(C₃₋₆-cycloalkyl)(3 to 6-membered heterocycloalkyl), C(O)N(C₃₋₆-cycloalkyl)(aryl), C(O)N(C₃₋₆-cycloalkyl)(5 or 6-membered heteroaryl), C(O)O-(C₁₋₆-alkyl), C(O)O-(C₃₋₆-cycloalkyl), C(O)O-(3 to 6-membered heterocycloalkyl), C(O)O-(aryl), C(O)O-(5 or 6-membered heteroaryl), S(O)-C₁₋₆-alkyl, S(O)₂-C₁₋₆-alkyl, S(O)-C₃₋₆-cycloalkyl, S(O)₂-C₃₋₆-cycloalkyl, S(O)-(3 to 6-membered heterocycloalkyl), S(O)₂-(3 to 6-membered heterocycloalkyl), S(O)-aryl; S(O)₂-aryl, S(O)-(5 or 6-membered heteroaryl), or S(O)₂-(5 or 6-membered heteroaryl);
wherein C₁₋₆-alkyl in each case independently from one another is linear or branched, saturated or unsaturated;
wherein C₁₋₆-alkylene is linear and saturated or unsaturated;
wherein C₁₋₆-alkyl, C₁₋₆-alkylene, C₃₋₆-cycloalkyl and 3 to 6-membered heterocycloalkyl in each case independently from one another are unsubstituted or mono- or polysubstituted with one or more substituents selected from the group consisting of F, Cl, CN, C₁₋₆-alkyl, C₁₋₄-alkylene-OH, C₁₋₄-alkylene-OCH₃, CF₃, CF₂H, CFH₂, CF₂Cl, CFCl₂, C(O)-C₁₋₆-alkyl, C(O)-OH, C(O)-OC₁₋₆-alkyl, C(O)-NH₂, C(O)-N(H)(C₁₋₆-alkyl), C(O)-N(C₁₋₆-alkyl)₂, OH, =O, OCF₃, OCF₂H, OCFH₂, OCF₂Cl, OCFCl₂, O-C₁₋₆-alkyl, O-C(O)-C₁-₆-alkyl, O-C(O)-O-C₁₋₆-alkyl, O-(CO)-N(H)(C₁₋₆-alkyl), O-C(O)-N(C₁₋₆-alkyl)₂, O-S(O)₂-NH₂, O-S(O)₂-N(H)(C₁₋₆-alkyl), O-S(O)₂-N(C₁₋₆-alkyl)₂, NH₂, N(H)(C₁₋₆-alkyl), N(C₁₋₆-alkyl)₂, N(H)-C(O)-C₁₋₆-alkyl, N(H)-C(O)-O-C₁₋₆-alkyl, N(H)-C(O)-NH₂, N(H)-C(O)-N(H)(C₁₋₆-alkyl), N(H)-C(O)-N(C₁₋₆-alkyl)₂, N(C₁-₆-alkyl)-C(O)-C₁₋₆-alkyl, N(C₁₋₆-alkyl)-C(O)-O-C₁₋₆-alkyl, N(C₁₋₆-alkyl)-C(O)-NH₂, N(C₁₋₆-alkyl)-C(O)-N(H)(C₁₋₆-alkyl), N(C₁₋₆-alkyl)-C(O)-N(C₁₋₆-alkyl)₂, N(H)-S(O)₂OH, N(H)-S(O)₂-C₁₋₆-alkyl, N(H)-S(O)₂-O-C₁₋₆-alkyl, N(H)-S(O)₂-NH₂, N(H)-S(O)₂-N(H)(C₁₋₆-alkyl), N(H)-S(O)₂N(C₁₋₆-alkyl)₂, N(C₁₋₆-alkyl)-S(O)₂-OH, N(C₁₋₆-alkyl)-S(O)₂-C₁₋₆-alkyl, N(C₁₋₆-alkyl)-S(O)₂-O-C₁₋₆-alkyl, N(C₁₋₆-alkyl)-S(O)₂-NH₂, N(C₁₋₆-alkyl)-S(O)₂-N(H)(C₁₋₆-alkyl), N(C₁₋₆-alkyl)-S(O)₂-N(C₁₋₆-alkyl)₂, SCF₃, SCF₂H, SCFH₂, S-C₁₋₆-alkyl, S(O)-C₁₋₆-alkyl, S(O)₂-C₁₋₆-alkyl, S(O)₂-OH, S(O)₂-O-C₁₋₆-alkyl, S(O)₂-NH₂, S(O)₂-N(H)(C₁₋₆-alkyl), S(O)₂-N(C₁₋₆-alkyl)₂, C₃₋₆-cycloalkyl, 3 to 6-membered heterocycloalkyl, phenyl, 5 or 6-membered heteroaryl, O-C₃₋₆-cycloalkyl, O-(3 to 6-membered heterocycloalkyl), O-phenyl, O-(5 or 6-membered heteroaryl), C(O)-C₃₋₆-cycloalkyl, C(O)-(3 to 6-membered heterocycloalkyl), C(O)-phenyl, C(O)-(5 or 6-membered heteroaryl), S(O)₂-(C₃₋₆-cycloalkyl), S(O)₂-(3 to 6-membered heterocycloalkyl) and S(O)₂-phenyl or S(O)₂-(5 or 6-membered heteroaryl);
wherein aryl, phenyl and 5 or 6-membered heteroaryl in each case independently from one another are unsubstituted or mono- or disubstituted with one or more substituents selected from the group consisting of F, Cl, Br, CN, C₁₋₆-alkyl, CF₃, CF₂H, CFH₂, C₁₋₄-alkylene-CF₃, C₁₋₄-alkylene-CF₂H, C₁₋₄-alkylene-CFH₂, C₁₋₄-alkylene-OH, C₁₋₄-alkylene-OCH₃, C(O)-C₁₋₆-alkyl, C(O)-OH, C(O)-OC₁₋₆-alkyl, C(O)-N(H)(OH), C(O)-NH₂, C(O)-N(H)(C₁₋₆-alkyl), C(O)-N(C₁₋₆-alkyl)₂, OH, OCF₃, OCF₂H, OCFH₂, OCF₂Cl, OCFCl₂, O-C₁₋₆-alkyl, O-C₃₋₆-cycloalkyl, O-(3 to 6-membered heterocycloalkyl), NH₂, N(H)(C₁₋₆-alkyl), N(H)(C₁₋₆-alkylene-OH), N(C₁₋₆-alkyl)₂, N(H)-C(O)-C₁₋₆-alkyl, N(C₁₆-alkyl)-C(O)-C₁₋₆-alkyl, N(H)-C(O)-NH₂, N(H)-C(O)-N(H)(C₁₋₆-alkyl), N(H)-C(O)-N(C₁₋₆-alkyl)₂, N(C₁₋₆-alkyl)-C(O)-N(H)(C₁₋₆-alkyl), N(C₁₋₆-alkyl)-C(O)-N(C₁₋₆-alkyl)₂, N(H)-S(O)₂-C₁₋₆-alkyl, SCF₃, S-C₁₋₆-alkyl, S(O)-C₁₋₆-alkyl, S(O)₂-C₁₋₆-alkyl, S(O)₂-NH₂, S(O)₂-N(H)(C₁₋₆-alkyl), S(O)₂-N(C₁₋₆-alkyl)₂, C₃₋₆-cycloalkyl, C₁₋₄-alkylene-C₃₋₆-cycloalkyl, 3 to 6-membered heterocycloalkyl, C₁₋₄-alkylene-(3 to 6-membered heterocycloalkyl), phenyl and 5 or 6-membered heteroaryl;
in the form of the free compound or a physiologically acceptable salt thereof;
with the proviso that the following compounds are excluded:
1-(4-Chlorophenyl)-3-[5-(6-methoxy-pyridin-3-yl)-2-oxo-1-pyrimidin-4-yl-piperidin-4-yl]-urea and
trans-1-[3-(5-Chloro-pyridin-2-yl)-1-(2-hydroxy-acetyl)-piperidin-4-yl]-3-phenyl-urea.

2. The compound according to claim 1, which is according to general formula (IIa) or (IIb)

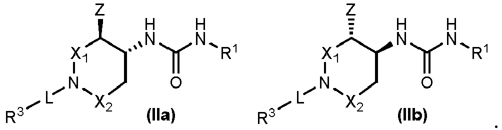

3. The compound according to claim 1 which is according to general formula (III)

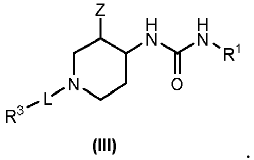

4. The compound according to claim 1 which is according to general formula (IV)

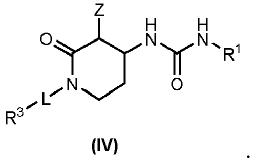

5. The compound according to claim 1 which is according to general formula (V)

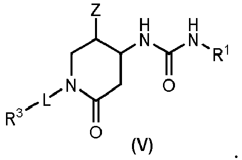

6. The compound according to any of the preceding claims, wherein R¹ represents phenyl or 5 or 6-membered heteroaryl, wherein the 5 or 6-membered heteroaryl is selected from the group consisting of isothiazolyl, pyridyl, pyrimidinyl, pyridazinyl, pyrazinyl, pyrrolyl, imidazolyl, pyrazolyl, oxazolyl, isoxazolyl, thiazolyl, furanyl, thienyl (thiophenyl), triazolyl, thiadiazolyl, benzofuranyl, benzoimidazolyl, benzothienyl, benzothiadiazolyl, benzothiazolyl, benzotriazolyl, benzooxazolyl, benzooxadiazolyl, quinazolinyl, quinoxalinyl, carbazolyl, quinolinyl, dibenzofuranyl, dibenzothienyl, imidazothiazolyl, indazolyl, indolizinyl, indolyl, isoquinolinyl, naphthyridinyl, oxazolyl, oxadiazolyl, phenazinyl, phenothiazinyl, phthalazinyl, purinyl, phenazinyl, tetrazolyl and triazinyl.

7. The compound according to any of the preceding claims, wherein Z represents a 5 or 6 membered heteroaryl which is selected from the group consisting of pyridyl, thienyl (thiophenyl), pyrimidinyl, pyridazinyl, pyrazinyl, pyrrolyl, imidazolyl, pyrazolyl, oxazolyl, isoxazolyl, thiazolyl, isothiazolyl, furanyl, triazolyl, thiadiazolyl, benzofuranyl, benzoimidazolyl, benzothienyl, benzothiadiazolyl, benzothiazolyl,
benzotriazolyl, benzooxazolyl, benzooxadiazolyl, quinazolinyl, quinoxalinyl, carbazolyl, quinolinyl, dibenzofuranyl, dibenzothienyl, imidazothiazolyl, indazolyl, indolizinyl, indolyl, isoquinolinyl, naphthyridinyl, oxazolyl, oxadiazolyl, phenazinyl, phenothiazinyl, phthalazinyl, purinyl, phenazinyl, tetrazolyl and triazinyl.

8. The compound according to any of the preceding claims, wherein Z is monosubstituted or disubstituted with R².

9. The compound according to any of the preceding claims, wherein R² represents O-CH₃, F, Cl, Br, OH, O-CH₂CH₃, O-(CH₂)₂CH₃, O-CH(CH₃)₂ or OCF₃.

10. The compound according to any of the preceding claims, wherein
L represents bond, CH₂, CH₂CH₂, C(O), phenyl or pyridyl; and/or
R³ represents H, C₁₋₆-alkyl, C₁₋₆-alkylene-OH, C₁₋₆-alkylene-O-C₁₋₆-alkyl, C₁₋₆-alkylene-NH₂, C₁₋₆-alkylene-N(H)-C₁₋₆-alkyl, C₁₋₆-alkylene-N(C₁₋₆-alkyl)₂, 3 to 6-membered cycloalkyl, 3 to 6-membered heterocycloalkyl, aryl, 5 or 6-membered heteroaryl, C(O)NH₂, C(O)N(H)(C₁₋₆-alkyl), C(O)N(C₁₋₆-alkyl)₂, C(O)N(H)(C₃₋₆-cycloalkyl), C(O)N(H)(C₁₋₆-alkylene-C₃₋₆-cycloalkyl), C(O)N(H)(3 to 6-membered heterocycloalkyl), C(O)N(H)(C₁₋₆-alkylene-(3 to 6-membered heterocycloalkyl)), C(O)N(H)(aryl), C(O)N(H)(C₁₋₆-alkylene-aryl), C(O)N(H)(5 or 6-membered heteroaryl), C(O)N(H)(C₁₋₆-alkylene-(5 or 6-membered heteroaryl)), or S(O)₂-C₁₋₆-alkyl.

11. The compound according to any of the preceding claims, wherein
X₂ represents CH₂ or C(O) and X₁ represent CH₂;
Z represents pyridyl, thienyl (thiophenyl), pyrimidinyl, pyridazinyl, pyrazinyl or pyrrolyl; wherein said pyridyl, thienyl (thiophenyl), pyrimidinyl, pyridazinyl, pyrazinyl and pyrrolyl are monosubstituted or disubstituted with R² which in each case independently is selected from the group consisting of O-CH₃, Cl and F;
R¹ represents phenyl, isothiazolyl, pyridyl, pyrimidinyl, thiophenyl, or thiazolyl; wherein said phenyl, isothiazolyl, pyridyl, pyrimidinyl, thiophenyl, and thiazolyl independently from one another are unsubstituted or monosubstituted with a substituents selected from the group consisting of F, Cl, Br, CN, unsubstituted C₁₋₆-alkyl and CF₃;
L represents bond, CH₂, CH₂CH₂, C(O), phenyl or pyridyl; and
R³ represents
H;
C₁₋₆-alkyl selected from the group consisting of methyl, ethyl, n-propyl, 2-propyl, n-butyl, isobutyl, sec-butyl, tert-butyl, n-pentyl, 2-pentyl, 3-pentyl, 2-methylbutyl, 3-methylbutyl, 3-methylbut-2-yl, 2-methylbut-2-yl, 2,2-dimethylpropyl and n-hexyl;
wherein C₁₋₆-alkyl is unsubstituted or mono- or polysubstituted with one or more substituents selected from the group consisting of F, Cl, Br, CN, CF₃, CHF₂, C(O)NH₂, OH, O-CH₃, O-CH₂CH₃, O-(CH₂)₂CH₃, O-CH(CH₃)₂, N(H)C(O)CH₃, N(H)S(O)₂CH₃, S(O)-CH₃, and S(O)₂-CH₃;
C₁₋₆-alkylene-OH selected from the group consisting of CH₂OH, CH₂CH₂OH, (CH₂)₃OH, (CH₂)₄OH, C(H)(OH)-CH₃, CH₂C(H)(OH)-CH₃, C(CH₃)₂-OH, C(H)(OH)-C(CH₃)₂, and CH₂C(CH₃)₂-OH,
wherein C₁₋₆-alkylene is unsubstituted or mono- or polysubstituted with one or more substituents selected from the group consisting of F, Cl, Br, CN, and OH;
C₁₋₆-alkylene-O-C₁₋₆-alkyl selected from the group consisting of CH₂OCH₃, CH₂CH₂OCH₃, (CH₂)₃OCH₃, (CH₂)₄OCH₃, (CH₂)₅OCH₃, and (CH₂)₆OCH₃,
wherein C₁₋₆-alkylene is unsubstituted or mono- or polysubstituted with one or more substituents selected from the group consisting of F, Cl, Br, CN, and OH;
C₁₋₆-alkylene-NH₂ selected from the group consisting of CH₂NH₂, CH₂CH₂NH₂, (CH₂)₃NH₂, (CH₂)₄NH₂, (CH₂)₅NH₂, and (CH₂)₆NH₂,
C₁₋₆-alkylene-N(H)-C₁₋₆-alkyl selected from the group consisting of CH₂N(H)CH₃, CH₂CH₂N(H)CH₃, (CH₂)₃N(H)CH₃, (CH₂)₄N(H)CH₃, (CH₂)₅N(H)CH₃, and (CH₂)₆N(H)CH₃,
C₁₋₆-alkylene-N(C₁₋₆-alkyl)₂ selected from the group consisting of CH₂N(CH₃)₂, CH₂CH₂N(CH₃)₂, (CH₂)₃N(CH₃)₂, (CH₂)₄N(CH₃)₂, (CH₂)₅N(CH₃)₂, and (CH₂)₆N(CH₃)₂,
3 to 6-membered cycloalkyl selected from the group consisting of cyclopropyl, cyclobutyl, cyclopentyl and cyclohexyl;
wherein 3 to 6-membered cycloalkyl is unsubstituted or mono- or polysubstituted with one or more substituents selected from the group consisting of F, Cl, Br, CN, CH₃, CF₃, C(O)NH₂, C(O)N(H)(CH₃), C(O)N(CH₃)₂, OH, CH₂OH, OCH₃, CH₂OCH₃, N(H)C(O)CH₃, N(H)S(O)₂CH₃, and S(O)₂-CH₃;
3 to 6-membered heterocycloalkyl selected from the group consisting of tetrahydropyranyl, piperidinyl, oxetanyl, azetidinyl, tetrahydrothiopyran 1,1-dioxide, morpholinyl, pyrrolidinyl, 4-methylpiperazinyl, morpholinonyl, aziridinyl, dithiolanyl, dihydropyrrolyl, dioxanyl, dioxolanyl, dihydropyridinyl, dihydrofuranyl, dihydroisoxazolyl, dihydrooxazolyl, imidazolidinyl, isoxazolidinyl, oxazolidinyl, oxiranyl, piperazinyl, pyrazolidinyl, pyranyl and tetrahydropyrrolyl;
wherein 3 to 6-membered heterocycloalkyl is unsubstituted or mono- or polysubstituted with one or more substituents selected from the group consisting of F, Cl, Br, CN, CH₃, CF₃, C(O)NH₂, C(O)N(H)(CH₃), C(O)N(CH₃)₂, OH, CH₂OH, OCH₃, CH₂OCH₃, N(H)C(O)CH₃, N(H)S(O)₂CH₃, and S(O)₂-CH₃;
C(O)NH₂;
C(O)N(H)(C₁₋₆-alkyl) selected from the group consisting of C(O)N(H)(CH₃) and C(O)N(H)(CH₂CH₃);
wherein C₁₋₆-alkyl is unsubstituted or mono- or polysubstituted with one or more substituents selected from the group consisting of F, Cl, Br, CN, CF₃, CHF₂, OH, and O-CH₃;
C(O)N(C₁₋₆-alkyl)₂ selected from the group consisting of C(O)N(CH₃)₂ and C(O)N(CH₂CH₃)₂;
C(O)N(H)(C₃₋₆-cycloalkyl) selected from the group consisting of C(O)N(H)(cyclopropyl), C(O)N(H)(cyclobutyl), C(O)N(H)(cyclopentyl) and C(O)N(H)(cyclohexyl);
C(O)N(H)(C₁₋₆-alkylene-C₃₋₆-cycloalkyl) selected from the group consisting of C(O)N(H)(CH₂-cyclopropyl), C(O)N(H)(CH₂-cyclobutyl), C(O)N(H)(CH₂CH₂-cyclopropyl), C(O)N(H)(CH₂CH₂-cyclobutyl), C(O)N(H)(CH₂-cyclopentyl) and C(O)N(H)(CH₂-cyclohexyl);
C(O)N(H)(3 to 6-membered heterocycloalkyl) selected from the group consisting of C(O)N(H)(tetrahydropyranyl), C(O)N(H)(piperidinyl), C(O)N(H)(oxetanyl), and C(O)N(H)(azetidinyl),
C(O)N(H)(C₁₋₆-alkylene-(3 to 6-membered heterocycloalkyl) selected from the group consisting of C(O)N(H)(CH₂-tetrahydropyranyl), C(O)N(H)(CH₂-piperidinyl), C(O)N(H)(CH₂-oxetanyl), and C(O)N(H)(CH₂-azetidinyl),
C(O)N(H)(aryl) selected from C(O)N(H)(phenyl);
C(O)N(H)(C₁₋₆-alkylene-aryl) selected from C(O)N(H)(CH₂-phenyl) and C(O)N(H)(CH₂CH₂-phenyl);
C(O)N(H)(5 or 6-membered heteroaryl) selected from the group consisting of C(O)N(H)(isoxazolyl), C(O)N(H)(pyridyl), C(O)N(H)(pyrazolyl), C(O)N(H)(oxazolyl), C(O)N(H)(thiazolyl), C(O)N(H)(triazolyl), C(O)N(H)(oxadiazolyl), C(O)N(H)(pyrimidinyl), C(O)N(H)(pyrazinyl), and C(O)N(H)(pyridazinyl);
wherein said 5- or 6-membered heteroaryl is unsubstituted or mono- or disubstituted with one or more substituents selected from the group consisting of F, Cl, Br, CN, CH₃, CF₃, cyclopropyl, OH, O-CH₃, and S(O)₂-CH₃;
C(O)N(H)(C₁₋₆-alkylene-5 or 6-membered heteroaryl) selected from the group consisting of C(O)N(H)(CH₂-isoxazolyl), C(O)N(H)(CH₂-pyridyl), C(O)N(H)(CH₂-pyrazolyl), C(O)N(H)(CH₂-oxazolyl), C(O)N(H)(CH₂-thiazolyl), C(O)N(H)(CH₂-triazolyl), C(O)N(H)(CH₂-oxadiazolyl), C(O)N(H)(CH₂-pyrimidinyl), C(O)N(H)(CH₂-pyrazinyl), and C(O)N(H)(CH₂-pyridazinyl);
wherein said 5- or 6-membered heteroaryl is unsubstituted or mono- or disubstituted with one or more substituents selected from the group consisting of F, Cl, Br, CN, CH₃, CF₃, cyclopropyl, OH, O-CH₃, and S(O)₂-CH₃;
S(O)₂-C₁₋₆-alkyl selected from the group consisting of S(O)₂-CH₃, S(O)₂-CH₂CH₃, S(O)₂-(CH₂)₂CH₃, and S(O)₂-CH(CH₃)₂;
phenyl, which is unsubstituted or mono- or disubstituted with one or more substituents selected from the group consisting of F, Cl, Br, CN, OH, CH₂OH, OCH₃, CH₂OCH₃, and CH₃; or
5- or 6-membered heteroaryl selected from the group consisting of pyrimidinyl, pyrazinyl, pyridazinyl, pyridyl, pyrazolyl, isoxazolyl, triazolyl, oxazolyl, oxadiazolyl, indazolyl, pyrazolopyridyl, isoindolinonyl, pyrrolyl, imidazolyl, thiazolyl, isothiazolyl, furanyl, thienyl, and thiadiazolyl;
wherein said 5- or 6-membered heteroaryl is unsubstituted or mono- or disubstituted with one or more substituents selected from the group consisting of F, Cl, Br, CN, CH₃, CF₃, cyclopropyl, N(H)CH₂CH₂OH, OH, CH₂OH, CH₂CH₂OH, OCH₃, CH₂OCH₃, CH₂CH₂OCH₃, and S(O)₂-CH₃.

12. The compound according to any of the preceding claims which is selected from the group consisting of
**Ex-1** *trans*-1-(4-Chlorophenyl)-3-[3-(6-methoxy-pyridin-3-yl)-1-pyrimidin-4-yl-piperidin-4-yl]-urea
**Ex-2** trans-1-(4-Chlorophenyl)-3-[1-(4-fluorophenyl)-3-(6-methoxy-pyridin-3-yl)-piperidin-4-yl]-urea
**Ex-3** trans-1-[1-(4-Fluorophenyl)-3-(6-methoxy-pyridin-3-yl)-piperidin-4-yl]-3-(3-methyl-isothiazol-5-yl)-urea
**Ex-4** trans-1-[3-(6-Methoxy-pyridin-3-yl)-1-pyrimidin-4-yl-piperidin-4-yl]-3-(3-methyl-isothiazol-5-yl)-urea
**Ex-5** trans-1-(4-Chlorophenyl)-3-[1-(4-fluorophenyl)-5-(6-methoxy-pyridin-3-yl)-2-oxo-piperidin-4-yl]-urea
**Ex-6** cis-1-(4-Chlorophenyl)-3-[1-(4-fluorophenyl)-5-(6-methoxy-pyridin-3-yl)-2-oxo-piperidin-4-yl]-urea
**Ex-7** trans-1-(4-Chlorophenyl)-3-[3-(5-methoxy-pyridin-2-yl)-1-pyrimidin-4-yl-piperidin-4-yl]-urea
**Ex-8** trans-1-[3-(5-Methoxy-pyridin-2-yl)-1-pyrimidin-4-yl-piperidin-4-yl]-3-(3-methyl-isothiazol-5-yl)-urea
**Ex-9** trans-1-(4-Chlorophenyl)-3-[3-(5-chloro-pyridin-2-yl)-1-pyrimidin-4-yl-piperidin-4-yl]-urea
**Ex-10** trans-1-(4-Chlorophenyl)-3-[1-(4-fluorophenyl)-5-(5-methoxy-pyridin-2-yl)-2-oxo-piperidin-4-yl]-urea
**Ex-11** cis-1-(4-Chlorophenyl)-3-[1-(4-fluorophenyl)-5-(5-methoxy-pyridin-2-yl)-2-oxo-piperidin-4-yl]-urea
**Ex-12** trans-1-(4-Chlorophenyl)-3-[3-(5-chloro-pyridin-2-yl)-1-(4-fluorophenyl)-piperidin-4-yl]-urea
**Ex-13** trans-1-[3-(5-Chloro-pyridin-2-yl)-1-pyrimidin-4-yl-piperidin-4-yl]-3-(3-methyl-isothiazol-5-yl)-urea
**Ex-14** trans-1-(4-Chlorophenyl)-3-[5-(5-chloro-pyridin-2-yl)-1-(4-fluorophenyl)-2-oxo-piperidin-4-yl]-urea
**Ex-15** cis-1-(4-Chlorophenyl)-3-[5-(5-chloro-pyridin-2-yl)-1-(4-fluorophenyl)-2-oxo-piperidin-4-yl]-urea
**Ex-16** trans-1-[3-(5-Chloro-pyridin-2-yl)-1-pyrimidin-4-yl-piperidin-4-yl]-3-(3-methyl-isothiazol-5-yl)-urea ent-1
**Ex-17** trans-1-[3-(5-Chloro-pyridin-2-yl)-1-pyrimidin-4-yl-piperidin-4-yl]-3-(3-methyl-isothiazol-5-yl)-urea ent-2
**Ex-18** trans-1-(4-Chlorophenyl)-3-[1-(4-fluorophenyl)-3-(5-methoxy-pyridin-2-yl)-piperidin-4-yl]-urea
**Ex-19** trans-1-[3-(5-Chloro-pyridin-2-yl)-1-pyrazin-2-yl-piperidin-4-yl]-3-(3-methyl-isothiazol-5-yl)-urea
**Ex-20** trans-1-[1-(4-Fluorophenyl)-3-(5-methoxy-pyridin-2-yl)-piperidin-4-yl]-3-(3-methyl-isothiazol-5-yl)-urea
**Ex-21** trans-1-[3-(5-Chloro-pyridin-2-yl)-1-(2-methyl-pyrimidin-4-yl)-piperidin-4-yl]-3-(3-methyl-isothiazol-5-yl)-urea
**Ex-22** trans-1-(4-Chlorophenyl)-3-[3-(5-chloro-pyridin-2-yl)-1-[(5-methyl-isoxazol-3-yl)-methyl]-piperidin-4-yl]-urea ent-1
**Ex-23** trans-1-(4-Chlorophenyl)-3-[3-(5-chloro-pyridin-2-yl)-1-[(5-methyl-isoxazol-3-yl)-methyl]-piperidin-4-yl]-urea ent-2
**Ex-25** 1-(4-Chlorophenyl)-3-[5-(5-methoxy-pyridin-2-yl)-2-oxo-1-pyrimidin-4-yl-piperidin-4-yl]-urea dia-1
**Ex-26** trans-1-[3-(5-Chloro-pyridin-2-yl)-1-(4-fluorophenyl)-piperidin-4-yl]-3-(3-methyl-isothiazol-5-yl)-urea
**Ex-27** trans-1-[3-(5-Chloro-pyridin-2-yl)-1-[6-(trifluoromethyl)-pyrimidin-4-yl]-piperidin-4-yl]-3-(3-methyl-isothiazol-5-yl)-urea
**Ex-28** trans-1-[3-(5-Chloro-pyridin-2-yl)-1-[2-(trifluoromethyl)-pyrimidin-4-yl]-piperidin-4-yl]-3-(3-methyl-isothiazol-5-yl)-urea
**Ex-29** trans-1-(4-Chlorophenyl)-3-[3-(5-methoxy-pyridin-2-yl)-1-[(5-methyl-isoxazol-3-yl)-methyl]-piperidin-4-yl]-urea ent-1
**Ex-30** trans-1-(4-Chlorophenyl)-3-[3-(5-methoxy-pyridin-2-yl)-1-[(5-methyl-isoxazol-3-yl)-methyl]-piperidin-4-yl]-urea ent-2
**Ex-31** trans-1-[3-(5-Chloro-pyridin-2-yl)-1-(6-methyl-pyrimidin-4-yl)-piperidin-4-yl]-3-(3-methyl-isothiazol-5-yl)-urea
**Ex-32** trans-1-[3-(5-Chloro-pyridin-2-yl)-1-pyridazin-3-yl-piperidin-4-yl]-3-(3-methyl-isothiazol-5-yl)-urea
**Ex-33** trans-1-[3-(5-Chloro-pyridin-2-yl)-1-(6-methyl-pyridazin-3-yl)-piperidin-4-yl]-3-(3-methyl-isothiazol-5-yl)-urea
**Ex-34** trans-1-[3-(5-Chloro-pyridin-2-yl)-1-(6-methyl-pyrazin-2-yl)-piperidin-4-yl]-3-(3-methyl-isothiazol-5-yl)-urea
**Ex-35** trans-1-(4-Chlorophenyl)-3-[1-(2,2-difluoro-ethyl)-3-(5-methoxy-pyridin-2-yl)-piperidin-4-yl]-urea ent-1
**Ex-36** trans-1-(4-Chlorophenyl)-3-[1-(2,2-difluoro-ethyl)-3-(5-methoxy-pyridin-2-yl)-piperidin-4-yl]-urea ent-2
**Ex-37** trans-1-(4-Chlorophenyl)-3-[3-(5-methoxy-pyridin-2-yl)-1-[[5-(trifluoromethyl)-isoxazol-3-yl]-methyl]-piperidin-4-yl]-urea ent-2
**Ex-38** trans-1-(4-Chlorophenyl)-3-[3-(5-methoxy-pyridin-2-yl)-1-[[5-(trifluoromethyl)-isoxazol-3-yl]-methyl]-piperidin-4-yl]-urea ent-2
**Ex-39** trans-1-(4-Chlorophenyl)-3-[3-(5-methoxy-pyridin-2-yl)-1-[(1-methyl-1H-pyrazol-3-yl)-methyl]-piperidin-4-yl]-urea ent-1
**Ex-40** trans-1-(4-Chlorophenyl)-3-[3-(5-methoxy-pyridin-2-yl)-1-[(1-methyl-1H-pyrazol-3-yl)-methyl]-piperidin-4-yl]-urea ent-2
**Ex-41** trans-1-[3-(5-Chloro-pyridin-2-yl)-1-(5-methyl-pyrazin-2-yl)-piperidin-4-yl]-3-(3-methyl-isothiazol-5-yl)-urea
**Ex-42** trans-1-(4-Chlorophenyl)-3-[3-(5-methoxy-pyridin-2-yl)-1-[(1-methylsulfonyl-1H-pyrazol-3-yl)-methyl]-piperidin-4-yl]-urea ent-1
**Ex-43** trans-1-(4-Chlorophenyl)-3-[3-(5-methoxy-pyridin-2-yl)-1-[(1-methylsulfonyl-1H-pyrazol-3-yl)-methyl]-piperidin-4-yl]-urea ent-2
**Ex-44** trans-1-(4-Chlorophenyl)-3-[3-(5-chloro-thiophen-2-yl)-1-[(5-methyl-isoxazol-3-yl)-methyl]-piperidin-4-yl]-urea ent-1
**Ex-45** trans-1-(4-Chlorophenyl)-3-[3-(5-chloro-thiophen-2-yl)-1-[(5-methyl-isoxazol-3-yl)-methyl]-piperidin-4-yl]-urea ent-2
**Ex-46** trans-1-(4-Chlorophenyl)-3-[3-(5-chloro-thiophen-2-yl)-1-(pyrimidin-2-yl-methyl)-piperidin-4-yl]-urea ent-1
**Ex-47** trans-1-(4-Chlorophenyl)-3-[3-(5-chloro-thiophen-2-yl)-1-(pyrimidin-2-yl-methyl)-piperidin-4-yl]-urea ent-2
**Ex-48** trans-1-[3-(5-Chloro-thiophen-2-yl)-1-[(5-methyl-isoxazol-3-yl)-methyl]-piperidin-4-yl]-3-(3-methyl-isothiazol-5-yl)-urea ent-1
**Ex-49** trans-1-[3-(5-Chloro-thiophen-2-yl)-1-[(5-methyl-isoxazol-3-yl)-methyl]-piperidin-4-yl]-3-(3-methyl-isothiazol-5-yl)-urea ent-2
**Ex-50** trans-1-(4-Chlorophenyl)-3-[3-(5-methoxy-pyridin-2-yl)-1-(tetrahydro-pyran-4-yl-methyl)-piperidin-4-yl]-urea ent-2
**Ex-51** trans-1-(4-Chlorophenyl)-3-[3-(5-methoxy-pyridin-2-yl)-1-(tetrahydro-pyran-4-yl-methyl)-piperidin-4-yl]-urea ent-2
**Ex-52** trans-1-(4-Chlorophenyl)-3-[3-(5-methoxy-pyridin-2-yl)-1-(4-methyl-pyridine-3-carbonyl)-piperidin-4-yl]-urea ent-1
**Ex-53** trans-1-(4-Chlorophenyl)-3-[3-(5-methoxy-pyridin-2-yl)-1-(4-methyl-pyridine-3-carbonyl)-piperidin-4-yl]-urea ent-2
**Ex-54** trans-1-(4-Chlorophenyl)-3-[3-(5-methoxy-pyridin-2-yl)-1-(6-methyl-pyridine-3-carbonyl)-piperidin-4-yl]-urea ent-1
**Ex-55** trans-1-(4-Chlorophenyl)-3-[3-(5-methoxy-pyridin-2-yl)-1-(6-methyl-pyridine-3-carbonyl)-piperidin-4-yl]-urea ent-2
**Ex-56** trans-1-[3-(5-Chloro-thiophen-2-yl)-1-[(1-methylsulfonyl-1H-pyrazol-3-yl)-methyl]-piperidin-4-yl]-3-phenyl-urea ent-1
**Ex-57** trans-1-[3-(5-Chloro-thiophen-2-yl)-1-[(1-methylsulfonyl-1H-pyrazol-3-yl)-methyl]-piperidin-4-yl]-3-phenyl-urea ent-2
**Ex-58** trans-1-[3-(5-Methoxy-pyridin-2-yl)-1-[(1-methylsulfonyl-1H-pyrazol-3-yl)-methyl]-piperidin-4-yl]-3-phenyl-urea ent-1
**Ex-59** trans-1-[3-(5-Methoxy-pyridin-2-yl)-1-[(1-methylsulfonyl-1H-pyrazol-3-yl)-methyl]-piperidin-4-yl]-3-phenyl-urea ent-2
**Ex-60** trans-1-(4-Chlorophenyl)-3-[3-(5-methoxy-pyridin-2-yl)-1-[[6-(trifluoromethyl)-pyridin-3-yl]-methyl]-piperidin-4-yl]-urea
**Ex-61** trans-1-(4-Chlorophenyl)-3-[3-(5-methoxy-pyridin-2-yl)-1-[(2-methyl-pyrimidin-5-yl)-methyl] -piperidin-4-yl]-urea
**Ex-62** trans-1-[3-(5-Chloro-thiophen-2-yl)-1-(pyrimidin-2-yl-methyl)-piperidin-4-yl]-3-(3-methyl-isothiazol-5-yl)-urea ent-1
**Ex-63** trans-1-[3-(5-Chloro-thiophen-2-yl)-1-(pyrimidin-2-yl-methyl)-piperidin-4-yl]-3-(3-methyl-isothiazol-5-yl)-urea ent-2
**Ex-64** trans-1-(4-Chlorophenyl)-3-[3-(5-methoxy-pyridin-2-yl)-1-[(5-methyl-pyridin-3-yl)-methyl]-piperidin-4-yl]-urea
**Ex-65** trans-1-(4-Chlorophenyl)-3-[1-[(6-cyclopropyl-pyridin-3-yl)-methyl]-3-(5-methoxy-pyridin-2-yl)-piperidin-4-yl]-urea
**Ex-66** trans-1-(4-Chlorophenyl)-3-[3-(5-methoxy-pyridin-2-yl)-1-[(2-methoxy-pyrimidin-5-yl)-methyl] -piperidin-4-yl]-urea
**Ex-67** trans-1-(4-Chlorophenyl)-3-[3-(5-methoxy-pyridin-2-yl)-1-[(6-methyl-pyridin-3-yl)-methyl]-piperidin-4-yl]-urea
**Ex-68** trans-1-(4-Chlorophenyl)-3-[1-[(5-fluoro-pyridin-3-yl)-methyl]-3-(5-methoxy-pyridin-2-yl)-piperidin-4-yl]-urea
**Ex-69** trans-1-(4-Chlorophenyl)-3-[1-[(5-chloro-pyridin-3-yl)-methyl]-3-(5-methoxy-pyridin-2-yl)-piperidin-4-yl]-urea
**Ex-70** trans-1-(4-Chlorophenyl)-3-[3-(5-chloro-thiophen-2-yl)-1-[(5-fluoro-pyridin-3-yl)-methyl]-piperidin-4-yl]-urea
**Ex-71** trans-1-(4-Chlorophenyl)-3-[1-[(5-chloro-pyridin-3-yl)-methyl]-3-(5-chloro-thiophen-2-yl)-piperidin-4-yl]-urea
**Ex-72** trans-1-(4-Chlorophenyl)-3-[3-(5-chloro-thiophen-2-yl)-1-[(5-methyl-pyridin-3-yl)-methyl]-piperidin-4-yl]-urea
**Ex-73** trans-1-(4-Chlorophenyl)-3-[3-(5-chloro-thiophen-2-yl)-1-[(6-cyclopropyl-pyridin-3-yl)-methyl]-piperidin-4-yl]-urea
**Ex-74** trans-1-(4-Chlorophenyl)-3-[3-(5-chloro-thiophen-2-yl)-1-[[6-(trifluoromethyl)-pyridin-3-yl]-methyl]-piperidin-4-yl]-urea
**Ex-75** trans-1-(4-Chlorophenyl)-3-[3-(5-chloro-thiophen-2-yl)-1-[(2-methoxy-pyrimidin-5-yl)-methyl]-piperidin-4-yl]-urea
**Ex-76** trans-1-(4-Chlorophenyl)-3-[3-(5-chloro-thiophen-2-yl)-1-[(2-methyl-pyrimidin-5-yl)-methyl]-piperidin-4-yl]-urea
**Ex-77** trans-1-(4-Chlorophenyl)-3-[3-(5-chloro-thiophen-2-yl)-1-[(1-methylsulfonyl-1H-pyrazol-3-yl)-methyl]-piperidin-4-yl]-urea ent-1
**Ex-78** trans-1-(4-Chlorophenyl)-3-[3-(5-chloro-thiophen-2-yl)-1-[(1-methylsulfonyl-1H-pyrazol-3-yl)-methyl]-piperidin-4-yl]-urea ent-2
**Ex-79** trans-1-(4-Chlorophenyl)-3-[1-(3-methoxy-propanoyl)-3-(5-methoxy-pyridin-2-yl)-piperidin-4-yl]-urea
**Ex-80** trans-1-(4-Chlorophenyl)-3-[1-(3-methoxy-propyl)-3-(5-methoxy-pyridin-2-yl)-piperidin-4-yl]-urea
**Ex-81** trans-1-(4-Chlorophenyl)-3-[1-(4-methoxy-cyclohexanecarbonyl)-3-(5-methoxy-pyridin-2-yl)-piperidin-4-yl]-urea
**Ex-82** trans-1-(4-Chlorophenyl)-3-[3-(5-methoxy-pyridin-2-yl)-1-(piperidin-4-yl-methyl)-piperidin-4-yl]-urea ent-2
**Ex-83** trans-1-(4-Chlorophenyl)-3-[3-(5-chloro-thiophen-2-yl)-1-(piperidin-4-yl-methyl)-piperidin-4-yl]-urea dihydrochloride ent-1
**Ex-84** trans-1-(4-Chlorophenyl)-3-[3-(5-chloro-thiophen-2-yl)-1-(piperidin-4-yl-methyl)-piperidin-4-yl]-urea dihydrochloride ent-2
**Ex-85** trans-1-(4-Chlorophenyl)-3-[3-(5-chloro-thiophen-2-yl)-1-(tetrahydro-pyran-4-yl-methyl)-piperidin-4-yl]-urea ent-1
**Ex-86** trans-1-(4-Chlorophenyl)-3-[3-(5-chloro-thiophen-2-yl)-1-(tetrahydro-pyran-4-yl-methyl)-piperidin-4-yl]-urea ent-2
**Ex-87** trans-1-(4-Chlorophenyl)-3-[3-(5-chloro-thiophen-2-yl)-1-[(6-methyl-pyridin-3-yl)-methyl]-piperidin-4-yl]-urea
**Ex-88** trans-1-(4-Chlorophenyl)-3-[3-(5-methoxy-pyridin-2-yl)-1-(piperidine-4-carbonyl)-piperidin-4-yl]-urea
**Ex-89** trans-1-(4-Chlorophenyl)-3-[3-(5-methoxy-pyridin-2-yl)-1-(1-methyl-piperidine-4-carbonyl)-piperidin-4-yl]-urea
**Ex-90** trans-1-(4-Chlorophenyl)-3-[1-(4-hydroxy-cyclohexanecarbonyl)-3-(5-methoxy-pyridin-2-yl)-piperidin-4-yl]-urea
**Ex-91** trans-1-(4-Chlorophenyl)-3-[3-(5-chloro-thiophen-2-yl)-1-(3-methoxy-propyl)-piperidin-4-yl]-urea
**Ex-92** trans-1-(4-Chlorophenyl)-3-[3-(5-methoxy-pyridin-2-yl)-1-(oxetane-3-carbonyl)-piperidin-4-yl]-urea
**Ex-93** trans-1-(4-Chlorophenyl)-3-[3-(5-methoxy-pyridin-2-yl)-1-(2-methylsulfonyl-ethyl)-piperidin-4-yl]-urea
**Ex-94** trans-1-(4-Chlorophenyl)-3-[1-(2-dimethylamino-acetyl)-3-(5-methoxy-pyridin-2-yl)-piperidin-4-yl]-urea
**Ex-95** trans-1-(4-Chlorophenyl)-3-[1-(3-dimethylamino-propanoyl)-3-(5-methoxy-pyridin-2-yl)-piperidin-4-yl]-urea
**Ex-96** trans-1-(4-Chlorophenyl)-3-[3-(5-methoxy-pyridin-2-yl)-1-(2-methylamino-acetyl)-piperidin-4-yl]-urea
**Ex-97** trans-1-(4-Chlorophenyl)-3-[3-(5-methoxy-pyridin-2-yl)-1-(3-methylamino-propanoyl)-piperidin-4-yl]-urea
**Ex-98** trans-1-(4-Chlorophenyl)-3-[3-(5-methoxy-pyridin-2-yl)-1-[4-(trifluoromethyl)-pyridine-3-carbonyl]-piperidin-4-yl]-urea ent-1
**Ex-99** trans-1-(4-Chlorophenyl)-3-[3-(5-methoxy-pyridin-2-yl)-1-[4-(trifluoromethyl)-pyridine-3-carbonyl]-piperidin-4-yl]-urea ent-2
**Ex-100** trans-1-(4-Chlorophenyl)-3-[3-(5-methoxy-pyridin-2-yl)-1-[6-(trifluoromethyl)-pyridine-3-carbonyl]-piperidin-4-yl]-urea ent-1
**Ex-101** trans-1-(4-Chlorophenyl)-3-[3-(5-methoxy-pyridin-2-yl)-1-[6-(trifluoromethyl)-pyridine-3-carbonyl]-piperidin-4-yl]-urea ent-2
**Ex-102** trans-1-(4-Chlorophenyl)-3-[1-(3-hydroxy-propanoyl)-3-(5-methoxy-pyridin-2-yl)-piperidin-4-yl]-urea
**Ex-103** trans-1-(4-Chlorophenyl)-3-[1-(3-hydroxy-propyl)-3-(5-methoxy-pyridin-2-yl)-piperidin-4-yl]-urea
**Ex-104** trans-1-(4-Chlorophenyl)-3-[3-(5-chloro-thiophen-2-yl)-1-(1-methyl-piperidine-4-carbonyl)-piperidin-4-yl]-urea
**Ex-105** trans-1-(4-Chlorophenyl)-3-[3-(5-chloro-thiophen-2-yl)-1-(3-hydroxy-propyl)-piperidin-4-yl]-urea
**Ex-106** trans-1-(4-Chlorophenyl)-3-[3-(5-chloro-thiophen-2-yl)-1-(3-methoxy-propanoyl)-piperidin-4-yl]-urea
**Ex-107** trans-1-(4-Chlorophenyl)-3-[3-(5-chloro-thiophen-2-yl)-1-(4-methoxy-cyclohexanecarbonyl)-piperidin-4-yl]-urea
**Ex-108** trans-1-(4-Chlorophenyl)-3-[3-(5-methoxy-pyridin-2-yl)-1-(oxetan-3-yl)-piperidin-4-yl]-urea
**Ex-109** trans-1-(4-Chlorophenyl)-3-[3-(5-methoxy-pyridin-2-yl)-1-(oxetan-3-yl-methyl)-piperidin-4-yl]-urea
**Ex-110** trans-1-(4-Chlorophenyl)-3-[1-(1,1-dioxo-thian-4-yl)-3-(5-methoxy-pyridin-2-yl)-piperidin-4-yl]-urea
**Ex-111** trans-1-(4-Chlorophenyl)-3-[1-(3-dimethylamino-propyl)-3-(5-methoxy-pyridin-2-yl)-piperidin-4-yl]-urea
**Ex-112** trans-1-(4-Chlorophenyl)-3-[3-(5-methoxy-pyridin-2-yl)-piperidin-4-yl]-urea
**Ex-113** trans-1-(4-Cyano-phenyl)-3-[1-(2,2-difluoro-ethyl)-3-(5-methoxy-pyridin-2-yl)-piperidin-4-yl]-urea
**Ex-114** trans-1-(4-Chlorophenyl)-3-[1-[(4-hydroxy-cyclohexyl)-methyl]-3-(5-methoxy-pyridin-2-yl)-piperidin-4-yl]-urea
**Ex-115** trans-1-(4-Chlorophenyl)-3-[1-[(4-methoxy-cyclohexyl)-methyl]-3-(5-methoxy-pyridin-2-yl)-piperidin-4-yl]-urea
**Ex-116** trans-1-(4-Chlorophenyl)-3-[3-(5-chloro-thiophen-2-yl)-1-[(4-methoxy-cyclohexyl)-methyl]-piperidin-4-yl]-urea
**Ex-117** trans-1-(4-Chlorophenyl)-3-[3-(5-methoxy-pyridin-2-yl)-1-(2-methylamino-ethyl)-piperidin-4-yl]-urea
**Ex-118** trans-1-[1-(2-Dimethylamino-acetyl)-3-(5-methoxy-pyridin-2-yl)-piperidin-4-yl]-3-(3-methyl-isothiazol-5-yl)-urea
**Ex-119** trans-1-[3-(5-Methoxy-pyridin-2-yl)-1-(oxetane-3-carbonyl)-piperidin-4-yl]-3-(3-methyl-isothiazol-5-yl)-urea
**Ex-120** trans-1-(4-Chlorophenyl)-3-[1-(2-hydroxy-2-methyl-propyl)-3-(5-methoxy-pyridin-2-yl)-piperidin-4-yl]-urea
**Ex-121** trans-1-[1-(2-Hydroxy-2-methyl-propyl)-3-(5-methoxy-pyridin-2-yl)-piperidin-4-yl]-3-(3-methyl-isothiazol-5-yl)-urea
**Ex-122** trans-1-[1-(2-Hydroxy-ethyl)-3-(5-methoxy-pyridin-2-yl)-piperidin-4-yl]-3-(3-methyl-isothiazol-5-yl)-urea
**Ex-123** trans-1-[1-(2-Hydroxy-acetyl)-3-(5-methoxy-pyridin-2-yl)-piperidin-4-yl]-3-(3-methyl-isothiazol-5-yl)-urea
**Ex-124** trans-1-(4-Chlorophenyl)-3-[3-(5-methoxy-pyridin-2-yl)-1-(piperidin-4-yl-methyl)-piperidin-4-yl]-urea ent-1
**Ex-125** trans-1-(4-Chlorophenyl)-3-[3-(5-methoxy-pyridin-2-yl)-1-[(1-methylsulfonyl-1H-pyrazol-4-yl)-methyl]-piperidin-4-yl]-urea
**Ex-126** trans-1-(4-Chlorophenyl)-3-[3-(5-methoxy-pyridin-2-yl)-1-methyl-piperidin-4-yl]-urea
**Ex-127** trans-1-(4-Chlorophenyl)-3-[1-(cyclopropyl-methyl)-3-(5-methoxy-pyridin-2-yl)-piperidin-4-yl]-urea
**Ex-128** trans-1-(4-Cyano-phenyl)-3-[3-(5-methoxy-pyridin-2-yl)-1-(6-methyl-pyridine-3-carbonyl)-piperidin-4-yl]-urea
**Ex-129** trans-2-[4-[[(4-Chlorophenyl)-carbamoyl]amino]-3-(5-methoxy-pyridin-2-yl)-piperidin-1-yl]-acetamide
**Ex-130** trans-1-(4-Chlorophenyl)-3-[3-(5-chloro-thiophen-2-yl)-1-(piperidine-4-carbonyl)-piperidin-4-yl]-urea
**Ex-131** trans-1-(4-Chlorophenyl)-3-[3-(5-chloro-thiophen-2-yl)-1-(3-hydroxy-propanoyl)-piperidin-4-yl]-urea
**Ex-132** trans-1-(4-Chlorophenyl)-3-[3-(5-chloro-thiophen-2-yl)-1-(4-hydroxy-cyclohexanecarbonyl)-piperidin-4-yl]-urea
**Ex-133** trans-1-[3-(5-Methoxy-pyridin-2-yl)-1-(oxetan-3-yl)-piperidin-4-yl]-3-(3-methyl-isothiazol-5-yl)-urea
**Ex-134** trans-1-[3-(5-Methoxy-pyridin-2-yl)-1-(2-methylamino-ethyl)-piperidin-4-yl]-3-(3-methyl-isothiazol-5-yl)-urea
**Ex-135** trans-1-[3-(5-Methoxy-pyridin-2-yl)-1-(3-methylamino-propanoyl)-piperidin-4-yl]-3-(3-methyl-isothiazol-5-yl)-urea
**Ex-136** trans-1-(4-Chlorophenyl)-3-[1-[2-(dimethylamino)ethyl]-3-(5-methoxy-pyridin-2-yl)-piperidin-4-yl]-urea
**Ex-137** trans-1-(4-Chlorophenyl)-3-[3-(5-methoxy-pyridin-2-yl)-1-(3-methylamino-propyl)-piperidin-4-yl]-urea
**Ex-138** trans-1-[1-(3-Hydroxy-cyclobutanecarbonyl)-3-(5-methoxy-pyridin-2-yl)-piperidin-4-yl]-3-(3-methyl-isothiazol-5-yl)-urea
**Ex-139** trans-1-[1-(2-Hydroxy-acetyl)-3-(5-methoxy-pyridin-2-yl)-piperidin-4-yl]-3-phenyl-urea
**Ex-140** trans-1-(4-Cyano-phenyl)-3-[3-(5-methoxy-pyridin-2-yl)-1-[(1-methylsulfonyl-1H-pyrazol-4-yl)-methyl]-piperidin-4-yl]-urea
**Ex-141** trans-1-(4-Cyano-phenyl)-3-[3-(5-methoxy-pyridin-2-yl)-1-[6-(trifluoromethyl)-pyridine-3-carbonyl]-piperidin-4-yl]-urea
**Ex-142** trans-3-[4-[[(4-Chlorophenyl)-carbamoyl]amino]-3-(5-methoxy-pyridin-2-yl)-piperidin-1-yl]-propionamide
**Ex-143** trans-1-[[4-[[(4-Chlorophenyl)-carbamoyl]amino]-3-(5-methoxy-pyridin-2-yl)-piperidin-1-yl]-methyl]-cyclopropane-1-carboxylic acid amide
**Ex-144** trans-1-(4-Chlorophenyl)-3-[1-(3-cyano-3-methyl-butyl)-3-(5-methoxy-pyridin-2-yl)-piperidin-4-yl]-urea
**Ex-145** trans-1-(4-Chlorophenyl)-3-[3-(5-methoxy-pyridin-2-yl)-1-[(1-methyl-1H-pyrazol-4-yl)-methyl]-piperidin-4-yl]-urea
**Ex-146** trans-1-(4-Cyano-phenyl)-3-[3-(5-methoxy-pyridin-2-yl)-1-[(1-methyl-1H-pyrazol-4-yl)-methyl]-piperidin-4-yl]-urea
**Ex-147** trans-1-(4-Chlorophenyl)-3-[3-(5-methoxy-pyridin-2-yl)-1-[(1-methyl-piperidin-4-yl)-methyl]-piperidin-4-yl]-urea
**Ex-148** trans-1-[3-(5-Methoxy-pyridin-2-yl)-1-(2-methylsulfonyl-ethyl)-piperidin-4-yl]-3-(3-methyl-isothiazol-5-yl)-urea
**Ex-149** trans-1-[1-(1,1-Dioxo-thian-4-yl)-3-(5-methoxy-pyridin-2-yl)-piperidin-4-yl]-3-(3-methyl-isothiazol-5-yl)-urea
**Ex-150** trans-1-[1-(2,2-Difluoro-ethyl)-3-(5-methoxy-pyridin-2-yl)-piperidin-4-yl]-3-phenyl-urea
**Ex-151** trans-1-[3-(5-Methoxy-pyridin-2-yl)-1-(piperidine-4-carbonyl)-piperidin-4-yl]-3-phenyl-urea
**Ex-152** trans-1-[3-(5-Chloro-pyridin-2-yl)-1-(2-hydroxy-ethyl)-piperidin-4-yl]-3-phenyl-urea
**Ex-153** trans-1-[3-(5-Chloro-pyridin-2-yl)-1-(2,2-difluoro-ethyl)-piperidin-4-yl]-3-phenyl-urea
**Ex-154** trans-1-(4-Cyano-phenyl)-3-[1-(2-methoxy-ethyl)-3-(5-methoxy-pyridin-2-yl)-piperidin-4-yl]-urea
**Ex-155** trans-1-(4-Chlorophenyl)-3-[1-[(1-cyano-cyclopropyl)-methyl]-3-(5-methoxy-pyridin-2-yl)-piperidin-4-yl]-urea
**Ex-156** trans-1-[3-(5-Methoxy-pyridin-2-yl)-1-(1-methyl-piperidine-4-carbonyl)-piperidin-4-yl]-3-phenyl-urea
**Ex-157** trans-1-[1-(2-Dimethylamino-acetyl)-3-(5-methoxy-pyridin-2-yl)-piperidin-4-yl]-3-phenyl-urea
**Ex-158** trans-1-[1-(3-Methoxy-propyl)-3-(5-methoxy-pyridin-2-yl)-piperidin-4-yl]-3-phenyl-urea
**Ex-159** trans-1-[1-(3-Hydroxy-propyl)-3-(5-methoxy-pyridin-2-yl)-piperidin-4-yl]-3-phenyl-urea
**Ex-160** trans-1-[3-(5-Methoxy-pyridin-2-yl)-1-(2-methylsulfonyl-ethyl)-piperidin-4-yl]-3-phenyl-urea
**Ex-161** trans-1-[3-(5-Methoxy-pyridin-2-yl)-1-(oxetan-3-yl-methyl)-piperidin-4-yl]-3-phenyl-urea
**Ex-162** trans-1-[3-(5-Methoxy-pyridin-2-yl)-1-(oxetan-3-yl)-piperidin-4-yl]-3-phenyl-urea
**Ex-163** trans-1-(4-Chlorophenyl)-3-[1-[(1-hydroxy-cyclopropyl)-methyl]-3-(5-methoxy-pyridin-2-yl)-piperidin-4-yl]-urea
**Ex-164** trans-1-[1-(2-Hydroxy-ethyl)-3-(5-methoxy-pyridin-2-yl)-piperidin-4-yl]-3-phenyl-urea
**Ex-165** trans-1-[1-(Azetidine-3-carbonyl)-3-(5-methoxy-pyridin-2-yl)-piperidin-4-yl]-3-phenyl-urea
**Ex-167** trans-1-[3-(5-Chloro-pyridin-2-yl)-1-(piperidine-4-carbonyl)-piperidin-4-yl]-3-phenyl-urea
**Ex-168** trans-1-(4-Chlorophenyl)-3-[3-(5-methoxy-pyridin-2-yl)-piperidin-4-yl]-urea ent-1
**Ex-169** trans-1-(4-Chlorophenyl)-3-[3-(5-methoxy-pyridin-2-yl)-piperidin-4-yl]-urea ent-2
**Ex-170** trans-2-[4-[[(4-Chlorophenyl)-carbamoyl]amino]-3-(5-methoxy-pyridin-2-yl)-piperidin-1-yl]-acetamide ent-1
**Ex-171** trans-2-[4-[[(4-Chlorophenyl)-carbamoyl]amino]-3-(5-methoxy-pyridin-2-yl)-piperidin-1-yl]-acetamide ent-2
**Ex-172** trans-1-(4-Chlorophenyl)-3-[1-(cyclopropyl-methyl)-3-(5-methoxy-pyridin-2-yl)-piperidin-4-yl]-urea ent-1
**Ex-173** trans-1-(4-Chlorophenyl)-3-[1-(cyclopropyl-methyl)-3-(5-methoxy-pyridin-2-yl)-piperidin-4-yl]-urea ent-2
**Ex-174** trans-1-(4-Cyano-phenyl)-3-[1-(2,2-difluoro-ethyl)-3-(5-methoxy-pyridin-2-yl)-piperidin-4-yl]-urea ent-2
**Ex-175** trans-1-(4-Cyano-phenyl)-3-[1-(2,2-difluoro-ethyl)-3-(5-methoxy-pyridin-2-yl)-piperidin-4-yl]-urea ent-2
**Ex-176** trans- 1-[1-(2-Hydroxy-2-methyl-propyl)-3-(5-methoxy-pyridin-2-yl)-piperidin-4-yl]-3-(3-methyl-isothiazol-5-yl)-urea ent-1
**Ex-177** trans-1-[1-(2-Hydroxy-2-methyl-propyl)-3-(5-methoxy-pyridin-2-yl)-piperidin-4-yl]-3-(3-methyl-isothiazol-5-yl)-urea ent-2
**Ex-178** trans-1-(4-Chlorophenyl)-3-[1-(2-hydroxy-2-methyl-propyl)-3-(5-methoxy-pyridin-2-yl)-piperidin-4-yl]-urea ent-1
**Ex-179** trans-1-(4-Chlorophenyl)-3-[1-(2-hydroxy-2-methyl-propyl)-3-(5-methoxy-pyridin-2-yl)-piperidin-4-yl]-urea ent-2
**Ex-180** trans-1-(4-Chlorophenyl)-3-[1-(1-cyano-cyclopropyl)-3-(5-methoxy-pyridin-2-yl)-piperidin-4-yl]-urea
**Ex-181** trans-1-[4-[[(4-Chlorophenyl)-carbamoyl]amino]-3-(5-methoxy-pyridin-2-yl)-piperidin-1-yl]-cyclopropane-1-carboxylic acid amide
**Ex-182** trans-1-[1-[(1-Hydroxy-cyclopropyl)-methyl]-3-(5-methoxy-pyridin-2-yl)-piperidin-4-yl]-3-(3-methyl-isothiazol-5-yl)-urea
**Ex-183** trans-1-[1-(3-Hydroxy-cyclobutanecarbonyl)-3-(5-methoxy-pyridin-2-yl)-piperidin-4-yl]-3-(3-methyl-isothiazol-5-yl)-urea
**Ex-184** trans-1-[3-(5-Chloro-pyridin-2-yl)-1-[(1-methylsulfonyl-1H-pyrazol-3-yl)-methyl]-piperidin-4-yl]-3-phenyl-urea
**Ex-185** trans-1-[1-(Azetidin-3-yl)-3-(5-chloro-pyridin-2-yl)-piperidin-4-yl]-3-phenyl-urea
**Ex-186** trans-1-[1-(Azetidin-3-yl-methyl)-3-(5-chloro-pyridin-2-yl)-piperidin-4-yl]-3-phenyl-urea
**Ex-187** trans-1-(4-Chlorophenyl)-3-[3-(5-methoxy-pyridin-2-yl)-1-(1-methyl-piperidine-4-carbonyl)-piperidin-4-yl]-urea ent-1
**Ex-188** trans-1-(4-Chlorophenyl)-3-[3-(5-methoxy-pyridin-2-yl)-1-(1-methyl-piperidine-4-carbonyl)-piperidin-4-yl]-urea ent-2
**Ex-189** trans-1-(4-Chlorophenyl)-3-[1-(2-dimethylamino-acetyl)-3-(5-methoxy-pyridin-2-yl)-piperidin-4-yl]-urea ent-1
**Ex-190** trans-1-(4-Chlorophenyl)-3-[1-(2-dimethylamino-acetyl)-3-(5-methoxy-pyridin-2-yl)-piperidin-4-yl]-urea ent-2
**Ex-191** trans-1-(4-Chlorophenyl)-3-[1-(3-methoxy-propyl)-3-(5-methoxy-pyridin-2-yl)-piperidin-4-yl]-urea ent-1
**Ex-192** trans-1-(4-Chlorophenyl)-3-[1-(3-methoxy-propyl)-3-(5-methoxy-pyridin-2-yl)-piperidin-4-yl]-urea ent-2
**Ex-193** trans-1-(4-Chlorophenyl)-3-[3-(3-fluoro-5-methoxy-pyridin-2-yl)-1-(3-methoxy-propyl)-piperidin-4-yl]-urea
**Ex-194** trans-1-(4-Chlorophenyl)-3-[1-[(3-hydroxy-oxetan-3-yl)-methyl]-3-(5-methoxy-pyridin-2-yl)-piperidin-4-yl]-urea
**Ex-195** trans-1-(4-Chlorophenyl)-3-[1-[(3-methoxy-cyclobutyl)-methyl]-3-(5-methoxy-pyridin-2-yl)-piperidin-4-yl]-urea
**Ex-196** trans-1-(4-Chlorophenyl)-3-[3-(3-fluoro-5-methoxy-pyridin-2-yl)-1-(2-methylsulfonyl-ethyl)-piperidin-4-yl]-urea
**Ex-197** trans-1-(4-Chlorophenyl)-3-[3-(3-fluoro-5-methoxy-pyridin-2-yl)-1-(3-hydroxy-propyl)-piperidin-4-yl]-urea
**Ex-198** trans-1-(4-Chlorophenyl)-3-[3-(3-fluoro-5-methoxy-pyridin-2-yl)-1-(2-methoxy-ethyl)-piperidin-4-yl]-urea
**Ex-199** trans-1-(4-Chlorophenyl)-3-[3-(3-fluoro-5-methoxy-pyridin-2-yl)-1-(2-hydroxy-ethyl)-piperidin-4-yl]-urea
**Ex-200** trans-1-(4-Chlorophenyl)-3-[3-(3-fluoro-5-methoxy-pyridin-2-yl)-1-(oxetan-3-yl-methyl)-piperidin-4-yl]-urea
**Ex-201** trans-1-(4-Chlorophenyl)-3-[1-[(1-methoxy-cyclopropyl)-methyl]-3-(5-methoxy-pyridin-2-yl)-piperidin-4-yl]-urea
**Ex-202** trans-1-(4-Chlorophenyl)-3-[1-[(3-methoxy-cyclobutyl)-methyl]-3-(5-methoxy-pyridin-2-yl)-piperidin-4-yl]-urea
**Ex-203** trans-1-(4-Chlorophenyl)-3-[1-[(3-hydroxy-cyclobutyl)-methyl]-3-(5-methoxy-pyridin-2-yl)-piperidin-4-yl]-urea
**Ex-204** trans-1-[3-(5-Methoxy-pyridin-2-yl)-1-(oxetan-3-yl-methyl)-piperidin-4-yl]-3-(3-methyl-isothiazol-5-yl)-urea
**Ex-205** trans-1-(4-Chlorophenyl)-3-[1-[(3-hydroxy-cyclobutyl)-methyl]-3-(5-methoxy-pyridin-2-yl)-piperidin-4-yl]-urea
**Ex-206** trans-1-(4-Chlorophenyl)-3-[1-(2-hydroxy-ethyl)-3-(5-methoxy-pyridin-2-yl)-piperidin-4-yl]-urea
**Ex-207** trans-1-(4-Chlorophenyl)-3-[1-[(3-hydroxy-3-methyl-cyclobutyl)-methyl]-3-(5-methoxy-pyridin-2-yl)-piperidin-4-yl]-urea
**Ex-208** trans-1-(4-Chlorophenyl)-3-[3-(3-fluoro-5-methoxy-pyridin-2-yl)-1-(2-hydroxy-2-methyl-propyl)-piperidin-4-yl]-urea
**Ex-209** trans-1-(4-Chlorophenyl)-3-[1-(cyclopropyl-methyl)-3-(3-fluoro-5-methoxy-pyridin-2-yl)-piperidin-4-yl]-urea
**Ex-210** trans-1-(4-Chlorophenyl)-3-[3-(5-methoxy-pyridin-2-yl)-1-(2-methyl-pyrimidin-4-yl)-piperidin-4-yl]-urea
**Ex-211** trans-1-[3-(5-Methoxy-pyridin-2-yl)-1-(2-methyl-pyrimidin-4-yl)-piperidin-4-yl]-3-(3-methyl-isothiazol-5-yl)-urea
**Ex-212** trans-1-(4-Chlorophenyl)-3-[1-(3-dimethylamino-propyl)-3-(3-fluoro-5-methoxy-pyridin-2-yl)-piperidin-4-yl]-urea
**Ex-213** trans-1-(4-Chlorophenyl)-3-[1-[2-(dimethylamino)ethyl]-3-(3-fluoro-5-methoxy-pyridin-2-yl)-piperidin-4-yl]-urea
**Ex-214** trans-1-[1-(2,2-Difluoro-ethyl)-3-(5-methoxy-pyridin-2-yl)-piperidin-4-yl]-3-(3-methyl-isothiazol-5-yl)-urea
**Ex-215** trans-2-[4-[[(4-Chlorophenyl)-carbamoyl]amino]-3-(5-methoxy-pyridin-2-yl)-piperidin-1-yl]-N-methyl-acetamide
**Ex-216** trans-2-[4-[[(4-Chlorophenyl)-carbamoyl]amino]-3-(5-methoxy-pyridin-2-yl)-piperidin-1-yl]-N,N -dimethyl-acetamide
**Ex-217** trans-1-[1-[(3-Hydroxy-oxetan-3-yl)-methyl]-3-(5-methoxy-pyridin-2-yl)-piperidin-4-yl]-3-(3-methyl-isothiazol-5-yl)-urea
**Ex-218** trans-1-(4-Chlorophenyl)-3-[3-(3-fluoro-5-methoxy-pyridin-2-yl)-1-(oxetan-3-yl)-piperidin-4-yl]-urea
**Ex-219** trans-1-(4-Chlorophenyl)-3-[1-(2-hydroxy-1,1-dimethyl-ethyl)-3-(5-methoxy-pyridin-2-yl)-piperidin-4-yl]-urea
**Ex-220** trans-1-(4-Chlorophenyl)-3-[1-[(1-hydroxy-cyclobutyl)-methyl]-3-(5-methoxy-pyridin-2-yl)-piperidin-4-yl]-urea
**Ex-221** trans-1-(4-Chlorophenyl)-3-[3-(3-fluoro-5-methoxy-pyridin-2-yl)-1-[(4-hydroxy-cyclohexyl)-methyl]-piperidin-4-yl]-urea
**Ex-222** trans-2-[4-[[(4-Chlorophenyl)-carbamoyl]amino]-3-(3-fluoro-5-methoxy-pyridin-2-yl)-piperidin-1-yl]-acetamide
**Ex-223** trans-1-(4-Chlorophenyl)-3-[3-(3-fluoro-5-methoxy-pyridin-2-yl)-1-[(4-methoxy-cyclohexyl)-methyl]-piperidin-4-yl]-urea
**Ex-224** trans-1-(4-Chlorophenyl)-3-[3-(5-methoxy-pyridin-2-yl)-1-(6-methyl-pyrazin-2-yl)-piperidin-4-yl]-urea
**Ex-225** trans-2-[4-[[(4-Chlorophenyl)-carbamoyl]amino]-3-(5-methoxy-pyridin-2-yl)-piperidin-1-yl]-2-methyl-propionamide
**Ex-226** trans-1-(4-Chlorophenyl)-3-[3-(5-methoxy-pyridin-2-yl)-1-pyrazin-2-yl-piperidin-4-yl]-urea
**Ex-227** trans-1-(4-Chlorophenyl)-3-[1-(2-methoxy-2-methyl-propyl)-3-(5-methoxy-pyridin-2-yl)-piperidin-4-yl]-urea
**Ex-228** trans-1-(4-Chlorophenyl)-3-[1-[(3-methoxy-3-methyl-cyclobutyl)-methyl]-3-(5-methoxy-pyridin-2-yl)-piperidin-4-yl]-urea
**Ex-229** trans-1-(4-Chlorophenyl)-3-[1-[(1-methoxy-cyclobutyl)-methyl]-3-(5-methoxy-pyridin-2-yl)-piperidin-4-yl]-urea
**Ex-230** trans-3-[4-[[(4-Chlorophenyl)-carbamoyl]amino]-3-(5-methoxy-pyridin-2-yl)-piperidin-1-yl]-2,2-dimethy 1- propionamide
**Ex-231** trans-1-(4-Chlorophenyl)-3-[3-(5-methoxy-pyridin-2-yl)-1-pyridazin-3-yl-piperidin-4-yl]-urea
**Ex-232** trans-1-(4-Chlorophenyl)-3-[3-(5-methoxy-pyridin-2-yl)-1-(6-methyl-pyrimidin-4-yl)-piperidin-4-yl]-urea
**Ex-233** trans-1-(4-Chlorophenyl)-3-[1-(1-cyano-1-methyl-ethyl)-3-(5-methoxy-pyridin-2-yl)-piperidin-4-yl]-urea
**Ex-234** trans-1-(4-Chlorophenyl)-3-[1-(2-cyano-2-methyl-propyl)-3-(5-methoxy-pyridin-2-yl)-piperidin-4-yl]-urea
**Ex-235** trans-1-(4-Chlorophenyl)-3-[1-[2-(1-cyano-cyclopropyl)-ethyl]-3-(5-methoxy-pyridin-2-yl)-piperidin-4-yl]-urea
**Ex-236** trans-1-(4-Chlorophenyl)-3-[3-(5-methoxy-pyridin-2-yl)-1-(2,2,6,6-tetramethyl-piperidin-4-yl)-piperidin-4-yl]-urea ent-1
**Ex-237** trans-1-(4-Chlorophenyl)-3-[3-(5-methoxy-pyridin-2-yl)-1-(2,2,6,6-tetramethyl-piperidin-4-yl)-piperidin-4-yl]-urea ent-2
**Ex-238** trans-1-(4-Chlorophenyl)-3-[1-(3-dimethylamino-2,2-difluoro-propyl)-3-(5-methoxy-pyridin-2-yl)-piperidin-4-yl]-urea
**Ex-239** trans-1-[4-[[(4-Chlorophenyl)-carbamoyl]amino]-3-(5-methoxy-pyridin-2-yl)-piperidin-1-yl]-N-methyl-cyclopropane-1-carboxylic acid amide
**Ex-240** trans-1-[4-[[(4-Chlorophenyl)-carbamoyl]amino]-3-(5-methoxy-pyridin-2-yl)-piperidin-1-yl]-N,N-dimethyl-cyclopropane-1-carboxylic acid amide
**Ex-241** trans-1-[1-[(3-Hydroxy-cyclobutyl)-methyl]-3-(5-methoxy-pyridin-2-yl)-piperidin-4-yl]-3-(3-methyl-isothiazol-5-yl)-urea
**Ex-242** trans-1-[1-[(3-Methoxy-cyclobutyl)-methyl]-3-(5-methoxy-pyridin-2-yl)-piperidin-4-yl]-3-(3-methyl-isothiazol-5-yl)-urea
**Ex-243** trans-1-[1-[(3-Hydroxy-cyclobutyl)-methyl]-3-(5-methoxy-pyridin-2-yl)-piperidin-4-yl]-3-(3-methyl-isothiazol-5-yl)-urea
**Ex-244** trans-1-[1-[(3-Methoxy-cyclobutyl)-methyl]-3-(5-methoxy-pyridin-2-yl)-piperidin-4-yl]-3-(3-methyl-isothiazol-5-yl)-urea
**Ex-245** trans-1-[1-[(3-Hydroxy-3-methyl-cyclobutyl)-methyl]-3-(5-methoxy-pyridin-2-yl)-piperidin-4-yl]-3-(3-methyl-isothiazol-5-yl)-urea
**Ex-246** trans-1-[1-[(3-Methoxy-3-methyl-cyclobutyl)-methyl]-3-(5-methoxy-pyridin-2-yl)-piperidin-4-yl]-3-(3-methyl-isothiazol-5-yl)-urea
**Ex-247** trans-1-(4-Chlorophenyl)-3-[1-[1-(hydroxymethyl)-cyclopropyl]-3-(5-methoxy-pyridin-2-yl)-piperidin-4-yl]-urea
**Ex-248** trans-1-(4-Chlorophenyl)-3-[1-[1-(methoxymethyl)-cyclopropyl]-3-(5-methoxy-pyridin-2-yl)-piperidin-4-yl]-urea
**Ex-249** trans-1-(4-Chlorophenyl)-3-[1-(2-methoxy-1,1-dimethyl-ethyl)-3-(5-methoxy-pyridin-2-yl)-piperidin-4-yl]-urea
**Ex-250** trans-1-(4-Chlorophenyl)-3-[1-[3-(methoxymethyl)-oxetan-3-yl]-3-(5-methoxy-pyridin-2-yl)-piperidin-4-yl]-urea
**Ex-251** trans-1-(4-Chlorophenyl)-3-[1-[3-(hydroxymethyl)-oxetan-3-yl]-3-(5-methoxy-pyridin-2-yl)-piperidin-4-yl]-urea
**Ex-252** trans-1-(4-Chlorophenyl)-3-[1-(2,2-difluoro-3-hydroxy-propyl)-3-(5-methoxy-pyridin-2-yl)-piperidin-4-yl]-urea
**Ex-253** trans-1-(4-Chlorophenyl)-3-[1-(2,2-difluoro-3-methoxy-propyl)-3-(5-methoxy-pyridin-2-yl)-piperidin-4-yl]-urea
**Ex-254** trans-1-[1-(2,2-Difluoro-3-hydroxy-propyl)-3-(5-methoxy-pyridin-2-yl)-piperidin-4-yl]-3-(3-methyl-isothiazol-5-yl)-urea
**Ex-255** trans-1-[1-(2,2-Difluoro-3-methoxy-propyl)-3-(5-methoxy-pyridin-2-yl)-piperidin-4-yl]-3-(3-methyl-isothiazol-5-yl)-urea
**Ex-256** trans-1-(4-Chlorophenyl)-3-[3-(5-methoxy-pyridin-2-yl)-1-pyridazin-4-yl-piperidin-4-yl]-urea
**Ex-257** trans-1-(4-Chlorophenyl)-3-[3-(5-methoxy-pyridin-2-yl)-1-(2-methyl-pyrimidin-5-yl)-piperidin-4-yl]-urea
**Ex-258** trans-1-(4-Chlorophenyl)-3-[1-[2-(hydroxymethyl)-pyrimidin-5-yl]-3-(5-methoxy-pyridin-2-yl)-piperidin-4-yl]-urea
**Ex-259** trans-1-(4-Chlorophenyl)-3-[1-[2-(hydroxymethyl)-phenyl]-3-(5-methoxy-pyridin-2-yl)-piperidin-4-yl]-urea
**Ex-260** trans-1-(4-Chlorophenyl)-3-[1-[3-(hydroxymethyl)-phenyl]-3-(5-methoxy-pyridin-2-yl)-piperidin-4-yl]-urea
**Ex-261** trans-1-(4-Chlorophenyl)-3-[1-[4-(hydroxymethyl)-phenyl]-3-(5-methoxy-pyridin-2-yl)-piperidin-4-yl]-urea
**Ex-262** trans-1-(4-Chlorophenyl)-3-[1-[6-(hydroxymethyl)-pyridin-3-yl]-3-(5-methoxy-pyridin-2-yl)-piperidin-4-yl]-urea
**Ex-263** trans-1-(4-Chlorophenyl)-3-[1-[6-(2-hydroxy-ethylamino)-pyridin-2-yl]-3-(5-methoxy-pyridin-2-yl)-piperidin-4-yl]-urea
**Ex-264** trans-1-(4-Chlorophenyl)-3-[1-[5-(2-hydroxy-ethylamino)-pyridin-2-yl]-3-(5-methoxy-pyridin-2-yl)-piperidin-4-yl]-urea
**Ex-265** trans-1-(4-Chlorophenyl)-3-[1-[4-(2-hydroxy-ethylamino)-pyridin-2-yl]-3-(5-methoxy-pyridin-2-yl)-piperidin-4-yl]-urea
**Ex-266** trans-1-(4-Chlorophenyl)-3-[1-[(1-hydroxy-cyclopropyl)-methyl]-3-(5-methoxy-pyridin-2-yl)-piperidin-4-yl]-urea ent-1
**Ex-267** trans-1-(4-Chlorophenyl)-3-[1-[(1-hydroxy-cyclopropyl)-methyl]-3-(5-methoxy-pyridin-2-yl)-piperidin-4-yl]-urea ent-2
**Ex-268** trans-1-(4-Chlorophenyl)-3-[3-(5-methoxy-pyridin-2-yl)-1-(3-methyl-pyridazin-4-yl)-piperidin-4-yl]-urea
**Ex-269** trans-1-(4-Chlorophenyl)-3-[1-(6-hydroxy-pyridin-2-yl)-3-(5-methoxy-pyridin-2-yl)-piperidin-4-yl]-urea
**Ex-270** trans-1-(4-Chlorophenyl)-3-[1-(2-hydroxy-pyridin-4-yl)-3-(5-methoxy-pyridin-2-yl)-piperidin-4-yl]-urea
**Ex-271** trans-1-(4-Chlorophenyl)-3-[3-(5-methoxy-pyridin-2-yl)-1-(5-methyl-pyridazin-4-yl)-piperidin-4-yl]-urea
**Ex-272** trans-1-(4-Chlorophenyl)-3-[1-(2-hydroxy-pyridin-3-yl)-3-(5-methoxy-pyridin-2-yl)-piperidin-4-yl]-urea
**Ex-273** trans-1-(4-Chlorophenyl)-3-[1-(6-hydroxy-pyridin-3-yl)-3-(5-methoxy-pyridin-2-yl)-piperidin-4-yl]-urea
**Ex-274** trans-1-(4-Chlorophenyl)-3-[3-(5-methoxy-pyridin-2-yl)-1-(6-methyl-pyridazin-4-yl)-piperidin-4-yl]-urea
**Ex-275** trans-1-(4-Chlorophenyl)-3-[3-(5-methoxy-pyridin-2-yl)-1-(3-methyl-pyrazin-2-yl)-piperidin-4-yl]-urea
**Ex-276** trans-1-(4-Chlorophenyl)-3-[1-[(3,5-dimethyl-isoxazol-4-yl)-methyl]-3-(5-methoxy-pyridin-2-yl)- piperidin-4- yl]-urea
**Ex-277** trans-1-(4-Chlorophenyl)-3-[3-(5-methoxy-pyridin-2-yl)-1-[(5-methyl-[1,2,4]oxadiazol-3-yl)-methyl]-piperidin-4-yl]-urea
**Ex-278** trans-1-(4-Chlorophenyl)-3 -[3 -(5-methoxy-pyridin-2-yl)-1-[(1-methyl-1H-[1,2,4]triazol-3-yl)-methyl]-piperidin-4-yl]-urea
**Ex-279** trans-1-(4-Chlorophenyl)-3-[3-(5-methoxy-pyridin-2-yl)-1-[(5-methyl-1H-[1,2,4]triazol-3-yl)-methyl]-piperidin-4-yl]-urea
**Ex-280** trans-1-(4-Chlorophenyl)-3-[3-(5-methoxy-pyridin-2-yl)-1-[(3-methyl-[1,2,4]oxadiazol-5-yl)-methyl]-piperidin-4-yl]-urea
**Ex-281** trans-1-(4-Chlorophenyl)-3-[1-[[5-(hydroxymethyl)-isoxazol-3-yl]-methyl]-3-(5-methoxy-pyridin-2-yl)-piperidin-4-yl]-urea
**Ex-282** trans-1-(4-Chlorophenyl)-3-[1-[[1-(2-hydroxy-ethyl)-1H-pyrazol-3-yl]-methyl]-3-(5-methoxy-pyridin-2-yl)-piperidin-4-yl]-urea
**Ex-283** trans-1-(4-Chlorophenyl)-3-[3-(5-methoxy-pyridin-2-yl)-1-[(1-methylsulfonyl-1H-[1,2,4]triazol-3-yl)-methyl]-piperidin-4-yl]-urea
**Ex-284** trans-2-[4-[[(4-Chlorophenyl)-carbamoyl]amino]-3-(5-methoxy-pyridin-2-yl)-piperidin-1-yl]-N-ethyl-acetamide
**Ex-285** trans-2-[4-[[(4-Chlorophenyl)-carbamoyl]amino]-3-(5-methoxy-pyridin-2-yl)-piperidin-1-yl]-N-propyl-acetamide
**Ex-286** trans-2-[4-[[(4-Chlorophenyl)-carbamoyl]amino]-3-(5-methoxy-pyridin-2-yl)-piperidin-1-yl]-N-(2-fluoro-ethyl)-acetamide
**Ex-287** trans-2-[4-[[(4-Chlorophenyl)-carbamoyl]amino]-3-(5-methoxy-pyridin-2-yl)-piperidin-1-yl]-N-(2,2-difluoro-ethyl)-acetamide
**Ex-288** trans-2-[4-[[(4-Chlorophenyl)-carbamoyl]amino]-3-(5-methoxy-pyridin-2-yl)-piperidin-1-yl]-N-(2,2,2-trifluoro-ethyl)-acetamide
**Ex-289** trans-2-[4-[[(4-Chlorophenyl)-carbamoyl]amino]-3-(5-methoxy-pyridin-2-yl)-piperidin-1-yl]-N-isopropyl-acetamide
**Ex-290** trans-2-[4-[[(4-Chlorophenyl)-carbamoyl]amino]-3-(5-methoxy-pyridin-2-yl)-piperidin-1-yl]-N-cyclopropyl-acetamide
**Ex-291** trans-2-[4-[[(4-Chlorophenyl)-carbamoyl]amino]-3-(5-methoxy-pyridin-2-yl)-piperidin-1-yl]-N-cyclobutyl-acetamide
**Ex-292** trans-2-[4-[[(4-Chlorophenyl)-carbamoyl]amino]-3-(5-methoxy-pyridin-2-yl)-piperidin-1-yl]-N-(2-methyl-propyl)-acetamide
**Ex-293** trans-2-[4-[[(4-Chlorophenyl)-carbamoyl]amino]-3-(5-methoxy-pyridin-2-yl)-piperidin-1-yl]-N-(2-fluoro-2-methyl-propyl)-acetamide
**Ex-294** trans-2-[4-[[(4-Chlorophenyl)-carbamoyl]amino]-3-(5-methoxy-pyridin-2-yl)-piperidin-1-yl]-N-(cyclopropyl-methyl)-acetamide
**Ex-295** trans-2-[4-[[(4-Chlorophenyl)-carbamoyl]amino]-3-(5-methoxy-pyridin-2-yl)-piperidin-1-yl]-N-(cyclobutyl-methyl)-acetamide
**Ex-296** trans-2-[4-[[(4-Chlorophenyl)-carbamoyl]amino]-3-(5-methoxy-pyridin-2-yl)-piperidin-1-yl]-N-(2-hydroxy-ethyl)-acetamide
**Ex-297** trans-2-[4-[[(4-Chlorophenyl)-carbamoyl]amino]-3-(5-methoxy-pyridin-2-yl)-piperidin-1-yl]-N-(2-methoxy-ethyl)-acetamide
**Ex-298** trans-2-[4-[[(4-Chlorophenyl)-carbamoyl]amino]-3-(5-methoxy-pyridin-2-yl)-piperidin-1-yl]-N-(oxetan-3-yl)-acetamide
**Ex-299** trans-2-[4-[[(4-Chlorophenyl)-carbamoyl]amino]-3-(5-methoxy-pyridin-2-yl)-piperidin-1-yl]-N-tetrahydro-pyran-4-yl-acetamide
**Ex-300** trans-2-[4-[[(4-Chlorophenyl)-carbamoyl]amino]-3-(5-methoxy-pyridin-2-yl)-piperidin-1-yl]-N-isoxazol-3-yl-acetamide
**Ex-301** trans-2-[4-[[(4-Chlorophenyl)-carbamoyl]amino]-3-(5-methoxy-pyridin-2-yl)-piperidin-1-yl]-N-isoxazol-4-yl-acetamide
**Ex-302** trans-2-[4-[[(4-Chlorophenyl)-carbamoyl]amino]-3-(5-methoxy-pyridin-2-yl)-piperidin-1-yl]-N-isoxazol-5-yl-acetamide
**Ex-303** trans-2-[4-[[(4-Chlorophenyl)-carbamoyl]amino]-3-(5-methoxy-pyridin-2-yl)-piperidin-1-yl]-N-pyridin-2-yl-acetamide
**Ex-304** trans-2-[4-[[(4-Chlorophenyl)-carbamoyl]amino]-3-(5-methoxy-pyridin-2-yl)-piperidin-1-yl]-N-(1H-pyrazol-3-yl)-acetamide
**Ex-305** trans-2-[4-[[(4-Chlorophenyl)-carbamoyl]amino]-3-(5-methoxy-pyridin-2-yl)-piperidin-1-yl]-N-(1H-pyrazol-4-yl)-acetamide
**Ex-306** trans-2-[4-[[(4-Chlorophenyl)-carbamoyl]amino]-3-(5-methoxy-pyridin-2-yl)-piperidin-1-yl]-N-(1-methyl-1H-pyrazol-3-yl)-acetamide
Ex-307 trans-2-[4-[[(4-Chlorophenyl)-carbamoyl]amino]-3-(5-methoxy-pyridin-2-yl)-piperidin-1-yl]-N-(1-methyl-1H-pyrazol-4-yl)-acetamide
**Ex-308** trans-2-[4-[[(4-Chlorophenyl)-carbamoyl]amino]-3-(5-methoxy-pyridin-2-yl)-piperidin-1-yl]-N-(2-methyl-2H-pyrazol-3-yl)-acetamide
**Ex-309** trans-2-[4-[[(4-Chlorophenyl)-carbamoyl]amino]-3-(5-methoxy-pyridin-2-yl)-piperidin-1-yl]-N-oxazol-4-yl-acetamide
**Ex-310** trans-2-[4-[[(4-Chlorophenyl)-carbamoyl]amino]-3-(5-methoxy-pyridin-2-yl)-piperidin-1-yl]-N-oxazol-5-yl-acetamide
**Ex-311** trans-2-[4-[[(4-Chlorophenyl)-carbamoyl]amino]-3-(5-methoxy-pyridin-2-yl)-piperidin-1-yl]-N-oxazol-2-yl-acetamide
**Ex-312** trans-2-[4-[[(4-Chlorophenyl)-carbamoyl]amino]-3-(5-methoxy-pyridin-2-yl)-piperidin-1-yl]-N-thiazol-4-yl-acetamide
**Ex-313** trans-2-[4-[[(4-Chlorophenyl)-carbamoyl]amino]-3-(5-methoxy-pyridin-2-yl)-piperidin-1-yl]-N-thiazol-5-yl-acetamide
**Ex-314** trans-2-[4-[[(4-Chlorophenyl)-carbamoyl]amino]-3-(5-methoxy-pyridin-2-yl)-piperidin-1-yl]-N-thiazol-2-yl-acetamide
**Ex-315** trans-2-[4-[[(4-Chlorophenyl)-carbamoyl]amino]-3-(5-methoxy-pyridin-2-yl)-piperidin-1-yl]-N-(1H-pyrazol-3-yl-methyl)-acetamide
**Ex-316** trans-2-[4-[[(4-Chlorophenyl)-carbamoyl]amino]-3-(5-methoxy-pyridin-2-yl)-piperidin-1-yl]-N-(1H-pyrazol-4-yl-methyl)-acetamide
**Ex-317** trans-2-[4-[[(4-Chlorophenyl)-carbamoyl]amino]-3-(5-methoxy-pyridin-2-yl)-piperidin-1-yl]-N-[(1-methyl-1H-pyrazol-3-yl)-methyl]-acetamide
**Ex-318** trans-2-[4-[[(4-Chlorophenyl)-carbamoyl]amino]-3-(5-methoxy-pyridin-2-yl)-piperidin-1-yl]-N-[(1-methyl-1H-pyrazol-4-yl)-methyl]-acetamide
**Ex-319** trans-2-[4-[[(4-Chlorophenyl)-carbamoyl]amino]-3-(5-methoxy-pyridin-2-yl)-piperidin-1-yl]-N-[(2-methyl-2H-pyrazol-3-yl)-methyl]-acetamide
**Ex-320** trans-2-[4-[[(4-Chlorophenyl)-carbamoyl]amino]-3-(5-methoxy-pyridin-2-yl)-piperidin-1-yl]-N-(1H-[1,2,3]triazol-4-yl)-acetamide
**Ex-321** trans-2-[4-[[(4-Chlorophenyl)-carbamoyl]amino]-3-(5-methoxy-pyridin-2-yl)-piperidin-1-yl]-N-(1-methyl-1H-[1,2,3]triazol-4-yl)-acetamide
**Ex-322** trans-2-[4-[[(4-Chlorophenyl)-carbamoyl]amino]-3-(5-methoxy-pyridin-2-yl)-piperidin-1-yl]-N-(3-methyl-3H-[1,2,3]triazol-4-yl)-acetamide
**Ex-323** trans-2-[4-[[(4-Chlorophenyl)-carbamoyl]amino]-3-(5-methoxy-pyridin-2-yl)-piperidin-1-yl]-N-(2H-[1,2,4]triazol-3 -yl)-acetamide
**Ex-324** trans-2-[4-[[(4-Chlorophenyl)-carbamoyl]amino]-3-(5-methoxy-pyridin-2-yl)-piperidin-1-yl]-N-(4-methyl-4H-[1,2,4]triazol-3-yl)-acetamide
**Ex-325** trans-2-[4-[[(4-Chlorophenyl)-carbamoyl]amino]-3-(5-methoxy-pyridin-2-yl)-piperidin-1-yl]-N-(2-methyl-2H-[1,2,4]triazol-3-yl)-acetamide
**Ex-326** trans-2-[4-[[(4-Chlorophenyl)-carbamoyl]amino]-3-(5-methoxy-pyridin-2-yl)-piperidin-1-yl]-N-(1-methyl-1H-[1,2,4]triazol-3-yl)-acetamide
**Ex-327** trans-2-[4-[[(4-Chlorophenyl)-carbamoyl]amino]-3-(5-methoxy-pyridin-2-yl)-piperidin-1-yl]-N-([1,2,4]oxadiazol-3-yl)-acetamide
**Ex-328** trans-2-[4-[[(4-Chlorophenyl)-carbamoyl]amino]-3-(5-methoxy-pyridin-2-yl)-piperidin-1-yl]-N-([1,2,4]oxadiazol-5-yl)-acetamide
**Ex-329** trans-2-[4-[[(4-Chlorophenyl)-carbamoyl]amino]-3-(5-methoxy-pyridin-2-yl)-piperidin-1-yl]-N-([1,3,4]oxadiazol-2-yl)-acetamide
**Ex-330** trans-1-(4-Chlorophenyl)-3-[1-[4-[1-(methanesulfonamido)-cyclopropyl]-phenyl]-3-(5-methoxy-pyridin-2-yl)-piperidin-4-yl]-urea
**Ex-331** trans-1-(4-Chlorophenyl)-3-[1-[4-[1-(methanesulfonamido)-1-methyl-ethyl]-phenyl]-3-(5-methoxy-pyridin-2-yl)-piperidin-4-yl]-urea
**Ex-332** trans-1-(4-Chlorophenyl)-3-[1-[4-(methanesulfonamido-methyl)-phenyl]-3-(5-methoxy-pyridin-2-yl)-piperidin-4-yl]-urea
**Ex-333** trans-1-(4-Chlorophenyl)-3-[1-[5-[1-(methanesulfonamido)-cyclopropyl]-pyridin-2-yl]-3-(5-methoxy-pyridin-2-yl)-piperidin-4-yl]-urea
**Ex-334** trans-1-(4-Chlorophenyl)-3-[1-[5-[1-(methanesulfonamido)-1-methyl-ethyl]-pyridin-2-yl]-3-(5-methoxy-pyridin-2-yl)-piperidin-4-yl]-urea
**Ex-335** trans-1-(4-Chlorophenyl)-3-[1-[5-(methanesulfonamido-methyl)-pyridin-2-yl]-3-(5-methoxy-pyridin-2-yl)-piperidin-4-yl]-urea
**Ex-336** trans-1-(4-Chlorophenyl)-3-[1-[6-[1-(methanesulfonamido)-cyclopropyl]-pyridin-3-yl]-3-(5-methoxy-pyridin-2-yl)-piperidin-4-yl]-urea
**Ex-337** trans-1-(4-Chlorophenyl)-3-[1-[6-[1-(methanesulfonamido)-1-methyl-ethyl]-pyridin-3-yl]-3-(5-methoxy-pyridin-2-yl)-piperidin-4-yl]-urea
**Ex-338** trans-1-(4-Chlorophenyl)-3-[1-[6-(methanesulfonamido-methyl)-pyridin-3-yl]-3-(5-methoxy-pyridin-2-yl)-piperidin-4-yl]-urea
**Ex-339** trans-N-[1-[4-[4-[[(4-Chlorophenyl)-carbamoyl]amino]-3-(5-methoxy-pyridin-2-yl)-piperidin-1-yl]-phenyl]-cyclopropyl]-acetamide
**Ex-340** trans-N-[1-[4-[4-[[(4-Chlorophenyl)-carbamoyl]amino]-3-(5-methoxy-pyridin-2-yl)-piperidin-1-yl]-phenyl]-1-methyl-ethyl]-acetamide
**Ex-341** trans-N-[[4-[4-[[(4-Chlorophenyl)-carbamoyl]amino]-3-(5-methoxy-pyridin-2-yl)-piperidin-1-yl] -phenyl] -methyl] -acetamide
**Ex-342** trans-N-[1-[6-[4-[[(4-Chlorophenyl)-carbamoyl]amino]-3-(5-methoxy-pyridin-2-yl)-piperidin-1-yl]-pyridin-3-yl]-cyclopropyl]-acetamide
**Ex-343** trans-N-[1-[6-[4-[[(4-Chlorophenyl)-carbamoyl]amino]-3-(5-methoxy-pyridin-2-yl)-piperidin-1-yl]-pyridin-3-yl]-1-methyl-ethyl]-acetamide
**Ex-344** trans-N-[[6-[4-[[(4-Chlorophenyl)-carbamoyl]amino]-3-(5-methoxy-pyridin-2-yl)-piperidin-1-yl]-pyridin-3-yl]-methyl]-acetamide
**Ex-345** trans-N-[1-[5-[4-[[(4-Chlorophenyl)-carbamoyl]amino]-3-(5-methoxy-pyridin-2-yl)-piperidin-1-yl]-pyridin-2-yl]-cyclopropyl]-acetamide
**Ex-346** trans-N-[1-[5-[4-[[(4-Chlorophenyl)-carbamoyl]amino]-3-(5-methoxy-pyridin-2-yl)-piperidin-1-yl]-pyridin-2-yl]-1-methyl-ethyl]-acetamide
**Ex-347** trans-N-[[5-[4-[[(4-Chlorophenyl)-carbamoyl]amino]-3-(5-methoxy-pyridin-2-yl)-piperidin-1-yl]-pyridin-2-yl]-methyl]-acetamide
**Ex-348** trans-1-(4-Chlorophenyl)-3-[3-(5-methoxy-pyridin-2-yl)-1-(2H-pyrazol-3-yl)-piperidin-4-yl]-urea
**Ex-349** trans-1-(4-Chlorophenyl)-3-[3-(5-methoxy-pyridin-2-yl)-1-(1H-pyrazol-4-yl)-piperidin-4-yl]-urea
**Ex-350** trans-1-(4-Chlorophenyl)-3-[3-(5-methoxy-pyridin-2-yl)-1-(2-methyl-2H-pyrazol-3-yl)-piperidin-4-yl]-urea
**Ex-351** trans-1-(4-Chlorophenyl)-3-[3-(5-methoxy-pyridin-2-yl)-1-(1-methyl-1H-pyrazol-4-yl)-piperidin-4-yl]-urea
**Ex-352** trans-1-(4-Chlorophenyl)-3-[3-(5-methoxy-pyridin-2-yl)-1-(1-methyl-1H-pyrazol-3-yl)-piperidin-4-yl]-urea
**Ex-353** trans-1-(4-Chlorophenyl)-3-[3-(5-methoxy-pyridin-2-yl)-1-(2-methylsulfonyl-2H-pyrazol-3-yl)-piperidin-4-yl]-urea
**Ex-354** trans-1-(4-Chlorophenyl)-3-[3-(5-methoxy-pyridin-2-yl)-1-(1-methylsulfonyl-1H-pyrazol-4-yl)-piperidin-4- yl]-urea
**Ex-355** trans-1-(4-Chlorophenyl)-3-[3-(5-methoxy-pyridin-2-yl)-1-(1-methylsulfonyl-1H-pyrazol-3-yl)-piperidin-4-yl]-urea
**Ex-356** trans-1-(4-Chlorophenyl)-3-[3-(5-methoxy-pyridin-2-yl)-1-(1H-[1,2,3]triazol-4-yl)-piperidin-4-yl]-urea
**Ex-357** trans-1-(4-Chlorophenyl)-3-[3-(5-methoxy-pyridin-2-yl)-1-(1-methyl-1H-[1,2,3]triazol-4-yl)-piperidin-4-yl]-urea
**Ex-358** trans-1-(4-Chlorophenyl)-3-[3-(5-methoxy-pyridin-2-yl)-1-(3-methyl-3H-[1,2,3]triazol-4-yl)-piperidin-4-yl]-urea
**Ex-359** trans-1-(4-Chlorophenyl)-3 -[3 -(5-methoxy-pyridin-2-yl)-1-(1-methylsulfonyl-1H-[1,2,3]triazol-4-yl)-piperidin-4-yl]-urea
**Ex-360** trans-1-(4-Chlorophenyl)-3-[1-isoxazol-5-yl-3-(5-methoxy-pyridin-2-yl)-piperidin-4-yl]-urea
**Ex-361** trans-1-(4-Chlorophenyl)-3-[1-isoxazol-4-yl-3-(5-methoxy-pyridin-2-yl)-piperidin-4-yl]-urea
**Ex-362** trans-1-(4-Chlorophenyl)-3-[1-isoxazol-3-yl-3-(5-methoxy-pyridin-2-yl)-piperidin-4-yl]-urea
**Ex-363** trans-1-(4-Chlorophenyl)-3-[3-(5-methoxy-pyridin-2-yl)-1-oxazol-2-yl-piperidin-4-yl]-urea
**Ex-364** trans-1-(4-Chlorophenyl)-3-[3-(5-methoxy-pyridin-2-yl)-1-oxazol-4-yl-piperidin-4-yl]-urea
**Ex-365** trans-1-(4-Chlorophenyl)-3-[3-(5-methoxy-pyridin-2-yl)-1-oxazol-5-yl-piperidin-4-yl]-urea
**Ex-366** trans-1-(4-Chlorophenyl)-3-[3-(5-methoxy-pyridin-2-yl)-1-thiazol-2-yl-piperidin-4-yl]-urea
**Ex-367** trans-1-(4-Chlorophenyl)-3-[3-(5-methoxy-pyridin-2-yl)-1-thiazol-4-yl-piperidin-4-yl]-urea
**Ex-368** trans-1-(4-Chlorophenyl)-3-[3-(5-methoxy-pyridin-2-yl)-1-thiazol-5-yl-piperidin-4-yl]-urea
**Ex-369** trans-1-(4-Chlorophenyl)-3-[3-(5-methoxy-pyridin-2-yl)-1-(2H-[1,2,4]triazol-3-yl)-piperidin-4-yl]-urea
**Ex-370** trans-1-(4-Chlorophenyl)-3-[3-(5-methoxy-pyridin-2-yl)-1-(1-methyl-1H-[1,2,4]triazol-3-yl)-piperidin-4-yl]-urea
**Ex-371** trans-1-(4-Chlorophenyl)-3-[3-(5-methoxy-pyridin-2-yl)-1-(1-methylsulfonyl-1H-[1,2,4]triazol-3-yl)-piperidin-4-yl]-urea
**Ex-372** trans-1-(4-Chlorophenyl)-3-[3-(5-methoxy-pyridin-2-yl)-1-([1,2,4]oxadiazol-5-yl)-piperidin-4-yl]-urea
**Ex-373** trans-1-(4-Chlorophenyl)-3-[3-(5-methoxy-pyridin-2-yl)-1-([1,2,4]oxadiazol-3 -yl)-piperidin-4-yl]-urea
**Ex-374** trans-1-(4-Chlorophenyl)-3-[3-(5-methoxy-pyridin-2-yl)-1-([1,3,4]oxadiazol-2-yl)-piperidin-4-yl]-urea
**Ex-375** trans-1-(4-Chlorophenyl)-3-[1-(1H-indazol-4-yl)-3-(5-methoxy-pyridin-2-yl)-piperidin-4-yl]-urea
**Ex-376** trans-1-(4-Chlorophenyl)-3-[1-(1H-indazol-5-yl)-3-(5-methoxy-pyridin-2-yl)-piperidin-4-yl]-urea
**Ex-377** trans-1-(4-Chlorophenyl)-3-[1-(1H-indazol-7-yl)-3-(5-methoxy-pyridin-2-yl)-piperidin-4-yl]-urea
**Ex-378** trans-1-(4-Chlorophenyl)-3-[1-(1H-indazol-6-yl)-3-(5-methoxy-pyridin-2-yl)-piperidin-4-yl]-urea
**Ex-379** trans-1-(4-Chlorophenyl)-3-[3-(5-methoxy-pyridin-2-yl)-1-(1H-pyrazolo[3,4-b]pyridin-4-yl)-piperidin-4-yl]-urea
**Ex-380** trans-1-(4-Chlorophenyl)-3-[3-(5-methoxy-pyridin-2-yl)-1-(1H-pyrazolo[4,3-b]pyridin-7-yl)-piperidin-4-yl]-urea
**Ex-381** trans-1-(4-Chlorophenyl)-3-[3-(5-methoxy-pyridin-2-yl)-1-(1H-pyrazolo[3,4-b]pyridin-5-yl)-piperidin-4-yl]-urea
**Ex-382** trans-1-(4-Chlorophenyl)-3-[3-(5-methoxy-pyridin-2-yl)-1-(1H-pyrazolo[4,3-b]pyridin-6-yl)-piperidin-4-yl]-urea
**Ex-383** trans-1-(4-Chlorophenyl)-3-[3-(5-methoxy-pyridin-2-yl)-1-(3-oxo-1,2-dihydro-isoindol-4-yl)-piperidin-4-yl]-urea
**Ex-384** trans-1-(4-Chlorophenyl)-3-[3-(5-methoxy-pyridin-2-yl)-1-(1-oxo-2,3-dihydro-isoindol-4-yl)-piperidin-4-yl]-urea
**Ex-385** trans-1-(4-Chlorophenyl)-3-[3-(5-methoxy-pyridin-2-yl)-1-(3-oxo-1,2-dihydro-isoindol-5-yl)-piperidin-4-yl]-urea
**Ex-386** trans-1-(4-Chlorophenyl)-3-[3-(5-methoxy-pyridin-2-yl)-1-(1-oxo-2,3-dihydro-isoindol-5-yl)-piperidin-4-yl]-urea
**Ex-387** trans-1-(4-Chlorophenyl)-3-[3-(5-methoxy-pyridin-2-yl)-1-(2-oxo-1,3-dihydro-indol-7-yl)-piperidin-4-yl]-urea
**Ex-388** trans-1-(4-Chlorophenyl)-3-[3-(5-methoxy-pyridin-2-yl)-1-(2-oxo-1,3-dihydro-indol-4-yl)-piperidin-4-yl]-urea
**Ex-389** trans-1-(4-Chlorophenyl)-3-[3-(5-methoxy-pyridin-2-yl)-1-(2-oxo-1,3-dihydro-indol-6-yl)-piperidin-4-yl]-urea
**Ex-390** trans-1-(4-Chlorophenyl)-3-[3-(5-methoxy-pyridin-2-yl)-1-(2-oxo-1,3-dihydro-indol-5-yl)-piperidin-4-yl]-urea
in the form of the free compound or a physiologically acceptable salt thereof.

13. A pharmaceutical dosage form comprising a compound according to any of claims 1 to 12.

14. A compound according to any of claims 1 to 12 for use in the treatment and/or prophylaxis of a disorder which is mediated at least in part by FPR2.

15. The compound according to claim 14 for use in the treatment and/or prophylaxis of a disorder selected from the group consisting of inflammatory diseases, diabetes, obstructive airway diseases, autoimmune diseases, allergic conditions, rheumatological disorders, HIV-mediated retroviral 5 infections, infectious diseases, sepsis, cardiovascular disorders, fibrotic disorders, neuroinflammation, neurological disorders, pain, prion-mediated diseases, amyloid-mediated disorders and Graft versus Host Disease (GvHD).
